# EUROPEAN PATENT APPLICATION

(11) **EP 4 834 373 A1**
(43) Date of publication of application: **04.11.2026**
(21) Application number: 24911503.1
(22) Date of filing: 27.12.2024
(51) Int. Cl.: A61K 47/68, A61K 31/52, A61K 31/708, A61P 35/00, C07H 21/00

(54) **CYCLIC DINUCLEOTIDE COMPOUND, CONJUGATE THEREOF, AND USE THEREOF**

(30) Priority: 27.12.2023 CN 202311818539
(71) Applicant: Shanghai de Novo Pharmatech Co., Ltd., Shanghai 201203 (CN)
(72) Inventor: ZHANG, Wei, Shanghai 201203 (CN); QIN, Ping, Shanghai 201203 (CN); WANG, Longsheng, Shanghai 201203 (CN); YANG, Wei, Shanghai 201203 (CN); LI, Meng, Shanghai 201203 (CN); GAO, Daxin, Shanghai 201203 (CN)
(74) Representative: IK-IP LTD
(86) International application number: PCT/CN2024/143264
(87) International publication number: WO 2025/140587

(57) **Abstract**

Disclosed in the present invention are a cyclic dinucleotide compound, a conjugate thereof, and use thereof, and particularly disclosed is an antibody-immunostimulant conjugate represented by formula II or a pharmaceutically acceptable salt thereof. The antibody-immunostimulant conjugate provided by the present invention has good tumor specificity and exhibits a significant inhibition effect on tumors.

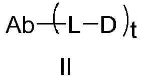

## Description

The present application claims priority to Chinese patent application 2023118185393 filed on December 27, 2023. The contents of the above Chinese patent applications are incorporated herein by reference in their entirety.

### TECHNICAL FIELD

The present disclosure relates to a cyclic dinucleotide compound, a conjugate thereof, a pharmaceutical composition thereof, and a use thereof.

### BACKGROUND

The stimulator of interferon genes (STING), also known as T1MEM173, MITA, MPYS, and ERIS, is an important signaling molecule in innate immune signaling. The protein encoded by this gene contains a five-transmembrane structure and plays an important regulatory role in immune responses associated with viral or bacterial infections. As a pattern recognition receptor, STING can detect and recognize exogenous nucleic acids in the cytoplasm and activate the signal transduction pathways related to type I interferon responses. In addition, studies have shown that STING regulates apoptotic signaling through its interaction with major histocompatibility complex class II (MHCII). Studies on human tumors with spontaneous T-cell infiltration have shown that CD8+ T-cell infiltration is closely associated with the transcriptional signature of type I interferon (Harlin et al, Cancer Res, 2009; 69(7): OF1). Mechanistic studies conducted on mouse models revealed that in experimental animals with defects in type I interferon signaling, the process of T cell activation against tumor-associated antigens is abnormal (Diamond et al, J. Exp. Med., 2011; 208(10):1989; Fuerte et al, J. Exp. Med., 2011; 208(10):2005). Further studies on the recognition process of tumors by the innate immune system *in vivo,* as well as on the related signaling pathways in which antigen-presenting cells (APCs) mediate IFN expression induced by tumors during this process, have revealed that the STING signaling pathway can be activated by DNA in the cytoplasm. These exogenous nucleic acids can be recognized by cyclic-GMP-AMP synthase (cGAS), which then catalyzes the generation of cyclic nucleic acid substances such as cyclic GMP-AMP (cGAMP). These cyclic nucleic acid substances can serve as endogenous ligands to activate STING signaling (Sun et al, Science, 2013; 339(15): 786). Activated STING can subsequently induce the autophosphorylation of TBK1 kinase and the phosphorylation of interferon regulatory factor 3 (IRF-3). Phosphorylated IRF3 can further activate the gene transcription process of type I interferon, regulate the synthesis and secretion of type I interferon, and consequently induce an immune response. In summary, existing studies have shown that the STING signaling pathway plays an extremely important role in the tumor recognition process involving the innate immune system. The activation of this signaling pathway on antigen-presenting cells is directly related to the T cell activation process against tumor-associated antigens. Based on its role in tumor immune recognition, it can be expected that activating the STING signal through drugs or other pharmacological methods could enhance the expression of IFN and produce positive effects on tumor treatment. Therefore, the development of STING signal agonists for the treatment of tumor diseases has become a research focus.

In addition, studies have shown that stimulating the activation of the STING signaling pathway also contributes to antiviral responses. Loss of functional response at the cellular or organismal level demonstrates that without STING, viral load cannot be controlled. The activation of the STING signaling pathway triggers immune responses, leading to anti-angiogenic and proinflammatory cytokines counteracting the virus, and mobilizing the innate and adaptive immune systems. Therefore, small-molecule compounds that have agonistic effects on the STING signaling pathway possess the potential to treat chronic viral infections, such as treatment of HBV.

At present, cyclic dinucleotide (CDN) analog STING agonists are mostly administered through intratumoral injection routes, and this administration method has several drawbacks, such as tumor accessibility for intratumoral injection, tumor exposure, and nonspecificity issues. Currently, no ADC drugs of CDN analogs have been marketed.

### SUMMARY

The technical problem to be solved by the present disclosure is to overcome the defects of existing cyclic dinucleotide analogs as STING agonists, such as single administration route, poor tumor accessibility, and weak specificity of interaction with tumors, thereby providing a cyclic dinucleotide compound, an antibody conjugate thereof, a pharmaceutical composition thereof, and a use thereof. The antibody conjugate of the cyclic dinucleotide compound of the present disclosure exhibits good tumor specificity and demonstrates significant inhibitory effects on tumors.

The present disclosure solves the above technical problems through the following technical solutions.

The present disclosure provides an immune-stimulating antibody conjugate represented by Formula II or a pharmaceutically acceptable salt thereof, 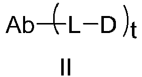
wherein Ab is an antibody;
t is 1 to 8;
L is a linker with the following combination: -(L₁)ₐ-(Z)_{b}-M-; L₁ is connected to D, and M is connected to Ab;
a is 0, 1, 2, 3, 4, 5, or 6; b is 0, 1, 2, 3, 4, 5, 6, 7, 8, 9, or 10;
D is a group formed by the loss of one hydrogen atom from a compound represented by 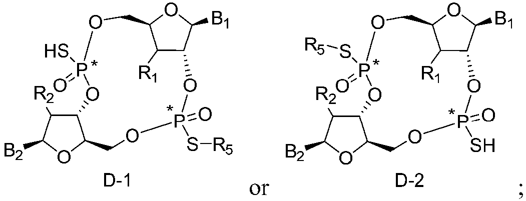 the ^{*}-marked P stereoconfigurations are each independently R, S, or R/S;
B₁ and B₂ are each independently 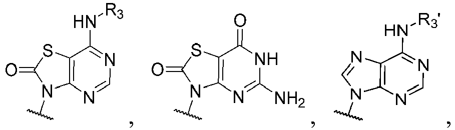 or 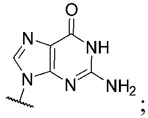 and at least one of B₁ or B₂ is 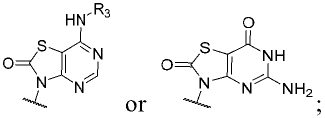
R₁ and R₂ are independently -OH, -N₃, F, -OCH₃, or -SCF₃;
R₃ and R_{3'} are independently H, -C(O)R₄, -C(O)OR₄, -OR₄, or -R₄;
R₄ is C₁₋₁₀ alkyl, C₆₋₁₀ aryl, 5- to 10-membered heteroaryl, C₃₋₁₀ cycloalkyl, 3- to 10-membered heterocycloalkyl, C₆₋₁₀ aryl-C₁₋₁₀ alkyl, 5- to 10-membered heteroaryl-C₁₋₁₀ alkyl, C₃₋₁₀ cycloalkyl-C₁₋₁₀ alkyl, or 3- to 10-membered heterocycloalkyl-C₁₋₁₀ alkyl; the R₄ is unsubstituted or optionally substituted at any position with 1 to 3 substituents selected from hydroxyl, thiol, dithiol, amino, C₁₋₆ alkylamino, and C₁₋₆ alkylamino-C₁₋₁₀ alkyl;
R₅ is H, -(L₂)_{d}-(Z)ₑ-(maleimido), or -(L₂)_{d}-(Z)ₑ-H;
d is 0, 1, 2, 3, 4, 5, or 6; e is 0, 1, 2, 3, 4, 5, 6, 7, 8, 9, or 10;
each L₁ and each L₂ is independently 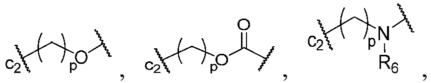 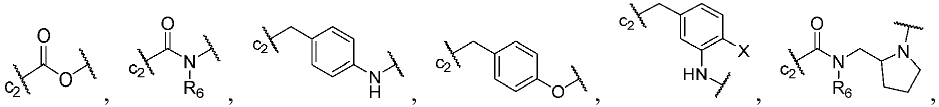 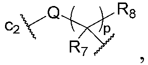 or 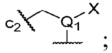 in L₁, the c₂ side is connected to D; in L₂, the c₂ side is connected to the thiol group in D;
X is 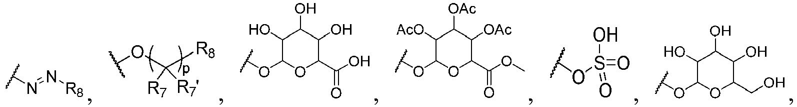 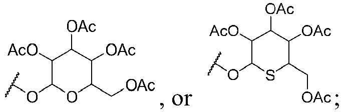
p is independently 1, 2, or 3;
Q is a linking bond, -C(O)O-, -C(O)N(R₆)-, or -C₁₋₃ alkylene-O-;
Q₁ is phenyl or pyridyl;
R₆ is H or C₁₋₆ alkyl; the C₁₋₆ alkyl is unsubstituted or optionally substituted at any position with one C₁₋₆ alkylamino or C₁₋₆ alkylsulfonyl;
R₇ and R_{7'} are each independently H or C₁₋₆ alkyl;
R₈ is phenyl or 5- to 10-membered heteroaryl; the R₈ is unsubstituted or optionally substituted at any position with 1-3 substituents selected from hydroxyl, amino, carboxyl, cyano, nitro, thiol, C₁₋₆ alkyl, C₃₋₈ cycloalkyl, halogen, halo-C₁₋₆ alkyl, C₁₋₆ alkylamino, C₁₋₆ alkoxy, halo-C₁₋₆ alkoxy, C₁₋₆ alkylthio, amido, hydroxyamino, aldehyde, acetyl, methoxycarbonyl, amino-C₁₋₆ alkyl, hydroxy-C₁₋₆ alkyl, and C₁₋₆ alkylsulfonyl;
each Z is independently -(A)ᵥ-, -PEG-, -C(O)-(CH₂)ₓ-, -NR₉-(CH₂)_{y}-, -O-(CH₂)_{y}-, -S-(CH₂)_{y}-, -(CH₂)ₓ-C₆₋₁₄ arylene-(CH₂)_{y}-, -(CH₂)ₓ-5- to 6-membered heteroarylene-(CH₂)_{y}-, - (CH₂)ₓ-C₃₋₆ cycloalkylene-(CH₂)_{y}-, -(CH₂)ₓ-3- to 6-membered heterocycloalkylene-(CH₂)_{y}-, - NR₉-(CH₂)ₓ-C(O)-, -O-(CH₂)ₓ-C(O)-, -S-(CH₂)ₓ-C(O)-, -(CH₂)ₓCH(-L₃-R₉)-C(O)-, - (CH₂)ₓCH(-L₃-R₉ₐ)-C(O)-, -(CH₂)ₓ-S-S-(CH₂)_{y}-, C₁₋₆ alkylene, or C₂₋₆ alkenylene;
L₃ is a linking bond, -NH-, or 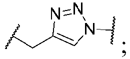
R₉ is independently hydrogen, C₁₋₆ alkyl, -PO(OH)₂, -PO(OCH₃)₂, -C(O)-(CH₂CH₂O)ₙ-CH₃, -(CH₂CH₂O)ₙ-CH₃, 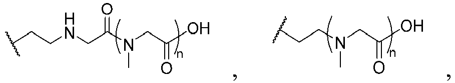 or 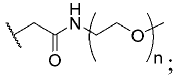
R₉ₐ is independently 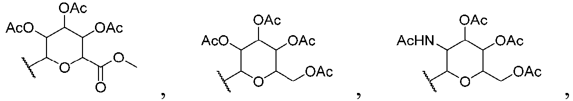 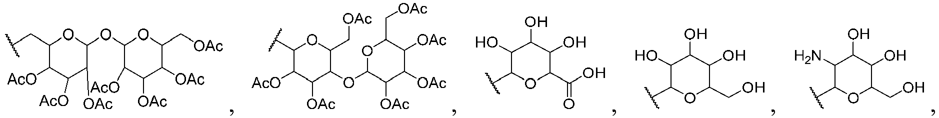 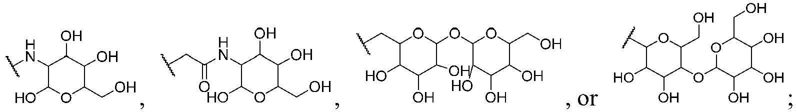
-PEG- is -(CH₂CH₂O)ₙ-(CH₂)ᵤ- or -(CH₂CH₂O)ₙ-(CH₂)ᵤ-C(O)-;
A is independently an amino acid residue;
x and y are independently 0, 1, 2, 3, 4, 5, 6, 7, 8, 9, or 10; v is independently 1, 2, 3, 4, or 5; n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50; u is independently 0, 1, 2, 3, 4, or 5;
M is a linker head connected to Ab.

In some embodiments, in the immune-stimulating antibody conjugate represented by Formula II or the pharmaceutically acceptable salt thereof, some of the groups are defined as follows, and the rest of the groups are defined as described in any other embodiment (hereinafter referred to as "in some embodiments"):
Ab is an antibody;
t is 1 to 8;
L is a linker with the following combination: -(L₁)ₐ-(Z)_{b}-M-; L₁ is connected to D, and M is connected to Ab;
a is 0, 1, 2, 3, 4, 5, or 6; b is 0, 1, 2, 3, 4, 5, 6, 7, 8, 9, or 10;
D is a group formed by the loss of one hydrogen atom from a compound represented by 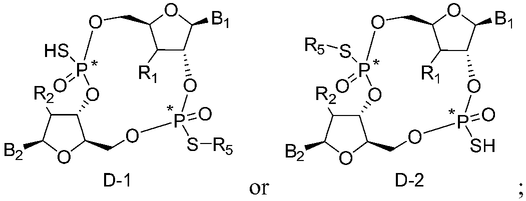 the ^{*}-marked P stereoconfigurations are each independently R, S, or R/S;
B₁ and B₂ are each independently 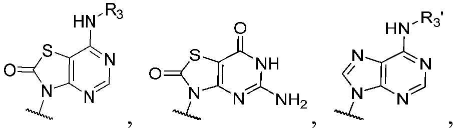 or 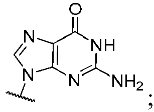 and at least one of B₁ or B₂ is 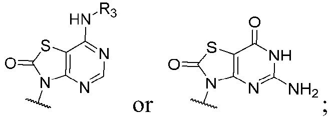
R₁ and R₂ are independently -OH, -N₃, F, -OCH₃, or -SCF₃;
R₃ and R_{3'} are independently H, -C(O)R₄, -C(O)OR₄, -OR₄, or -R₄;
R₄ is C₁₋₁₀ alkyl, C₆₋₁₀ aryl, 5- to 10-membered heteroaryl, C₃₋₁₀ cycloalkyl, 3- to 10-membered heterocycloalkyl, C₆₋₁₀ aryl-C₁₋₁₀ alkyl, 5- to 10-membered heteroaryl-C₁₋₁₀ alkyl, C₃₋₁₀ cycloalkyl-C₁₋₁₀ alkyl, or 3- to 10-membered heterocycloalkyl-C₁₋₁₀ alkyl; the R₄ is unsubstituted or optionally substituted at any position with 1 to 3 substituents selected from hydroxyl, thiol, dithiol, amino, C₁₋₆ alkylamino, and C₁₋₆ alkylamino-C₁₋₁₀ alkyl;
R₅ is H, -(L₂)_{d}-(Z)ₑ-(maleimido), or -(L₂)_{d}-(Z)ₑ-H;
d is 0, 1, 2, 3, 4, 5, or 6; e is 0, 1, 2, 3, 4, 5, 6, 7, 8, 9, or 10;
each L₁ and each L₂ is independently 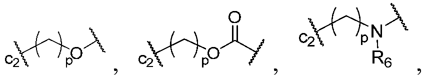 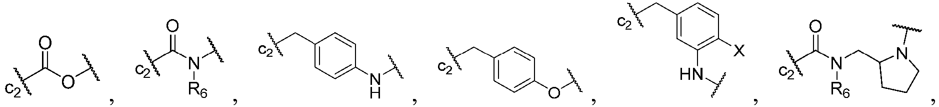 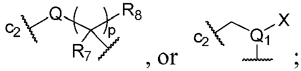 the c₂ side is connected to D;
X is 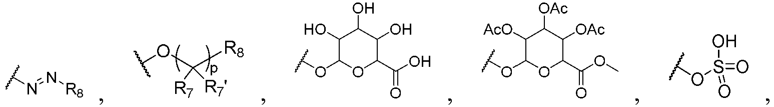 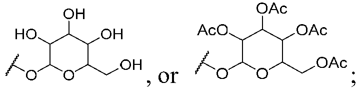
p is independently 1, 2, or 3;
Q is a linking bond, -C(O)O-, -C(O)N(R₆)-, or -C₁₋₃ alkylene-O-;
Q₁ is phenyl or pyridyl;
R₆ is H or C₁₋₆ alkyl; the C₁₋₆ alkyl is unsubstituted or optionally substituted at any position with one C₁₋₆ alkylamino or C₁₋₆ alkylsulfonyl;
R₇ and R_{7'} are each independently H or C₁₋₆ alkyl;
R₈ is phenyl or 5- to 10-membered heteroaryl; the R₈ is unsubstituted or optionally substituted at any position with 1-3 substituents selected from hydroxyl, amino, carboxyl, cyano, nitro, thiol, C₁₋₆ alkyl, C₃₋₈ cycloalkyl, halogen, halo-C₁₋₆ alkyl, C₁₋₆ alkylamino, C₁₋₆ alkoxy, halo-C₁₋₆ alkoxy, C₁₋₆ alkylthio, amido, hydroxyamino, aldehyde, acetyl, methoxycarbonyl, amino-C₁₋₆ alkyl, hydroxy-C₁₋₆ alkyl, and C₁₋₆ alkylsulfonyl;
each Z is independently -(A)ᵥ-, -PEG-, -C(O)-(CH₂)ₓ-, -NR₉-(CH₂)_{y}-, -O-(CH₂)_{y}-, -S-(CH₂)_{y}-, -(CH₂)ₓ-C₆₋₁₄ arylene-(CH₂)_{y}-, -(CH₂)ₓ-5- to 6-membered heteroarylene-(CH₂)_{y}-, - (CH₂)ₓ-C₃₋₆ cycloalkylene-(CH₂)_{y}-, -(CH₂)ₓ-3- to 6-membered heterocycloalkylene-(CH₂)_{y}-, - NR₉-(CH₂)ₓ-C(O)-, -O-(CH₂)ₓ-C(O)-, -S-(CH₂)ₓ-C(O)-, -(CH₂)ₓCH(NHR₉)-C(O)-, -(CH₂)ₓ-S-S-(CH₂)_{y}-, C₁₋₆ alkylene, or C₂₋₆ alkenylene;
R₉ is independently hydrogen, C₁₋₆ alkyl, -PO(OH)₂, -PO(OCH₃)₂, -C(O)-(CH₂CH₂O)ₙ-CH₃, or -(CH₂CH₂O)ₙ-CH₃; -PEG- is -(CH₂CH₂O)ₙ-(CH₂)ᵤ- or -(CH₂CH₂O)ₙ-(CH₂)ᵤ-C(O)-;
A is independently an amino acid residue;
x and y are independently 0, 1, 2, 3, 4, 5, 6, 7, 8, 9, or 10; v is independently 1, 2, 3, 4, or 5; n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50; u is independently 0, 1, 2, 3, 4, or 5;
M is a linker head connected to Ab.

In some embodiments, the antibody may comprise one or more antigen-binding domains capable of binding to an antigen.

In some embodiments, the antibody may comprise one or two antigen-binding domains capable of binding to an antigen.

In some embodiments, the antibody may comprise one antigen-binding domain capable of binding to an antigen.

In some embodiments, the antibody may comprise only one antigen-binding domain capable of binding to an antigen.

In some embodiments, the antibody may comprise one Fc region.

In some embodiments, the antibody may comprise only one Fc region.

In some embodiments, the antibody may comprise only one antigen-binding domain capable of binding to an antigen and one Fc region.

In some embodiments, the antibody may be a monoclonal antibody.

In some embodiments, the antibody is an anti-HER2 antibody, an anti-EGFR antibody, or an anti-5T4 antibody.

In some embodiments, the anti-HER2 monoclonal antibody includes, but is not limited to: Trastuzumab, Trastuzumab biosimilar, Pertuzumab, Pertuzumab biosimilar, Margetuximab, HT-19, *etc.*

In some embodiments, the antibody is Trastuzumab or Pertuzumab.

In some embodiments, the anti-EGFR antibody includes, but is not limited to: Cetuximab.

In some embodiments, the anti-EGFR antibody is Cetuximab or Nimotuzumab.

In some embodiments, the anti-5T4 antibody includes, but is not limited to: huA1.

In some embodiments, the antibody is Trastuzumab, Pertuzumab, Cetuximab, or huA1.

In some embodiments, the antibody is Trastuzumab, Pertuzumab, Cetuximab, huA1, Cetuximab, Nimotuzumab; or a variant thereof.

In the immune-stimulating antibody conjugate represented by Formula II or the pharmaceutically acceptable salt thereof, the t is an integer or a non-integer. When it is a non-integer, it means that the immune-stimulating antibody conjugate represented by Formula II is a mixture of immune-stimulating antibody conjugates with different drug-to-antibody ratios; when it is an integer, it may mean that the immune-stimulating antibody conjugate represented by Formula II is a single immune-stimulating antibody conjugate with a fixed drug-to-antibody ratio, or it may mean that the immune-stimulating antibody conjugate represented by Formula II is a mixture of immune-stimulating antibody conjugates with different drug-to-antibody ratios.

In some embodiments, the t may be any value from 2 to 8.

In some embodiments, the t may be any value from 1 to 3.

In some embodiments, the t may be any value from 3 to 5.

In some embodiments, the t may be any value from 6 to 8.

In some embodiments, a is 0, 1, 2, 3, or 4; a is preferably 0, 1, or 2.

In some embodiments, b is 1, 2, 3, 4, 5, 6, 7, 8, 9, or 10; b is preferably 1, 2, 3, 4, 5, 6, 7, or 8; b is more preferably 1, 2, 3, 4, 5, or 6.

In some embodiments, d is 0, 1, 2, or 3; d is preferably 1 or 2; d is more preferably 1.

In some embodiments, e is 0, 1, 2, 3, 4, 5, or 6; e is preferably 0, 1, 2, 3, or 4; e is more preferably 0, 1, 2, or 3.

In some embodiments, R₆ is H or C₁₋₆ alkyl; the C₁₋₆ alkyl is unsubstituted or optionally substituted at any position with one dimethylamino or methylsulfonyl.

In some embodiments, R₆ is H, -CH₃, -CH₂CH₂N(CH₃)₂, or -CH₂CH₂S(O)₂CH₃.

In some embodiments, each L₁ and each L₂ is independently 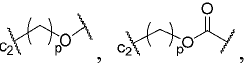 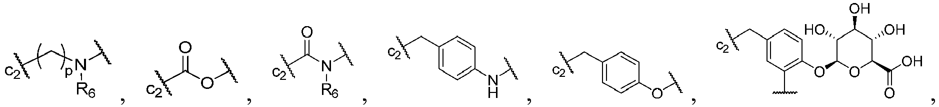 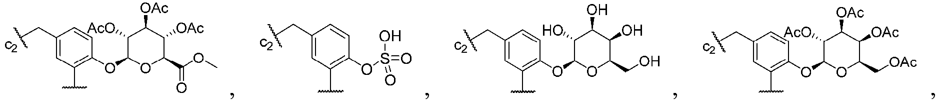 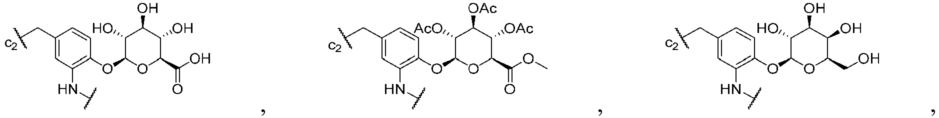 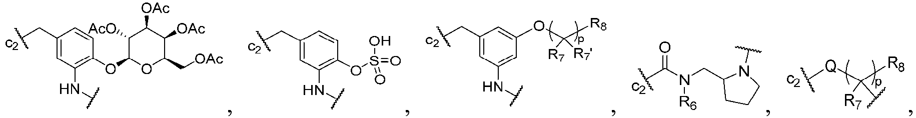 or 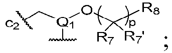 in L₁, the c₂ side is connected to D; in L₂, the c₂ side is connected to the thiol group in D.

In some embodiments, each L₁ and each L₂ is independently 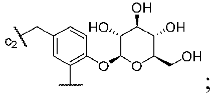 in L₁, the c₂ side is connected to D; in L₂, the c₂ side is connected to the thiol group in D.

In some embodiments, -(L₁)ₐ- is 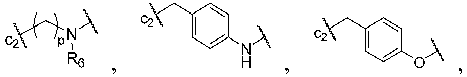 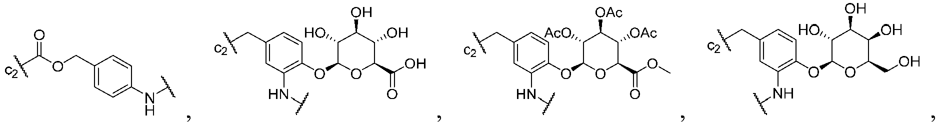 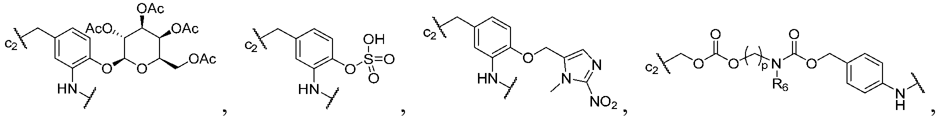 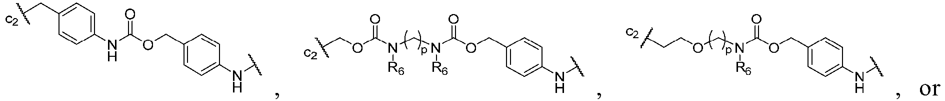 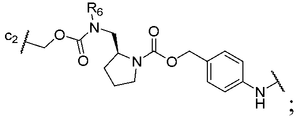 the c₂ side is connected to D.

In some embodiments, -(L₁)ₐ- is 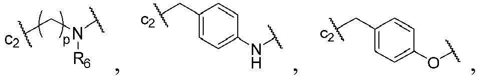 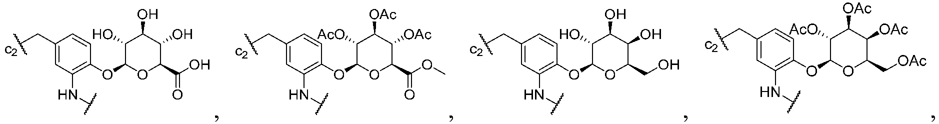 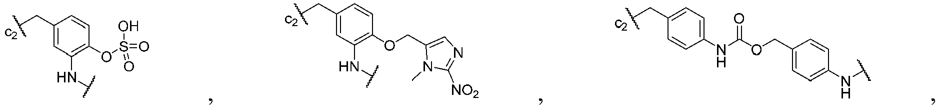 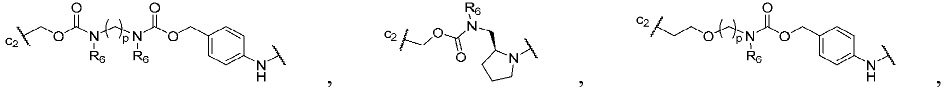 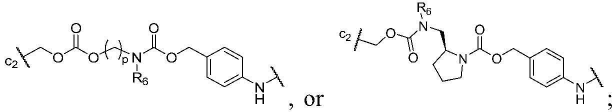 the c₂ side is connected to the thiol group in D.

In some embodiments, -(L₁)ₐ- is 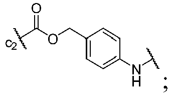 the c₂ side is connected to the amino in D.

In some embodiments, -(L₁)ₐ- is 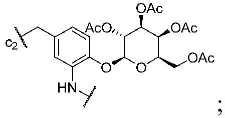 the c₂ side is connected to the amino in D.

In some embodiments, -(L₁)ₐ- is absent, 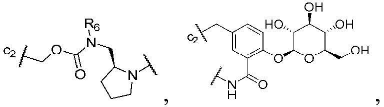 or 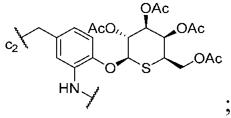 the c₂ side is connected to the thiol group in D.

In some embodiments, Z is independently -(A)ᵥ-, -PEG-, -C(O)-(CH₂)ₓ-, -NR₉-(CH₂)_{y}-, -O-(CH₂)_{y}-, -S-(CH₂)_{y}-, -(CH₂)ₓ-C₆₋₁₄ arylene-(CH₂)_{y}-, -(CH₂)ₓ-5- to 6-membered heteroarylene-(CH₂)_{y}-, -(CH₂)ₓ-C₃₋₆ cycloalkylene-(CH₂)_{y}-, -(CH₂)ₓ-3- to 6-membered heterocycloalkylene-(CH₂)_{y}-, -NR₉-(CH₂)ₓ-C(O)-, -O-(CH₂)ₓ-C(O)-, -S-(CH₂)ₓ-C(O)-, - (CH₂)ₓCH(NHR₉)-C(O)-, -(CH₂)ₓ-S-S-(CH₂)_{y}-, C₁₋₆ alkylene, or C₂₋₆ alkenylene; x and y are independently any integer of 0, 1, 2, 3, 4, 5, 6, 7, 8, 9 or 10; v is independently any integer of 1, 2, 3, 4 or 5; in the "5- to 6-membered heteroarylene" of -(CH₂)ₓ-5- to 6-membered heteroarylene-(CH₂)_{y}-, the heteroatom is selected from one or more kinds of N, O and S, and the number of heteroatoms is 1, 2 or 3; in the "3- to 6-membered heterocycloalkylene" of - (CH₂)ₓ-3- to 6-membered heterocycloalkylene-(CH₂)_{y}-, the heteroatom is selected from one or more kinds of N, O and S, and the number of heteroatoms is 1, 2 or 3.

In some embodiments, in A, the amino acid is a natural amino acid or a non-natural amino acid.

In some embodiments, in A, the amino acid residue is modified with 1 to 3 R₁₀; R₁₀ is independently C₁₋₆ alkyl, C₁₋₆ alkylacyl, C₁₋₆ alkoxyacyl, -C(O)-(CH₂CH₂O)ₙ-CH₃, or - (CH₂CH₂O)ₙ-CH₃; the C₁₋₆ alkyl, C₁₋₆ alkylacyl, or C₁₋₆ alkoxyacyl may further be substituted with one R₈ at any position.

In some embodiments, each A is independently 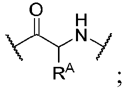 each R^{A} is independently an amino acid side chain or an amino acid side chain modified with 1 to 3 R₁₀; or R^{A} and the adjacent nitrogen atom form a five-membered heterocyclic group;
the R₁₀ is independently C₁₋₆ alkyl, C₁₋₆ alkylacyl, C₁₋₆ alkoxyacyl, -C(O)-(CH₂CH₂O)ₙ-CH₃, or -(CH₂CH₂O)ₙ-CH₃; the C₁₋₆ alkyl, C₁₋₆ alkylacyl, or C₁₋₆ alkoxyacyl may further be substituted with one R₈ at any position.

In some embodiments, the 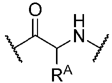 may be 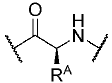 or 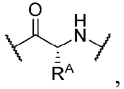 or may also be

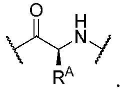

In some embodiments, in A, each R^{A} is independently hydrogen, methyl, ethyl, n-propyl, isopropyl, isobutyl, 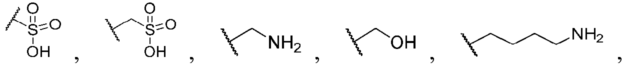 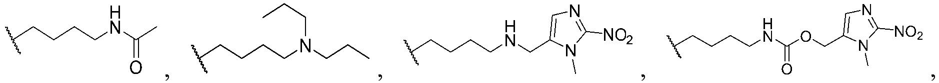 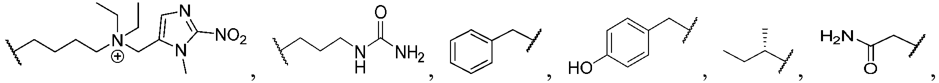 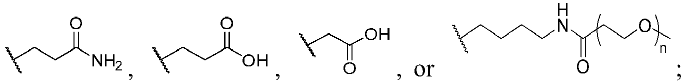 or R^{A} and the adjacent nitrogen atom form a five-membered heterocyclic group.

In some embodiments, -(A)ᵥ- is 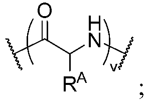 each R^{A} is independently hydrogen, methyl, ethyl, n-propyl, isopropyl, isobutyl, 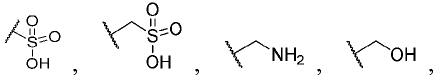 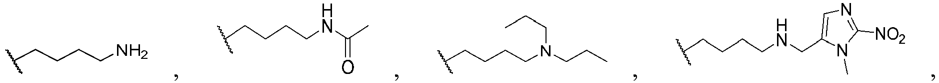 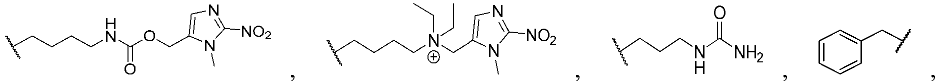 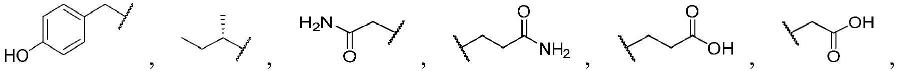 or 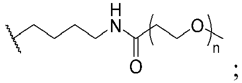 or R^{A} and the adjacent nitrogen atom form a five-membered heterocyclic group; v is 1, 2, 3, or 4, preferably 1 or 2.

In some embodiments, -(A)ᵥ- is

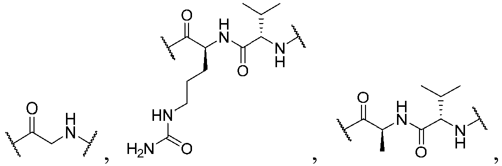

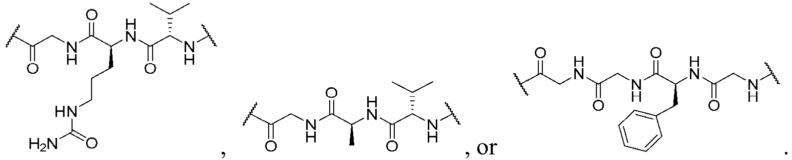

In some embodiments, -(A)ᵥ- is 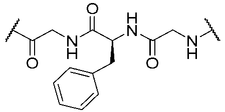 or

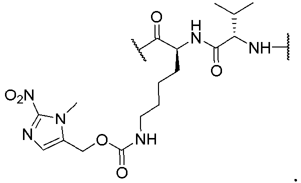

In some embodiments, -(Z)_{b}- is any one of the following combinations:
1) -(CH₂)ₓ-(c₁),
2) -(CH₂CH₂O)ₙ-(CH₂)ᵤ-NHC(O)-(CH₂)ₓ- (c₁),
3) -C(O)-(CH₂)ₓ-(c₁),
4) -C(O)-(CH₂)ₓ-C₃₋₆ cycloalkylene-(CH₂)_{y}-(c₁),
5) -C(O)-(CH₂)ₓ-phenylene-(CH₂)_{y}-(c₁),
6) -C(O)-(CH₂CH₂O)ₙ-(CH₂)ᵤ-NHC(O)-(CH₂)ₓ-(c₁),
7) -C(O)-(CH₂CH₂O)ₙ-(CH₂)ᵤ-NHC(O)-(CH₂)ₓ-phenylene-(CH₂)_{y}-(c₁),
8) -(A)ᵥ-(c₁),
9) -(A)ᵥ-C(O)-(CH₂)ₓ-(c₁),
10) -(A)ᵥ-C(O)-(CH₂)ₓ-phenylene-(CH₂)_{y}-(c₁),
11) -(A)ᵥ-C(O)-(CH₂CH₂O)ₙ-(CH₂)ᵤ-NHC(O)-(CH₂)ₓ-(c₁), or
12) -(A)ᵥ-C(O)-(CH₂CH₂O)ₙ-(CH₂)ᵤ-NHC(O)-(CH₂)ₓ-phenylene-(CH₂)_{y}-(c₁); wherein the c₁ side is connected to M.

In some embodiments, -(Z)_{b}- is any one of the following combinations:
13) -C(O)-(CH₂)ₓ-(A)ᵥ-C(O)-(CH₂)ₓ-(c₁);
14) -(A)ᵥ-C(O)-(CH₂CH₂O)ₙ-(CH₂)ᵤ-(c₁);
15) -C(O)-(CH₂)ₓ-NH-(c₁);
16) -C(O)-CH(-L₃-R₉)-NHC(O)-(CH₂)ₓ-phenylene-(CH₂)_{y}-;
17) -C(O)-CH(-L₃-R₉)-NHC(O)-(CH₂)ₓ-, wherein the c₁ side is connected to M.

In some embodiments, in M, the linker head is a group formed through click reaction, click-like reaction, thiol ligation, amine ligation, oxime ligation, or hydrazone ligation.

In some embodiments, in M, the linker head is a group formed through thiol ligation on the side chain of antibody cysteine residues or amine ligation on the side chain of lysine residues.

In some embodiments, in M, the linker head is a group formed through thiol ligation, preferably 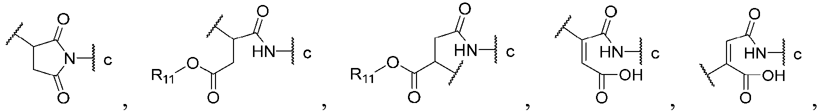 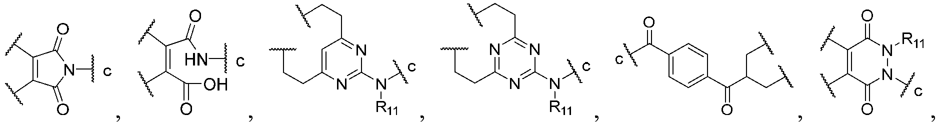 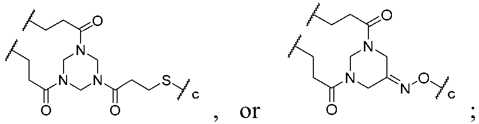 more preferably, M is 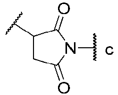 or 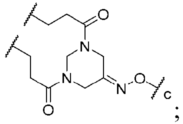 R₁₁ is hydrogen or C₁₋₄ alkyl; the linker head is connected to the rest of L through the c side.

In some embodiments, R₁₁ is hydrogen, methyl, or ethyl.

In some embodiments, in M, the linker head is a group formed through oxime ligation, preferably 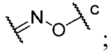 the linker head is connected to the rest of L through the c side.

In some embodiments, in M, the linker head is a group formed through hydrazone ligation, preferably 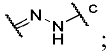 the linker head is connected to the rest of L through the c side.

In some embodiments, in M, the linker head is a group formed through amine ligation, preferably a group formed through lysine side chain amine ligation; more preferably

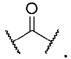

In some embodiments, the linker head is a group formed through a click reaction or click-like reaction, preferably: 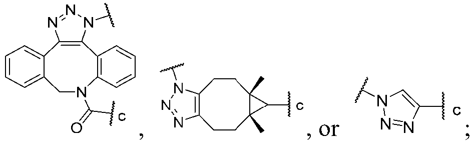 the linker head is connected to the rest of L through the c side.

In some embodiments, in M, the linker head is 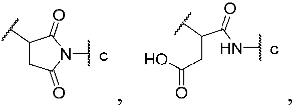 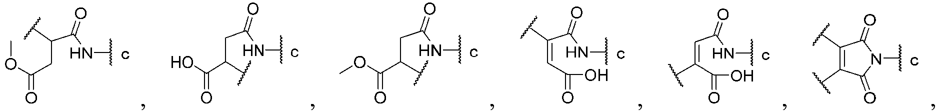 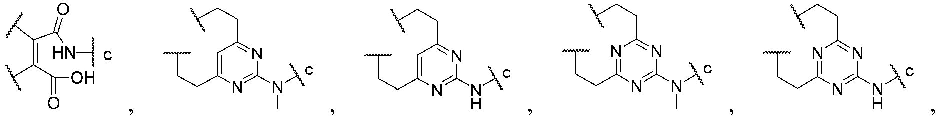 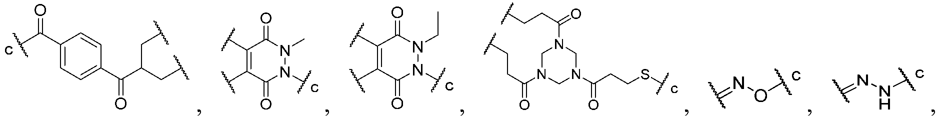 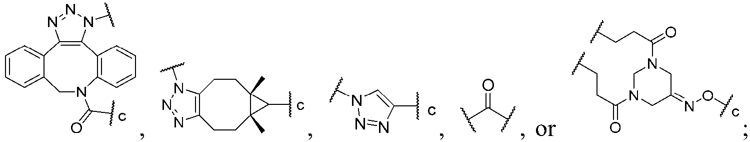 the linker head is connected to the rest of L through the c side.

In some embodiments, L is 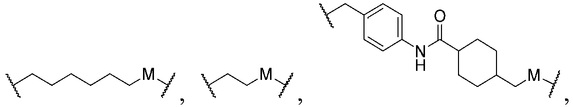 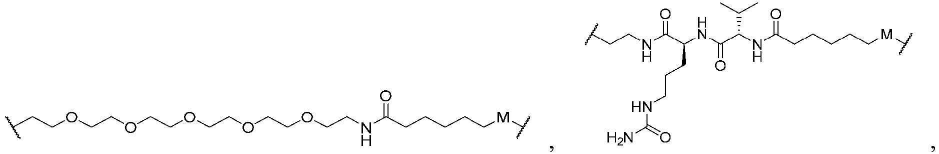 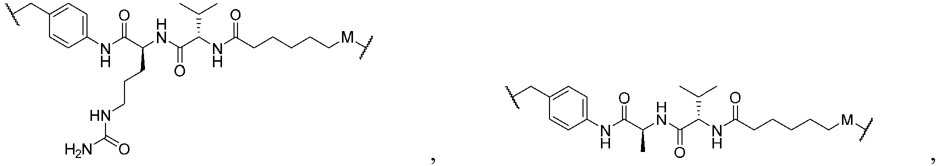 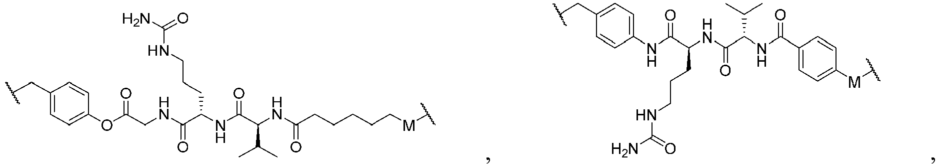 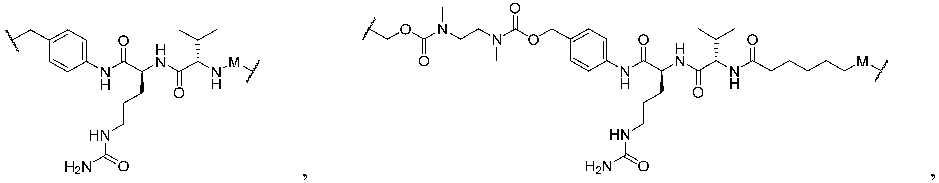 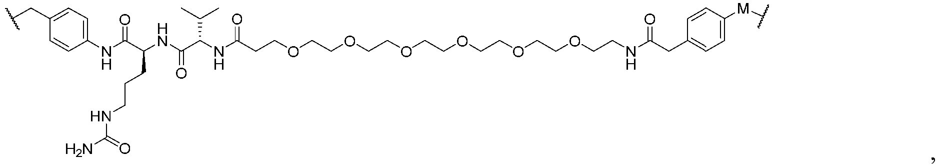 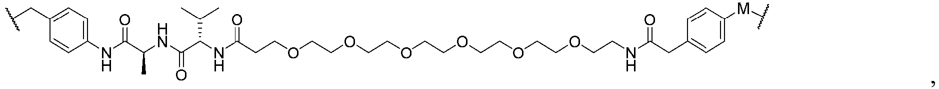 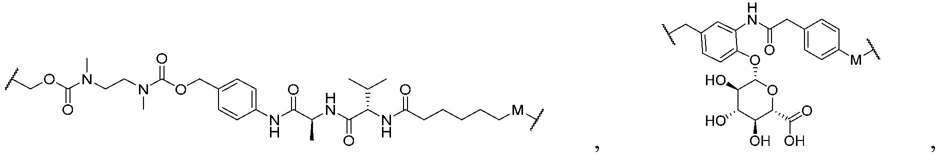 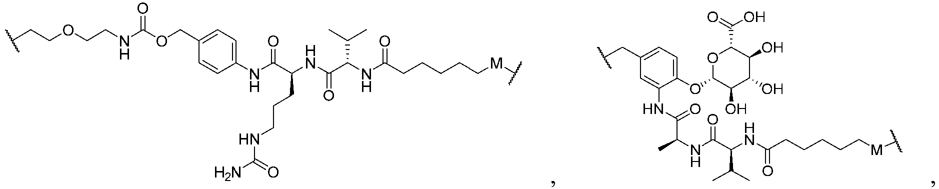 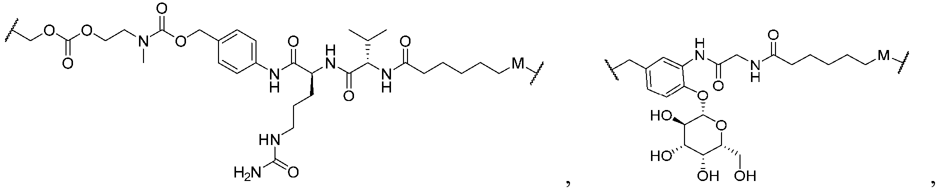 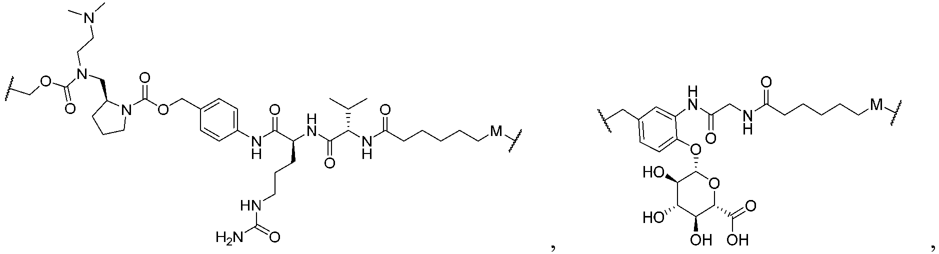 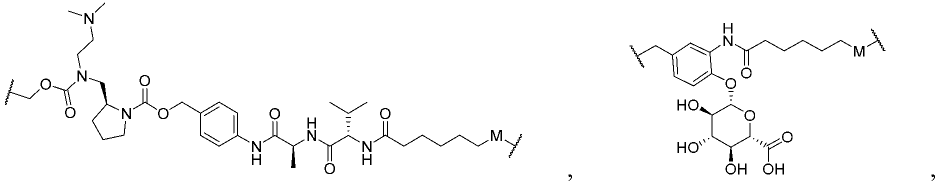 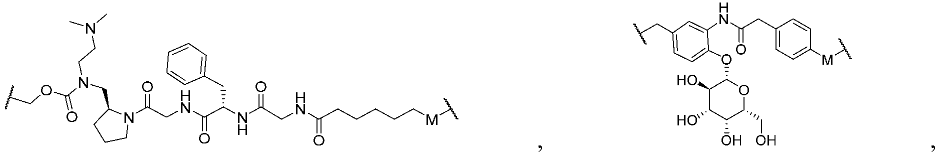 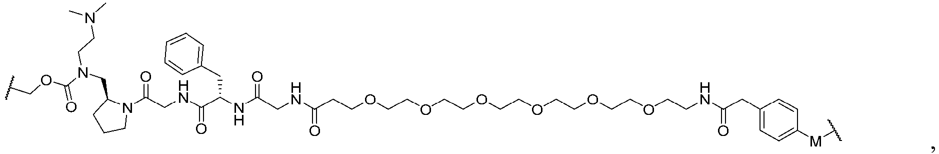 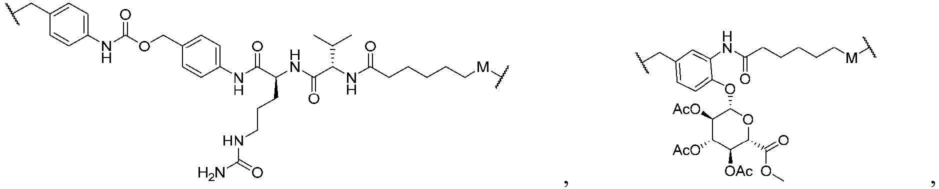 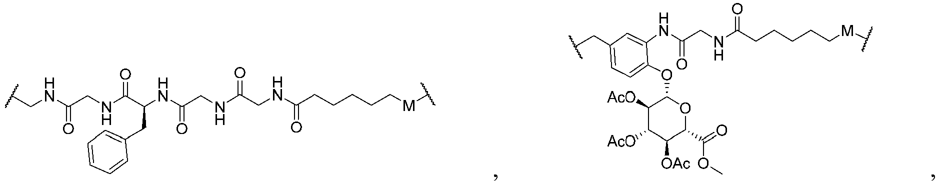 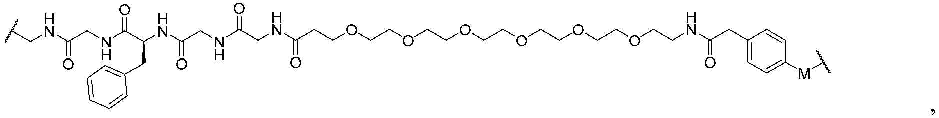 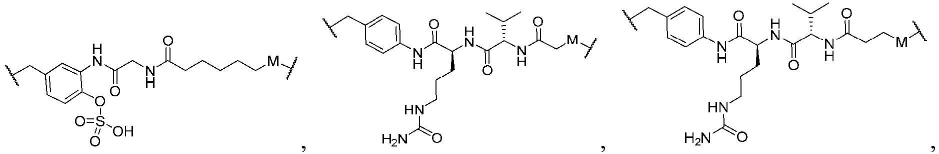 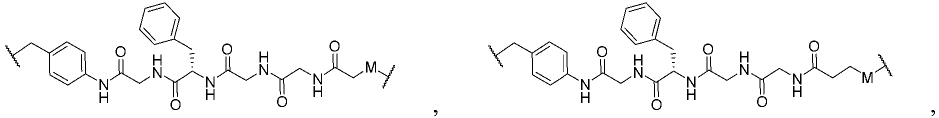 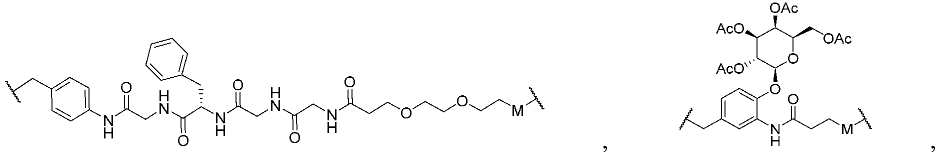 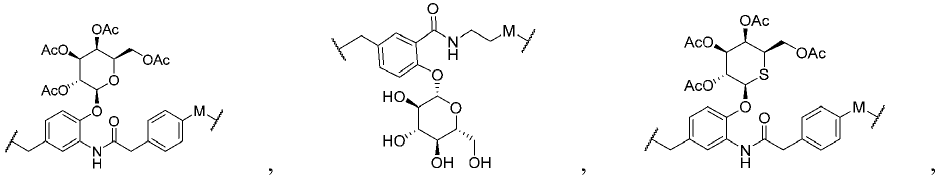 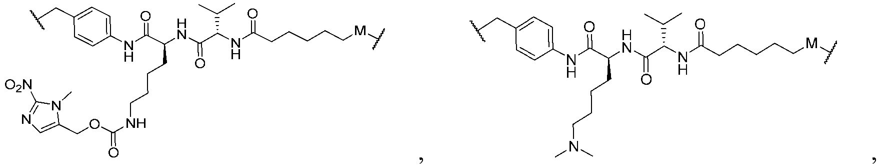 or

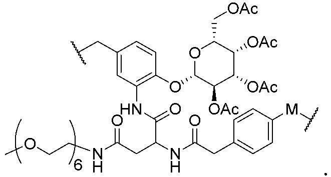

In some embodiments, L is 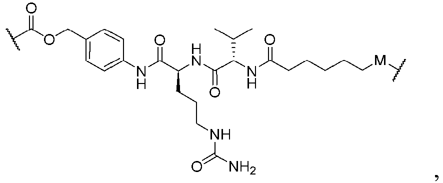 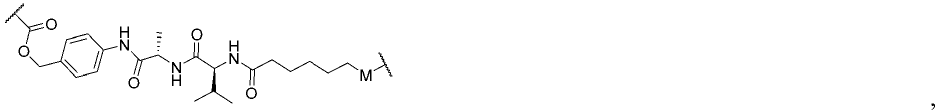 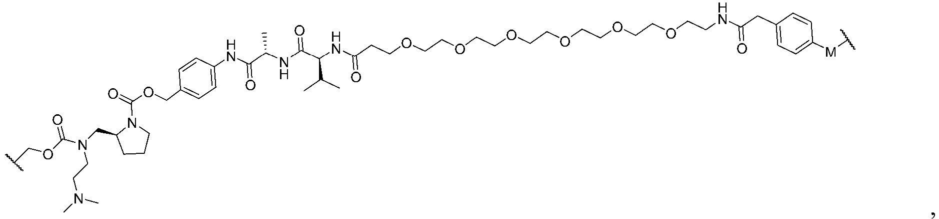 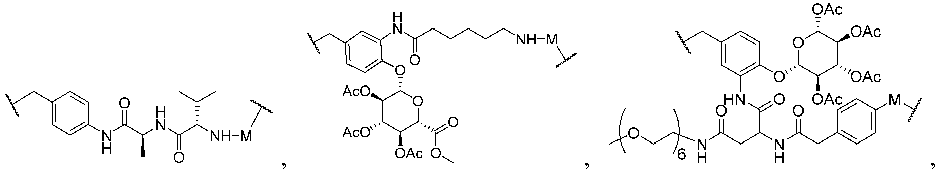 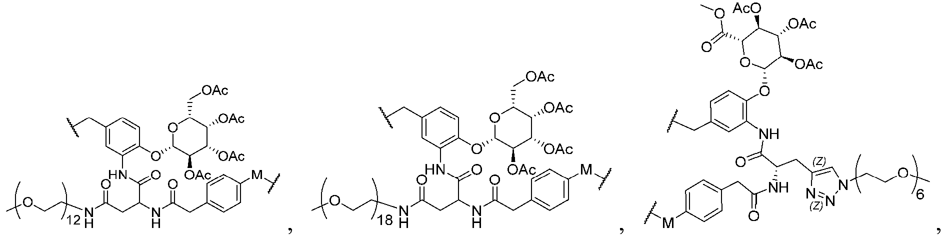 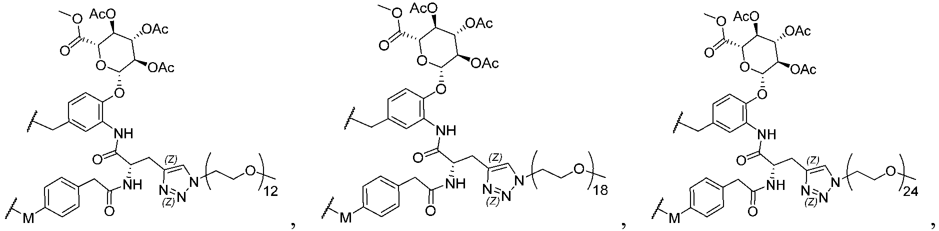 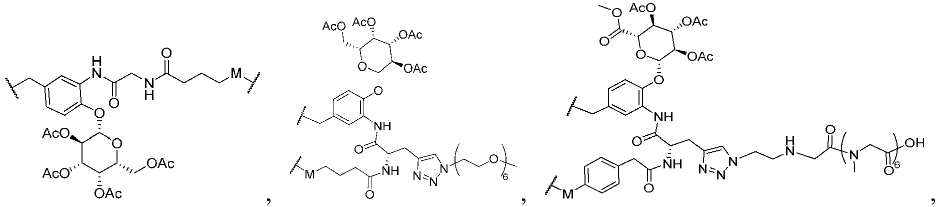 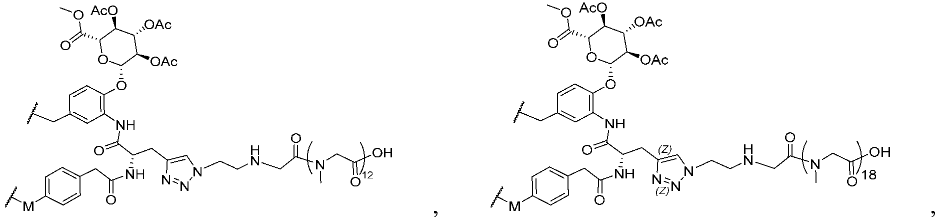 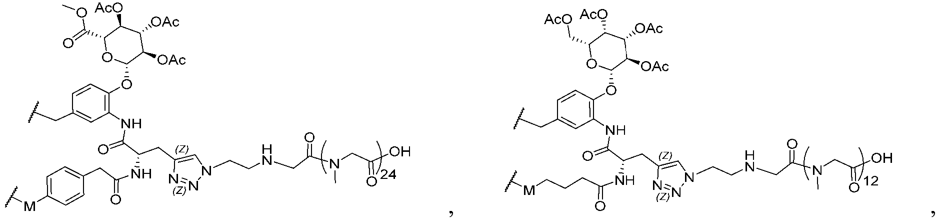 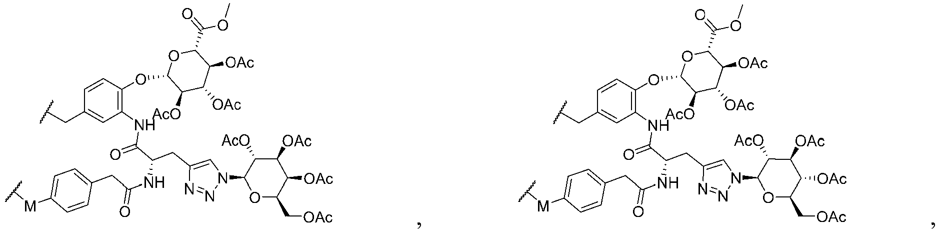 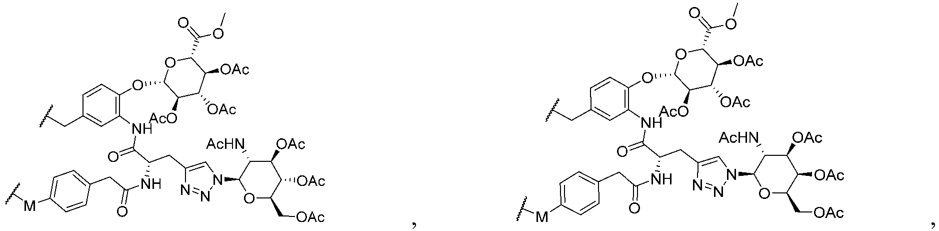 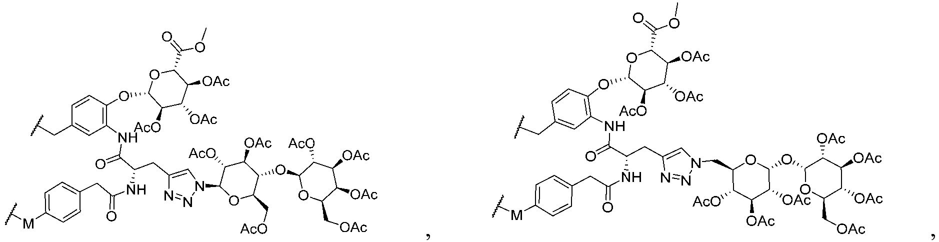 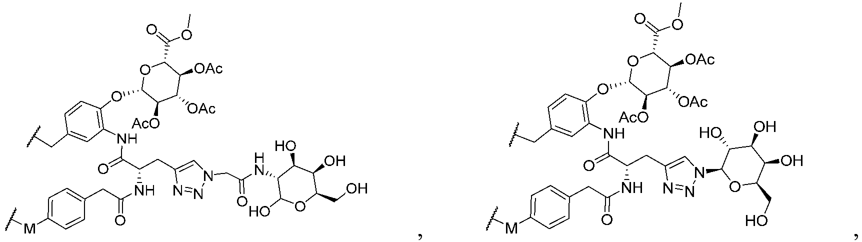 or

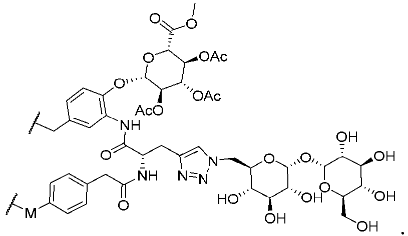

In some embodiments, D is a group formed by the loss of one hydrogen atom from a compound represented by 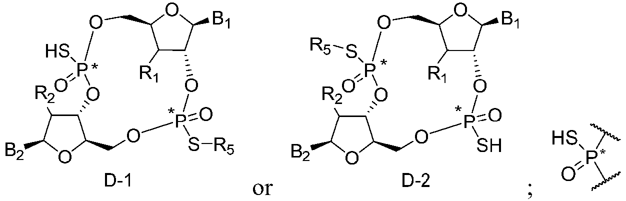 is 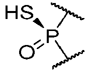 and/or 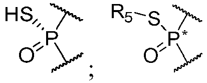 is 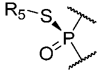 and/or

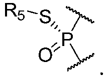

In some embodiments, d is 1.

In some embodiments, R₅ is H, -L₂-(Z)ₑ-(maleimido), or -L₂-(Z)ₑ-H.

In some embodiments, R₅ is -L₂-(Z)ₑ-H; -L₂- is 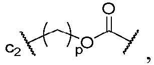 with the c₂ side connected to the thiol group in D; e is 1; -(Z)ₑ-H is C₁₋₆ alkyl.

In some embodiments, R₅ is -L₂-(Z)ₑ-H; -L₂- is 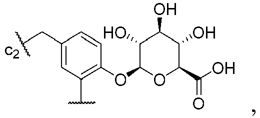 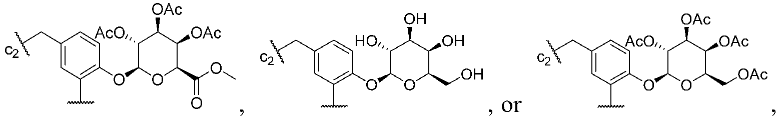 with the c₂ side connected to the thiol group in D; e is 3, -(Z)ₑ-H is -C(O)NH-(CH₂)_{y}-O-(CH₂)_{y}-H.

In some embodiments, R₅ is -L₂-(Z)ₑ-H; -L₂- is 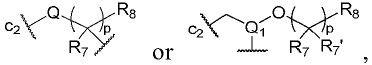 with the c₂ side connected to the thiol group in D; e is 0 or 1, -(Z)ₑ-H is H or methyl.

In some embodiments, R₅ is 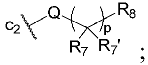 Q is a linking bond, -C(O)O-, or -C₁₋₃ alkylene-O-; R₇ and R_{7'} are each independently H or methyl.

In some embodiments, R₅ is 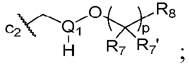 Q₁ is phenyl; R₇ and R_{7'} are each independently H or methyl.

In some embodiments, R₈ is phenyl or 5- to 6-membered heteroaryl; the 5- to 6-membered heteroaryl is preferably pyridyl, pyrimidinyl, pyrazinyl, pyrrolyl, thienyl, furyl, pyrazolyl, thiazolyl, or oxazolyl; R₈ is unsubstituted or optionally substituted at any position with 1 to 3 substituents selected from hydroxyl, amino, cyano, carboxyl, nitro, thiol, C₁₋₆ alkyl, halogen, halo-C₁₋₆ alkyl, C₁₋₆ alkylamino, C₁₋₆ alkoxy, halo-C₁₋₆ alkoxy, C₁₋₆ alkylthio, amido, aldehyde, acetyl, methoxycarbonyl, amino-C₁₋₆ alkyl, hydroxy-C₁₋₆ alkyl, and methylsulfonyl.

In some embodiments, R₅ is -L₂-(Z)ₑ-(maleimido); -L₂- is 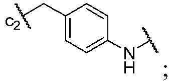 the c₂ side is connected to the thiol group in D; -(Z)ₑ- is -(A)ᵥ-C(O)-(CH₂)ₓ-(c₃); the c₃ side is connected to the maleimido.

In some embodiments, R₅ is 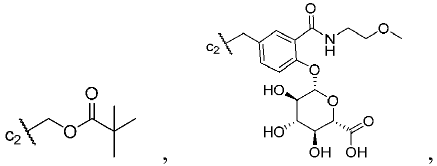 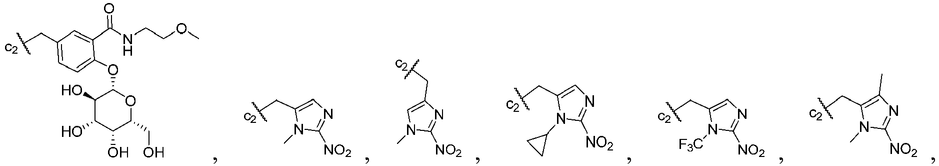 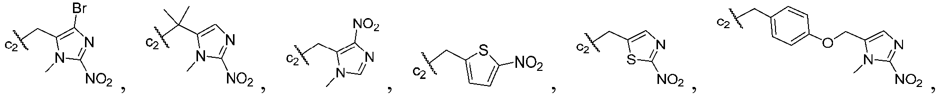 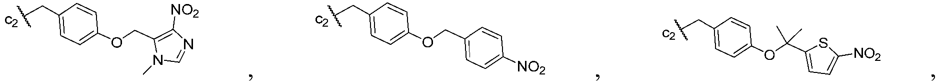 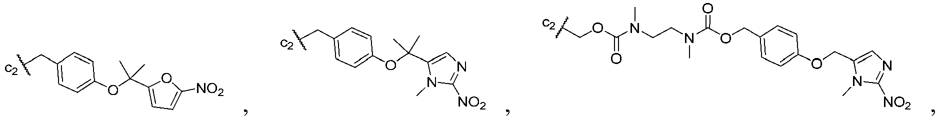 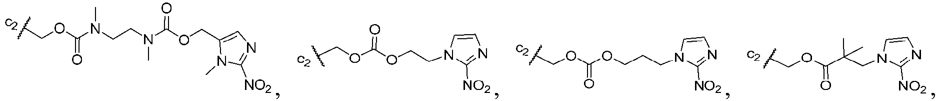 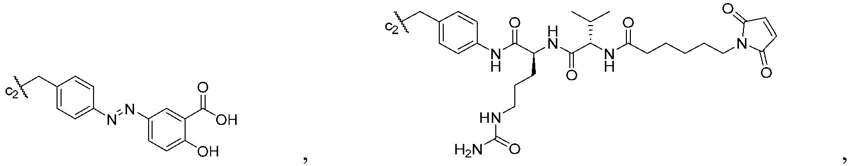 or 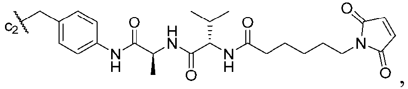 with the c₂ side connected to the thiol group in D.

In some embodiments, R₅ is 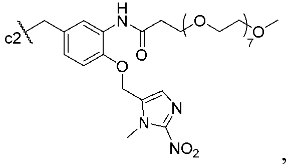 with the c₂ side connected to the thiol group in D.

In some embodiments, B₁ and B₂ are each independently 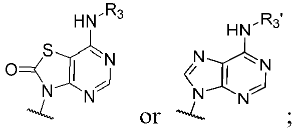 and at least one of B₁ or B₂ is

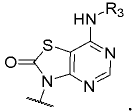

In some embodiments, D is a group formed by the loss of one hydrogen atom from the amino or thiol group in a compound represented by 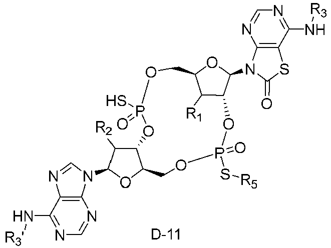 or

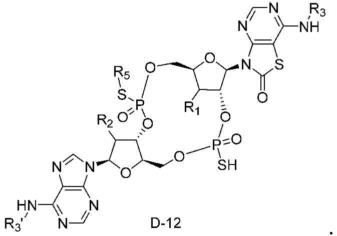

In some embodiments, D is a group formed by the loss of one hydrogen atom from the amino group in a compound represented by 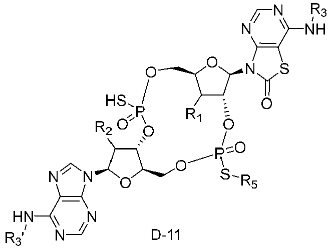 or 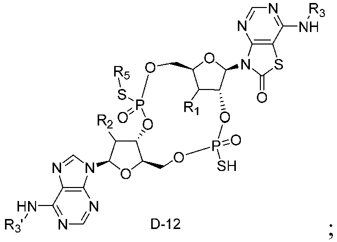 R₃ or R_{3'} is each independently

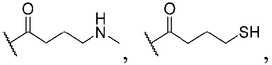

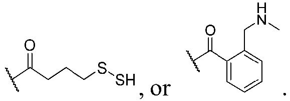

In some embodiments, D is a group formed by the loss of one hydrogen atom from the thiol group in a compound represented by 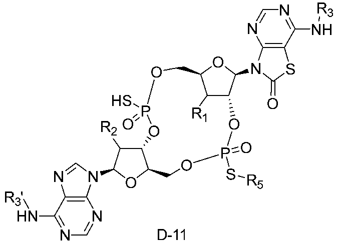 or

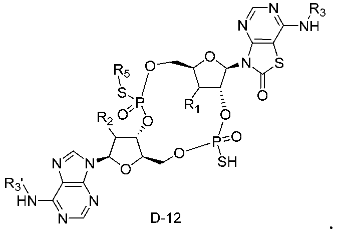

In some embodiments, D is a group formed by the loss of one hydrogen atom from the thiol group in a compound represented by 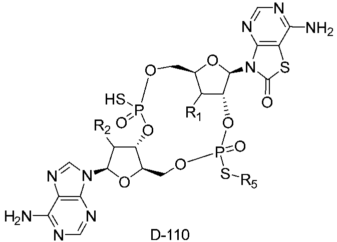 or

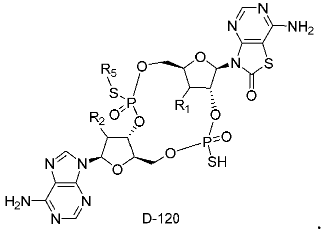

In some embodiments, R₁ is -OCH₃; R₂ is F.

In some embodiments, D is any one of the following structures or a pharmaceutically acceptable salt thereof: 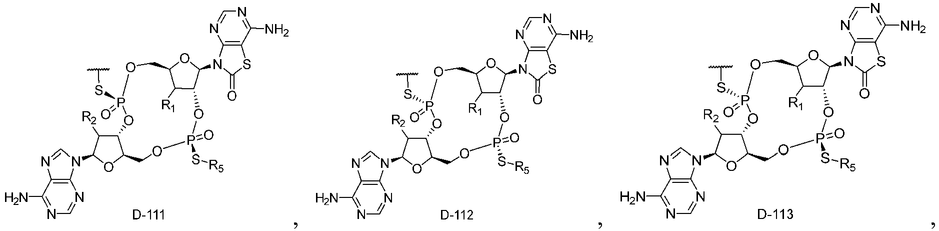 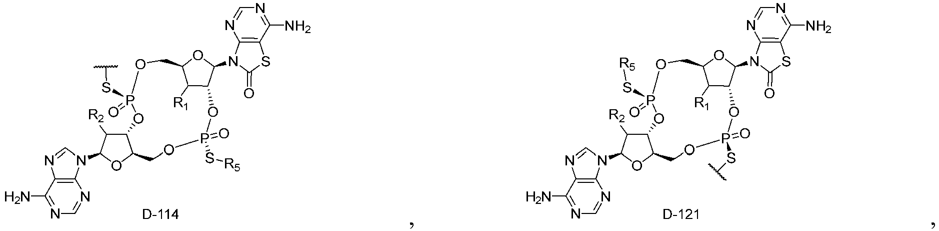 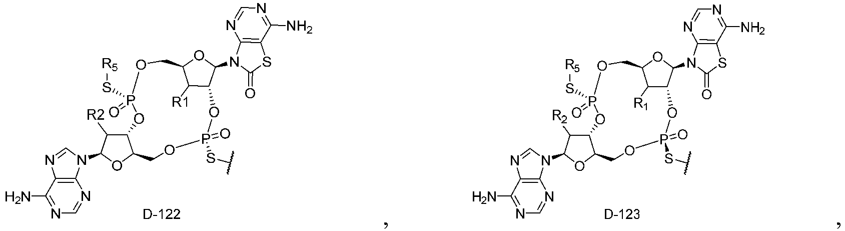 or 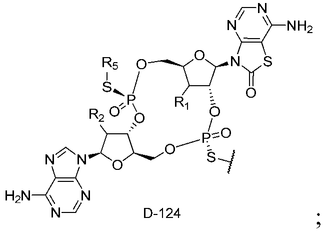 wherein R₁, R₂, and R₅ are as defined above.

In some embodiments, D is any one of the following structures: 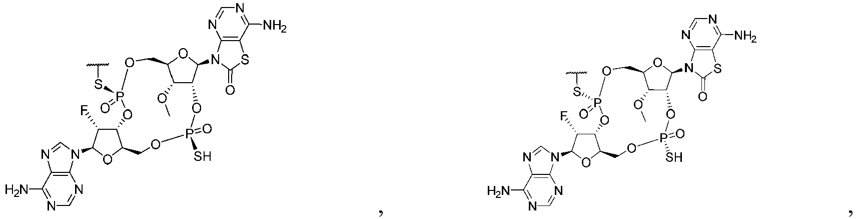 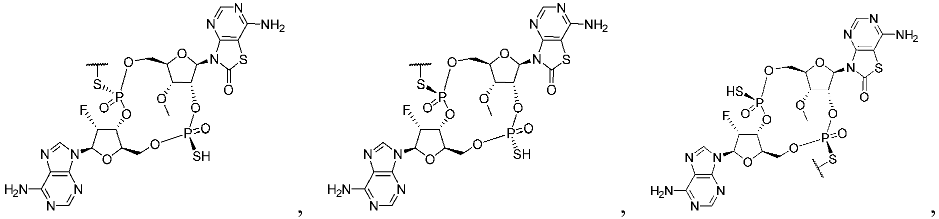 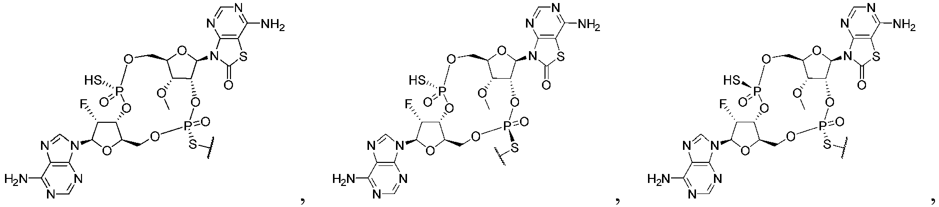 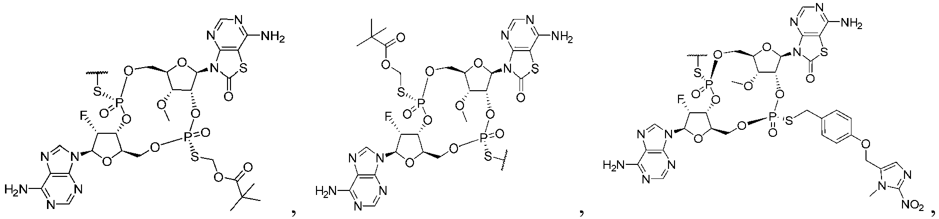 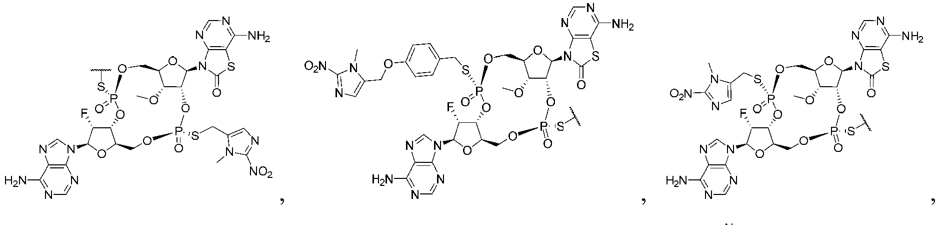 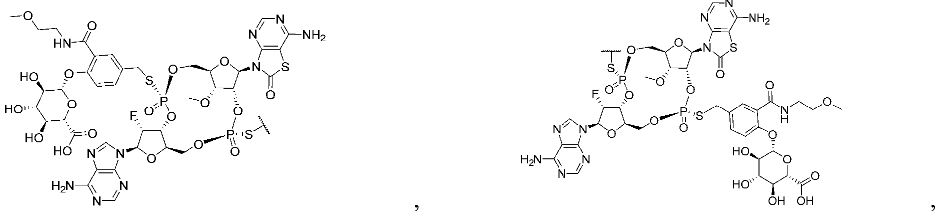 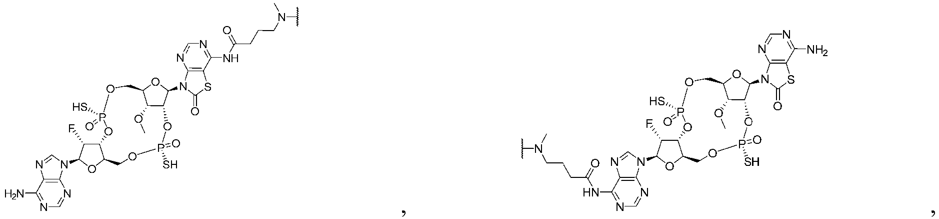 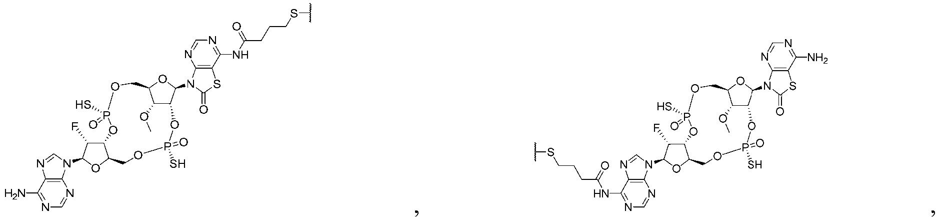 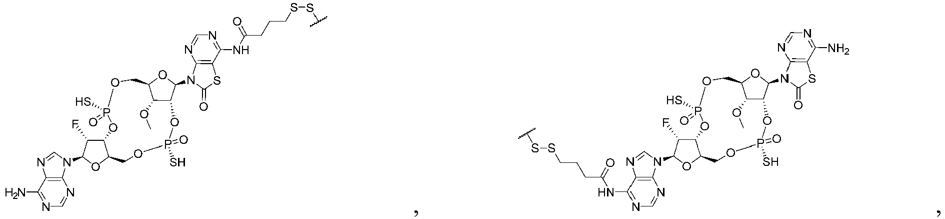 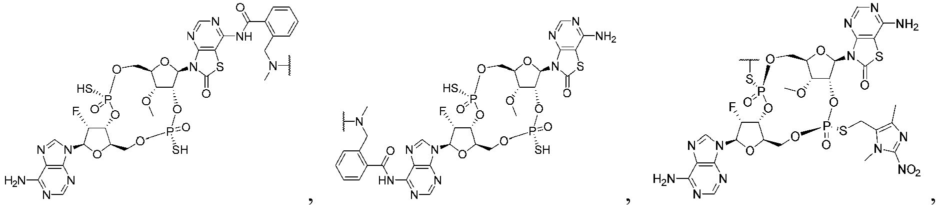 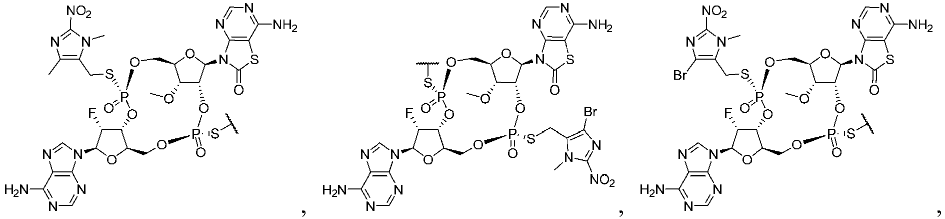 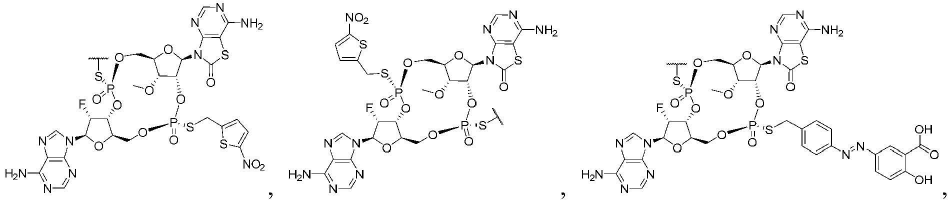 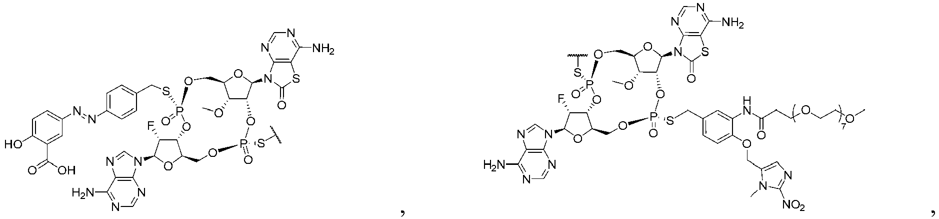 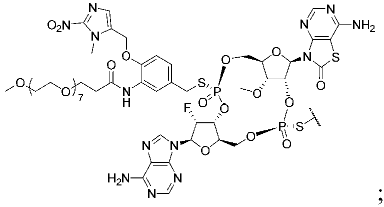 or a pharmaceutically acceptable salt thereof.

In some embodiments, D is any one of the following structures: 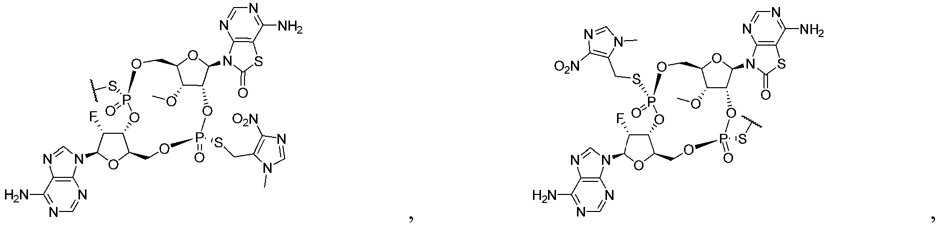 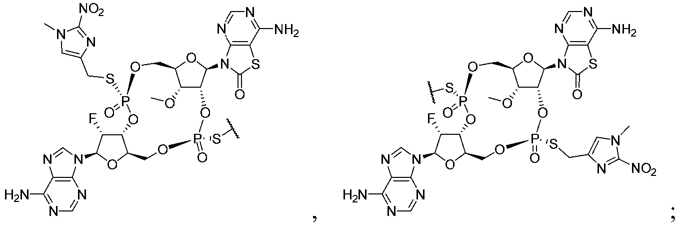 or a pharmaceutically acceptable salt thereof.

In some embodiments, Ab is an anti-HER2 antibody, an anti-EGFR antibody, or an anti-5T4 antibody;
L is a linker with the following combination: -(L₁)ₐ-(Z)_{b}-M-; L₁ is connected to D, and M is connected to Ab;
-(L₁)ₐ- is 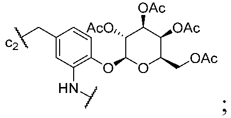
-(Z)_{b}- is -(CH₂)ₓ-(c₁) or -C(O)-(CH₂)ₓ-phenylene-(CH₂)_{y}-;
x and y are independently 0, 1, 2, 3, 4, 5, or 6;
M is a linker head connected to Ab;
D is a group formed by the loss of one hydrogen atom from the thiol group in a compound represented by 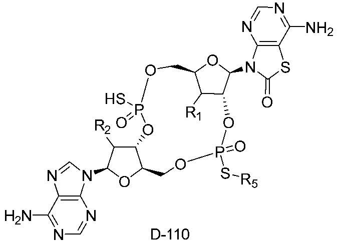 or 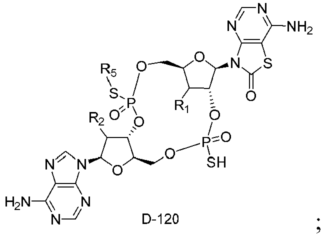
R₅ is H.

In some embodiments, in the immune-stimulating antibody conjugate represented by Formula II or the pharmaceutically acceptable salt thereof, the immune-stimulating antibody conjugate is any one immune-stimulating antibody conjugate listed in Table A:

**Table A**

| No. | Structure | | Ab | t |
|---|---|---|---|---|
| II-1 | 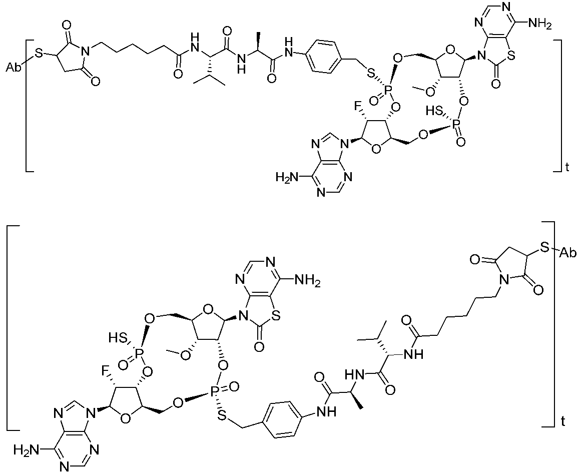 | or | Antibody 1 | 7.91 |
| II-3 | | | Antibody 2 | 7.94 |
| II-2 | 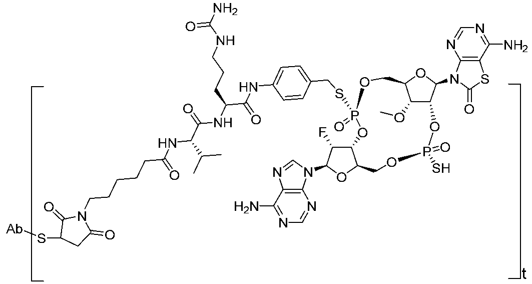 | or | Antibody 1 | 7.93 |
| II-4 | | | Antibody 2 | 7.96 |
| II-5 | | | Antibody 1 | 3.10 |
| II-6 | | | Antibody 3 | 6.62 |
| II-42 | | | Antibody 1 | 3.71 |
| II-43 | 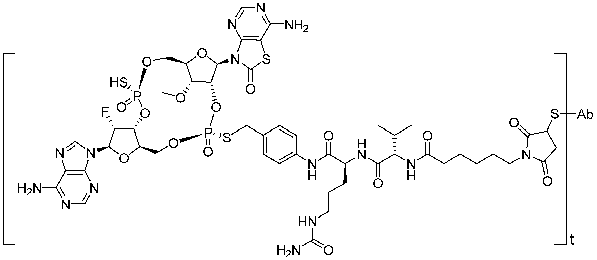 | | Antibody 1 | 7.31 |
| II-44 | | | Antibody 4 | 7.96 |
| II-7 | 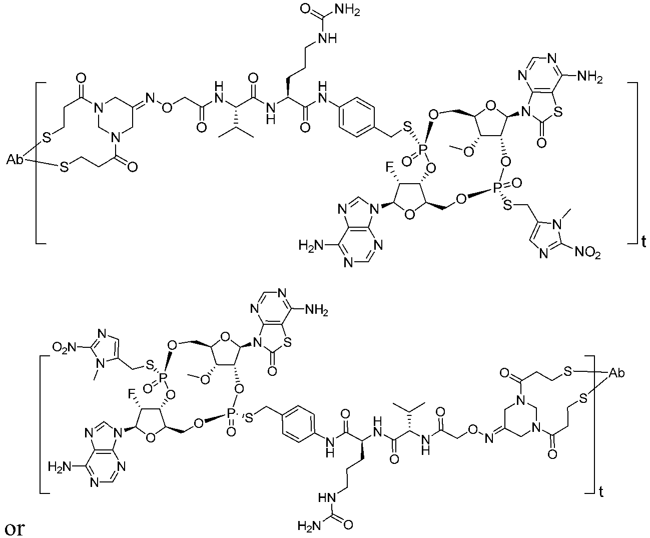 | | Antibody 1 | 3.87 |
| II-9 | 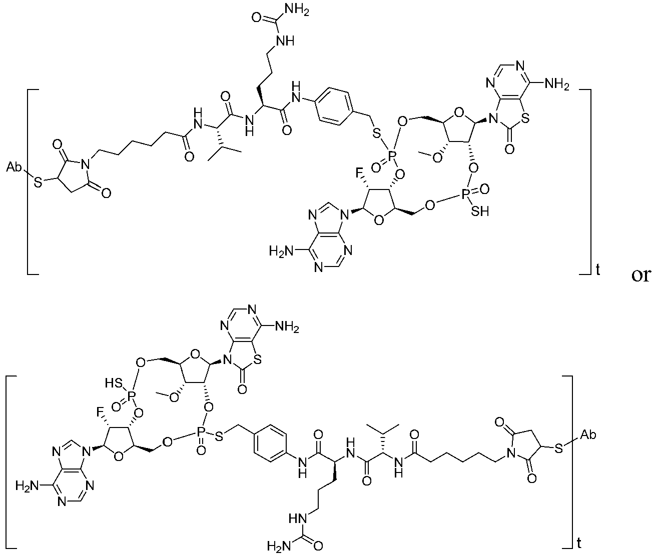 | | Antibody 1 | 7.81 |
| II-10 | 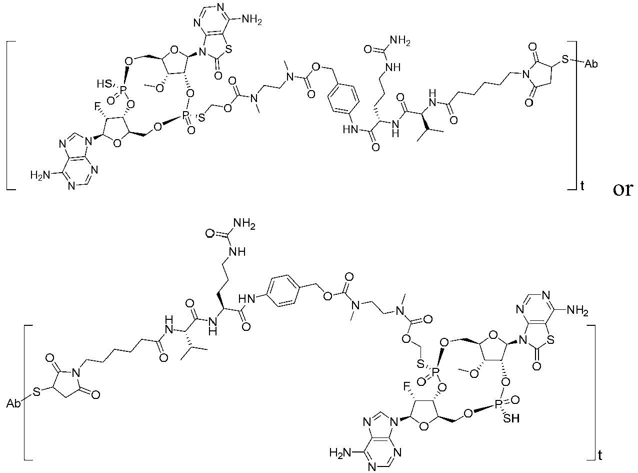 | | Antibody 1 | 3.58 |
| II-11 | 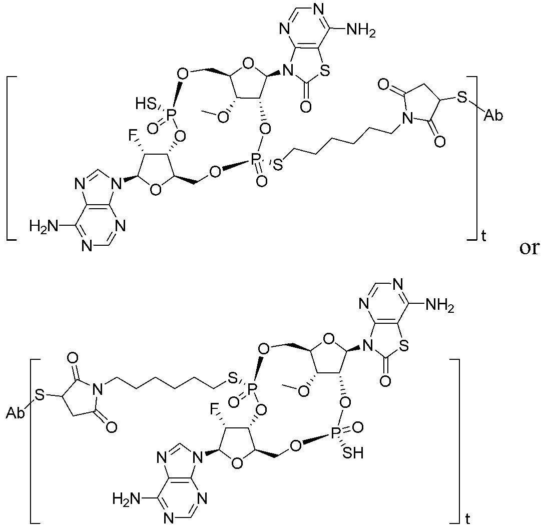 | | Antibody 1 | 4.22 |
| II-12 | | | Antibody 3 | 3.80 |
| II-13 | 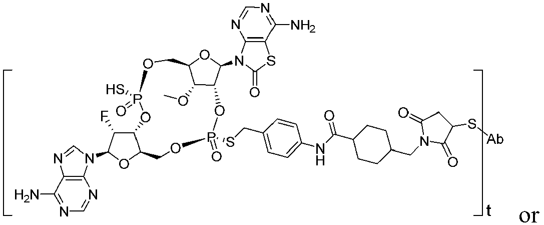 | | Antibody 1 | 4.20 |
| II-14 | | | Antibody 3 | 3.64 |
| | 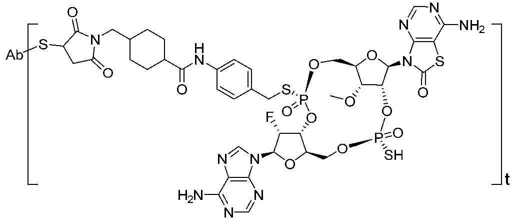 | | | |
| II-15 | 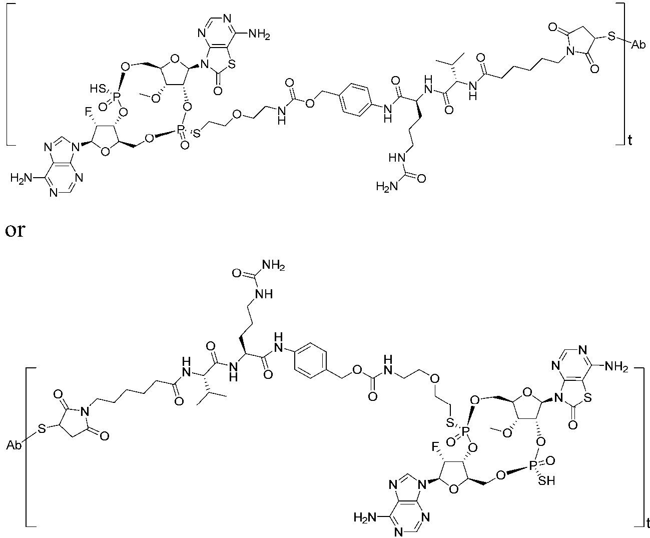 | | Antibody 1 | 3.93 |
| II-16 | 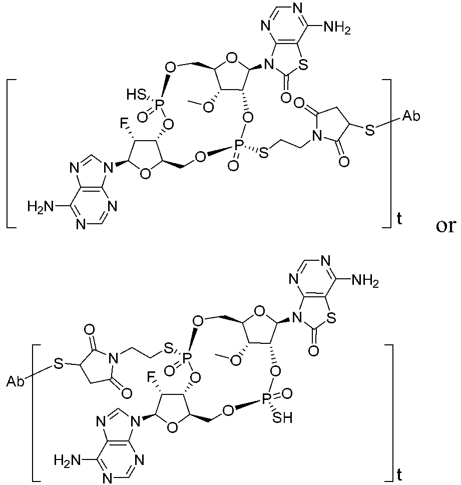 | | Antibody 1 | 3.72 |
| II-17 | 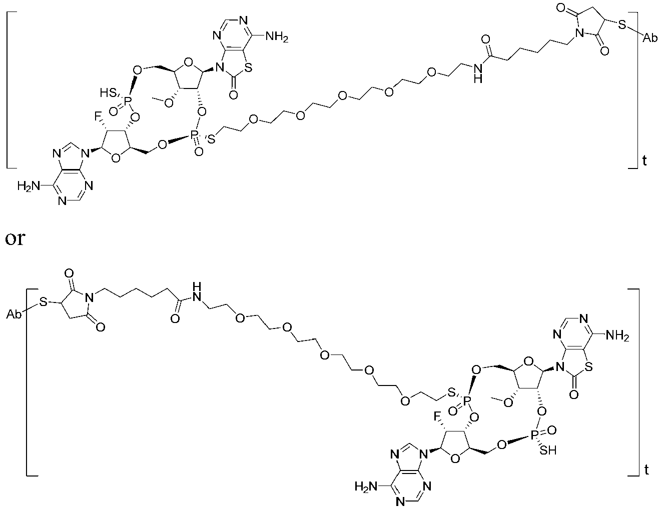 | | Antibody 1 | 3.74 |
| II-18 | 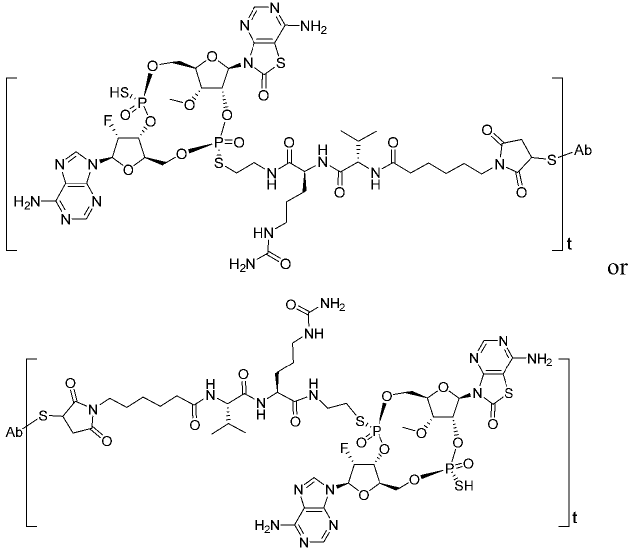 | | Antibody 1 | 3.97 |
| II-19 | 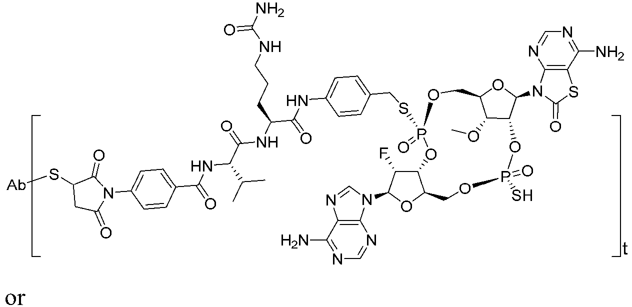 | | Antibody 1 | 3.17 |
| | 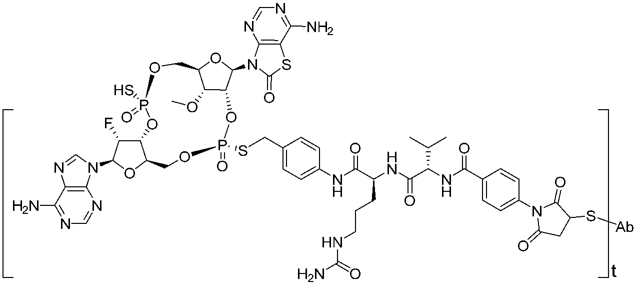 | | | |
| II-20 | 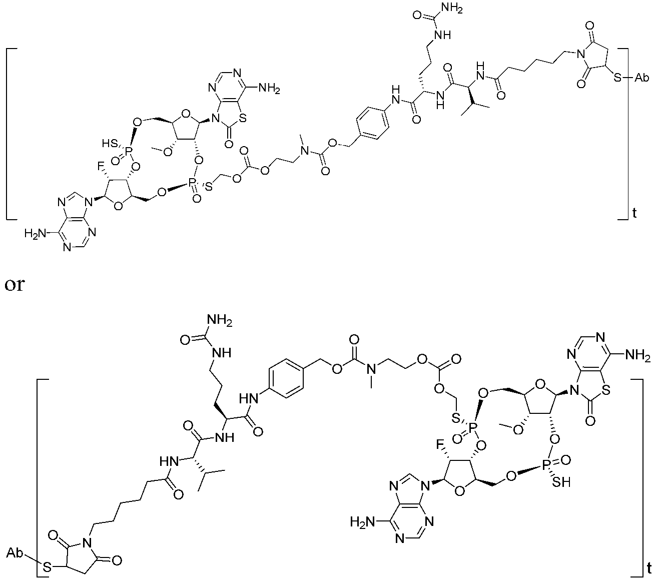 | | Antibody 1 | 3.90 |
| II-21 | 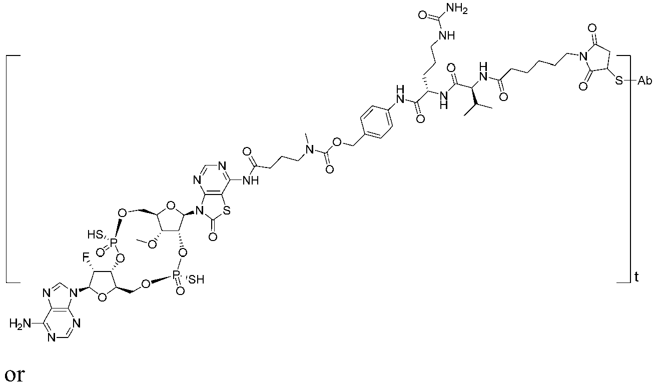 | | Antibody 1 | 3.78 |
| | 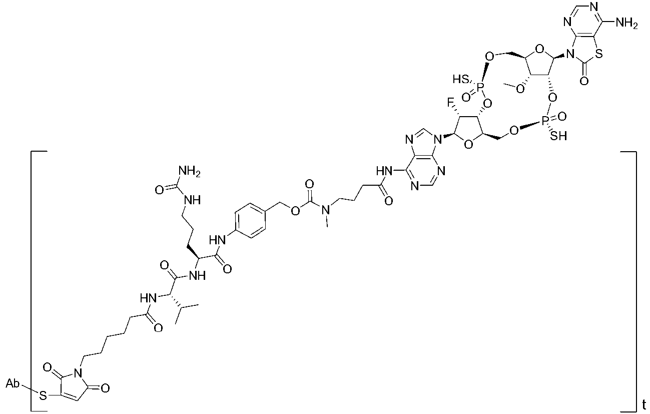 | | | |
| II-22 | 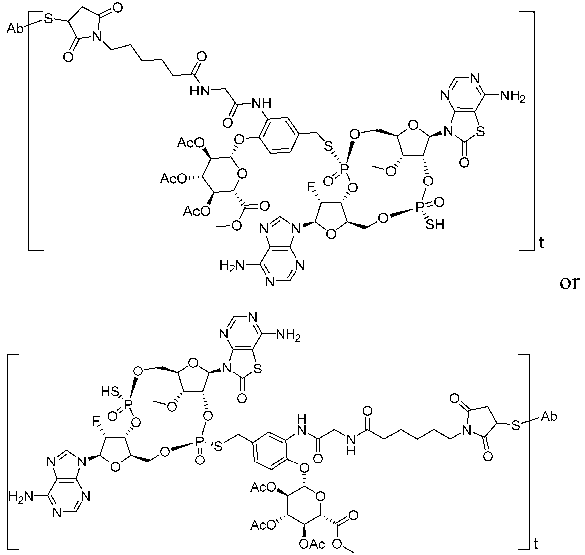 | | Antibody 1 | 3.70 |
| II-23 | 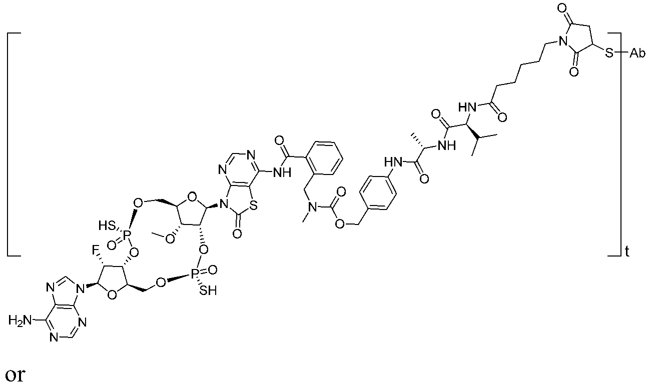 | | Antibody 1 | 3.81 |
| | 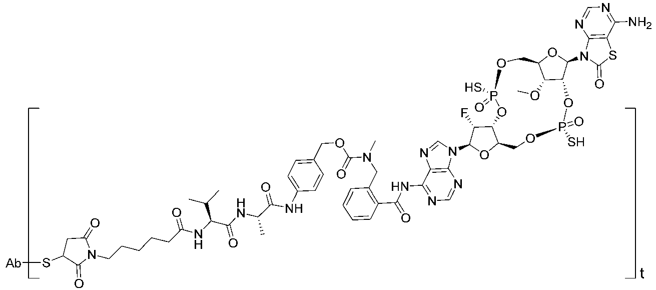 | | | |
| II-24 | 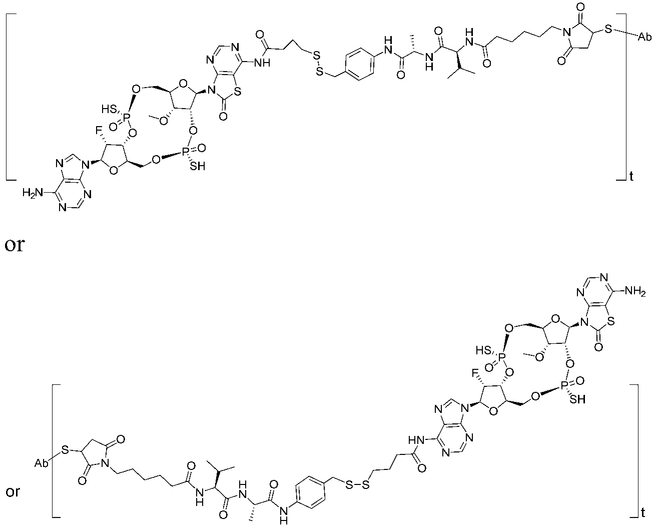 | | Antibody 1 | 3.62 |
| II-25 | 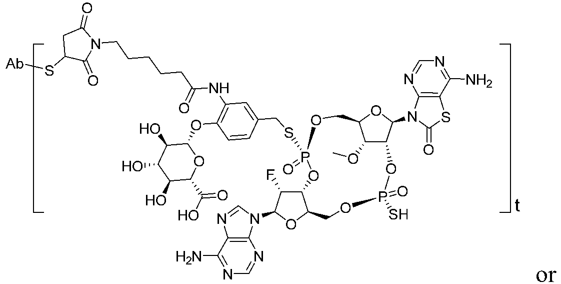 | | Antibody 1 | 3.66 |
| II-26 | | | Antibody 3 | 3.58 |
| | 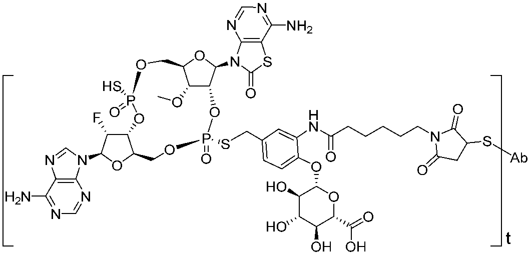 | | | |
| II-27 | 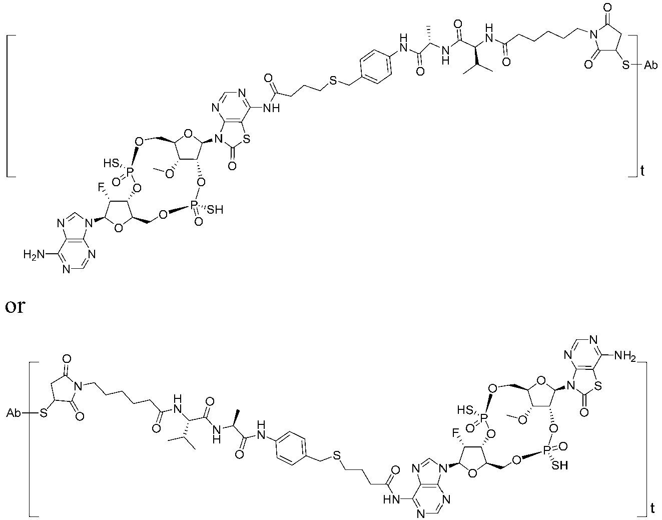 | Antibody 1 | 4.00 | |
| II-28 | 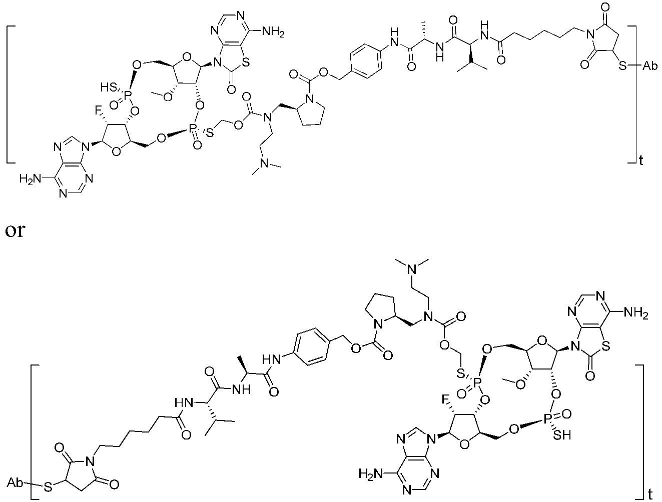 | Antibody 1 | 3.77 | |
| II-29 | 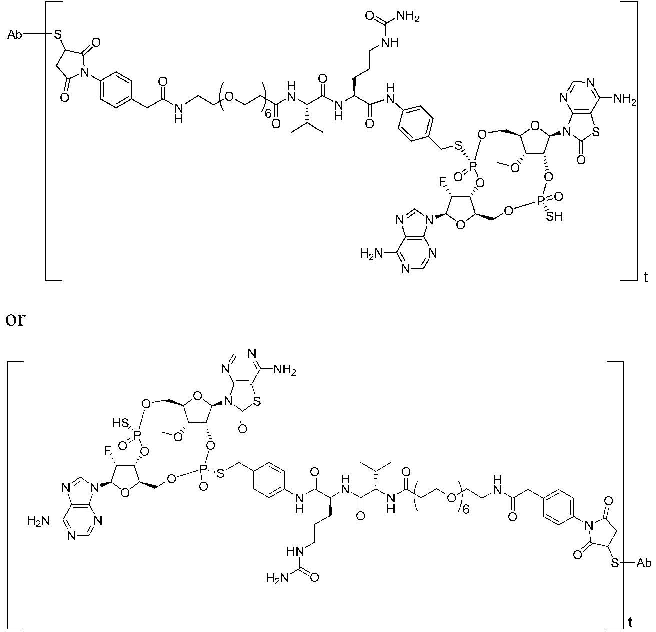 | Antibody 1 | 3.81 | |
| II-30 | 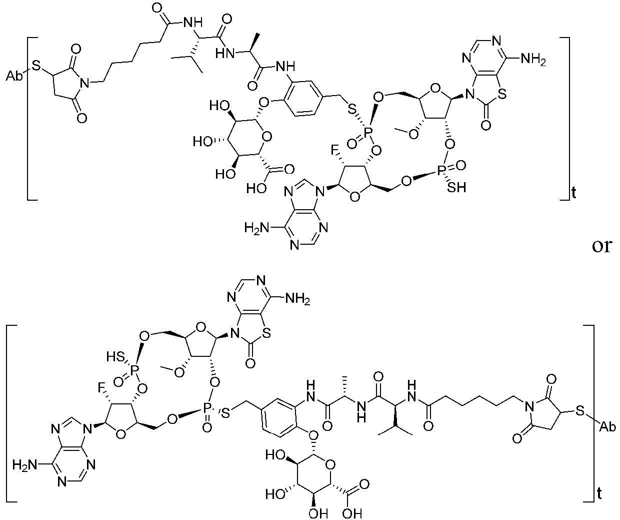 | Antibody 1 | 3.73 | |
| II-31 | 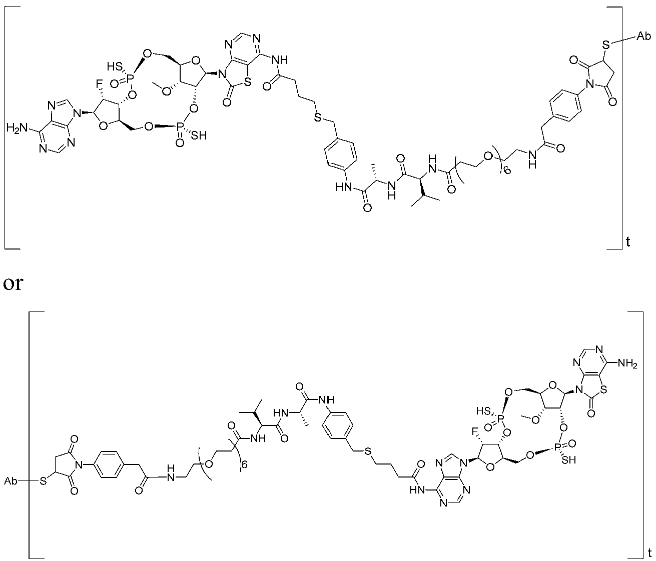 | Antibody 1 | 4.26 | |
| II-32 | 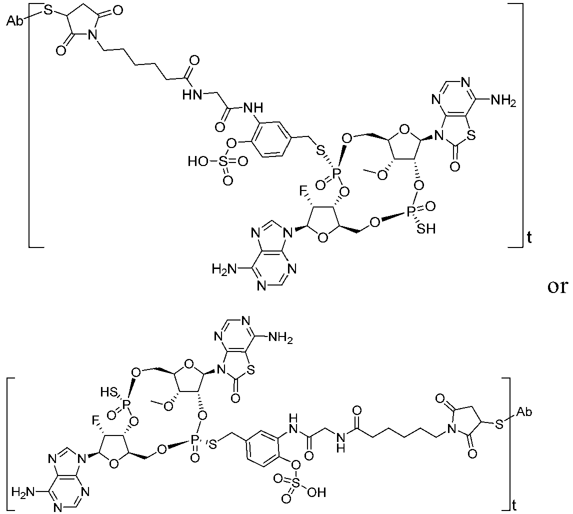 | Antibody 1 | 4.18 | |
| II-33 | 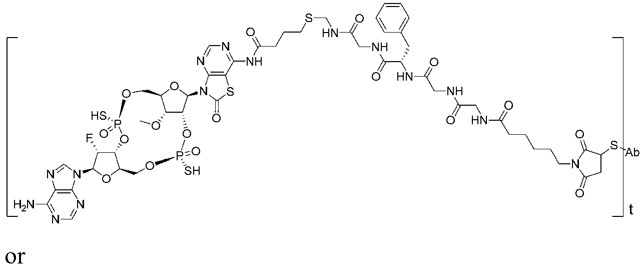 | Antibody 1 | 4.41 | |
| | 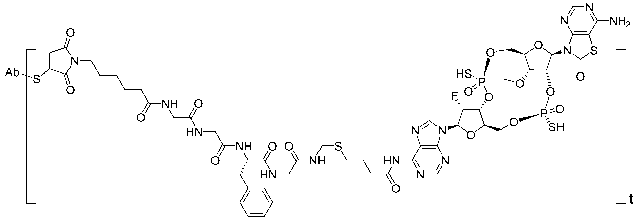 | | | |
| II-34 | 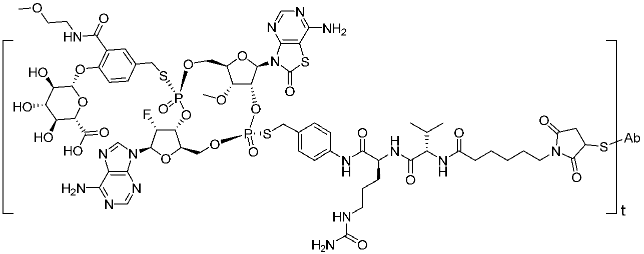 | Antibody 1 | 4.06 | |
| II-35 | 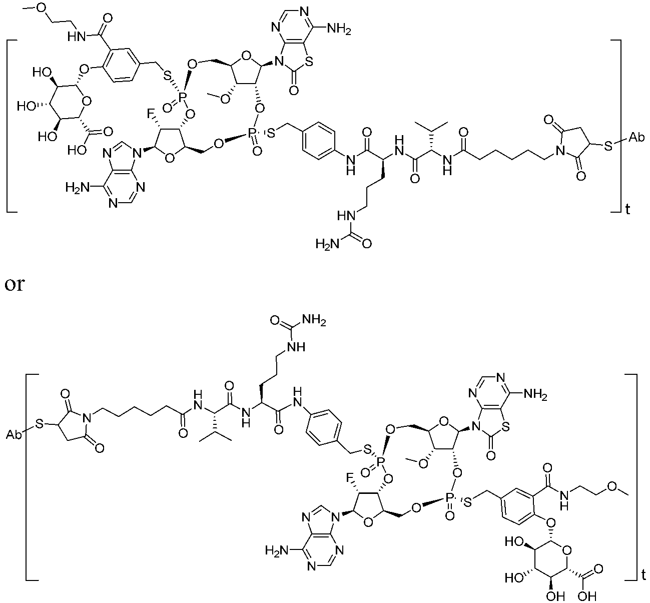 | Antibody 3 | 3.79 | |
| II-36 | 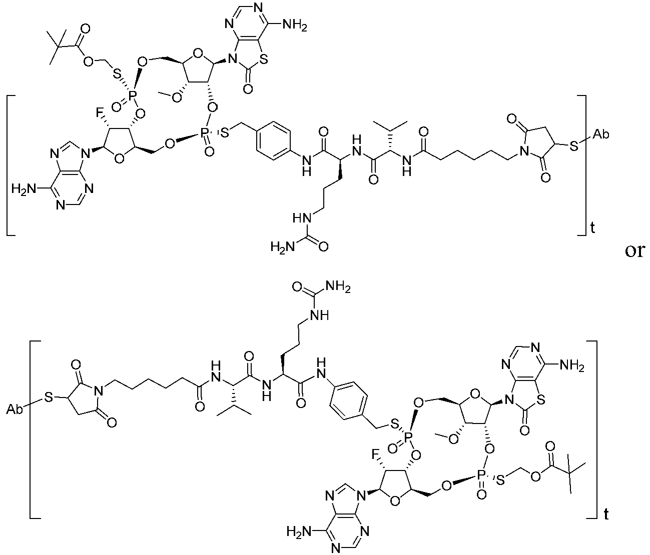 | Antibody 1 | 3.72 | |
| II-37 | 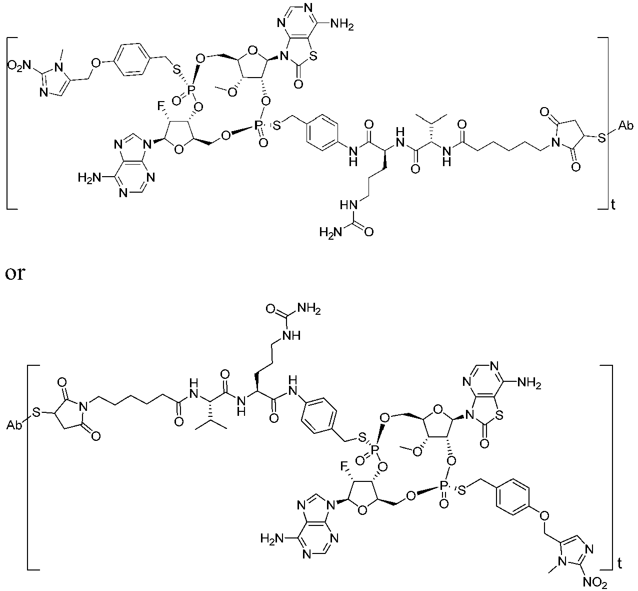 | Antibody 1 | 3.70 | |
| II-39 | 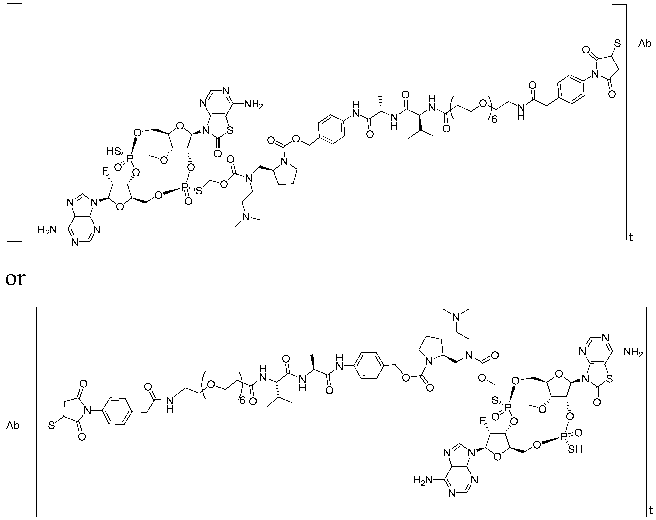 | Antibody 1 | 4.03 | |
| II-40 | | Antibody 1 | 7.71 | |
| II-41 | | Antibody 3 | 6.57 | |
| II-38 | 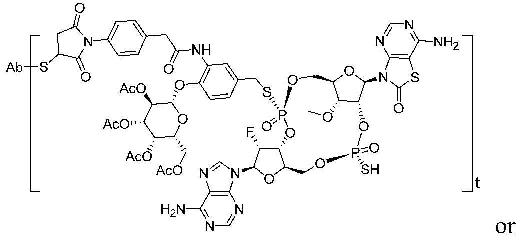 | Antibody 1 | 4.50 | |
| II-45 | | Antibody 1 | 7.62 | |
| II-46 | | Antibody 5 | 2.00 | |
| II- | | Antibody | 4.07 | |
| 47 | 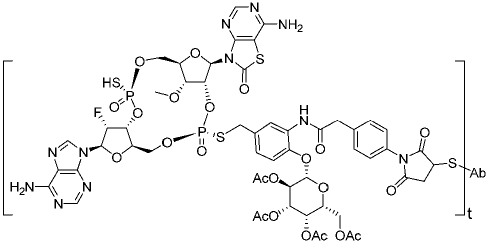 | | 7 | |
| II-48 | | | Antibody 8 | 4.06 |
| II-54 | | | Antibody 1 | 7.82 |
| II-55 | | | Antibody 5 | 2.00 |
| II-56 | | | Antibody 9 | 2.32 |
| II-83 | | | Antibody 11 | 3.67 |
| II-85 | | | Antibody 13 | 4.34 |
| II-86 | | | Antibody 7 | 2.30 |
| II-49 | 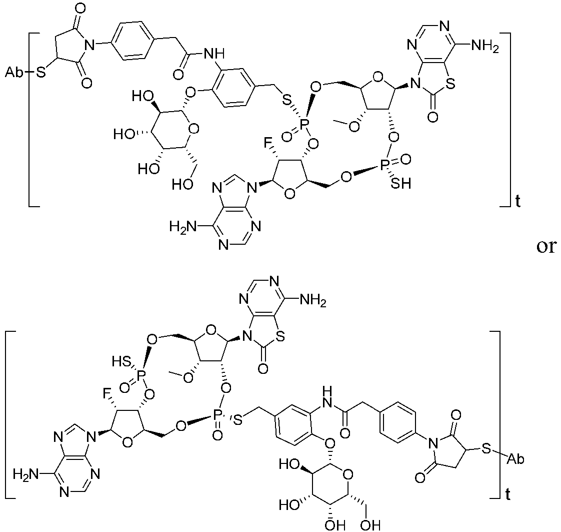 | | Antibody 1 | 3.58 |
| II-50 | 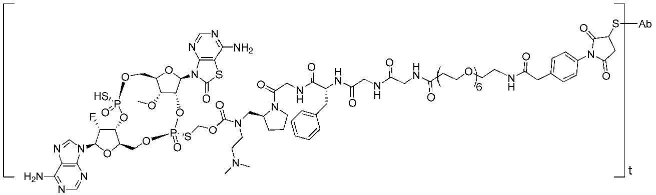 | | Antibody 1 | 3.82 |
| | 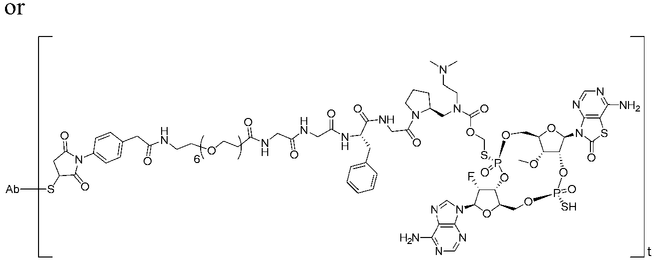 | | | |
| II-51 | 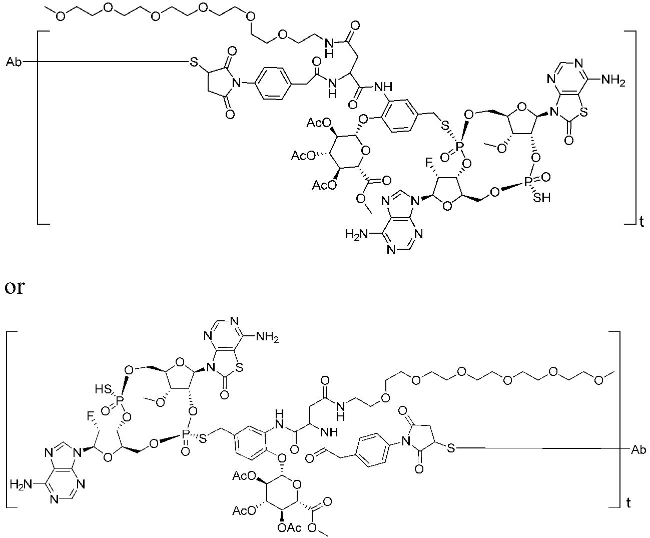 | | Antibody 1 | 7.83 |
| II-52 | 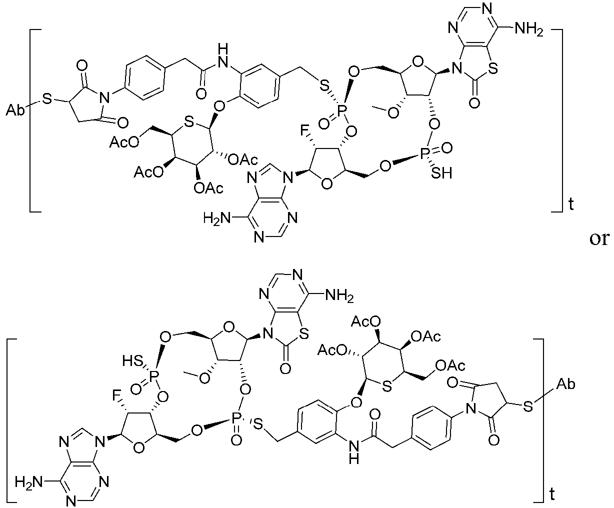 | | Antibody 1 | 4.12 |
| II-53 | 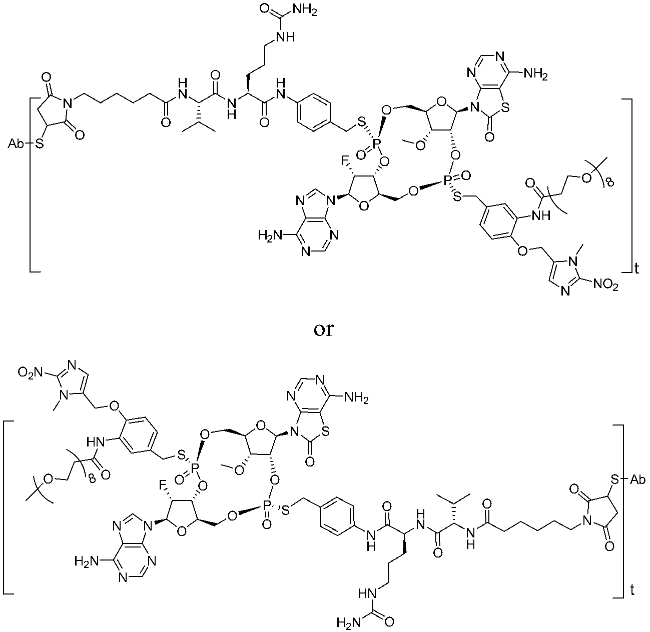 | | Antibody 1 | 7.95 |
| II-57 | 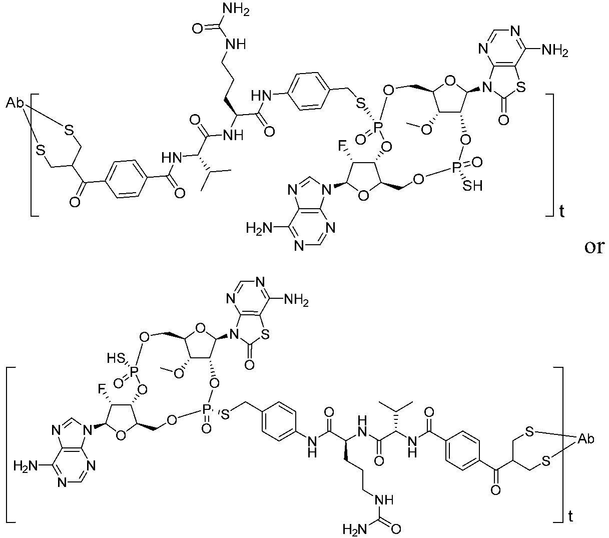 | | Antibody 1 | 3.82 |
| II-58 | 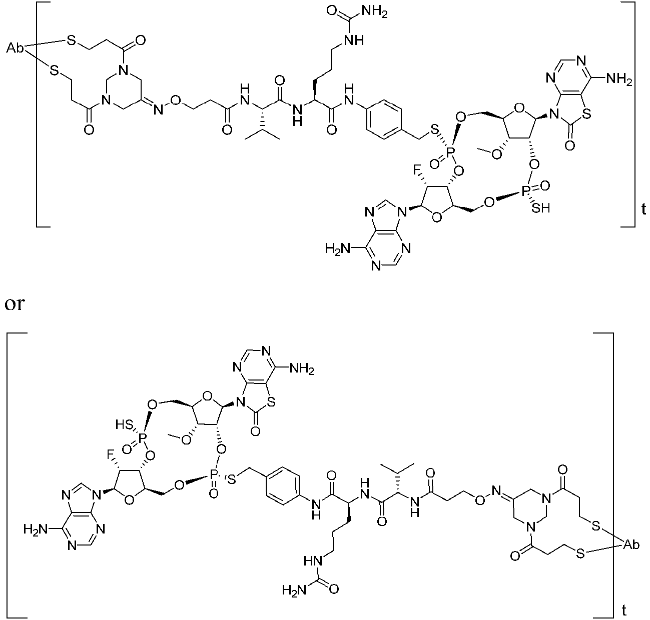 | Antibody 1 | 3.95 | |
| II-59 | 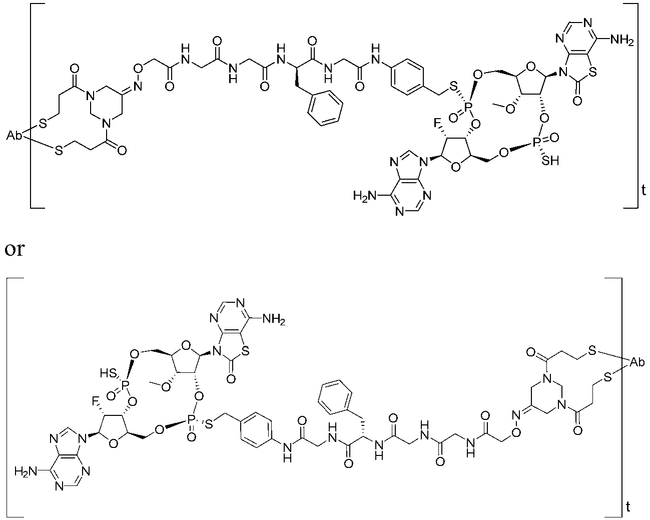 | Antibody 1 | 4.11 | |
| II-60 | 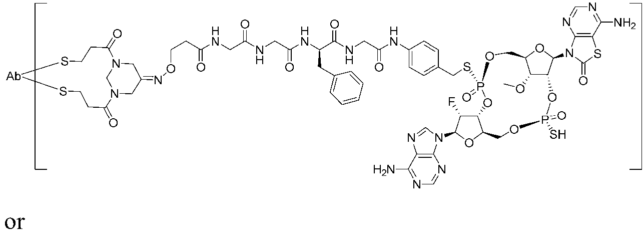 | Antibody 1 | 3.66 | |
| | 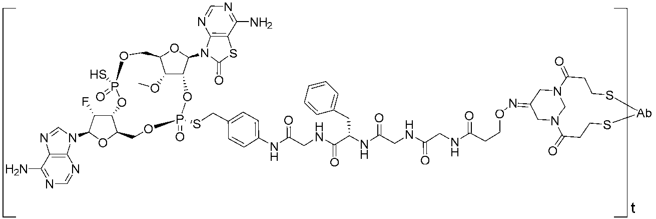 | | | |
| II-61 | 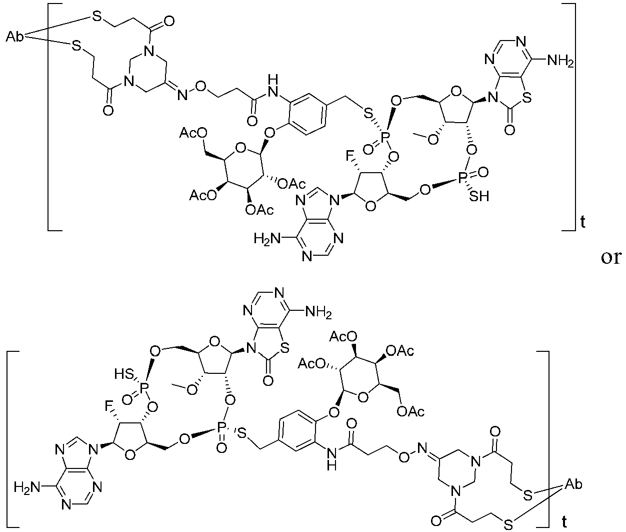 | Antibody 1 | 3.80 | |
| II-63 | | Antibody 7 | 4.23 | |
| II-64 | 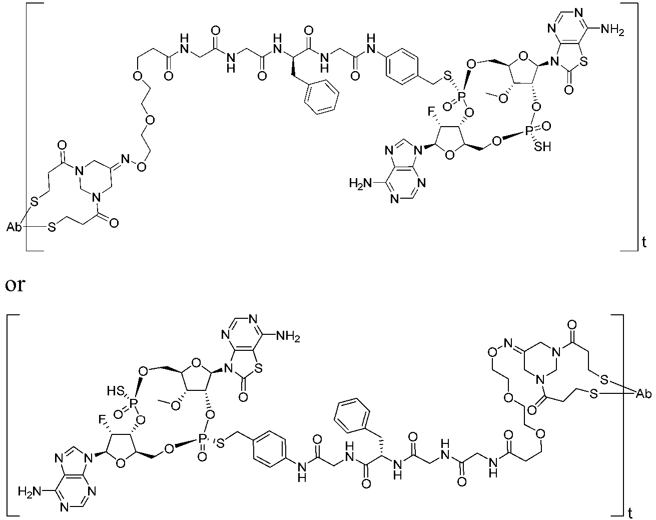 | Antibody 1 | 3.94 | |
| II-65 | 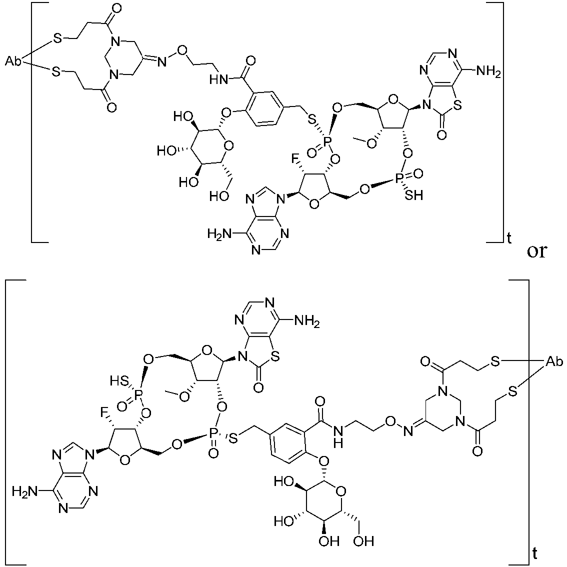 | | Antibody 1 | 3.80 |
| II-66 | 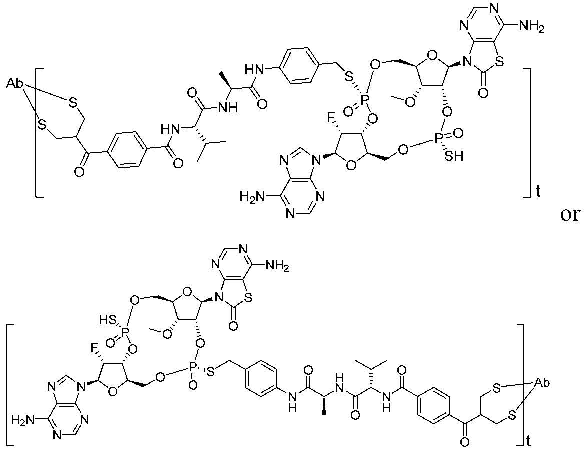 | | Antibody 1 | 3.83 |
| II-67 | 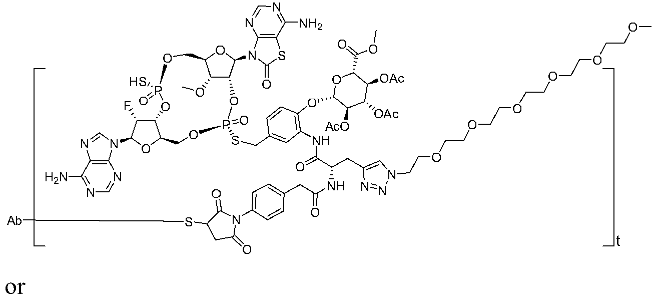 | | Antibody 1 | 3.88 |
| | 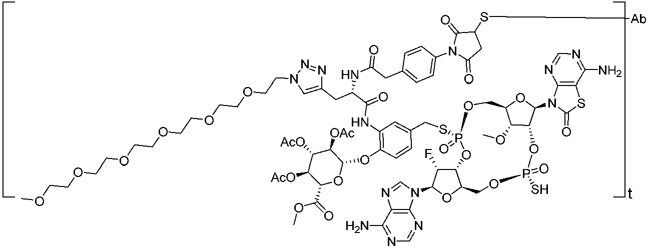 | | | |
| II-68 | 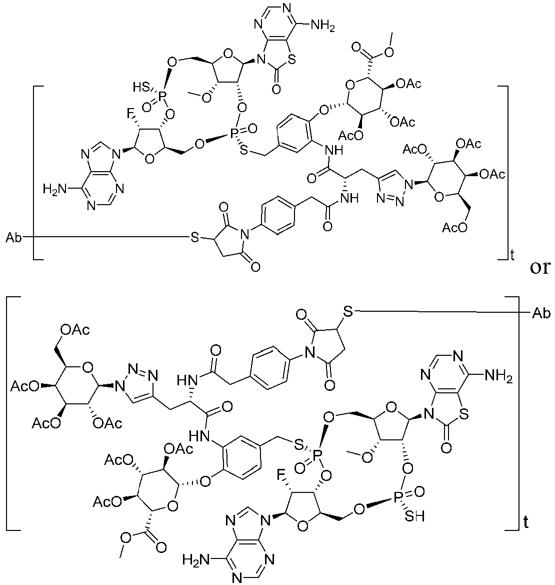 | | Antibody 1 | 3.17 |
| II-69 | 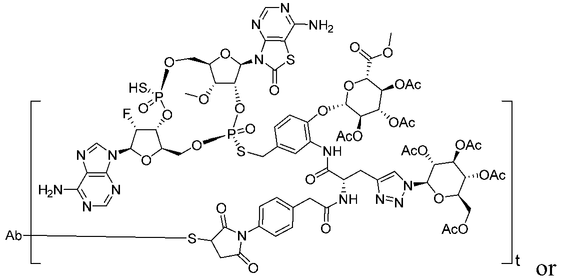 | | Antibody 1 | 3.55 |
| | 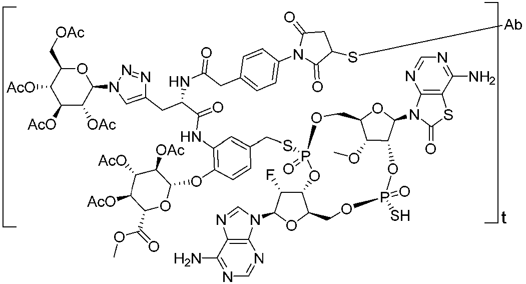 | | | |
| II-70 | 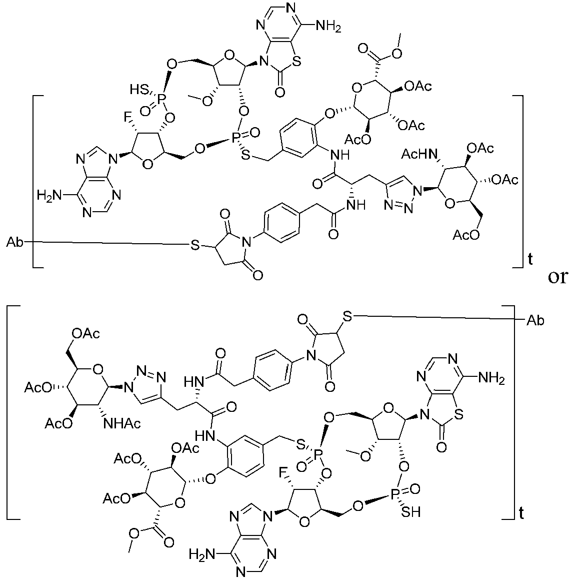 | Antibody 1 | 3.56 | |
| II-71 | 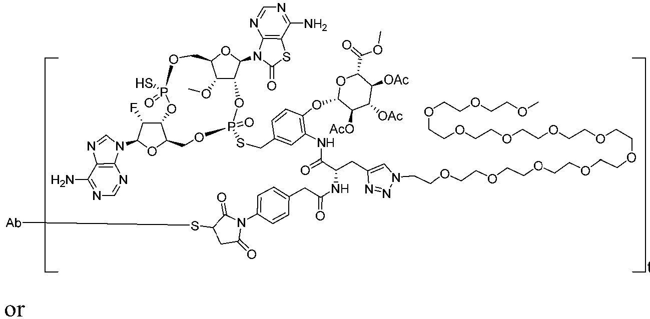 | Antibody 1 | 3.46 | |
| | 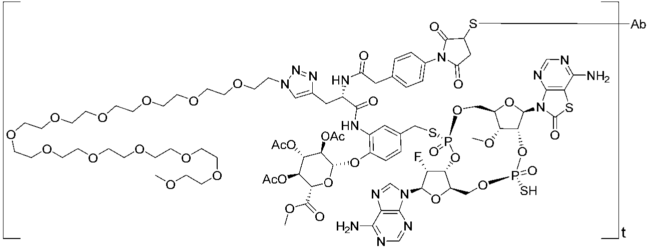 | | | |
| II-72 | 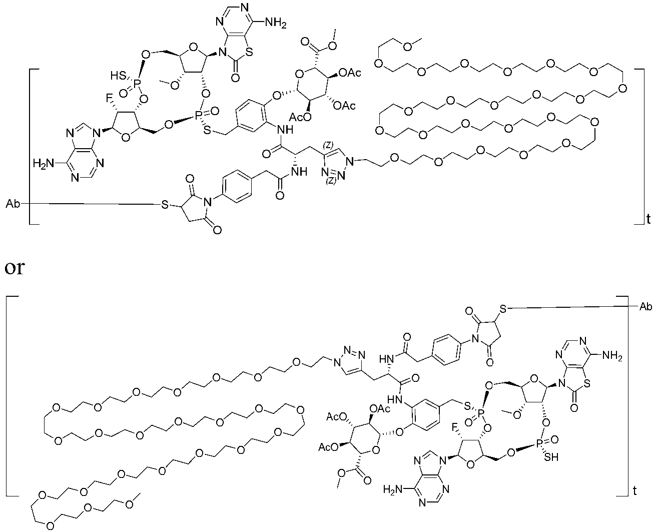 | Antibody 1 | 3.77 | |
| II-73 | 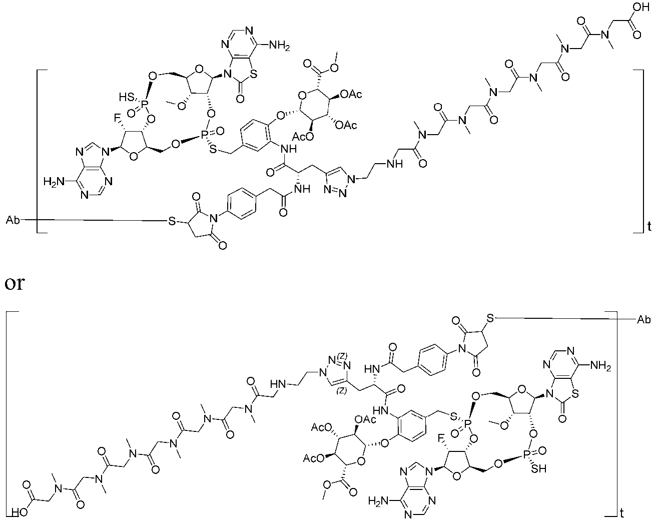 | Antibody 1 | 4.08 | |
| II-74 | 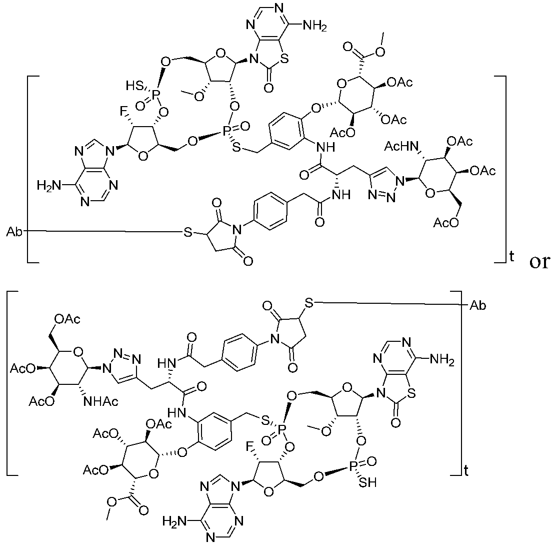 | | Antibody 1 | 3.67 |
| II-75 | 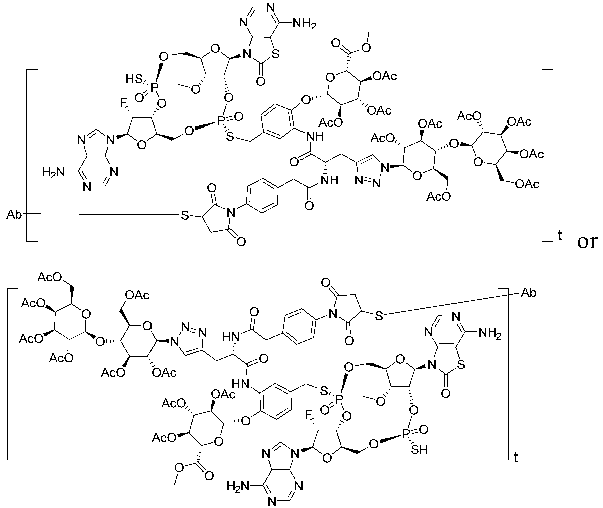 | | Antibody 1 | 3.94 |
| II-76 | 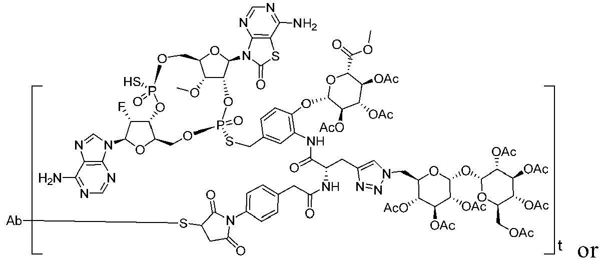 | | Antibody 1 | 4.03 |
| | 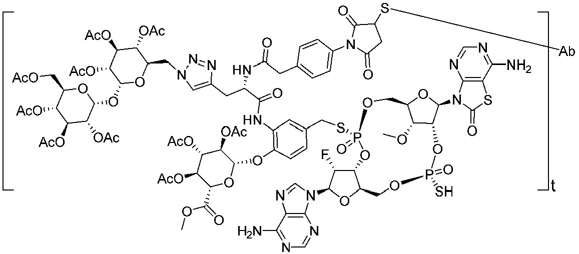 | | | |
| II-77 | 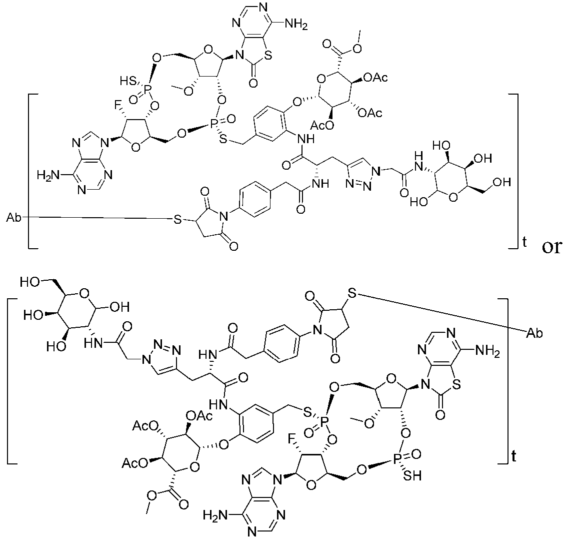 | | Antibody 1 | 3.80 |
| II-78 | 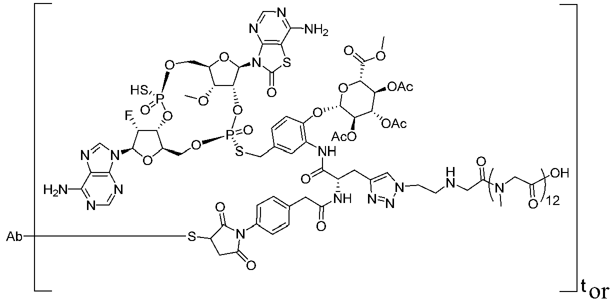 | | Antibody 1 | 3.66 |
| | 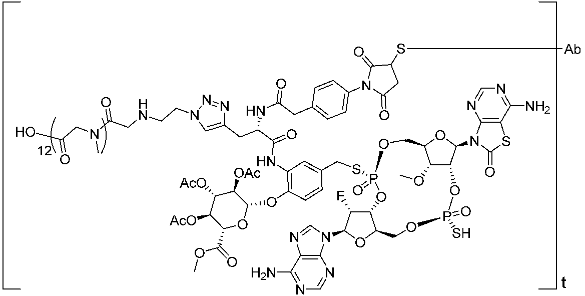 | | | |
| II-79 | 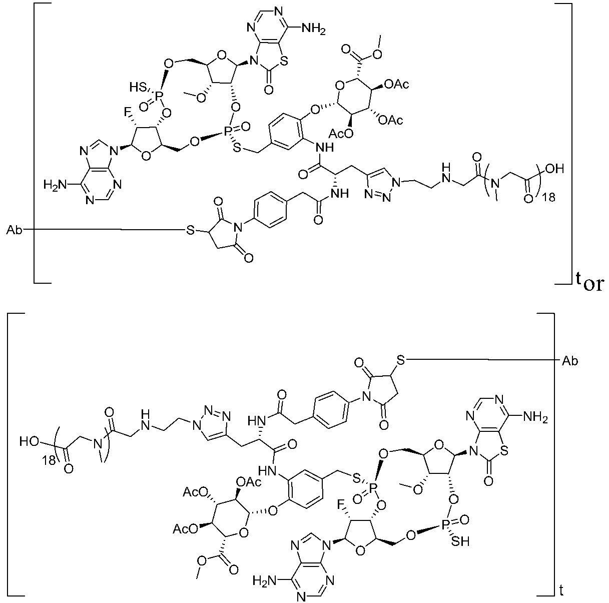 | | Antibody 1 | 3.83 |
| II-80 | 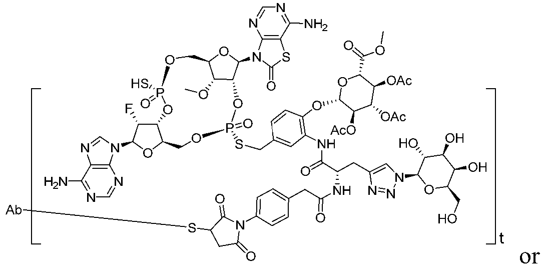 | | Antibody 1 | 3.83 |
| | 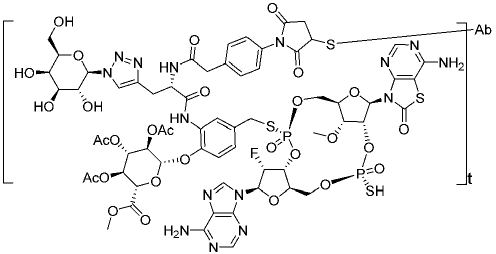 | | | |
| II-81 | 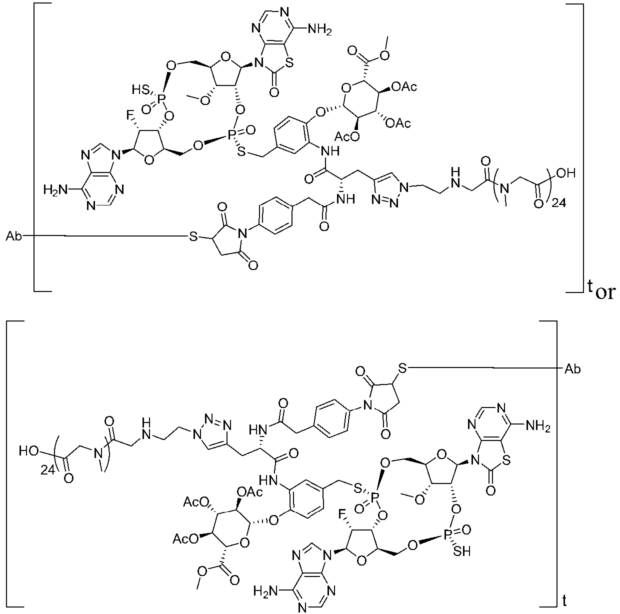 | | Antibody 1 | 3.77 |
| II-82 | 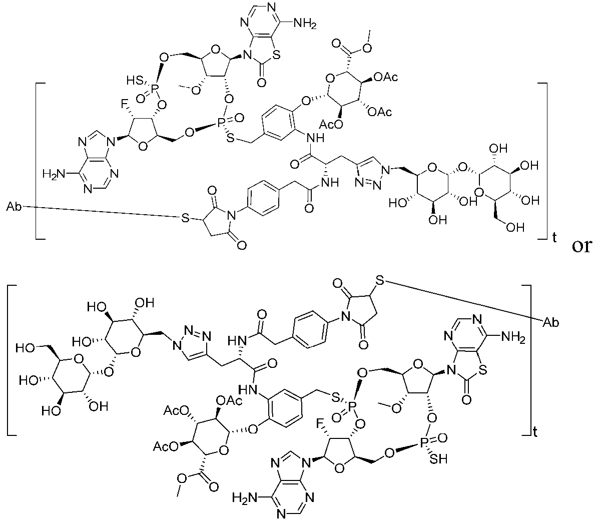 | | Antibody 1 | 4.02 |
| II-84 | 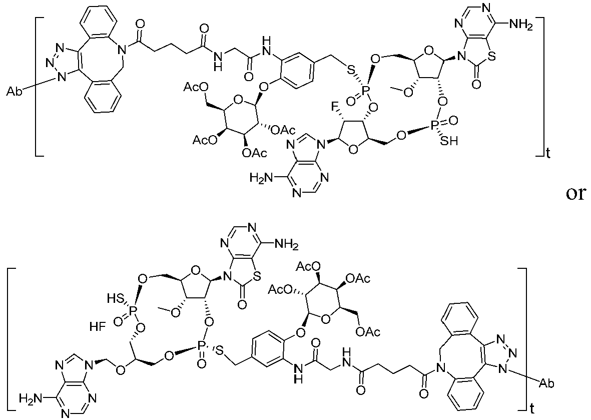 | | Antibody 12 | 1.51 |

wherein Antibody 1 is Trastuzumab; Antibody 2 is Pertuzumab; Antibody 3 is Trastuzumab-LALA (L234A/L235A); Antibody 4 is Trastuzumab-AAG (L234A/L235A/P329G); Antibody 5 is an engineered Trastuzumab with a cysteine inserted between positions 239 and 240 of the heavy chain; Antibody 7 is Cetuximab; Antibody 8 is huAl (V*_{H}*v2.0 + V*_{L}*v2.4); Antibody 9 is Trastuzumab (HC-s239.5, L234A/L235A/P329G); Antibody 11 is Enfortumab (Ha22-2); Antibody 12 is an antibody prepared using the method disclosed in Example 25 of CN115209921A with Cetuximab; Antibody 13 is Nimotuzumab.

In some embodiments, Antibody 3 is purchased from Shanghai Biointron Biological Inc., with catalog number B801901.

In some embodiments, Antibody 4 is purchased from Shanghai Biointron Biological Inc., with catalog number MHDDD001.

In some embodiments, Antibody 5 is purchased from Shanghai Biointron Biological Inc., with catalog number LPDDFD001.

In some embodiments, Antibody 8 is huAl (V*_{H}*v2.0 + V*_{L}*v2.4) antibody expressed and purified according to the sequence information table (SEQ ID NO: 54 and SEQ ID NO: 70) disclosed in US8044178B2.

In some embodiments, Antibody 9 is purchased from Shanghai Biointron Biological Inc., with catalog number CZ7DUD001.

The present disclosure provides a cyclic dinucleotide compound represented by Formula I or a pharmaceutically acceptable salt thereof,

D-LX **(I)**

wherein LX is a linker precursor with the following combination: -(L₁)ₐ-(Z)_{b}-M'; L₁ is connected to D;
a is 0, 1, 2, 3, 4, 5, or 6; b is 0, 1, 2, 3, 4, 5, 6, 7, 8, 9, or 10;
D is a group formed by the loss of one hydrogen atom from a compound represented by 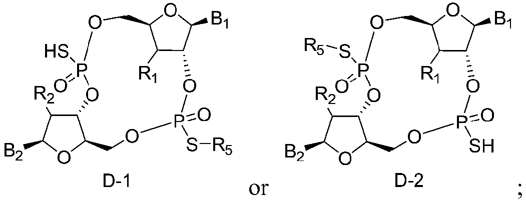
B₁ and B₂ are each independently 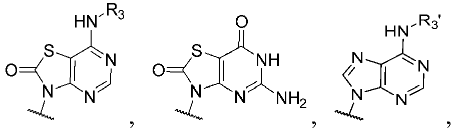 or 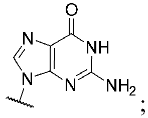 and at least one of B₁ or B₂ is 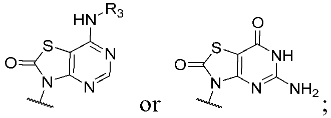
R₁ and R₂ are independently -OH, -N₃, F, -OCH₃, or -SCF₃;
R₃ and R_{3'} are independently H, -C(O)R₄, -C(O)OR₄, -OR₄, or -R₄;
R₄ is C₁₋₁₀ alkyl, C₆₋₁₀ aryl, 5- to 10-membered heteroaryl, C₃₋₁₀ cycloalkyl, 3- to 10-membered heterocycloalkyl, C₆₋₁₀ aryl-C₁₋₁₀ alkyl, 5- to 10-membered heteroaryl-C₁₋₁₀ alkyl, C₃₋₁₀ cycloalkyl-C₁₋₁₀ alkyl, or 3- to 10-membered heterocycloalkyl-C₁₋₁₀ alkyl; the R₄ is unsubstituted or optionally substituted at any position with 1 to 3 substituents selected from hydroxyl, thiol, dithiol, amino, C₁₋₆ alkylamino, and C₁₋₆ alkylamino-C₁₋₁₀ alkyl;
R₅ is H, -(L₂)_{d}-(Z)ₑ-(maleimido), or -(L₂)_{d}-(Z)ₑ-H;
d is 0, 1, 2, 3, 4, 5, or 6; e is 0, 1, 2, 3, 4, 5, 6, 7, 8, 9, or 10;
each L₁ and each L₂ is independently 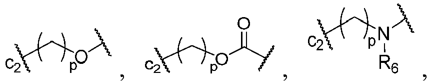 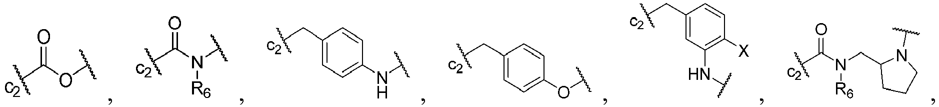 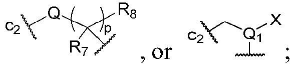 in L₁, the c₂ side is connected to D; in L₂, the c₂ side is connected to the thiol group in D;
X is 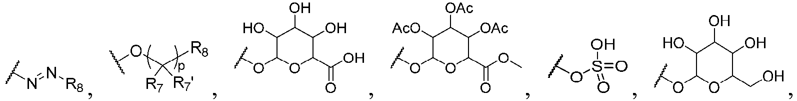 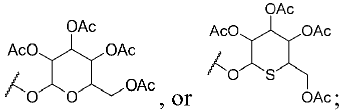
p is independently 1, 2, or 3;
Q is a linking bond, -C(O)O-, -C(O)N(R₆)-, or -C₁₋₃ alkylene-O-;
Q₁ is phenyl or pyridyl;
R₆ is H or C₁₋₆ alkyl; the C₁₋₆ alkyl is unsubstituted or optionally substituted at any position with one C₁₋₆ alkylamino or C₁₋₆ alkylsulfonyl;
R₇ and R_{7'} are each independently H or C₁₋₆ alkyl;
R₈ is phenyl or 5- to 10-membered heteroaryl; the R₈ is unsubstituted or optionally substituted at any position with 1-3 substituents selected from hydroxyl, amino, carboxyl, cyano, nitro, thiol, C₁₋₆ alkyl, C₃₋₈ cycloalkyl, halogen, halo-C₁₋₆ alkyl, C₁₋₆ alkylamino, C₁₋₆ alkoxy, halo-C₁₋₆ alkoxy, C₁₋₆ alkylthio, amido, hydroxyamino, aldehyde, acetyl, methoxycarbonyl, amino-C₁₋₆ alkyl, hydroxy-C₁₋₆ alkyl, and C₁₋₆ alkylsulfonyl;
each Z is independently -(A)ᵥ-, -PEG-, -C(O)-(CH₂)ₓ-, -NR₉-(CH₂)_{y}-, -O-(CH₂)_{y}-, -S-(CH₂)_{y}-, -(CH₂)ₓ-C₆₋₁₄ arylene-(CH₂)_{y}-, -(CH₂)ₓ-5- to 6-membered heteroarylene-(CH₂)_{y}-, - (CH₂)ₓ-C₃₋₆ cycloalkylene-(CH₂)_{y}-, -(CH₂)ₓ-3- to 6-membered heterocycloalkylene-(CH₂)_{y}-, - NR₉-(CH₂)ₓ-C(O)-, -O-(CH₂)ₓ-C(O)-, -S-(CH₂)ₓ-C(O)-, -(CH₂)ₓCH(-L₃-R₉)-C(O)-, - (CH₂)ₓCH(-L₃-R₉ₐ)-C(O)-, -(CH₂)ₓ-S-S-(CH₂)_{y}-, C₁₋₆ alkylene, or C₂₋₆ alkenylene;
L₃ is a linking bond, -NH-, or 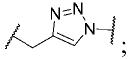
R₉ is independently hydrogen, C₁₋₆ alkyl, -PO(OH)₂, -PO(OCH₃)₂, -C(O)-(CH₂CH₂O)ₙ-CH3, -(CH₂CH₂O)ₙ-CH₃, 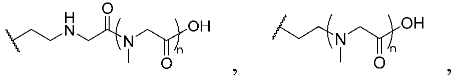 or 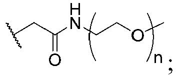
R₉ₐ is independently 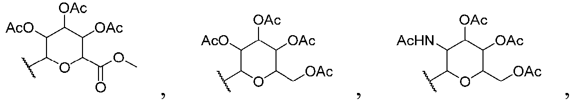 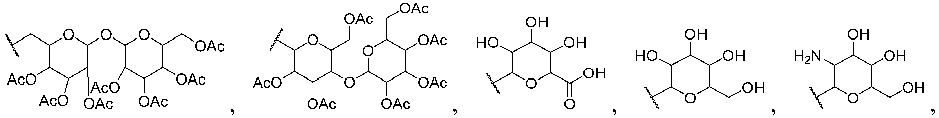 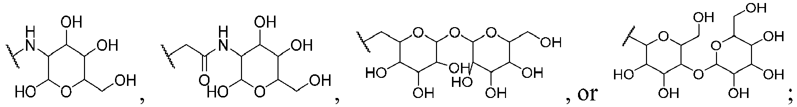
-PEG- is -(CH₂CH₂O)ₙ-(CH₂)ᵤ- or -(CH₂CH₂O)ₙ-(CH₂)ᵤ-C(O)-;
A is independently an amino acid residue;
x and y are independently 0, 1, 2, 3, 4, 5, 6, 7, 8, 9, or 10; v is independently 1, 2, 3, 4, or 5; n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50; u is independently 0, 1, 2, 3, 4, or 5;
M' is a linker head precursor.

In some embodiments, LX is a linker precursor with the following combination: - (L₁)ₐ-(Z)_{b}-M'; L₁ is connected to D;
a is 0, 1, 2, 3, 4, 5, or 6; b is 0, 1, 2, 3, 4, 5, 6, 7, 8, 9, or 10;
D is a group formed by the loss of one hydrogen atom from a compound represented by 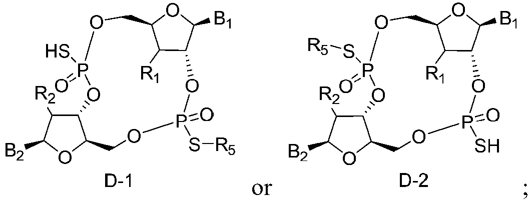
B₁ and B₂ are each independently 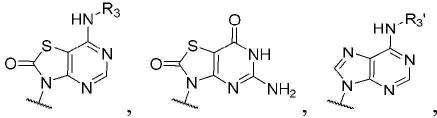 or 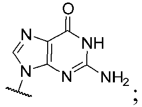 and at least one of B₁ or B₂ is 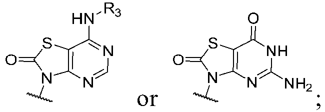
R₁ and R₂ are independently -OH, -N₃, F, -OCH₃, or -SCF₃;
R₃ and R_{3'} are independently H, -C(O)R₄, -C(O)OR₄, -OR₄, or -R₄;
R₄ is C₁₋₁₀ alkyl, C₆₋₁₀ aryl, 5- to 10-membered heteroaryl, C₃₋₁₀ cycloalkyl, 3- to 10-membered heterocycloalkyl, C₆₋₁₀ aryl-C₁₋₁₀ alkyl, 5- to 10-membered heteroaryl-C₁₋₁₀ alkyl, C₃₋₁₀ cycloalkyl-C₁₋₁₀ alkyl, or 3- to 10-membered heterocycloalkyl-C₁₋₁₀ alkyl; the R₄ is unsubstituted or optionally substituted at any position with 1 to 3 substituents selected from hydroxyl, thiol, dithiol, amino, C₁₋₆ alkylamino, and C₁₋₆ alkylamino-C₁₋₁₀ alkyl;
R₅ is H, -(L₂)_{d}-(Z)ₑ-(maleimido), or -(L₂)_{d}-(Z)ₑ-H;
d is 0, 1, 2, 3, 4, 5, or 6; e is 0, 1, 2, 3, 4, 5, 6, 7, 8, 9, or 10;
each L₁ and each L₂ is independently 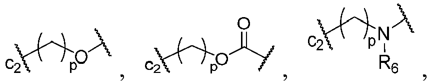 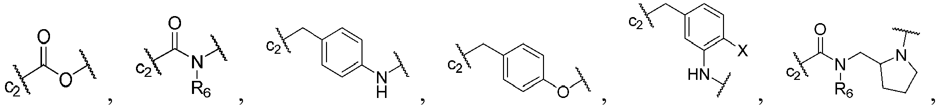 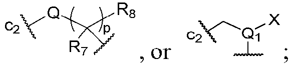 in L₁, the c₂ side is connected to D; in L₂, the c₂ side is connected to the thiol group in D;
X is 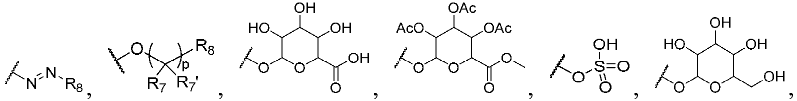 or 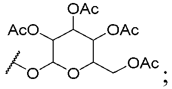
p is independently 1, 2, or 3;
Q is a linking bond, -C(O)O-, -C(O)N(R₆)-, or -C₁₋₃ alkylene-O-;
Q₁ is phenyl or pyridyl;
R₆ is H or C₁₋₆ alkyl; the C₁₋₆ alkyl is unsubstituted or optionally substituted at any position with one C₁₋₆ alkylamino or C₁₋₆ alkylsulfonyl;
R₇ and R_{7'} are each independently H or C₁₋₆ alkyl;
R₈ is phenyl or 5- to 10-membered heteroaryl; the R₈ is unsubstituted or optionally substituted at any position with 1-3 substituents selected from hydroxyl, amino, carboxyl, cyano, nitro, thiol, C₁₋₆ alkyl, C₃₋₈ cycloalkyl, halogen, halo-C₁₋₆ alkyl, C₁₋₆ alkylamino, C₁₋₆ alkoxy, halo-C₁₋₆ alkoxy, C₁₋₆ alkylthio, amido, hydroxyamino, aldehyde, acetyl, methoxycarbonyl, amino-C₁₋₆ alkyl, hydroxy-C₁₋₆ alkyl, and C₁₋₆ alkylsulfonyl;
each Z is independently -(A)ᵥ-, -PEG-, -C(O)-(CH₂)ₓ-, -NR₉-(CH₂)_{y}-, -O-(CH₂)_{y}-, -S-(CH₂)_{y}-, -(CH₂)ₓ-C₆₋₁₄ arylene-(CH₂)_{y}-, -(CH₂)ₓ-5- to 6-membered heteroarylene-(CH₂)_{y}-, - (CH₂)ₓ-C₃₋₆ cycloalkylene-(CH₂)_{y}-, -(CH₂)ₓ-3- to 6-membered heterocycloalkylene-(CH₂)_{y}-, - NR₉-(CH₂)ₓ-C(O)-, -O-(CH₂)ₓ-C(O)-, -S-(CH₂)ₓ-C(O)-, -(CH₂)ₓCH(NHR₉)-C(O)-, -(CH₂)ₓ-S-S-(CH₂)_{y}-, C₁₋₆ alkylene, or C₂₋₆ alkenylene;
R₉ is independently hydrogen, C₁₋₆ alkyl, -PO(OH)₂, -PO(OCH₃)₂, -C(O)-(CH₂CH₂O)ₙ-CH₃, or -(CH₂CH₂O)ₙ-CH₃;
-PEG- is -(CH₂CH₂0)ₙ-(CH₂)ᵤ- or -(CH₂CH₂O)ₙ-(CH₂)ᵤ-C(O)-;
A is independently an amino acid residue;
x and y are independently 0, 1, 2, 3, 4, 5, 6, 7, 8, 9, or 10; v is independently 1, 2, 3, 4, or 5; n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50; u is independently 0, 1, 2, 3, 4, or 5;
M' is a linker head precursor.

In some embodiments, in the cyclic dinucleotide compound represented by Formula I or the pharmaceutically acceptable salt thereof, some of the groups are defined as follows, and the rest of the groups are defined as described in any other embodiment (hereinafter referred to as "in some embodiments"): the a is 0, 1, 2, 3, or 4; a is preferably 0, 1, or 2.

In some embodiments, b is 1, 2, 3, 4, 5, 6, 7, 8, 9, or 10; b is preferably 1, 2, 3, 4, 5, 6, 7, or 8; b is more preferably 1, 2, 3, 4, 5, or 6.

In some embodiments, d is 0, 1, 2, or 3; d is preferably 1 or 2; d is more preferably 1.

In some embodiments, e is 0, 1, 2, 3, 4, 5, or 6; e is preferably 0, 1, 2, 3, or 4; e is more preferably 0, 1, 2, or 3.

In some embodiments, R₆ is H or C₁₋₆ alkyl; the C₁₋₆ alkyl is unsubstituted or optionally substituted at any position with one dimethylamino or methylsulfonyl.

In some embodiments, R₆ is H, -CH₃, -CH₂CH₂N(CH₃)₂, or -CH₂CH₂S(O)₂CH₃.

In some embodiments, each L₁ and each L₂ is independently 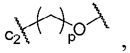 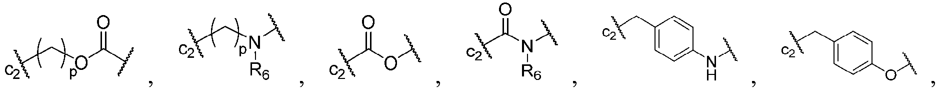 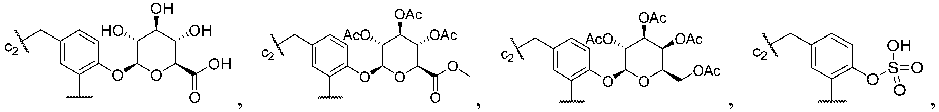 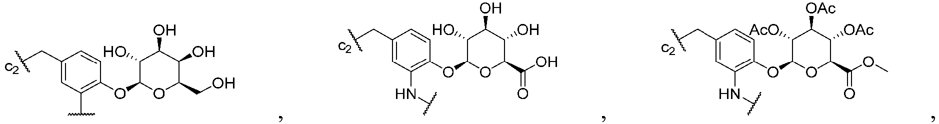 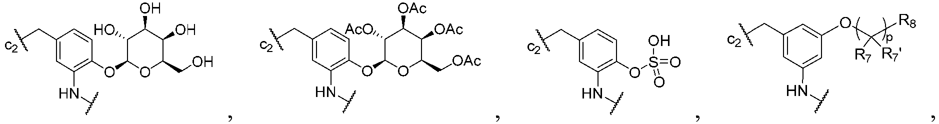 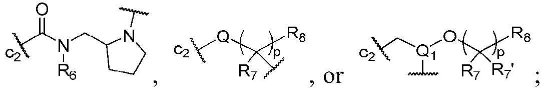 in L₁, the c₂ side is connected to D; in L₂, the c₂ side is connected to the thiol group in D.

In some embodiments, each L₁ and each L₂ is independently 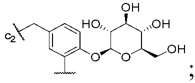 in L₁, the c₂ side is connected to D; in L₂, the c₂ side is connected to the thiol group in D.

In some embodiments, -(L₁)ₐ- is 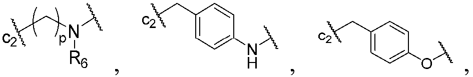 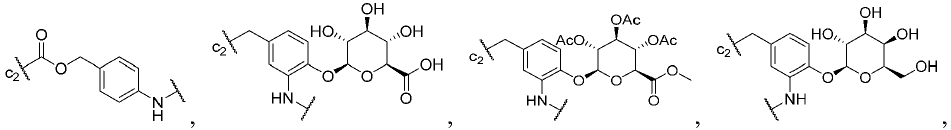 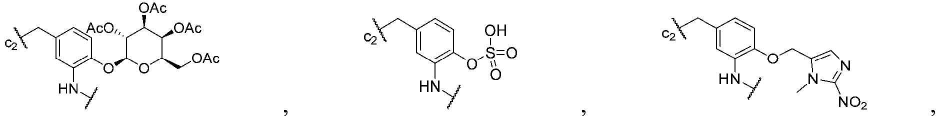 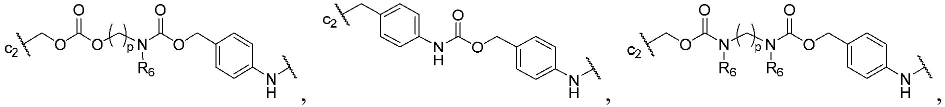 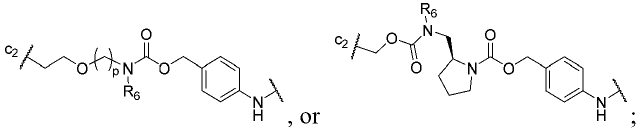 the c₂ side is connected to D.

In some embodiments, -(L₁)ₐ- is 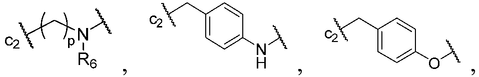 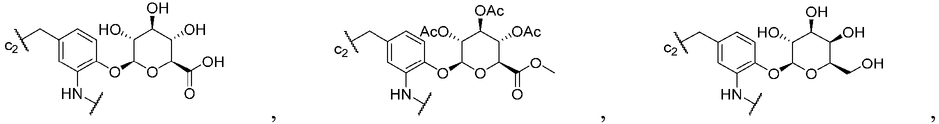 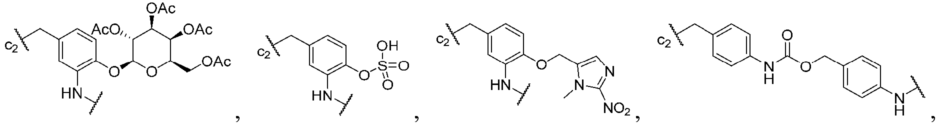 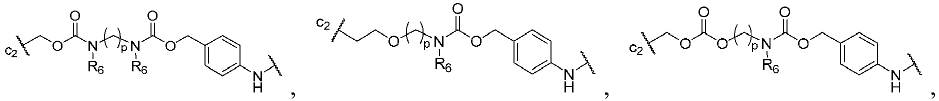 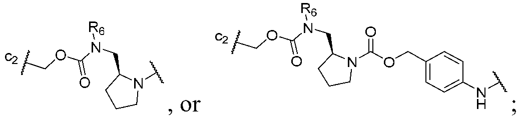 the c₂ side is connected to the thiol group in D.

In some embodiments, -(L₁)ₐ- is 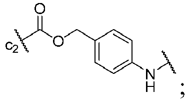 the c₂ side is connected to the amino in D.

In some embodiments, -(L₁)ₐ- is 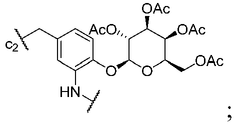 the c₂ side is connected to the amino in D.

In some embodiments, -(L₁)ₐ- is absent, 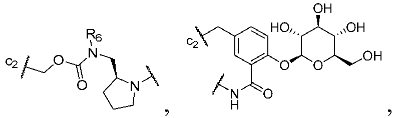 or 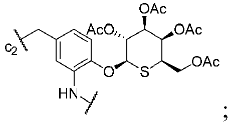 the c₂ side is connected to the thiol group in D.

In some embodiments, Z is independently -(A)ᵥ-, -PEG-, -C(O)-(CH₂)ₓ-, -NR₉-(CH₂)_{y}-, -O-(CH₂)_{y}-, -S-(CH₂)_{y}-, -(CH₂)ₓ-C₆₋₁₄ arylene-(CH₂)_{y}-, -(CH₂)ₓ-5- to 6-membered heteroarylene-(CH₂)_{y}-, -(CH₂)ₓ-C₃₋₆ cycloalkylene-(CH₂)_{y}-, -(CH₂)ₓ-3- to 6-membered heterocycloalkylene-(CH₂)_{y}-, -NR₉-(CH₂)ₓ-C(O)-, -O-(CH₂)ₓ-C(O)-, -S-(CH₂)ₓ-C(O)-, - (CH₂)ₓCH(NHR₉)-C(O)-, -(CH₂)ₓ-S-S-(CH₂)_{y}-, C₁₋₆ alkylene, or C₂₋₆ alkenylene; x and y are independently any integer of 0, 1, 2, 3, 4, 5, 6, 7, 8, 9 or 10; v is independently any integer of 1, 2, 3, 4 or 5; in the "5- to 6-membered heteroarylene" of -(CH₂)ₓ-5- to 6-membered heteroarylene-(CH₂)_{y}-, the heteroatom is selected from one or more kinds of N, O and S, and the number of heteroatoms is 1, 2 or 3; in the "3- to 6-membered heterocycloalkylene" of - (CH₂)ₓ-3- to 6-membered heterocycloalkylene-(CH₂)_{y}-, the heteroatom is selected from one or more kinds of N, O and S, and the number of heteroatoms is 1, 2 or 3.

In some embodiments, in A, the amino acid is a natural amino acid or a non-natural amino acid.

In some embodiments, in A, the amino acid residue is modified with 1 to 3 R₁₀; R₁₀ is independently C₁₋₆ alkyl, C₁₋₆ alkylacyl, C₁₋₆ alkoxyacyl, -C(O)-(CH₂CH₂O)ₙ-CH₃, or - (CH₂CH₂O)ₙ-CH₃; the C₁₋₆ alkyl, C₁₋₆ alkylacyl, or C₁₋₆ alkoxyacyl may further be substituted with one R₈ at any position.

In some embodiments, each A is independently 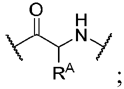 each R^{A} is independently an amino acid side chain or an amino acid side chain modified with 1 to 3 R₁₀; or R^{A} and the adjacent nitrogen atom form a five-membered heterocyclic group;
the R₁₀ is independently C₁₋₆ alkyl, C₁₋₆ alkylacyl, C₁₋₆ alkoxyacyl, -C(O)-(CH₂CH₂O)ₙ-CH3, or -(CH₂CH₂O)ₙ-CH₃; the C₁₋₆ alkyl, C₁₋₆ alkylacyl, or C₁₋₆ alkoxyacyl may further be substituted with one R₈ at any position.

In some embodiments, the 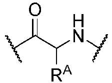 may be 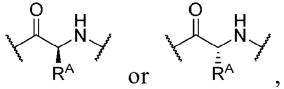 or may also be

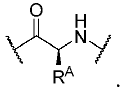

In some embodiments, in A, each R^{A} is independently hydrogen, methyl, ethyl, n-propyl, isopropyl, isobutyl, 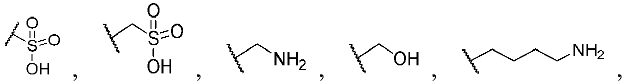 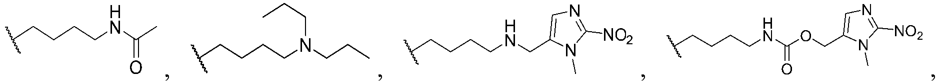 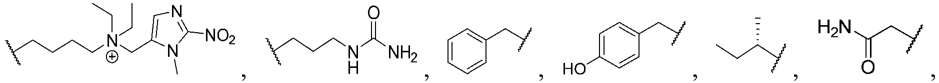 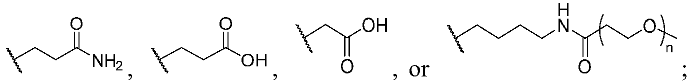 or R^{A} and the adjacent nitrogen atom form a five-membered heterocyclic group.

In some embodiments, -(A)ᵥ- is 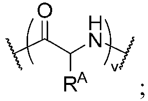 each R^{A} is independently hydrogen, methyl, ethyl, n-propyl, isopropyl, isobutyl, 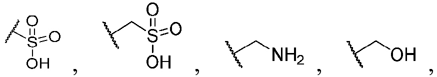 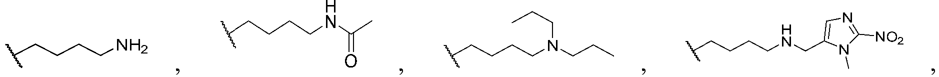 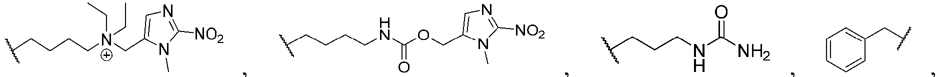 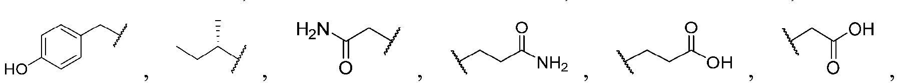 or 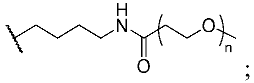 or R^{A} and the adjacent nitrogen atom form a five-membered heterocyclic group; v is any integer from 1 to 4, preferably 1 or 2.

In some embodiments, -(A)ᵥ- is

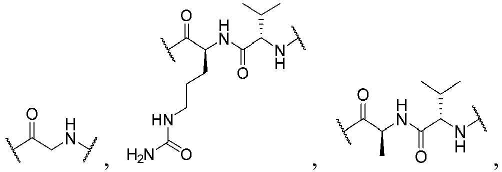

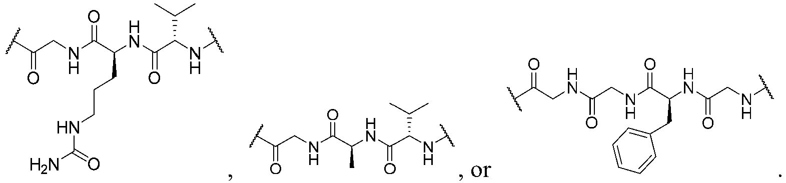

In some embodiments, -(A)ᵥ- is 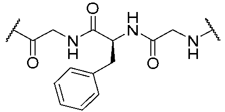 or

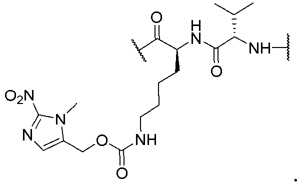

In some embodiments, -(Z)_{b}- is any one of the following combinations:
1) -(CH₂)ₓ-(c₁),
2) -(CH₂CH₂O)ₙ-(CH₂)ᵤ-NHC(O)-(CH₂)ₓ- (c₁),
3) -C(O)-(CH₂)ₓ-(c₁),
4) -C(O)-(CH₂)ₓ-C₃₋₆ cycloalkylene-(CH₂)_{y}-(c₁),
5) -C(O)-(CH₂)ₓ-phenylene-(CH₂)_{y}-(c₁),
6) -C(O)-(CH₂CH₂O)ₙ-(CH₂)ᵤ-NHC(O)-(CH₂)ₓ-(c₁),
7) -C(O)-(CH₂CH₂O)ₙ-(CH₂)ᵤ-NHC(O)-(CH₂)ₓ-phenylene-(CH₂)_{y}-(c₁),
8) -(A)ᵥ-(c₁),
9) -(A)ᵥ-C(O)-(CH₂)ₓ-(c₁),
10) -(A)ᵥ-C(O)-(CH₂)ₓ-phenylene-(CH₂)_{y}-(c₁),
11) -(A)ᵥ-C(O)-(CH₂CH₂O)ₙ-(CH₂)ᵤ-NHC(O)-(CH₂)ₓ-(c₁), or
12) -(A)ᵥ-C(O)-(CH₂CH₂O)ₙ-(CH₂)ᵤ-NHC(O)-(CH₂)ₓ-phenylene-(CH₂)_{y}-(c₁); wherein the c₁ side is connected to M'.

In some embodiments, -(Z)_{b}- is any one of the following combinations:
13) -C(O)-(CH₂)ₓ-(A)ᵥ-C(O)-(CH₂)ₓ-(c₁);
14) -(A)ᵥ-C(O)-(CH₂CH₂O)ₙ-(CH₂)ᵤ-(c₁);
15) -C(O)-(CH₂)ₓ-NH-(c₁);
16) -C(O)-CH(-L₃-R₉)-NHC(O)-(CH₂)ₓ-phenylene-(CH₂)_{y}-;
17) -C(O)-CH(-L₃-R₉)-NHC(O)-(CH₂)ₓ-, wherein the c₁ side is connected to M'.

In some embodiments, M' is a linker head precursor for reacting with the side chain of an antibody amino acid residue, preferably a linker head precursor for reacting with amino, thiol, azido, aldehyde, acyl, or phenolic hydroxyl of the side chain of the antibody amino acid residue.

In some embodiments, M' is a linker head precursor for reacting with the side chain of an antibody amino acid residue, preferably a linker head precursor for reacting with the amino or thiol group of the side chain of the antibody amino acid residue.

In some embodiments, M' is ethynyl, vinyl, hydroxylamino, 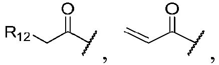 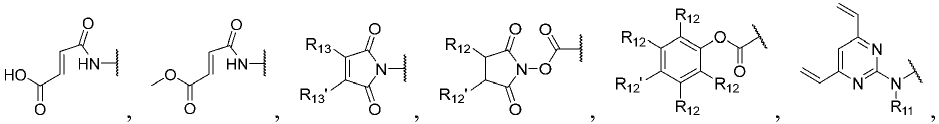 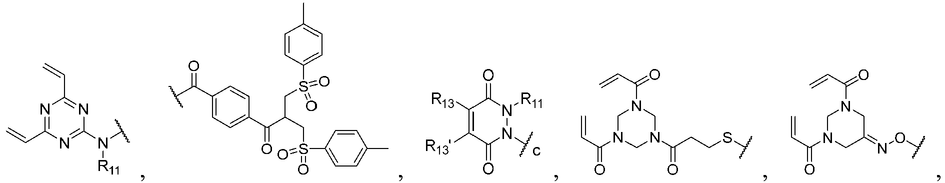 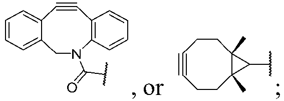
R₁₁ is hydrogen or C₁₋₄ alkyl;
each of R₁₂ and R_{12'} is independently halogen (e.g., bromine or iodine), nitro, or -SO₃⁻;
R₁₃ and R_{13'} are each independently hydrogen, halogen (e.g., bromine), phenylthio, or pyridylthio.

In some embodiments, R₁₁ is hydrogen, methyl, or ethyl.

In some embodiments, M' is 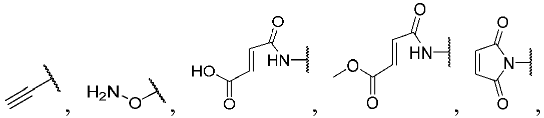 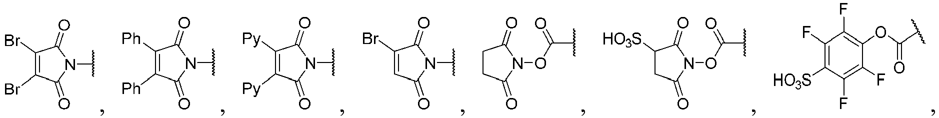 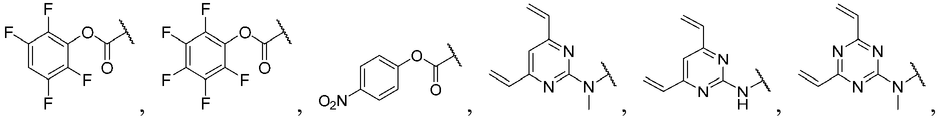 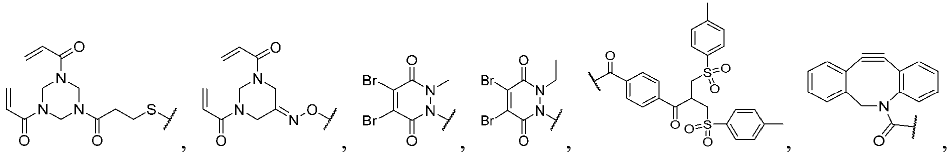 or

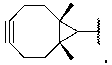

In some embodiments, M' is

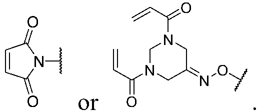

In some embodiments, LX is 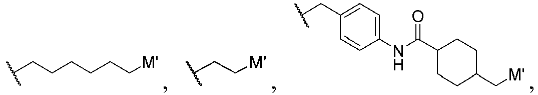 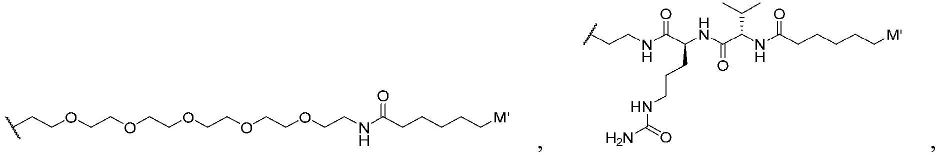 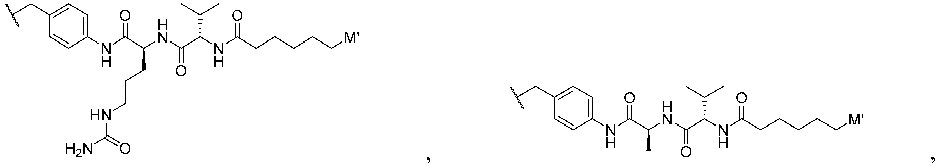 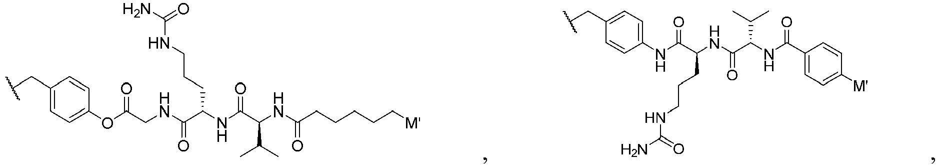 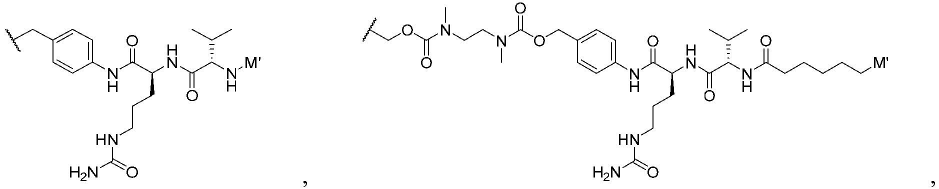 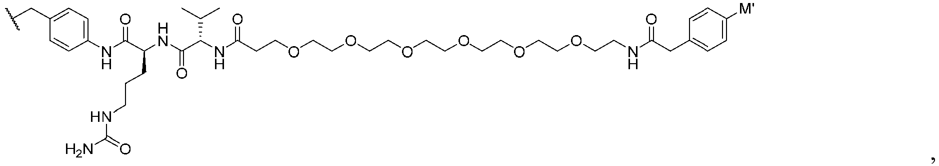 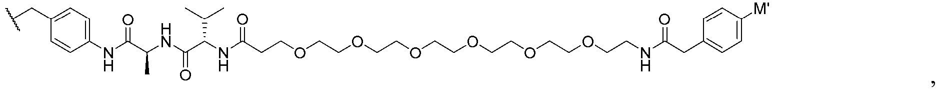 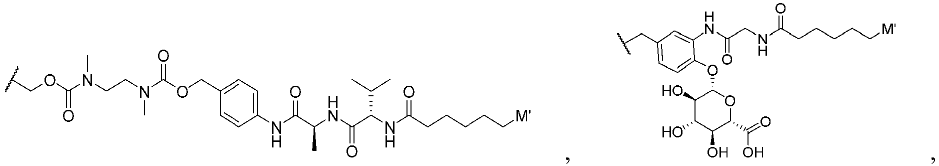 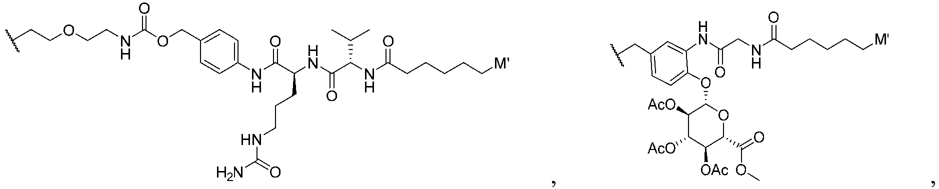 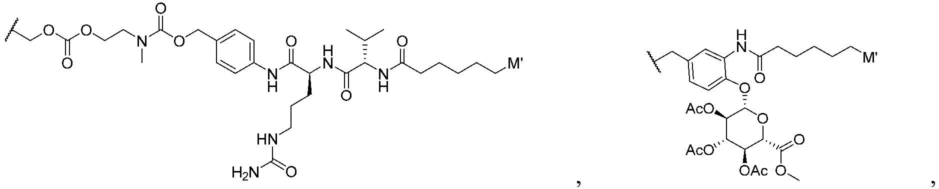 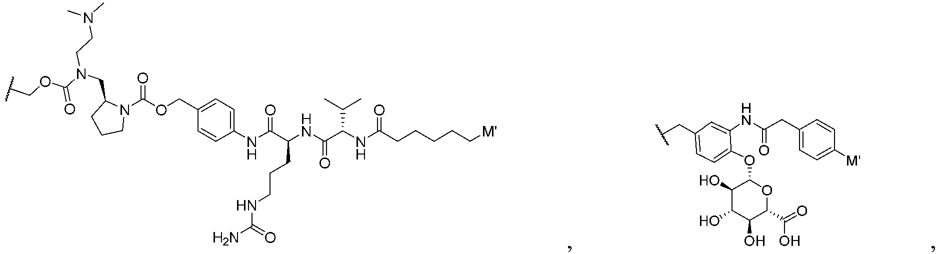 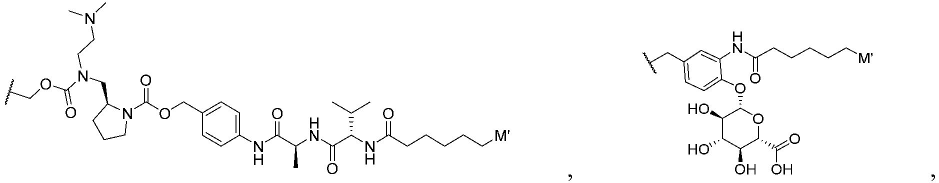 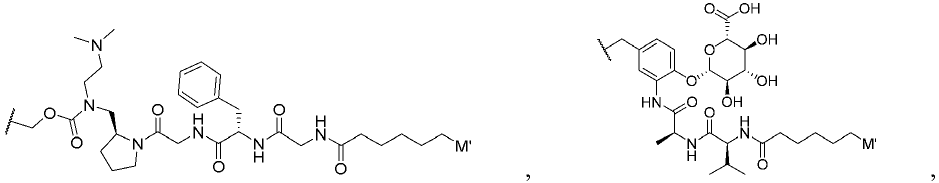 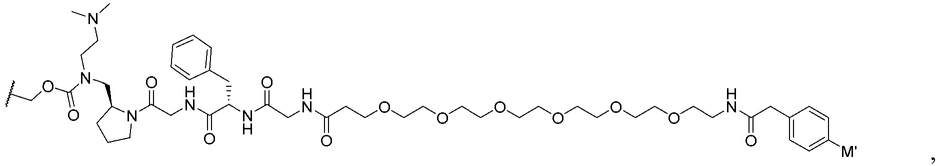 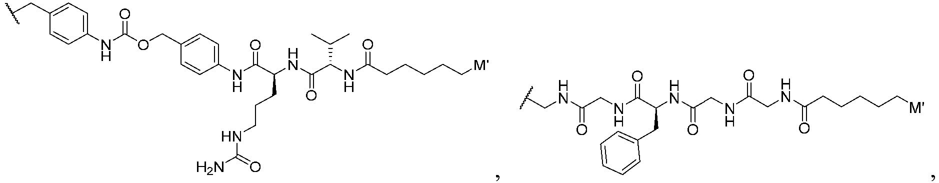 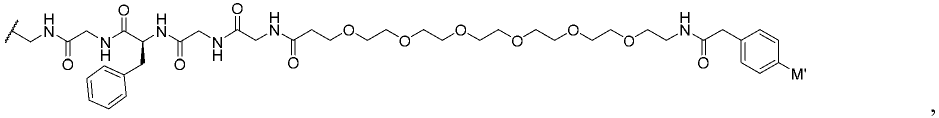 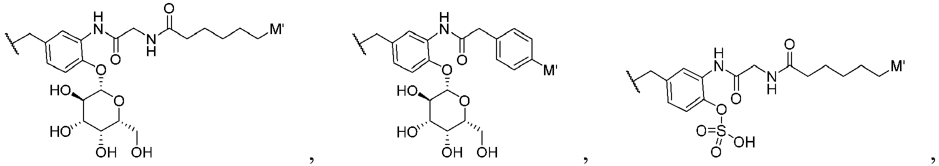 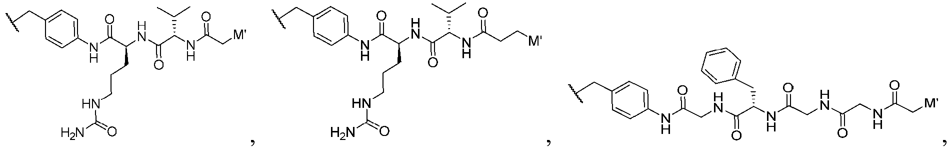 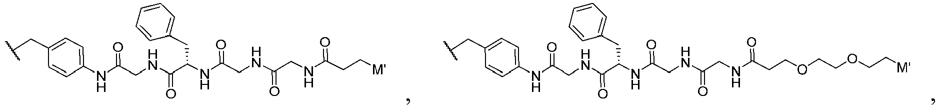 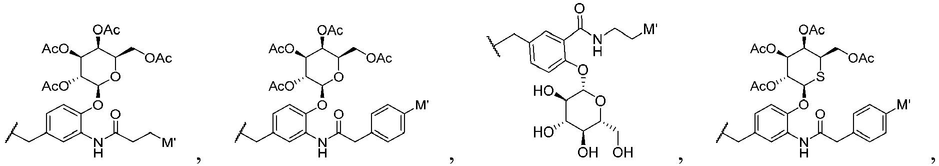 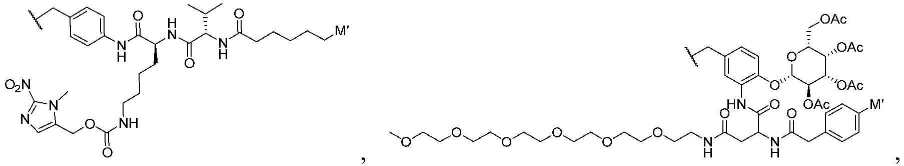 or

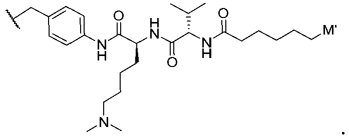

In some embodiments, LX is

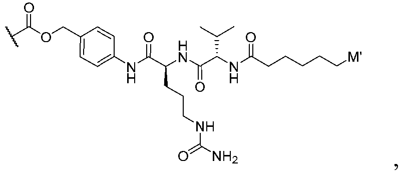

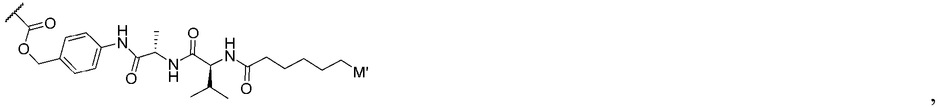

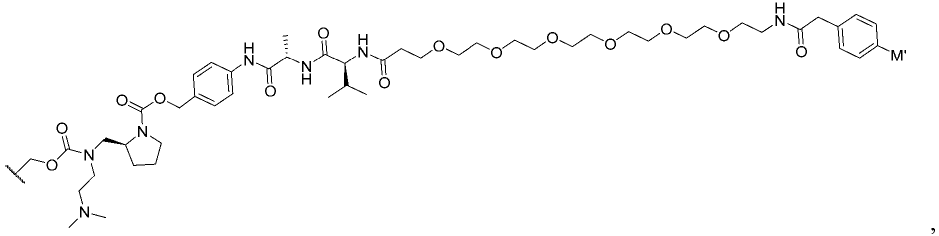

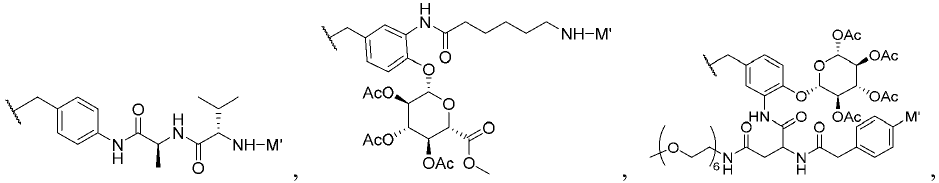

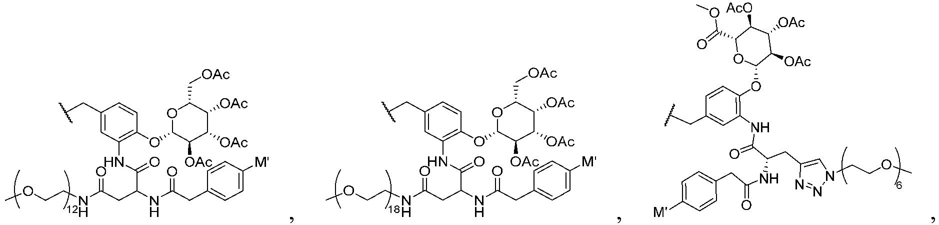

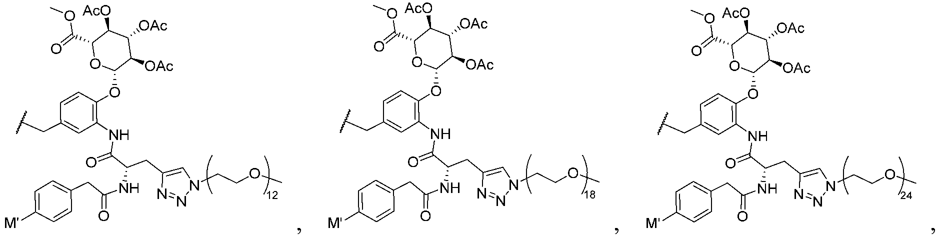

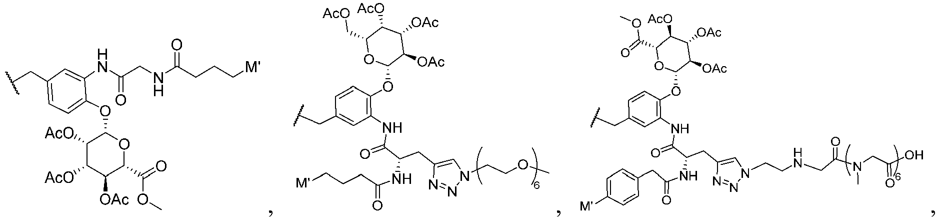

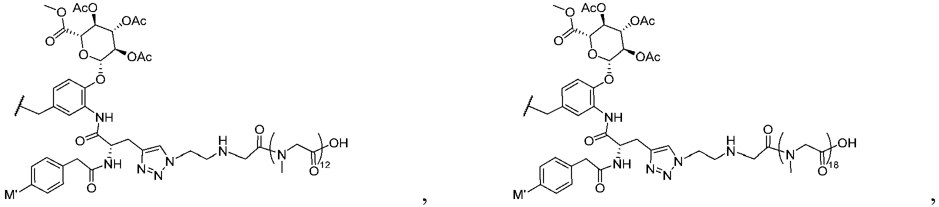

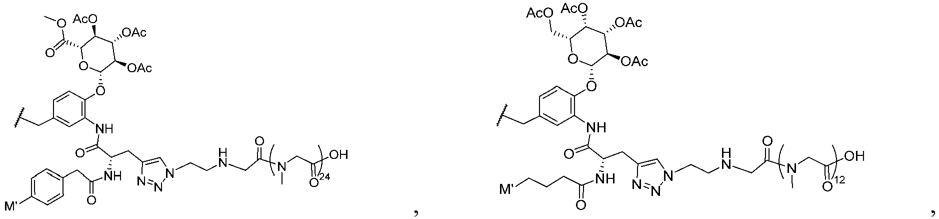

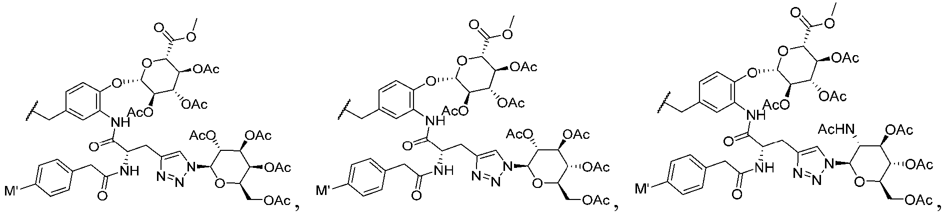

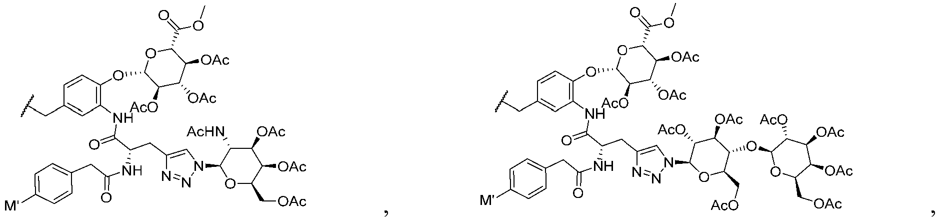

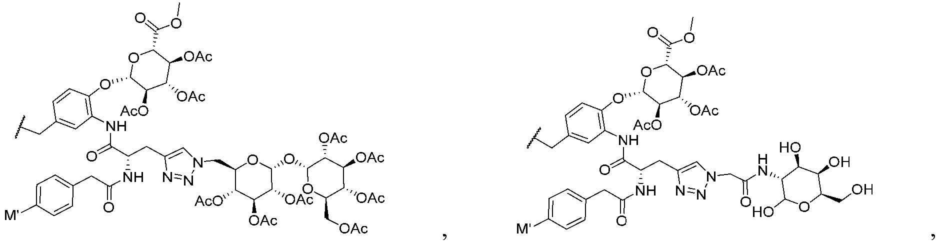

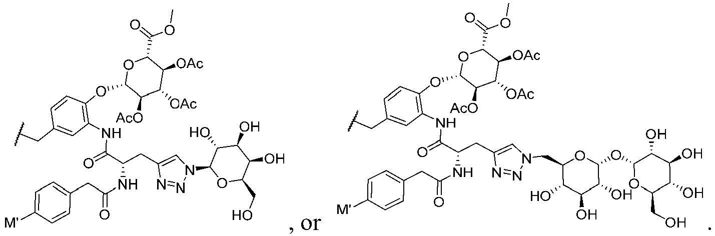

In some embodiments, D is a group formed by the loss of one hydrogen atom from a compound represented by 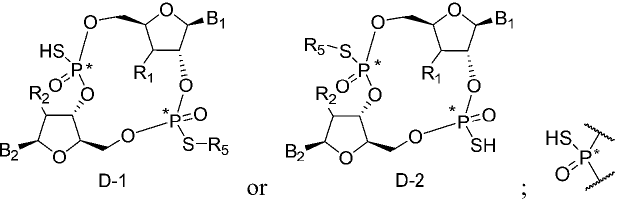 is 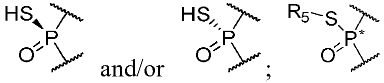 is

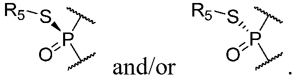

In some embodiments, d is 1.

In some embodiments, R₅ is H, -L₂-(Z)ₑ-(maleimido), or -L₂-(Z)ₑ-H.

In some embodiments, R₅ is -L₂-(Z)ₑ-H; -L₂- is 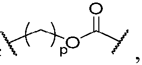 with the c₂ side connected to the thiol group in D; e is 1; -(Z)ₑ-H is C₁₋₆ alkyl.

In some embodiments, R₅ is -L₂-(Z)ₑ-H; -L₂- is 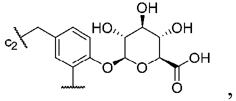 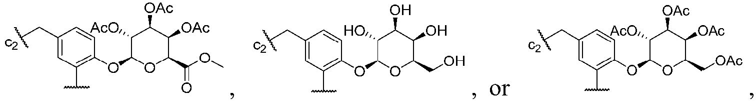 with the c₂ side connected to the thiol group in D; e is 3, -(Z)ₑ-H is -C(O)NH-(CH₂)_{y}-O-(CH₂)_{y}-H.

In some embodiments, R₅ is -L₂-(Z)ₑ-H; -L₂- is 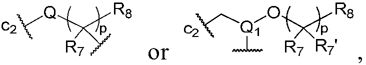 with the c₂ side connected to the thiol group in D; e is 0 or 1, -(Z)ₑ-H is H or methyl.

In some embodiments, R₅ is 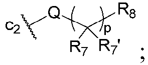 Q is a linking bond, -C(O)O-, or -C₁₋₃ alkylene-O-; R₇ and R_{7'} are each independently H or methyl.

In some embodiments, R₅ is 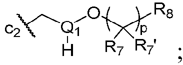 Q₁ is phenyl; R₇ and R_{7'} are each independently H or methyl.

In some embodiments, R₈ is phenyl or 5- to 6-membered heteroaryl; the 5- to 6-membered heteroaryl is preferably pyridyl, pyrimidinyl, pyrazinyl, pyrrolyl, thienyl, furyl, pyrazolyl, thiazolyl, or oxazolyl; R₈ is unsubstituted or optionally substituted at any position with 1 to 3 substituents selected from hydroxyl, amino, cyano, nitro, thiol, C₁₋₆ alkyl, halogen, halo-C₁₋₆ alkyl, C₁₋₆ alkylamino, C₁₋₆ alkoxy, halo-C₁₋₆ alkoxy, C₁₋₆ alkylthio, amido, aldehyde, acetyl, methoxycarbonyl, amino-C₁₋₆ alkyl, hydroxy-C₁₋₆ alkyl, and methylsulfonyl.

In some embodiments, R₅ is -L₂-(Z)ₑ-(maleimido); -L₂- is 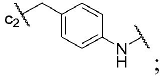 the c₂ side is connected to the thiol group in D; -(Z)ₑ- is -(A)ᵥ-C(O)-(CH₂)ₓ-(c₃); the c₃ side is connected to the maleimido.

In some embodiments, R₅ is 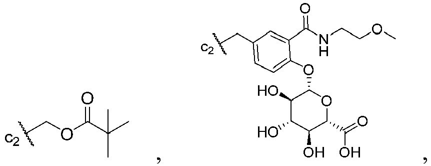 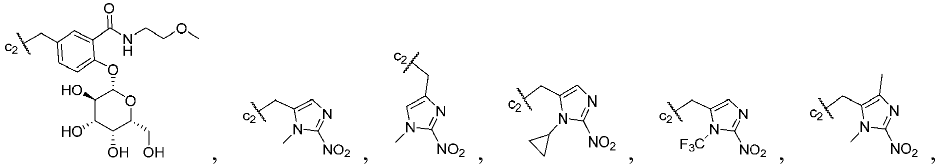 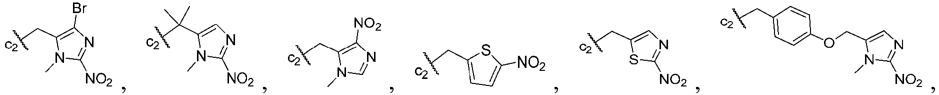 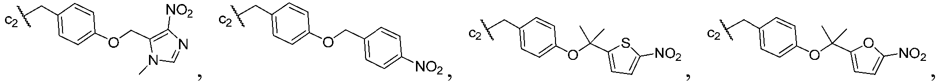 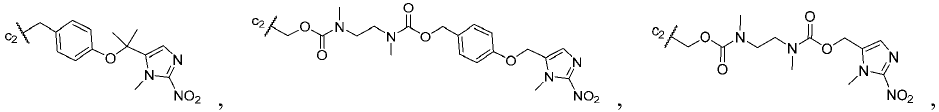 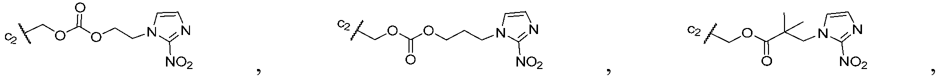 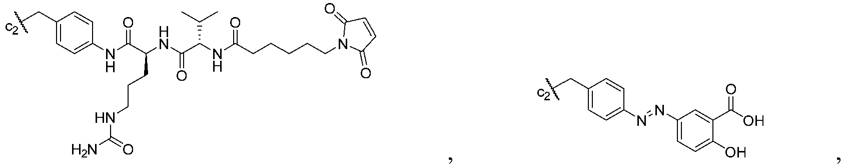 or 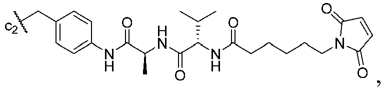 with the c₂ side connected to the thiol group in D.

In some embodiments, R₅ is 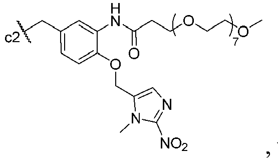 with the c₂ side connected to the thiol group in D.

In some embodiments, B₁ and B₂ are each independently 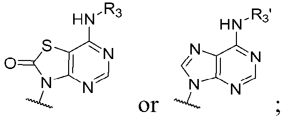 and at least one of B₁ or B₂ is

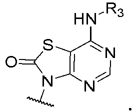

In some embodiments, D is a group formed by the loss of one hydrogen atom from the amino or thiol group in a compound represented by 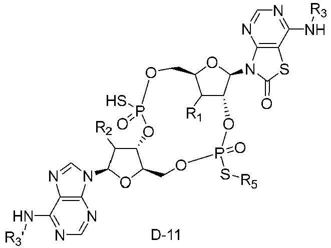 or

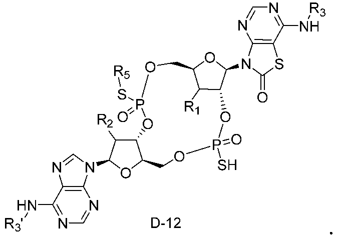

In some embodiments, D is a group formed by the loss of one hydrogen atom from the amino group in a compound represented by 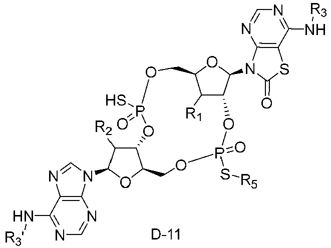 or 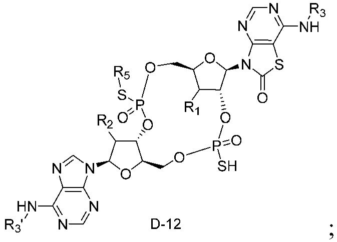 R₃ or R₃, is each independently

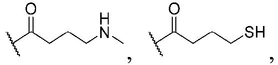

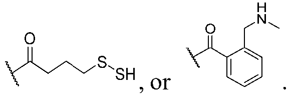

In some embodiments, D is a group formed by the loss of one hydrogen atom from the thiol group in a compound represented by 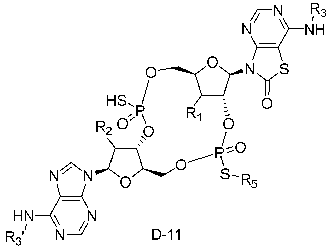 or

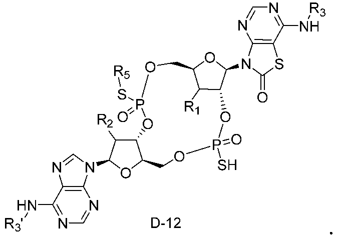

In some embodiments, D is a group formed by the loss of one hydrogen atom from the thiol group in a compound represented by 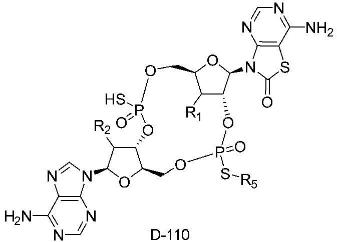 or

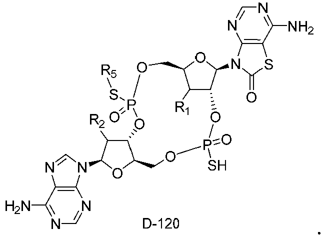

In some embodiments, D is any one of the following structures or a pharmaceutically acceptable salt thereof: 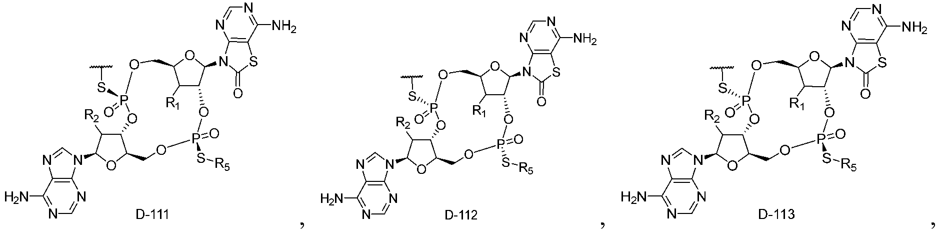 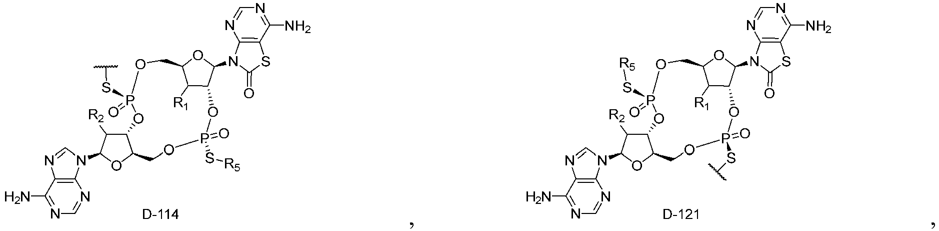 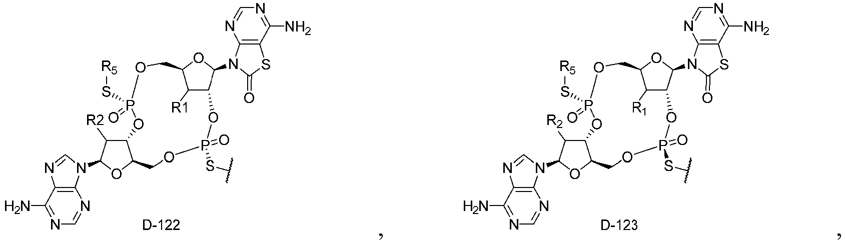 or 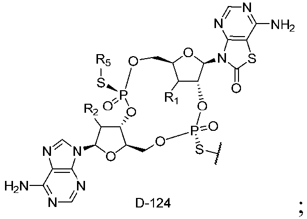 wherein R₁, R₂, and R₅ are as defined above.

In some embodiments, D is any one of the following structures: 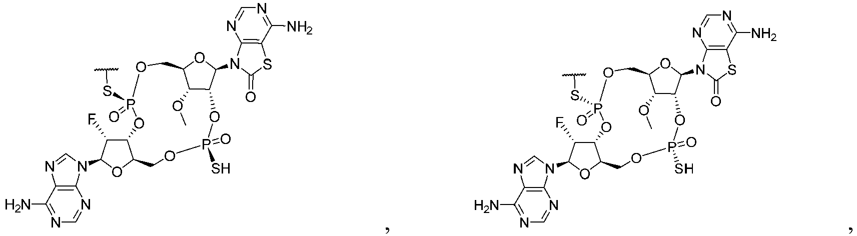 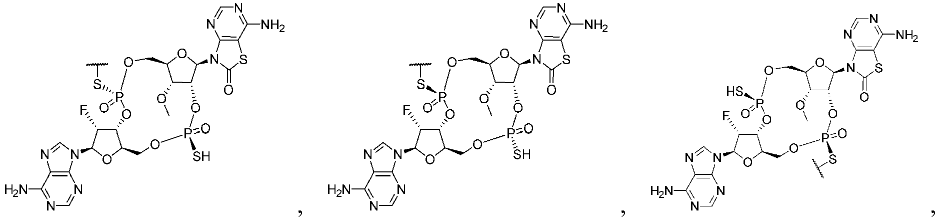 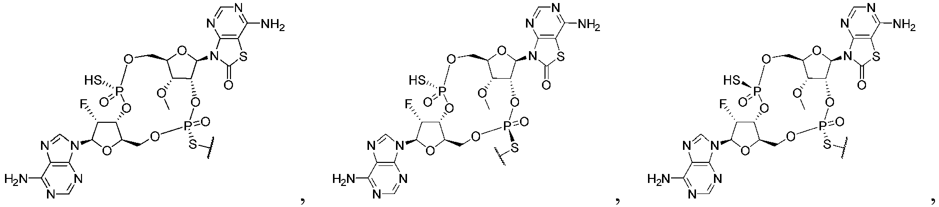 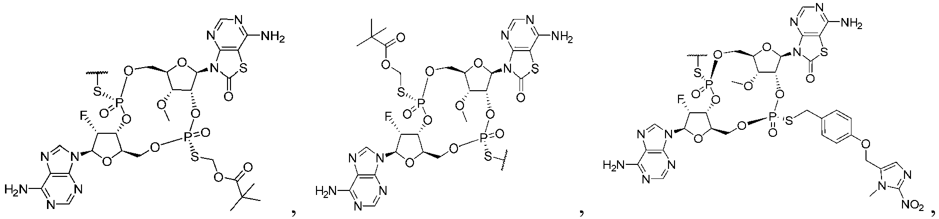 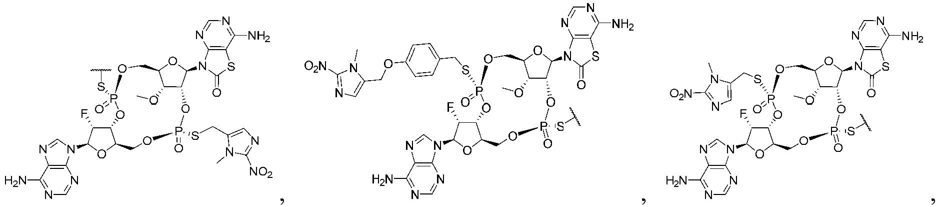 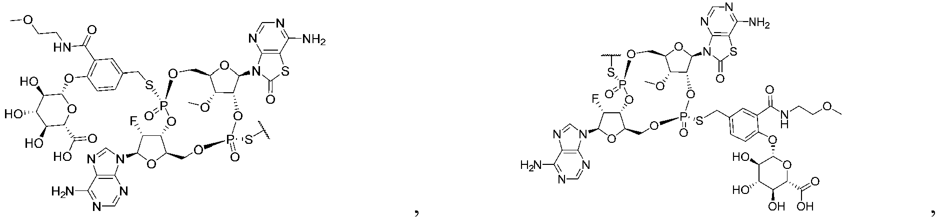 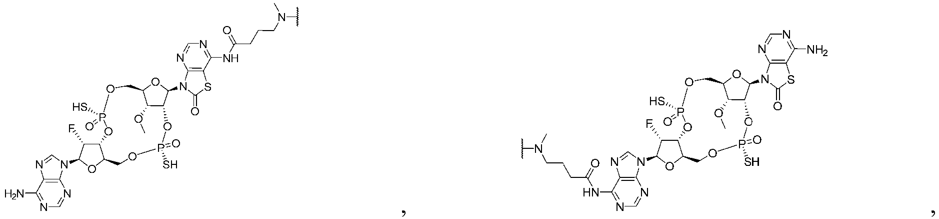 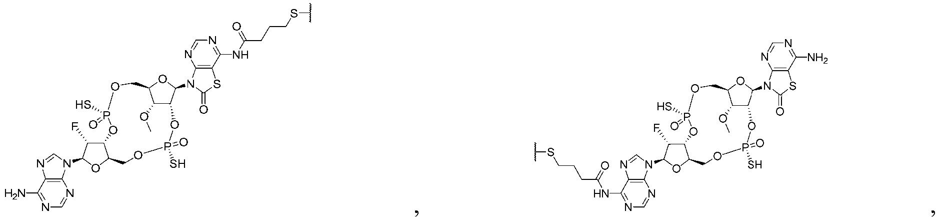 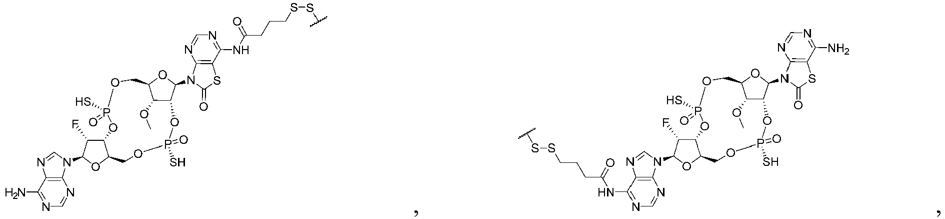 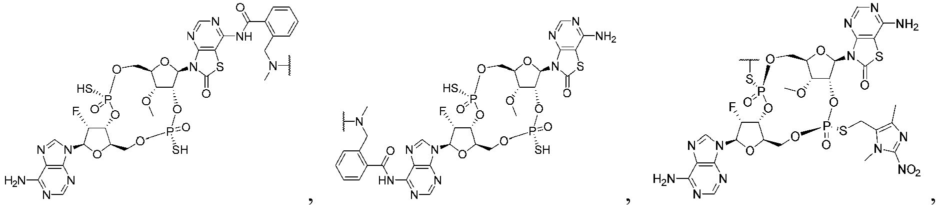 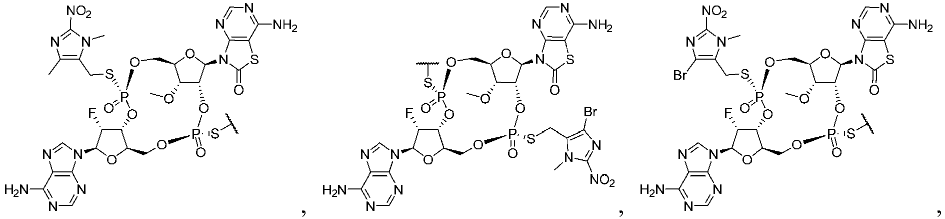 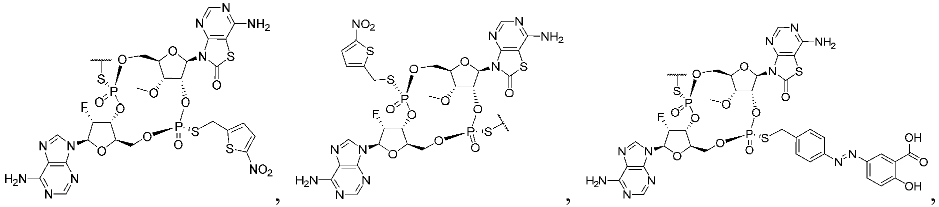 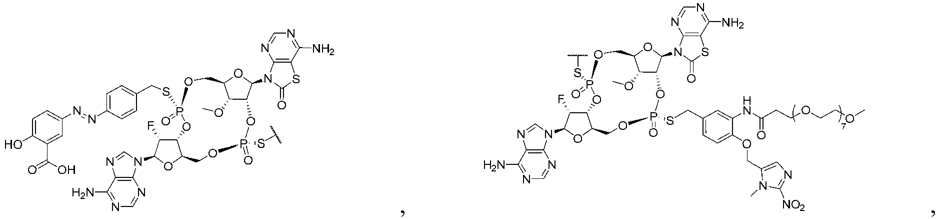 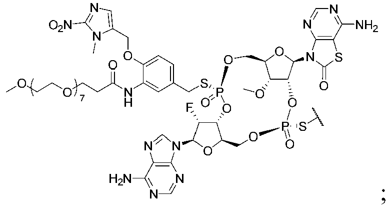 or a pharmaceutically acceptable salt thereof.

In some embodiments, D is any one of the following structures: 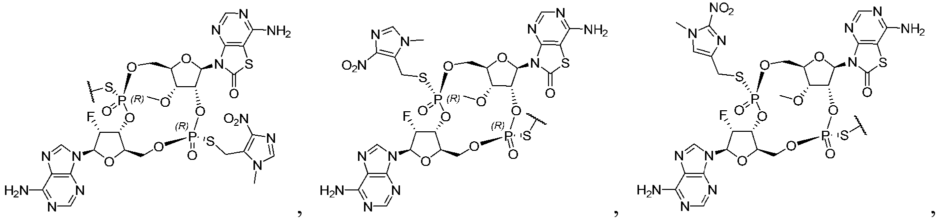 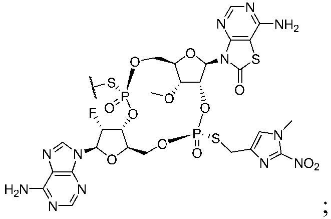 or a pharmaceutically acceptable salt thereof.

In some embodiments, in the cyclic dinucleotide derivative represented by Formula I or the pharmaceutically acceptable salt thereof, the cyclic dinucleotide derivative is selected from: 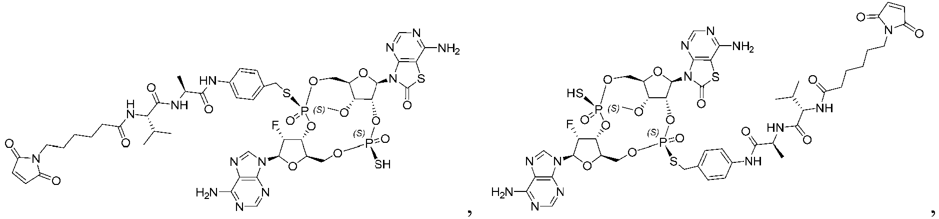 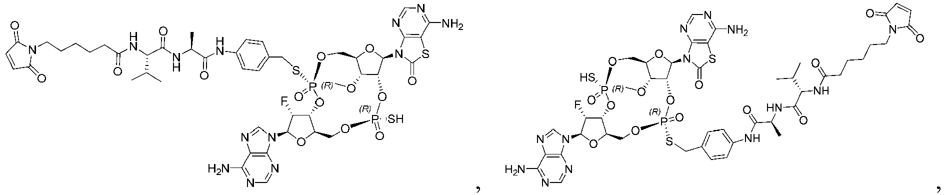 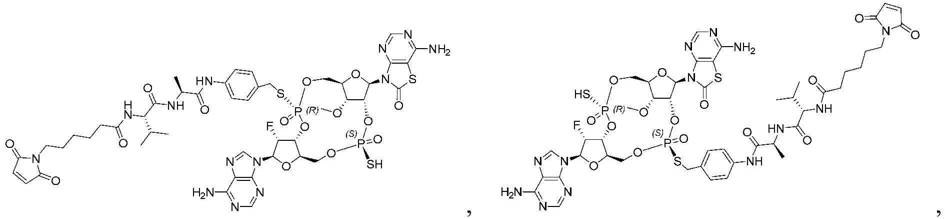 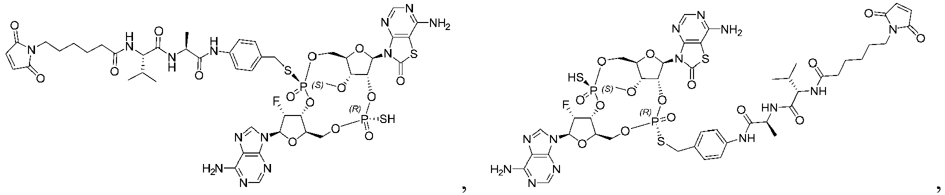 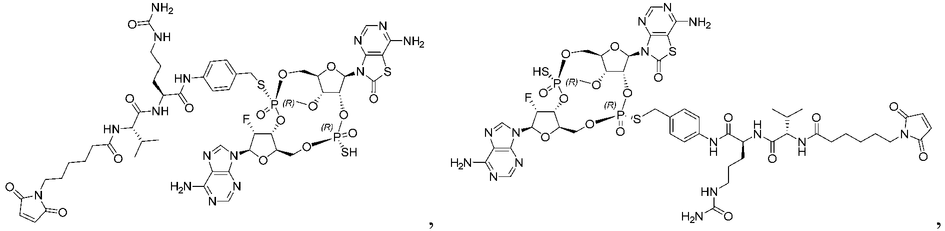 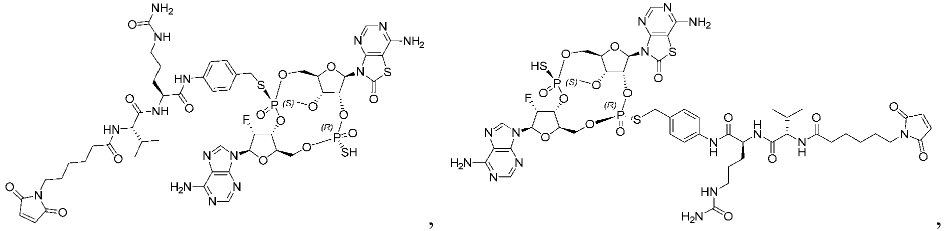 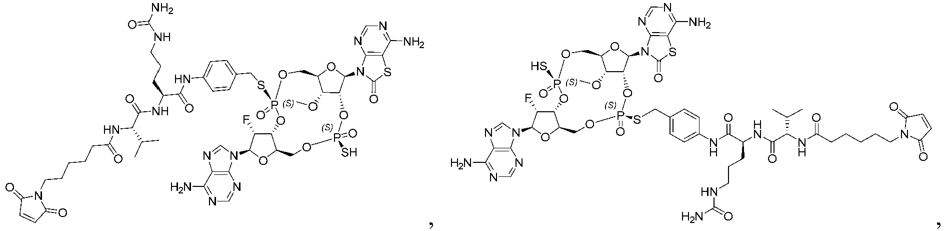 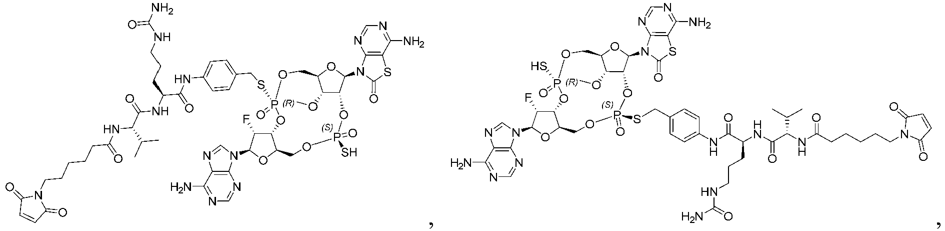 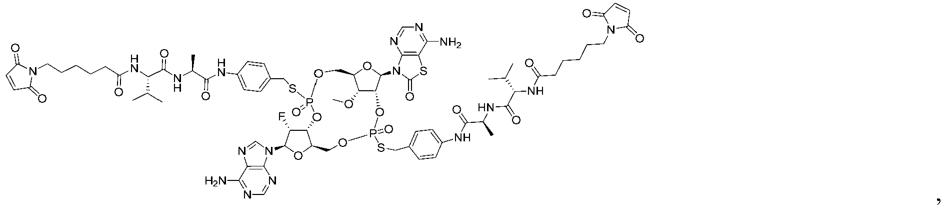 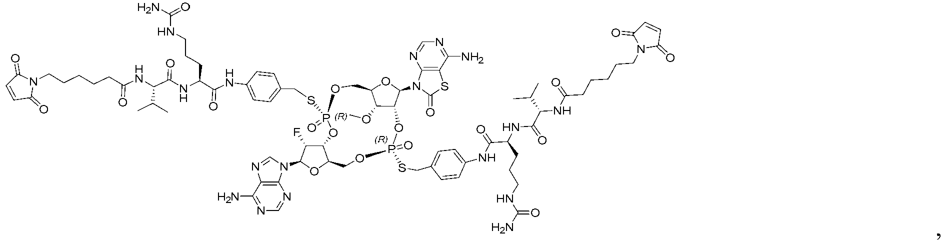 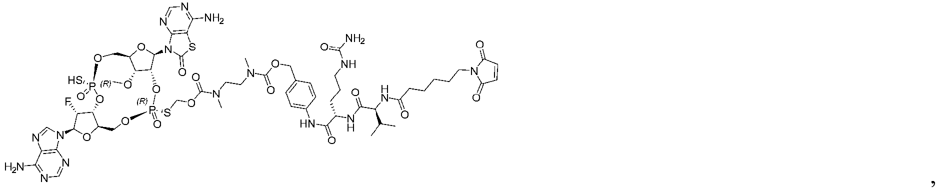 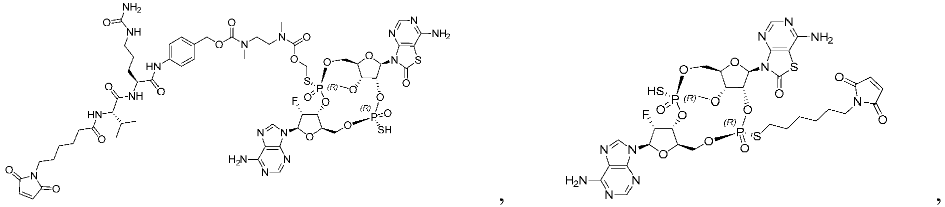 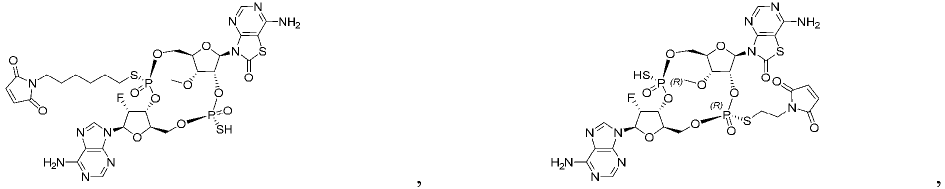 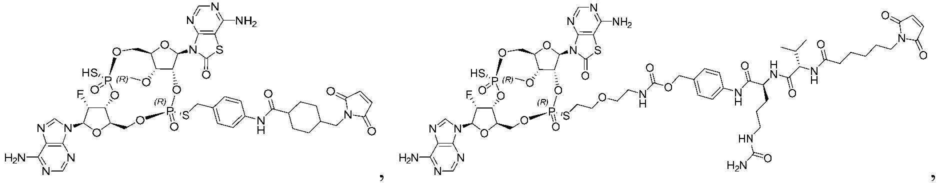 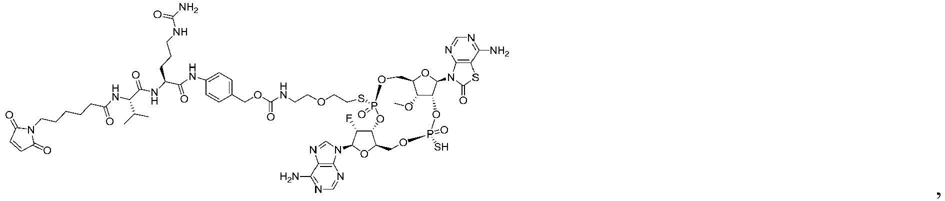 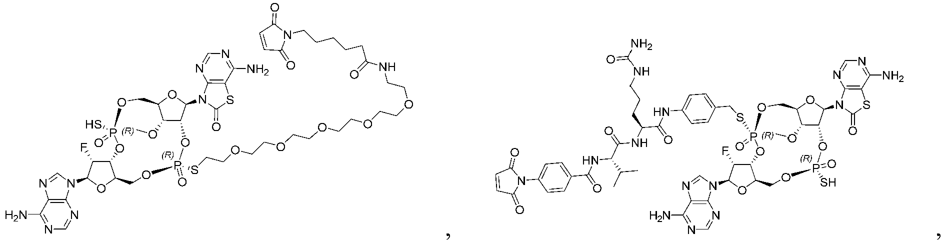 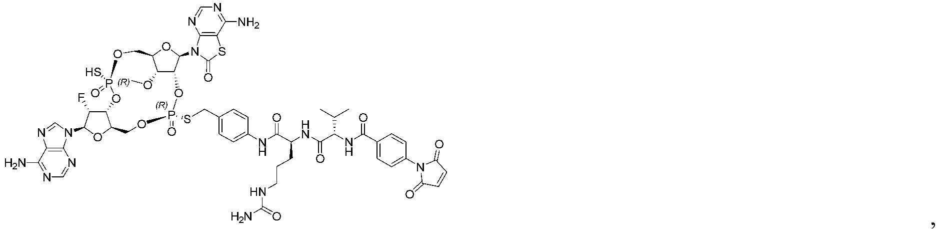 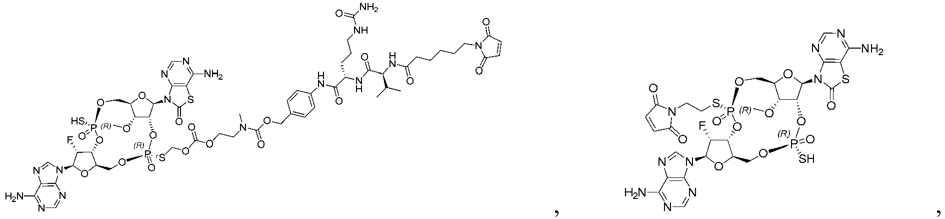 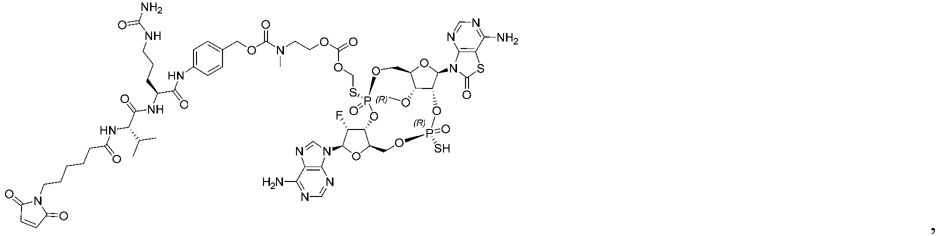 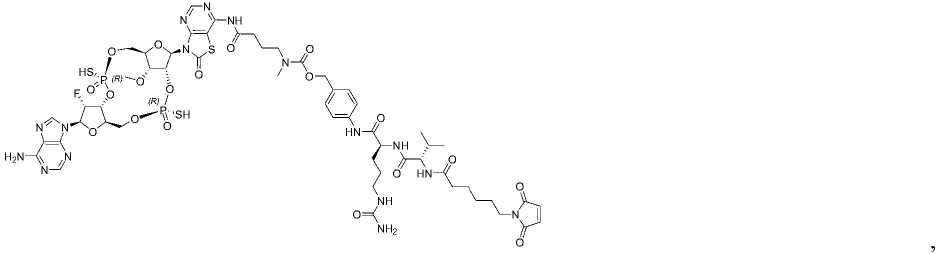 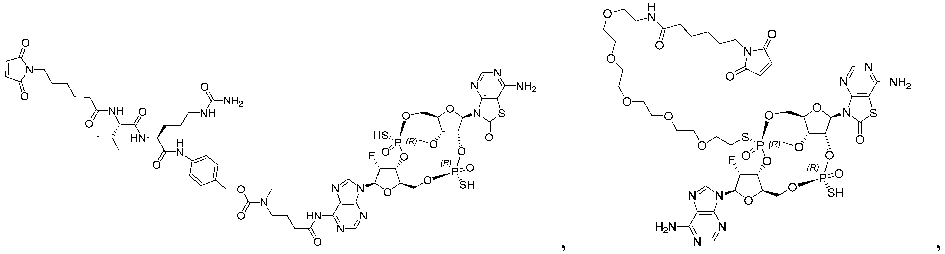 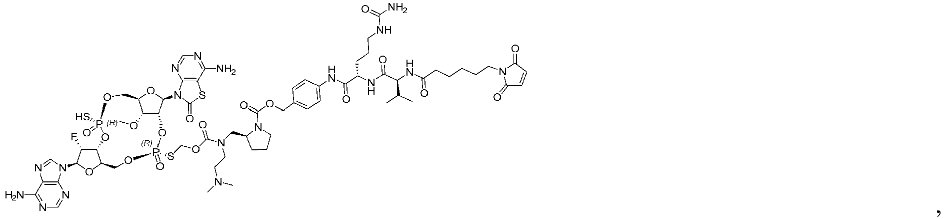 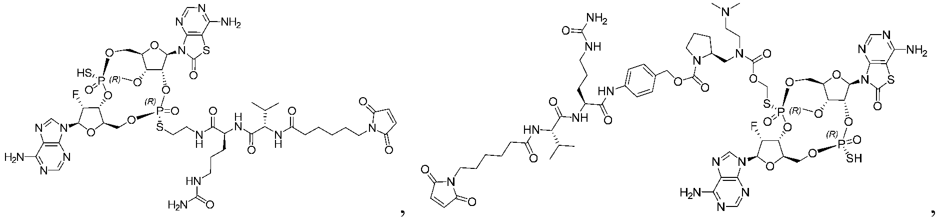 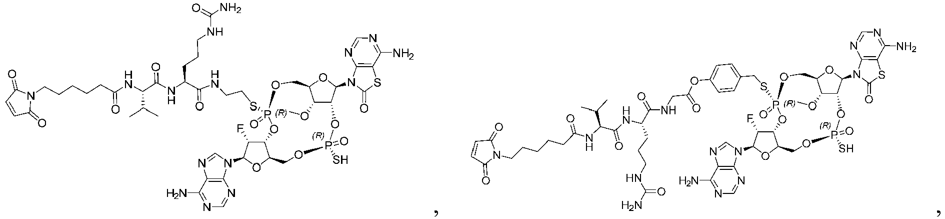 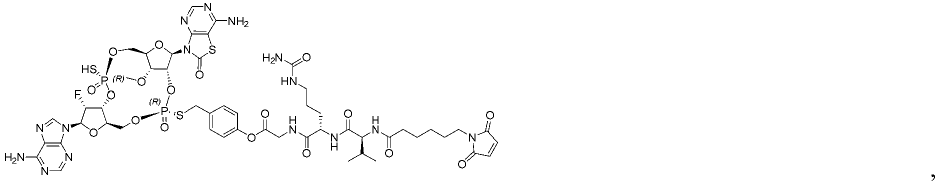 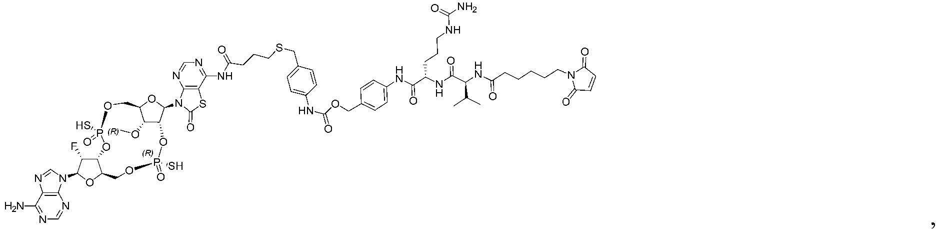 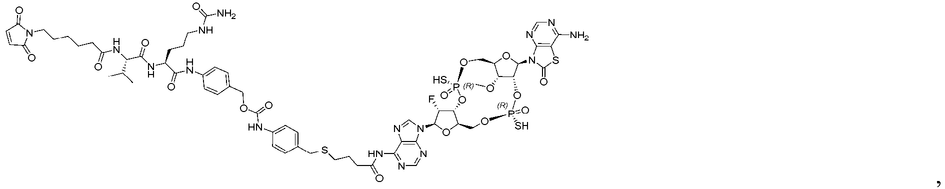 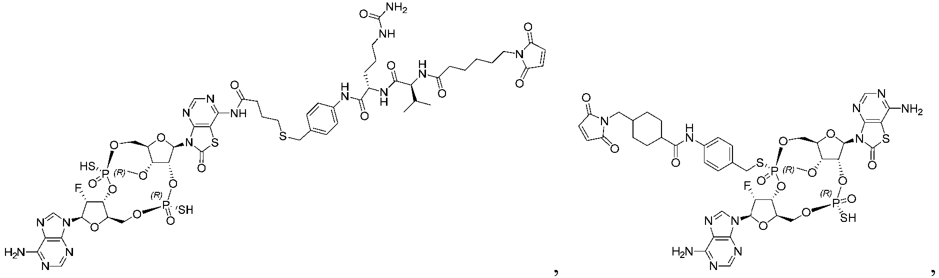 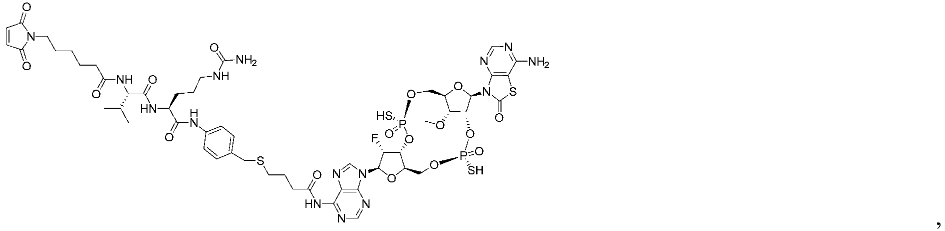 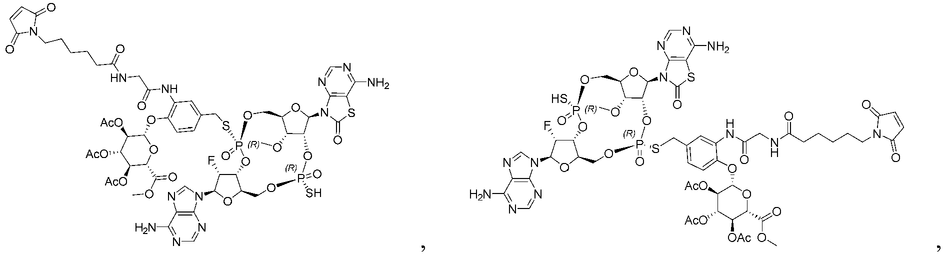 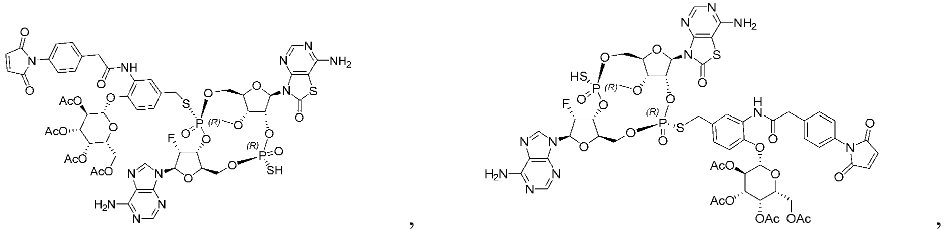 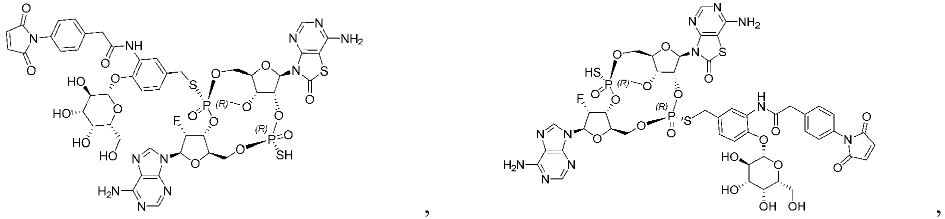 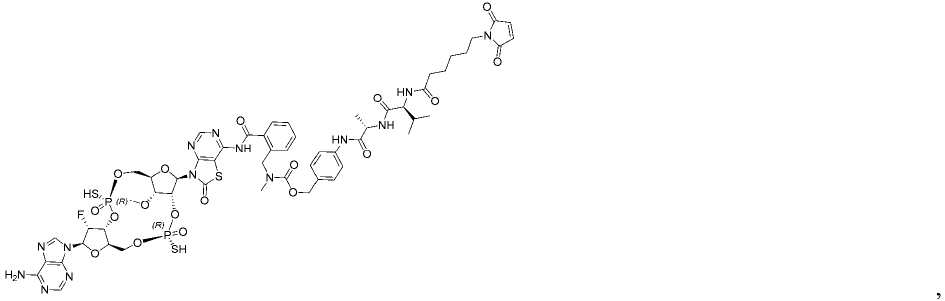 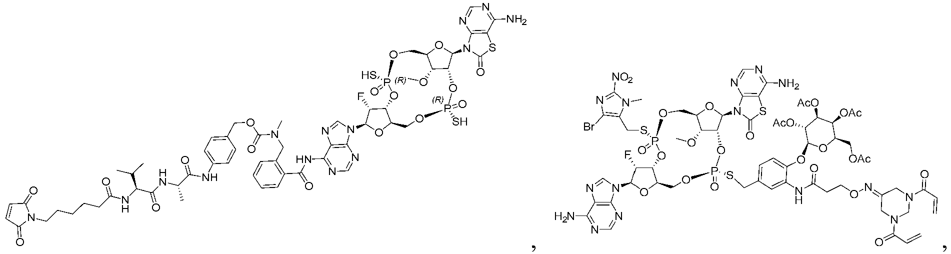 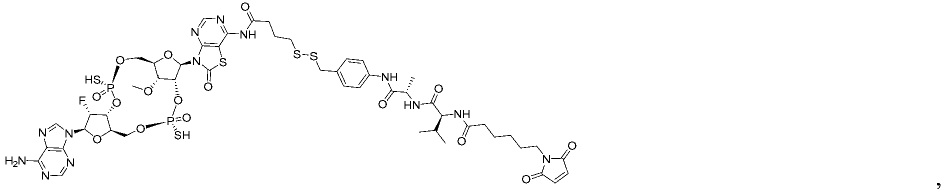 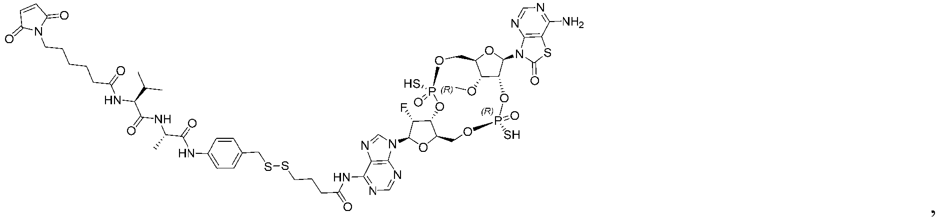 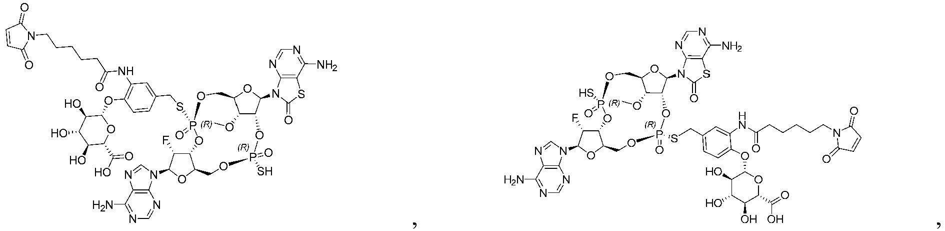 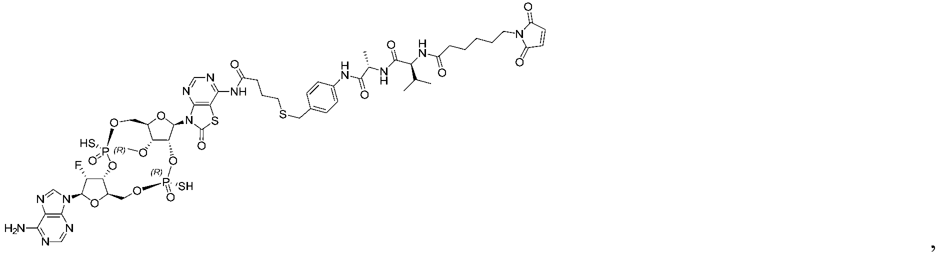 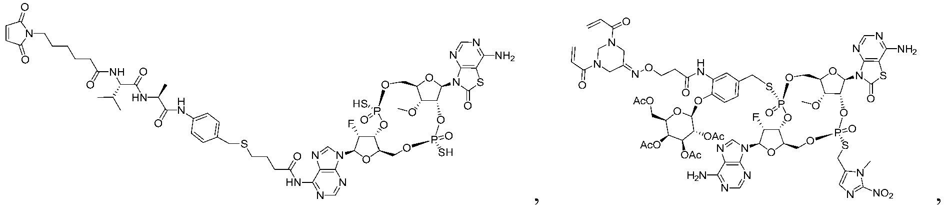 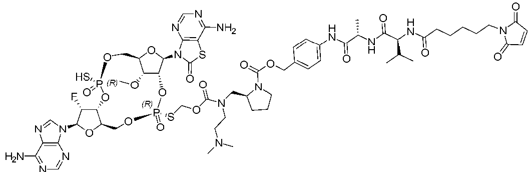 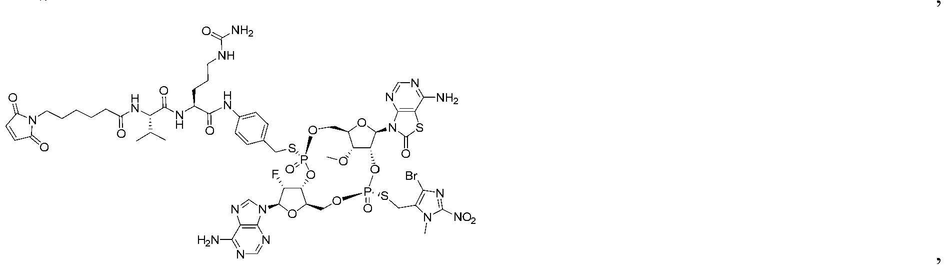 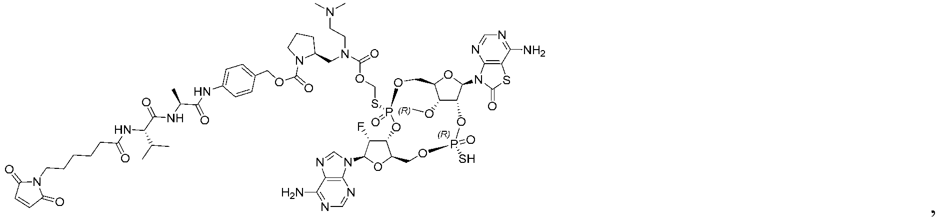 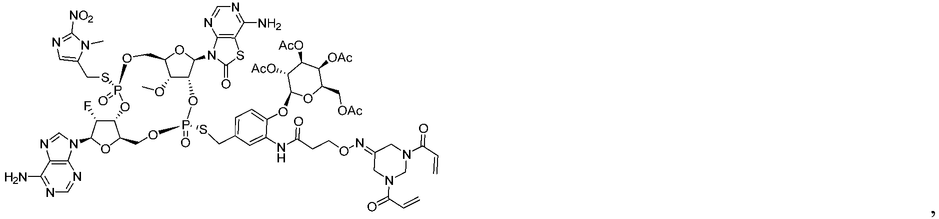 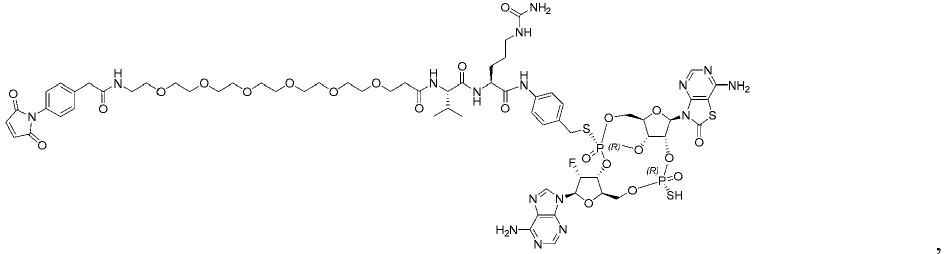 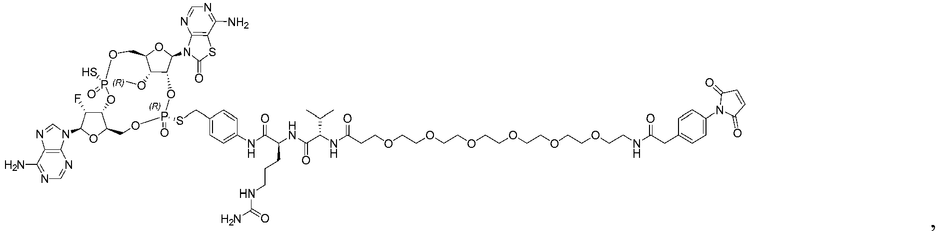 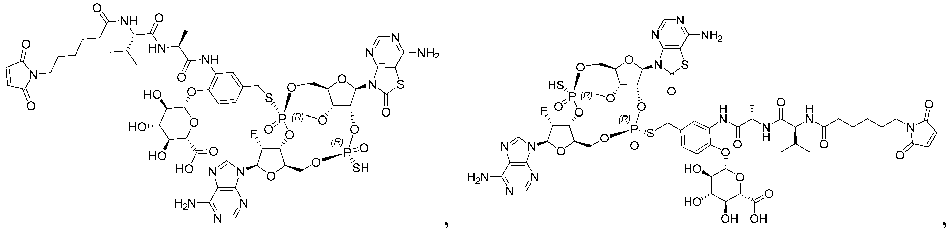 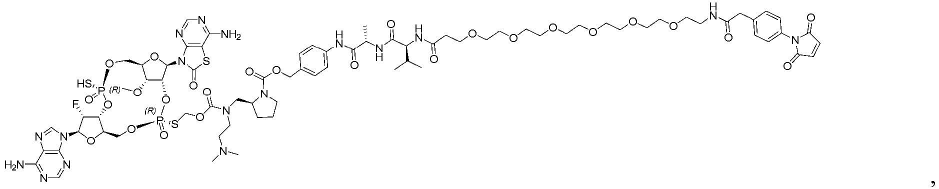 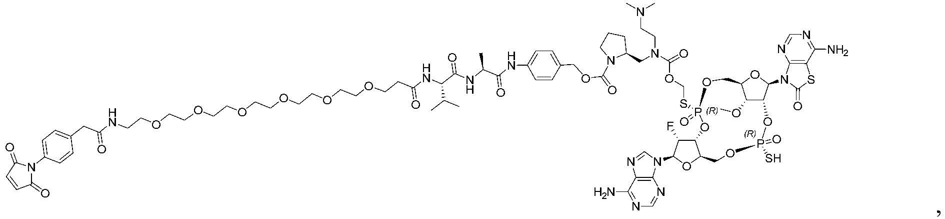 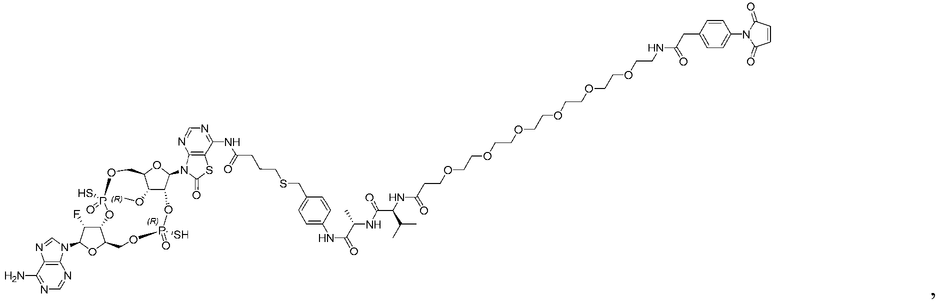 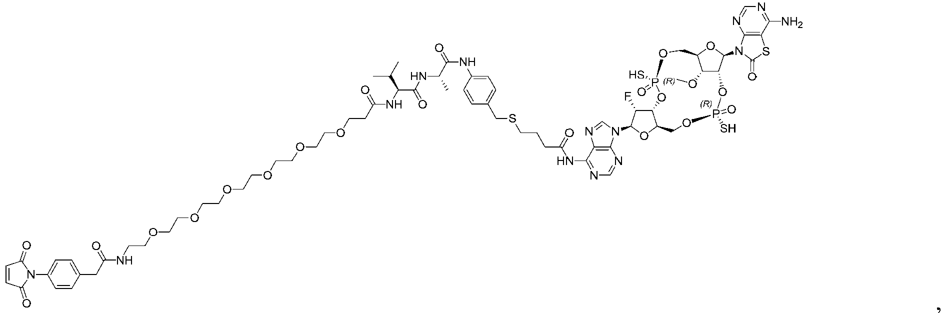 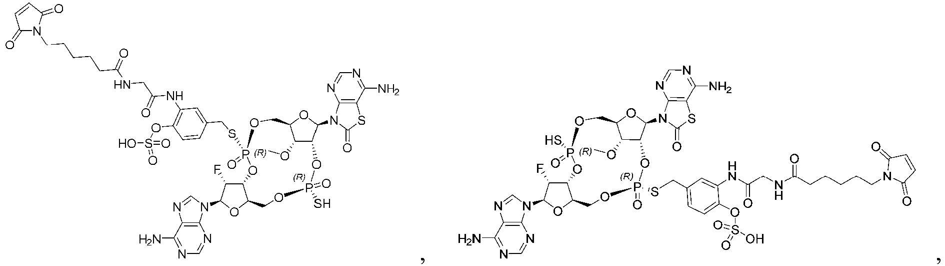 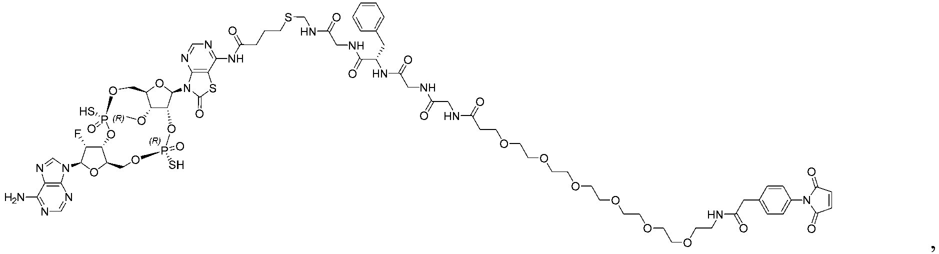 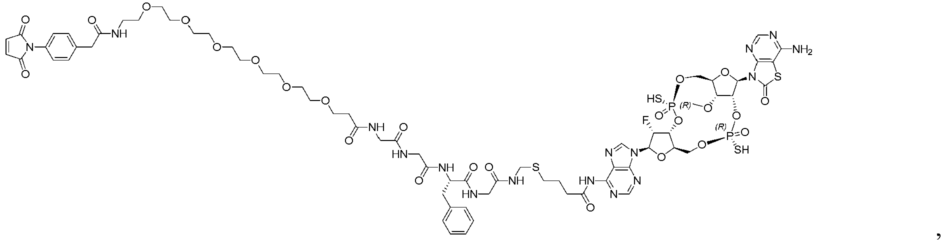 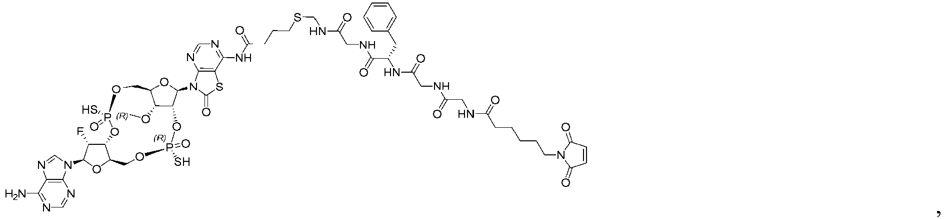 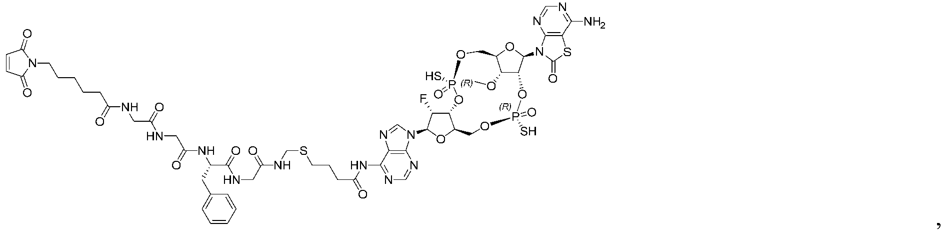 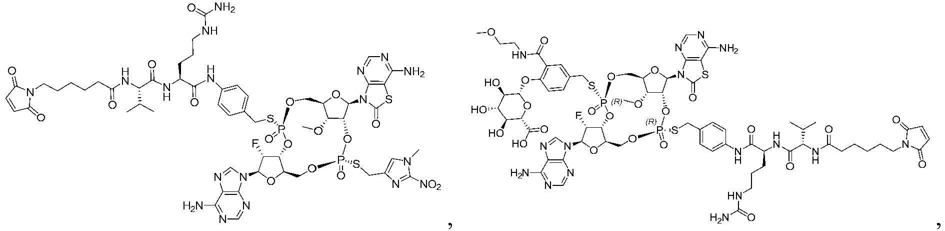 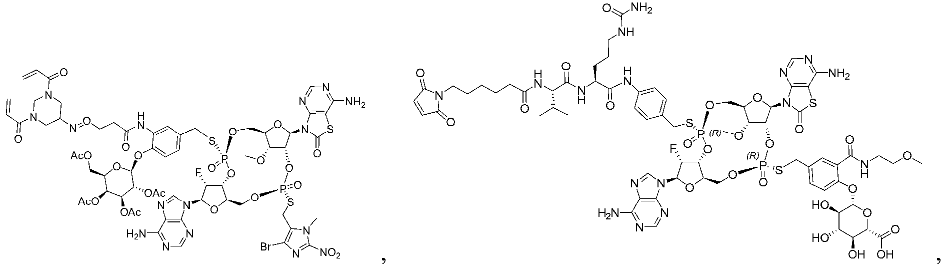 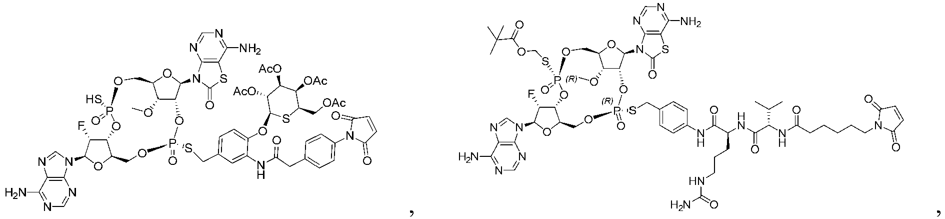 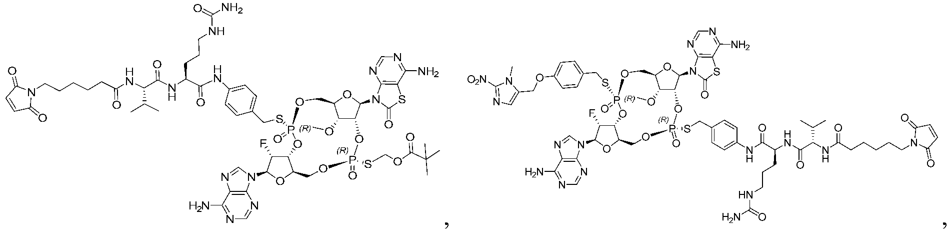 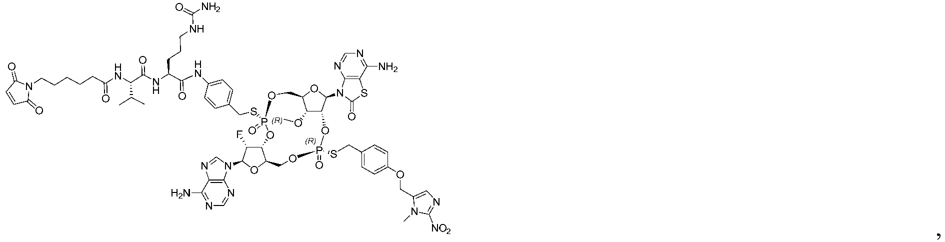 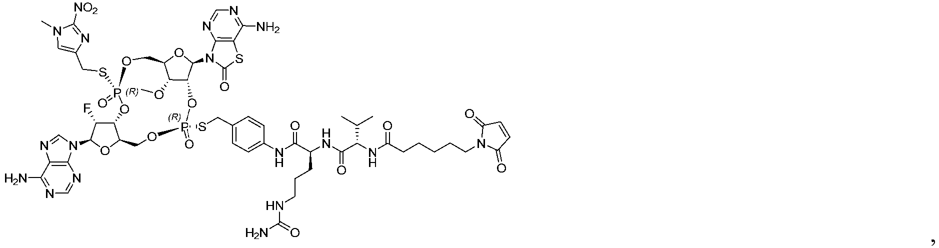 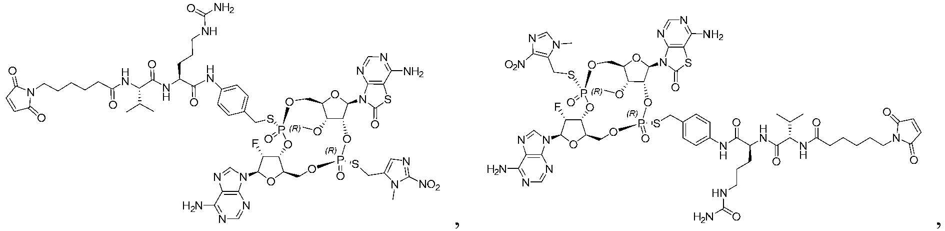 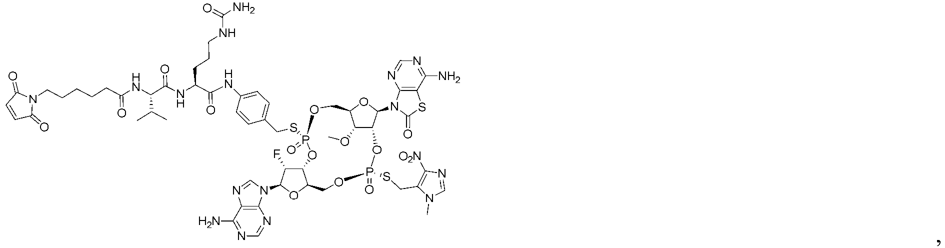 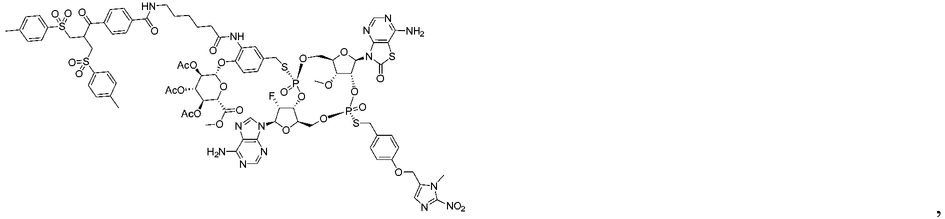 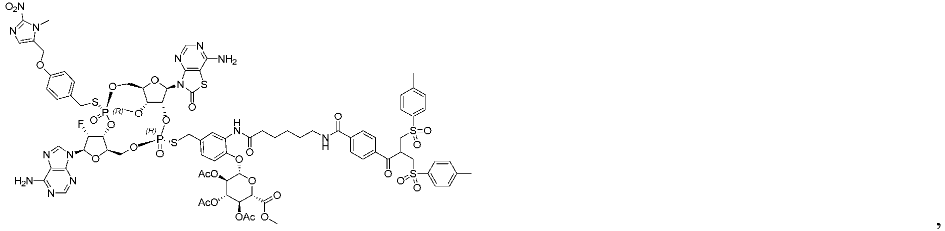 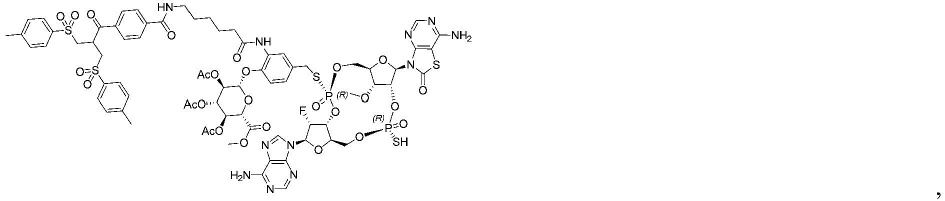 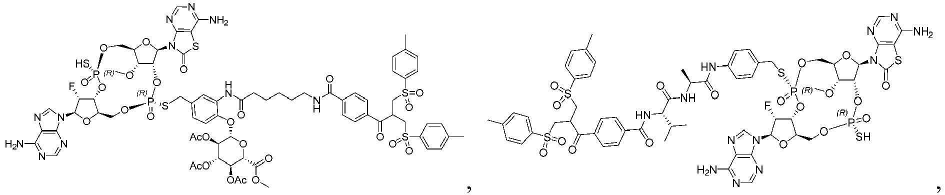 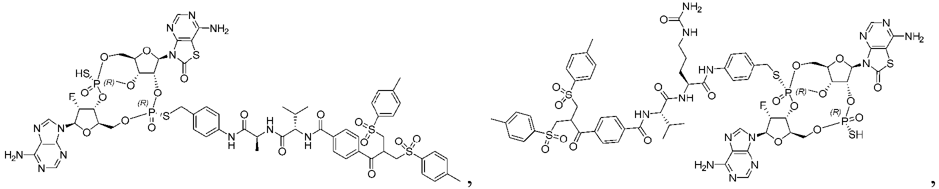 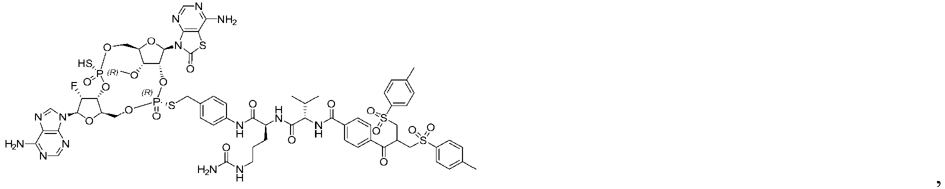 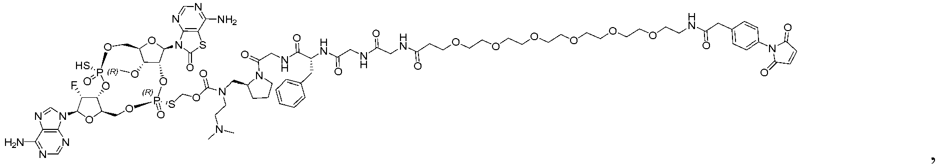 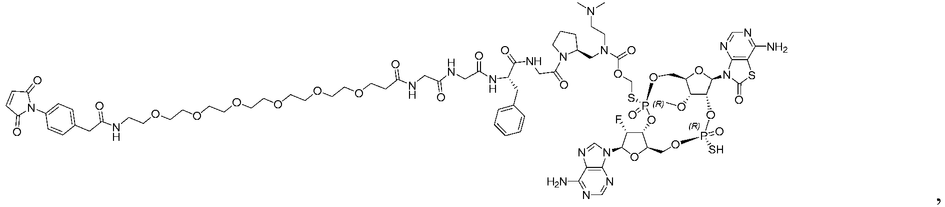 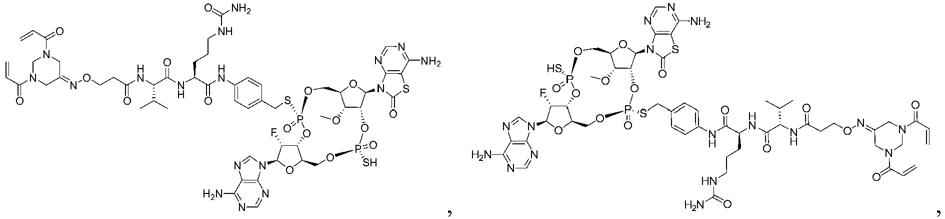 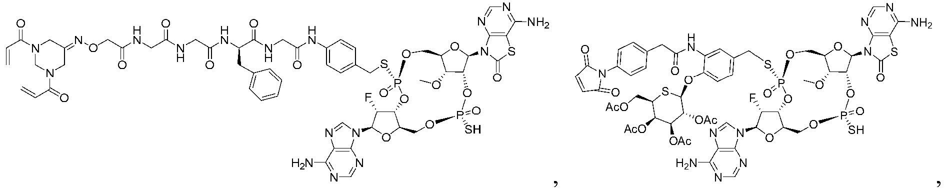 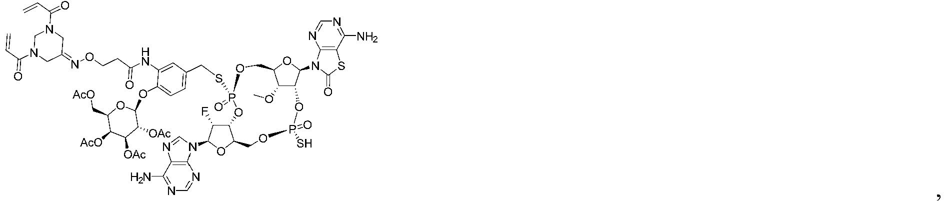 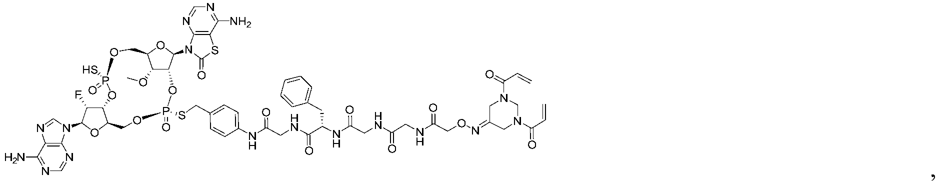 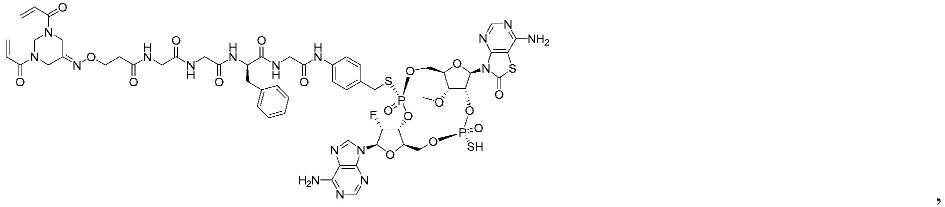 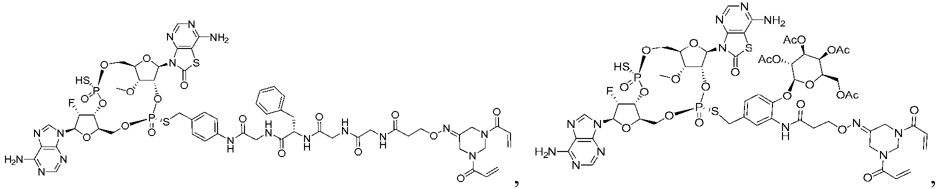 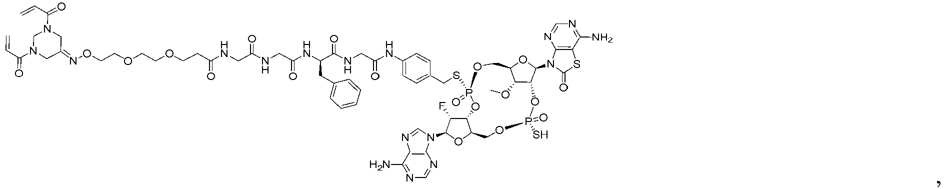 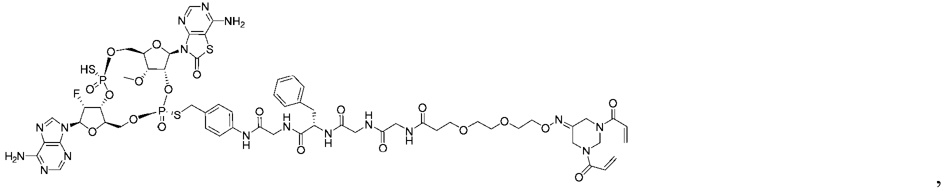 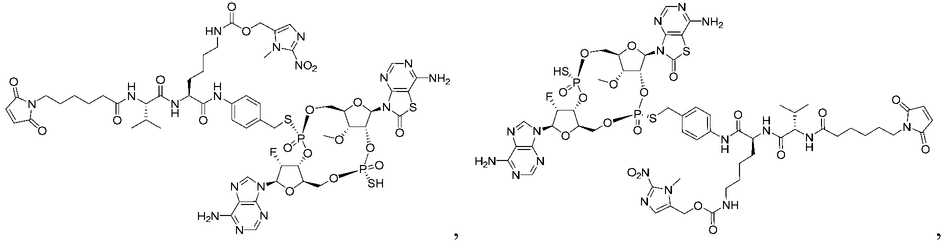 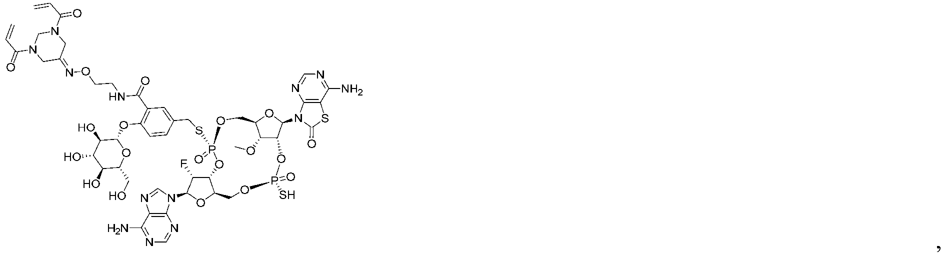 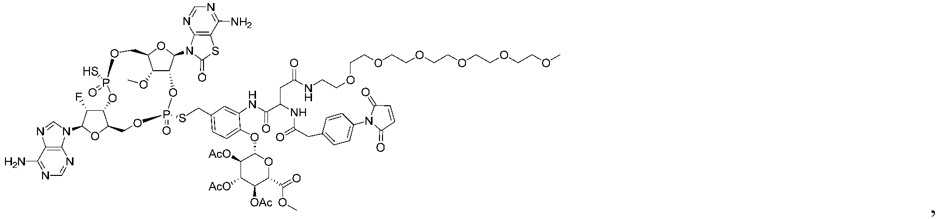 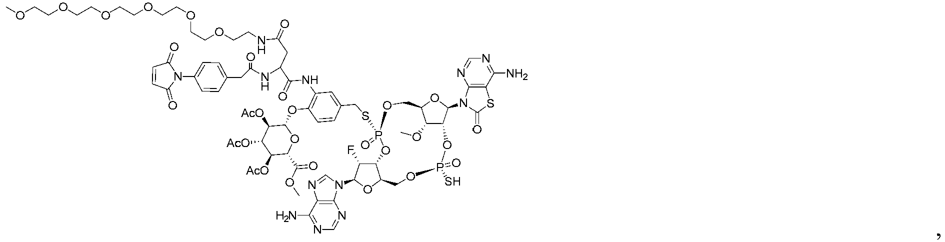 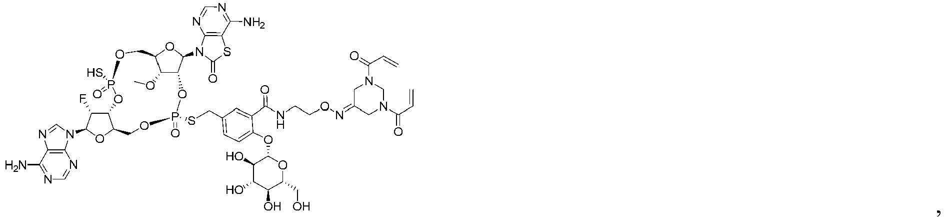 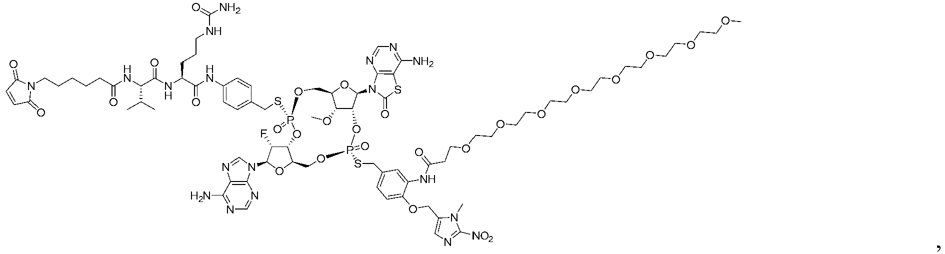 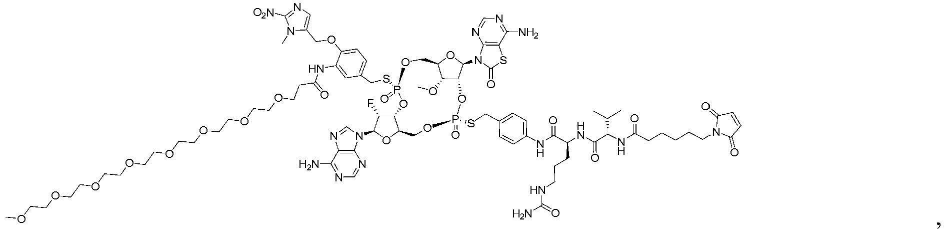 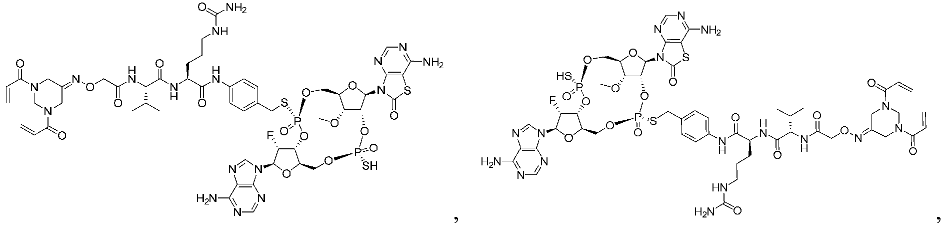 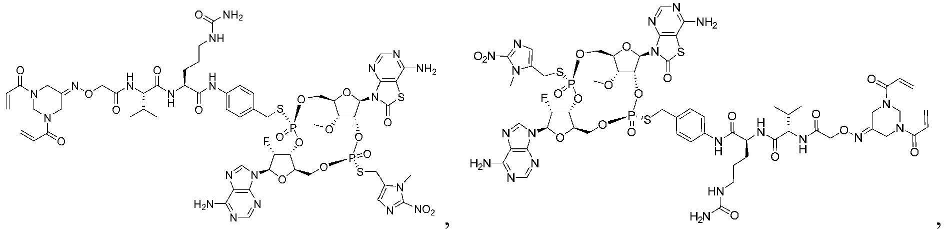 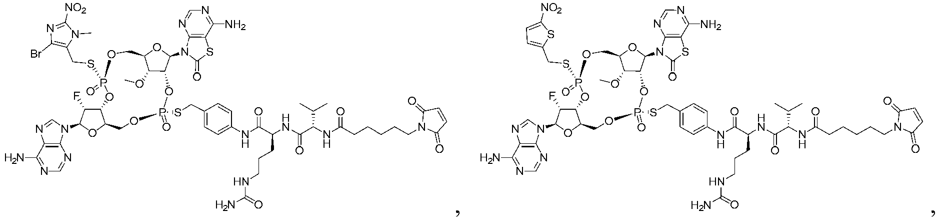 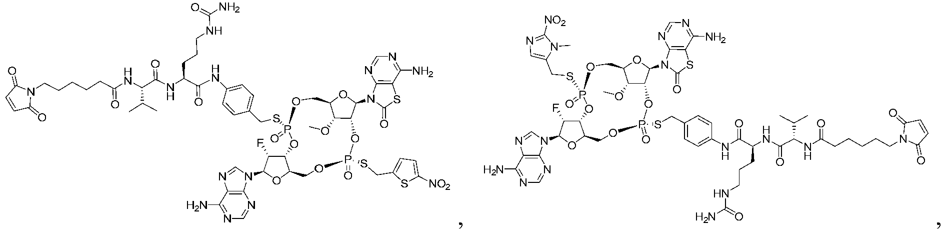 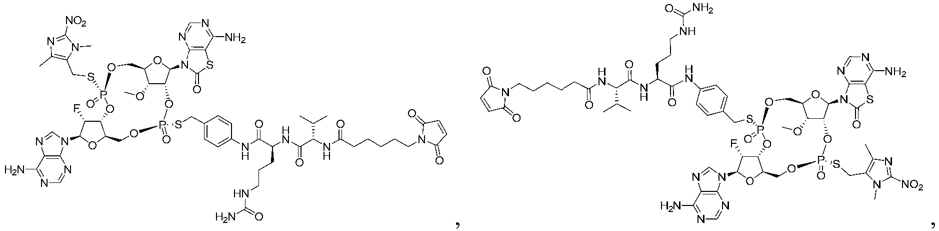 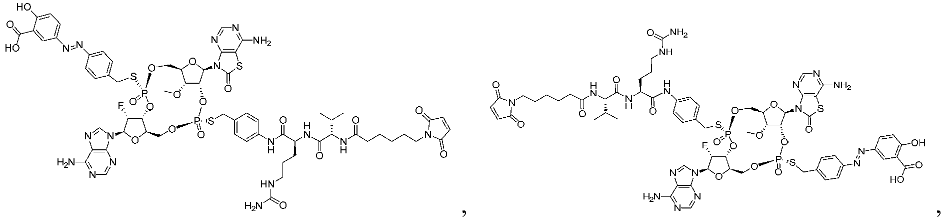 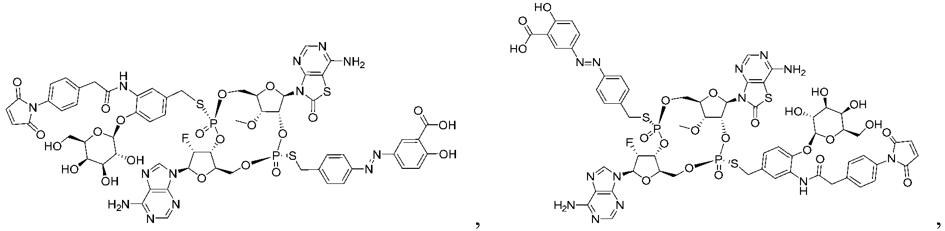 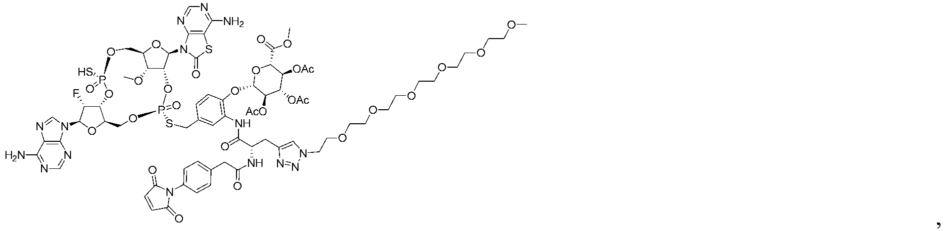 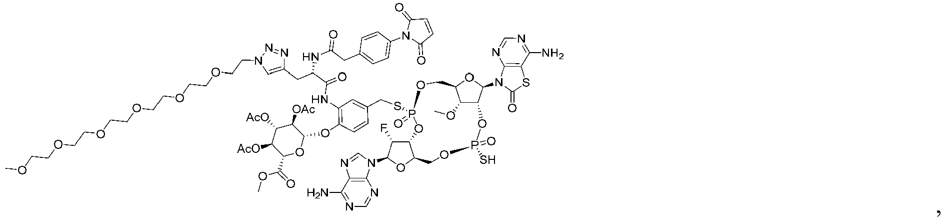 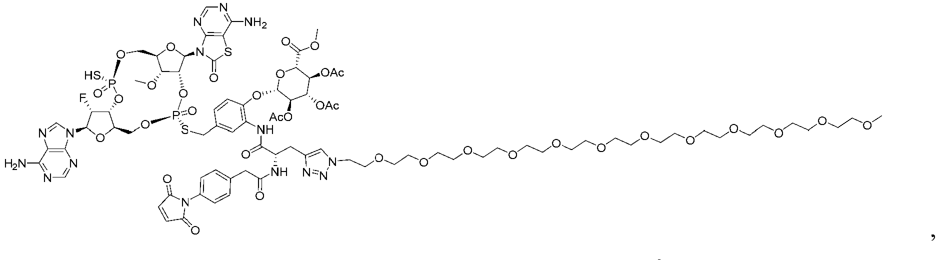 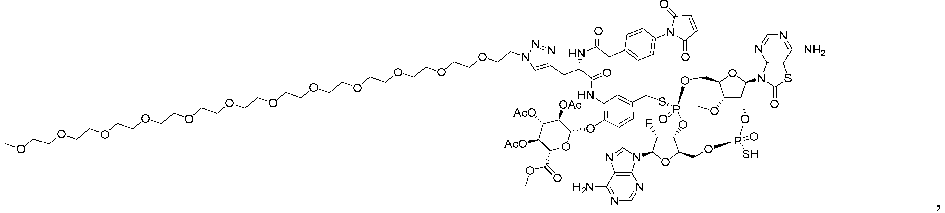 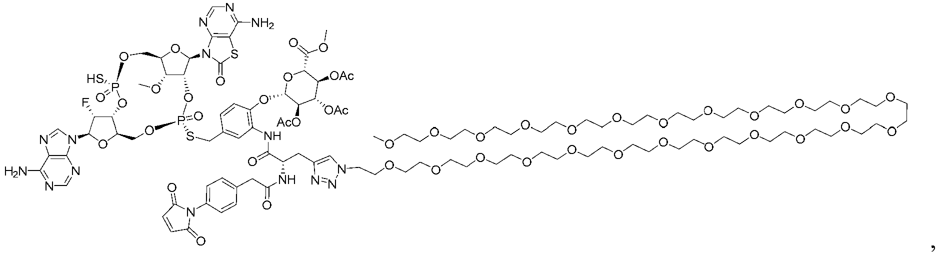 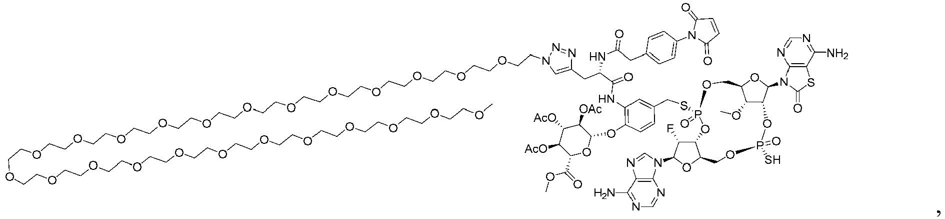 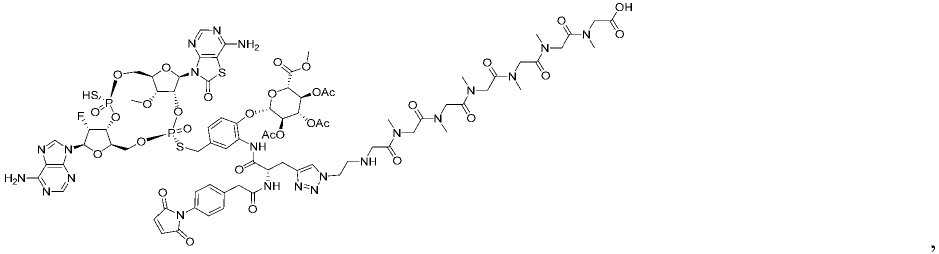 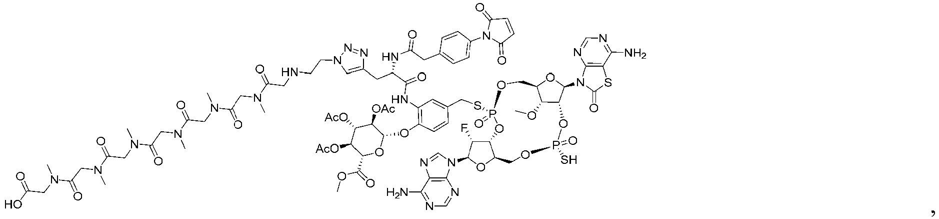 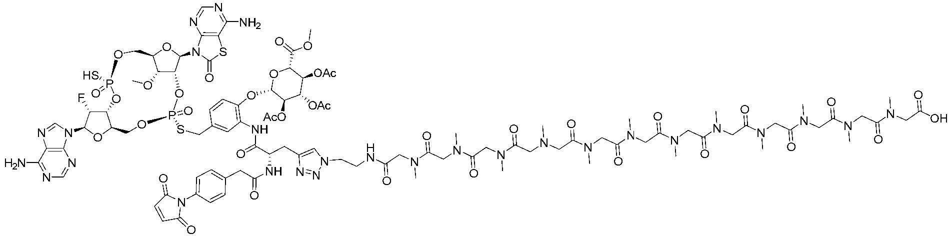 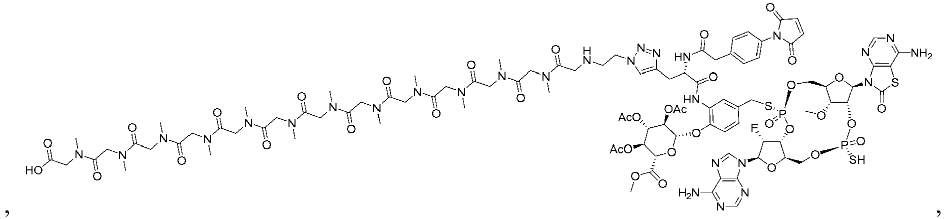 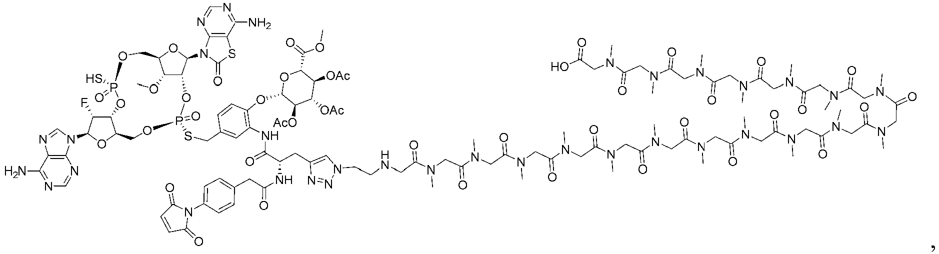 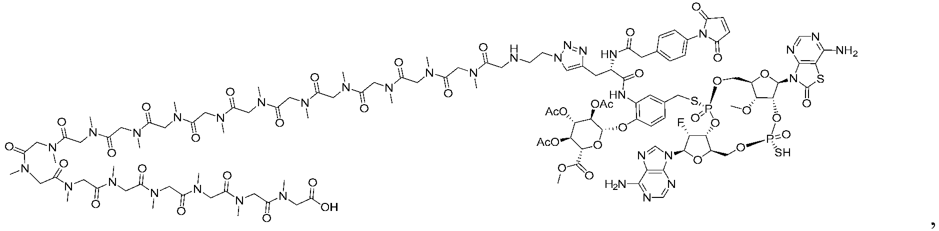 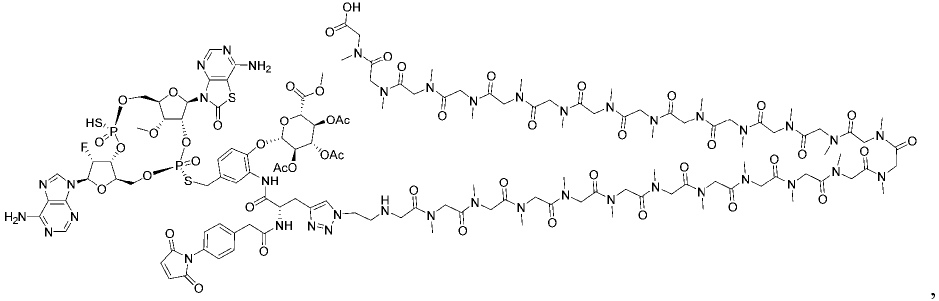 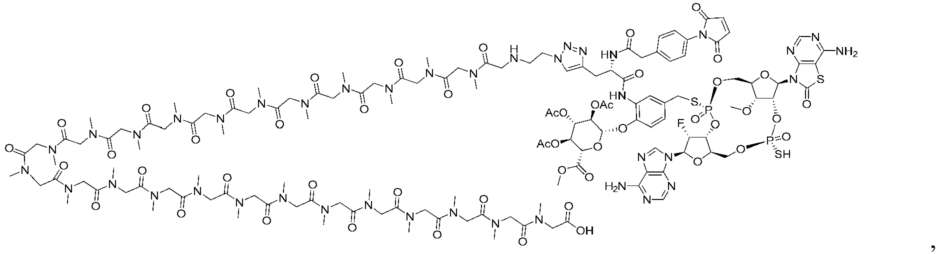 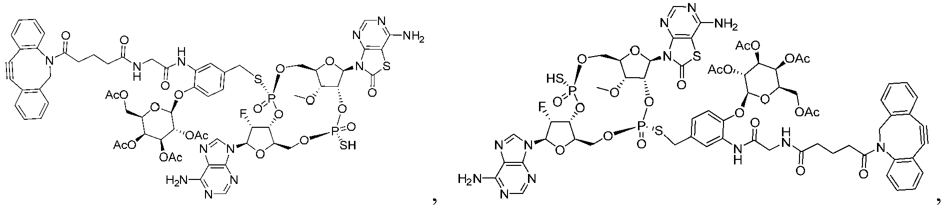 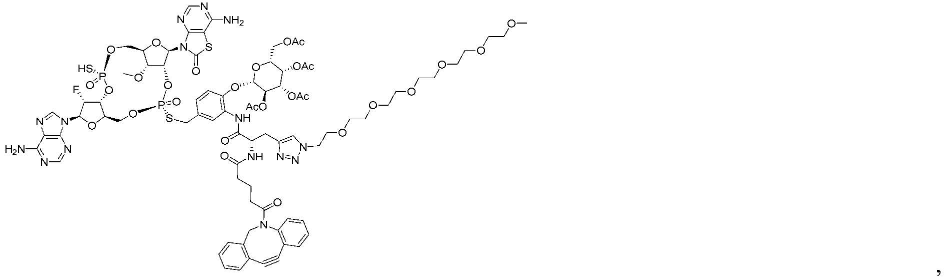 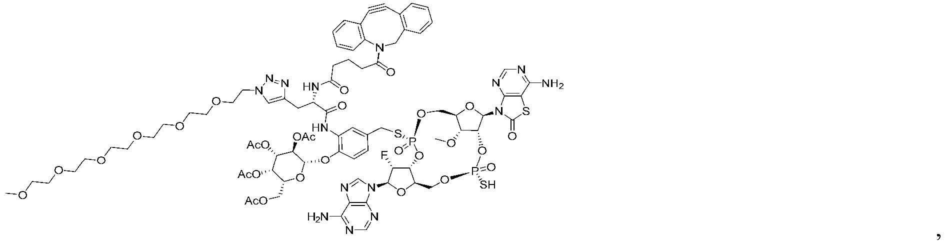 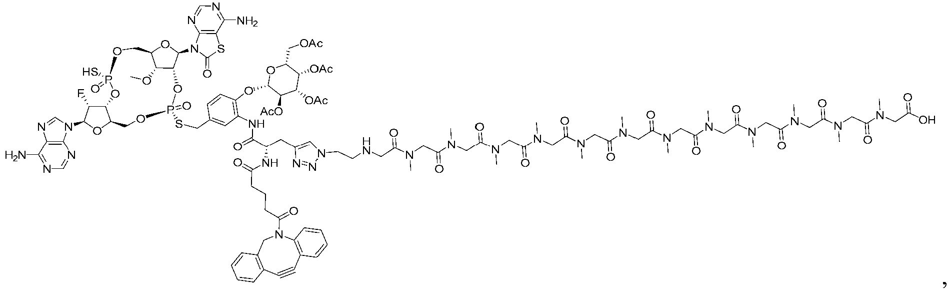 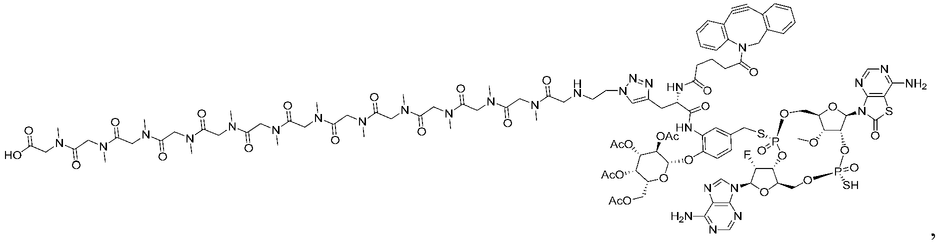 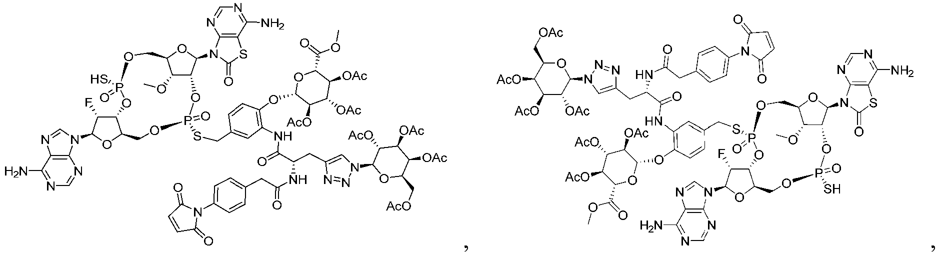 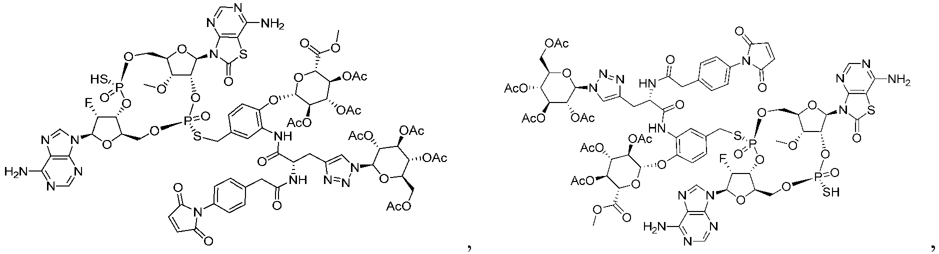 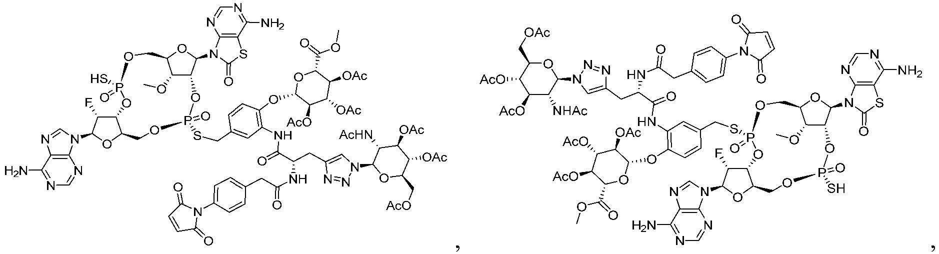 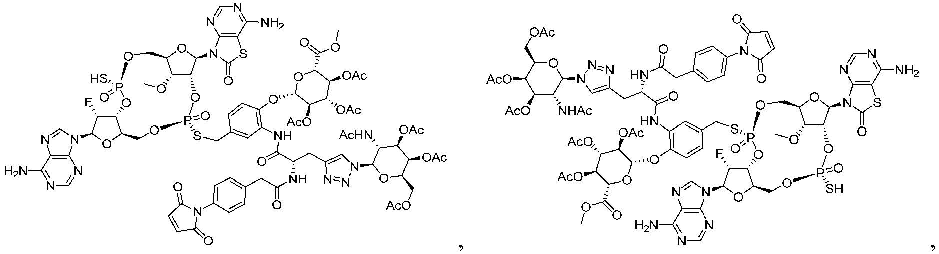 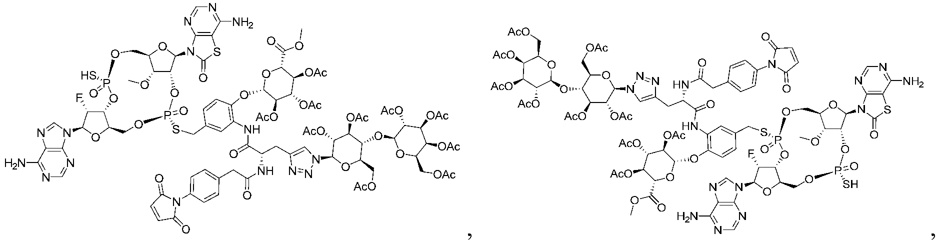 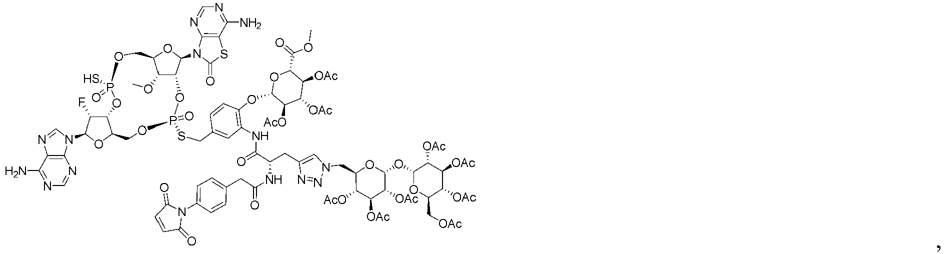 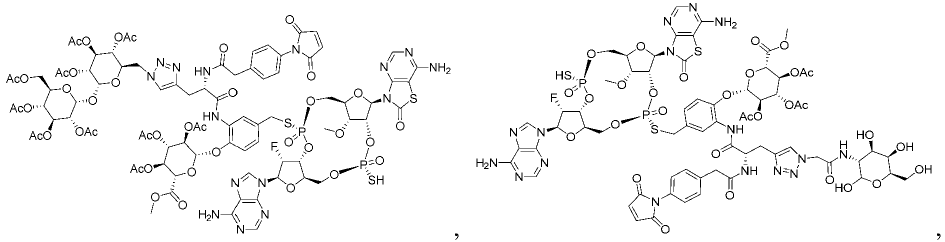 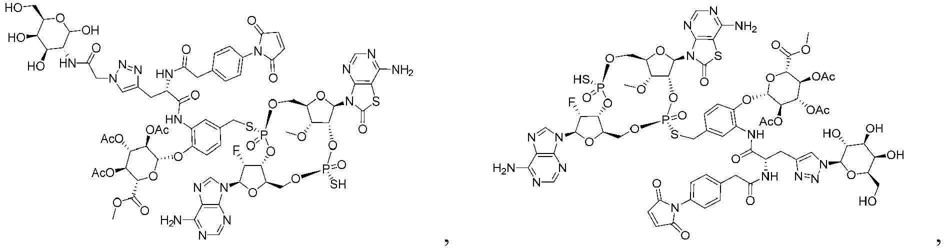 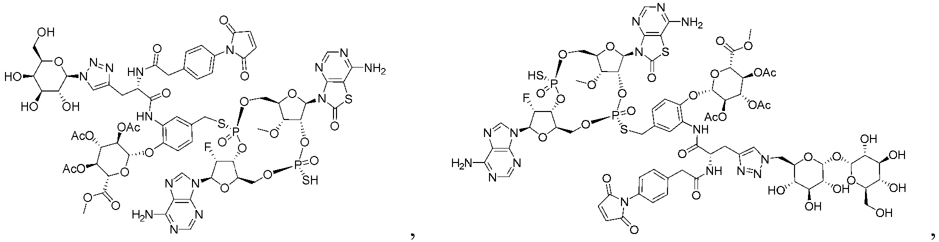 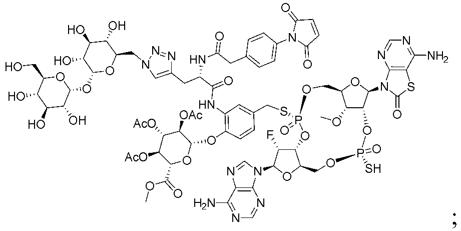 or a pharmaceutically acceptable salt thereof.

The present disclosure also provides a compound represented by Formula D'-1 or D'-2, a stereoisomer thereof, or a pharmaceutically acceptable salt thereof; 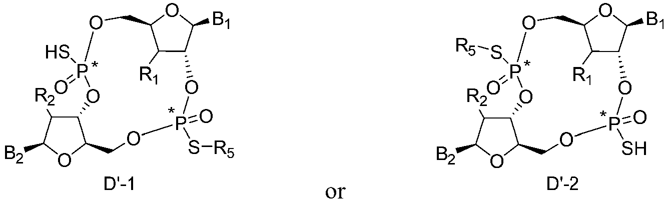
B₁ and B₂ are each independently 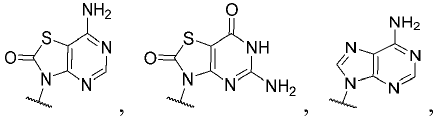 or 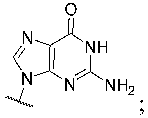 and at least one of B₁ or B₂ is 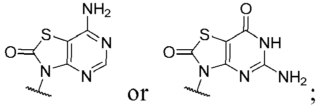 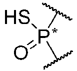
is 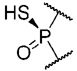 and/or 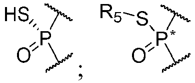 is 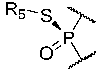 and/or 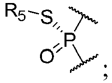
R₁ and R₂ are independently -OH, -N₃, F, -OCH₃, or -SCF₃;
R₅ is -(L₂)_{d}-(Z)ₑ-R;
d is 0, 1, 2, 3, 4, 5, or 6; e is 0, 1, 2, 3, 4, 5, 6, 7, 8, 9, or 10;
each L₂ is independently 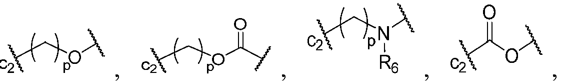 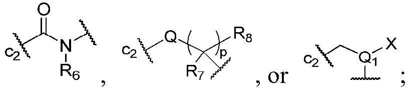 the c₂ side is connected to D;
X is 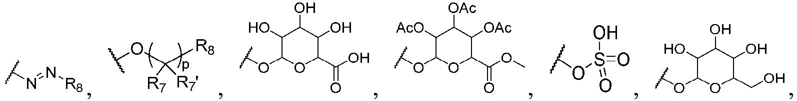 or 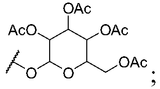
p is independently 1, 2, or 3;
Q is a linking bond, -C(O)O-, -C(O)N(R₆)-, or -C₁₋₃ alkylene-O-;
Q₁ is phenyl or pyridyl;
R₆ is H or C₁₋₆ alkyl; the C₁₋₆ alkyl is unsubstituted or optionally substituted at any position with one C₁₋₆ alkylamino or C₁₋₆ alkylsulfonyl;
R₇ and R_{7'} are each independently H or C₁₋₆ alkyl;
R₈ is phenyl or 5- to 10-membered heteroaryl; the R₈ is unsubstituted or optionally substituted at any position with 1-3 substituents selected from hydroxyl, amino, carboxyl, cyano, nitro, thiol, C₁₋₆ alkyl, C₃₋₈ cycloalkyl, halogen, halo-C₁₋₆ alkyl, C₁₋₆ alkylamino, C₁₋₆ alkoxy, halo-C₁₋₆ alkoxy, C₁₋₆ alkylthio, amido, hydroxyamino, aldehyde, acetyl, methoxycarbonyl, amino-C₁₋₆ alkyl, hydroxy-C₁₋₆ alkyl, and C₁₋₆ alkylsulfonyl;
each Z is independently -C(O)-(CH₂)ₓ-, -NH-(CH₂)_{y}-, -O-(CH₂)_{y}-, C₁₋₆ alkylene, or C₂₋₆ alkenylene;
each x and each y is independently 0, 1, 2, 3, 4, 5, 6, 7, 8, 9, or 10.

In some embodiments, in the cyclic dinucleotide derivative represented by Formula D'-1 or D'-2 or the pharmaceutically acceptable salt thereof, some of the groups are defined as follows, and the rest of the groups are defined as described in any other embodiment (hereinafter referred to as "in some embodiments"): R₁ is -OCH₃; R₂ is F.

In some embodiments, d is 1.

In some embodiments, e is 0, 1, 2, 3, or 4; e is preferably 0, 1, 2, or 3.

In some embodiments, R₅ is -L₂-(Z)ₑ-H.

In some embodiments, R₅ is -L₂-(Z)ₑ-H; -L₂- is 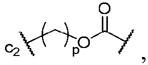 with the c₂ side connected to the thiol group in D; e is 1; -(Z)ₑ-H is C₁₋₆ alkyl.

In some embodiments, R₅ is -L₂-(Z)ₑ-H; -L₂- is 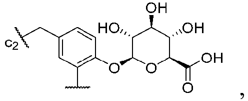 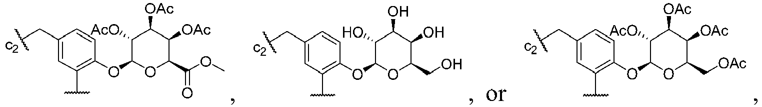 with the c₂ side connected to the thiol group in D; e is 3, -(Z)ₑ-H is -C(O)NH-(CH₂)_{y}-O-(CH₂)_{y}-H.

In some embodiments, R₅ is -L₂-(Z)ₑ-H; -L₂- is 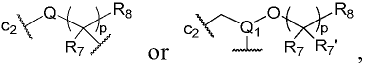 with the c₂ side connected to the thiol group in D; e is 0 or 1, -(Z)ₑ-H is H or methyl.

In some embodiments, R₅ is 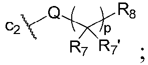 Q is a linking bond, -C(O)O-, or -C₁₋₃ alkylene-O-; R₇ and R_{7'} are each independently H or methyl.

In some embodiments, R₅ is 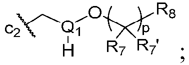 Q₁ is phenyl; R₇ and R_{7'} are each independently H or methyl.

In some embodiments, R₈ is phenyl or 5- to 6-membered heteroaryl; the 5- to 6-membered heteroaryl is preferably pyridyl, pyrimidinyl, pyrazinyl, pyrrolyl, thienyl, furyl, pyrazolyl, thiazolyl, or oxazolyl; R₈ is unsubstituted or optionally substituted at any position with 1 to 3 substituents selected from hydroxyl, amino, cyano, nitro, thiol, C₁₋₆ alkyl, halogen, halo-C₁₋₆ alkyl, C₁₋₆ alkylamino, C₁₋₆ alkoxy, halo-C₁₋₆ alkoxy, C₁₋₆ alkylthio, amido, aldehyde, acetyl, methoxycarbonyl, amino-C₁₋₆ alkyl, hydroxy-C₁₋₆ alkyl, and methylsulfonyl.

In some embodiments, R₅ is 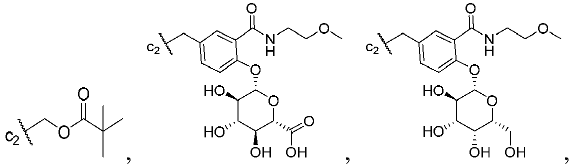 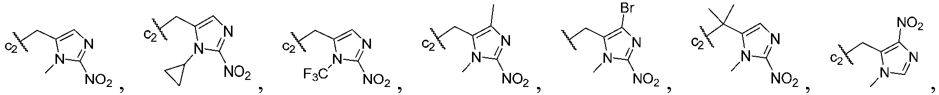 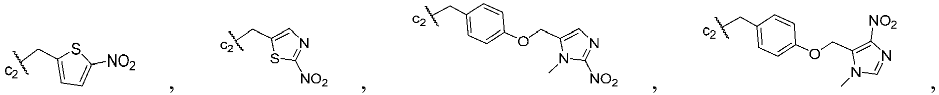 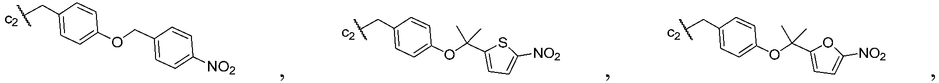 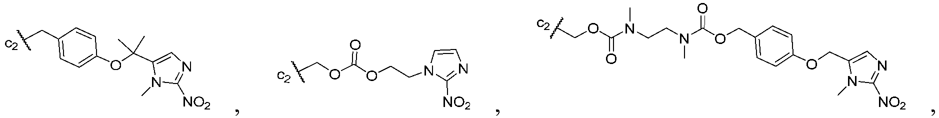 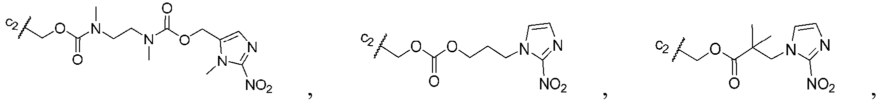 or 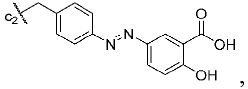 with the c₂ side connected to the thiol group in D.

In some embodiments, R₅ is 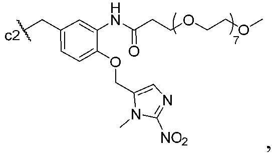 with the c₂ side connected to the thiol group in D.

In some embodiments, D'-1 or D'-2 is any one of the following structures: 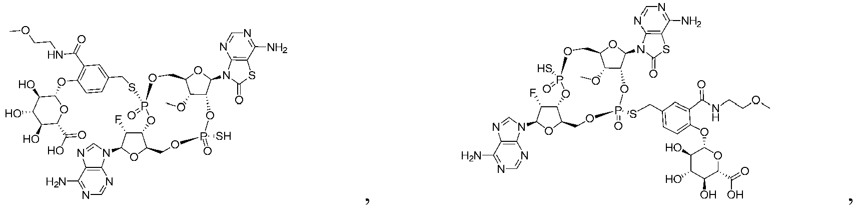 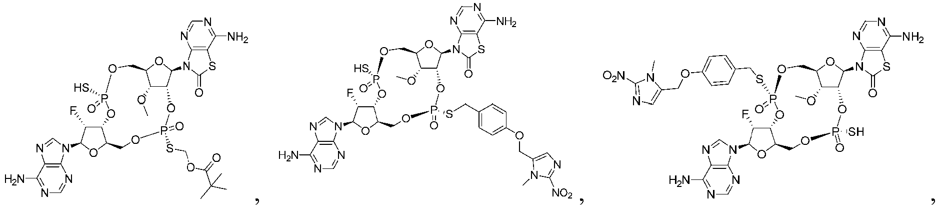 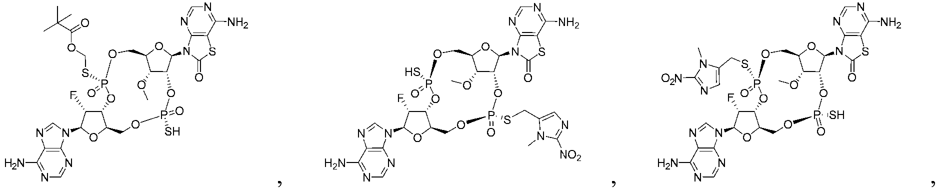 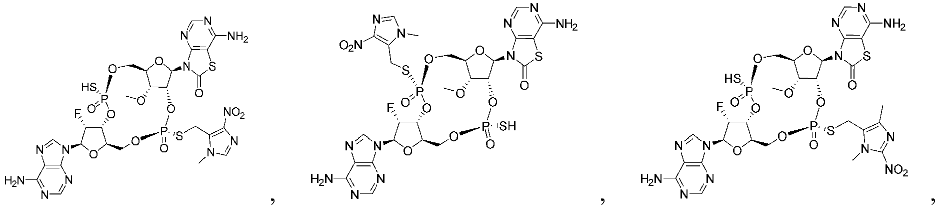 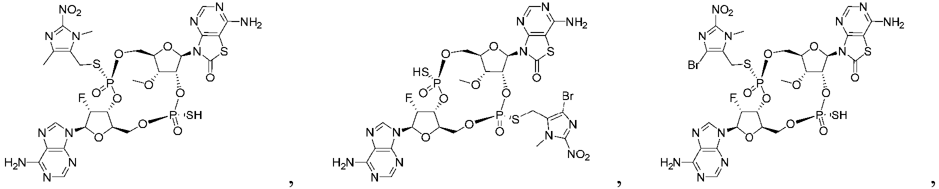 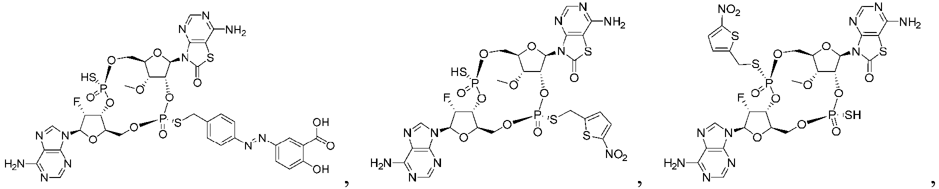 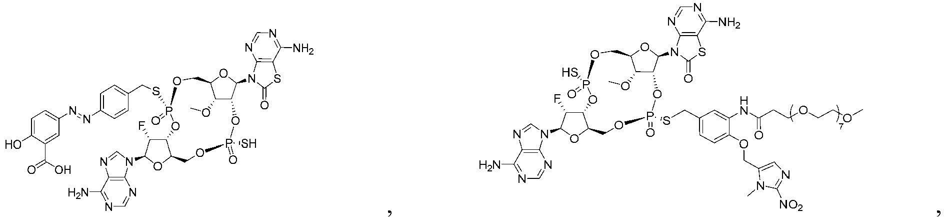 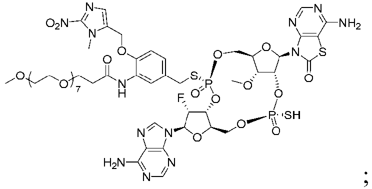 or a pharmaceutically acceptable salt thereof.

The present disclosure provides a pharmaceutical composition comprising substance K and a pharmaceutically acceptable excipient;
the substance K is substance K-1, substance K-2, or substance K-3;
the substance K-1 is the above immune-stimulating antibody conjugate represented by Formula II or the pharmaceutically acceptable salt thereof;
the substance K-2 is the above cyclic dinucleotide compound represented by Formula I or the pharmaceutically acceptable salt thereof;
the substance K-3 is the above compound represented by Formula D'-1 or D'-2, the stereoisomer thereof, or the pharmaceutically acceptable salt thereof.

In some embodiments, the amount of substance K may be a therapeutically effective amount.

In some embodiments, the amount of the immune-stimulating antibody conjugate represented by Formula II or the pharmaceutically acceptable salt thereof, the cyclic dinucleotide compound represented by Formula I or the pharmaceutically acceptable salt thereof, or the compound represented by Formula D'-1 or D'-2, the stereoisomer thereof, or the pharmaceutically acceptable salt thereof may be a therapeutically effective amount.

In the pharmaceutical composition, the pharmaceutically acceptable excipient may comprise a pharmaceutically acceptable carrier, diluent, and/or formulating agent.

The pharmaceutical composition can be administered by conventional routes, comprising (but not limited to): intramuscular, intraperitoneal, intravenous, subcutaneous, intradermal, local administration (such as intratumoral injection), *etc.*

The present disclosure also provides a use of substance K or the above pharmaceutical composition in the manufacture of a medicament for regulating T cells and other immune cells, and the substance K is substance K-1, substance K-2, or substance K-3;

the substance K-1 is the above immune-stimulating antibody conjugate represented by Formula II or the pharmaceutically acceptable salt thereof;
the substance K-2 is the above cyclic dinucleotide compound represented by Formula I or the pharmaceutically acceptable salt thereof;
the substance K-3 is the above compound represented by Formula D'-1 or D'-2, the stereoisomer thereof, or the pharmaceutically acceptable salt thereof.

In some embodiments, the amount of substance K may be a therapeutically effective amount.

The present disclosure provides a use of substance K or the above pharmaceutical composition in the manufacture of a medicament for treating and/or alleviating tumors, and the substance K is substance K-1, substance K-2, or substance K-3;
the substance K-1 is the above immune-stimulating antibody conjugate represented by Formula II or the pharmaceutically acceptable salt thereof;
the substance K-2 is the above cyclic dinucleotide compound represented by Formula I or the pharmaceutically acceptable salt thereof;
the substance K-3 is the above compound represented by Formula D'-1 or D'-2, the stereoisomer thereof, or the pharmaceutically acceptable salt thereof.

In some embodiments, the amount of substance K may be a therapeutically effective amount.

The present disclosure provides a use of substance K or the above pharmaceutical composition in the manufacture of a medicament for treating, alleviating, and/or preventing STING-mediated related diseases, and the substance K is substance K-1, substance K-2, or substance K-3;
the substance K-1 is the above immune-stimulating antibody conjugate represented by Formula II or the pharmaceutically acceptable salt thereof;
the substance K-2 is the above cyclic dinucleotide compound represented by Formula I or the pharmaceutically acceptable salt thereof;
the substance K-3 is the above compound represented by Formula D'-1 or D'-2, the stereoisomer thereof, or the pharmaceutically acceptable salt thereof.

In some embodiments, the STING-mediated related diseases refer to tumors or viral infectious diseases.

In some embodiments, the amount of substance K may be a therapeutically effective amount.

The tumor may be a malignant tumor, comprising metastatic and non-metastatic cancer, as well as familial hereditary and incidental cancer, and may also comprise solid tumors and non-solid tumors.

In the present disclosure, the terms "tumor" and "cancer" have the same meaning.

In the present disclosure, unless otherwise specified, the term "optionally substituted at any position by one or more substituents" means that any one or more than one hydrogen atom of one or more than one atom designated on the group is substituted with the designated substituent provided that the normal valence of the designated atom is not exceeded, and that the substitution in any position is a common and reasonable substitution in the art.

In the present disclosure, when the bonding to a substituent is shown to intersect the bonding of two atoms in a linking ring, then such a substituent can be bonded to any bondable ring atom on the ring.

In the present disclosure, any combination of variables is allowed only if such a combination results in a stable compound.

In the present disclosure, when any variable occurs in the constitution or structure of the compound more than once, the definition of the variable at each occurrence is independent. For example, when R is substituted by one or more substituents, each substituent is an independent substituent and may be the same or different.

Unless otherwise specified, the terms used in the description and claims of the present disclosure have the following meanings:

The term "antibody" means any form of antibody that exhibits a desired biological activity (such as inhibiting the binding of a ligand to its receptor or inhibiting ligand-induced receptor signal transduction). Therefore, "antibody" is used in its broadest sense and expressly includes, but is not limited to, monoclonal antibodies (including full-length monoclonal antibodies), polyclonal antibodies, and multispecific antibodies (including bispecific antibodies). Naturally occurring "antibodies" are glycoproteins containing at least two heavy (H) chains and two light (L) chains interconnected by disulfide bonds. Each heavy chain consists of a heavy chain variable region and a heavy chain constant region. The heavy chain constant region consists of three domains (CH1, CH2, and CH3). Each light chain consists of a light chain variable region and a light chain constant region. The light chain constant region contains a CL structural domain. The variable regions of the heavy and light chains contain binding domains (antigen-binding domains) that interact with the antigen. The antigen-binding domain can be provided by one or more than one variable region on the antibody. Specifically, the antigen-binding domain contains the variable domain of light chain (VL) and variable domain of heavy chain (VH) on the antibody. In some cases, the antigen that the antibody can bind to is a tumor-associated antigen. In some cases, the antigen that the antibody can bind to may be a tumor-specific antigen. The antibody may be a monoclonal antibody, a human antibody, a humanized antibody, or a chimeric antibody. These antibodies may be of any class (IgG, IgE, IgM, IgD, IgA, and IgY) or subtype (IgG1, IgG2, IgG3, IgG4, IgA1, and IgA2). The present disclosure includes not only complete antibodies, but also immunologically active antibody fragments (including Fab, F(ab')₂, scFv, or Fv fragments) or fusion proteins formed by antibodies and other sequences. Therefore, "antibodies" as used in the present disclosure also include fragments, derivatives, and analogs of the antibodies.

In the present disclosure, the "antibodies" may further include engineered antibodies. The engineered antibody may 1) comprise one or more non-natural amino acids incorporated into the heavy chain, light chain, or both heavy and light chains, wherein the one or more non-natural amino acids include but are not limited to one or more of the following non-natural amino acids: *p*-acetylphenylalanine, *o*-acylphenylalanine, *m*-acylphenylalanine, *p*-acetyl-*L-*phenylalanine, *p*-acyl-L-phenylalanine, *p*-propargyloxy-L-phenylalanine, 4-azido-*L-*phenylalanine, *p*-azidoethoxyphenylalanine, *p*-azidomethyl-phenylalanine, *etc*.; 2) insert cysteine residues into different positions of the antibody heavy chain or light chain, or replace specific amino acid residues of the antibody heavy chain or light chain with cysteine residues, thereby forming unpaired cysteines for conjugation.

In the present disclosure, the "antibody" or an antigen-binding fragment thereof may include an Fc region, and the Fc region may be further modified. In some cases, one or more mutations in the Fc regions result in improvements in drugs containing such modified Fc regions, such as reduced effector function, changes in the regulation of metabolic half-life of drugs, and drug stability. In some cases, the modified Fc regions can contain one or more mutations that will reduce or eliminate the interaction between the antibody and the immune system. Key interactions can include the interaction of the antibody Fc with the Fcy receptor, and the interaction with the C1q of the complement system. In the case of using IgG1 as the same isotype of the antibody of the present disclosure, the effector function can be adjusted by substituting part of the amino acid residues of the constant region. Variants of IgG1 that reduce or weaken the effector function include, but are not limited to: IgG1 LALA (IgG1-L234A, L235A), IgG1 LAGA (IgG1-L235A, G237A), IgG1 AAG (IgG1-L234A, L235A, P329G), *etc.* The above L234A and L235A indicate that the leucine at positions 234 and 235 determined by the EU index (Proc. Natl. Acad. Sci. U.S.A., Vol.63, No.1 (May 15, 1969), p78-85) is replaced by alanine; G237A indicates that the glycine at position 237 determined by the EU index is replaced by alanine; P329G indicates that the proline at position 329 determined by the EU index is replaced by alanine. In some cases, glycosylation modification of the Fc segment can alter the pharmacological efficacy and pharmacokinetic properties of a drug (Journal of Pharmaceutical Sciences. 2015, 104(6), 1866-1884). For example, natural antibodies produced by mammalian cells usually contain branched, biantennary-type oligosaccharides, which are typically attached to the Asn297 of the CH2 domain of the Fc segment through an N-linkage bond. The oligosaccharides may include various carbohydrates, such as mannose, N-acetylglucosamine (GlcNAc), galactose, and sialic acid, as well as fucose attached to GlcNAc in the stem region of the biantennary oligosaccharide structure. Modification of oligosaccharides in antibodies can produce antibody variants with certain improved properties; for example, afucosylation can enhance antibody-dependent cell cytotoxicity (ADCC) and antibody-dependent cellular phagocytosis (ADCP); reducing terminal sialylation or increasing terminal acetylglucosamination can enhance ADCC activity; treating CHO cells with a mannosidase inhibitor can increase mannosylation and afucosylation, thereby enhancing ADCC effect and slightly reducing CDC effect, *etc.* In addition, glycosylation modification can also be performed through mutation, for example, the N297Q mutation, which substitutes the asparagine at position 297 with glutamine.

The term "mAb", also known as "monoclonal antibody", refers to polypeptides (including antibodies, bispecific antibodies, etc.) that have essentially the same amino acid sequence or are derived from the same genetic source. Monoclonal antibodies are highly specific and can target a single antigenic site. Furthermore, in contrast to conventional (polyclonal) antibody preparations, which typically include multiple different antibodies directed against multiple different determinant clusters (epitopes), each monoclonal antibody is directed against only a single determinant cluster on the antigen. In some specific embodiments, the antigen that the monoclonal antibody can bind to is a tumor-associated antigen; in some specific embodiments, the antigen that the monoclonal antibody can bind to is a tumor-specific antigen; in some specific embodiments, the monoclonal antibody binds to optional antigens including but not limited to: HER2, 5T4 (TPBG), EGFR, or Nectin-4.

The terms "fragment", "derivative", and "analog" refer to polypeptides that essentially maintain the same biological function or activity of the antibodies of the present disclosure. The polypeptide fragment, derivative, or analog of the present disclosure can be a) a polypeptide in which one or more than one conservative or non-conservative amino acid residues (preferably conservative amino acid residues) are replaced, and such replaced amino acid residues can or can not be encoded by genetic code, or b) a polypeptide having substituent groups in one or more than one amino acid residue, or c) a polypeptide formed by fusion of a mature polypeptide with another compound (such as a compound that prolongs the half-life of the polypeptide, such as polyethylene glycol), or d) a polypeptide formed by fusion of an additional amino acid sequence to this polypeptide sequence (such as a leader sequence, secretion sequence, a sequence used for purification of this peptide, or a proteinogenic sequence, or a fusion protein with a 6His tag).

In the present disclosure, the biosimilar of the monoclonal antibody refers to a biosimilar of the monoclonal antibody, despite minor differences in inactive components clinically, is highly similar to the monoclonal antibody and has no clinically meaningful differences in safety and/or efficacy.

The term "HER2" (also known as ERBB2, NEU, NGL, TKR1, CD340, p185, MLN19, HER-2/neu) refers to the transmembrane tyrosine kinase receptor of the epidermal growth factor (EGF) receptor family. HER2 contains an extracellular binding domain, a transmembrane domain, and an intracellular tyrosine kinase domain. HER2 does not have its own ligand-binding domain and therefore cannot bind growth factors. However, HER2 binds tightly to other ligand-binding EGF receptor family members (such as HER1 or HER3) to form heterodimers that stabilize ligand binding and enhance kinase-mediated activation of downstream signaling pathways. In humans, there are isotypes of HER2 species: A, B, C, D, and E. "HER2" as used in the present disclosure includes all HER2 isotypes.

The term "anti-HER2 antibody" refers to an antibody that targets the HER2 protein, and the anti-HER2 antibody can be derived from any species, such as humans, rats, mice, and rabbits. The anti-HER2 antibody is preferably a monoclonal anti-HER2 antibody, and the anti-HER2 antibody is more preferably a humanized anti-HER2 antibody. The anti-HER2 antibodies include, but are not limited to: pertuzumab, trastuzumab, trastuzumab biosimilar (*e.g*., inetetamab), pertuzumab biosimilar, margetuximab, HT-19, *etc*.;
wherein Trastuzumab (also known as Herceptin or Herclon) is a humanized monoclonal antibody that can bind to the near-membrane region of the extracellular structure of the HER2 receptor (Hudis CA, N Engl J Med. 2007; 357(1):39-51). The amino acid sequences of heavy chain and light chain variable regions of Trastuzumab are disclosed in US Patent 5,821,337. Trastuzumab interacts with the tricyclic region formed by human HER2 residues 557 to 561, 570 to 573, and 593 to 603 (Cho et al., Nature 421: 756-760, 2003). Trastuzumab can interfere with HER2 signal transduction by preventing HER2 receptor dimerization, promoting HER2 receptor endocytosis, and inhibiting the shedding of the extracellular domain. In addition, another important mechanism of action of anti-HER2 antibodies is to mediate antibody-dependent cellular cytotoxicity (ADCC). In ADCC, anti-HER2 antibodies bind to tumor cells and then recruit immune cells, such as macrophages, through interaction with Fcy receptor (FcyR). Trastuzumab has a conserved human IgG Fc region and can recruit immune effector cells responsible for antibody-dependent cytotoxicity (Hudis CA, N Engl J Med. 2007; 357(1):39-51). Trastuzumab was approved by the US FDA in September 1998 for the treatment of tumors overexpressing HER2 and patients with metastatic breast cancer who have received one or more than one chemotherapy regimen. In some embodiments, to achieve site-specific conjugation, an engineered Trastuzumab with cysteine inserted between positions 239 and 240 of the heavy chain can be prepared by methods disclosed in Molecular Pharmaceutics. 2017, 14, 1501-1516; in some embodiments, Trastuzumab IgG1 variants with reduced or weakened effector functions can be obtained by partially substituting amino acid residues in the constant region, including but not limited to: IgG1 LALA (IgG1-L234A, L235A), IgG1 LAGA (IgG1-L235A, G237A), IgG1 AAG (IgG1-L234A, L235A, P329G), *etc.* The above L234A and L235A indicate that the leucine at positions 234 and 235 determined by the EU index (Proc. Natl. Acad. Sci. U.S.A., Vol.63, No.1 (May 15, 1969), p78-85) is replaced by alanine; G237A indicates that the glycine at position 237 determined by the EU index is replaced by alanine; P329G indicates that the proline at position 329 determined by the EU index is replaced by alanine.

Pertuzumab (also known as Perjeta, Omnitarg) is a humanized monoclonal antibody that binds to the extracellular domain of the HER2 receptor and inhibits the dimerization of HER2 and the HER receptor. The amino acid sequences of heavy chain and light chain variable regions of Pertuzumab are disclosed in US Patent 7,560,111. Pertuzumab mainly interacts with residues in the 245 to 333 region of human HER2, specifically residues His245, Val286, Ser288, Leu295, His296, or Lys311 (Franklin et al., Cancer Cell 5: 317-328, 2004). Studies have shown that Pertuzumab is more effective than Trastuzumab in disrupting HER1-HER2 and HER3-HER2 complex formation in breast and prostate cancer cell lines (Agus et al., J Clin Oncol. 2005; 23(11):2534-43. Epub Feb 7, 2005). Pertuzumab was approved by the US FDA in June 2012, and is used in combination with Trastuzumab and Docetaxel to treat patients with HER2-positive metastatic breast cancer who have not received anti-HER2 therapy or chemotherapy.

Margetuximab (also known as MGAH22, Margenza) is an Fc-engineered monoclonal antibody targeting the HER2 protein and binds to the extracellular region of HER2. Margetuximab only differs from the variable region sequence of Trastuzumab by a few amino acids. The Fc has been mutated at five sites: F243L/R292P/Y300L/L235V/P396L, which improves the affinity for CD16A and enhances the activity of ADCC. The modified Fc region of Margetuximab increases the binding to the activating Fc receptor FCGR3A (CD16A) and reduces the binding to the inhibitory Fc receptor FCGR2B (CD32B), resulting in stronger ADCC effects and NK cell activation (Nordstrom J. et al., Breast Cancer Research, 2011; 13: R123). Margetuximab was approved by the US FDA in December 2020 for the treatment of adult patients with HER2-positive metastatic breast cancer (MBC) who have received two or more than two anti-HER2-targeted therapies, wherein at least one anti-HER2-targeted therapy is for the treatment of metastatic breast cancer.

In the present disclosure, the anti-HER2 antibody is not limited to the above-listed antibodies as long as it specifically binds to HER2 (for example, an anti-HER2 antibody that has the internalizing activity in HER2-expressing cells through binding to HER2).

In the present disclosure, the isotypes of the "anti-HER2 antibody" include IgG1, IgG2, IgG3, IgG4, *etc*., preferably IgG1, IgG2, or IgG4.

The term "low HER2 expression" generally refers to a clinical test result of HER2 expression level of IHC 1+, or IHC 2+/FISH negative (i.e., IHC 2+, and the FISH test is negative at the same time). The terms "high HER2 expression" and "HER2 positive" are used interchangeably and generally refer to a clinical test result of HER2 expression level of IHC 2+/FISH positive (*i.e*., IHC 2+, and the FISH test is positive at the same time), or IHC 3+. When IHC staining intensity is reported as a range, the term "low HER2 expression" herein includes not only IHC 1+, or IHC 2+/FISH negative, but also ranges from IHC 0 to 1+ and IHC 1+ to 2+. The terms "high HER2 expression" and "HER2 positive" each include not only IHC 2+/FISH positive, or IHC 3+, but also a range from IHC 2+ to 3+. In the present disclosure, FISH negative means a FISH test result showing no amplification of the HER2 gene, while FISH positive means a FISH test result showing amplification of the HER2 gene.

The term "EGFR" refers to epidermal growth factor receptor, which belongs to the ErbB family. Homodimerization or heterodimerization of EGFR and other members of the ErbB family activates the cytoplasmic tyrosine kinase domain to initiate intracellular signal transduction. Overexpression or activating mutations of EGFR are associated with the development of various types of cancer, such as pancreatic cancer, breast cancer, glioblastoma multiforme, head and neck cancer, and non-small cell lung cancer. "Anti-EGFR monoclonal antibody" includes but is not limited to: cetuximab, panitumumab, nimotuzumab, necitumumab, depatuxizumab, amivantamab, *etc*.;
wherein cetuximab (also known as Erbitux) is a chimeric antibody of human and mouse. In addition to competitively binding to the extracellular epitope of the natural ligand EGF to block the transmission of signaling pathways, cetuximab can also induce receptor internalization, downregulation, and degradation. In addition, cetuximab can also block the cell cycle and cause antibody-dependent cytotoxic effects. Cetuximab was first approved by the U.S. FDA in 2004 for the treatment of patients with EGFR-positive metastatic colorectal cancer who had failed treatment with the chemotherapy drug irinotecan; in 2006, it was approved again by the U.S. FDA for use in combination with radiotherapy for locally or regionally advanced squamous cell carcinoma of the head and neck.

Panitumumab (also known as Vectibix) is a fully humanized IgG2-type antibody. Panitumumab, like Cetuximab, can rapidly bind to EGFR and has a low dissociation constant, thus effectively competitively blocking the binding of EGF and TGFα ligands. Panitumumab exhibits good antitumor activity in xenograft models of various tumor cells, including pancreatic cancer, breast cancer, renal cancer, prostate cancer, and head and neck cancer (Yang X.D. et al., Critical Reviews in Oncology/hematology. 2001; 38(1):17-23). Panitumumab was first approved by the U.S. FDA in 2006 for the treatment of metastatic colorectal cancer patients who are EGFR-positive and have been treated with irinotecan, oxaliplatin, and 5-fluorouracil.

Nimotuzumab (also known as Tai Xin Sheng) is a fully humanized IgG1-type antibody. The variable region of the heavy chain of Nimotuzumab differs significantly from that of Cetuximab. After nimotuzumab binds to EGFR, it does not remain completely in an inhibited conformation like cetuximab; the extracellular domains I and III of EGFR may still interact with each other to form a conformational equilibrium in the activated state, and its affinity for the EGFR epitope is also much weaker than that of cetuximab. Clinically, a larger dosage is required to achieve the efficacy of cetuximab, but at the same time, the probability of adverse effects such as rash is also lower (Talavera A. et al., Cancer Research. 2009; 69(14): 5851-9). In 2014, the FDA approved nimotuzumab for the treatment of head and neck cancer and glioma.

Necitumumab (also known as Portrazza) is a fully humanized IgG1-type antibody. The cavity between the heavy and light chains of necitumumab is larger than that of cetuximab. Some cetuximab-resistant mutations, such as S492R, which are highly prevalent in metastatic colorectal cancer, are precisely located in this binding region. Therefore, necitumumab can overcome the antibody drug resistance phenomenon caused by these EGFR extracellular point mutations. In 2015, the FDA approved necitumumab for the treatment of non-small cell lung cancer.

In the present disclosure, the anti-EGFR antibody is not limited to the above-listed antibodies as long as it specifically binds to EGFR (for example, an anti-EGFR antibody that has the internalizing activity in EGFR-expressing cells through binding to EGFR).

In the present disclosure, the isotypes of the "anti- EGFR antibody" include IgG1, IgG2, IgG3, IgG4, *etc*., preferably IgG1, IgG2, or IgG4.

The term "5T4," also known as 5T4 oncofetal antigen or trophoblast glycoprotein TPBG, is a 72 kDa glycoprotein defined by a monoclonal antibody produced from a glycoprotein isolated with wheat germ agglutinin from the microvillous membrane of human placental syncytiotrophoblasts. It has limited expression in normal tissues but is overexpressed in various types of cancer cells. "Anti-5T4 monoclonal antibody" includes but is not limited to: H8 and its humanized monoclonal antibody disclosed in WO2006/031653A1, A1, A2, A3, and their humanized monoclonal antibodies (e.g., huA1) disclosed in US8044178B2, engineered A1 monoclonal antibody disclosed in WO2013068874, naptumomab, *etc.*

The term "Nectin-4", also known as the 191P4D12 protein, is a cell adhesion molecule belonging to the family of nectin surface molecules, which plays a key role in various biological processes of epithelial cells, endothelial cells, immune cells, and nerve cells during development and adulthood. Nectin-4 is a tumor-associated antigen on major tumors with poor prognosis. Anti-Nectin-4 monoclonal antibodies include but are not limited to: enfortumab (Ha22-2), *etc.*

The term "linker" refers to a degradable or non-degradable linking fragment used to attach the small molecule drug D to the antibody. One antibody molecule can be attached to a plurality of linkers carrying a small molecule drug D. Normally, each linker can be attached to one or more small molecule drugs. In the present disclosure, preferably, each linker is attached to one small molecule drug D. Normally, each antibody can be attached to a plurality of linkers. In the present disclosure, preferably, each antibody is attached to 1 to 8 linkers.

In the present disclosure, the non-degradable linker means that the linker has enzyme stability and/or chemical stability *in vivo* and *in vitro,* and the release of small molecule drug D can not depend on the differential nature of plasma, tumor tissue, and intracellular enzyme levels. The release of small molecule drug D can be achieved through antigen-mediated phagocytosis by degradation of the antibody to the amino acid level after endocytosis of the immune-stimulating antibody conjugate, which in turn releases a derivative of small molecule drug D. The derivative of small molecule drug D is composed of a small molecule drug D, a linker, and an amino acid residue or is a residue consisting of a small molecule drug D covalently attached to a linker. The immune-stimulating antibody conjugates constructed by such non-degradable linkers have better stability. The non-degradable linkers include alkylene chains and their polymers (for example: alkylene amide polymers, alkylene glycol polymers, or combinations including alkylene glycol, alkylene amide polymer fragments) or ethylene glycol fragments and polyethylene glycol fragments, and combinations thereof.

In the present disclosure, the degradable linker can be degraded *in vivo* or *in vitro,* and it contains a linker that can be degraded by a specific enzyme *in vivo* or *in vitro* or a chemically unstable linker. The degradable linker can be degraded inside the cell to release the small molecule drug D. For example, the linker can be reduced in the cytoplasm, degraded under lysosomal acidic conditions, or degraded by specific proteases or other enzymes within the cell. The degradable linker includes one or more enzymatically degradable linkers, chemically unstable linkers, or other degradable linkers, while other portions may be linkers that are not degraded by enzymes or are chemically stable. The chemically unstable linker includes an oxime, hydrazone, and/or disulfide group (*e*.*g*., 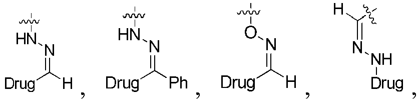 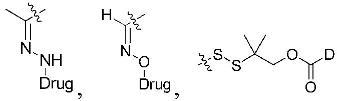 ). The linker specifically degraded by enzymes is a linker based on 1) amino acid residues or peptides. The peptide bond can have good serum stability due to the fact that lysosomal proteases are much less active in blood than in certain tumor tissues. Therefore, the linker can be selectively degraded in certain tumor tissues or cells to release the small molecule drug D. The lysosomal enzyme can be selected from cathepsin B, cathepsin S, plasmin, elastase, β-glucuronidase, or β-galactosidase, *etc.* The linker formed based on peptides (-(A)ᵥ-) may be a tetrapeptide (including but not limited to: -Gly-Phe-Leu-Gly-, -Ala-Leu-Ala-Leu-, -Gly-Gly-Phe-Gly-, -Leu-Ser-Gly-lys-, -Ala-Ala-Pro-Val-, -Glu-Val-Ala-Gly-, -Glu-Val-Cit-Gly-, -Leu-Ala-Glu-Gly-), tripeptide (including but not limited to: -Val-Leu-Lys-, -Ala-Pro-Val-, -Ala-Ala-Asn-, -Glu-Val-Cit-, -Leu-Ala-Glu-, -Glu-Val-Ala-, - Val-Cit-Gly-, -Val-Ala-Gly-, -Leu-Ala-Glu-), dipeptide (including but not limited to: -Val-Cit-, -Cit-Val-, -Val-Ala-, -Ala-Val-, -Ala-Cit-, -Cit-Ala-, -Asn-Cit-, -Cit-Asn-, -Cit-Cit-, -Val-Glu-, -Glu-Val-, -Ser-Cit-, -Cit-Ser-, -Lys-Cit-, -Cit-Lys-, -Asp-Cit-, -Cit-Asp-, -Ala-Val-, -Val-Ala-, -Phe-Lys-, -Lys-Phe-, -Val-Lys-, -Lys-Val-, -Ala-Lys-, -Lys-Ala-, -Phe-Cit-, -Cit-Phe-, -Leu-Cit-, -Cit-Leu-, -Ile-Cit-, -Cit-Ile-, -Phe-Arg-, -Arg-Phe-, -Cit-Trp-, -Trp-Cit-, -Pro-Val-) or amino acid monomer (including but not limited to: -Lys-, -Gly-, -Cit-), the linker formed based on peptides is preferably a dipeptide linker, tripeptide linker, or tetrapeptide linker, and more preferably a dipeptide linker. The other degradable linkers can include ester linkage bonds formed by the reaction of PEG carboxylic acid or activated PEG carboxylic acid with the hydroxyl groups on the small molecule drug D, wherein such ester linkage bonds can be hydrolyzed under physiological conditions, thereby releasing the small molecule drug D. Hydrolytically degradable linkage bonds include, but are not limited to, carbonate linkage bonds, imine linkage bonds resulting from the reaction of amines and aldehydes, phosphate ester linkage bonds resulting from the reaction of hydroxyl groups and phosphoric acid groups, acetal linkage bonds resulting from the reaction of hydroxyl groups and aldehydes, and orthoester linkage bonds resulting from the reaction of formate groups and hydroxyl groups. The degradable linkers can also include non-degradable fragments, such as polyethylene glycol (PEG) and its related polymers. 2) A linker formed via pyrophosphate or phosphate esters. Lysosomal acidic pyrophosphate and acidic phosphatase are enzymes that hydrolyze pyrophosphate and terminal monophosphate, respectively, into their parent alcohols in lysosomes. Targeting these enzymes can effectively release the small molecule drug D with an alkyl alcohol terminal.

In the present disclosure, the "linker" can further include a Spacer, and the linker head can be directly attached to a linker in the linker moiety, or can be attached to a linker in the linker moiety via a Spacer. The spacer may be polyethylene glycol and its related polymer, an alkylene group containing 1 to 10 carbons, a cyclohexyl group, a phenyl group, a 1,3-dioxanyl group, an amide group, an ester group, an oxo group, a substituted amino group, a triazolyl group, or 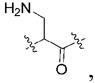 and any one or more combinations of the above spacers.

In the present disclosure, the "linker" may further comprise a self-immolative group, which spatially separates the small molecule drug D from the enzyme degradation site.

In the present disclosure, the "linker" further comprises a linker head that can be connected to an antibody, which may be formed through the reaction of a linker head precursor with a thiol group in the antibody (for example: cysteine), an amino group (for example: lysine), a carbonyl group (for example: *p*-acetylphenylalanine), an aldehyde group, an azido group (for example: *p*-azidomethylphenylalanine), a phenol group (for example: tyrosine), *etc.*

In the present disclosure, the "linker head precursor" can react with an antibody and form a linker head that is connected to the antibody.

In the present disclosure, the linker head precursor can react with thiol groups (for example: cysteine), amino groups (for example: lysine), carbonyl groups (for example: p-acetylphenylalanine), aldehyde groups, azido groups (for example: p-azidomethylphenylalanine), phenolic hydroxyl groups (for example: tyrosine) in the antibody; the linker head precursor can also be connected to specific groups of the antibody through enzyme-catalyzed reactions.

The linker head precursors capable of reacting with thiol groups on amino acid side chains of the antibody include but are not limited to: ethynyl, vinyl, hydroxylamino, 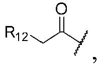 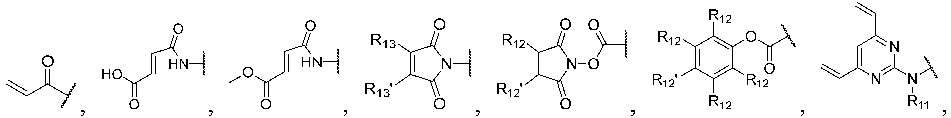 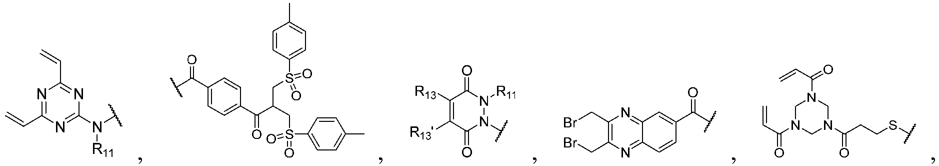 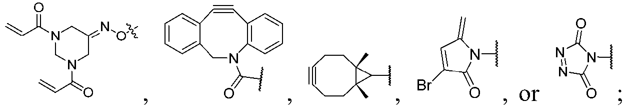 R₁₂, R₁₃, R_{13'}, and R₁₁ are as defined above.

The reaction between the linker head precursor and the thiol group (for example: cysteine) in the antibody is specific, for example, as shown in Formulas 1-6 and 12:

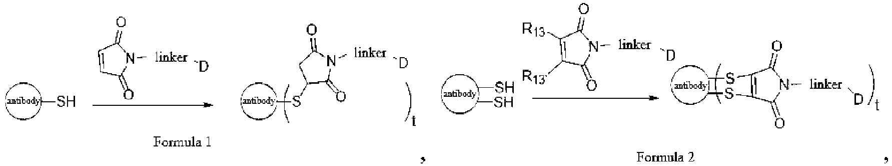

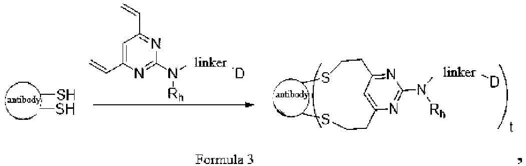

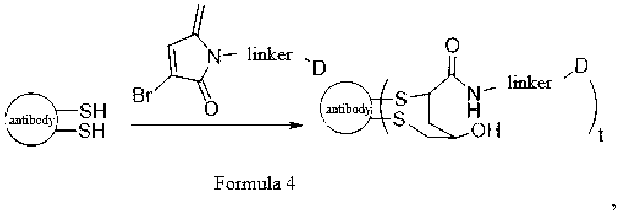

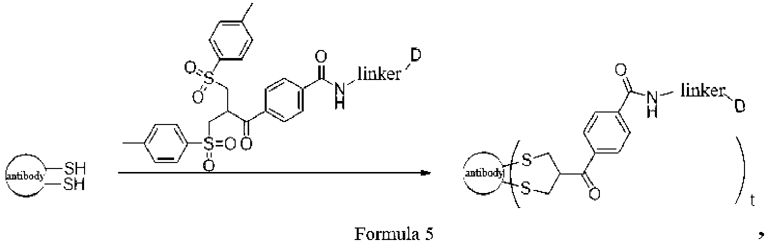

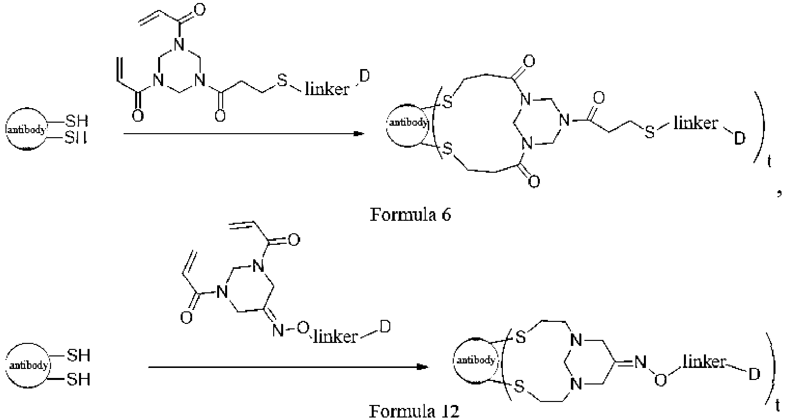

When the linker head is maleimide or succinimide, it may be further hydrolyzed to obtain hydrolysis products, for example, as shown in Reaction Formula 1' or Reaction Formula 2':

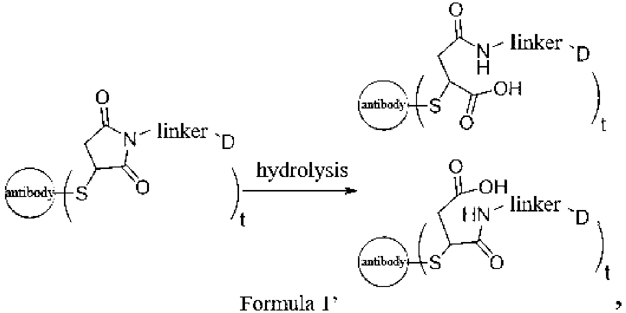

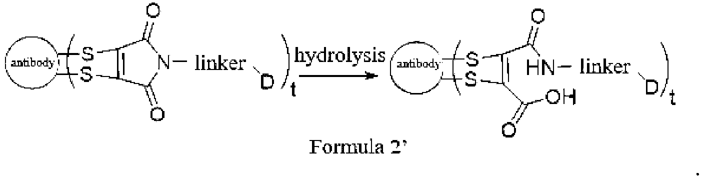

The linker head precursor capable of reacting with the azido group of the antibody amino acid residue side chain includes but is not limited to: ethynyl, 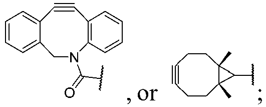 the click reaction between the linker head precursor and the azido group in the antibody (e.g., p-azidomethylphenylalanine) is specific, for example, as shown in Formula 7 or 13:

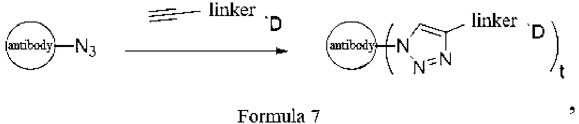

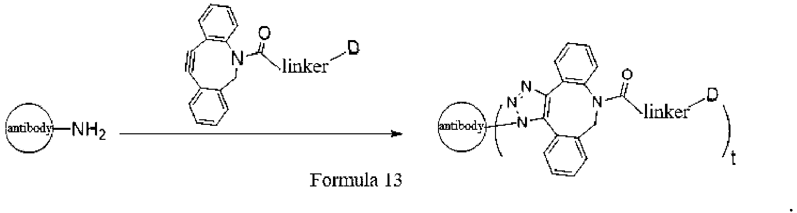

The linker head precursor capable of reacting with the carbonyl or aldehyde group on the antibody amino acid residue side chain includes but is not limited to: hydroxylamino or hydrazino. The reaction between the linker head precursor and the acyl (e.g., *p-*acetylphenylalanine) or aldehyde group on the antibody is specific, for example, as shown in Formulas 8-9:

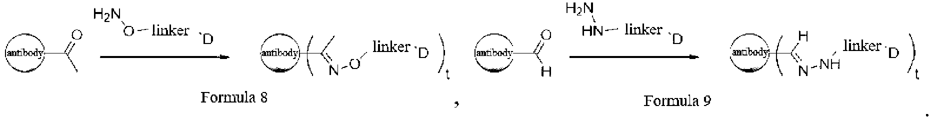

The linker head precursor capable of reacting with the phenol group on the antibody amino acid residue side chain includes but is not limited to: 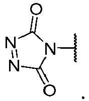 The click-like reaction between the linker head precursor and the phenol group (*e*.*g*., tyrosine) on the antibody is specific, for example, as shown in Formula 10:

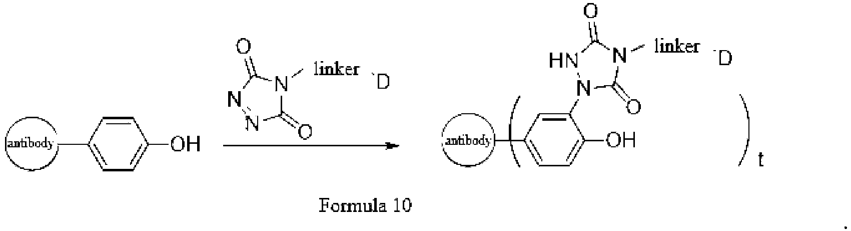

The linker head precursor that directly reacts by reaction with the side chain amino group of the amino acid residue of the antibody through the linker and amino group is an active ester group, including but not limited to: 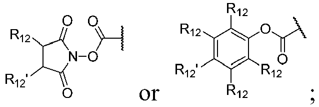 R₁₂ and R_{12'} are as defined above; the reaction between the linker head precursor and the amino group (for example: lysine) in the antibody is specific, for example, as shown in Formula 11:

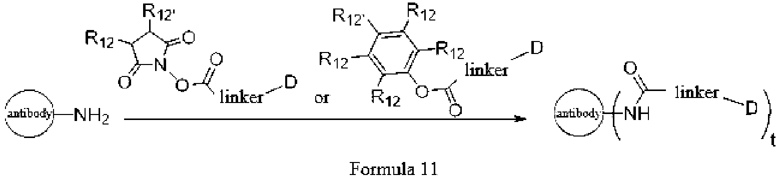

The term "linker precursor" can directly react with the antibody and form a degradable or non-degradable linker used for linking the small molecule drug D and the antibody.

The term "and/or" includes any and all combinations of one or more related listed items.

The term "C_{A-B}" refers to the range from the starting point to the end point, wherein A, B, and each point in the range are integers, indicating the number of the carbon atom, for example, C₁₋₄ means that the number of the carbon atom is 1, 2, 3, or 4; C₁₋₆ means the number of the carbon atom is 1, 2, 3, 4, 5, or 6; C₃₋₈ means the number of the carbon atom is 3, 4, 5, 6, 7, or 8; C_{A-B} can be used in combination with any group containing the carbon atom for the purpose of defining the number of the carbon atom, such as C₁₋₆ alkyl, C₃₋₈ cycloalkyl, C₆₋₁₀ aryl, C₁₋₄ alkoxy, C₃₋₈ cycloalkyl-C₁₋₄ alkyl, *etc.*

The term "alkyl" refers to a saturated straight-chain or branched hydrocarbon group containing 1 to 20 carbon atoms, preferably 1 to 10 carbon atoms, more preferably 1 to 8, 1 to 6, 1 to 4, or 1 to 3 carbon atoms. Representative examples of alkyl include, but are not limited to, methyl, ethyl, *n*-propyl, isopropyl, *n*-butyl, *sec*-butyl, *tert*-butyl*,* isobutyl, *n*-pentyl, *n*-hexyl, *n-heptyl,* octyl, nonyl, decyl, 1,1-dimethylpropyl, 1,2-dimethylpropyl, 2,2-dimethylpropyl, 1-ethylpropyl, 2-methylbutyl, 3-methylbutyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2,3-dimethylbutyl, 2-ethylbutyl, 2-methylpentyl, 3-methylpentyl, 4-methylpentyl, 4,4-dimethylpentyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylhexyl, 3-ethylhexyl, 2,2,4-trimethylpentyl, undecyl, dodecyl, and various isomers thereof.

The term "alkenyl" refers to a straight-chain or branched non-aromatic hydrocarbon group containing at least one carbon-carbon double bond. There can be 1 to 3 carbon-carbon double bonds, preferably one carbon-carbon double bond. The term "C₂₋₄ alkenyl" refers to an alkenyl group having 2-4 carbon atoms. The term "C₂₋₆ alkenyl" refers to an alkenyl group having 2-6 carbon atoms, including vinyl, propenyl, butenyl, and 2-methylbutenyl.

The term "alkynyl" refers to a straight-chain or branched hydrocarbon group containing at least one carbon-carbon triple bond. There can be 1 to 3 carbon-carbon triple bonds, preferably one carbon-carbon triple bond. The term "C₂₋₆ alkynyl" refers to an alkynyl group with 2 to 6 carbon atoms, including ethynyl, propynyl, butynyl, and 3-methylbutynyl.

The term "alkylene" refers to a saturated straight-chain or branched non-bridged divalent alkyl group containing 1 to 20 carbon atoms, preferably 1 to 10 carbon atoms, more preferably 1 to 8, 1 to 6, or 1 to 4 carbon atoms, and the examples include but are not limited to -CH₂-, -CH₂CH₂-, -CH₂CH₂CH₂-, -CH₂CH₂CH₂CH₂-, -CH₂CH(CH₃)-, -CH₂C(CH₃)₂CH₂-, -CH₂C(CH₃)₂CH₂CH₂-, =CH₂, =CHCH₃, and =C(CH₃)₂.

The term "alkenylene" refers to a straight-chain or branched non-aromatic divalent hydrocarbon group containing at least one carbon-carbon double bond, where there may be 1 to 3 carbon-carbon double bonds, preferably 1 carbon-carbon double bond. The alkenylene preferably contains 2 to 10 carbon atoms, more preferably 2 to 8, 2 to 6, or 2 to 4 carbon atoms.

The term "alkynylene" refers to a straight-chain or branched non-aromatic divalent hydrocarbon group containing at least one carbon-carbon triple bond. There can be 1 to 3 carbon-carbon triple bonds, preferably one carbon-carbon triple bond. The alkynylene preferably contains 2 to 10 carbon atoms, more preferably 2 to 8, 2 to 6, or 2 to 4 carbon atoms.

The term "cycloalkyl" refers to a saturated or partially unsaturated (containing 1 or 2 double bonds) monocyclic or polycyclic group containing 3 to 20 carbon atoms. The "monocyclic cycloalkyl" is preferably a 3- to 10-membered monocycloalkyl group, more preferably a 3- to 8-membered monocycloalkyl group, such as cyclopropyl, cyclobutyl, cyclopentyl, cyclohexyl, cycloheptyl, cyclooctyl, cyclodecyl, cyclododecyl, and cyclohexenyl. The "polycyclic cycloalkyl" includes "bridged cycloalkyl", "fused cycloalkyl", and "spirocycloalkyl". Representative examples of "bridged cycloalkyl" include, but are not limited to, bornyl, bicyclo[2.2.1]heptenyl, bicyclo[3.1.1]heptyl, bicyclo[2.2.1]heptyl, bicyclo[2.2.2]octyl, bicyclo[3.2.2]nonyl, bicyclo[3.3.1]nonyl, bicyclo[4.2.1]nonyl, adamantyl, *etc.* The "fused cycloalkyl" includes a cycloalkyl ring fused to phenyl, cycloalkyl, or heteroaryl. The fused cycloalkyl includes, but is not limited to: benzocyclobutenyl, 2,3-dihydroindenyl, decahydronaphthalenyl, *etc.* Monocyclic cycloalkyl or polycyclic cycloalkyl can be attached to the parent molecule via any carbon atom on the ring.

The term "cycloalkylene" refers to a divalent cycloalkyl group. Thus, "cycloalkylene" includes the definitions of cycloalkyl described above. The "cycloalkylene" is preferably C₃₋₁₀ cycloalkylene, more preferably C₃₋₈ cycloalkylene or C₃₋₆ cycloalkylene.

The term "heterocycloalkyl" means a saturated or partially unsaturated (containing 1 or 2 double bonds) non-aromatic cyclic group consisting of a carbon atom and a heteroatom selected from nitrogen, oxygen, sulfur, boron, *etc.,* and/or a sulfur-containing heteroatom group, which can be monocyclic or polycyclic, wherein the sulfur-containing heteroatom group is selected from, but is not limited to, S(O), S(O)₂, and S(O)(NH). In the present disclosure, the number of the heteroatoms and/or the heteroatom groups in the heterocycloalkyl is preferably 1, 2, 3, or 4, and the boron, nitrogen, or carbon atom in the heterocycloalkyl can be optionally oxidized. The nitrogen atom can selectively be further substituted by other groups to form tertiary amines or quaternary ammonium salts. The "monocyclic heterocycloalkyl" is preferably a 3- to 10-membered monocyclic heterocycloalkyl group, and more preferably a 3-to 8-membered monocyclic heterocycloalkyl group. For example: pyrrolidinyl, dihydropyrrolidinyl, dihydroimidazolyl, dihydropyrazolyl, tetrahydrofuranyl, tetrahydropyrazinyl, dihydrofuranyl, tetrahydrothiophenyl, tetrahydropyranyl, dihydropyranyl, tetrahydrothiopyranyl, tetrahydropyridinyl, tetrahydropyrimidinyl, piperidinyl, aziridinyl, morpholinyl, thiomorpholinyl, thiomorpholin-S-oxide-4-yl, piperidinyl, piperazinyl, 1,4-dioxanyl, homopiperazinyl, 1-imino-1-oxo-tetrahydro-2*H*-thiopyranyl, 1,1-dioxotetrahydrothiophenyl, 1-imino-1-oxo-tetrahydrothiophenyl, 1,1-dioxo-3,4-dihydro-2*H-*thiopyranyl, 1-imino-1-oxo-3,4-dihydro-2*H*-thiopyranyl, 1,1-dioxo-2,3-dihydrothiophenyl, 1-imino-1-oxo-2,3-dihydrothiophenyl, *etc.* The "polycyclic heterocycloalkyl" includes "fused heterocycloalkyl", "spiroheterocyclyl", and "bridged heterocycloalkyl". The "fused heterocycloalkyl" includes a monocyclic heterocycloalkyl ring fused to phenyl, cycloalkyl, heterocycloalkyl, or heteroaryl. The fused heterocycloalkyl includes, but is not limited to: 2,3-dihydrobenzofuranyl, 1,3-dihydroisobenzofuranyl, dihydroindolyl, 2,3-dihydrobenzo[*b*]thiophenyl, dihydrobenzopyranyl, 1,2,3,4-tetrahydroquinolyl, 1,2,3,4-tetrahydroisoquinolyl, 1,2,3,4-tetrahydronaphthyridinyl, 5,6,7,8-tetrahydronaphthyridinyl, 1-hydroxy-1,3-dihydrobenzo[c][1,2]oxaborolanyl, *etc.* The monocyclic heterocycloalkyl and polycyclic heterocycloalkyl can be attached to the parent molecule via any ring atom on the ring. The ring atom specifically means the carbon atom and/or nitrogen atom constituting the ring skeleton.

The term "heterocycloalkylene" refers to a divalent heterocycloalkyl group. Thus, "heterocycloalkylene" includes the definitions of heterocycloalkyl described above. The "cycloalkylene" is preferably a 3- to 10-membered heterocycloalkylene group, more preferably a 3- to 8-membered heterocycloalkylene group or a 3- to 6-membered heterocycloalkylene group.

The term "cycloalkylalkyl" refers to the cycloalkyl that is attached to the parent structure via an alkyl group. Thus, "cycloalkylalkyl" includes the above definitions of alkyl and cycloalkyl.

The term "heterocycloalkylalkyl" refers to the heterocycloalkyl that is attached to the parent structure via an alkyl group. Thus, "heterocycloalkylalkyl" includes the above definitions of alkyl and heterocycloalkyl.

The term "alkoxy" refers to an alkyloxy group connected via an oxygen bridge with the specified number of carbon atoms. Thus, "alkoxy" includes the definitions of alkyl described above.

The term "alkylthio" refers to an alkylthio group connected via a thio bridge with the specified number of carbon atoms. Thus, "alkylthio" includes the definitions of alkyl described above.

The term "hydroxyalkyl" means that any hydrogen atom on the alkyl group is substituted by a hydroxyl group, including but not limited to: -CH₂OH, -CH₂CH₂OH, - CH₂CH₂C(CH₃)₂OH.

The term "aryl" refers to any stable 6- to 20-membered monocyclic or polycyclic aromatic group, such as phenyl, naphthyl, tetrahydronaphthyl, 2,3-dihydroindenyl, or biphenyl.

The term "arylene" refers to a divalent heteroaryl group. Thus, "arylene" includes the definitions of aryl described above. The "arylene" is preferably C₆₋₁₀ arylene, more preferably phenylene.

The term "heteroaryl" refers to an aromatic ring group formed by replacing at least one carbon atom on the ring by a heteroatom selected from nitrogen, oxygen, or sulfur. It can be a 5- to 7-membered monocyclic structure or a 7- to 20-membered fused ring structure, preferably a 5- to 6-membered heteroaryl group. In the present disclosure, the number of the heteroatoms is preferably 1, 2, or 3, including: pyridinyl, pyridonyl, pyrimidinyl, pyrimidine-2,4(1*H*,3*H*)-dione, pyrimidonyl, piperazinyl, pyridazinonyl, furanyl, thiophenyl, thiazolyl, pyrrolyl, imidazolyl, pyrazolyl, oxazolyl, isoxazolyl, 1,2,5-oxadiazolyl, 1,2,4-oxadiazolyl, 1,3,4-oxadiazolyl, 1,3,4-thiadiazolyl, 1,2,4-triazolyl, 1,2,3-triazolyl, tetrazolyl, indazolyl, isoindazolyl, indolyl, isoindolyl, benzofuranyl, benzothiophenyl, benzo[*d*][1,3]dioxolanyl, benzothiazolyl, benzoxazolyl, quinolinyl, isoquinolinyl, isoquinolinonyl, quinazolinyl, 4-hydroxythieno[3,2-c]pyridinyl, 4,5-dihydro-4-oxofuro[3,2]pyridinyl, 4-hydroxy-5-azaindolyl, furo[2,3-*c*]pyridin-7(6*H*)-one, thieno[2,3-c]pyridin-7(6*H*)-one, *etc.*

The term "heteroarylene" refers to a divalent heteroaryl group. Thus, "heteroarylene" includes the definitions of heteroaryl described above. The "heteroarylene" is preferably a 5-to 10-membered heteroarylene group, more preferably a 5- to 6-membered heteroarylene group.

The term "arylalkyl" refers to an aryl group connected to the parent structure via an alkyl group. Thus, "arylalkyl" includes the definitions of alkyl and aryl described above.

The term "heteroarylalkyl" refers to a heterocycloalkyl group connected to the parent structure via an alkyl group. Thus, "heteroarylalkyl" includes the definitions of alkyl and heteroaryl described above.

The term "halogen" refers to fluorine, chlorine, bromine, or iodine.

The term "haloalkyl" refers to an alkyl group optionally substituted with halogens. Thus, "haloalkyl" includes the definitions of halogen and alkyl described above.

The term "haloalkoxy" refers to an alkoxy group optionally substituted with halogens. Thus, "haloalkoxy" includes the definitions of halogen and alkoxy described above.

The term "amino" refers to -NH₂, and the term "alkylamino" refers to an amino group in which at least one hydrogen atom is substituted by an alkyl group, including but not limited to: -NHCH₃, -N(CH₃)₂, -NHCH₂CH₃, -N(CH₂CH₃)₂. Thus, "alkylamino" includes the definitions of alkyl and amino described above.

The term "aminoalkyl" refers to an alkyl group in which any hydrogen atom is substituted by an amino group, including but not limited to: -CH₂NH₂, -CH₂CH₂NH₂, - CH₂CH₂C(CH₃)₂NH₂.

The term "acyl" refers to -C(O)-C₁₋₆ alkyl; preferably formyl, acetyl, propionyl, or isopropionyl.

The term "nitro" refers to -NO₂.

The term "cyano" refers to -CN.

The term "oxo" refers to =O.

The term "thiol" refers to -SH.

The term "amido" refers to -C(O)NH₂.

The term "hydroxyamino" refers to -NHOH.

The term "carboxyl" refers to -C(O)OH.

The term "aldehyde" refers to -C(O)H.

The term "methoxycarbonyl" refers to -C(O)OCH₃.

The term "maleimido" refers to

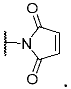

In the present disclosure, amino acids are abbreviated according to conventional abbreviations (refer to the Nomenclature Principles of Organic Chemistry by the Chinese Chemical Society, 2017), for example: alanine (Ala), arginine (Arg), aspartic acid (Asp), asparagine (Asn), cysteine (Cys), glutamic acid (Glu), glutamine (Gln), glycine (Gly), histidine (His), isoleucine (Ile), leucine (Leu), lysine (Lys), methionine (Met), phenylalanine (Phe), proline (Pro), serine (Ser), threonine (Thr), tryptophan (Trp), tyrosine (Tyr), citrulline (Cit), valine (Val). In the present disclosure, the peptide symbol (taking -Gly-(-NH-CH₂-CO-) as an example) indicates that when the hyphen is to the right of Gly, it means the OH group has been removed from the -COOH group of the amino acid; when the hyphen is to the left of Gly, it means one H atom has been removed from the -NH₂- group of the amino acid.

In the present disclosure, it is generally considered that in ³¹P NMR, the chemical shift of ³¹P is related to the shape of the extranuclear electron cloud. When the sphericity of the electron cloud symmetry is better, the chemical shift moves towards a higher field; when the ligands of phosphorus are the same or the electronic properties of the ligands are similar, the ³¹P chemical shift moves towards a higher field, especially when the ligand is a highly electronegative group, the change is particularly pronounced. Aromatic hydrocarbon substitution, as opposed to aliphatic hydrocarbon substitution, causes the ³¹P chemical shift to shift toward a high field. This is because the conjugation of the groups adjacent to the phosphorus atom also affects the ³¹P chemical shift; the enhancement of electron-donating conjugation causes the ³¹P chemical shift to shift toward a high field (Hu WX, et al., Chin Chem Lett 1992, 3(4).). In the present disclosure, when phosphorus is substituted, the chemical shift also follows the above principle. For example, by comparing the chemical shifts of phosphorus at position 1 and phosphorus at position 2 in compound 1M and compound 1-3, it can be determined that the chemical shift of phosphorus at position 2 tends to move toward a higher field. When the hydrogen of the thiol group bound to phosphorus in compound 1-3 is substituted by a benzyl group, the change in the ³¹P chemical shift can be used to infer that the benzyl substitution site in compound A is on the thiol group at position 2.

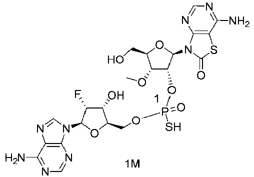

³¹P NMR (162 MHz, DMSO-d₆+D₂O): δ 55.847.

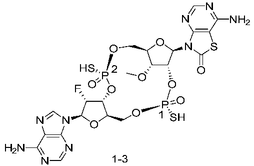

³¹P NMR (DMSO-d₆+D₂O): δ 55.759, 51.401.

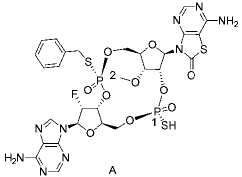

³¹P NMR (DMSO-d₆+D₂O): δ 53.113, 27.573.

The "room temperature" described in the present disclosure refers to 15-30°C.

In the "immune-stimulating antibody conjugates", "compounds", and "pharmaceutically acceptable salts" of the present disclosure, if stereoisomers exist in the substituents R₁, R₂, R₃, R₄, R_{4'}, R₅, R₆, R₇, R₈, R₉, R₁₀, R₁₁, R₁₂, R_{12'}, R₁₃, R_{13'}, R_{A} and L_{1~2}, they may exist in the form of a single stereoisomer or a mixture thereof.

The "pharmaceutically acceptable salt" of the present disclosure is discussed in Berge, et al., "Pharmaceutically acceptable salts", J. Pharm. Sci., 66, 1-19 (1977), and it is obvious to the medicinal chemist that the salts are essentially non-toxic and can provide the desired pharmacokinetic properties, palatability, absorption, distribution, metabolism, excretion, *etc.* The compounds of the present disclosure may have an acidic group, a basic group, or an amphoteric group. Typical pharmaceutically acceptable salts include salts prepared by reacting the compounds of the present disclosure with acids.

On the basis of common knowledge in the art, the above preferable conditions may be arbitrarily combined to obtain various preferable examples of the present disclosure.

The reagents and raw materials used in the present disclosure are all commercially available.

### DETAILED DESCRIPTION OF THE PREFERRED EMBODIMENT

The structures of all compounds of the present disclosure can be identified by nuclear magnetic resonance (¹H NMR) and/or mass spectrometry (MS).

¹H NMR chemical shifts (δ) were recorded in ppm (10⁻⁶). NMR was performed with a Bruker AVANCE-400 spectrometer. Suitable solvents were deuterated chloroform (CDCl₃), deuterated methanol (CD₃OD), and deuterated dimethyl sulfoxide (DMSO-*d₆*), with tetramethylsilane (TMS) as an internal standard.

Liquid chromatography-mass spectrometry (LCMS) was measured by Agilent 1200HPLC/6120 mass spectrometer, using a chromatographic column: Xtimate C18, 3.0 × 50 mm, 3 µm, with a column temperature of 40°C; or by Thermo UltiMate 3000HPLC/MSQ PLUS mass spectrometer using a chromatographic column of XBridge C18, 3.0 × 50 mm, 3.5 µm, with a column temperature of 30°C. Agilent gradient elution condition I: 95% to 5% solvent A₁ and 5% to 95% solvent B₁ (0 to 2.0 minutes), then 95% solvent B₁ and 5% solvent A₁ (held for 1.1 minutes). The percentage was the volume percentage of a specific solvent in the total solvent volume. Solvent A₁: 0.01% trifluoroacetic acid (TFA) aqueous solution; solvent B₁: 0.01% acetonitrile solution of trifluoroacetic acid; the percentage was the volume percentage of the solute in the solution. Thermo gradient elution condition II: 95% to 5% solvent A₂ and 5% to 95% solvent B₂ (0 to 2 minutes), then 95% solvent B₂ and 5% solvent A₂ (held for 1.8 minutes). The percentage was the volume percentage of a specific solvent in the total solvent volume. Solvent A₂: 10 mM ammonium bicarbonate aqueous solution; solvent B₂: acetonitrile.

All compounds of the present disclosure could be separated by preparative high-performance liquid chromatography or flash column chromatography.

Preparative high-performance liquid chromatography (*prep*-HPLC) was performed using an Agela FLEXA-HP preparative liquid chromatograph, with the chromatographic column being: Welch Xtimate C18 10 µm, 21.2 mm × 250 mm. Separation conditions: mobile phase A: acetonitrile, mobile phase B: 10 mmol/L ammonium bicarbonate aqueous solution; detection wavelength: 214 nm & 254 nm; flow rate: 15.0 mL/minute; gradient elution conditions:

| Time (minutes) | Mobile phase A (%) | Mobile phase B (%) |
|---|---|---|
| 0.00 | 0.0 | 100.0 |
| 20.00 | 15.0 | 85.0 |
| 21.00 | 85.0 | 15.0 |
| 24.00 | 85.0 | 15.0 |
| 25.00 | 0.0 | 100.0 |

Flash column chromatography (flash system/CheetahTM) was performed on Agela Technologies MP200, and the accompanying normal phase separation column was Flash column Silica-CS (25 g, 40 g, 80 g, 120 g, or 330 g) from Tianjin Bonna-Agela. The elution system was ethyl acetate/petroleum ether, or dichloromethane/methanol; the reverse phase separation column was a C18 reverse phase column (Spherical C18, 40 to 75 µm, 100 Å, SepaFlash^{®} model: SW-040), and the elution system was acetonitrile/0.1% ammonium bicarbonate aqueous solution.

All compounds of the present disclosure could be analyzed by high-performance liquid chromatography (HPLC) using a Waters e2695 with a 2498 UV/VIS Detector.

Condition 1: chromatographic column: Waters Xselect CHS C18 (4.6 × 150 mm) 5 µm, mobile phase A: methanol, mobile phase B: 10 mm potassium dihydrogen phosphate buffer (the pH value was adjusted to 8.0 with ammonia water); flow rate: 1.0 mL/min; column temperature: 35°C; detection wavelength: 214 nm & 254 nm; gradient elution condition:

| Time (minutes) | Mobile phase A (%) | Mobile phase B (%) |
|---|---|---|
| 0.00 | 5.0 | 95.0 |
| 5.00 | 5.0 | 95.0 |
| 10.00 | 35.0 | 65.0 |
| 14.00 | 60.0 | 40.0 |
| 15.00 | 5.0 | 95.0 |
| 30.00 | 5.0 | 95.0 |

Condition 2: chromatographic column: Waters Xselect CHS C18 (4.6 × 250 mm) 5 µm, mobile phase A: methanol, mobile phase B: 10 mM potassium dihydrogen phosphate buffer (the pH value was adjusted to 8.0 with ammonia water); flow rate: 1.0 mL/min; column temperature: 35°C; detection wavelength: 214 nm & 254 nm; gradient elution condition:

| Time (minutes) | Mobile phase A (%) | Mobile phase B (%) |
|---|---|---|
| 0.00 | 10.0 | 90.0 |
| 5.00 | 25.0 | 75.0 |
| 17.50 | 35.0 | 65.0 |
| 17.60 | 10.0 | 90.0 |
| 23.00 | 10.0 | 90.0 |

Ultra-high performance liquid chromatography (UPLC) employed a Waters H-Class UPLC under the following conditions: chromatographic column: Waters ACQUITY UPLC BEH Shield RP18 2.1 mm × 100 mm, 1.7 µm; mobile phase A: acetonitrile; mobile phase B: 5 mm potassium dihydrogen phosphate buffer (the pH value was adjusted to 2.5 with phosphoric acid); flow rate: 0.4 mL/min; column temperature: 40°C; detection wavelength: 214 nm & 262 nm; gradient elution conditions:

| Time (minutes) | Mobile phase A (%) | Mobile phase B (%) |
|---|---|---|
| 0.00 | 10.0 | 90.0 |
| 5.00 | 40.0 | 60.0 |
| 7.00 | 90.0 | 10.0 |
| 13.00 | 90.0 | 10.0 |
| 13.10 | 10.0 | 90.0 |
| 15.00 | 10.0 | 90.0 |

The abbreviations used in the examples of the present disclosure have the following meanings:

(Boc)₂O: di*-tert*-butyl dicarbonate; BINAP: 2,2'-bis(diphenylphosphino)-1,1'-binaphthyl; DBAD: di-*tert*-butyl azodicarboxylate; DCA: dichloroacetic acid; DDTT: *N,N-*dimethyl-*N*'-(3-thioxo-3*H*-1,2,4-dithiazol-5-yl)formimidamide; DPCP: diphenyl chlorophosphate; DMF: *N,N*-dimethylformamide; DMSO: dimethyl sulfoxide; DIPEA: *N,N-*diisopropylethylamine; EDCI: 1-(3-dimethylaminopropyl)-3-ethylcarbodiimide hydrochloride; EEDQ: 2-ethoxy-1-ethoxycarbonyl-1,2-dihydroquinoline; HATU: O-(7-azabenzotriazol-1-yl)-N,N,N',N'-tetramethyluronium; PyBOP: (benzotriazol-1-yloxy)tripyrrolidinophosphonium hexafluorophosphate; TBSCl: *tert*-butyldimethylsilyl chloride.

### Synthesis of intermediates:

### Synthesis of Linker-1

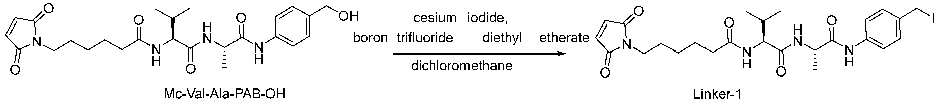

To a solution of Mc-Val-Ala-PAB-OH (200 mg, 0.41 mmol) in dry dichloromethane (10 mL) were sequentially added cesium iodide (127 mg, 0.49 mmol) and boron trifluoride diethyl etherate (45% BF₃, 69 mg, 0.49 mmol) under ice bath conditions. The reaction mixture was stirred at room temperature overnight. Water was added at 0°C to quench the reaction. The aqueous phase was extracted with dichloromethane, and the combined organic phases were dried over anhydrous sodium sulfate, filtered, and the filtrate was concentrated under reduced pressure. The residue was purified by flash column chromatography (petroleum ether/ethyl acetate = 1/1) to obtain Linker-1 (50 mg) as an off-white solid. m/z: [M+H]⁺ 597.0.

### Synthesis of Linker-2

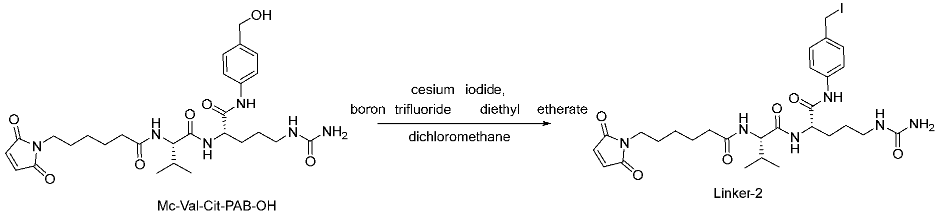

To a solution of Mc-Val-Cit-PAB-OH (260 mg, 0.45 mmol) in dry dichloromethane (10 mL) were sequentially added cesium iodide (140 mg, 0.54 mmol) and boron trifluoride diethyl etherate (45% BF₃, 77 mg, 0.54 mmol) under ice bath conditions. The reaction mixture was stirred at room temperature overnight. The reaction mixture was filtered and concentrated under reduced pressure. The residue was purified by flash column chromatography (dichloromethane/ethyl acetate = 1/1) to obtain Linker-2 (120 mg) as an off-white solid. m/z: [M+H]⁺ 682.5.

### Synthesis of Linker-3

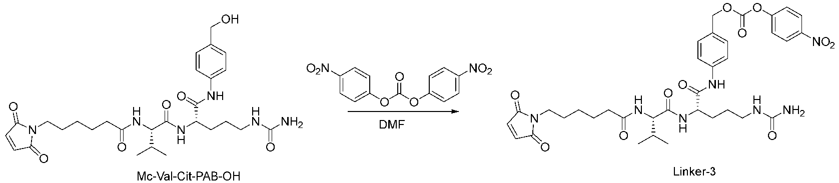

To a solution of compound Mc-Val-Cit-PAB-OH (150 mg, 0.26 mmol) in DMF (4 mL) were sequentially added bis(4-nitrophenyl) carbonate (158 mg, 0.52 mmol) and DIPEA (101 mg, 0.78 mmol). The reaction mixture was stirred at room temperature overnight. The reaction mixture was filtered and concentrated under reduced pressure. The residue was purified by flash column chromatography (C18, eluent: 0-60% acetonitrile in 10 mM ammonium bicarbonate aqueous solution) to obtain Linker-3 (125 mg) as a pale yellow solid. m/z: [M+H]⁺ 738.1.

### Synthesis of Linker-4

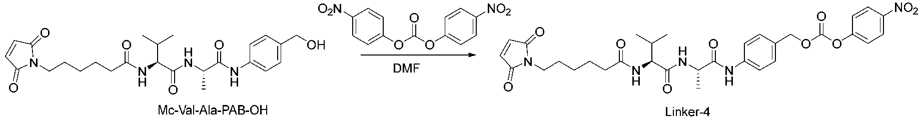

To a solution of Mc-Val-Ala-PAB-OH (120 mg, 0.25 mmol) in DMF (3 mL) were sequentially added bis(4-nitrophenyl) carbonate (152 mg, 0.50 mmol) and DIPEA (97 mg, 0.75 mmol). The reaction mixture was stirred at room temperature overnight. The reaction mixture was filtered and concentrated under reduced pressure. The residue was purified by flash column chromatography (C18, eluent: 0-85% acetonitrile in 10 mM ammonium bicarbonate aqueous solution) to obtain Linker-4 (115 mg) as a pale yellow solid. m/z: [M+H]⁺ 652.1.

### Synthesis of Linker-5

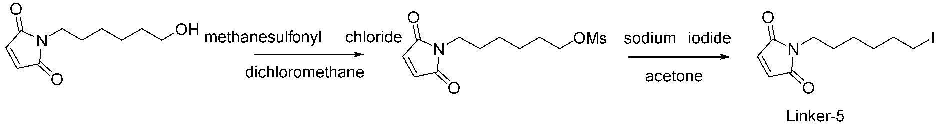

Step 1: To a solution of 1-(6-hydroxyhexyl)-1*H*-pyrrole-2,5-dione (75 mg, 0.38 mmol) in dichloromethane (5 mL) were sequentially added dropwise methanesulfonyl chloride (65 mg, 0.57 mmol) and pyridine (75 mg, 0.95 mmol) under ice bath conditions. The reaction mixture was slowly warmed to room temperature and stirred for an additional 2 hours. Ice water was added to quench the reaction, and the aqueous phase was extracted with dichloromethane. The combined organic phases were washed with saturated brine and concentrated under reduced pressure to obtain 6-(2,5-dioxo-2,5-dihydro-1*H*-pyrrol-1-yl)hexyl methanesulfonate (100 mg). m/z: [M+H]⁺ 276.0.

Step 2: To a solution of the product from Step 1 (90 mg, 0.33 mmol) in acetone (10 mL) was added sodium iodide (250 mg, 1.65 mmol). The reaction mixture was stirred at 50°C for 12 hours. The residue was dissolved in ethyl acetate, washed sequentially with water and saturated brine. The organic phase was separated, dried over anhydrous sodium sulfate, filtered, and the filtrate was concentrated under reduced pressure to obtain Linker-5 (105 mg). m/z: [M+H]⁺ 308.0.

### Synthesis of Linker-6

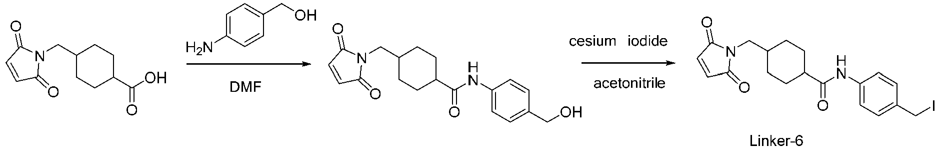

Step 1: To a solution of 4-((2,5-dioxo-2,5-dihydro-1*H*-pyrrol-1-yl)methyl)cyclohexane-1-carboxylic acid (2 g, 8.43 mmol) in *N,N*-dimethylformamide (15 mL) were sequentially added HATU (3.85 g, 10.1 mmol) and DIPEA (2.18 g, 16.9 mmol) under ice bath conditions. The reaction mixture was stirred at room temperature for 5 minutes, then (4-aminophenyl)methanol (1.14 g, 9.27 mmol) was added, and the reaction mixture was stirred at room temperature for an additional 3 hours. Ice water was added to quench the reaction, and the aqueous phase was extracted with ethyl acetate. The combined organic phases were concentrated under reduced pressure to remove most of the organic solvent. Petroleum ether was added to the residue, followed by sonication and filtration. The filter cake was dried to obtain 4-((2,5-dioxo-2,5-dihydro-1*H*-pyrrol-1-yl)methyl)-N-(4-(hydroxymethyl)phenyl)cyclohexane-1-carboxamide (2.8 g). m/z: [M+H]⁺ 343.0.

Step 2: To a solution of the product from Step 1 (500 mg, 1.46 mmol) in acetonitrile (50 mL) were added cesium iodide (610 mg, 2.34 mmol) and boron trifluoride diethyl etherate (310 mg, 2.19 mmol). The reaction mixture was slowly warmed to room temperature and stirred for an additional 12 hours. The reaction mixture was directly concentrated under reduced pressure to obtain Linker-6 (530 mg). m/z: [M+H]⁺ 453.1.

### Synthesis of Linker-8

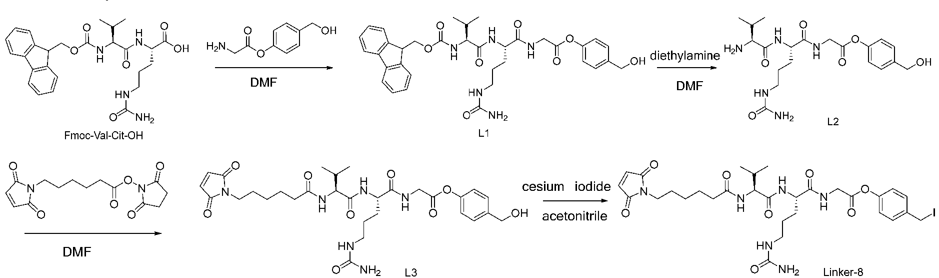

Step 1: To a solution of Fmoc-Val-Cit-OH (480 mg, 0.97 mmol), DIPEA (238 mg, 1.84 mmol), and HATU (420 mg, 1.1 mmol) in DMF (5 mL) was added 4-(hydroxymethyl)phenyl glycinate hydrochloride (200 mg, 0.92 mmol). The reaction mixture was stirred at room temperature for 2 days and directly purified by flash column chromatography (C18, eluent: 0-60% acetonitrile in 10 mM ammonium bicarbonate aqueous solution) to obtain L1 (200 mg) as a white solid. m/z: [M+H]⁺ 660.2.

Step 2: To a solution of L1 (200 mg, 0.3 mmol) in DMF (2 mL) was added diethylamine (350 mg, 4.85 mmol). The reaction mixture was stirred at room temperature for 2 hours and directly purified by flash column chromatography (C18, eluent: 100% 0.1% trifluoroacetic acid in water) to obtain L2 (160 mg) as an off-white solid. m/z: [M+H]⁺ 438.2.

Step 3: To a solution of L2 (160 mg, 0.29 mmol) in DMF (2 mL) were sequentially added DIPEA (45 mg, 0.35 mmol) and N-succinimidyl 6-maleimidohexanoate (98 mg, 0.32 mmol). The reaction mixture was stirred at room temperature for 3 hours and directly purified by flash column chromatography (C18, eluent: 0-60% acetonitrile in 0.1% trifluoroacetic acid aqueous solution) to obtain L3 (20 mg) as an off-white solid. m/z: [M+H]⁺ 631.2.

Step 4: Following the synthetic method of Linker-1, L3 was reacted to obtain Linker-8. m/z: [M+H]⁺ 741.1.

### Synthesis of Linker-9

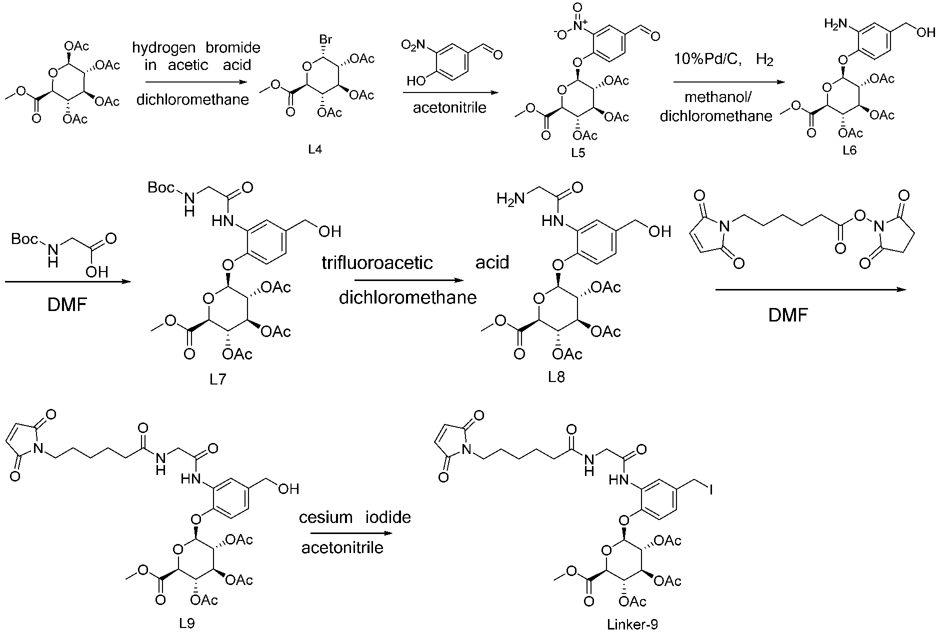

Step 1: To a solution of methyl 1,2,3,4-tetra-O-acetyl-β-D-glucuronate (16 g, 42.5 mmol) in dichloromethane (160 mL) was added dropwise hydrogen bromide in acetic acid (33%, 50 mL) under ice bath conditions. The reaction mixture was stirred at room temperature for 12 hours. The reaction mixture was then concentrated under reduced pressure, and the residue was diluted with dichloromethane. The organic phase was washed with saturated sodium bicarbonate aqueous solution and saturated brine, separated, and concentrated under reduced pressure to obtain L4 (15.9 g) as a white solid. m/z: [M+Na]⁺ 421.1.

Step 2: To a solution of L4 (5.23 g, 13.2 mmol) and 4-hydroxy-3-nitrobenzaldehyde (2 g, 12.0 mmol) in acetonitrile (25 mL) was added silver oxide (4.16 g, 18.0 mmol). The reaction mixture was stirred at room temperature in the dark overnight. The reaction mixture was then filtered through diatomite, and the filtrate was concentrated under reduced pressure. The residue was purified by flash column chromatography (dichloromethane/methanol = 20/1) to obtain L5 (5 g) as a white solid. m/z: [M+Na]⁺ 506.0.

Step 3: L5 (2.3 g, 4.76 mmol) was dissolved in a mixed solution of methanol (10 mL) and dichloromethane (30 mL), then 10% palladium on carbon (400 mg) was added. The reaction system was purged with hydrogen three times and stirred under a hydrogen atmosphere for 5 hours. The reaction mixture was then filtered through diatomite, and the filtrate was concentrated under reduced pressure to obtain L6 (1.5 g) as a white solid. m/z: [M+H]⁺ 456.2.

Step 4: To a solution of Boc-glycine (46 mg, 0.26 mmol) in DMF (5 mL) were added HATU (120 mg, 0.31 mmol), DIPEA (50 mg, 0.39 mmol), and L6 (95 mg, 0.21 mmol), respectively. The reaction mixture was stirred at room temperature for 2 hours. The reaction mixture was directly purified by flash column chromatography (C18, eluent: 0%-75% acetonitrile in 10 mM ammonium bicarbonate aqueous solution) to obtain L7 (160 mg) as a white solid. m/z: [M+H]⁺ 613.2.

Step 5: To a solution of L7 (150 mg, 0.24 mmol) in dichloromethane (3 mL) was added dropwise trifluoroacetic acid (1 mL) under ice bath conditions. The reaction mixture was stirred at room temperature for 1.5 hours. The reaction mixture was directly purified by flash column chromatography (C18, eluent: 0%-50% acetonitrile in 10 mM ammonium bicarbonate aqueous solution) to obtain L8 (110 mg) as a white solid. m/z: [M+H]⁺ 513.2.

Step 6: To a solution of L8 (80 mg, 0.16 mmol) in DMF (5 mL) was added N-succinimidyl 6-maleimidohexanoate (54 mg, 0.18 mmol). The reaction mixture was stirred at room temperature for 2 hours. The reaction mixture was directly purified by flash column chromatography (C18, eluent: 0%-70% acetonitrile in 0.1% trifluoroacetic acid aqueous solution) to obtain L9 (110 mg) as a white solid. m/z: [M+H]⁺ 706.2.

Step 7: To a solution of L9 (100 mg, 0.14 mmol) in acetonitrile (5 mL) were added cesium iodide (58 mg, 0.22 mmol) and boron trifluoride diethyl etherate (36 mg, 0.25 mmol) under ice bath conditions. The reaction mixture was stirred at room temperature for 3 hours and then directly concentrated under reduced pressure to obtain Linker-9 (110 mg). m/z: [M+H]⁺ 816.2.

### Synthesis of Linker-10

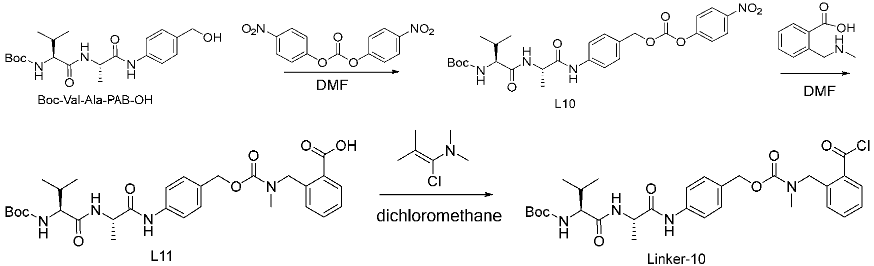

Step 1: A solution of Boc-Val-Ala-PAB-OH (2 g, 5.08 mmol), bis(4-nitrophenyl) carbonate (3.1 g, 10.2 mmol), and DIPEA (1.97 g, 15.2 mmol) in DMF (10 mL) was stirred at room temperature for 3 hours. The reaction mixture was directly purified by flash column chromatography (C18, eluent: 0%-80% acetonitrile in 10 mM ammonium bicarbonate aqueous solution) to obtain L10 (1.6 g). m/z: [M+Na]⁺ 581.2.

Step 2: A solution of L10 (1 g, 1.79 mmol), 2-((methylamino)methyl)benzoic acid (300 mg, 1.79 mmol), and DIPEA (470 mg, 3.64 mmol) in DMF (5 mL) was stirred at room temperature for 3 hours. The reaction mixture was directly purified by flash column chromatography (C18, eluent: 0%-80% acetonitrile in 0.1% trifluoroacetic acid aqueous solution) to obtain L11 (1.01 g). m/z: [M+Na]⁺ 607.2.

Step 3: To a suspension of L11 (1 g, 1.71 mmol) in dichloromethane (20 mL) and tetrahydrofuran (2 mL) was added a solution of Ghosez reagent (449 mg, 3.36 mmol) in dichloromethane (2 mL) under ice bath conditions. The reaction mixture was stirred at 0°C for 1 hour. The reaction mixture was concentrated under reduced pressure to obtain Linker-10 (1.2 g). m/z: [M+H]⁺ 603.4.

### Synthesis of Linker-11

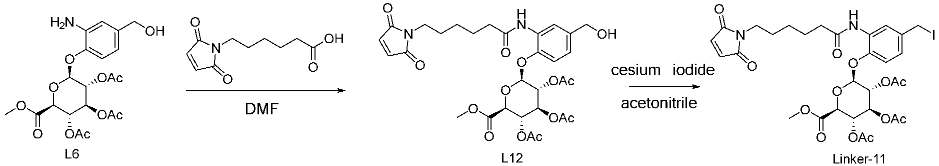

Step 1: To a solution of 6-maleimidohexanoic acid (50 mg, 0.24 mmol) in DMF (3 mL) were sequentially added HATU (110 mg, 0.29 mmol) and DIPEA (62 mg, 0.48 mmol) under ice bath conditions. After stirring for 10 minutes, L6 (87 mg, 0.19 mmol) was added. The reaction mixture was stirred at 0°C for 1 hour and directly purified by flash column chromatography (C18, eluent: 0-60% acetonitrile in 0.1% trifluoroacetic acid aqueous solution) to obtain L12 (110 mg). m/z: [M+H]⁺ 648.5.

Step 2: Following the synthetic method of Linker-1, L12 was reacted to obtain Linker-11. m/z: [M+H]⁺ 759.0.

### Synthesis of Linker-12

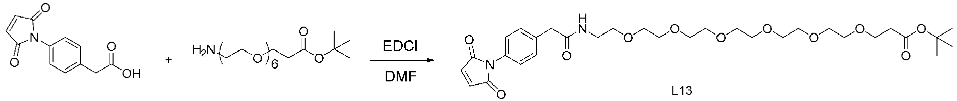

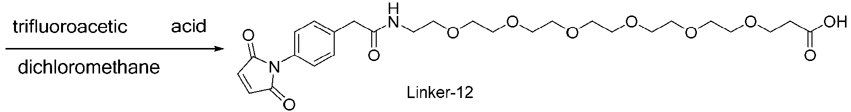

Step 1: To a solution of H2N-PEG6-tBu (266 mg, 0.65 mmol) in DMF (5 mL) were sequentially added EDCI (187 mg, 0.98 mmol) and HOBT (132 mg, 0.98 mmol). The reaction mixture was stirred at room temperature for 5 minutes, then 2-(4-(2,5-dioxo-2,5-dihydro-1*H*-pyrrol-1-yl)phenyl)acetic acid (150 mg, 0.65 mmol) was added. The reaction mixture was stirred at room temperature for an additional 1 hour and directly purified by flash column chromatography (C18, eluent: 0-40% acetonitrile in 0.1% trifluoroacetic acid aqueous solution) to obtain L13 (260 mg) as a pale yellow solid. m/z: [M+H]⁺623.2.

Step 2: To a solution of L13 (130 mg, 0.21 mmol) in dichloromethane (5 mL) was added dropwise trifluoroacetic acid (2 mL) under ice bath cooling. After the addition, the reaction mixture was stirred at room temperature for 2 hours. The reaction mixture was concentrated under reduced pressure, and the residue was purified by flash column chromatography (C18, eluent: 0-45% acetonitrile in 0.1% trifluoroacetic acid aqueous solution) to obtain Linker-12 (66 mg) as a light yellow oil. m/z: [M+H]⁺ 567.2.

### Synthesis of Linker-14

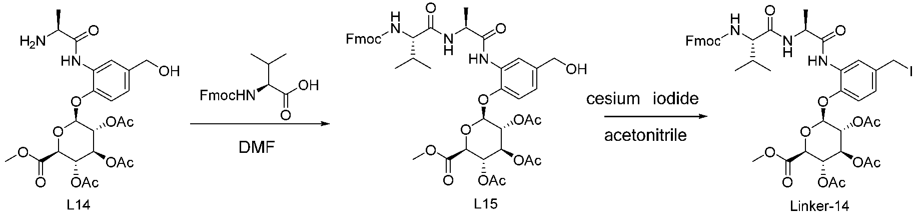

Following the synthetic method of Linker-6, L14 (prepared by the synthetic method for L8, replacing Boc-glycine with Boc-L-alanine in Step 4 to obtain L14) and Fmoc-L-valine were reacted to synthesize Linker-14. m/z: [M+Na]⁺ 980.0.

### Synthesis of Linker-18

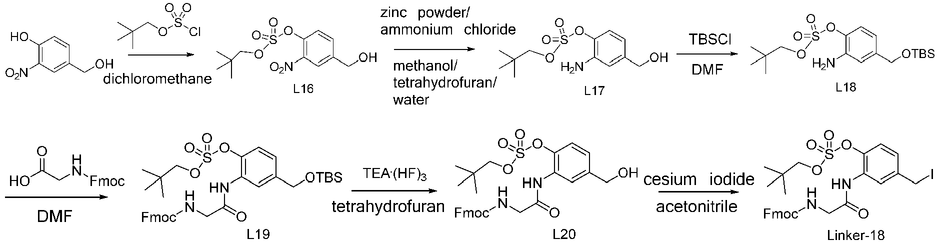

Step 1: To a solution of 4-(hydroxymethyl)-2-nitrophenol (3 g, 17.7 mmol) and 1,8-diazabicyclo[5.4.0]undec-7-ene (3.5 g, 23.1 mmol) in dichloromethane (40 mL) was slowly added dropwise neopentyl sulfurochloridate (4.3 g, 23.1 mmol) under ice bath conditions. The reaction mixture was stirred at room temperature for 6 hours. The reaction was quenched by adding saturated ammonium chloride aqueous solution. The aqueous phase was extracted with ethyl acetate, and the combined organic phases were washed with saturated brine, then concentrated under reduced pressure. The residue was purified by silica gel column chromatography (petroleum ether/ethyl acetate = 100-2/1) to obtain L16 (960 mg) as a yellow oil. m/z: [M+H₂O]⁺ 337.2.

Step 2: To a mixture of L16 (3 g, 9.39 mmol) and ammonium chloride (5.02 g, 93.9 mmol) in methanol (15 mL), tetrahydrofuran (30 mL), and water (30 mL) was slowly added zinc powder (6.14 g, 93.9 mmol) under ice bath conditions. The reaction mixture was stirred at room temperature for 3 hours. The reaction mixture was concentrated under reduced pressure, and the residue was extracted with ethyl acetate. The combined organic phases were concentrated under reduced pressure, and the residue was purified by flash column chromatography (C18, eluent: 0%-60% acetonitrile in 0.1% trifluoroacetic acid aqueous solution) to obtain L17 (1.5 g) as a yellow solid. m/z: [M+H]⁺ 290.1.

Step 3: To a solution of L17 (500 mg, 1.73 mmol) in dichloromethane (15 mL) were added TBSCl (313 mg, 2.08 mmol) and imidazole (153 mg, 2.25 mmol). The reaction mixture was stirred at room temperature for 2 hours, concentrated under reduced pressure, and the residue was purified by flash column chromatography (petroleum ether/ethyl acetate = 4/1) to obtain L18 (650 mg) as a yellow solid. m/z: [M+H]⁺ 404.2.

Step 4: To a solution of L18 (620 mg, 1.54 mmol) and Fmoc-glycine (504 mg, 1.69 mmol) in DMF (5 mL) were sequentially added HATU (761 mg, 2 mmol) and DIPEA (398 mg, 3.08 mmol). The reaction mixture was stirred at room temperature for 16 hours, quenched with water, and the aqueous phase was extracted with ethyl acetate. The combined organic phases were concentrated under reduced pressure. The residue was purified by flash column chromatography (petroleum ether/ethyl acetate = 4/1) to obtain L19 (880 mg) as a pale yellow oil. m/z: [M+H]⁺ 683.2.

Step 5: To a solution of L19 (850 mg, 1.24 mmol) in tetrahydrofuran (10 mL) was added triethylamine trihydrofluoride (1 g, 6.2 mmol) under ice bath conditions. The reaction mixture was stirred at room temperature for 5 hours, and the pH was adjusted to 7 with saturated sodium bicarbonate aqueous solution at 0°C. The mixture was extracted with ethyl acetate, and the combined organic phases were concentrated under reduced pressure. The residue was purified by flash column chromatography (petroleum ether/ethyl acetate = 1/3) to obtain L20 (550 mg) as a colorless oil. m/z: [M+H]⁺ 569.2.

Step 6: To a solution of L20 (200 mg, 0.35 mmol) in acetonitrile (20 mL) were added cesium iodide (164 mg, 0.63 mmol) and boron trifluoride diethyl etherate (1396 mg, 0.98 mmol) under ice bath conditions. The reaction mixture was stirred at room temperature for 48 hours, then directly concentrated under reduced pressure. The residue was purified by flash column chromatography (petroleum ether/ethyl acetate = 1/1) to obtain Linker-18 (140 mg) as a white solid. m/z: [M+H]⁺ 679.0.

### Synthesis of Linker-20

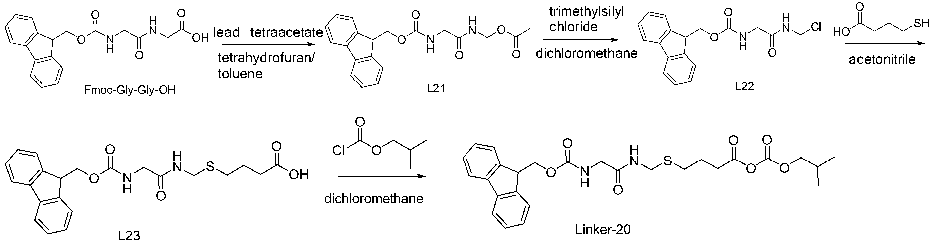

Step 1: To a mixed solution of Fmoc-Gly-Gly-OH (10 g, 28.2 mmol) in tetrahydrofuran (75 mL) and toluene (25 mL) was added lead tetraacetate (17.5 g, 39.5 mmol). The reaction mixture was stirred at 85°C for 2 hours, cooled to room temperature, and filtered. The filtrate was concentrated under reduced pressure. The residue was purified by silica gel column chromatography (petroleum ether/ethyl acetate = 9/1-2/3) to obtain L21 (9.5 g) as a white solid. m/z: [M+Na]⁺ 391.2.

Steps 2 & 3: To a solution of L21 (500 mg, 1.36 mmol) in dichloromethane (10 mL) was added trimethylsilyl chloride (148 mg, 1.36 mmol). The reaction mixture was stirred at room temperature for 1 hour and then concentrated under reduced pressure to obtain L22. L22 was dissolved in acetonitrile (10 mL), and 4-mercaptobutyric acid (160 mg, 1.36 mmol) was added. After stirring at room temperature overnight, the reaction mixture was directly purified by flash column chromatography (C18, eluent: 0-85% acetonitrile in 0.1% trifluoroacetic acid aqueous solution) to obtain L23 (400 mg) as a white solid. m/z: [M+H]⁺ 429.2.

Step 4: To a solution of L23 (390 mg, 0.91 mmol) in dichloromethane (5 mL) were sequentially added triethylamine (100 mg, 1 mmol) and isobutyl chloroformate (140 mg, 1 mmol) under ice bath conditions. The reaction mixture was stirred at 0°C for 1 hour, filtered through a short silica gel column, and the filtrate was concentrated under reduced pressure to obtain Linker-20 (380 mg).

### Synthesis of Linker-21

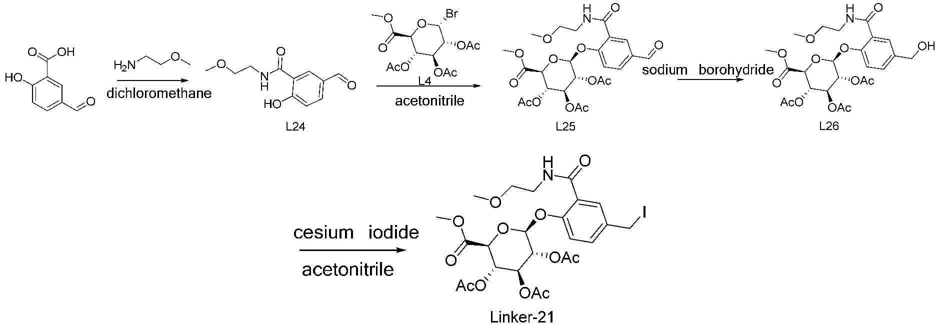

Step 1: To a solution of 5-formyl-2-hydroxybenzoic acid (10 g, 60.2 mmol) in dichloromethane (100 mL) were sequentially added EDCI (17.3 g, 90.3 mmol), HOBT (16.3 g, 120 mmol), DIPEA (15.56 g, 120 mmol), and 2-methoxyethylamine (5.43 g, 72.2 mmol) under ice bath conditions. The reaction mixture was stirred at room temperature for 12 hours and directly purified by flash column chromatography (C18, eluent: 0-65% acetonitrile in 0.1% trifluoroacetic acid aqueous solution) to obtain L24 (7.9 g). m/z: [M+H]⁺ 224.2.

Step 2: To a solution of L24 (1.5 g, 6.72 mmol) in acetonitrile (80 mL) were added L4 (3.09 g, 8.06 mmol) and silver oxide (4.67 g, 20.2 mmol). The reaction mixture was stirred at room temperature for 12 hours, filtered through diatomite, and the filtrate was concentrated under reduced pressure. The residue was purified by flash column chromatography (dichloromethane/methanol = 20/1) to obtain L25 (2.7 g) as a brown foamy solid. m/z: [M+H]⁺ 540.2.

Step 3: To a solution of L25 (2.18 g, 4.05 mmol) in ethanol (10 mL) and tetrahydrofuran (20 mL) was added sodium borohydride (310 mg, 8.08 mmol) under ice bath conditions. The reaction mixture was stirred at 0°C for 2 hours, then quenched with water. The aqueous phase was extracted with ethyl acetate, and the combined organic phases were washed with saturated brine. The organic phase was separated and concentrated under reduced pressure. The residue was purified by flash column chromatography (dichloromethane/methanol = 20/1) to obtain L26 (2.7 g) as a brown foamy solid. m/z: [M+H]⁺ 542.2.

Step 4: Following the synthetic method of Linker-1, L26 was reacted to obtain Linker-21. m/z: [M+H]⁺ 652.2.

### Synthesis of Linker-22

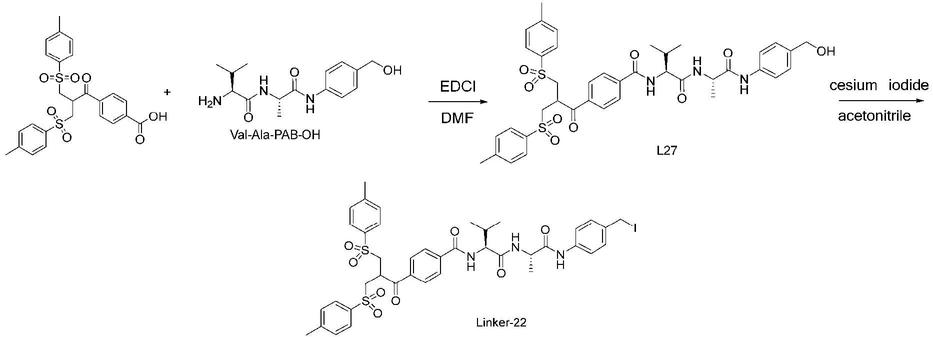

Step 1: To a solution of 4-(3-tosyl-2-(tosylmethyl)propanoyl)benzoic acid (100 mg, 0.2 mmol) in DMF (30 mL) were sequentially added EDCI (58 mg, 0.3 mmol), HOBT (43 mg, 0.32 mmol), and Val-Ala-PAB-OH (59 mg, 0.2 mmol) under ice bath conditions. The reaction mixture was stirred at room temperature for 1 hour and directly purified by flash column chromatography (C18, eluent: 0-75% acetonitrile in 10 mM ammonium bicarbonate aqueous solution) to obtain L27 (100 mg).

Step 2: Following the synthetic method of Linker-1, L27 was reacted to obtain Linker-22. m/z: [M+H]⁺ 886.0.

### Synthesis of Linker-23

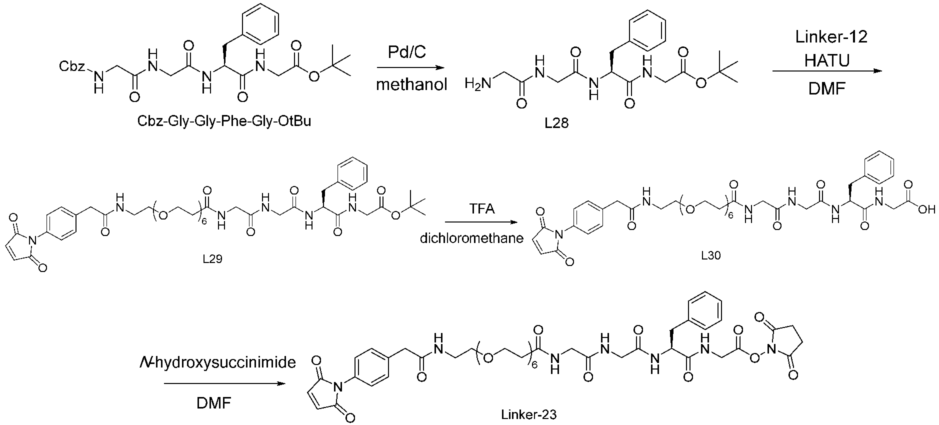

Step 1: To a mixed solution of Cbz-Gly-Gly-Phe-Gly-OtBu (350 mg, 0.66 mmol) in methanol (15 mL) and dichloromethane (5 mL) was added palladium on carbon (70 mg, 10% wt). The reaction system was purged with hydrogen three times and then stirred under a hydrogen atmosphere for 2 hours. The reaction mixture was filtered through diatomite, and the filtrate was concentrated under reduced pressure to obtain L28 (250 mg) as a yellow oil. m/z: [M+H]⁺ 393.2.

Steps 2 & 3: Following the synthetic method of Linker-12, L28 and Linker-12 were reacted to synthesize L30. m/z: [M+H]⁺ 884.4.

Step 4: To a solution of L30 (130 mg, 0.15 mmol) and *N*-hydroxysuccinimide (51.8 mg, 0.45 mmol) in DMF (2 mL) was added *N,N*'-diisopropylcarbodiimide (56.8 mg, 0.45 mmol) under ice bath conditions. The reaction mixture was stirred at room temperature overnight and directly purified by flash column chromatography (C18, eluent: 0%-50% acetonitrile in 0.05% trifluoroacetic acid aqueous solution) to obtain Linker-23 (60 mg) as a yellow solid. m/z: [M+H]⁺ 982.4.

### Synthesis of Linker-24

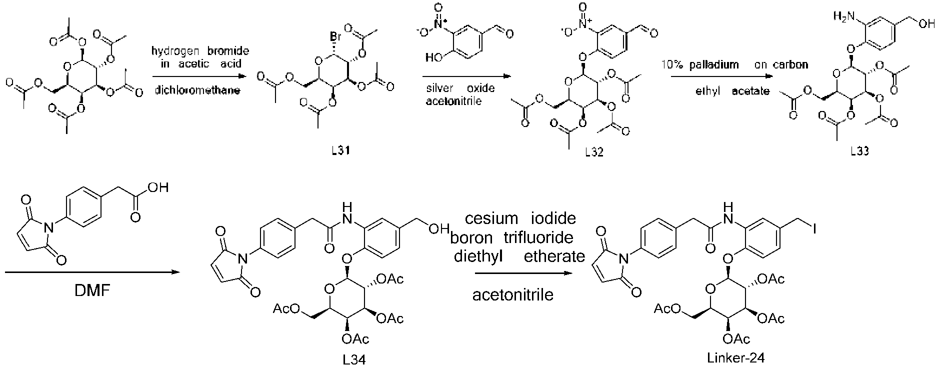

Step 1: To a solution of β-D-galactose pentaacetate (6.20 g, 15.9 mmol) in dichloromethane (60 mL) was added dropwise 33% hydrogen bromide in acetic acid (15 mL) under ice bath conditions. The reaction mixture was stirred at room temperature for 2 hours. The reaction mixture was then poured into ice water (300 mL) and extracted with ice-cold dichloromethane. The combined organic phases were washed with saturated sodium bicarbonate solution, dried over anhydrous sodium sulfate, filtered, and concentrated under reduced pressure to obtain L31 (6.53 g) as a white solid. m/z: [M+Na]⁺ 433.0.

Step 2: To a solution of L31 (6.53 g, 15.9 mmol) and 4-hydroxy-3-nitrobenzaldehyde (2.92 g, 17.5 mmol) in acetonitrile (100 mL) was added silver oxide (13.1 g, 56.4 mmol). The reaction mixture was stirred at room temperature in the dark overnight. The reaction mixture was then filtered through diatomite and concentrated under reduced pressure. The residue was purified by flash column chromatography (dichloromethane/methanol = 20/1) to obtain L32 (7.78 g) as a white solid. m/z: [M+Na]⁺ 520.2.

Step 3: L32 (2 g, 4.02 mmol) was dissolved in ethyl acetate (100 mL), followed by the addition of 10% palladium on carbon (0.50 g) and one drop of triethylamine. The reaction mixture was stirred under a hydrogen balloon overnight. The reaction mixture was then filtered through diatomite and concentrated under reduced pressure to obtain L33 (1.88 g) as a white solid. m/z: [M+H]⁺ 470.2.

Step 4: To a solution of L33 (1 g, 2.13 mmol) in DMF (10 mL) were added EDCI (0.61 g, 3.18 mmol) and HOBT (0.43 g, 3.18 mmol) under ice bath conditions. Then, a solution of 4-maleimidobenzoic acid (0.49 g, 2.12 mmol) in DMF (2 mL) was slowly added dropwise to the above mixture. The reaction mixture was stirred at room temperature overnight. The reaction mixture was directly purified by flash column chromatography (C18, eluent: 0%-52% acetonitrile in 0.05% trifluoroacetic acid aqueous solution) to obtain L34 (442 mg) as a yellow solid. m/z: [M+Na]⁺ 705.2.

Step 5: To a solution of L34 (200 mg, 0.29 mmol) and cesium iodide (151 mg, 0.58 mmol) in acetonitrile (5 mL) was added dropwise boron trifluoride diethyl etherate (82.3 mg, 0.58 mmol) under ice bath conditions. The reaction mixture was stirred at room temperature for 2 hours. The reaction mixture was then filtered and concentrated under reduced pressure. The residue was purified by prep-TLC (petroleum ether/ethyl acetate = 1/1) to obtain Linker-24 (144 mg) as a yellow solid. m/z: [M+Na]⁺ 815.1.

### Synthesis of Linker-26

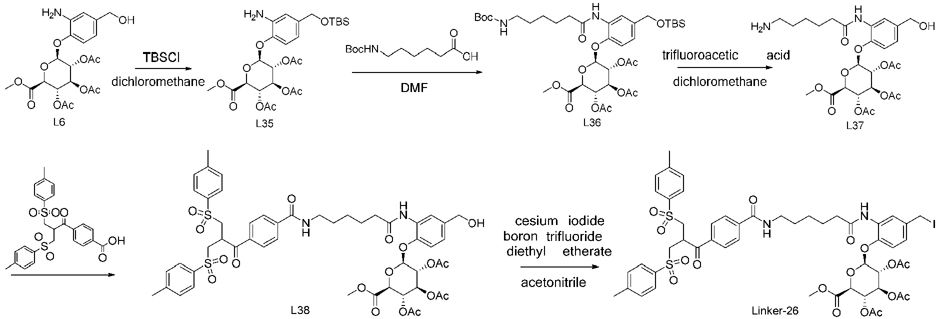

Step 1: To a solution of L6 (800 mg, 1.76 mmol) in dichloromethane (10 mL) were added imidazole (140 mg, 2.11 mmol) and TBSCl (530 mg, 3.52 mmol) under ice bath conditions. The reaction mixture was stirred at room temperature for 1 hour and concentrated under reduced pressure. The residue was purified by flash column chromatography (C18, eluent: 0%-85% acetonitrile in 10 mM ammonium bicarbonate aqueous solution) to obtain L35 (920 mg). m/z: [M+H]⁺ 570.2.

Step 2: To a solution of 6-((tert-butoxycarbonyl)amino)hexanoic acid (100 mg, 0.43 mmol) in DMF (3 mL) were added HATU (210 mg, 0.56 mmol) and DIPEA (110 mg, 0.86 mmol) under ice bath conditions. The reaction mixture was stirred at 0°C for 5 minutes, and then L35 (240 mg, 0.43 mmol) was added to the above system. The reaction mixture was stirred at room temperature for an additional 12 hours and then directly purified by flash column chromatography (C18, eluent: 0-85% acetonitrile in 10 mM ammonium bicarbonate aqueous solution) to obtain L36 (300 mg). m/z: [M+H]⁺ 783.3.

Step 3: A mixed solution of L36 (165 mg, 0.21 mmol) in dichloromethane (2 mL) and trifluoroacetic acid (1.5 mmol) was stirred at room temperature for 1.5 hours, concentrated under reduced pressure, and the residue was purified by flash column chromatography (C18, eluent: 0-65% acetonitrile in 10 mM ammonium bicarbonate aqueous solution) to obtain L37 (115 mg). m/z: [M+H]⁺ 569.2.

Steps 4 & 5: Following the synthetic method of Linker-22, 4-(3-tosyl-2-(tosylmethyl)propanoyl)benzoic acid and L37 were reacted to obtain Linker-26. m/z: [M+H]⁺ 1161.2.

### Synthesis of Linker-27

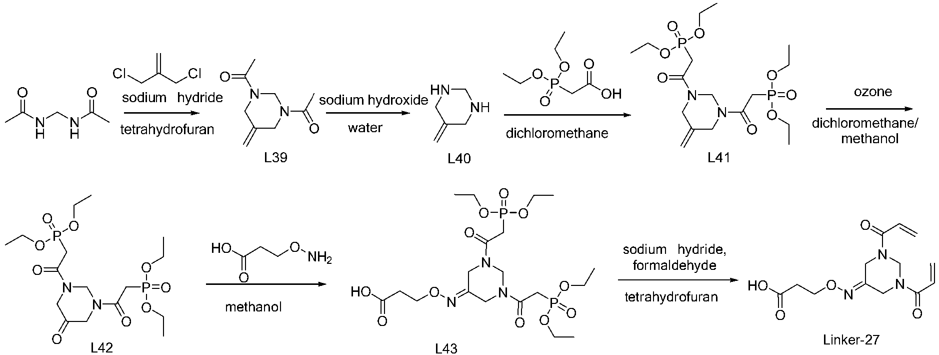

Step 1: To a solution of *N,N*'-methylenediacetamide (20 g, 154 mmol) in anhydrous tetrahydrofuran (1 L) was added sodium hydride (60%, 8.61 g, 215 mmol). The reaction mixture was stirred at room temperature for 10 minutes, followed by the dropwise addition of 3-chloro-2-chloromethylpropene (20.2 g, 161 mmol). The reaction mixture was refluxed for 48 hours, cooled to room temperature, filtered, and the filtrate was concentrated under reduced pressure. The residue was purified by silica gel column chromatography (methanol/dichloromethane = 50/1) to obtain L39 (12 g) as a yellow oil. m/z: [M+H]⁺ 183.3.

Step 2: To a suspension of L39 (4 g, 22.0 mmol) in water (20 mL) was added sodium hydroxide (4.39 g, 110 mmol). The reaction mixture was stirred at 110°C overnight. The reaction mixture was cooled with an ice bath, and solid sodium hydroxide (1.4 g) was added. The aqueous phase was extracted with dichloromethane, and the combined organic phases were dried over anhydrous sodium sulfate, filtered, and the filtrate was concentrated under reduced pressure to obtain crude L40 (4.3 g), which was used directly in the next step.

Step 3: To a solution of L40 (2.15 g, 21.9 mmol) in dichloromethane (40 mL) were sequentially added HOBT (11.8 g, 87.6 mmol), EDCI (16.8 g, 87.6 mmol), diethyl phosphonoacetic acid (14 g, 71.4 mmol), and DIPEA (14.2 g, 110 mmol) under ice bath conditions. The reaction mixture was slowly warmed to room temperature and stirred overnight, then diluted with dichloromethane (20 mL). The organic phase was washed with water, separated, and concentrated under reduced pressure. The residue was purified by flash column chromatography (C18, eluent: 0%-60% acetonitrile in 0.05% trifluoroacetic acid aqueous solution) to obtain L41 (3.5 g) as a colorless oil. m/z: [M+H]⁺ 455.2.

Step 4: At -70°C, ozone was bubbled into a mixed solution of L41 (1 g, 2.2 mmol) in dichloromethane (20 mL) and methanol (5 mL) with stirring for 10 minutes. Excess ozone in the reaction system was purged with nitrogen, and dimethyl sulfide (409 mg, 6.6 mmol) was added. The reaction mixture was stirred and warmed to room temperature, then concentrated under reduced pressure to obtain L42 (1 g) as a yellow oil. m/z: [M+H]⁺ 457.2.

Step 5: To a solution of L42 (500 mg, 1.1 mmol) in methanol (5 mL) were sequentially added 3-(aminooxy)propanoic acid (220 mg, 2.09 mmol) and sodium acetate (451 mg, 5.5 mmol). The reaction mixture was stirred at room temperature overnight, quenched with water, and directly purified by flash column chromatography (C18, eluent: 0%-35% acetonitrile in 0.05% trifluoroacetic acid aqueous solution) to obtain L43 (560 mg) as a pale yellow oil. m/z: [M+H]⁺ 544.2.

Step 6: To a solution of L43 (97 mg, 0.18 mmol) in anhydrous tetrahydrofuran (5 mL) was added sodium hydride (60%, 36 mg, 0.9 mmol) under ice bath conditions. The reaction mixture was stirred at 0°C for 30 minutes, followed by the dropwise addition of a freshly prepared solution of formaldehyde in tetrahydrofuran (1.38 mL, 0.29 M). The resulting reaction mixture was stirred at room temperature for 10 minutes, then quenched with water and directly purified by flash column chromatography (C18, eluent: 0.05% trifluoroacetic acid aqueous solution, 1 minute) to obtain Linker-27 (133 mg) as a yellow oil. m/z: [M+H]⁺ 296.2.

### Synthesis of Linker-32

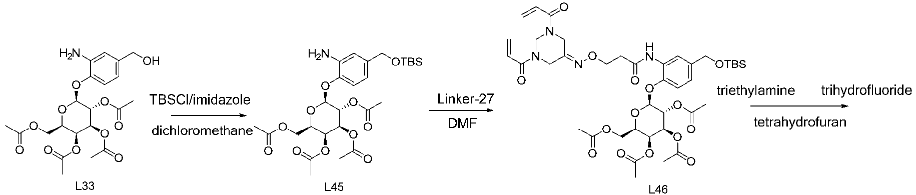

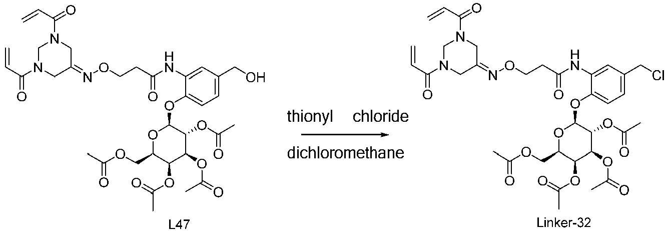

Step 1: To a solution of L33 (1 g, 2.13 mmol) in dichloromethane (10 mL) were added TBSCl (390 mg, 2.56 mmol) and imidazole (190 mg, 2.77 mmol) under ice bath conditions. The reaction mixture was stirred at room temperature for 2 hours, then quenched with water. The aqueous phase was extracted with dichloromethane, and the combined organic phases were dried over anhydrous sodium sulfate, filtered, and the filtrate was concentrated under reduced pressure. The residue was purified by flash column chromatography (ethyl acetate/petroleum ether = 1/3) to obtain L45 (900 mg) as an off-white solid. m/z: [M+H]⁺ 584.2.

Step 2: To a solution of L45 (120 mg, 0.21 mmol) in DMF (3 mL) were sequentially added Linker-27 (124 mg, 0.21 mmol), DIPEA (54 mg, 0.42 mmol), and HATU (120 mg, 0.32 mmol) under ice bath conditions. The reaction mixture was stirred at room temperature for 2 days and directly purified by flash column chromatography (C18, eluent: 0%-75% acetonitrile in 10 mM ammonium bicarbonate aqueous solution) to obtain L46 (10 mg) as an off-white solid. m/z: [M+Na]⁺ 883.4.

Step 3: To a solution of L46 (10 mg, 0.012 mmol) in tetrahydrofuran (1 mL) was added dropwise triethylamine trihydrofluoride (10 mg, 0.06 mmol) under ice bath conditions. The reaction mixture was stirred at room temperature overnight, then quenched with water. The aqueous phase was extracted with ethyl acetate, and the combined organic phases were dried over anhydrous sodium sulfate, filtered, and the filtrate was concentrated under reduced pressure to obtain L47 (9 mg) as an off-white solid. m/z: [M+Na]⁺ 769.2.

Step 4: To a solution of L47 (9 mg, 0.012 mmol) in dichloromethane (2 mL) was added dropwise thionyl chloride (3 mg, 0.024 mmol) under ice bath conditions. The reaction mixture was stirred at room temperature for 2 hours and directly concentrated under reduced pressure to obtain Linker-32 (9 mg) as a yellow solid. m/z: [M+Na]⁺ 787.2.

### Synthesis of Linker-33

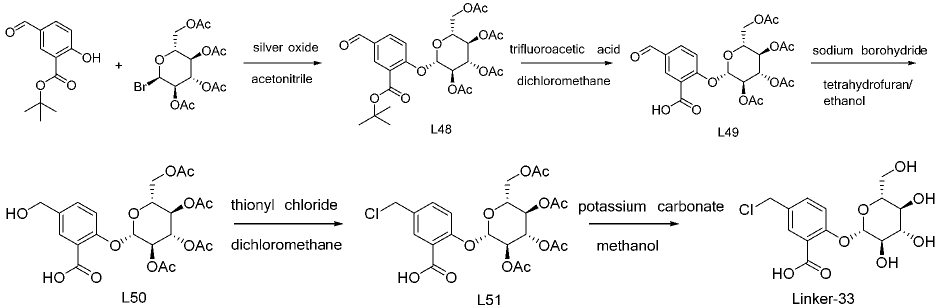

Step 1: To a solution of *tert*-butyl 5-formyl-2-hydroxybenzoate (1 g, 4.50 mmol) in acetonitrile (40 mL) were added acetobromo-α-D-glucose (1.85 g, 4.5 mmol) and silver oxide (3.13 g, 13.5 mmol). The reaction mixture was stirred at room temperature for 12 hours, filtered, and the filtrate was concentrated under reduced pressure. The residue was purified by flash column chromatography (methanol/dichloromethane = 0%-5%) to obtain L48 (2.2 g) as a pale yellow solid. m/z: [M+Na]⁺ 575.2.

Step 2: A mixed solution of L48 (2.2 g, 3.98 mmol) in dichloromethane (10 mL) and trifluoroacetic acid (2.5 mL) was stirred at room temperature for 1 hour. The reaction mixture was diluted with dichloromethane, and a saturated sodium bicarbonate aqueous solution was slowly added to adjust the pH to 6.0-6.5. The aqueous phase was extracted with ethyl acetate, and the combined organic phases were dried over anhydrous sodium sulfate, filtered, and the filtrate was concentrated under reduced pressure to obtain L49 (1.5 g) as a pale yellow solid. m/z: [M+Na]⁺ 519.2.

Step 3: To a mixed solution of L49 (1.5 g, 3.02 mmol) in tetrahydrofuran (5 mL) and ethanol (1 mL) was added sodium borohydride (110 mg, 3.02 mmol) in portions under ice bath conditions. The reaction mixture was stirred at room temperature for 3 hours. Acetone was added to quench the reaction, followed by filtration. The filtrate was concentrated under reduced pressure, and the residue was purified by flash column chromatography (C18, 0%-50% acetonitrile in 0.1% trifluoroacetic acid aqueous solution) to obtain L50 (1.3 g) as a white solid. m/z: [M+Na]⁺ 521.2.

Step 4: To a solution of L50 (500 mg, 1 mmol) in dichloromethane (2 mL) was added thionyl chloride (240 mg, 2 mmol) under ice bath conditions. The reaction mixture was stirred at room temperature overnight. The reaction mixture was concentrated under reduced pressure, and dichloromethane (5 mL) was added to the residue. The solution was concentrated under reduced pressure, and this operation was repeated twice to obtain L51 (500 mg) as a white solid. m/z: [M+Na]⁺ 539.0.

Step 5: To a solution of L51 (260 mg, 0.5 mmol) in methanol (5 mL) was added (69.1 mg, 0.5 mmol). The reaction mixture was stirred at room temperature for 0.5 hours, concentrated under reduced pressure, and directly purified by flash column chromatography (C18, 0%-60% acetonitrile in 0.1% trifluoroacetic acid aqueous solution) to obtain Linker-33 (80 mg) as a white solid. m/z: [M+Na]⁺ 371.0.

### Synthesis of Linker-34

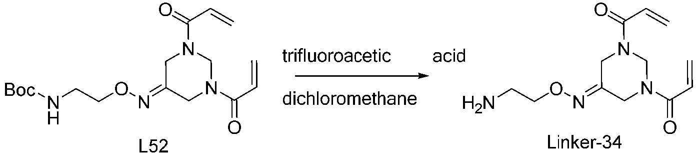

Synthesis of L52: Following the synthetic method of Linker-27, 3-(aminooxy)propanoic acid in Step 5 was replaced with *tert*-butyl (2-(aminooxy)ethyl)carbamate to obtain L52. m/z: [M+Na]⁺ 389.2.

To a solution of L52 (1 g, 2.73 mmol) in dichloromethane (9 mL) was added trifluoroacetic acid (1 mL) under ice bath conditions. The reaction mixture was stirred at room temperature for 1 hour and then directly concentrated under reduced pressure. The residue was purified by flash column chromatography (C18, 0%-60% acetonitrile in 10 mM ammonium bicarbonate aqueous solution) to obtain Linker-34 (400 mg) as a colorless oil. m/z: [M+H]⁺ 267.2.

### Synthesis of Linker-35

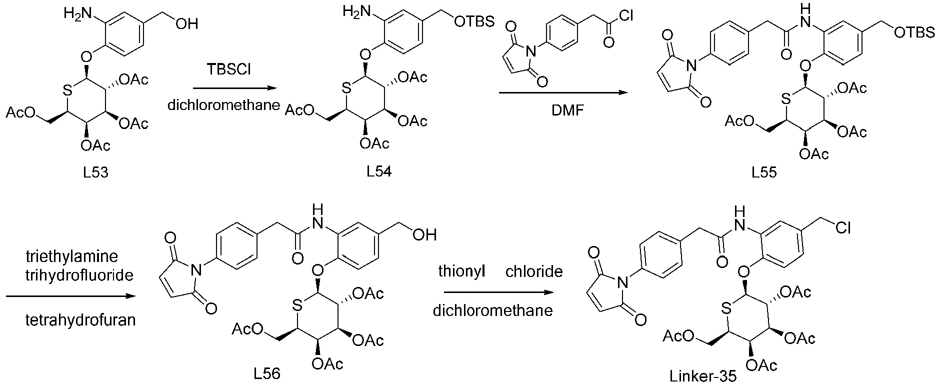

Step 1: To a solution of L53 (prepared by the synthetic method of L33 using 1,2,3,4,6-penta-O-acetyl-5-thio-D-glucopyranose to obtain L53) (400 mg, 0.82 mmol) in dichloromethane (40 mL) were sequentially added TBSCl (160 mg, 1.07 mmol) and imidazole (150 mg, 2.13 mmol) under ice bath conditions. The reaction mixture was stirred at room temperature for 2 hours, then quenched with methanol, and concentrated under reduced pressure at low temperature. The residue was purified by flash column chromatography (C18, 0%-95% acetonitrile in 10 mM ammonium bicarbonate aqueous solution) to obtain L54 (470 mg) as a white solid. m/z: [M+H]⁺ 600.2.

Step 2: To a solution of L54 (300 mg, 0.5 mmol) in DMF (2 mL) were added 2-(4-(2,5-dioxo-2,5-dihydro-1*H*-pyrrol-1-yl)phenyl)acetyl chloride (153 mg, 0.6 mmol) and pyridine (79 mg, 1 mmol) under ice bath conditions. The reaction mixture was stirred at room temperature for 2 hours and directly purified by flash column chromatography (C18, 0%-85% acetonitrile in 10 mM ammonium bicarbonate aqueous solution) to obtain L55 (400 mg) as a white solid. m/z: [M+H]⁺ 813.2.

Step 3: To a solution of L55 (390 mg, 0.31 mmol) in tetrahydrofuran (5 mL) was added dropwise triethylamine trihydrofluoride (0.25 mL, 1.55 mmol) under ice bath conditions. The reaction mixture was stirred at room temperature for 2.5 hours and directly purified by flash column chromatography (C18, 0%-49% acetonitrile in 0.05% trifluoroacetic acid aqueous solution) to obtain L56 (210 mg) as a yellow solid. m/z: [M+H]⁺ 699.2.

Step 4: To a solution of L56 (50 mg, 0.07 mmol) in dichloromethane (10 mL) was added dropwise thionyl chloride (26 mg, 0.22 mmol) under ice bath conditions. The reaction mixture was stirred at room temperature for 3 hours, and concentrated under reduced pressure to obtain Linker-35 (50 mg) as a yellow solid. m/z: [M+Na]⁺ 739.2.

### Synthesis of Linker-38

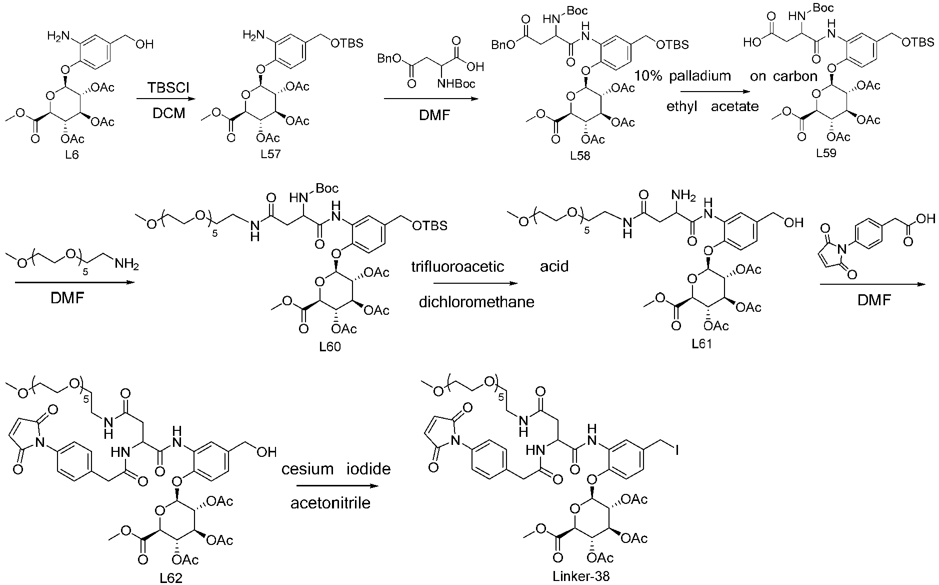

Step 1: To a solution of L6 (500 mg, 1.1 mmol) in dichloromethane (10 mL) were sequentially added imidazole (90 mg, 1.32 mmol) and TBSCl (330 mg, 2.2 mmol) under ice bath conditions. The reaction mixture was stirred at room temperature for 1 hour and concentrated under reduced pressure to remove the solvent. The residue was purified by flash column chromatography (C18, 90% acetonitrile in 10 mM ammonium bicarbonate aqueous solution) to obtain L57 (600 mg) as a white solid. m/z: [M+H]⁺ 570.2.

Step 2: To a solution of L57 (200 mg, 0.62 mmol) in DMF (8 mL) were sequentially added HATU (280 mg, 0.74 mmol), DIPEA (160 mg, 1.24 mmol), and L57 (390 mg, 0.68 mmol) under ice bath conditions. The reaction mixture was stirred at room temperature for 2 hours and directly purified by flash column chromatography (C18, 95% acetonitrile in 10 mM ammonium bicarbonate aqueous solution) to obtain L58 (500 mg) as a white solid. m/z: [M+Na]⁺ 897.3.

Step 3: To a solution of L58 (350 mg, 0.4 mmol) in ethyl acetate (5 mL) was added palladium on carbon (10%, 355 mg). The reaction system was purged with hydrogen and then stirred at room temperature under a hydrogen atmosphere for 2 hours. The reaction mixture was filtered through diatomite, and the filtrate was concentrated under reduced pressure to obtain L59 (300 mg) as a white solid. m/z: [M+H]⁺ 785.0.

Step 4: To a solution of L59 (230 mg, 0.29 mmol) in DMF (10 mL) were sequentially added EDCI (110 mg, 0.58 mmol), HOBT (78 mg, 0.58 mmol), DIPEA (75 mg, 0.58 mmol), and 2,5,8,11,14,17-hexaoxanonadecan-19-amine (86 mg, 0.29 mmol) under ice bath conditions. The reaction mixture was stirred at room temperature for 2 hours and directly purified by flash column chromatography (C18, 95% acetonitrile in 10 mM ammonium bicarbonate aqueous solution) to obtain L60 (300 mg) as a colorless oil. m/z: [M+H]⁺ 1062.5.

Steps 5 & 6: Following the synthetic method of Linker-6, L60 was reacted with 2-(4-(2,5-dioxo-2,5-dihydro-1*H*-pyrrol-1-yl)phenyl)acetic acid to synthesize Linker-38. m/z: [M+H]⁺ 1171.1.

### Synthesis of Linker-39

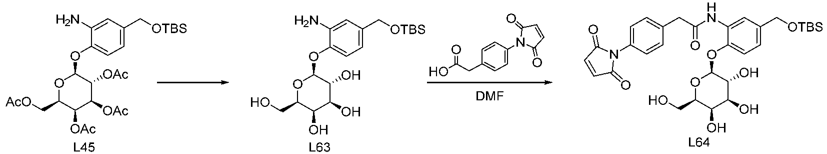

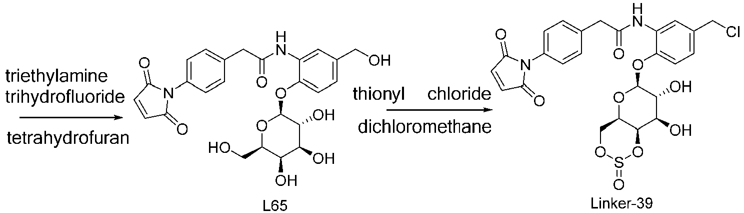

Step 1: To a solution of compound L45 (500 mg, 0.86 mmol) in methanol (15 mL) was added potassium carbonate (0.36 g, 2.58 mmol). The reaction mixture was stirred at room temperature for 1 hour, adjusted to pH = 6-7 with acetic acid, concentrated under reduced pressure at low temperature, and directly purified by flash column chromatography (C18, eluent: 10%-90% acetonitrile in 10 mM ammonium bicarbonate aqueous solution) to obtain L63 (330 mg) as a white solid. m/z: [M+H]⁺ 416.3.

Step 2: To a solution of L63 (180 mg, 0.43 mmol) in DMF (5 mL) were added 2-(4-(2,5-dioxo-2,5-dihydro-1*H*-pyrrol-1-yl)phenyl)acetic acid, HOBT (100 mg, 0.77 mmol), and EDCI (150 mg, 0.77 mmol) under ice bath conditions. The reaction mixture was stirred at room temperature for 2 hours and directly purified by flash column chromatography (C18, eluent: 0%-85% acetonitrile in 10 mM ammonium bicarbonate aqueous solution) to obtain L64 (270 mg) as a white solid. m/z: [M+Na]⁺ 651.2.

Step 3: To a solution of L64 (200 mg, 0.32 mmol) in tetrahydrofuran (5 mL) was added triethylamine trihydrofluoride (150 mg, 0.96 mmol) under ice bath conditions. The reaction mixture was stirred at room temperature for 2 hours and directly purified by flash column chromatography (C18, eluent: 0%-75% acetonitrile in 10 mM ammonium bicarbonate aqueous solution) to obtain L65 (160 mg) as a white solid. m/z: [M+H]⁺ 515.3.

Step 4: To a solution of L65 (30 mg, 0.058 mmol) in dichloromethane (3 mL) were added thionyl chloride (21 mg, 0.17 mmol) and a drop of DIPEA. The reaction mixture was stirred at room temperature for 12 hours and concentrated under reduced pressure to obtain Linker-39 (33 mg) as a pale yellow oil. m/z: [M+H]⁺ 579.0.

### Synthesis of Linker-40

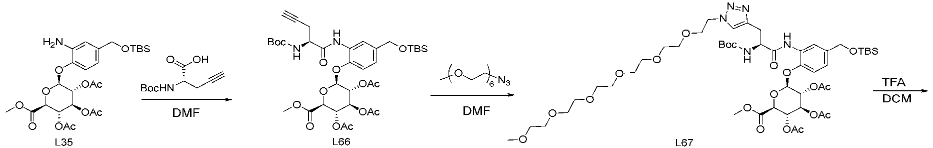

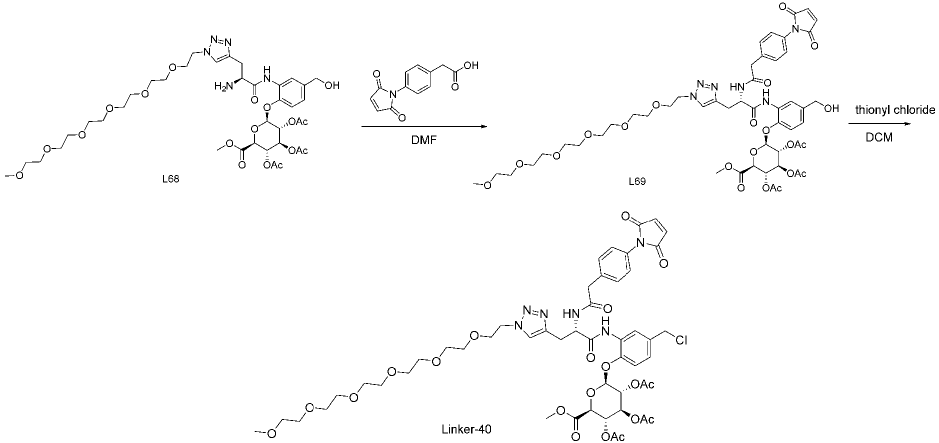

Step 1: To a solution of L35 (1.33 g, 2.34 mmol) and (*S*)-2-((*tert-*butoxycarbonyl)amino)pent-4-ynoic acid (500 mg, 2.34 mmol) in DMF (6 mL) were sequentially added HATU (1.16 g, 3.04 mmol) and DIPEA (756 mg, 5.85 mmol). The reaction mixture was stirred at room temperature for 5 hours, quenched with water, and the aqueous phase was extracted with ethyl acetate. The combined organic phases were concentrated under reduced pressure. The residue was purified by silica gel column chromatography (petroleum ether/ethyl acetate = 1/1) to obtain L66 (1.4 g) as a white solid. m/z: [M+Na]⁺ 787.2.

Step 2: To a solution of L66 (320 mg, 0.42 mmol) and 19-azido-2,5,8,11,14,17-hexaoxanonadecane (148 mg, 0.46 mmol) in DMF (2 mL) were added sodium ascorbate (166 mg, 0.84 mmol), copper(I) iodide (80 mg, 0.42 mmol), and DIPEA (54 mg, 0.42 mmol) under a nitrogen atmosphere. The reaction mixture was stirred at 60°C for 2 hours, cooled to room temperature, quenched with water, and the aqueous phase was extracted with ethyl acetate. The combined organic phases were concentrated under reduced pressure. The residue was purified by flash column chromatography (petroleum ether/ethyl acetate = 1/1) to obtain L67 (400 mg) as a white solid. m/z: [M+H]⁺ 1086.5.

Step 3: To a solution of L67 (347 mg, 0.32 mmol) in dichloromethane (4 mL) was added trifluoroacetic acid (2 mL). The reaction mixture was stirred at room temperature for 2 hours and directly concentrated under reduced pressure. The residue was purified by *prep-*HPLC (mobile phase A: 0.1% trifluoroacetic acid in water, mobile phase B: acetonitrile; gradient elution with mobile phase B from 10%-60%, elution time: 25 minutes) to obtain L68 (280 mg) as a pale yellow solid. m/z: [M+H]⁺ 872.4.

Step 4: To a solution of L68 (200 mg, 0.23 mmol) and 4-maleimidophenylacetic acid (53 mg, 0.23 mmol) in DMF (2 mL) were added HATU (105 mg, 0.28 mmol) and DIPEA (60 mg, 0.46 mmol) under ice bath conditions. The reaction mixture was stirred at 0°C for 2 hours and directly purified by *prep-*HPLC (mobile phase A: 0.1% trifluoroacetic acid in water, mobile phase B: acetonitrile; gradient elution with mobile phase B from 10%-60%, elution time: 20 minutes) to obtain L69 (300 mg) as a yellow solid. m/z: [1/2M+H]⁺ 543.2.

Step 5: To a solution of L69 (60 mg, 0.06 mmol) in dichloromethane (2 mL) was added thionyl chloride (26 mg, 0.22 mmol) under ice bath conditions. The reaction mixture was slowly warmed to room temperature and stirred for an additional 1 hour. The reaction mixture was concentrated under reduced pressure to obtain Linker-40 (60 mg) as a yellow solid. m/z: [M+H]⁺ 1103.4.

### Synthesis of Linker-41 to Linker-44

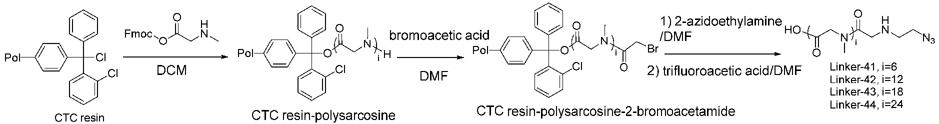

Step 1: To a solution of CTC resin (2 g, 2.2 mmol) in dichloromethane (20 mL) were added Fmoc-sarcosine (1.03 g, 3.30 mmol) and *N,N*-diisopropylethylamine (2.13 g, 16.5 mmol). The reaction mixture was shaken on a shaker for 2 hours, then sequentially washed with DMF and DCM 4 times, followed by methanol 2 times (10 minutes each). The resin was washed twice with a 20% diethylamine/DMF mixed solution (15 minutes each), then washed 4 times with DMF and DCM, after which DMF (30 mL) was added. Subsequently, Fmoc-sarcosine (1.34 g, 4.32 mmol), HATU (1.67 g, 4.4 mmol), and DIPEA (1.4 g, 10.8 mmol) were added sequentially. The reaction mixture was shaken on a shaker for 2 hours, then washed 4 times with DMF and DCM. The resin was washed twice with a 20% diethylamine/DMF mixed solution (15 minutes each time), followed by washing with DMF and DCM 4 times, then dried under reduced pressure. The above steps were repeated until the target i-value CTC resin-polysarcosine was obtained.

Step 2: To a solution of CTC resin-polysarcosine obtained from Step 1 in DMF (30 mL) were added bromoacetic acid (2.45 g, 17.6 mmol) and *N,N'*-diisopropylcarbodiimide (3.6 g, 28.6 mmol). The reaction mixture was shaken on a shaker for 1 hour, washed with DMF 4 times, to obtain CTC resin-polysarcosine-2-bromoacetamide.

Step 3: To a solution of CTC resin-polysarcosine-2-bromoacetamide obtained from Step 2 in DMF (20 mL) were added 2-azidoethylamine (280 mg, 3.3 mmol) and *N,N'-*diisopropylcarbodiimide (430 mg, 3.3 mmol). The reaction mixture was shaken for 2 hours, then washed with DMF and DCM four times, followed by resin cleavage with 1% TFA/DMF mixed solution (twice, 5 minutes each). The solution was concentrated under reduced pressure. The residue was directly purified by *prep-*HPLC (mobile phase A: 0.1% trifluoroacetic acid in water, mobile phase B: acetonitrile; gradient elution with mobile phase B from 10%-35%, elution time: 20 minutes) to obtain Linker-41 to Linker-44 as white solids.
Linker-41, i = 6, m/z: [M+H]⁺571.4.
Linker-42, i = 12, m/z: [M+H]⁺ 997.6.
Linker-43, i = 18, m/z: [1/2M+H]⁺ 712.4.
Linker-44, i = 24, m/z: [1/2M+H]⁺ 925.5.

### Synthesis of Linker-47

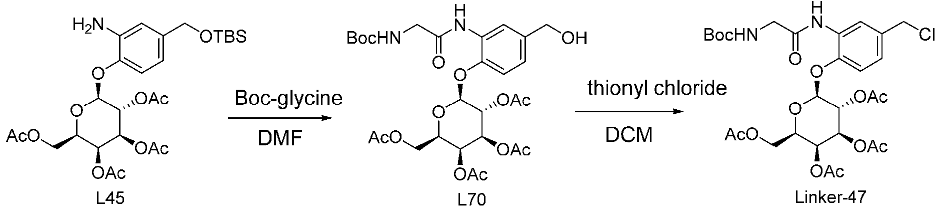

Step 1: To a solution of L45 (150 mg, 0.26 mmol) and Boc-glycine (46 mg, 0.26 mmol) in DMF (3 mL) were added HATU (129 mg, 0.34 mmol) and DIPEA (67 mg, 0.52 mmol). The reaction mixture was stirred at room temperature for 3 hours, then directly purified by *prep-*HPLC (mobile phase A: 0.1% trifluoroacetic acid in water, mobile phase B: acetonitrile; gradient elution with mobile phase B from 10%-95%, elution time: 20 minutes). The collected eluent was mixed with 0.1% trifluoroacetic acid in water (20 mL) and stirred at room temperature. After the reaction was confirmed complete by LCMS, the solution was directly lyophilized to obtain L70 (95 mg) as a white solid. m/z: [M+Na]⁺ 649.2.

Step 2: To a solution of L70 (95 mg, 0.15 mmol) in dichloromethane (2 mL) was added thionyl chloride (36 mg, 0.3 mmol) under ice bath conditions. The reaction mixture was slowly warmed to room temperature and stirred for an additional 1 hour. The reaction mixture was concentrated under reduced pressure to obtain Linker-47 (100 mg) as a white solid. m/z: [M+Na]⁺ 667.2.

Following the above synthetic methods, the Linkers shown in Table 1 were synthesized:

**Table 1**

| Name | Structure | MS Data |
|---|---|---|
| Linker-7 | 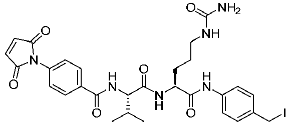 | m/z: [M+H]⁺ 689.2 |
| Linker-13 | 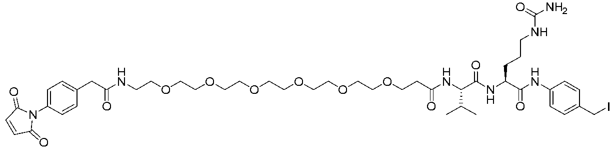 | m/z: [1/2M+H] ⁺ 519.8 |
| Linker-15 | 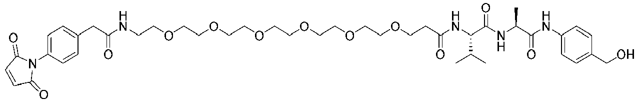 | m/z: [M+Na]⁺ 864.4 |
| Linker-16 | 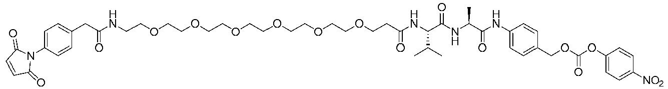 | m/z: [1/2M+H] ⁺ 504.2 |
| Linker-17 | 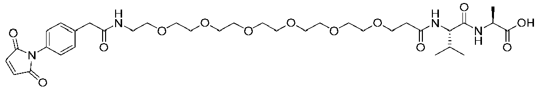 | m/z: [M+H]⁺ 737.4 |
| Linker-19 | 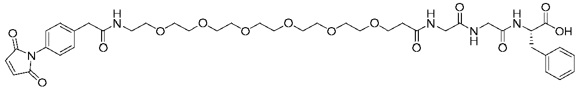 | m/z: [M+H]⁺ 828.4 |
| Linker-25 | 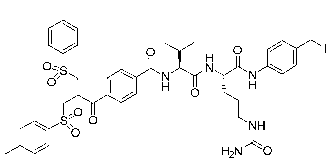 | m/z: [M+H]⁺ 972.0 |
| Linker-28 | 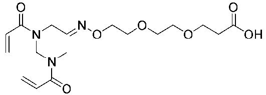 | m/z: [M+H]⁺ 632.2 |
| Linker-29 | 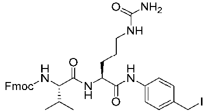 | m/z: [M+H]⁺ 712.2 |
| Linker-30 | 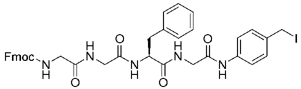 | m/z: [M+H]⁺ 774.2 |
| Linker-31 | 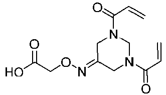 | m/z: [M+H]⁺ 282.2 |
| Linker-36 | 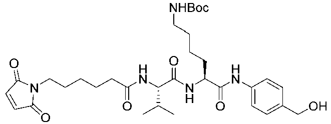 | m/z: [M+Na]⁺ 666.4 |
| Linker-37 | 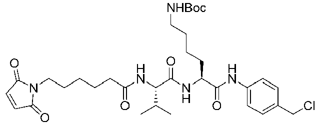 | m/z: [M+Na]⁺ 684.3 |
| Linker-45 | 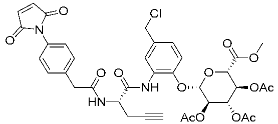 | m/z: [M+H]⁺ 782.4 |
| Linker-46 | 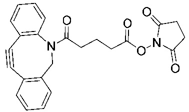 | m/z: [M+H]⁺ 417.2 |
| Linker-48 | 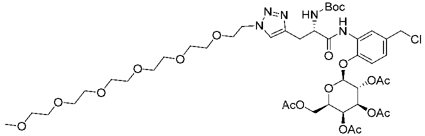 | m/z: [M+H]⁺ 1004.5 |
| Linker-49 | 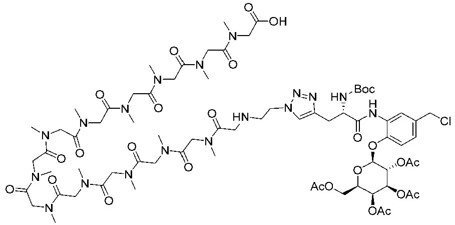 | m/z: [1/2M+H] ⁺ 840.2 |

### Synthesis of tert-butyl (chloromethyl) ethane-1,2-diylbis(methylcarbamate)

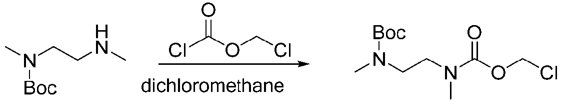

To a solution of *tert*-butyl methyl(2-(methylamino)ethyl)carbamate (1.8 g, 9.56 mmol) in dichloromethane (20 mL) were added pyridine (1.14 g, 14.4 mmol) and chloromethyl chloroformate (1.48 g, 11.5 mmol) under ice bath conditions. The reaction mixture was slowly warmed to room temperature and stirred for an additional 12 hours. Water was added to quench the reaction, and the aqueous phase was extracted with dichloromethane. The organic phase was separated and concentrated under reduced pressure. The residue was purified by flash column chromatography (petroleum ether/ethyl acetate = 1/3) to obtain *tert-*butyl (chloromethyl) ethane-1,2-diylbis(methylcarbamate) (2.3 g).

### Synthesis of 4-((tert-butoxycarbonyl)(methyl)amino)butanoic (isobutyl carbonic) anhydride

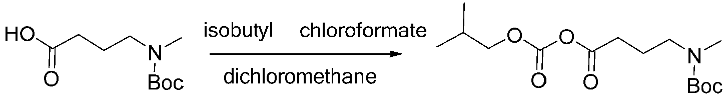

To a solution of 4-((*tert*-butoxycarbonyl)(methyl)amino)butanoic acid (300 mg, 1.38 mmol) and triethylamine (170 mg, 1.70 mmol) in dichloromethane (7 mL) was slowly added isobutyl chloroformate (230 mg, 1.70 mmol) under ice bath conditions. The reaction mixture was stirred at room temperature for 2 hours, concentrated under reduced pressure, and the residue was dissolved in dichloromethane and filtered through a short silica gel column. The filtrate was concentrated under reduced pressure to obtain 4-((*tert-*butoxycarbonyl)(methyl)amino)butanoic (isobutyl carbonic) anhydride (110 mg) as a colorless oil. m/z: [M+Na]⁺ 340.2.

### Synthesis of tert-butyl (S)-2-((((chloromethoxy)carbonyl)(2-(dimethylamino)ethyl)amino)methyl)pyrrolidine-1-carboxylate

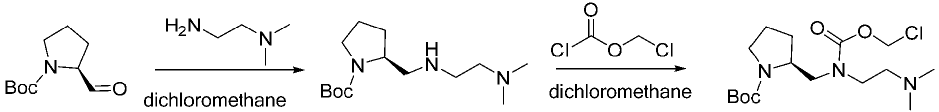

Step 1: To a solution of *tert*-butyl (*S*)-2-acetylpyrrolidine-1-carboxylate (15.8 g, 79.4 mmol) in dichloromethane (20 mL) were sequentially added *N,N*-dimethylethylenediamine (7 g, 79.4 mmol) and sodium triacetoxyborohydride (21 g, 99.3 mmol) under ice bath conditions. The reaction mixture was slowly warmed to room temperature and stirred for an additional 3 hours. Water was added to quench the reaction, and the aqueous phase was extracted with dichloromethane. The combined organic phases were concentrated under reduced pressure, and the residue was purified by flash column chromatography (dichloromethane/methanol = 1/8) to obtain *tert*-butyl (*S*)-2-(((2-(dimethylamino)ethyl)amino)methyl)pyrrolidine-1-carboxylate (900 mg). m/z: [M+H]⁺ 272.2.

Step 2: To a solution of the product obtained from Step 1 (300 mg, 1.11 mmol) in dichloromethane (15 mL) were sequentially added pyridine (180 mg, 2.22 mmol) and chloromethyl chloroformate (190 mg, 1.44 mmol) under ice bath conditions. The reaction mixture was slowly warmed to room temperature and stirred for 12 hours, then quenched with water (15 mL). The aqueous phase was extracted with dichloromethane, and the combined organic phases were concentrated under reduced pressure. The residue was purified by flash column chromatography (dichloromethane/methanol = 1/5) to obtain *tert*-butyl (S)-2-((((chloromethoxy)carbonyl)(2-(dimethylamino)ethyl)amino)methyl)pyrrolidine-1-carboxylate (110 mg). m/z: [M+H]⁺ 364.2.

### Synthesis of 4-((4-((tert-butoxycarbonyl)amino)benzyl)mercapto)butanoic (isobutyl carbonic) anhydride

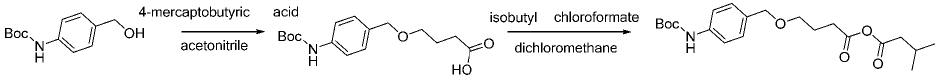

Step 1: To a solution of *tert-*butyl (4-(hydroxymethyl)phenyl)carbamate (2 g, 8.96 mmol) and cesium carbonate (3.72 g, 14.3 mmol) in acetonitrile (40 mL) was slowly added dropwise boron trifluoride diethyl etherate (2.03 g, 14.3 mmol) under ice bath conditions. After the addition, the reaction mixture was stirred at room temperature for 2 hours. To the above reaction mixture was slowly added a solution of 4-mercaptobutyric acid (1.08 g, 8.96 mmol) and DIPEA (3.47 g, 26.9 mmol) in acetonitrile (25 mL). The reaction mixture was stirred at room temperature for an additional 3 hours, concentrated under reduced pressure, and the residue was purified by flash column chromatography (C18, eluent: 0-60% acetonitrile in 0.1% trifluoroacetic acid aqueous solution) to obtain *4-((4-((tert-*butoxycarbonyl)amino)benzyl)oxy)butanoic acid (860 mg). m/z: [M+Na]⁺ 348.2.

Step 2: To a solution of the product obtained from Step 1 (350 mg, 1.08 mmol) and triethylamine (142 mg, 1.4 mmol) in dichloromethane (10 mL) was slowly added isobutyl chloroformate (134 mg, 0.98 mmol) under ice bath conditions. The reaction mixture was stirred at 0°C for 1 hour and then directly concentrated under reduced pressure. Dichloromethane (3 mL) was added to the residue, and the mixture was filtered through a short silica gel column. The filtrate was concentrated under reduced pressure to obtain 4-((4-((*tert-*butoxycarbonyl)amino)benzyl)mercapto)butanoic (isobutyl carbonic) anhydride (500 mg) as a pale yellow oil. m/z: [M+Na]⁺ 448.2.

### Synthesis of 4-((4-((tert-butoxycarbonyl)amino)benzyl)disulfanyl)butanoic acid

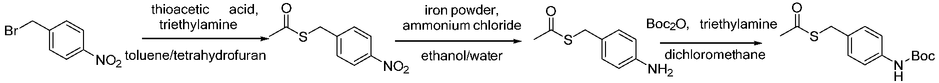

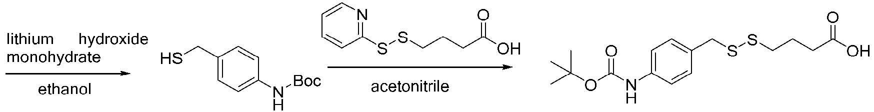

Step 1: To a solution of thioacetic acid (5.3 g, 70 mmol) and triethylamine (8.85 g, 87.5 mmol) in toluene (75 mL) was slowly added a mixed solution of 4-nitrobenzyl bromide (7.56 g, 35 mmol) in toluene (60 mL) and tetrahydrofuran (30 mL). After the addition, the reaction mixture was stirred at room temperature overnight. The reaction mixture was concentrated under reduced pressure, and the residue was redissolved in a small amount of ethyl acetate. Ten volumes of *n*-heptane were added to precipitate solids, which were filtered off. The filtrate was concentrated under reduced pressure, and the residue was purified by flash column chromatography (petroleum ether/ethyl acetate = 3/1) to obtain *S*-(4-nitrophenyl) ethanethioate (7.7 g) as a yellow solid. m/z: [M+H]⁺ 212.0.

Step 2: To ethanol (50 mL) and water (15 mL) were added *S-*(4-nitrophenyl) ethanethioate (2 g, 9.5 mmol), iron powder (2.6 g, 47.4 mmol), and ammonium chloride (2.53 g, 47.4 mmol). The reaction mixture was stirred at 90°C for 1.5 hours, filtered through diatomite, and the filtrate was concentrated under reduced pressure. The residue was purified by flash column chromatography (C18, eluent: 0-30% acetonitrile in 0.1% trifluoroacetic acid aqueous solution) to obtain *S*-(4-aminophenyl) ethanethioate (1.3 g) as a yellow solid. m/z: [M+H]⁺ 182.0.

Step 3: A solution of *S*-(4-aminophenyl) ethanethioate (1.2 g, 6.62 mmol), di-*tert-butyl* dicarbonate (2.89 g, 13.2 mmol), and triethylamine (1.34 g, 13.2 mmol) in dichloromethane (20 mL) was stirred at room temperature overnight. The reaction mixture was concentrated under reduced pressure, and the residue was purified by flash column chromatography (petroleum ether/ethyl acetate = 3/1) to obtain *S*-(4-((*tert*-butoxycarbonyl)amino)phenyl) ethanethioate (840 mg) as a white solid. m/z: [M+Na]⁺ 304.2.

Step 4: To a solution of *S*-(4-((*tert*-butoxycarbonyl)amino)phenyl) ethanethioate (550 mg, 1.95 mmol) and 4-mercaptobutyric acid (9 mg, 0.072 mmol) in ethanol (1 mL) was added lithium hydroxide monohydrate (94 mg, 3.9 mmol) under ice bath conditions. The reaction mixture was stirred at 0°C for 50 minutes, then directly concentrated under reduced pressure. The residue was purified by flash column chromatography (C18, eluent: 0-70% acetonitrile in 0.1% trifluoroacetic acid aqueous solution) to obtain *tert-*butyl (4-(mercaptomethyl)phenyl)carbamate (430 mg) as a white solid. m/z: [M+Na]⁺ 262.2.

Step 5: To a solution of 4-(pyridin-2-yldisulfanyl)butanoic acid (1 g, 4.38 mmol) in acetonitrile (10 mL) was slowly added a solution of *tert-*butyl *(4-*(mercaptomethyl)phenyl)carbamate (420 mg, 1.75 mmol) in acetonitrile (20 mL) over 30 minutes. After the addition, the reaction mixture was stirred at room temperature for 2 hours and then directly purified by flash column chromatography (C18, eluent: 0-80% acetonitrile in 0.1% trifluoroacetic acid aqueous solution) to obtain 4-((4-((*tert-*butoxycarbonyl)amino)benzyl)disulfanyl)butanoic acid (480 mg) as a white solid. m/z: [M+Na]⁺ 380.0.

### Synthesis of 5-((4-(chloromethyl)phenoxy)methyl)-1-methyl-2-nitro-1H-imidazole

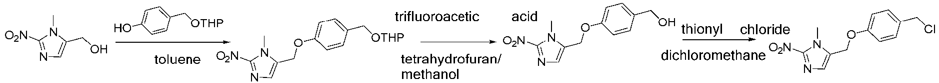

Step 1: A solution of (1-methyl-2-nitro-1*H*-imidazol-5-yl)methanol (400 mg, 2.55 mmol), 4-(((tetrahydro-2*H*-pyran-2-yl)oxy)methyl)phenol (930 mg, 4.47 mmol), triphenylphosphine (1.34 g, 5.1 mmol), and DBAD (1.17 g, 5.1 mmol) in toluene (10 mL) was stirred at 50°C for 5 hours. The reaction mixture was concentrated under reduced pressure, and the residue was purified by flash column chromatography (ethyl acetate/petroleum ether = 1/3) to obtain 1-methyl-2-nitro-5-((4-(((tetrahydro-2*H*-pyran-2-yl)oxy)methyl)phenoxy)methyl)-1*H*-imidazole (600 mg, 60%). m/z: [M+Na]⁺ 370.2.

Step 2: To a mixed solution of the product obtained from Step 1 (600 mg, 1.51 mmol) in tetrahydrofuran (2 mL) and methanol (2 mL) was added trifluoroacetic acid (1.5 mL). The reaction mixture was stirred at room temperature overnight, then directly concentrated under reduced pressure. The residue was purified by flash column chromatography (C18, eluent: 0-50% acetonitrile in 10 mM ammonium bicarbonate aqueous solution) to obtain (4-((1-methyl-2-nitro-1*H*-imidazol-5-yl)methoxy)phenyl)methanol (260 mg). m/z: [M+H]⁺ 264.2.

Step 3: To a mixed solution of the product obtained from Step 2 (20 mg, 0.076 mmol) in dichloromethane (5 mL) was added thionyl chloride (90 mg, 0.076 mmol) under ice bath conditions. The reaction mixture was stirred at 0°C for 1 hour, then directly concentrated under reduced pressure to obtain 5-((4-(chloromethyl)phenoxy)methyl)-1-methyl-2-nitro-1*H-*imidazole (20 mg). m/z: [M+H]⁺ 282.2.

### Synthesis of (1-methyl-2-nitro-1H-imidazol-5-yl)methyl (4-nitrophenyl) carbonate

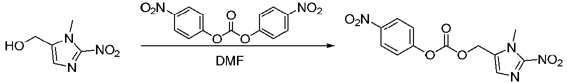

A solution of (1-methyl-2-nitro-1*H*-imidazol-5-yl)methanol (225 mg, 1.43 mmol), DIPEA (554 mg, 4.29 mmol), and bis(4-nitrophenyl) carbonate (870 mg, 2.86 mmol) in DMF (2 mL) was stirred at room temperature for 2 hours. The reaction mixture was directly purified by flash column chromatography (C18, 0%-65% acetonitrile in 0.05% trifluoroacetic acid aqueous solution) to obtain (1-methyl-2-nitro-1*H*-imidazol-5-yl)methyl (4-nitrophenyl) carbonate (350 mg) as a white solid. m/z: [M+H]⁺ 323.0.

### Synthesis of 5-(chloromethyl)-1,4-dimethyl-2-nitro-1H-imidazole

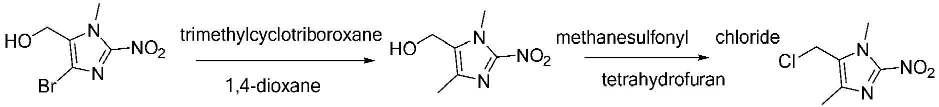

Step 1: To a solution of compound 1.1 (400 mg, 1.69 mmol) in 1,4-dioxane (5 mL) were added 1,1'-bis(diphenylphosphino)ferrocene-palladium(II)dichloride dichloromethane complex (414 mg, 0.51 mmol) and cesium carbonate (1.10 g, 3.38 mmol) under a nitrogen atmosphere. After the addition, the reaction system was purged with nitrogen three times, followed by the addition of trimethylcyclotriboroxane (0.97 mL, 3.38 mmol). The reaction system was purged with nitrogen three times again, sealed, and stirred at 100°C for 3 hours. The reaction mixture was then cooled to room temperature, filtered through diatomite, and the filtrate was concentrated under reduced pressure. The residue was purified by flash column chromatography (C18, eluent: 0%-23% acetonitrile in 0.05% trifluoroacetic acid aqueous solution) to obtain (1,4-dimethyl-2-nitro-1*H*-imidazol-5-yl)methanol (80.7 mg) as a yellow solid. m/z: [M+H]⁺ 172.2.

Step 2: To a solution of (1,4-dimethyl-2-nitro-1*H*-imidazol-5-yl)methanol (27.4 mg, 0.16 mmol) in tetrahydrofuran (2 mL) was added DIPEA (51.7 mg, 0.40 mmol) under ice bath conditions, followed by the dropwise addition of methanesulfonyl chloride (40.3 mg, 0.35 mmol). The reaction mixture was stirred at room temperature for 1 hour. The reaction mixture was then cooled to 0°C, diluted with ethyl acetate (20 mL), washed with hydrochloric acid (1 M, 20 mL) and saturated brine, dried over anhydrous sodium sulfate, filtered, and concentrated under reduced pressure at low temperature to obtain 5-(chloromethyl)-1,4-dimethyl-2-nitro-1*H*-imidazole (28.0 mg) as a yellow oil. m/z: [M+H]⁺ 190.0.

### Synthesis of (E)-4-((4-(chloromethyl)phenyl)diazenyl)-2-hydroxybenzoic acid

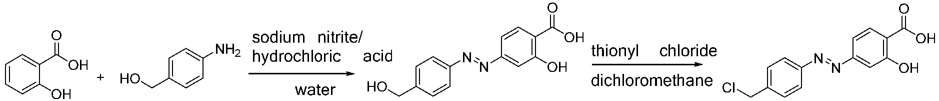

Step 1: To a solution of (4-aminophenyl)methanol in water (100 mL) were added sodium nitrite (2.1 g, 30.5 mmol) and hydrochloric acid (6 g, 165 mmol). The reaction mixture was stirred at 0°C for 20 minutes. The above reaction mixture was then added to a solution of 2-hydroxybenzoic acid (3.08 g, 22.3 mmol), sodium carbonate (3.23 g, 30.5 mmol), and sodium hydroxide (1.06 g, 26.4 mmol) in water (100 mL). The reaction mixture was stirred at 0°C for an additional 1 hour, adjusted to pH = 3-4 with hydrochloric acid (1 M), and the aqueous phase was extracted with ethyl acetate. The combined organic phases were concentrated under reduced pressure. The residue was purified by flash column chromatography (C18, eluent: 0%-85% acetonitrile in 0.1% trifluoroacetic acid aqueous solution) to obtain (*E*)-2-hydroxy-4-((4-(hydroxymethyl)phenyl)diazenyl)benzoic acid (500 mg) as a yellow solid. m/z: [M+H]⁺ 273.2.

Step 2: To a solution of (*E*)-2-hydroxy-4-((4-(hydroxymethyl)phenyl)diazenyl)benzoic acid (35 mg, 0.13 mmol) in dichloromethane (5 mL) was added thionyl chloride (39 mg, 0.33 mmol) under ice bath conditions. The reaction mixture was slowly warmed to room temperature and stirred for an additional 1 hour, then concentrated under reduced pressure to obtain (E)-4-((4-(chloromethyl)phenyl)diazenyl)-2-hydroxybenzoic acid (36 mg) as a yellow solid. m/z: [M+H]⁺ 291.0.

### Synthesis of N-(5-(chloromethyl)-2-((1-methyl-2-nitro-1H-imidazol-5-yl)methoxy)phenyl)-2,5,8,11,14,17,20,23-octaoxahexacosan-26-amine

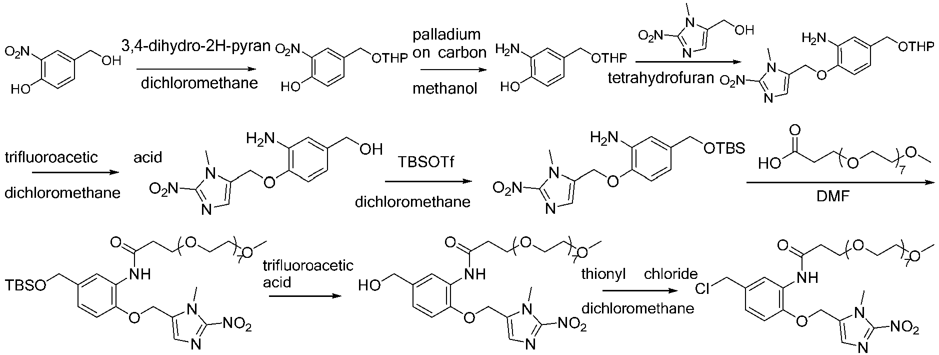

Step 1: To a solution of 4-hydroxy-3-nitrobenzyl alcohol (2.0 g, 11.8 mmol) in dichloromethane (100 mL) were added 3,4-dihydro-2*H*-pyran (1.02 g, 12.2 mmol) and pyridinium *p*-toluenesulfonate (0.3 g, 1.18 mmol). The reaction mixture was stirred at room temperature for 16 hours. The reaction mixture was then washed with saturated sodium bicarbonate aqueous solution and water, dried over anhydrous sodium sulfate, filtered, and concentrated under reduced pressure. The residue was purified by flash column chromatography (petroleum ether/ethyl acetate = 4/1) to obtain 2-nitro-4-(((tetrahydro-2*H-*pyran-2-yl)oxy)methyl)phenol (2.89 g) as a yellow oil. m/z: [M+Na]⁺ 276.1.

Step 2: To a solution of the product from Step 1 (2.89 g, 11.4 mmol) in methanol (150 mL) was added palladium on carbon (10%, 364 mg) under a nitrogen atmosphere. The reaction system was then purged with hydrogen three times, and the reaction mixture was stirred at room temperature under a hydrogen atmosphere for 2 hours. The reaction mixture was then filtered through diatomite and concentrated under reduced pressure to obtain 2-amino-4-(((tetrahydro-2*H*-pyran-2-yl)oxy)methyl)phenol (2.54 g) as a gray-black oil. m/z: [M+Na]⁺ 246.2.

Step 3: To a solution of 3-methyl-2-nitroimidazole-4-methanol (1.0 g, 6.36 mmol), the product from Step 2 (1.70 g, 7.63 mmol), and triphenylphosphine (2.50 g, 9.54 mmol) in tetrahydrofuran (10 mL) was added di-*tert*-butyl azodicarboxylate (2.20 g, 9.54 mmol) under ice bath conditions. The reaction mixture was stirred at room temperature for 2 hours. The reaction mixture was directly purified by flash column chromatography (C18, eluent: 0%-25% acetonitrile in 0.1% trifluoroacetic acid aqueous solution) to obtain 2-((1-methyl-2-nitro-1*H-*imidazol-5-yl)methoxy)-5-(((tetrahydro-2*H*-pyran-2-yl)oxy)methyl)aniline (0.51 g) as a yellow solid. m/z: [M+Na]⁺ 385.2.

Step 4: To a solution of the product from Step 3 (510 mg, 1.41 mmol) in dichloromethane (6 mL) was added dropwise trifluoroacetic acid (2 mL) under ice bath conditions. The reaction mixture was stirred at room temperature for 1 hour. The reaction mixture was concentrated under reduced pressure and purified by flash column chromatography (C18, eluent: 0%-42% acetonitrile in 10 mM ammonium bicarbonate aqueous solution) to obtain (3-amino-4-((1-methyl-2-nitro-1*H*-imidazol-5-yl)methoxy)phenyl)methanol (391 mg) as a yellow solid. m/z: [M+H]⁺ 279.2.

Step 5: To a solution of the product from Step 4 (280 mg, 1.01 mmol) in dichloromethane (10 mL) were added *tert*-butyldimethylsilyl trifluoromethanesulfonate (320 mg, 1.21 mmol) and imidazole (89.4 mg, 1.31 mmol) under ice bath conditions. The reaction mixture was stirred at room temperature for 10 minutes. The reaction mixture was then washed with water, and the aqueous phase was extracted with dichloromethane. The combined organic phases were dried over anhydrous sodium sulfate, filtered, and concentrated under reduced pressure. The residue was purified by flash column chromatography (petroleum ether/ethyl acetate = 2/1) to obtain 5-(((*tert*-butyldimethylsilyl)oxy)methyl)-2-((1-methyl-2-nitro-1*H*-imidazol-5-yl)methoxy)aniline (233 mg) as a yellow solid. m/z: [M+Na]⁺ 415.2.

Steps 6 & 7: To a solution of 4,7,10,13,16,19,22,25-octaoxahexacosanoic acid (65.8 mg, 0.16 mmol) in DMF (3 mL) were added HATU (79.1 mg, 0.21 mmol) and DIPEA (41.4 mg, 0.32 mmol) under ice bath conditions. The reaction mixture was stirred at 0°C for 5 minutes. The product from Step 5 (60 mg, 0.15 mmol) was then added to the above reaction system, and the resulting mixture was slowly warmed to room temperature and stirred for an additional 16 hours. The reaction mixture was directly purified by flash column chromatography (C18, eluent: 0%-64% acetonitrile in 0.05% trifluoroacetic acid aqueous solution). The collected solution was stirred at room temperature for 4.5 hours and then lyophilized to obtain *N*-(5-(hydroxymethyl)-2-((1-methyl-2-nitro-1*H*-imidazol-5-yl)methoxy)phenyl)-2,5,8,11,14,17,20,23-octaoxahexacosan-26-amine (88.7 mg) as a yellow oil. m/z: [M+H]⁺ 673.4.

Step 8: To a solution of the product from Step 7 (21.7 mg, 0.032 mmol) in dichloromethane (3 mL) was added dropwise thionyl chloride (7.6 mg, 0.064 mmol) under ice bath conditions. The reaction mixture was stirred at 0°C for 1 hour. The reaction mixture was concentrated under reduced pressure at low temperature to obtain *N*-(5-(chloromethyl)-2-((1-methyl-2-nitro-1*H*-imidazol-5-yl)methoxy)phenyl)-2,5,8,11,14,17,20,23-octaoxahexacosan-26-amine (22.3 mg) as a yellow oil. m/z: [M+H]⁺ 691.2.

### Synthesis of (2R,3R,4R,5R,6R)-5-acetamido-2-(acetoxymethyl)-6-azidotetrahydro-2H-pyran-3,4-diyl diacetate

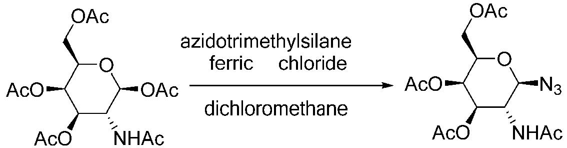

To a solution of β-D-galactosamine pentaacetate (1.0 g, 2.57 mmol) in dichloromethane (10 mL) were added ferric chloride (0.08 g, 0.51 mmol) and azidotrimethylsilane (0.44 g, 3.85 mmol) under a nitrogen atmosphere. After the addition, the reaction system was purged with nitrogen three times, and then the reaction mixture was stirred at room temperature for 72 hours. The reaction mixture was sequentially washed with saturated sodium bicarbonate aqueous solution and saturated brine, dried over anhydrous sodium sulfate, filtered, and the filtrate was concentrated under reduced pressure. The residue was purified by flash column chromatography (C18, eluent: 0%-39% acetonitrile in 0.02% trifluoroacetic acid aqueous solution) to obtain (2*R*,3*R,*4R,5R,6R)-5-acetamido-2-(acetoxymethyl)-6-azidotetrahydro-2*H*-pyran-3,4-diyl diacetate (0.52 g) as a white solid. m/z: [M+H]⁺ 373.2.

### Synthesis of (2R,3R,4S,5R,6R)-2-(acetoxymethyl)-6-(((2R,3R,4S,5R,6R)-3,4,5-triacetoxy-6-(azidomethyl)tetrahydro-2H-pyran-2-yl)oxy)tetrahydro-2H-pyran-3,4,5-triyl triacetate and (2R,3S,4S,5R,6R)-2-(azidomethyl)-6-(((2R,3R,4S,5S,6R)-3,4,5-trihydroxy-6-(hydroxymethyl)tetrahydro-2H-pyran-2-yl)oxy)tetrahydro-2H-pyran-3,4,5-triol

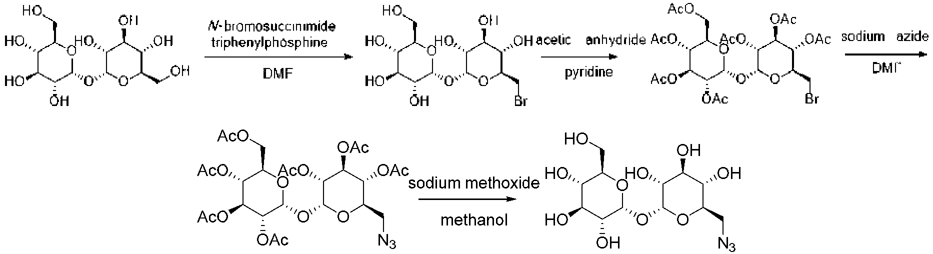

Step 1: To a solution of trehalose (10 g, 29.2 mmol) in DMF (100 mL) was added triphenylphosphine (15.3 g, 58.4 mmol) under a nitrogen atmosphere. The reaction mixture was stirred at room temperature for 15 minutes. Under ice bath conditions, N-bromosuccinimide (6.76 g, 38 mmol) was added to the above solution, and the reaction mixture was stirred at 80°C for 8 hours. The reaction mixture was then cooled to room temperature, quenched with methanol, and concentrated under reduced pressure to remove the solvent. Water (100 mL) was added to dissolve the residue, and the aqueous phase was back-extracted with dichloromethane. The aqueous phase was concentrated under reduced pressure to obtain (2*S,*3*S,*4*S,*5*R,*6*R*)-2-(bromomethyl)-6-(((2*R,*3*R,*4*S,*5*S,*6*R*)-3,4,5-trihydroxy-6-(hydroxymethyl)tetrahydro-2*H*-pyran-2-yl)oxy)tetrahydro-2*H*-pyran-3,4,5-triol (11.8 g) as a yellow transparent solid. m/z: [M+Na]⁺ 427.0.

Step 2: To a solution of (2*S*,3*S*,4*S*,5*R*,6*R*)-2-(bromomethyl)-6-(((2*R*,3*R*,*4*S,5*S*,6*R*)-3,4,5-trihydroxy-6-(hydroxymethyl)tetrahydro-2*H*-pyran-2-yl)oxy)tetrahydro-2*H*-pyran-3,4,5-triol (11.8 g, 29.2 mmol) in pyridine (110 mL) was added dropwise acetic anhydride (37 mL, 395 mmol). The reaction mixture was stirred at room temperature overnight. The reaction mixture was concentrated under reduced pressure, and the residue was dissolved in ethyl acetate (100 mL). The solution was successively washed with saturated sodium bicarbonate aqueous solution and saturated brine, dried over anhydrous sodium sulfate, filtered, and the filtrate was concentrated under reduced pressure to obtain (2*R,*3*R,*4*S,*5*R,*6*R*)*-*2-(acetoxymethyl)-6-(((2*R*,3*R*,4*S*,5*S*,6*S*)-3,4,5-triacetoxy-6-(bromomethyl)tetrahydro-2*H-*pyran-2-yl)oxy)tetrahydro-2*H-*pyran-3,4,5-triyl triacetate (17.4 g) as a yellow solid. m/z: [M+Na]⁺ 721.2.

Step 3: To a solution of (2*R*,3*R*,4*S*,5*R*,6*R*)-2-(acetoxymethyl)-6-(((2*R*,3*R*,4*S*,5*S*,6*S*)-3,4,5-triacetoxy-6-(bromomethyl)tetrahydro-2*H*-pyran-2-yl)oxy)tetrahydro-2*H*-pyran-3,4,5-triyl triacetate (1.18 g, 1.69 mmol) in DMF (11.8 mL) was added sodium azide (0.55 g, 8.45 mmol). The reaction mixture was stirred at 55°C overnight. The reaction mixture was then cooled to room temperature, diluted with ethyl acetate, washed with water and saturated brine, dried over anhydrous sodium sulfate, filtered, and the filtrate was concentrated under reduced pressure. The residue was purified by flash column chromatography (C18, eluent: 0%-57% acetonitrile in 0.05% trifluoroacetic acid aqueous solution) to obtain (2*R,*3*R,*4*S,*5*R,*6*R*)-2-(acetoxymethyl)-6-(((2*R*,3*R*,4*S*,5*R*,6*R*)-3,4,5-triacetoxy-6-(azidomethyl)tetrahydro-2*H*-pyran-2-yl)oxy)tetrahydro-2*H*-pyran-3,4,5-triyl triacetate (0.73 g) as a white solid. m/z: [M+Na]⁺ 684.2.

Step 4: To a solution of (2*R*,3*R*,4*S*,5*R*,6*R*)-2-(acetoxymethyl)-6-(((2*R*,3*R*,4*S*,5*R*,6*R*)-3,4,5-triacetoxy-6-(azidomethyl)tetrahydro-2*H*-pyran-2-yl)oxy)tetrahydro-2*H*-pyran-3,4,5-triyl triacetate (430 mg, 0.65 mmol) in methanol (13 mL) was added sodium methoxide (35 mg, 0.65 mmol) under a nitrogen atmosphere. The reaction mixture was stirred at room temperature for 20 hours. The reaction mixture was then adjusted to neutral pH with ion exchange resin AMBERLITE IR-120(H), filtered to remove the resin, and the filtrate was concentrated under reduced pressure to obtain (2*R*,3*S,*4*S*,5*R*,6*R*)-2-(azidomethyl)-6-(((2*R*,3*R*,4*S*,5*S*,6*R*)-3,4,5-trihydroxy-6-(hydroxymethyl)tetrahydro-2*H*-pyran-2-yl)oxy)tetrahydro-2*H*-pyran-3,4,5-triol (256 mg) as a pink solid. m/z: [M+Na]⁺ 390.2.

### Synthesis of 2-azido-N-((3R,4R,5R,6R)-2,4,5-trihydroxy-6-(hydroxymethyl)tetrahydro-2H-pyran-3-yl)acetamide

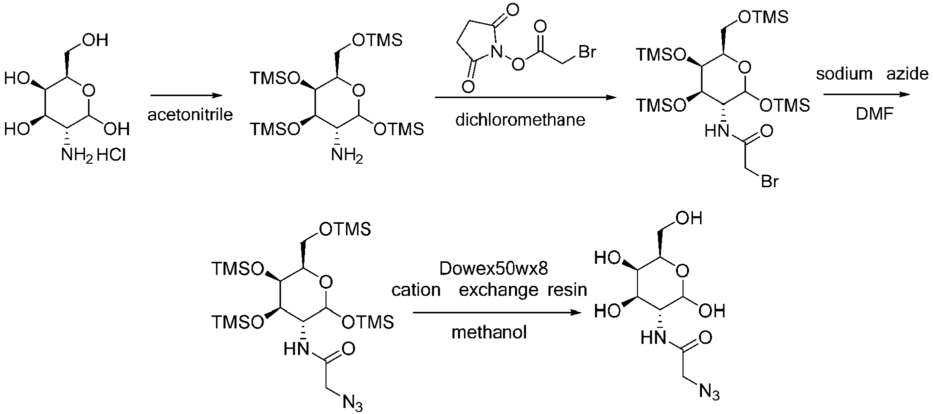

Step 1: To a solution of D-(+)-galactosamine hydrochloride (2.15 g, 9.97 mmol) in acetonitrile (20 mL) was added hexamethyldisilazane (5.2 mL, 24.9 mmol) in portions under a nitrogen atmosphere. The reaction mixture was stirred at room temperature for 3 hours. The reaction mixture was filtered through diatomite, and the filtrate was concentrated under reduced pressure to obtain (3*R*,4*R*,5*S*,6*R*)-2,4,5-tris((trimethylsilyl)oxy)-6-(((trimethylsilyl)oxy)methyl)tetrahydro-2*H*-pyran-3-amine (4.36 g) as a yellow oil. m/z: [M-TMS+H]⁺ 396.2.

Step 2: To a solution of (3*R*,4*R*,5*S*,6*R*)-2,4,5-tris((trimethylsilyl)oxy)-6-(((trimethylsilyl)oxy)methyl)tetrahydro-2*H*-pyran-3-amine (4.36 g, 9.32 mmol) in dichloromethane (20 mL) was added bromoacetic acid N-hydroxysuccinimide ester (3.32 g, 14.1 mmol) under ice bath conditions. The reaction mixture was stirred at room temperature for 2 hours and then directly concentrated under reduced pressure. The residue was triturated with petroleum ether/ethyl acetate = 10/1 (22 mL), and the solid was removed by filtration. The filtrate was concentrated under reduced pressure to obtain 2-bromo-*N*-((3*R*,4*R*,5*S*,6*R*)-2,4,5-tris((trimethylsilyl)oxy)-6-(((trimethylsilyl)oxy)methyl)tetrahydro-2H-pyran-3-yl)acetamide (5 g) as a yellow semi-oily semi-solid. m/z: [M+H]⁺ 588.2, 590.2.

Step 3: To a solution of 2-bromo-*N*-((3*R*,4*R*,5*S*,6*R*)-2,4,5-tris((trimethylsilyl)oxy)-6-(((trimethylsilyl)oxy)methyl)tetrahydro-2*H*-pyran-3-yl)acetamide (5 g, 8.49 mmol) in DMF (15 mL) was added sodium azide (0.68 g, 10.5 mmol), and the reaction mixture was stirred at 20°C overnight. The reaction mixture was then diluted with ethyl acetate (20 mL), washed with a large amount of water, and the aqueous phase was extracted with ethyl acetate. The combined organic phases were concentrated under reduced pressure to obtain 2-azido-*N-*((3*R*,4*R*,5*S*,6*R*)-2,4,5-tris((trimethylsilyl)oxy)-6-(((trimethylsilyl)oxy)methyl)tetrahydro-2*H-*pyran-3-yl)acetamide (3.9 g) as a yellow oil.

Step 4: To a solution of 2-azido-*N*-((3*R*,4*R*,5*S*,6*R*)-2,4,5-tris((trimethylsilyl)oxy)-6-(((trimethylsilyl)oxy)methyl)tetrahydro-2*H*-pyran-3-yl)acetamide (3.9 g, 7.1 mmol) in methanol (15 mL) was added Dowex 50wx8 cation exchange resin (1 g). The reaction mixture was stirred at room temperature for 2 hours. The reaction mixture was filtered through diatomite, and the filter cake was rinsed with a small amount of methanol. The combined filtrates were concentrated under reduced pressure, and the residue was triturated with ethanol (20 mL). The solid was collected and dried to obtain 2-azido*-N*-((3*R*,4*R*,5*R*,6*R*)-2,4,5-trihydroxy-6-(hydroxymethyl)tetrahydro-2*H*-pyran-3-yl)acetamide (1 g) as a white solid. m/z: [M+Na]⁺ 285.2.

### Synthesis of compounds:

### Example 1: Synthesis of compounds 1-1, 1-2, 1-3, and 1-4

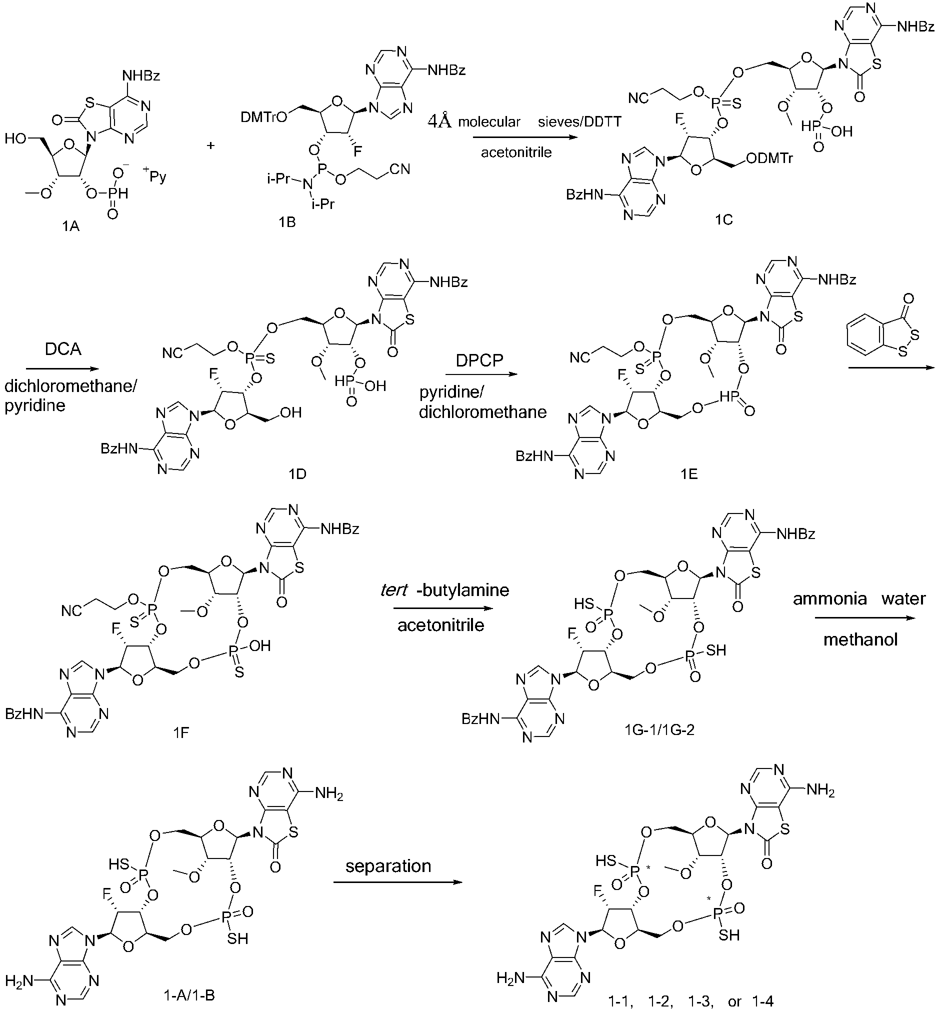

Step 1: Compounds 1A (1.2 g, 2.49 mmol) and 1B (2.4 g, 2.74 mmol) were each azeotropically concentrated with acetonitrile (10 mL) twice, then separately dissolved in acetonitrile (5 mL) and set aside. Under a nitrogen atmosphere and ice bath conditions, the acetonitrile solution of compound 1B was slowly added to the acetonitrile solution of compound 1A containing 4Å molecular sieves. The resulting mixture was stirred for 1 hour, then DDTT (0.61 g, 2.99 mmol) was added to the reaction system. After stirring for an additional half hour, the molecular sieves were removed by filtration, and the filtrate was concentrated under reduced pressure to obtain compound 1C (1.3 g, crude). m/z: [M+H]⁺ 1289.2.

Step 2: Under a nitrogen atmosphere and in an ice-water bath, a dichloromethane solution of DCA (0.6 M, 9 mL, 5.39 mmol) was added to a solution of compound 1C (1.0 g, 0.77 mmol) in dichloromethane (10.0 mL). The reaction mixture was stirred at room temperature for 0.5 hours. Triethylsilane (0.5 mL) and pyridine (1.0 mL) were added, and the mixture was concentrated under reduced pressure. The residue was purified by flash column chromatography (0.1% NH₄HCO₃/CH₃CN = 0-50%) to obtain compound 1D (510 mg, pyridinium salt, crude) as a white solid. m/z: [M+H]⁺ 987.0.

Step 3: Compound 1D (500 mg, 0.51 mmol) was azeotropically concentrated with pyridine (2 mL) three times, then redissolved in pyridine (2 mL). This solution was slowly added dropwise at -40°C to a pyridine solution of DPCP (685 mg, 2.55 mmol), and the mixture was stirred at -20°C for 1 hour. The resulting reaction mixture containing compound 1E was directly used in the next step.

Step 4: Water (459 mg, 25.5 mmol) and 3*H*-1,2-benzodithiol-3-one (102 mg, 0.61 mmol) were directly added to the reaction mixture of compound 1E prepared in Step 3. After stirring at room temperature for 20 minutes, the reaction mixture was directly purified by flash column chromatography (0.1% NH₄HCO₃/CH₃CN = 0-40%) to obtain compound 1F (200 mg) as a white solid. m/z: [M+H]⁺ 1000.1.

Step 5: To a solution of compound 1F (180 mg, 0.18 mmol) in acetonitrile (2 mL) was added tert-butylamine (2 mL). The resulting mixture was stirred at room temperature for 0.5 hours, then concentrated under reduced pressure. The residue was purified by flash column chromatography (C18, eluent: 0-35% acetonitrile in 10 mM ammonium bicarbonate aqueous solution) to first collect compound 1G-1 (130 mg), followed by 1G-2 (35 mg), both as white solids. m/z: [M+H]⁺ 948.0.

Step 6: To a solution of compound 1G-1 (120 mg) in methanol (3 mL) was added ammonia water (3 mL). The reaction mixture was stirred at 50°C in a sealed tube for 5 hours. After cooling to room temperature, the reaction mixture was quenched with acetic acid and directly lyophilized to obtain compound 1-A.

Compound 1-A was purified by *prep-*HPLC to obtain the following compounds, all as ammonium salts:

1-1: white solid, 10.6 mg, m/z: [M+H]⁺ 740.0, HPLC-RT (condition 2): 10.814 min, ¹H NMR (400 MHz, D₂O): δ 7.70-8.35 (m, 3H), 6.38 (d, *J =* 13.6Hz, 1H), 6.02-6.12 (m, 2H), 5.27-5.54 (m, 2H), 4.35-4.49 (m, 4H), 3.95-4.11 (m, 3H), 3.48 (s, 3H); ³¹P NMR (162 MHz, D₂O): δ 54.72, 54.22, 53.46; ¹⁹F NMR (376 MHz, D₂O): δ -201.09;

1-2: white solid, 8.2 mg, m/z: [M+H]⁺740.0, HPLC-RT (condition 2): 11.380 min, ¹H NMR (400 MHz, D₂O): δ 8.08-8.35 (m, 3H), 6.34-6.36 (m, 1H), 6.07 (d, *J =* 7.6Hz, 1H), 5.73-5.85 (m, 1H), 4.88-5.00 (m, 2H), 4.47 (s, 3H), 4.02-4.17 (m, 4H), 3.50 (s, 3H); ³¹P NMR (162 MHz, D₂O): δ 54.94, 52.83, 51.54; ¹⁹F NMR (376 MHz, D₂O): δ -202.86;

1-3: white solid, 8.2 mg, m/z: [M+H]⁺740.0, HPLC-RT (condition 2): 10.370 min, ¹H NMR (400 MHz, D₂O): δ 8.36 (s, 2H), 8.12 (s, 1H), .61 (s, 1H), 6.11 (s, 1H), 5.79 (s, 1H), 5.39 (d, *J =* 51.6Hz, 1H), 4.93-5.02 (m, H), 4.36-4.48 (m, 3H), 3.84-4.03 (m, 4H), 3.51 (s, 3H); ³¹P NMR (162 MHz, DMSO-d₆+D₂O): δ 55.759, 51.401; ¹⁹F NMR (376 MHz, D₂O): δ -202.92.

Following the synthesis method of compound 1-A, compound 1G-2 was reacted to obtain compound 1-B, which was purified by *prep-*HPLC to obtain compound 1-4: ammonium salt, white solid, 5.2 mg, m/z: [M+H]⁺ 740.0, HPLC-RT (condition 1): 11.650 min, ¹H NMR (400 MHz, D₂O): δ 8.51 (s, 1H), 8.28 (s, 1H), 8.18 (s, 1H), 6.64 (s, 1H), 6.45 (d, *J =* 16.0Hz, 1H), 5.68-5.82 (m, 1H), 5.26-5.43 (m, 2H), 4.07-4.57 (m, 7H), 3.41 (s, 3H); ³¹P NMR (162 MHz, D₂O): δ 54.64, 52.83; ¹⁹F NMR (376 MHz, D₂O): δ -201.55.

### Single crystal diffraction experiment of compound 1-3

1. Single crystal cultivation: Compound 1-3 (8 mg) was dissolved in a mixed solution of water, acetonitrile, and tetrahydrofuran (1/8/1, 0.5 mL). The solution was allowed to slowly evaporate at room temperature to form crystals, and single crystals were collected for single crystal diffraction testing.
2. Test parameters are shown in Table 2 below:

**Table 2**

| | |
|---|---|
| Wavelength | 1.3405 Å |
| Crystal system | Orthorhombic |
| Space group | *P*2₁2₁2 |
| Unit cell dimensions | a = 35.4619(6) Å, α = 90° |
| | b = 26.8875(6) Å, β = 90° |
| | c = 15.7430(4) Å, γ = 90° |
| Unit cell volume | 15010.7(6) Å³ |
| Z | 16 |
| Density (calculated) | 1.306 g/cm³ |
| Data collection θ range | 3.586 to 96.268° |
| Reflections collected | 239637/21512 |
| Independent reflections | Rint = 0.1675, Rsigma = 0.0708 |
| Completeness | 99.65% |
| Data restraints | 21512/175/1700 |
| Refinement on F² | 1.039 |

3. Test results: The stereostructure of compound 1-3 is shown below, with all chiral centers in the molecule exhibiting *R* configuration.

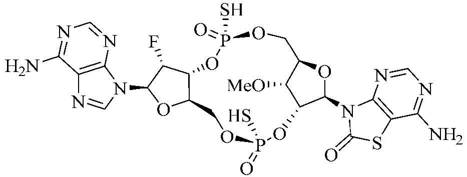

### Example 2: Synthesis of compound 1M and compound A

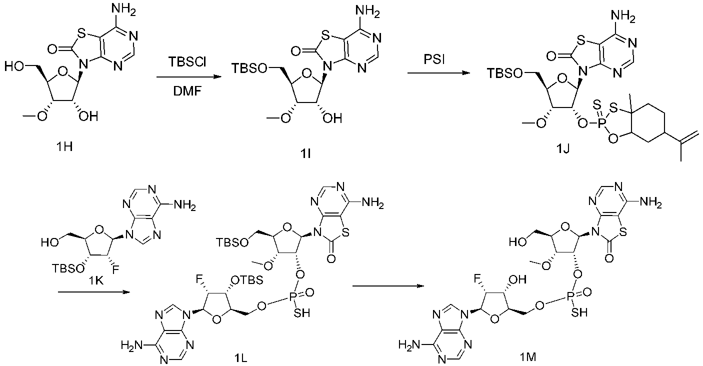

Step 1: To a solution of compound 1H (2.5 g, 7.9 mmol) in DMF (12 mL) were sequentially added TBSCl (1.44 g, 10 mmol) and imidazole (1.62 g, 23.8 mmol). The reaction mixture was stirred at room temperature for 4 hours, then quenched with water, and the aqueous phase was extracted with ethyl acetate. The combined organic phases were concentrated under reduced pressure. The residue was purified by silica gel column chromatography (petroleum ether/ethyl acetate = 1/1) to obtain compound 1I (2.9 g, yield: 86%) as a white solid. m/z: [M+H]⁺ 429.2.

Step 2: To a solution of compound 1I (2.9 g, 6.8 mmol) in anhydrous acetonitrile (25 mL) were added PSI (3.92 g, 8.8 mmol) and DBU (1.34 g, 8.8 mmol). The reaction mixture was stirred at room temperature for half an hour, then directly concentrated under reduced pressure. The residue was purified by silica gel column chromatography (petroleum ether/ethyl acetate = 1/1) to obtain compound 1J (3 g, yield: 65%) as a white solid. m/z: [M+H]⁺ 675.2.

Step 3: Under a nitrogen atmosphere, a solution of compounds 1J (3 g, 4.4 mmol), 1K (1.7 g, 4.4 g), and DBU (2 g, 13.2 mmol) in anhydrous acetonitrile (30 mL) was stirred at room temperature for 1 hour, then concentrated under reduced pressure. The residue was purified by silica gel column chromatography (petroleum ether/ethyl acetate = 1/1 to 1/4) to obtain compound 1L (2.7 g, yield: 69%) as a white solid.

Step 4: A solution of compound 1L (2.7 g, 3.0 mmol) in hydrogen chloride-1,4-dioxane (40 mL, 2 M) was stirred at room temperature for 16 hours, then directly concentrated under reduced pressure. The residue was purified by flash column chromatography (C18, eluent: 0-35% acetonitrile in 10 mM ammonium bicarbonate aqueous solution) to first obtain compound 1M (1.1 g, yield: 55%) as a pale yellow solid. m/z: [M+H]⁺ 662.1. ³¹P NMR (162 MHz, DMSO-d₆+D₂O): δ 55.847.

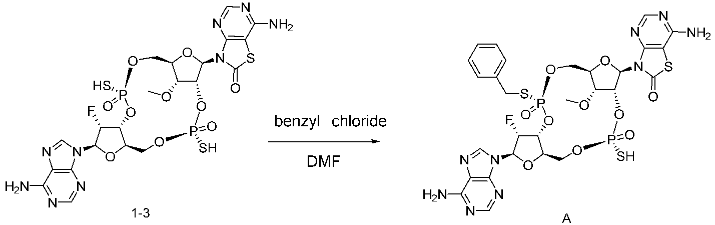

To a solution of compound 1-3 (300 mg, 0.41 mmol) and benzyl chloride (52 mg, 0.41 mmol) in DMF (1 mL) was added sodium iodide (3.6 mg, 0.024 mmol). The reaction mixture was stirred at room temperature for 24 hours and directly purified by *prep-*HPLC (eluent: 10% to 55% acetonitrile in 0.1% ammonium bicarbonate aqueous solution) to obtain compound A (50 mg, yield: 15%) as a white solid. m/z: [M+H] ⁺ 830.0; ³¹P NMR (162 MHz, DMSO-d₆+D₂O): δ 53.113, 27.573.

### Example 3: Synthesis of compound 2

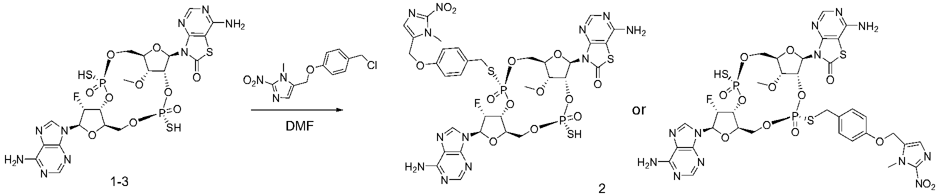

To a mixed solution of compound 1-3 (62 mg, 0.084 mmol) in anhydrous acetonitrile (1 mL) and dimethylacetamide (1 mL) were sequentially added 5-((4-(chloromethyl)phenoxy)methyl)-1-methyl-2-nitro-1*H*-imidazole (20 mg, 0.07 mmol) and sodium iodide (31 mg, 0.21 mmol) under ice bath conditions. The reaction mixture was stirred at room temperature for 2 hours and then directly purified by flash column chromatography (C18, eluent: 0-60% acetonitrile in 10 mM ammonium bicarbonate aqueous solution) to obtain compound 2 (40 mg, yield: 58%) as a white solid. m/z: [1/2M+H]⁺493.2.

### Example 4: Synthesis of compound 3 and compound 4

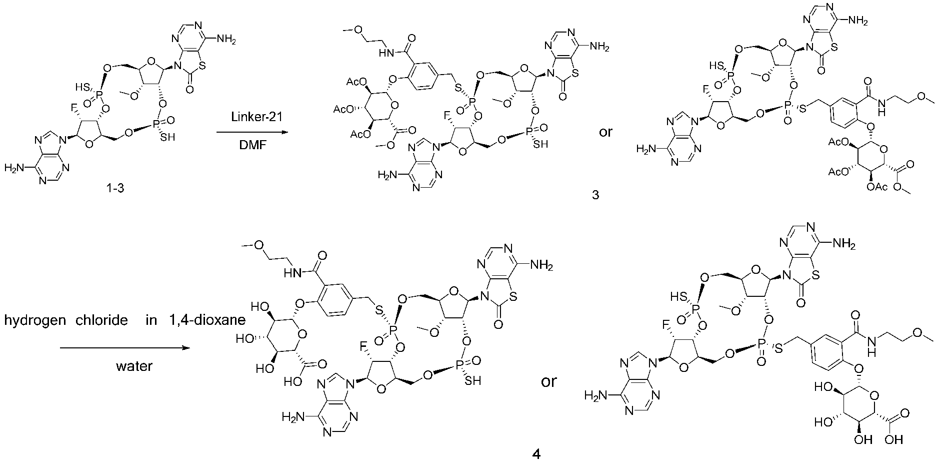

Step 1: To a solution of compound 1-3 (200 mg, 0.27 mmol) in DMF (5 mL) was added Linker-21 (180 mg, 0.27 mmol). The reaction mixture was stirred at room temperature for 2 hours and directly purified by flash column chromatography (C18, eluent: 0-70% acetonitrile in 10mM ammonium bicarbonate aqueous solution) to obtain 3 (135 mg, yield: 40%) as a white solid. m/z: [1/2M+H]⁺ 632.2.

Step 2: To a solution of compound 3 (80 mg, 0.063 mmol) in water (2 mL) was added hydrogen chloride-1,4-dioxane solution (2 mL, 4 M). The reaction mixture was stirred at 35°C for 12 hours and then directly purified by flash column chromatography (C18, eluent: 0-50% acetonitrile in 0.1% trifluoroacetic acid aqueous solution) to obtain compound 4 (50 mg, yield: 70%) as a white solid. m/z: [1/2M+H]⁺ 562.2.

### Example 5: Synthesis of compound 5

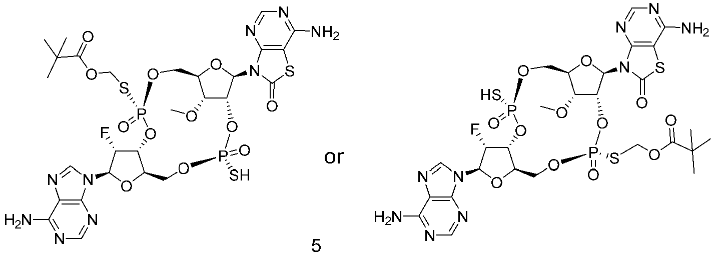

Following the synthetic method of compound 3, compound 1-3 was reacted with iodomethyl pivalate to obtain compound 5. m/z: [M+H]⁺ 853.9.

### Example 6: Synthesis of compound 6

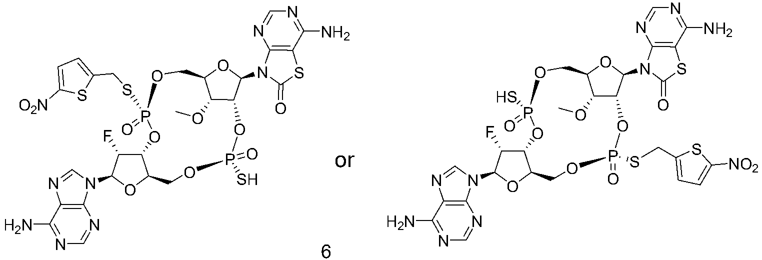

Following the synthetic method of compound 2, compound 1-3 was reacted with 2-(chloromethyl)-5-nitrothiophene to obtain compound 6. m/z: [M+H]⁺ 880.9.

### Example 7: Synthesis of compound 7

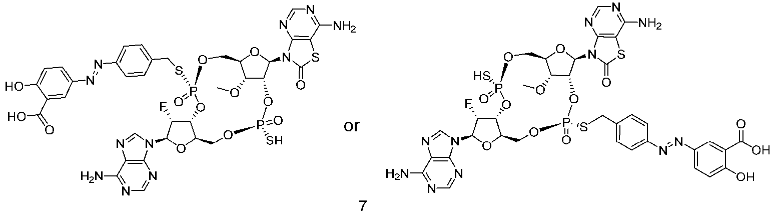

Following the synthetic method of compound 2, compound 1-3 and (E)-4-((4-(chloromethyl)phenyl)diazinyl)-2-hydroxybenzoic acid were reacted to obtain compound 7. m/z: [M+H]⁺ 994.0.

### Example 8: Synthesis of I-1

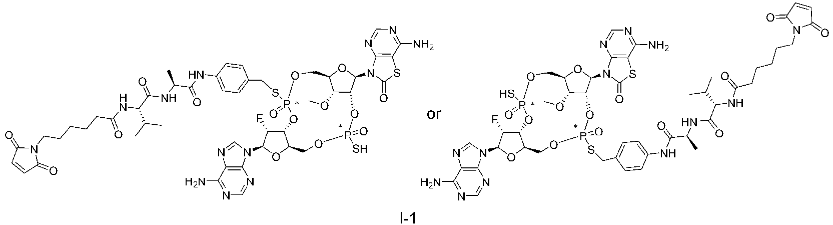

To a solution of compound 1-1 (10 mg, 0.01 mmol) in anhydrous DMF (2 mL) was added dropwise a solution of Linker-1 (5.9 mg, 0.01 mmol) in anhydrous DMF (0.3 mL). The reaction mixture was stirred at 50°C for 3 hours, and then directly purified by *prep-*HPLC (mobile phase A: 10 mmol/L ammonium bicarbonate aqueous solution, mobile phase B: acetonitrile; gradient elution with mobile phase B from 15% to 50%, elution time: 20 minutes) to obtain I-1 (3.6 mg) as a white solid. UPLC RT = 6.003 minutes; m/z: [M+H]⁺ 1208.2.

### Example 9: Synthesis of I-2

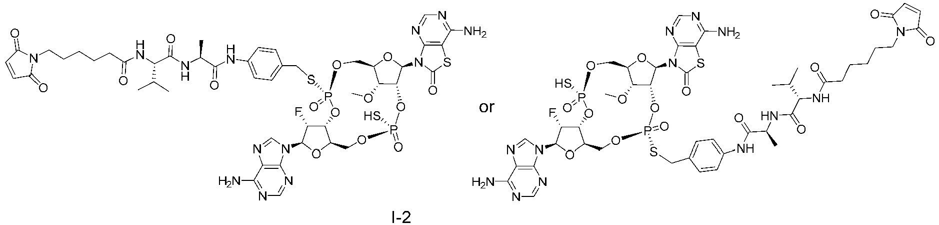

To a solution of compound 1-3 (10 mg, 0.01 mmol) in anhydrous DMF (2 mL) was added dropwise a solution of Linker-1 (5.9 mg, 0.01 mmol) in anhydrous DMF (0.3 mL). The reaction mixture was stirred at room temperature for 3 hours, and then directly purified by *prep-*HPLC (mobile phase A: 10 mmol/L ammonium bicarbonate aqueous solution, mobile phase B: acetonitrile; gradient elution with mobile phase B from 15% to 65%, elution time: 25 minutes) to obtain I-2 (3.6 mg) as a white solid. UPLC RT = 6.609 minutes; m/z: [M+H]⁺ 1208.1.

### Example 10: Synthesis of I-3

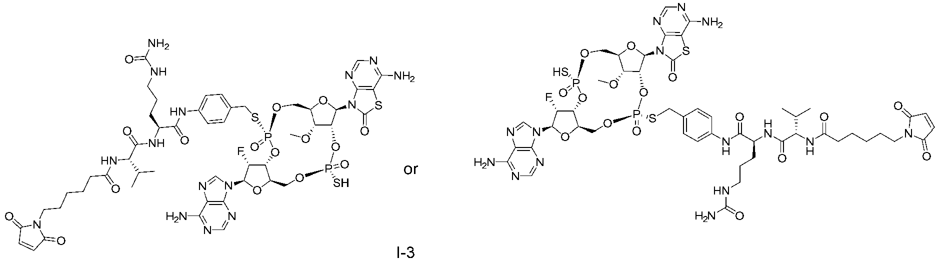

To a solution of compound 1-3 (15 mg, 0.02 mmol) in anhydrous DMF (2 mL) was added dropwise a solution of Linker-2 (13.7 mg, 0.01 mmol) in anhydrous DMF (0.3 mL). The reaction mixture was stirred at room temperature for 3 hours, and then directly purified by *prep-*HPLC (mobile phase A: 10 mmol/L ammonium bicarbonate aqueous solution, mobile phase B: acetonitrile; gradient elution with mobile phase B from 15% to 65%, elution time: 25 minutes) to obtain I-3 (8.9 mg) as a white solid. UPLC RT = 6.329 minutes; m/z: [M+H]⁺ 1294.3. ³¹P NMR (162MHz, DMSO-d₆+D₂O): δ 54.318, 27.733.

### Example 11: Synthesis of I-4

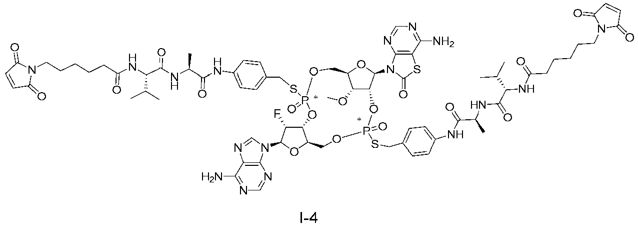

To a solution of compound 1-1 (10 mg, 0.013 mmol) in anhydrous DMF (2 mL) was added Linker-1 (17 mg, 0.029 mmol). The reaction mixture was stirred at 50°C for 5 hours and directly purified by *prep-*HPLC (mobile phase A: 10 mmol/L ammonium bicarbonate aqueous solution, mobile phase B: acetonitrile; gradient elution with mobile phase B from 15% to 85%, elution time: 25 minutes) to obtain I-4 (6.3 mg) as a white solid. UPLC RT = 6.893 minutes; m/z: [M+H]⁺ 1677.8.

### Example 12: Synthesis of I-5

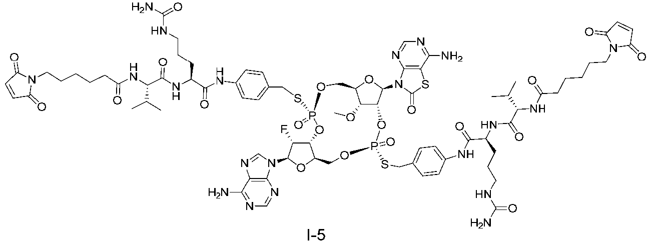

To a solution of compound 1-3 (150 mg, 0.20 mmol) in anhydrous DMF (3.5 mL) was added Linker-2 (340 mg, 0.50 mmol). The reaction mixture was stirred at room temperature for 2 hours and directly purified by flash column chromatography (C18, eluent: 0-65% acetonitrile in 10 mM ammonium bicarbonate aqueous solution) to obtain I-5 (140 mg) as a white solid. UPLC RT = 6.577 minutes; m/z: [1/2M+H]⁺ 924.8.

### Example 13: Synthesis of I-6

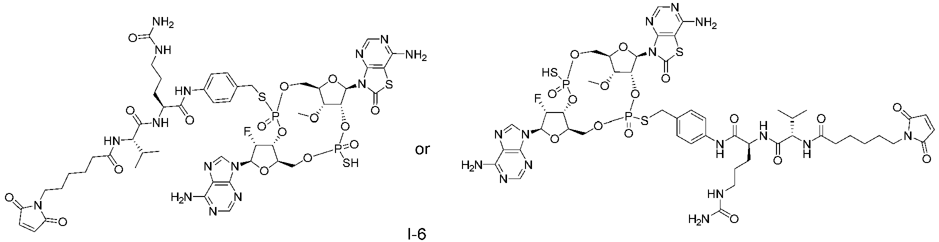

Following the synthetic method of I-3, compound 1-1 was reacted with Linker-2 to obtain I-6 as a white solid. HPLC RT = 13.448 minutes; m/z: [1/2M+H]⁺ 647.8.

### Example 14: Synthesis of I-7

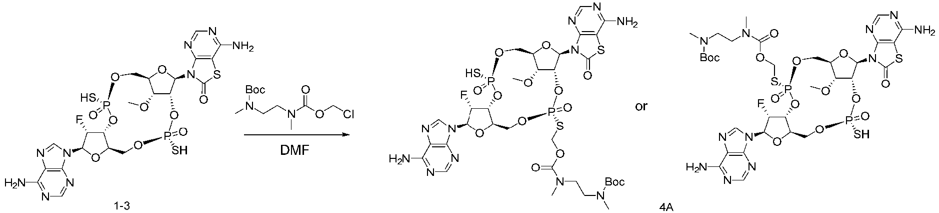

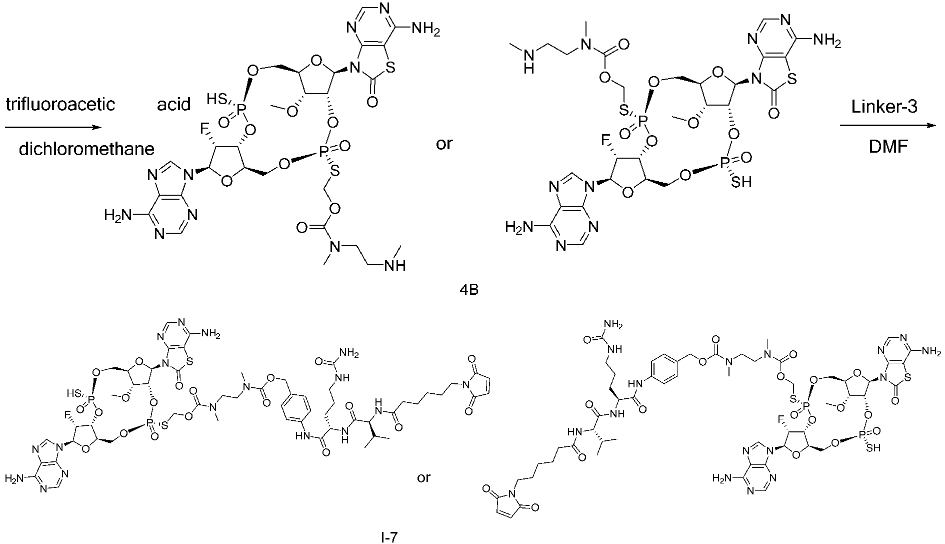

Step 1: To a solution of compound 1-3 (140 mg, 0.19 mmol) in DMF (3 mL) were sequentially added *tert*-butyl (chloromethyl) ethane-1,2-diylbis(methylcarbamate) (69 mg, 0.25 mmol) and sodium iodide (290 mg, 1.9 mmol). The reaction mixture was stirred at room temperature for 3 hours and then directly purified by flash column chromatography (C18, eluent: 0-55% acetonitrile in 10 mM ammonium bicarbonate aqueous solution) to obtain compound 4A (110 mg, yield: 59%). m/z: [M+H]⁺ 983.9.

Step 2: To a solution of compound 4A (80 mg, 0.081 mmol) in dichloromethane (2 mL) was added dropwise trifluoroacetic acid (1 mL) under ice bath conditions. The reaction mixture was stirred at room temperature for 2 hours, concentrated under reduced pressure, and the residue was purified by flash column chromatography (C18, eluent: 0-50% acetonitrile in 10 mM ammonium bicarbonate aqueous solution) to obtain compound 4B (70 mg, yield: 96%). m/z: [M+H]⁺ 883.9.

Step 3: To a solution of compound 4B (40 mg, 0.045 mmol) in DMF (2 mL) were sequentially added Linker-3 (43 mg, 58 mmol) and DIPEA (15 mg, 0.11 mmol). The reaction mixture was stirred at room temperature for 2 hours, concentrated under reduced pressure, and the residue was purified by flash column chromatography (C18, eluent: 0-50% acetonitrile in 10 mM ammonium bicarbonate aqueous solution) to obtain compound I-7 (55 mg, yield: 82%) as a white solid. HPLC RT = 14.510 minutes; m/z: [1/2M+H]⁺ 741.8.

### Example 15: Synthesis of I-8

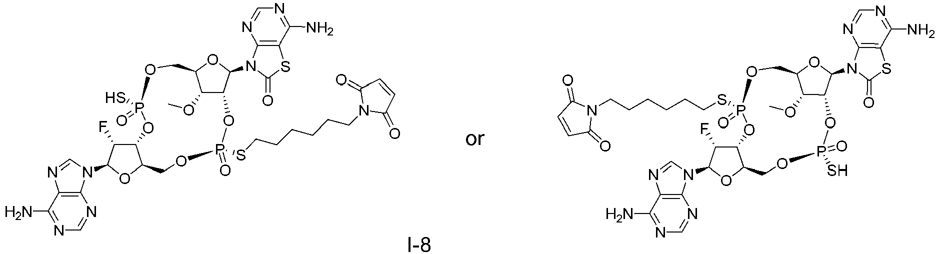

To a solution of compound 1-3 (25 mg, 0.034 mmol) in anhydrous DMF (2 mL) was added Linker-5 (10 mg, 0.034 mmol). The reaction mixture was stirred at room temperature for 12 hours and directly purified by *prep-*HPLC (mobile phase A: 10 mmol/L ammonium bicarbonate aqueous solution, mobile phase B: acetonitrile; gradient elution with mobile phase B from 10% to 45%, elution time: 20 minutes) to obtain I-8 (3.2 mg) as a white solid. HPLC RT = 14.350 minutes; m/z: [M+H]⁺ 919.1.

### Example 16: Synthesis of I-9

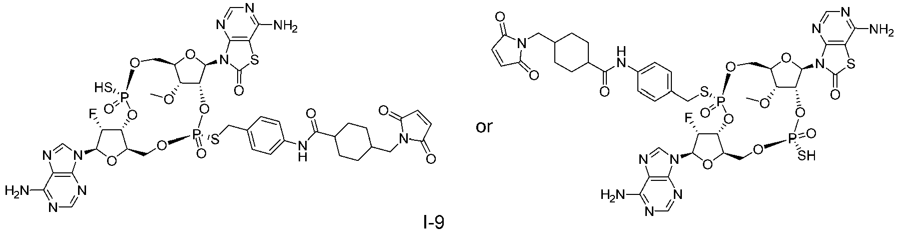

To a solution of compound 1-3 (20 mg, 0.027 mmol) in anhydrous DMF (3 mL) was added Linker-6 (18 mg, 0.041 mmol). The reaction mixture was stirred at room temperature for 2 hours and directly purified by *prep-*HPLC (mobile phase A: 10 mmol/L ammonium bicarbonate aqueous solution, mobile phase B: acetonitrile; gradient elution with mobile phase B from 5% to 65%, elution time: 20 minutes) to obtain I-9 (3.2 mg) as a white solid. HPLC RT = 14.622 minutes; m/z: [M+H]⁺ 1064.0.

### Example 17: Synthesis of I-10

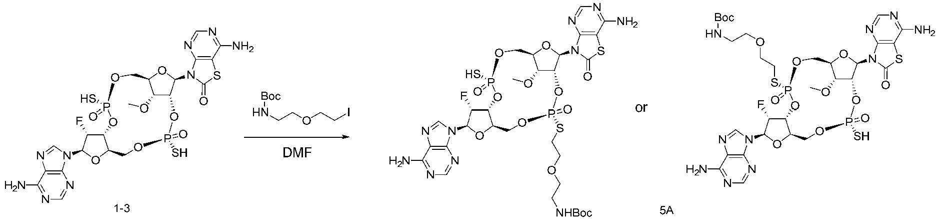

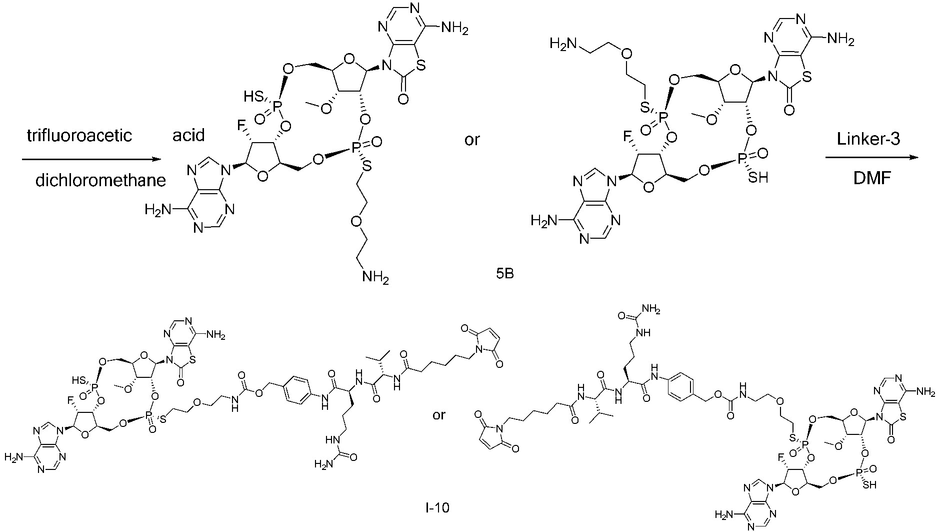

Step 1: To a solution of compound 1-3 (80 mg, 0.11 mmol) in DMF (3 mL) was added *tert-*butyl (2-(2-iodoethoxy)ethyl)carbamate (35 mg, 0.11 mmol). The reaction mixture was stirred at room temperature for 12 hours and then directly purified by flash column chromatography (C18, eluent: 0-70% acetonitrile in 10mM ammonium bicarbonate aqueous solution) to obtain compound 5A (32 mg, yield: 52%). m/z: [M+H]⁺ 927.0.

Steps 2 & 3: Following the synthetic methods of Step 2 and Step 3 in III-8, compound 5A was reacted to obtain compound I-10 (*prep-*HPLC separation: mobile phase A: 10 mmol/L ammonium bicarbonate aqueous solution, mobile phase B: acetonitrile; gradient elution with mobile phase B from 3% to 45%, elution time: 20 minutes) as a white solid. HPLC RT = 14.034 minutes; m/z: [M+H]⁺ 1424.9.

### Example 18: Synthesis of I-11

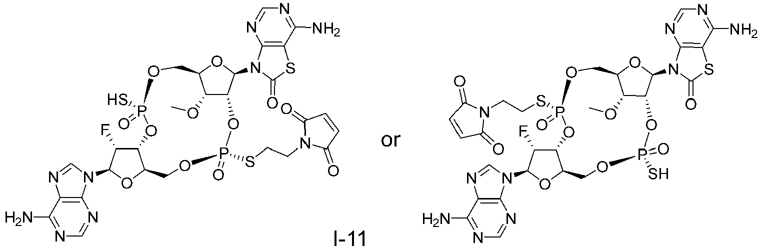

Following the synthetic method of compound I-8, compound 1-3 was reacted with 1-(2-iodoethyl)-1*H*-pyrrole-2,5-dione to obtain compound I-11 (*prep-*HPLC separation: mobile phase A: 10 mmol/L ammonium bicarbonate aqueous solution, mobile phase B: acetonitrile; gradient elution with mobile phase B from 5% to 45%, elution time: 20 minutes) as a white solid. HPLC RT = 12.959 minutes; m/z: [M+H]⁺ 863.1.

### Example 19: Synthesis of I-12

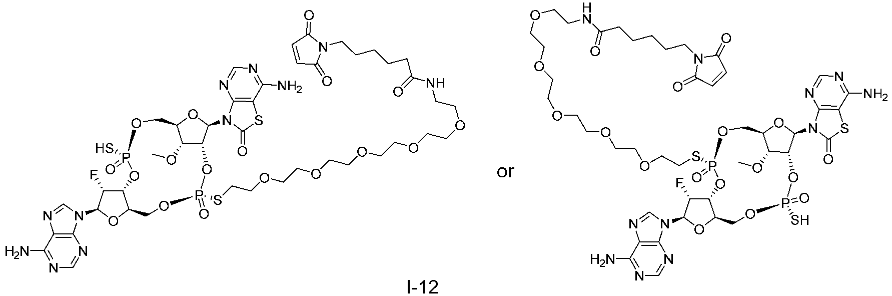

Following the synthetic method of compound I-10, compound 1-3 and *tert*-butyl (17-iodo-3,6,9,12,15-pentaoxaheptadecyl)carbamate were reacted to obtain compound I-12 (*prep-*HPLC: mobile phase A: 10 mmol/L ammonium bicarbonate aqueous solution, mobile phase B: acetonitrile; gradient elution with mobile phase B from 5% to 45%, elution time: 20 minutes) as a white solid. HPLC RT = 13.701 minutes; m/z: [M+H]⁺ 1196.2.

### Example 20: Synthesis of I-13

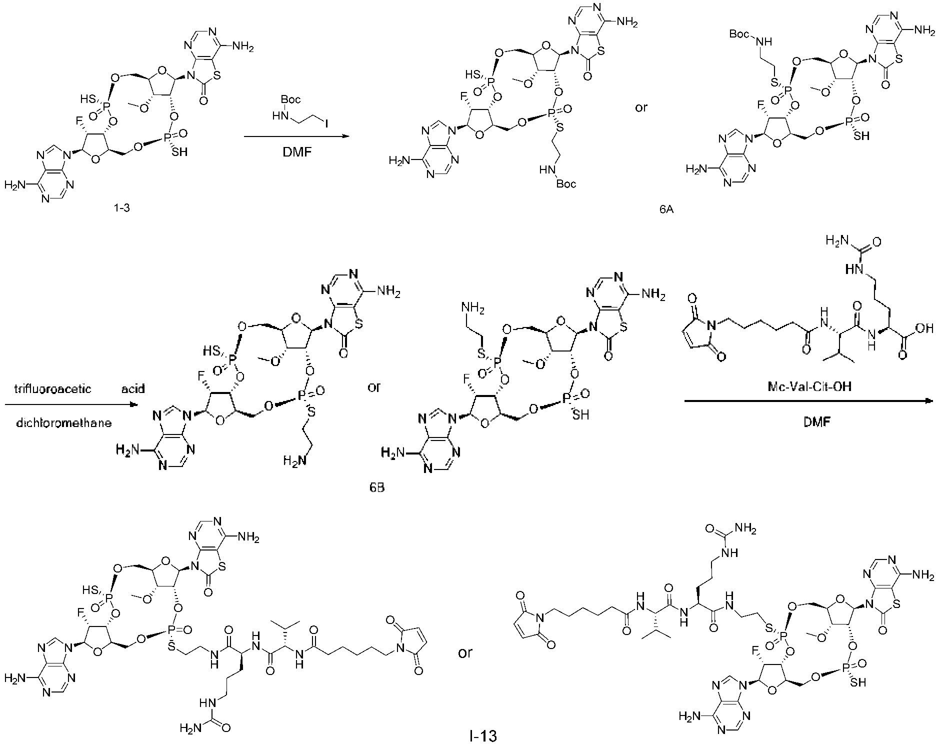

Steps 1 & 2: Following the synthetic method of 5B, 1-3 was reacted with tert-butyl (2-iodoethyl)carbamate to obtain compound 6B as a pale yellow solid. m/z: [1/2M+H]⁺ 392.0.

Step 3: To a solution of compound 6B (25 mg, 0.032 mmol) in DMF (2 mL) were sequentially added Mc-Val-Cit-OH (18 mg, 0.038 mmol), HATU (20 mg, 0.038 mmol), and DIPEA (60 mg, 0.038 mmol) under ice bath conditions. The reaction mixture was stirred at room temperature for 1 hour and then directly purified by *prep-HPLC* (mobile phase A: 0.1% trifluoroacetic acid aqueous solution, mobile phase B: acetonitrile; gradient elution with mobile phase B from 5% to 45%, elution time: 20 minutes) to obtain compound I-13 (5.2 mg, yield: 13%) as an off-white solid. UPLC RT = 5.299 minutes; m/z: [1/2M+H]⁺ 616.8.

### Example 21: Synthesis of I-14

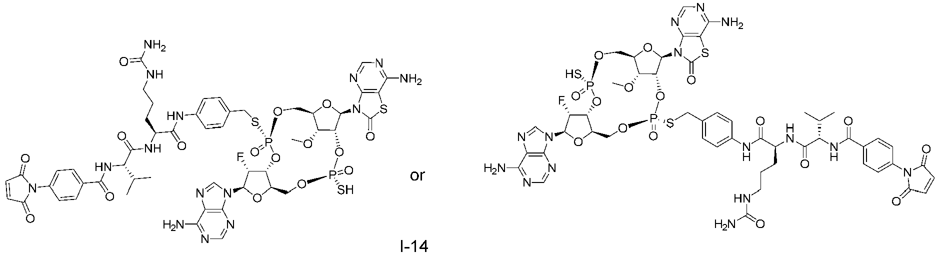

Following the synthetic method of compound I-9, compound 1-3 and Linker-7 were reacted to obtain compound I-14 (*prep-HPLC:* mobile phase A: 10 mmol/L ammonium bicarbonate aqueous solution, mobile phase B: acetonitrile; gradient elution with mobile phase B from 5% to 50%, elution time: 20 minutes) as a white solid. HPLC RT = 13.968 minutes; m/z: [1/2M+H]⁺ 650.7.

### Example 22: Synthesis of I-15

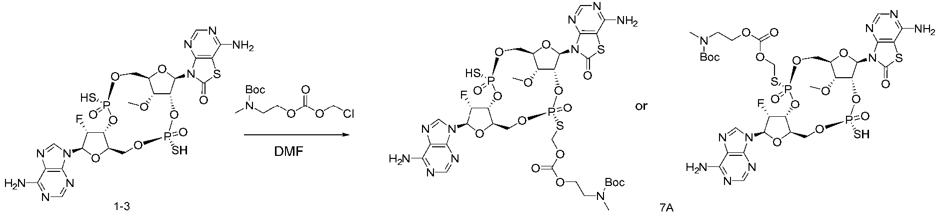

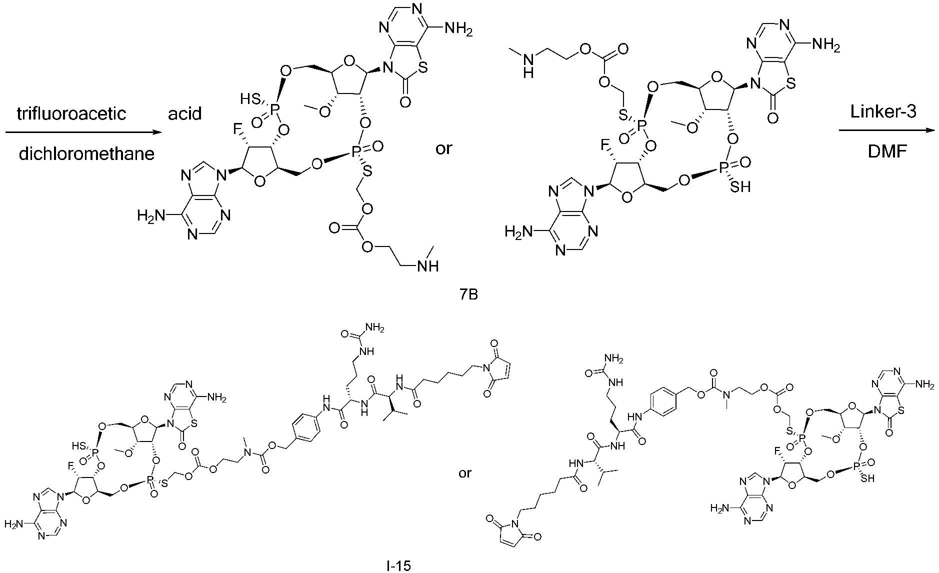

Steps 1 & 2: Following the synthetic method of compound 4B, compound 1-3 was reacted with *tert-*butyl (2-(((chloromethoxy)carbonyl)oxy)ethyl)(methyl)carbamate to obtain compound 7B as a pale yellow solid. m/z: [M+H]⁺ 871.0.

Step 3: To a solution of compound 7B (10 mg, 0.011 mmol) in DMF (2 mL) were sequentially added Linker-3 (8.1 mg, 0.11 mmol), DIPEA (2.8 mg, 0.022 mmol), and HOBT (2.2 mg, 0.017 mmol). The reaction mixture was stirred at room temperature for 2 hours, concentrated under reduced pressure, and the residue was purified by *prep-HPLC* (mobile phase A: 10 mM ammonium bicarbonate aqueous solution, mobile phase B: acetonitrile; gradient elution with mobile phase B from 5% to 55%, elution time: 20 minutes) to obtain compound I-15 (5 mg, yield: 28%) as an off-white solid. HPLC RT = 14.228 minutes; m/z: [1/2M+H]⁺ 735.2.

### Example 23: Synthesis of I-16

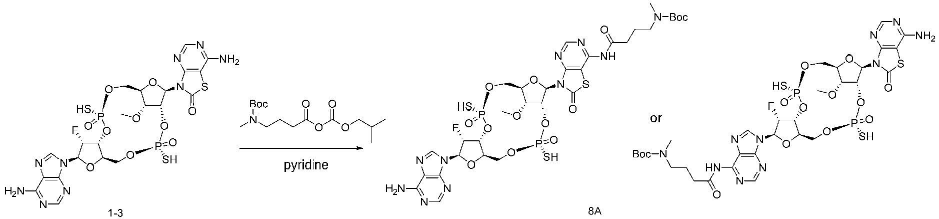

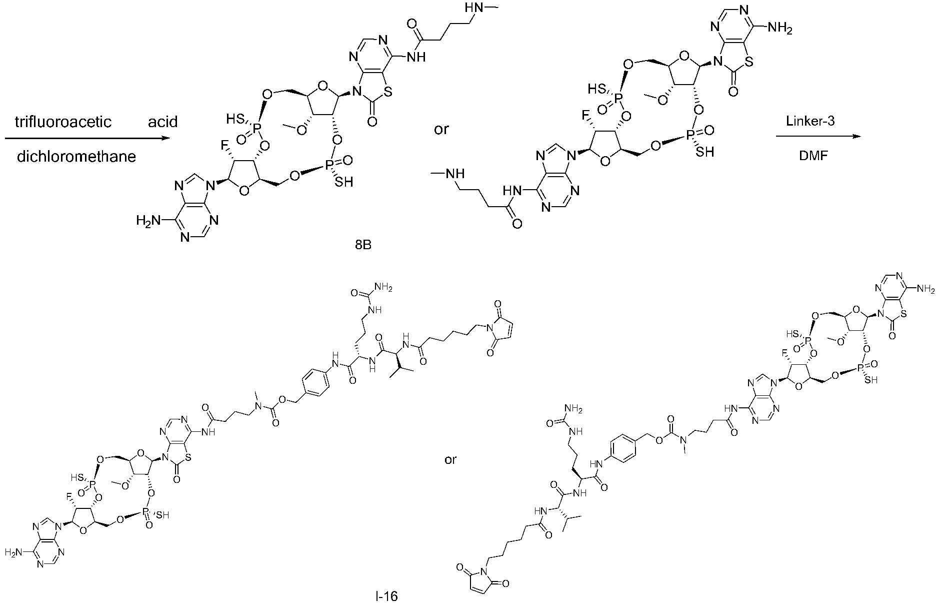

Step 1: Compound 1-3 (50 mg, 0.068 mmol) was dissolved in dry pyridine (2 mL) and concentrated under reduced pressure. This operation was repeated twice. Under ice bath conditions and nitrogen atmosphere, pyridine (2 mL) and trimethylsilyl chloride (44 mg, 0.41 mmol) were sequentially added to the above 1-3. After the addition, the reaction mixture was stirred at room temperature for 0.5 hours. Then, a solution of 4-((*tert-*butoxycarbonyl)(methyl)amino)butanoic (isobutyl carbonic) anhydride (110 mg) in dry pyridine (0.5 mL) was added to the reaction mixture, and stirring was continued for 3 hours. The reaction mixture was directly purified by flash column chromatography (C18, eluent: 0-60% acetonitrile in 0.1% trifluoroacetic acid aqueous solution) to obtain compound 8A (30 mg, yield: 47%) as a white solid. m/z: [M+H]⁺ 939.3.

Step 2: Following the synthetic method of 7B, 8A was reacted to obtain 8B as a white solid. m/z: [M+H]⁺ 839.2.

Step 3: Following the synthetic method of Step 3 in I-15, compound 8B was reacted to obtain compound I-16 (*prep-*HPLC*:* mobile phase A: 10 mM ammonium bicarbonate aqueous solution, mobile phase B: acetonitrile; gradient elution with mobile phase B from 15% to 70%, elution time: 20 minutes) as a white solid. HPLC RT = 13.067 minutes; m/z: [1/2M+H]⁺ 719.3.

### Example 24: Synthesis of I-17

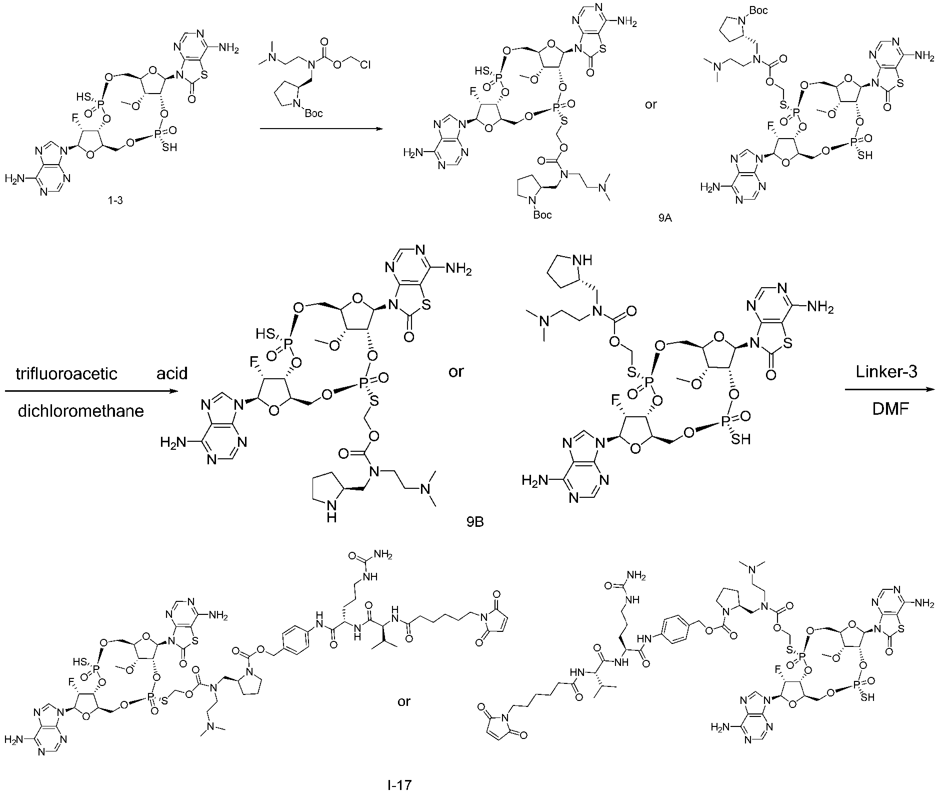

Steps 1 & 2: Following the synthetic method of compound 4B, compound 1-3 and *tert*-butyl (*S*)-2-((((chloromethoxy)carbonyl)(2-(dimethylamino)ethyl)amino)methyl)pyrrolidine-1-carboxylate were reacted to obtain compound 9B as a pale yellow solid. m/z: [M+H]⁺ 967.2.

Step 3: Following the synthetic method of Step 3 in compound I-15, 9B was reacted to obtain compound I-17 *(prep-HPLC:* mobile phase A: 10 mM ammonium bicarbonate aqueous solution, mobile phase B: acetonitrile; gradient elution with mobile phase B from 2% to 65%, elution time: 20 minutes) as a white solid. HPLC RT = 13.891 minutes; m/z: [1/2M+H]⁺ 783.0.

### Example 25: Synthesis of I-18

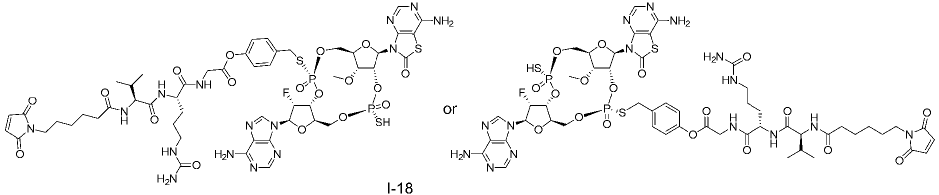

Following the synthetic method of III-3, compound 1-3 and Linker-8 were reacted to obtain compound I-18 *(prep-HPLC:* mobile phase A: 10 mM ammonium bicarbonate aqueous solution, mobile phase B: acetonitrile; gradient elution with mobile phase B from 10% to 55%, elution time: 20 minutes) as a white solid. HPLC RT = 13.777 minutes; m/z: [M+H]⁺ 1352.2.

### Example 26: Synthesis of I-19

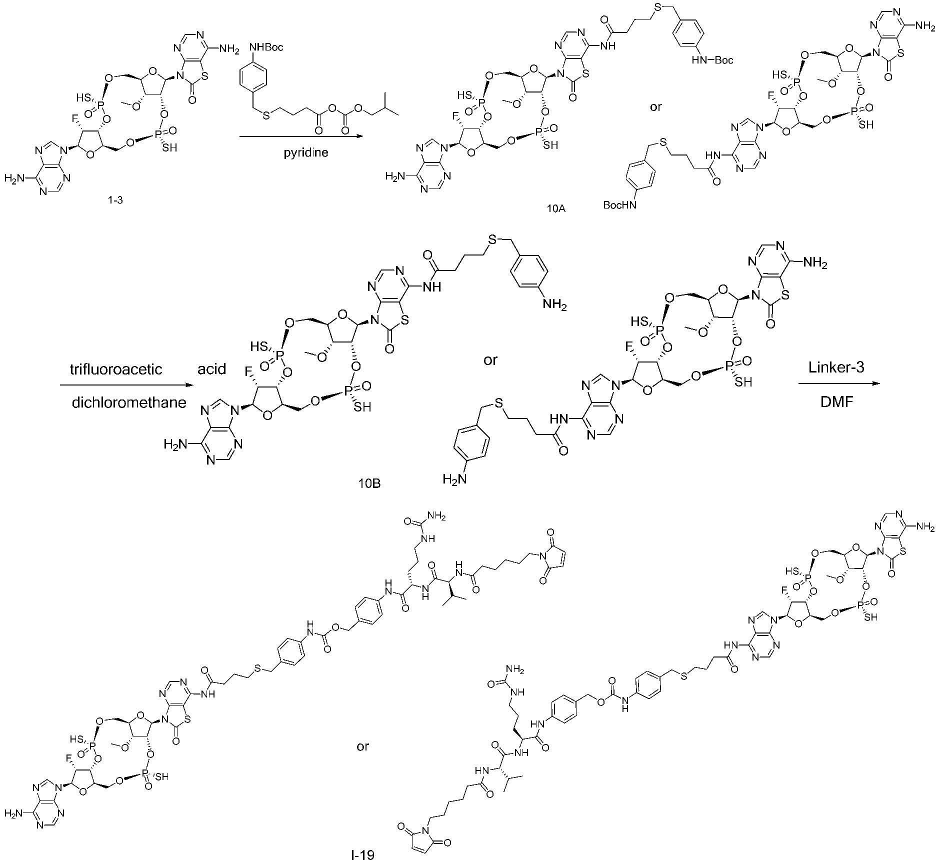

Steps 1 & 2: Following the synthetic method of compound 8B, compound 1-3 was reacted with 4-((4-((tert-butoxycarbonyl)amino)benzyl)thio)butanoic (isobutyl carbonic) anhydride to obtain compound 10B as a yellow solid. m/z: [M+H]⁺ 947.0.

Step 3: Following the synthetic method of Step 3 in I-15, compound 10B was reacted to obtain compound I-19 (*prep-*HPLC separation: mobile phase A: 10 mM ammonium bicarbonate aqueous solution, mobile phase B: acetonitrile; gradient elution with mobile phase B from 5% to 70%, elution time: 20 minutes) as a white solid. HPLC RT = 13.762 minutes; m/z: [1/2M+H]⁺ 773.2.

### Example 27: Synthesis of I-20

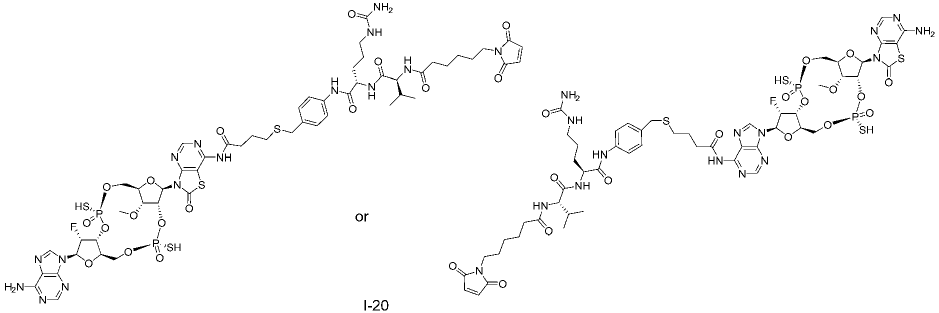

A mixed solution of Mc-Val-Cit-OH (7.9 mg, 17 µM), compound 10B (8 mg, 8.4 µM), and EEDQ (4.15 mg, 17 µM) in dichloromethane (0.4 mL) and methanol (0.2 mL) was stirred at room temperature for 2 hours. The reaction mixture was directly purified by *prep-*HPLC (mobile phase A: 10 mM ammonium bicarbonate aqueous solution, mobile phase B: acetonitrile; gradient elution with mobile phase B from 5% to 70%, elution time: 20 minutes) to obtain I-20 (3.73 mg, yield: 30%) as a white solid. HPLC RT = 13.114 minutes; m/z: [1/2M+H]⁺ 698.6.

### Example 28: Synthesis of I-21

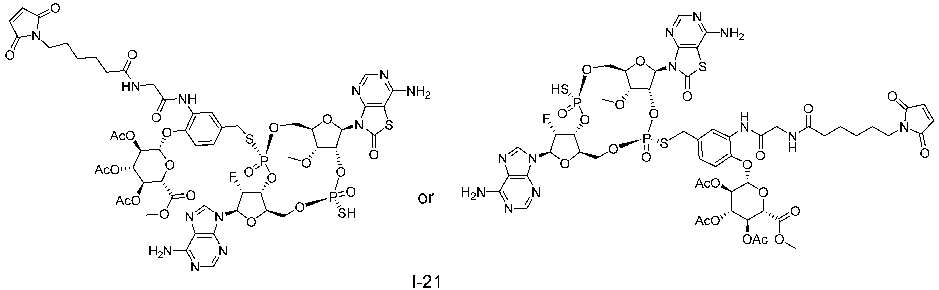

Following the synthetic method of I-3, compound 1-3 and Linker-9 were reacted to obtain I-21 (*prep-*HPLC*:* mobile phase A: 10 mM ammonium bicarbonate aqueous solution, mobile phase B: acetonitrile; gradient elution with mobile phase B from 5% to 65%, elution time: 20 minutes) as a white solid. HPLC RT = 14.432 minutes; m/z: [M+H]⁺ 1427.4.

### Example 29: Synthesis of I-22

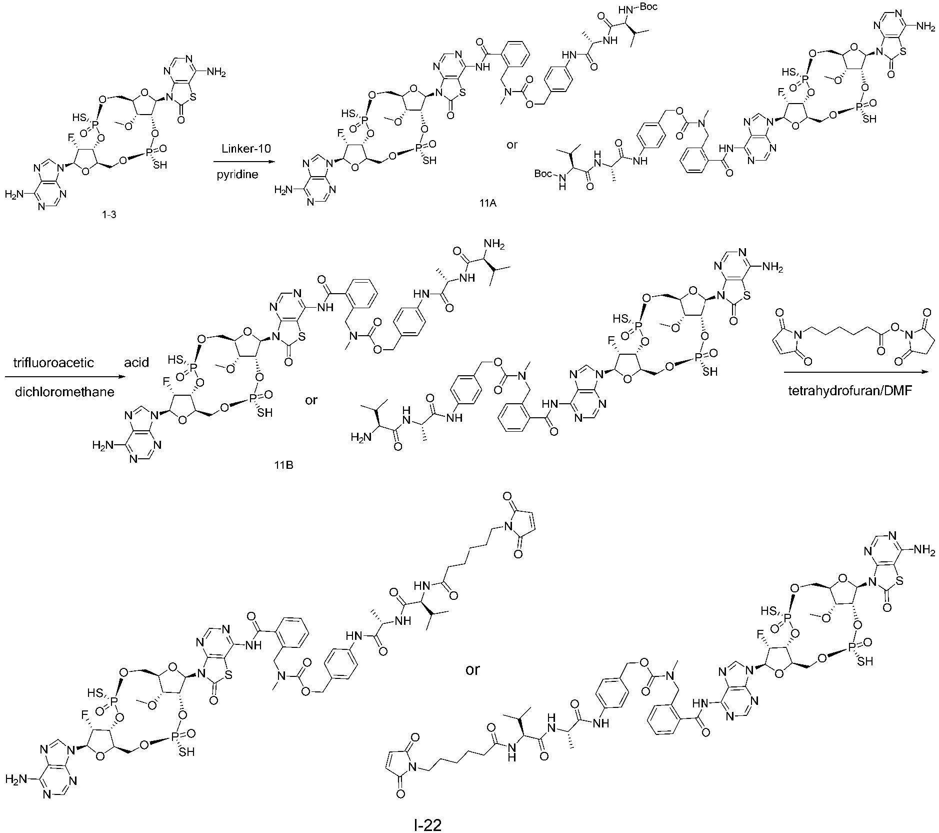

Steps 1 & 2: Following the synthetic method of compound 8B, compound 1-3 and Linker-10 were reacted to obtain compound 11B as a pale yellow solid. m/z: [1/2M+H]⁺ 603.6.

Step 3: To a mixed solution of compound 11B (5.6 mg, 0.018 mmol) and N-succinimidyl 6-maleimidohexanoate (6 mg, 0.02 mmol) in tetrahydrofuran (0.5 mL) and DMF (0.5 mL) was added DIPEA (3.1 mg, 0.024 mmol). The reaction mixture was stirred at room temperature for 1 hour and then directly purified by *prep-*HPLC (mobile phase A: 10 mM ammonium bicarbonate aqueous solution, mobile phase B: acetonitrile; gradient elution with mobile phase B from 5% to 70%, elution time: 20 minutes) to obtain compound I-22 (1.35 mg, yield: 8%) as a white solid. HPLC RT = 13.496 minutes; m/z: [1/2M+H]⁺ 700.2.

### Example 30: Synthesis of I-23

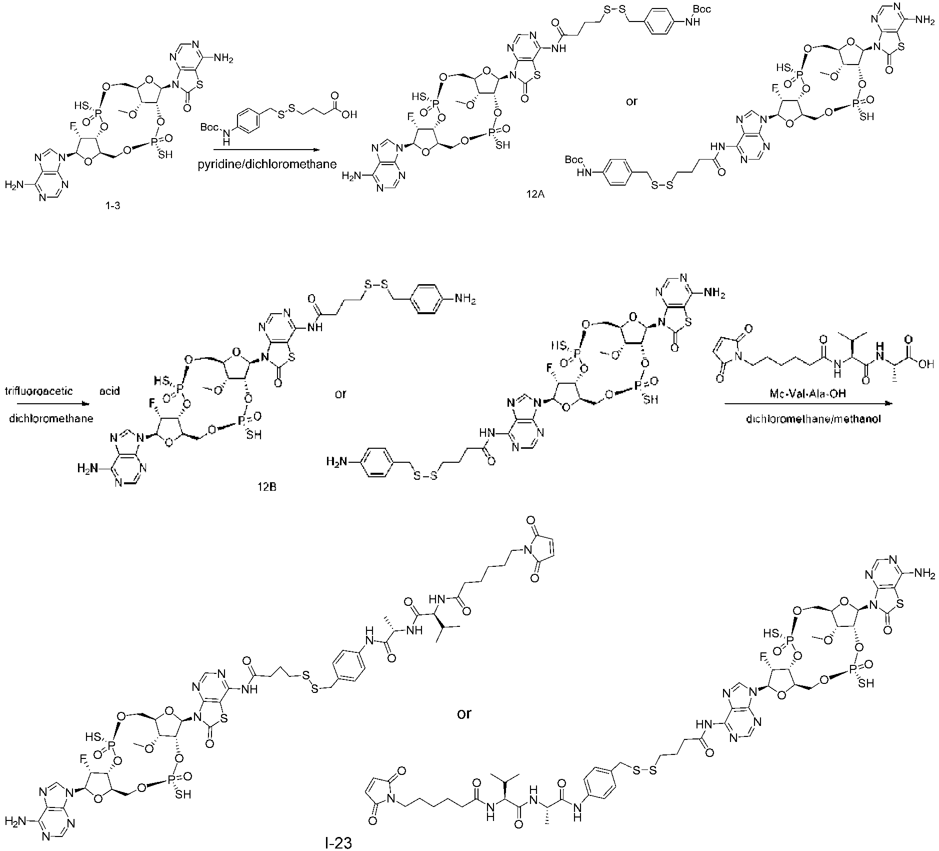

Step 1: To a solution of 4-((4-((*tert-*butoxycarbonyl)amino)benzyl)disulfanyl)butanoic acid (350 mg, 0.98 mmol) and triethylamine (141 mg, 1.4 mmol) in dichloromethane (10 mL) was added isobutyl chloroformate (134 mg, 0.98 mmol) under ice bath conditions. The reaction mixture was stirred at 0°C for 0.5 hours and then concentrated under reduced pressure. The residue was diluted with a small amount of dichloromethane and filtered through a short silica gel column, and the resulting filtrate was set aside for further use. Compound 1-3 (100 mg, 0.14 mmol) was dissolved in dry pyridine (2 mL) and concentrated under reduced pressure. This process was repeated twice, and the compound was redissolved in dry pyridine (3 mL). Under a nitrogen atmosphere and ice bath conditions, trimethylsilyl chloride (91 mg, 0.84 mmol) was added to the pyridine solution of compound 1-3. After the addition, the reaction mixture was stirred at room temperature for 0.5 hours, followed by the addition of the aforementioned dichloromethane filtrate. The reaction mixture was stirred at room temperature for an additional 3 hours, then concentrated under reduced pressure. The residue was purified by flash column chromatography (C18, eluent: 0-40% acetonitrile in 10 mM ammonium bicarbonate aqueous solution) to obtain compound 12A (125 mg, yield: 86%) as a white solid. m/z: [1/2M+H]⁺ 540.0.

Step 2: To a solution of 12A (120 mg, 0.11 mmol) in dichloromethane (10 mL) was added trifluoroacetic acid (3 mL) under ice bath conditions. The reaction mixture was stirred at 0°C for 2 hours, then concentrated under reduced pressure. The residue was purified by flash column chromatography (C18, eluent: 0-30% acetonitrile in 0.1% trifluoroacetic acid aqueous solution) to obtain compound 12B (55.6 mg, yield: 51%) as a white solid. m/z: [M+H]⁺ 979.0.

Step 3: Following the synthetic method of I-20, 12B was reacted to obtain I-23 (*prep-*HPLC: mobile phase A: 10 mM ammonium bicarbonate aqueous solution, mobile phase B: acetonitrile; gradient elution with mobile phase B from 5% to 70%, elution time: 20 minutes) as a white solid. HPLC RT = 13.798 minutes; m/z: [1/2M+H]⁺ 671.8.

### Example 31: Synthesis of I-24

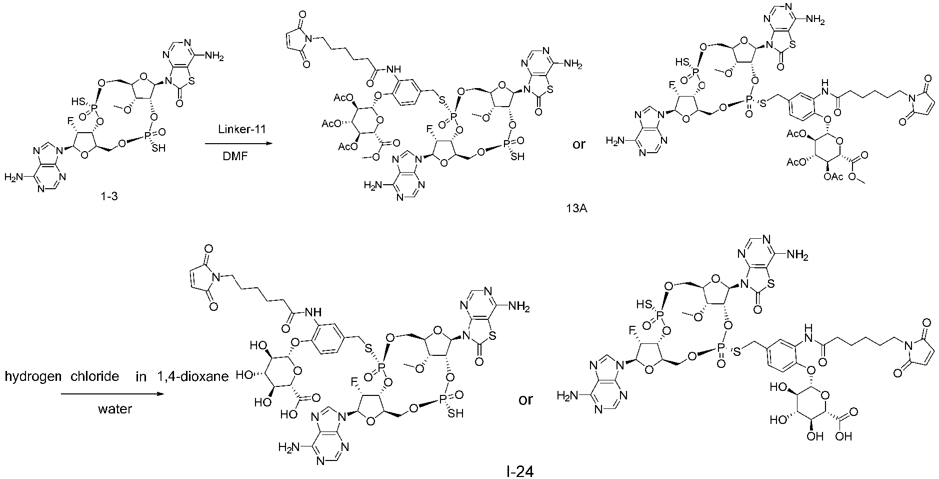

Step 1: To a solution of compound 1-3 (50 mg, 0.068 mmol) in DMF (3 mL) was added Linker-11 (52 mg, 0.068 mmol) under ice bath conditions. The reaction mixture was stirred at room temperature for 1.5 hours and then directly purified by flash column chromatography (C18, eluent: 0-75% acetonitrile in 10 mM ammonium bicarbonate aqueous solution) to obtain compound 13A (38 mg, yield: 41%) as a pale yellow solid. m/z: [1/2M+H]⁺ 685.8.

Step 2: A mixed solution of compound 13A (20 mg, 0.015 mmol) in water (0.5 mL) and hydrogen chloride-1,4-dioxane (0.5 mL, 4 M) was stirred at 38°C for 12 hours. The reaction mixture was concentrated under reduced pressure, and the residue was purified by *prep-*HPLC (mobile phase A: 10 mM ammonium bicarbonate aqueous solution, mobile phase B: acetonitrile; gradient elution with mobile phase B from 5% to 55%, elution time: 20 minutes) to obtain compound I-24 (5.76 mg, yield: 31%) as a white solid. HPLC RT = 11.793 minutes; m/z: [1/2M+H]⁺ 615.8.

### Example 32: Synthesis of I-25

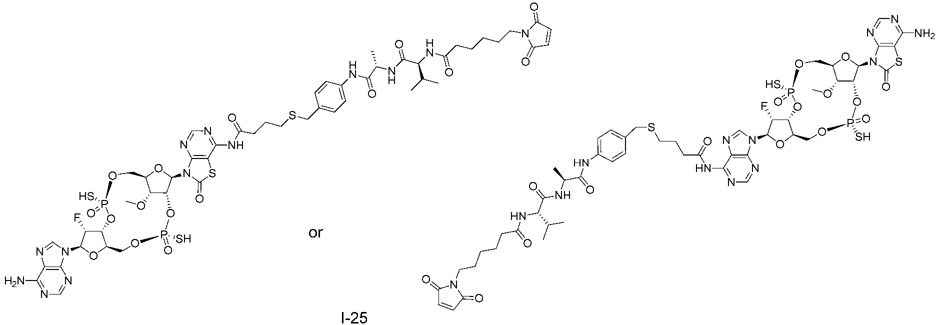

Following the synthetic method of compound I-20, compound 10B and Mc-Val-Ala-OH were reacted to obtain compound I-25 (flash column chromatography: C18, eluent: 0-60% acetonitrile in 10 mM ammonium bicarbonate aqueous solution) as a white solid. HPLC RT = 13.447 minutes; m/z: [M+H]⁺ 1310.0.

### Example 33: Synthesis of I-26

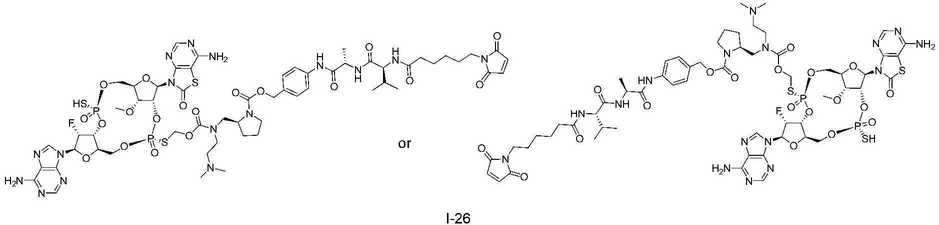

Following the synthetic method of compound I-17, Linker-3 in Step 3 was replaced with Linker-4 to react and obtain compound I-26 (*prep-*HPLC*:* mobile phase A: 10 mM ammonium bicarbonate aqueous solution, mobile phase B: acetonitrile; gradient elution with mobile phase B from 5% to 60%, elution time: 20 minutes) as a white solid. HPLC RT = 14.249 minutes; m/z: [M+H]⁺ 1478.9.

### Example 34: Synthesis of I-27

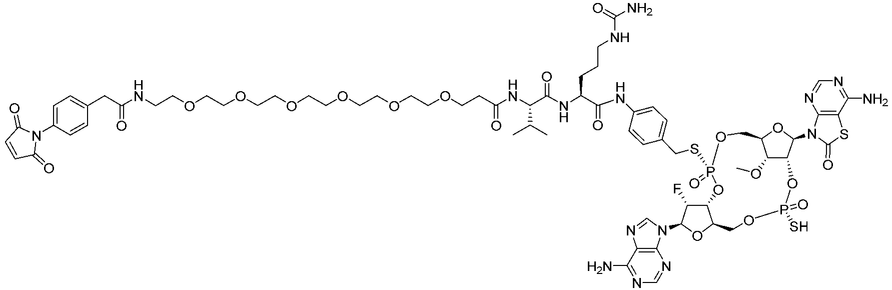

or

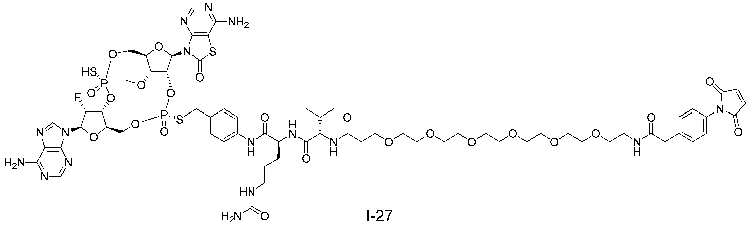

Following the synthetic method of I-3, compound 1-3 and Linker-13 were reacted to obtain I-27 (*prep-*HPLC*:* mobile phase A: 10 mM ammonium bicarbonate aqueous solution, mobile phase B: acetonitrile; gradient elution with mobile phase B from 3% to 50%, elution time: 20 minutes) as a white solid. HPLC RT = 13.656 minutes; m/z: [1/2M+H]⁺ 825.3.

### Example 35: Synthesis of I-28

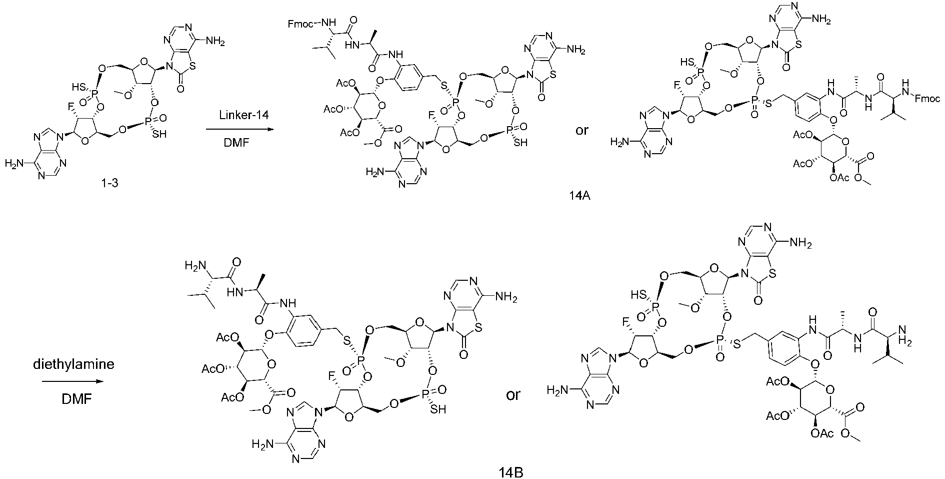

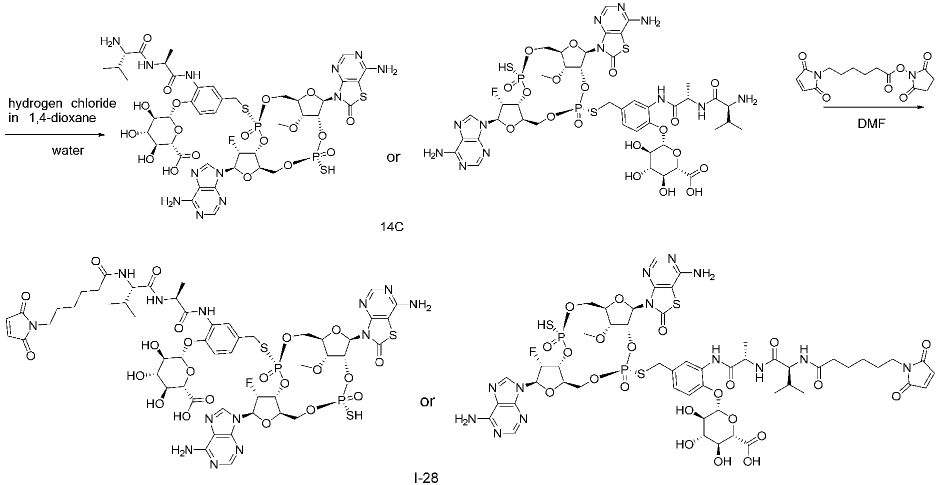

Step 1: Following the synthetic method of I-3, compound 1-3 was reacted with Linker-14 to obtain 14A as a white solid. m/z: [M+H]⁺ 1569.0.

Step 2: To a solution of compound 14A (70 mg, 0.045 mmol) in DMF (2 mL) was added diethylamine (16 mg, 0.22 mmol). The reaction mixture was stirred at room temperature for 1 hour and then directly purified by flash column chromatography (C18, eluent: 0-65% acetonitrile in 10 mM ammonium bicarbonate aqueous solution) to obtain compound 14B (55 mg, yield: 92%). m/z: [M+H]⁺ 1347.0.

Step 3: A solution of compound 14B (40 mg, 0.03 mmol) in water and hydrogen chloride-1,4-dioxane (2 mL, 4 M) was stirred at 20°C for 12 hours. The reaction mixture was directly purified by flash column chromatography (C18, eluent: 0-70% acetonitrile in 10 mM ammonium bicarbonate aqueous solution) to obtain compound 14C (35 mg, yield: 98%). m/z: [M+H]⁺ 1207.2.

Step 4: To a solution of compound 14C (18 mg, 0.015 mmol) in DMF (2 mL) were added *N*-succinimidyl 6-maleimidohexanoate (14 mg, 0.045 mmol) and 3 drops of DIPEA. The reaction mixture was stirred at room temperature for 1.5 hours. The reaction mixture was directly purified by *prep-*HPLC (mobile phase A: 10 mM ammonium bicarbonate aqueous solution, mobile phase B: acetonitrile; gradient elution with mobile phase B from 2% to 50%, elution time: 20 minutes) to obtain compound I-28 (3.1 mg, yield: 14%) as a white solid. HPLC RT = 13.946 minutes; m/z: [M+H]⁺ 1400.4.

### Example 36: Synthesis of I-29

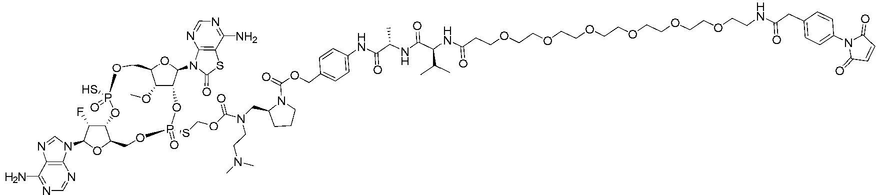

or

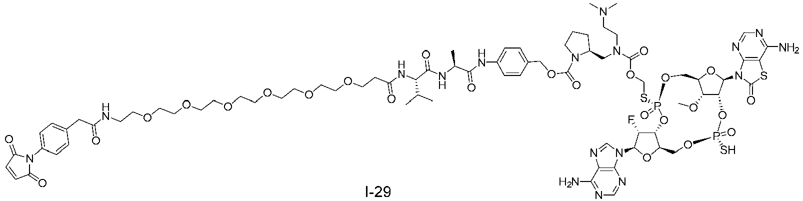

Following the synthetic method of compound I-17, Linker-3 in Step 3 was replaced with Linker-16 to react and obtain compound I-29 (*prep-*HPLC*:* mobile phase A: 10 mM ammonium bicarbonate aqueous solution, mobile phase B: acetonitrile; gradient elution with mobile phase B from 3% to 65%, elution time: 20 minutes) as a white solid. UPLC RT = 6.378 minutes; m/z: [1/2M+H]⁺ 917.0.

### Example 37: Synthesis of I-30

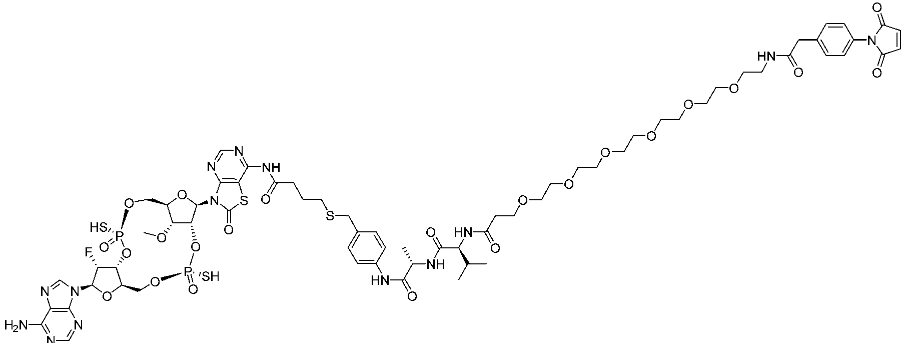

or

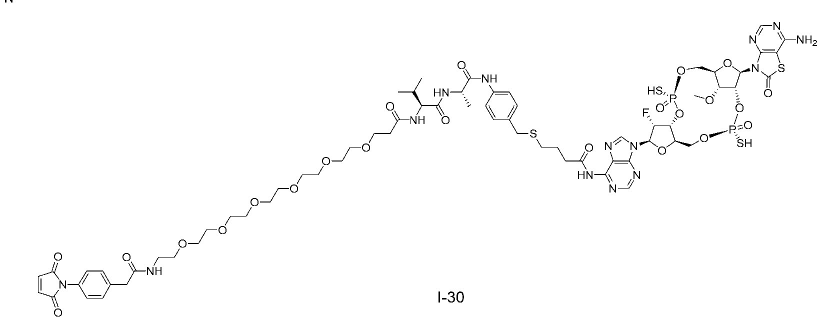

Following the synthetic method of compound I-20, compound 10B and Linker-17 were reacted to obtain compound I-30 (*prep-*HPLC*:* mobile phase A: 10 mM ammonium bicarbonate aqueous solution, mobile phase B: acetonitrile; gradient elution with mobile phase B from 5% to 70%, elution time: 20 minutes) as a white solid. UPLC RT = 13.414 minutes; m/z: [1/2M+H]⁺ 833.4.

### Example 38: Synthesis of I-31

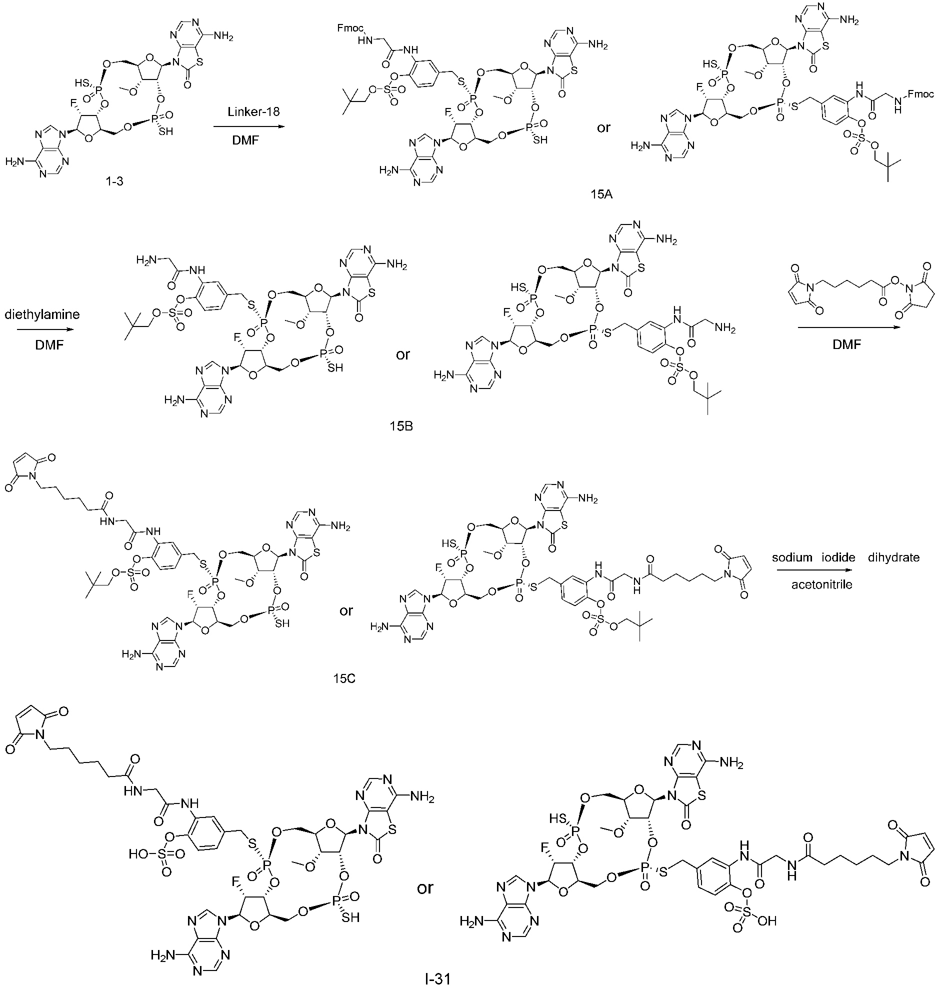

Step 1: Following the synthetic method of I-3, compound 1-3 was reacted with Linker-18 to obtain 15A as a white solid. m/z: [1/2M+H]⁺ 645.2.

Step 2: To a solution of compound 15A (50 mg, 0.039 mmol) in DMF (2 mL) was added diethylamine (14.2 mg, 0.20 mmol). The reaction mixture was stirred at room temperature for 1 hour and then directly purified by flash column chromatography (C18, eluent: 0-60% acetonitrile in 10 mM ammonium bicarbonate aqueous solution) to obtain compound 15B (55 mg, yield: 92%). m/z: [1/2M+H]⁺ 534.6.

Step 3: To a solution of compound 15B (18 mg, 0.015 mmol) in DMF (2 mL) were added *N*-succinimidyl 6-maleimidohexanoate (13 mg, 0.042 mmol) and 3 drops of DIPEA. The reaction mixture was stirred at room temperature for 1 hour. The reaction mixture was directly purified by flash column chromatography (C18, eluent: 0-60% acetonitrile in 0.1% trifluoroacetic acid aqueous solution) to obtain 15C (40 mg, yield: 97%) as a white solid. m/z: [1/2M+H]⁺ 631.2.

Step 4: To a solution of compound 15C (15 mg, 0.012 mmol) in acetonitrile (3 mL) was added sodium iodide dihydrate (12 mg, 0.06 mmol). The reaction mixture was stirred at 40°C for 20 hours. After cooling to room temperature, the reaction mixture was directly purified by *prep-*HPLC (mobile phase A: 10 mM ammonium bicarbonate aqueous solution, mobile phase B: acetonitrile; gradient elution with mobile phase B from 2% to 60%, elution time: 20 minutes) to obtain compound I-31 (1.2 mg, yield: 9%) as a white solid. HPLC RT = 12.657 minutes; m/z: [M+H]⁺ 1191.1.

### Example 39: Synthesis of I-32

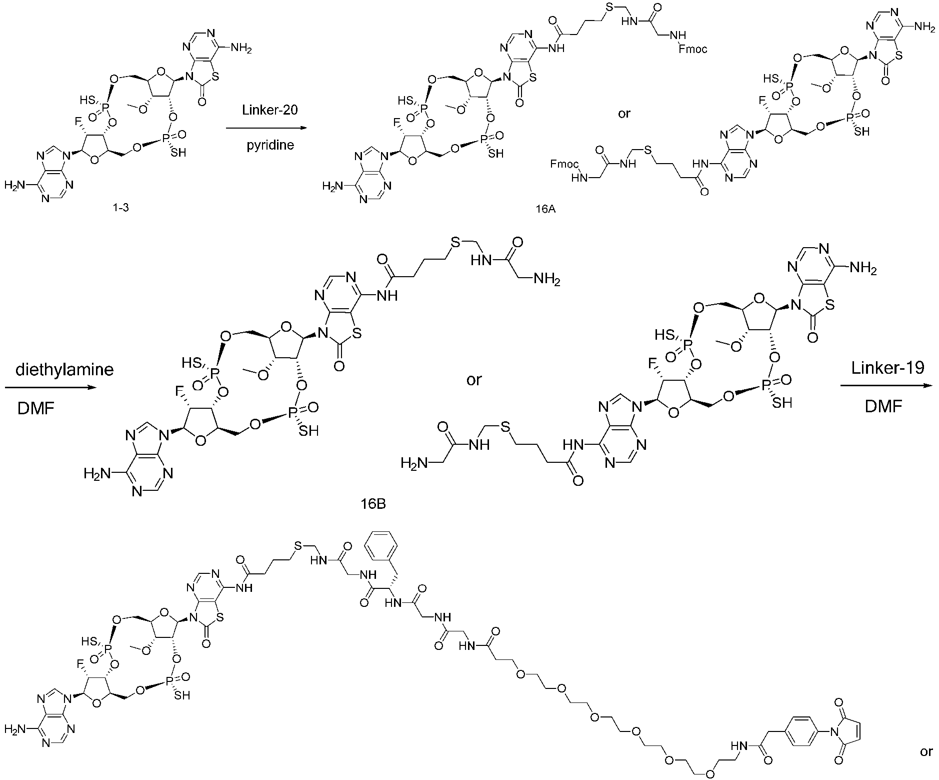

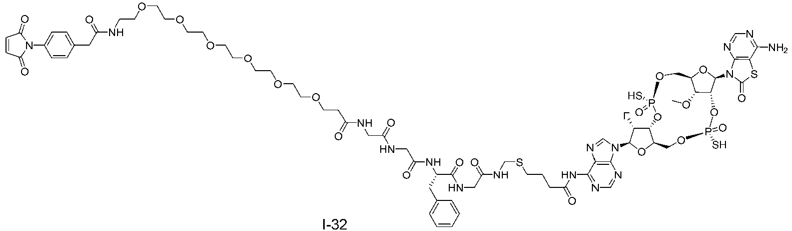

Step 1: Following the synthetic method of 8A, compound 1-3 was reacted with Linker-20 to obtain 16A as a white solid.

Step 2: To a solution of 16A (65 mg, 0.057 mmol) in DMF (2 mL) was added diethylamine (25 mg, 0.34 mmol). The reaction mixture was stirred at room temperature for 2 hours and directly purified by flash column chromatography (C18, eluent: 0-40% acetonitrile in 0.1% trifluoroacetic acid aqueous solution) to obtain 16B (30 mg, yield: 57%) as a white solid. m/z: [M+H]⁺ 928.2.

Step 3: Under ice bath conditions, a solution of Linker-19 (3 mg, 0.004 mmol), DIPEA (0.5 mL), and HATU (1.6 mg, 0.004 mmol) in DMF (0.5 mL) was stirred for 5 minutes. To the above reaction mixture was added compound 16B (3.3 mg, 0.004 mmol). The reaction mixture was stirred at room temperature overnight and then directly purified by *prep-*HPLC (mobile phase A: 10 mM ammonium bicarbonate aqueous solution, mobile phase B: acetonitrile; gradient elution with mobile phase B from 5% to 70%, elution time: 20 minutes) to obtain compound I-32 (1.8 mg, yield: 27%) as an off-white solid. HPLC RT = 13.021 minutes; m/z: [1/2M+H]⁺ 869.3.

### Example 40: Synthesis of I-33

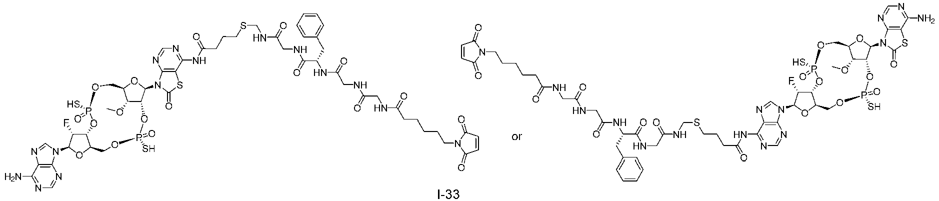

Following the synthetic method of I-32, Linker-19 in Step 3 was replaced with Mc-Gly-Gly-Phe-OH to obtain I-33 (*prep-*HPLC*:* mobile phase A: 10 mM ammonium bicarbonate aqueous solution, mobile phase B: acetonitrile; gradient elution with mobile phase B from 5% to 80%, elution time: 20 minutes) as a white solid. UPLC RT = 10.141 minutes; m/z: [1/2M+H]⁺ 691.8.

### Example 41: Synthesis of I-34

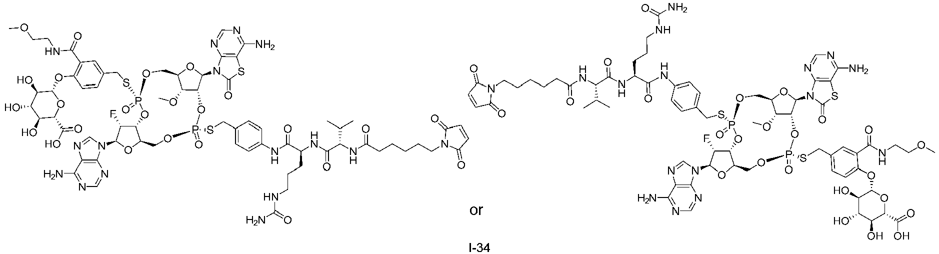

To a solution of compound 4 (10 mg, 0.009 mmol) in DMF (2 mL) was added Linker-2 (7.3 mg, 0.011 mmol). The reaction mixture was stirred at room temperature for 1 hour and then directly purified by *prep-*HPLC (mobile phase A: 10 mM ammonium bicarbonate aqueous solution, mobile phase B: acetonitrile; gradient elution with mobile phase B from 5% to 65%, elution time: 20 minutes) to obtain compound I-34 (2 mg, yield: 13%) as a white solid. HPLC RT = 13.860 minutes; m/z: [1/2M+H]⁺ 838.8.

### Example 42: Synthesis of I-35

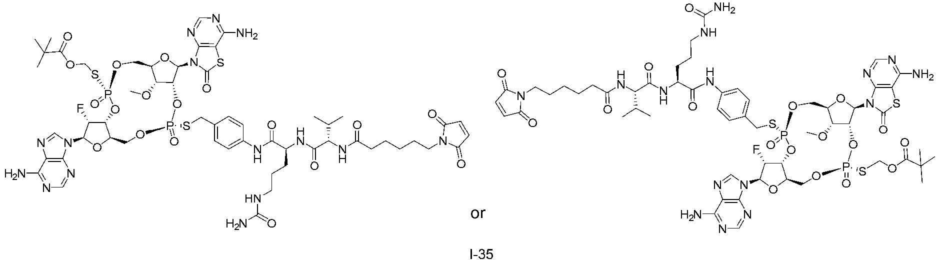

To a solution of compound 5 (20 mg, 0.023 mmol) in DMF (2 mL) was added Linker-2 (16 mg, 0.023 mmol). The reaction mixture was stirred at room temperature for 1 hour and then directly purified by *prep-*HPLC (mobile phase A: 10 mM ammonium bicarbonate aqueous solution, mobile phase B: acetonitrile; gradient elution with mobile phase B from 5% to 50%, elution time: 20 minutes) to obtain compound I-35 (12.3 mg, yield: 35%) as a white solid. HPLC RT = 16.147 minutes; m/z: [1/2M+H]⁺ 704.8.

### Example 43: Synthesis of I-36

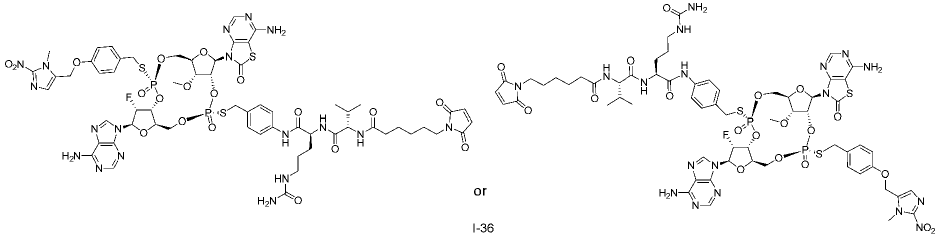

To a solution of compound 2 (18 mg, 0.018 mmol) in DMF (1 mL) was added Linker-2 (16 mg, 0.023 mmol). The reaction mixture was stirred at room temperature for 1 hour and then directly purified by *prep-*HPLC (mobile phase A: 10 mM ammonium bicarbonate aqueous solution, mobile phase B: acetonitrile; gradient elution with mobile phase B from 10% to 30%, elution time: 20 minutes) to obtain compound I-36 (7.8 mg, yield: 27%) as a white solid. HPLC RT = 15.783 minutes; m/z: [1/2M+H]⁺ 770.4.

### Example 44: Synthesis of I-37

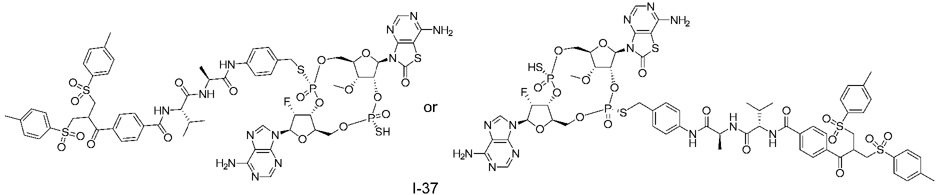

Following the synthetic method of I-3, compound 1-3 and Linker-22 were reacted to obtain I-37 (*prep-*HPLC*:* mobile phase A: 10 mM ammonium bicarbonate aqueous solution, mobile phase B: acetonitrile; gradient elution with mobile phase B from 3% to 75%, elution time: 20 minutes) as a white solid. HPLC RT = 15.308 minutes; m/z: [M+H]⁺ 1497.4.

### Example 45: Synthesis of I-38

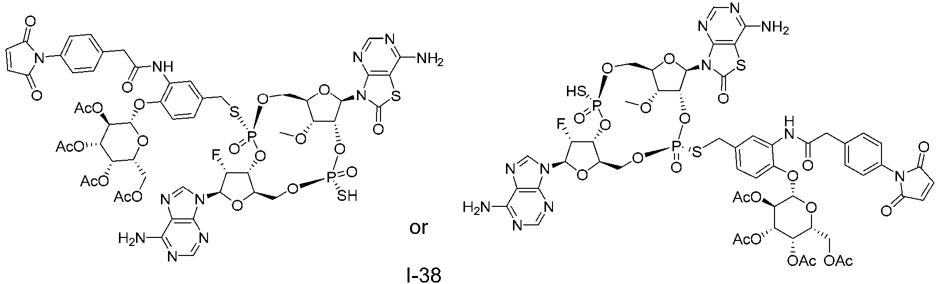

Following the synthetic method of I-3, compound 1-3 was reacted with Linker-24 to obtain I-38 (flash column chromatography: C18, eluent: 0%-48% acetonitrile in 10 mM ammonium bicarbonate aqueous solution) as a white solid. UPLC RT = 7.599 minutes; m/z: [M+H]⁺ 1404.4. ³¹P NMR (162MHz, DMSO-d₆+D₂O): δ 52.848, 27.645.

### Example 46: Synthesis of I-39

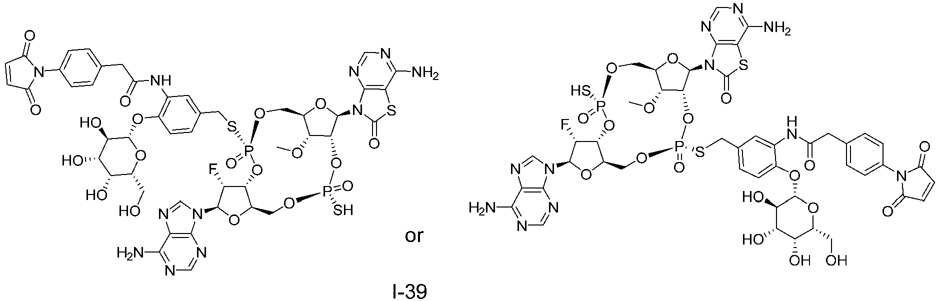

To a solution of compound I-38 (25 mg, 0.018 mmol) in water (1.5 mL) was added hydrogen chloride/1,4-dioxane (4 M, 1.5 mL). The reaction mixture was stirred at room temperature for 20 hours. The reaction mixture was directly purified by *prep-*HPLC (mobile phase A: 10 mM ammonium bicarbonate aqueous solution, mobile phase B: acetonitrile; gradient elution with mobile phase B from 5% to 70%, elution time: 20 minutes) to obtain compound I-39 (0.77 mg, yield: 3%) as a white solid. UPLC RT = 4.985 minutes; m/z: [M+H]⁺ 1236.1.

### Example 47: Synthesis of I-40

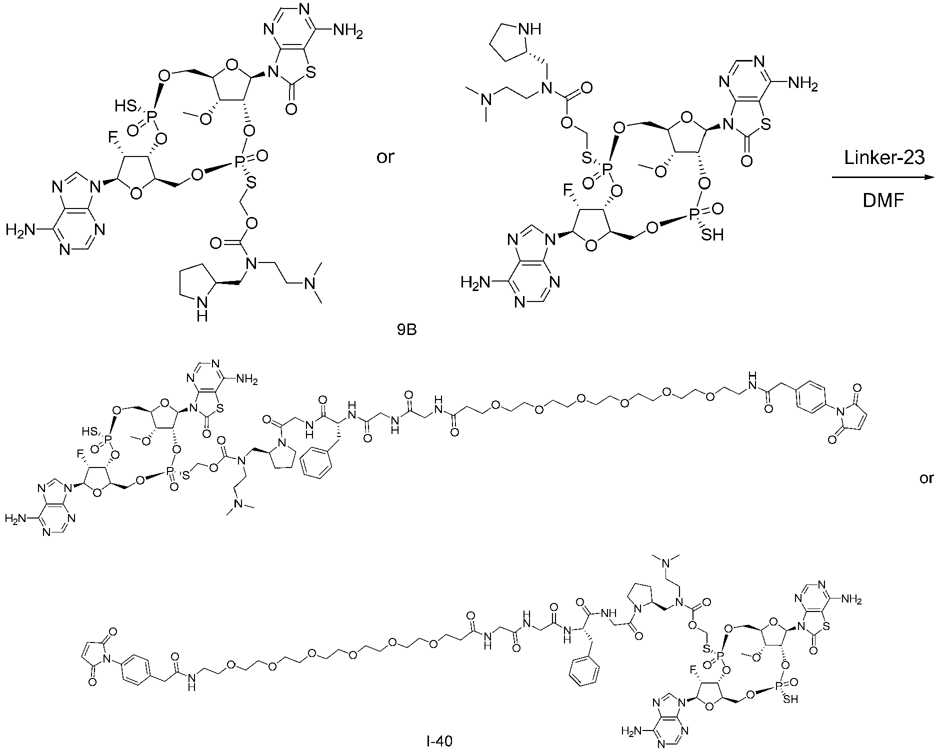

To a solution of Linker-23 (12 mg, 0.012 mmol) and compound 9B (11.6 mg, 0.012 mmol) in DMF (0.5 mL) was added DIPEA (2.3 mg, 0.02 mmol) under ice bath conditions. The reaction mixture was stirred at room temperature for 1 hour and then directly purified by *prep-*HPLC (mobile phase A: 10 mM ammonium bicarbonate aqueous solution, mobile phase B: acetonitrile; gradient elution with mobile phase B from 5% to 50%, elution time: 20 minutes) to obtain compound I-40 (4.3 mg, yield: 19%) as a white solid. UPLC RT = 5.459 minutes; m/z: [1/2M+H]⁺ 917.5.

### Example 48: Synthesis of I-41

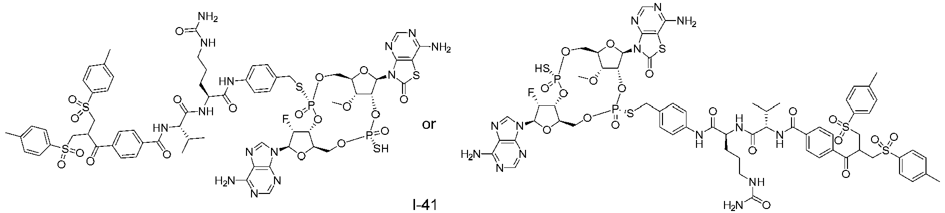

Following the synthetic method of I-3, compound 1-3 and Linker-25 were reacted to obtain I-41 (*prep-*HPLC*:* mobile phase A: 10 mM ammonium bicarbonate aqueous solution, mobile phase B: acetonitrile; gradient elution with mobile phase B from 5% to 75%, elution time: 20 minutes) as a white solid. UPLC RT = 7.650 minutes; m/z: [M+H]⁺ 1583.3.

### Example 49: Synthesis of I-42

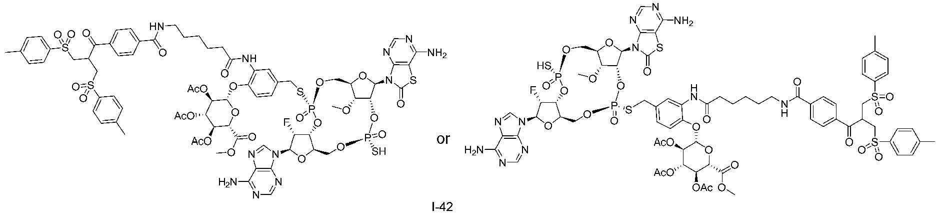

Following the synthetic method of I-3, compound 1-3 and Linker-26 were reacted to obtain I-42 (*prep-*HPLC*:* mobile phase A: 10 mM ammonium bicarbonate aqueous solution, mobile phase B: acetonitrile; gradient elution with mobile phase B from 5% to 85%, elution time: 20 minutes) as a white solid. UPLC RT = 8.348 minutes; m/z: [1/2M+H]⁺ 886.8.

### Example 50: Synthesis of I-43

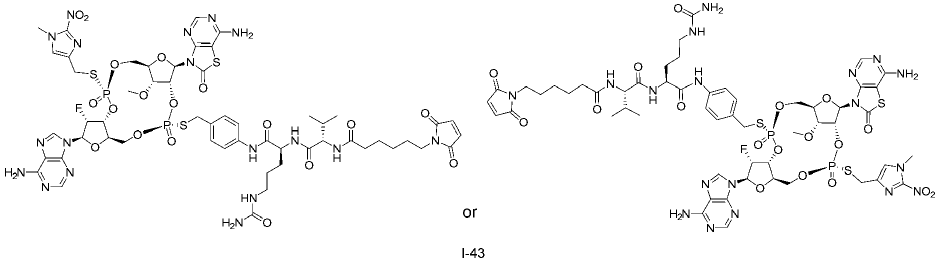

To a solution of compound I-3 (22 mg, 0.017 mmol) in DMF (2 mL) were added sodium iodide (7.64 mg, 0.051 mmol) and 4-(chloromethyl)-1-methyl-2-nitro-1*H*- imidazole (3 mg, 0.017 mmol). The reaction mixture was stirred at room temperature for 2.5 hours and then directly purified by *prep-*HPLC (mobile phase A: 10 mM ammonium bicarbonate aqueous solution, mobile phase B: acetonitrile; gradient elution with mobile phase B from 5% to 60%, elution time: 20 minutes) to obtain compound I-43 (2.5 mg, yield: 10%) as a white solid. UPLC RT = 6.180 minutes; m/z: [1/2M+H]⁺ 717.2.

### Example 51: Synthesis of I-44

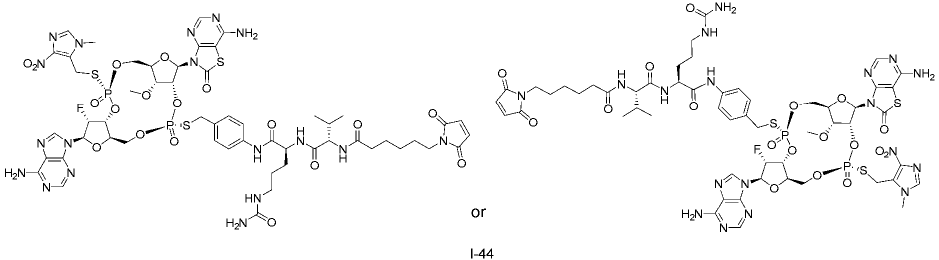

Following the synthetic method of I-43, compound I-3 was reacted with 5-(chloromethyl)-1-methyl-4-nitro-1*H*-imidazole to obtain I-44 (flash column chromatography: eluent: 0-55% acetonitrile in 0.1% trifluoroacetic acid aqueous solution) as a white solid. UPLC RT = 6.039 minutes; m/z: [1/2M+H]⁺ 717.2.

### Example 52: Synthesis of I-45

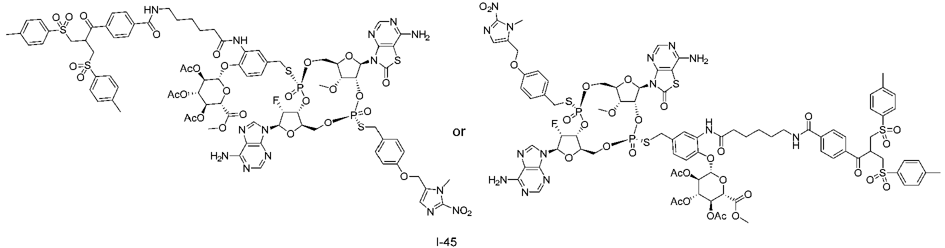

Following the synthetic method of I-36, compound 2 and Linker-26 were reacted to obtain I-45 (*prep-*HPLC*:* mobile phase A: 10 mM ammonium bicarbonate aqueous solution, mobile phase B: acetonitrile; gradient elution with mobile phase B from 5% to 75%, elution time: 20 minutes) as a white solid. UPLC RT = 7.765 minutes; m/z: [1/2M+CH₃CN+H]⁺ 1050.4.

### Example 53: Synthesis of I-46

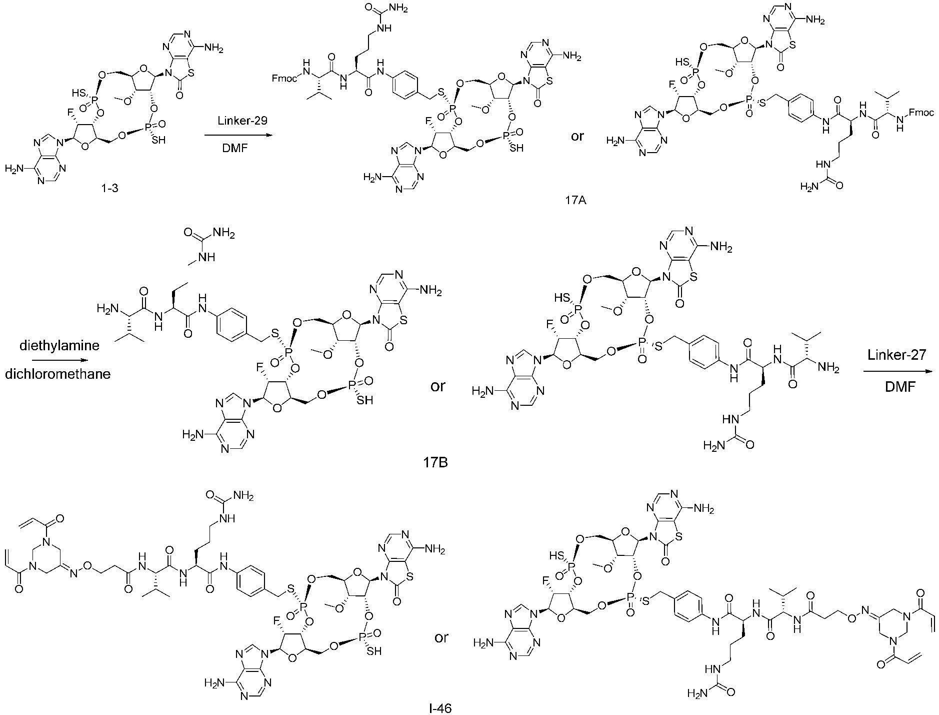

Step 1: Compound 1-3 (90 mg, 0.12 mmol) and Linker-29 (94 mg, 0.13 mmol) in DMF (5 mL) were stirred at room temperature for 1 hour, then directly purified by flash column chromatography (C18, eluent: 0-45% acetonitrile in 10 mM ammonium bicarbonate aqueous solution) to obtain 17A (40 mg, yield: 20%) as an off-white solid. m/z: [1/2M+H]⁺ 661.5.

Step 2: To a solution of 17A (40 mg, 0.03 mmol) in DMF (5 mL) was added diethylamine (22 mg, 0.3 mmol). The reaction mixture was stirred at room temperature overnight and directly purified by flash column chromatography (C18, eluent: 0-45% acetonitrile in 0.05% trifluoroacetic acid aqueous solution) to obtain 17B (25 mg, yield: 52%) as a white solid. m/z: [1/2M+H]⁺ 551.2.

Step 3: Under ice bath conditions, a solution of 17B (8 mg, 0.007 mmol), Linker-27 (4.1 mg, 0.008 mmol), DIPEA (3 mg, 0.022 mmol), and HATU (3 mg, 0.008 mmol) in DMF (3 mL) was stirred for 2 hours. The reaction mixture was directly purified by *prep-*HPLC (mobile phase A: 10 mM ammonium bicarbonate aqueous solution, mobile phase B: acetonitrile; gradient elution with mobile phase B from 5% to 45%, elution time: 20 minutes) to obtain compound I-46 (1.03 mg, yield: 11%) as a white solid. UPLC RT = 5.702 minutes; m/z: [M+H]⁺ 1378.5.

### Example 54: Synthesis of I-47

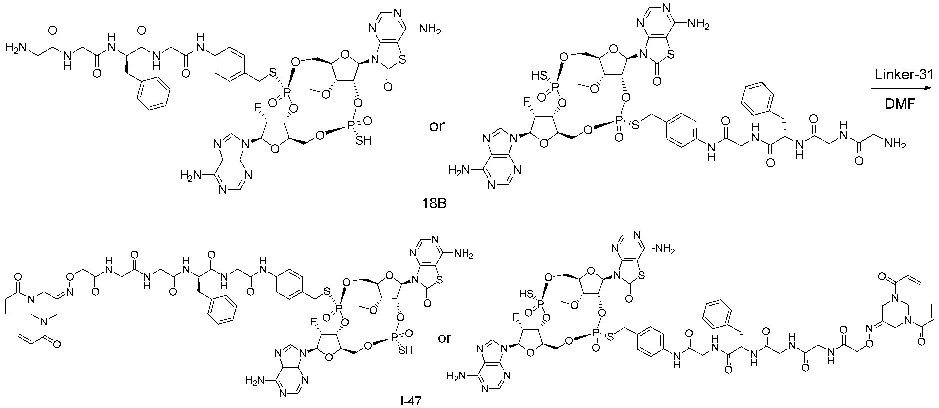

Synthesis of 18B: Following the synthetic method of 17B, compound 1-3 was reacted with Linker-30 to obtain 18B as a white solid. m/z: [1/2M+H]⁺ 582.5.

Following the synthetic method of I-46, compound 18B and Linker-31 were reacted to obtain I-47 (*prep-*HPLC*:* mobile phase A: 0.1% trifluoroacetic acid aqueous solution, mobile phase B: acetonitrile; gradient elution with mobile phase B from 5% to 45%, elution time: 20 minutes) as a white solid. UPLC RT = 6.211 minutes; m/z: [1/2M+H]⁺ 713.8.

### Example 55: Synthesis of I-48

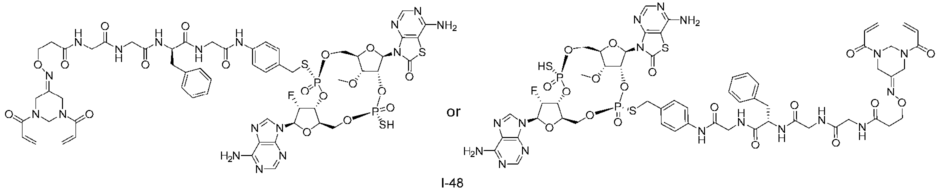

Following the synthetic method of I-46, compound 18B and Linker-27 were reacted to obtain I-48 (*prep-*HPLC*:* mobile phase A: 0.1% trifluoroacetic acid aqueous solution, mobile phase B: acetonitrile; gradient elution with mobile phase B from 5% to 45%, elution time: 20 minutes) as a white solid. UPLC RT = 6.021 minutes; m/z: [1/2M+H]⁺ 720.7.

### Example 56: Synthesis of I-49

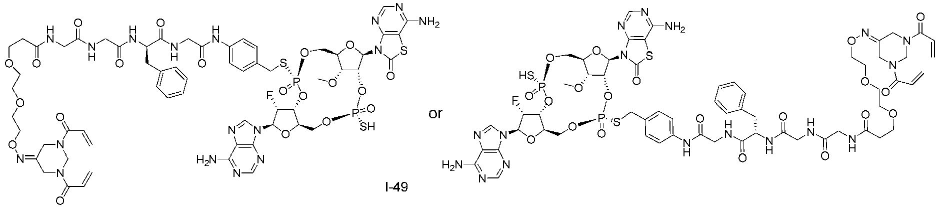

Following the synthetic method of I-46, compound 18B and Linker-28 were reacted to obtain I-49 (*prep-*HPLC*:* mobile phase A: 0.1% trifluoroacetic acid aqueous solution, mobile phase B: acetonitrile; gradient elution with mobile phase B from 5% to 45%, elution time: 20 minutes) as a white solid. UPLC RT = 6.420 minutes; m/z: [1/2M+H]⁺ 764.8.

### Example 57: Synthesis of I-50

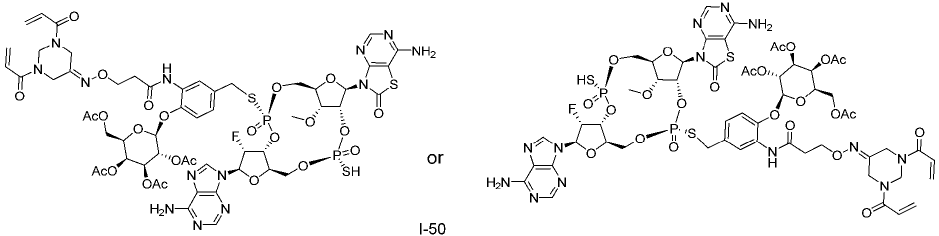

To a solution of compound 1-3 (10 mg, 0.014 mmol) and Linker-32 (9 mg, 0.013 mmol) in DMF (2 mL) was added sodium iodide (4 mg, 0.028 mmol). The reaction mixture was stirred at room temperature for 2 days and directly purified by *prep-*HPLC (mobile phase A: 10 mM ammonium bicarbonate aqueous solution, mobile phase B: acetonitrile; gradient elution with mobile phase B from 5% to 75%, elution time: 20 minutes) to obtain compound I-50 (3 mg, yield) as a white solid. UPLC RT = 7.140 minutes; m/z: [1/2M+H]⁺ 734.8. ³¹P NMR (162MHz, DMSO-d₆+D₂O): δ 52.902, 27.667.

### Example 58: Synthesis of I-51

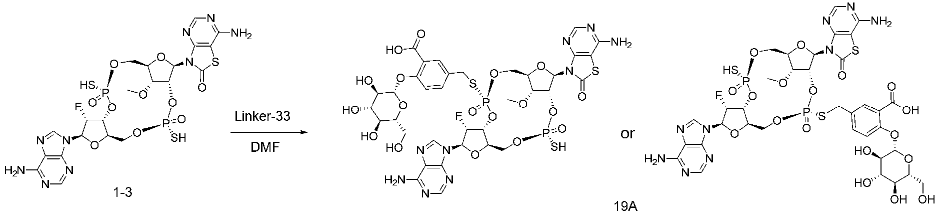

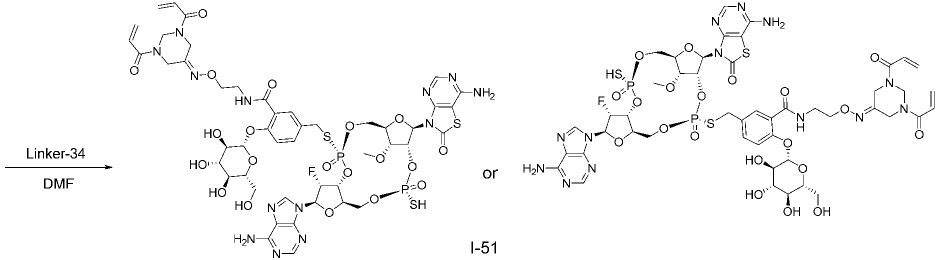

Step 1: A solution of compound 1-3 (74 mg, 0.1 mmol), Linker-33 (35 mg, 0.1 mmol), and sodium iodide (18 mg, 0.12 mmol) in DMF (1.2 mL) was stirred at room temperature for 5 hours, then directly purified by flash column chromatography (C18, 0-65% acetonitrile in 0.1% trifluoroacetic acid aqueous solution) to obtain 19A (45 mg, yield: 43%) as a pale yellow solid. m/z: [1/2M+H]⁺ 526.5.

Step 2: Under ice bath conditions, a solution of 19A (45 mg, 0.043 mmol), Linker-34 (10 mg, 0.038 mmol), DIPEA (14.7 mg, 0.11 mmol) and HATU (21.7 mg, 0.057 mmol) in DMF (1.2 mL) was stirred for 2 hours. The reaction mixture was directly purified by *prep-*HPLC (mobile phase A: 10 mM ammonium bicarbonate aqueous solution, mobile phase B: acetonitrile; gradient elution with mobile phase B from 15% to 70%, elution time: 20 minutes) to obtain compound I-51 (22 mg, yield: 44%) as a white solid. UPLC RT = 4.785 minutes; m/z: [1/2M+H]⁺ 650.7.

### Example 59: Synthesis of I-52

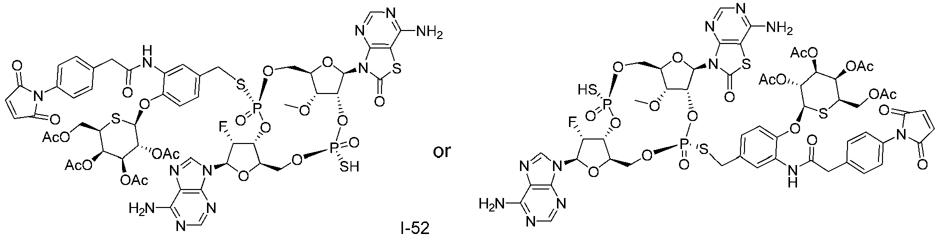

Following the synthetic method of I-50, compound 1-3 and Linker-35 were reacted to obtain I-52 (*prep-*HPLC*:* mobile phase A: 10 mM ammonium bicarbonate aqueous solution, mobile phase B: acetonitrile; gradient elution with mobile phase B from 5% to 75%, elution time: 20 minutes) as a white solid. HPLC RT = 14.703 minutes; m/z: [M+H]⁺ 1420.9.

### Example 60: Synthesis of I-53

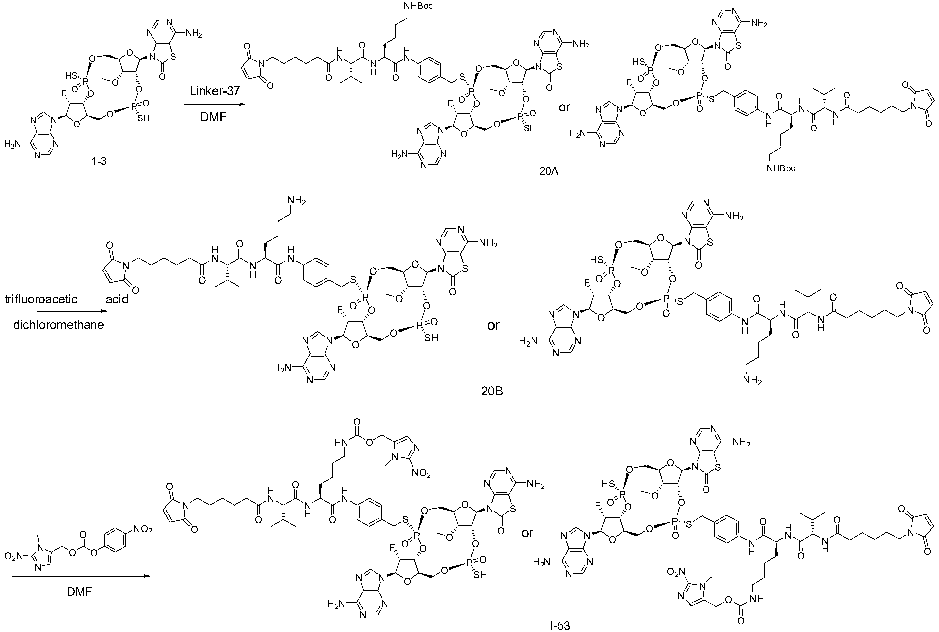

Steps 1 & 2: Following the synthetic method of compound 4B, compound 1-3 and Linker-37 were reacted to obtain compound 20B as a white solid. m/z: [M+H]⁺ 1265.5.

Step 3: To a solution of compound 20B (20 mg, 0.016 mmol) in DMF (1 mL) were added (1-methyl-2-nitro-1*H*-imidazol-5-yl)methyl (4-nitrophenyl) carbonate (6 mg, 0.018 mmol) and DIPEA (4 mg, 0.032 mmol). The reaction mixture was stirred at room temperature for 3 hours and then directly purified by *prep-*HPLC (mobile phase A: 10 mM ammonium bicarbonate aqueous solution, mobile phase B: acetonitrile; gradient elution with mobile phase B from 10% to 85%, elution time: 20 minutes) to obtain compound I-53 (5 mg, yield: 22%) as a white solid. UPLC RT = 7.240 minutes; m/z: [M+H]⁺ 1448.5.

### Example 61: Synthesis of I-54

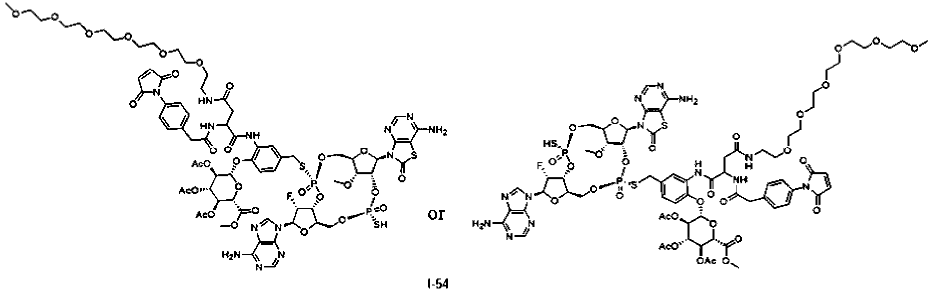

Following the synthetic method of I-3, compound 1-3 and Linker-38 were reacted to obtain I-54 (*prep-*HPLC*:* mobile phase A: 10 mM ammonium bicarbonate aqueous solution, mobile phase B: acetonitrile; gradient elution with mobile phase B from 5% to 70%, elution time: 20 minutes) as a white solid. UPLC RT = 7.233 minutes; m/z: [M+H]⁺ 1782.5.

### Example 62: Synthesis of I-55

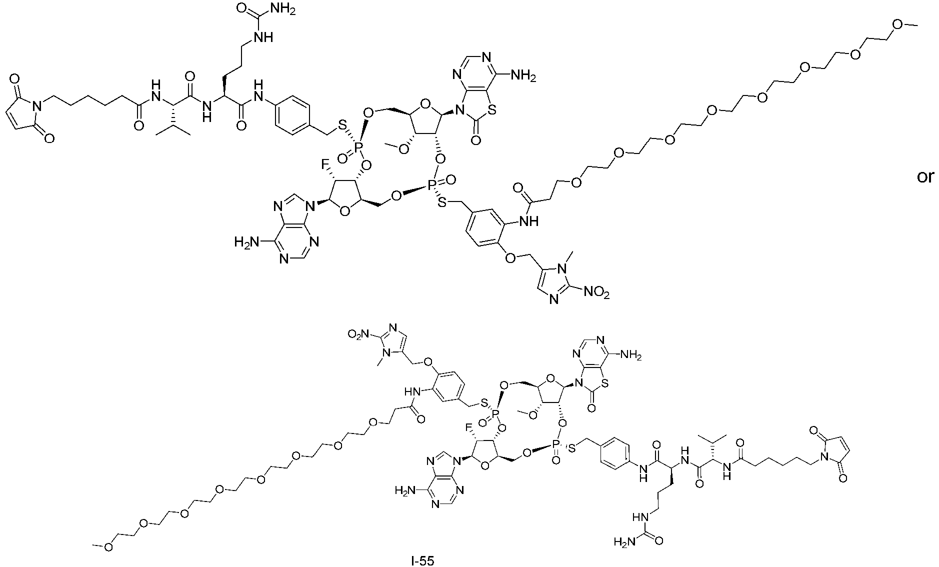

To a solution of I-3 (22 mg, 0.017 mmol) and sodium iodide (10.3 mg, 0.069 mmol) in DMF (2 mL) was added dropwise a solution of *N*-(5-(chloromethyl)-2-((1-methyl-2-nitro-1*H-*imidazol-5-yl)methoxy)phenyl)-2,5,8,11,14,17,20,23-octaoxahexacosan-26-amine (22.3 mg, 0.023 mmol) in acetonitrile (1 mL) under ice bath conditions. The reaction mixture was stirred at room temperature for 2 days. The reaction mixture was directly purified by *prep-*HPLC (mobile phase A: 0.1% trifluoroacetic acid aqueous solution, mobile phase B: acetonitrile; gradient elution with mobile phase B from 5% to 55%, elution time: 20 minutes) to obtain I-55 (6.25 mg, yield: 12%) as a white solid. UPLC RT = 6.864 minutes; m/z:[1/2M+H]⁺ 974.8.

### Example 63: Synthesis of I-56

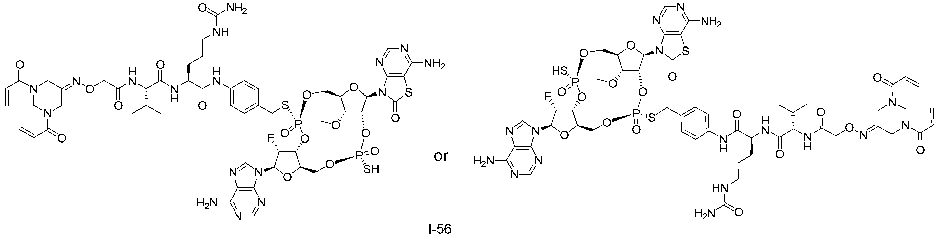

Following the synthetic method of I-46, Linker-27 in Step 3 was replaced with Linker-31 to obtain I-56 (C18, eluent: 0-40% acetonitrile in 10 mM ammonium bicarbonate aqueous solution) as a white solid. UPLC RT = 5.722 minutes; m/z: [1/2M+H]⁺ 682.8.

### Example 64: Synthesis of I-57

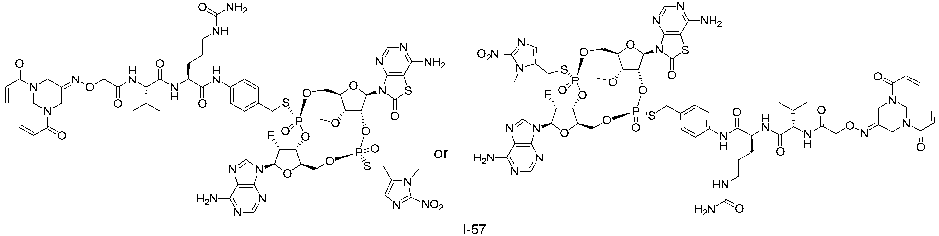

Following the synthetic method of I-43, I-56 was reacted with 5-(chloromethyl)-1-methyl-2-nitro-1*H*-imidazole to obtain I-57 (*prep-*HPLC*:* mobile phase A: 0.1% trifluoroacetic acid aqueous solution, mobile phase B: acetonitrile; gradient elution with mobile phase B from 5% to 85%, elution time: 20 minutes) as a white solid. UPLC RT = 6.160 minutes; m/z: [1/2M+H]⁺ 752.8.

### Example 65: Synthesis of I-58

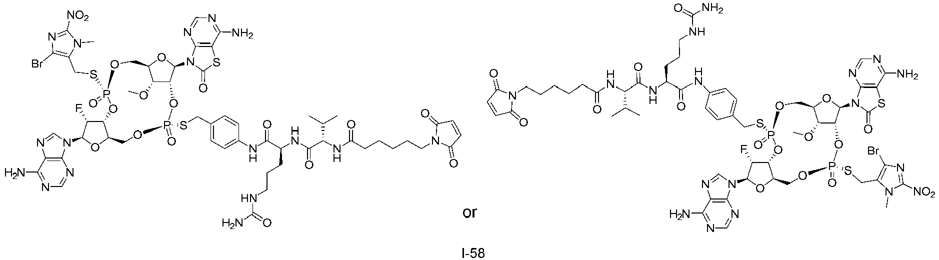

Following the synthetic method of I-43, I-3 was reacted with 4-bromo-5-(chloromethyl)-1-methyl-2-nitro-1*H*-imidazole to obtain I-58 (*prep-*HPLC*:* mobile phase A: 0.1% trifluoroacetic acid aqueous solution, mobile phase B: acetonitrile; gradient elution with mobile phase B from 5% to 50%, elution time: 20 minutes) as a white solid. UPLC RT = 6.730 minutes; m/z: [1/2M+H]⁺ 756.2.

### Example 66: Synthesis of I-59

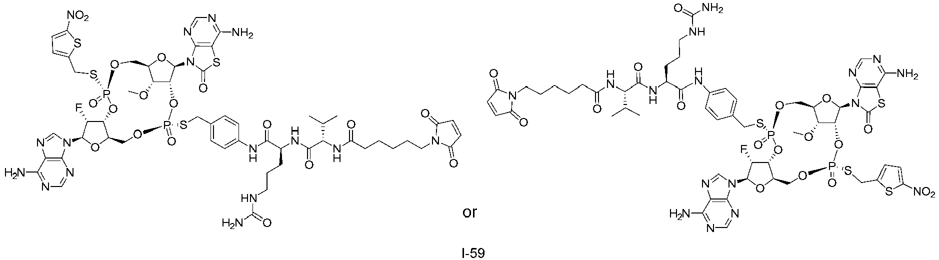

Following the synthetic method of I-3, compound 6 and Linker-2 were reacted to obtain I-59 (*prep-*HPLC*:* mobile phase A: 10 mM ammonium bicarbonate aqueous solution, mobile phase B: acetonitrile; gradient elution with mobile phase B from 5% to 70%, elution time: 20 minutes) as a white solid. UPLC RT = 7.007 minutes; m/z: [M+H]⁺ 1435.3.

### Example 67: Synthesis of I-60

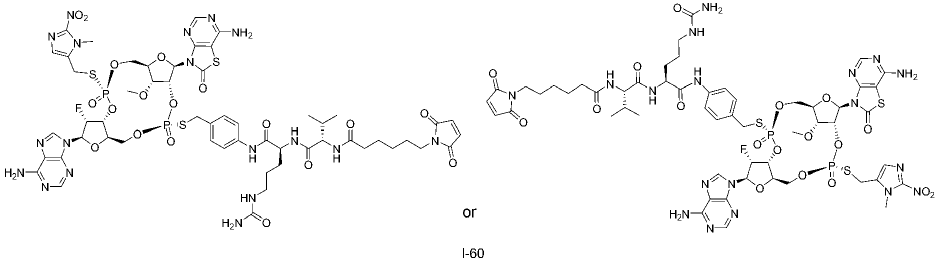

Following the synthetic method of I-43, I-3 was reacted with 5-(chloromethyl)-1-methyl-2-nitro-1*H*-imidazole to obtain I-60 (*prep-*HPLC*:* mobile phase A: 10 mM ammonium bicarbonate aqueous solution, mobile phase B: acetonitrile; gradient elution with mobile phase B from 5% to 85%, elution time: 20 minutes) as a white solid. UPLC RT = 6.539 minutes; m/z: [M+H]⁺ 1433.2.

### Example 68: Synthesis of I-61

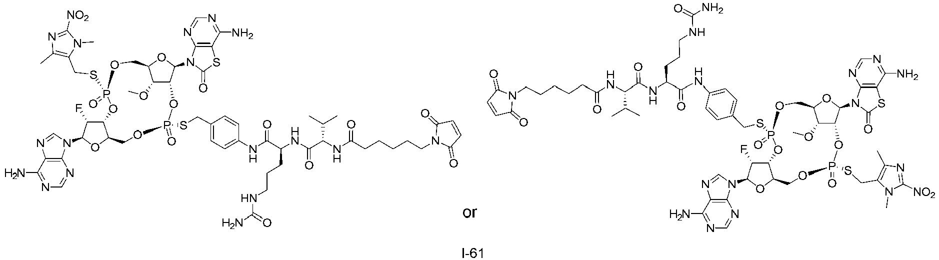

Following the synthetic method of I-43, I-3 was reacted with 5-(chloromethyl)-1,4-dimethyl-2-nitro-1*H*-imidazole to obtain I-61 (*prep-*HPLC*:* mobile phase A: 0.1% trifluoroacetic acid aqueous solution, mobile phase B: acetonitrile; gradient elution with mobile phase B from 5% to 55%, elution time: 20 minutes) as a white solid. UPLC RT = 6.448 minutes; m/z: [1/2M+H]⁺ 724.2.

### Example 69: Synthesis of I-62

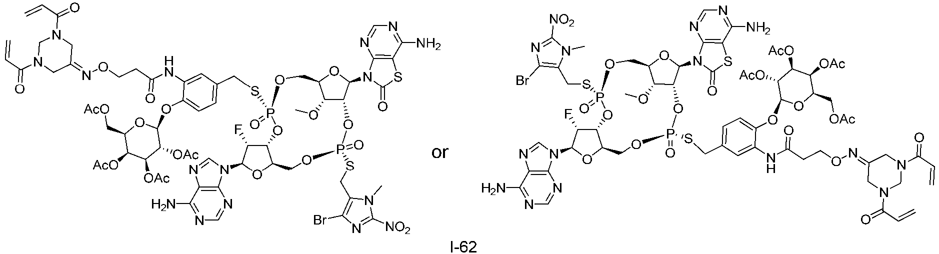

Following the synthetic method of I-43, I-50 was reacted with 4-bromo-5-(chloromethyl)-1-methyl-2-nitro-1*H*-imidazole to obtain I-62 (*prep-*HPLC*:* mobile phase A: 10 mM ammonium bicarbonate aqueous solution, mobile phase B: acetonitrile; gradient elution with mobile phase B from 5% to 65%, elution time: 20 minutes) as a white solid. UPLC RT = 7.154 minutes; m/z: [M+H]⁺ 1686.1.

### Example 70: Synthesis of I-63

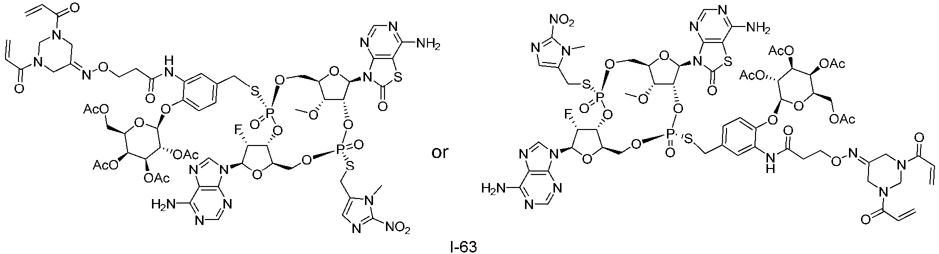

Following the synthetic method of I-43, I-50 was reacted with 5-(chloromethyl)-1-methyl-2-nitro-1*H*-imidazole to obtain I-63 (*prep-*HPLC*:* mobile phase A: 10 mM ammonium bicarbonate aqueous solution, mobile phase B: acetonitrile; gradient elution with mobile phase B from 5% to 65%, elution time: 20 minutes) as a white solid. UPLC RT = 6.919 minutes; m/z: [M+H]⁺ 1607.5.

### Example 71: Synthesis of I-64

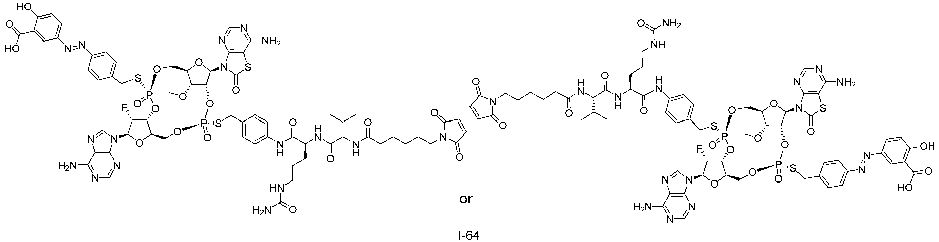

Following the synthetic method of I-3, compound 7 and Linker-2 were reacted to obtain I-64 (*prep-*HPLC*:* mobile phase A: 10 mM ammonium bicarbonate aqueous solution, mobile phase B: acetonitrile; gradient elution with mobile phase B from 5% to 55%, elution time: 20 minutes) as a white solid. UPLC RT = 7.388 minutes; m/z: [M+H]⁺ 1548.6.

### Example 72: Synthesis of I-65

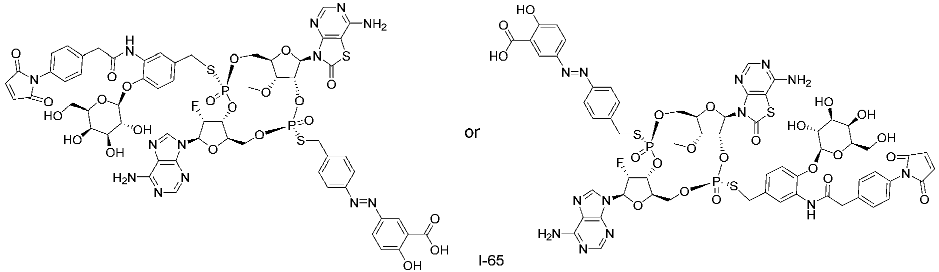

Following the synthetic method of I-3, compound 7 and Linker-39 were reacted to obtain I-65 (*prep-*HPLC*:* mobile phase A: 10 mM ammonium bicarbonate aqueous solution, mobile phase B: acetonitrile; gradient elution with mobile phase B from 5% to 80%, elution time: 20 minutes) as a white solid. UPLC RT = 9.125 minutes; m/z: [M+H]⁺ 1490.5.

### Example 73: Synthesis of I-66

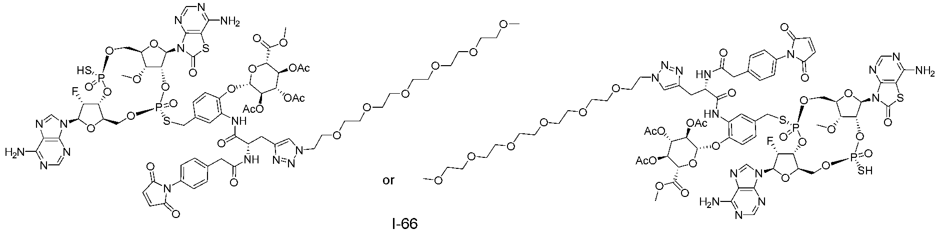

To a solution of Linker-40 (60 mg, 0.05 mmol) and compound 1-3 (40 mg, 0.05 mmol) in DMF (1.5 mL) was added sodium iodide (4 mg, 0.03 mmol). The reaction mixture was stirred at 30°C for 2 days and then directly purified by *prep-*HPLC (mobile phase A: 10 mM ammonium bicarbonate aqueous solution, mobile phase B: acetonitrile; gradient elution with mobile phase B from 10% to 65%, elution time: 20 minutes) to obtain I-66 (14.7 mg, yield: 98%) as a white solid. HPLC RT = 14.541 minutes; m/z: [1/2M+H] ⁺903.8.

### Example 74: Synthesis of I-67

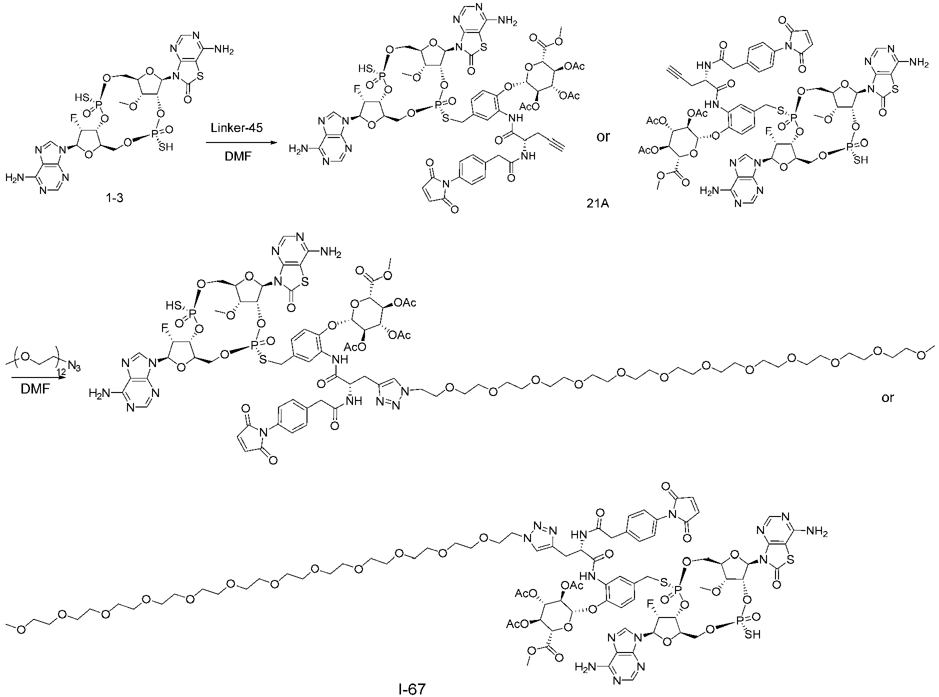

Step 1: To a solution of Linker-45 (102 mg, 0.13 mmol) and compound 1-3 (80 mg, 0.11 mmol) in DMF (1.5 mL) was added sodium iodide (20 mg, 0.13 mmol). The reaction mixture was stirred at 30°C for 1 day and then directly purified by *prep-HP*L*C* (mobile phase A: 10 mM ammonium bicarbonate aqueous solution, mobile phase B: acetonitrile; gradient elution with mobile phase B from 10% to 60%, elution time: 20 minutes) to obtain 21A(65 mg, yield: 34%) as a white solid. m/z: [1/2M+H] ⁺743.8.

Step 2: To a solution of 21A (15 mg, 0.01 mmol) and 37-azido-2,5,8,11,14,17,20,23,26,29,32,35-dodecaoxaheptatriacontane (12 mg, 0.02 mmol) in DMF (1 mL) were added copper(I) iodide (3.8 mg, 0.02 mmol) and DIPEA (4 mg, 0.03 mmol) under a nitrogen atmosphere. The reaction mixture was stirred at 60°C for 2 hours, cooled to room temperature, quenched with water, and the aqueous phase was extracted with ethyl acetate. The combined organic phases were concentrated under reduced pressure. The residue was purified by *prep-*HPLC (mobile phase A: 10 mM ammonium bicarbonate aqueous solution, mobile phase B: acetonitrile; gradient elution with mobile phase B from 10% to 60%, elution time: 20 minutes) to obtain compound I-67 (3.4 mg, yield: 95%) as a white solid. HPLC RT = 17.565 minutes; m/z: [1/2M+H]⁺ 1035.8.

### Example 74: Synthesis of I-68

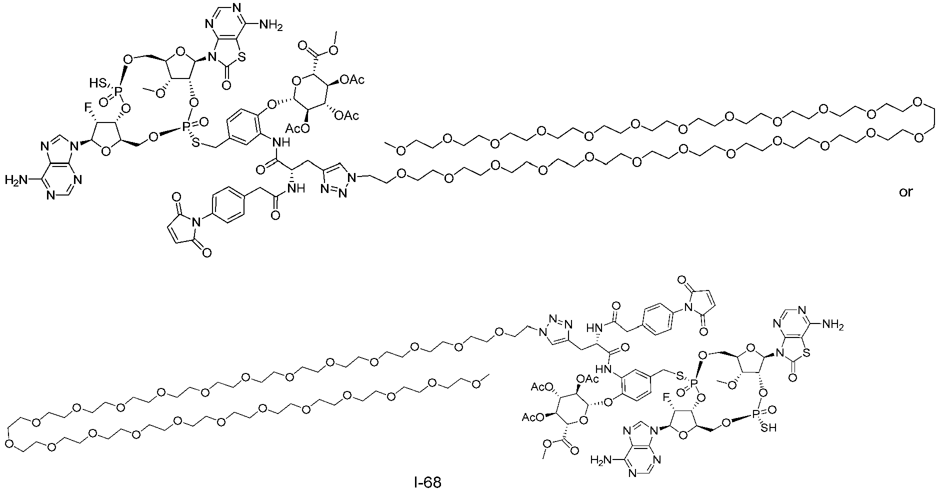

Following the synthetic method of I-67, 21A was reacted with 73-azido-2,5,8,11,14,17,20,23,26,29,32,35,38,41,44,47,50,53,56,59,62,65,68,71-tetracosaoxatriheptacontane to obtain I-68 (mobile phase A: 10 mM ammonium bicarbonate aqueous solution, mobile phase B: acetonitrile; gradient elution with mobile phase B from 10% to 60%, elution time: 20 minutes) as a white solid. HPLC RT = 14.705 minutes; m/z: [1/2M+H]⁺ 1300.8.

### Example 75: Synthesis of I-69

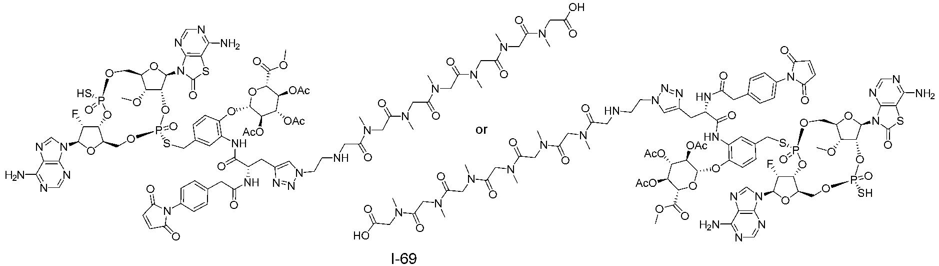

To a solution of compound 21A (15 mg, 0.01 mmol) and Linker-41 (11 mg, 0.02 mmol) in DMF (1 mL) were added copper(I) iodide (4 mg, 0.02 mmol) and *N,N-*diisopropylcarbodiimide (4 mg, 0.03 mmol) under a nitrogen atmosphere. The reaction mixture was stirred at 60°C for 2 hours, cooled to room temperature, and directly purified by *prep-*HPLC (mobile phase A: 0.1% trifluoroacetic acid aqueous solution, mobile phase B: acetonitrile; gradient elution with mobile phase B from 10% to 65%, elution time: 20 minutes) to obtain compound I-69 (4.17 mg, yield: 20%) as a white solid. UPLC RT = 5.785 minutes; m/z: [1/2M+H] ⁺ 1028.6.

### Example 76: Synthesis of I-70

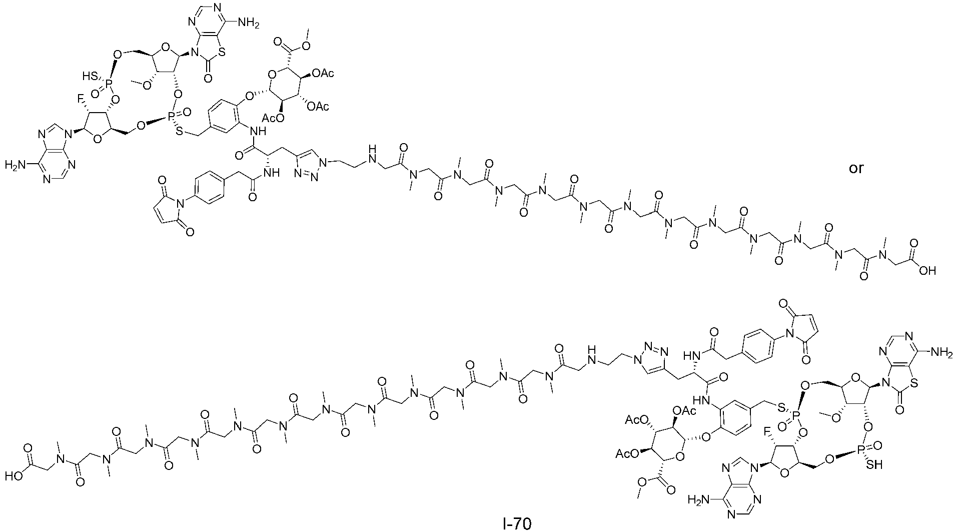

Following the synthetic method of I-69, 21A and Linker-42 were reacted to obtain I-70 (mobile phase A: 0.1% trifluoroacetic acid aqueous solution, mobile phase B: acetonitrile; gradient elution with mobile phase B from 10% to 65%, elution time: 20 minutes) as a white solid. HPLC RT = 13.507 minutes; m/z: [1/2M+H]⁺ 1241.8.

### Example 77: Synthesis of I-71

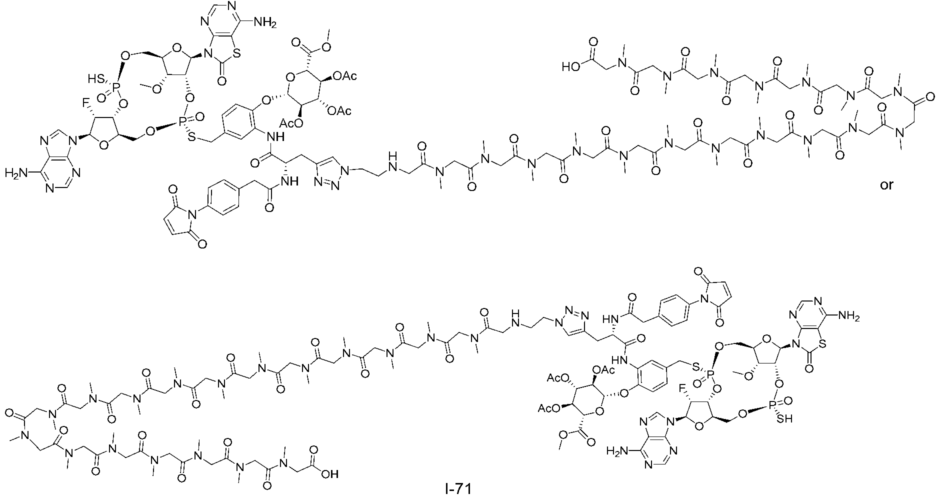

Following the synthetic method of I-69, 21A, and Linker-43 were reacted to obtain compound I-71 (mobile phase A: 0.1% trifluoroacetic acid aqueous solution, mobile phase B: acetonitrile; gradient elution with mobile phase B from 10% to 65%, elution time: 20 minutes) as a white solid. UPLC RT = 5.478 minutes; m/z: [1/2M+H]⁺ 1454.2.

### Example 78: Synthesis of I-72

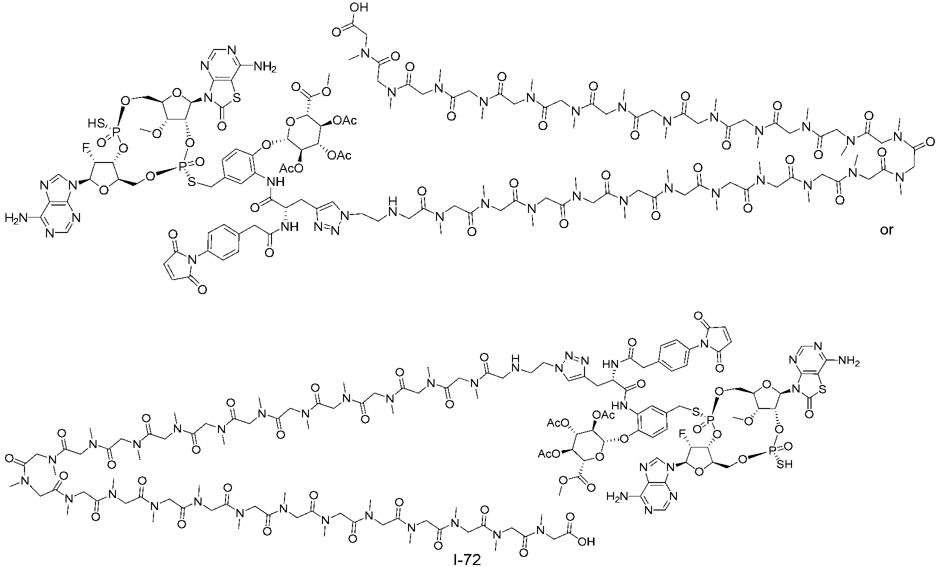

Following the synthetic method of I-69, 21A, and Linker-44 were reacted to obtain compound I-72 (mobile phase A: 0.1% trifluoroacetic acid aqueous solution, mobile phase B: acetonitrile; gradient elution with mobile phase B from 10% to 65%, elution time: 20 minutes) as a white solid. HPLC RT = 13.407 minutes; m/z: [1/2M+H]⁺ 1668.1.

### Example 79: Synthesis of I-73

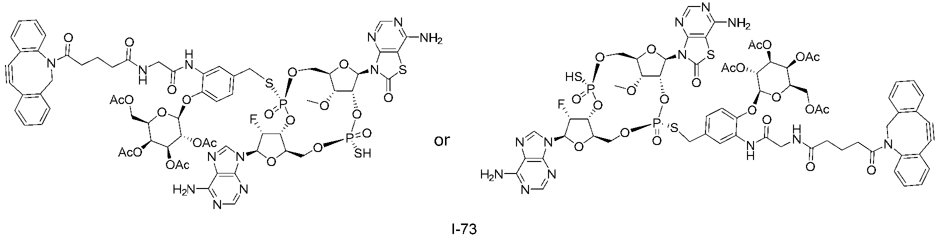

Following the synthetic method of I-7, compound 1-3 and Linker-47 were reacted to obtain compound I-73 (mobile phase A: 10 mM ammonium bicarbonate aqueous solution, mobile phase B: acetonitrile; gradient elution with mobile phase B from 10% to 45%, elution time: 20 minutes) as a white solid. HPLC RT = 15.289 minutes; m/z: [M+H]⁺ 1549.9.

### Example 80: Synthesis of I-74

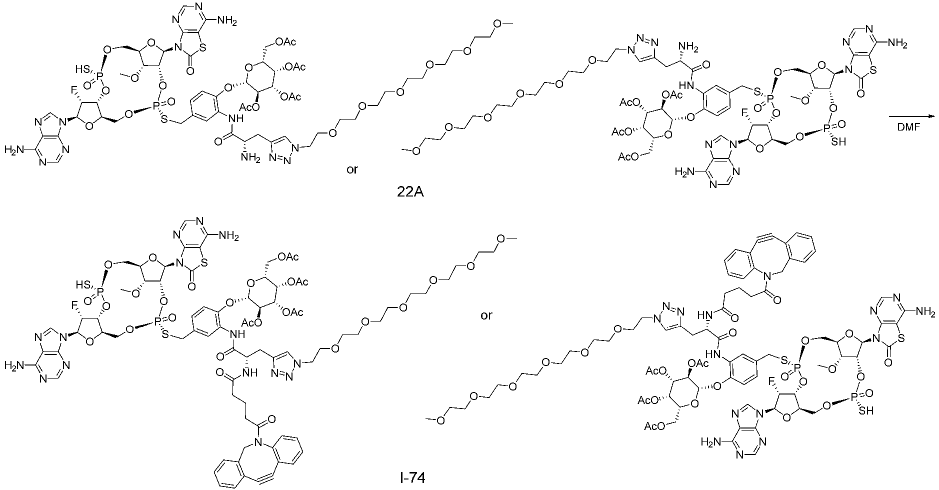

Synthesis of compound 22A: Following the synthetic methods of Steps 1 & 2 of compound I-7, compound 1-3 was reacted with Linker-48 to obtain 22A as a white solid. m/z: [M+H]⁺ 1607.4.

To a solution of compound 22A (15 mg, 9.3 µM) and compound 6-1 (5 mg, 14 µM) in DMF (1 mL) were added PyBOP (8 mg, 14 µM) and DIPEA (3 mg, 19 µM) under ice bath conditions. The reaction mixture was slowly warmed to room temperature and stirred for 2 hours. The reaction mixture was directly purified by *prep-*HPLC (mobile phase A: 10 mM ammonium bicarbonate aqueous solution, mobile phase B: acetonitrile; gradient elution with mobile phase B from 10% to 50%, elution time: 20 minutes) to obtain compound I-74 (4.6 mg, yield: 26%) as a white solid. HPLC RT = 15.276 minutes; m/z: [M+H]⁺ 1908.7.

### Example 81: Synthesis of I-75

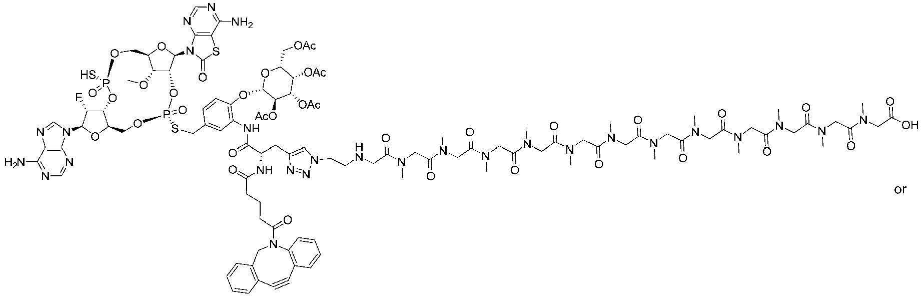

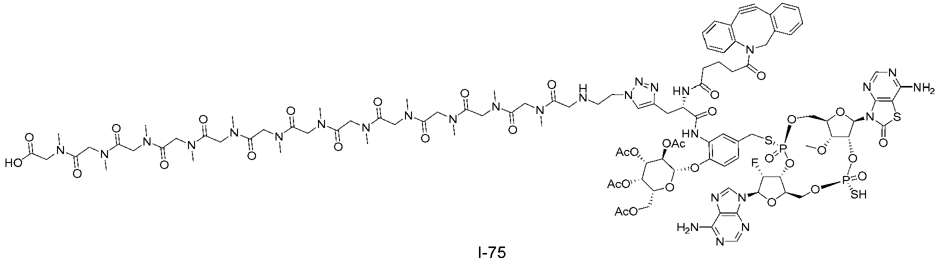

Following the synthetic method of I-74, compound 1-3 and Linker-49 were reacted to obtain compound I-75 (mobile phase A: 10 mM ammonium bicarbonate aqueous solution, mobile phase B: acetonitrile; gradient elution with mobile phase B from 10% to 55%, elution time: 20 minutes) as a white solid. HPLC RT = 14.320 minutes; m/z: [1/2M+H]⁺ 1292.8.

### Example 82: Synthesis of I-76

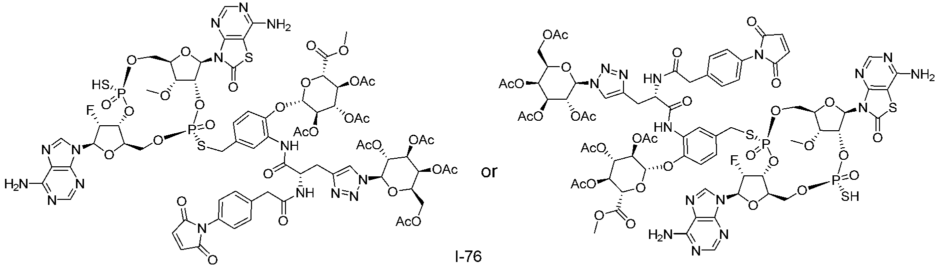

Following the synthetic method of I-67, 21A was reacted with 2,3,4,6-tetra-O-acetyl-β-D-galactopyranosyl azide to obtain compound I-76 (mobile phase A: 1 mM ammonium bicarbonate aqueous solution, mobile phase B: acetonitrile; gradient elution with mobile phase B from 5% to 65%, elution time: 20 minutes) as a white solid. HPLC RT = 15.305 minutes; m/z: [1/2M+H]⁺ 929.8.

### Example 83: Synthesis of I-77

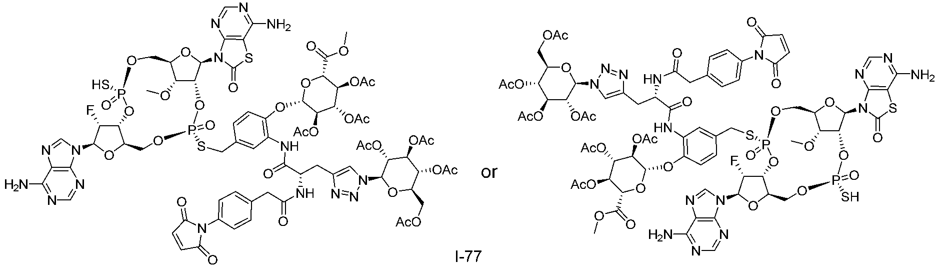

Following the synthetic method of I-67, 21A was reacted with 2,3,4,6-tetra-O-acetyl-β-D-glucopyranosyl azide to obtain compound I-77 (mobile phase A: 1 mM ammonium bicarbonate aqueous solution, mobile phase B: acetonitrile; gradient elution with mobile phase B from 5% to 65%, elution time: 20 minutes) as a white solid. HPLC RT = 15.360 minutes; m/z: [1/2M+H]⁺ 929.8.

### Example 84: Synthesis of I-78

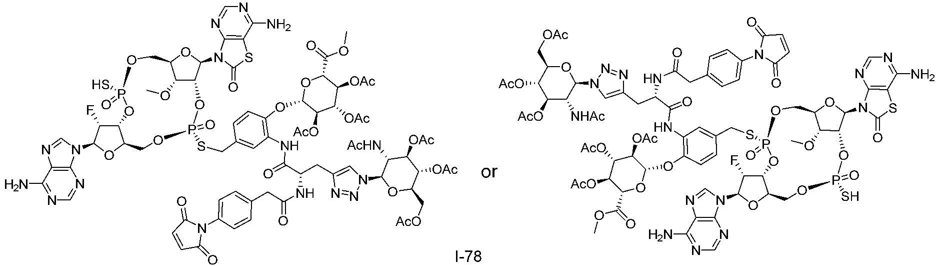

Following the synthetic method of I-67, 21A was reacted with 2-acetamido-3,4,6-triO-acetyl-2-deoxy-β-D-glucopyranosyl azide to obtain compound I-78 (mobile phase A: 1 mM ammonium bicarbonate aqueous solution, mobile phase B: acetonitrile; gradient elution with mobile phase B from 5% to 65%, elution time: 20 minutes) as a white solid. HPLC RT = 15.334 minutes; m/z: [1/2M+H]⁺ 929.3.

### Example 85: Synthesis of I-79

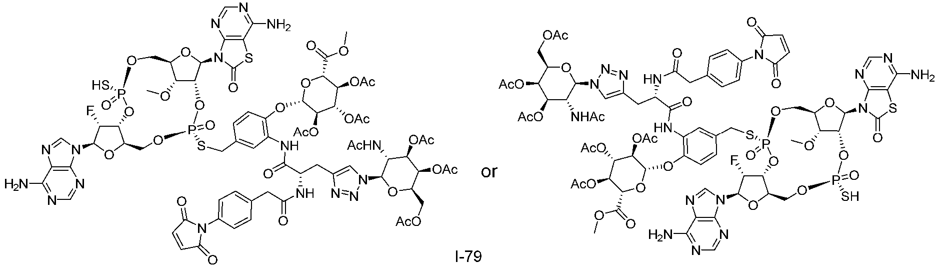

Following the synthetic method of I-67, 21A was reacted with *(2R,3R,4R,5R,6R)-5-*acetamido-2-(acetoxymethyl)-6-azidotetrahydro-2*H*-pyran-3,4-diyl diacetate to obtain compound I-79 (mobile phase A: 1 mM ammonium bicarbonate aqueous solution, mobile phase B: acetonitrile; gradient elution with mobile phase B from 5% to 65%, elution time: 20 minutes) as a white solid. UPLC RT = 7.300 minutes; m/z: [1/2M+H]⁺ 929.4.

### Example 86: Synthesis of I-80

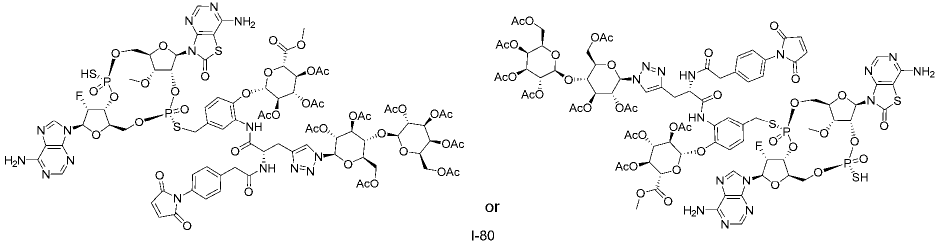

Following the synthetic method of I-67, 21A was reacted with 2,3,6,2',3',4',6'-hepta-O-acetyl-β-lactosyl azide to obtain compound I-80 (mobile phase A: 1 mM ammonium bicarbonate aqueous solution, mobile phase B: acetonitrile; gradient elution with mobile phase B from 5% to 65%, elution time: 20 minutes) as a white solid. UPLC RT = 7.781 minutes; m/z: [1/2M+H]⁺ 1073.7.

### Example 87: Synthesis of I-81

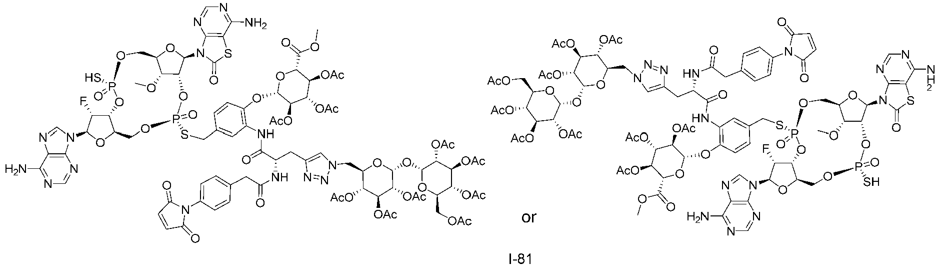

Following the synthetic method of I-67, 21A was reacted with (2*R,*3*R,4*S*,*5*R,*6*R*)-2-(acetoxymethyl)-6-(((2*R*,3*R*,4*S*,5*R*,6*R*)-3,4,5-triacetoxy-6-(azidomethyl)tetrahydro-2*H*-pyran-2-yl)oxy)tetrahydro-2*H*-pyran-3,4,5-triyl triacetate to obtain compound I-80 (mobile phase A: 1 mM ammonium bicarbonate aqueous solution, mobile phase B: acetonitrile; gradient elution with mobile phase B from 5% to 50%, elution time: 20 minutes) as a white solid. UPLC RT = 7.818 minutes; m/z: [1/2M+H]⁺ 1073.8.

### Example 88: Synthesis of I-82

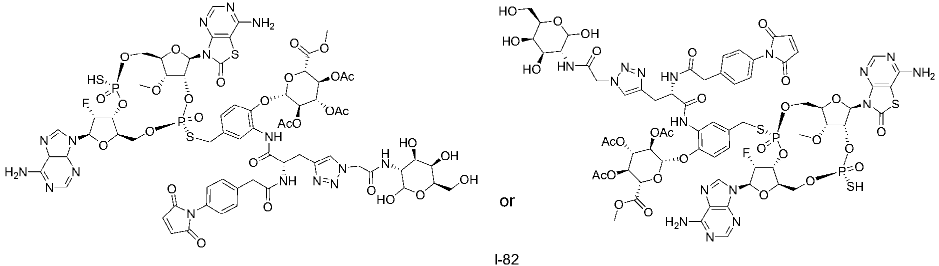

Following the synthetic method of I-67, 21A was reacted with 2-azido-*N-*((3*R,*4*R,*5*R,*6*R*)*-*2,4,5-trihydroxy-6-(hydroxymethyl)tetrahydro-2*H*-pyran-3-yl)acetamide to obtain compound I-82 (mobile phase A: 0.05% trifluoroacetic acid aqueous solution, mobile phase B: acetonitrile; gradient elution with mobile phase B from 5% to 50%, elution time: 20 minutes) as a white solid. HPLC RT = 14.408 minutes; m/z: [1/2M+H]⁺ 874.3.

### Example 89: Synthesis of I-83

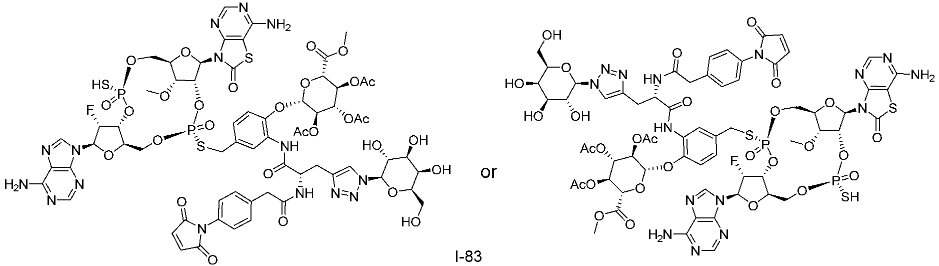

Following the synthetic method of I-67, 21A was reacted with 1-azido-1-deoxy-β-D-galactose to obtain compound I-83 (mobile phase A: 1 mM ammonium bicarbonate aqueous solution, mobile phase B: acetonitrile; gradient elution with mobile phase B from 5% to 65%, elution time: 20 minutes) as a white solid. UPLC RT = 6.499 minutes; m/z: [1/2M+H]⁺ 845.6.

### Example 90: Synthesis of I-84

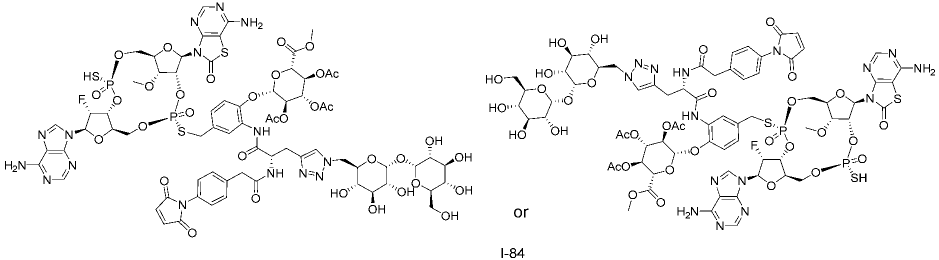

Following the synthetic method of I-67, 21A was reacted with (2*R,*3*S,*4*S,*5*R,*6*R*)-2-(azidomethyl)-6-(((2*R*,3*R*,4*S*,5*S*,6*R*)-3,4,5-trihydroxy-6-(hydroxymethyl)tetrahydro-2H-pyran-2-yl)oxy)tetrahydro-2*H*-pyran-3,4,5-triol to obtain compound I-84 (mobile phase A: 0.05% trifluoroacetic acid aqueous solution, mobile phase B: acetonitrile; gradient elution with mobile phase B from 5% to 50%, elution time: 20 minutes) as a white solid. HPLC RT = 13.832 minutes; m/z: [1/2M+H]⁺ 926.8.

### Example 91: Antibody conjugation reaction

### I. General method A for antibody-drug conjugation reaction (as shown in Formula 1):

Method 1: The antibody was buffer-exchanged using phosphate buffered saline (50 mM PBS, 2 mM EDTA, pH 6.5) via ultrafiltration, and the protein concentration was adjusted to 5-10 mg/mL. TCEP aqueous solution (6-12 equivalents relative to one molecule of antibody) was added, and the reaction was carried out at room temperature for 60-120 minutes. After the reduction reaction was completed, the antibody concentration was diluted to about 5 mg/mL with the above phosphate buffered saline, then a compound represented by Formula I (Linker-payload), 10-20 times the molar amount of the antibody, was dissolved in an organic solvent (DMA or DMSO) accounting for 5%-20% of the total volume, and added to the reaction system, which was stirred at room temperature for 30-120 min. After the conjugation reaction was completed, the mixture was buffer-exchanged into MES buffer (25 mM, pH 6.5) using an ultrafiltration centrifuge tube with a molecular weight cut-off of 30 KDa, and the immune-stimulating antibody conjugate was obtained.

Method 2: The antibody was buffer-exchanged using phosphate buffered saline (50 mM sodium dihydrogen phosphate-disodium hydrogen phosphate + 50 mM sodium chloride + 2 mM EDTA, pH 7.4 ± 0.2) via ultrafiltration, and the protein concentration was adjusted to 10 ± 2 mg/mL. TCEP aqueous solution was added (2.2 equivalents relative to one molecule of antibody). The mixture was stirred at 25 ± 2°C for more than 2 hours. The protein concentration was adjusted to 5.0 ± 0.5 mg/mL with phosphate buffered saline, and under stirring conditions, an appropriate amount of DMA was added dropwise to the reaction system. After mixing evenly, the DMA solution of the compound represented by Formula I (Linker-payload) was added dropwise (5.5 equivalents relative to one molecule of antibody). The reaction was stirred at 25 ± 2°C for more than 1 hour. The mixture was buffer-exchanged using exchange buffer (50 mM sodium dihydrogen phosphate-disodium hydrogen phosphate + 6% trehalose, pH 6.5 ± 0.1) via ultrafiltration to obtain the immune-stimulating antibody conjugate.

Method 3: The antibody was diluted with phosphate buffered saline (25 mM PBS, 1 mM EDTA, pH 7.4) to an antibody concentration of 5 mg/mL, and after shaking thoroughly, a 2 mg/mL aqueous solution of TCEP (8 equivalents relative to one molecule of antibody) was added to perform antibody reduction. After shaking thoroughly, the mixture was placed on a refrigerated thermomixer and reacted at 37°C for 2 hours. The antibody was buffer-exchanged four times using phosphate buffered saline via ultrafiltration, and the recovered antibody was mixed with a dimethyl sulfoxide solution of the compound represented by Formula I (Linker-payload)(4 equivalents relative to one molecule of antibody). Dimethyl sulfoxide was supplemented to 10% of the total reaction volume, and the mixture was shaken thoroughly before being reacted at 20°C for 2 hours on a refrigerated thermomixer. An additional dimethyl sulfoxide solution of Linker-payload (4 equivalents relative to one molecule of antibody) was then added, and the reaction was allowed to proceed overnight at 20°C. The sample was buffer-exchanged into storage buffer using an ultrafiltration tube and ultrafiltered 10 times with 30 mM His-HAc, pH 5.5 to obtain the immune-stimulating antibody conjugate.

Method 4: Antibody 5 or 8 was adjusted to a concentration of 3 mg/mL with phosphate buffered saline (50 mM PBS, 2 mM EDTA, pH 6.5). 20 equivalents of TCEP aqueous solution (TCEP/antibody) was added, mixed thoroughly and then placed in a thermomixer to react at room temperature for 17 hours, centrifuged at 4300 rpm for 10 minutes with an ultrafiltration centrifuge tube (Millipore UFC803096), and the ultrafiltration was repeated three times. The sample collected from the previous step was added in the centrifuge tube with 30 equivalents of DHAA oxidant (DHAA/antibody), mixed thoroughly, and then allowed to react under stirring at room temperature in a thermomixer for 2 hours, ultrafiltration was performed three times. The sample was cooled to about 4°C, and the compound represented by Formula I (Linker-payload) (10 equivalents relative to one molecule of antibody) was added, mixed thoroughly, and then placed in a thermomixer at 4°C to react for 1 hour. Ultrafiltration was performed three times to obtain the immune-stimulating antibody conjugate.

### II. General method B for antibody-drug conjugation reaction (as shown in Formula 1'):

The immune-stimulating antibody conjugate obtained by General Method A was buffer-exchanged into phosphate buffered saline (50 mM PBS, 2 mM EDTA, pH 8.0) using an ultrafiltration tube (MWCO 30 KD, 4 mL, manufacturer: Millipore), and the reaction was carried out overnight at 37°C. The mixture was buffer-exchanged using 30 mM His-HAc without dimethyl sulfoxide, pH 5.5 via ultrafiltration 10 times to obtain the immune-stimulating antibody conjugate.

### III. General method C for antibody-drug conjugation reaction (as shown in Formula 5 or 12):

The antibody was diluted with phosphate buffered saline (25 mM PBS, 1 mM EDTA, pH 7.4) to an antibody concentration of 5 mg/mL, and after shaking thoroughly, a 2 mg/mL aqueous solution of TCEP (8 equivalents relative to one molecule of antibody) was added to perform antibody reduction. After shaking thoroughly, the mixture was placed on a refrigerated thermomixer and reacted at 37°C for 2 hours. The mixture was buffer-exchanged using 50 mM BBS, pH 8.0 buffer via ultrafiltration 4 times, the antibody after ultrafiltration was recovered, and a dimethyl sulfoxide solution of the compound represented by Formula I (Linker-payload) (6-8 equivalents relative to one molecule of antibody) was added. The dimethyl sulfoxide solution was supplemented to 10% of the total reaction volume, shaken thoroughly, and placed on a refrigerated thermomixer to react at 20°C for 1 hour. The sample was buffer-exchanged into storage buffer using an ultrafiltration tube and ultrafiltered 10 times with 30 mM His-HAc, pH 5.5 to obtain the immune-stimulating antibody conjugate.

### IV. General method D for antibody-drug conjugation reaction (as shown in Formula 13):

The phosphate buffered saline of antibody 12 (25 mM PBS, 1 mM EDTA, pH 7.4) and the dimethyl sulfoxide solution (10 mM, 6-8 equivalents relative to one molecule of antibody) of the compound represented by Formula I (Linker-payload) were reacted overnight at 37°C. The sample was buffer-exchanged into storage buffer using an ultrafiltration tube and ultrafiltered 5 times with 30 mM His-HAc, pH 5.5 to obtain the immune-stimulating antibody conjugate.

In the above general method, the antibody molecules used were respectively anti-HER2 antibody 1: Trastuzumab, Roche; anti-HER2 antibody 2: Pertuzumab, Roche; anti-HER2 antibody 3: Trastuzumab-LALA (L234A/L235A), B801901, Shanghai Biointron Biological Inc; anti-HER2 antibody 4: Trastuzumab-AAG (L234A/L235A/P329G), MHDDD001, Shanghai Biointron Biological Inc; anti-HER2 antibody 5: Trastuzumab (HC-s239.5) (an engineered Trastuzumab with cysteine inserted between heavy chain positions 239 and 240, prepared by the method disclosed in Molecular Pharmaceutics. 2017, 14, 1501-1516), LPDDFD001, Shanghai Biointron Biological Inc; antibody 6: anti-HEL human IgG1-Kappa Isotype control, B117901, Shanghai Biointron Biological Inc; anti-EGFR antibody 7: Cetuximab, Merck; anti-5T4 antibody 8: huA1 (V*_{H}*v2.0 + V*_{L}*v2.4) (expressed and purified according to the sequence information table disclosed in US8044178B2 (SEQ ID NO: 54 and SEQ ID NO: 70)); anti-HER2 antibody 9: Trastuzumab (HC-s239.5, L234A/L235A/P329G), CZ7DUD001, Shanghai Biointron Biological Inc; antibody 10: anti-HEL human IgG1 (L234A/L235A/P329G)-Kappa Isotype control, B422203, Shanghai Biointron Biological Inc; antibody 11: Enfortumab (Ha22-2) (expressed and purified according to the sequence information table disclosed in US10894090B2); anti-EGFR antibody 12: prepared from Cetuximab by the method disclosed in Example 25 of CN115209921A; anti-EGFR antibody 13: Nimotuzumab, Biotech Pharmaceutical Co., Ltd.

### V: Determination of the drug-to-antibody ratio (DAR) per antibody molecule in antibody-drug conjugates

For the drug-to-antibody ratio per antibody molecule in antibody-drug conjugates, the DAR could be determined by high-performance liquid chromatography (HPLC) analysis using the following method.

HPLC instrument: Waters/Waters e2695; mobile phase A: 1.5 M (NH₄)₂SO₄ + 50 mM potassium phosphate (pH 7.0); mobile phase B: 50 mM sodium phosphate (pH 7.0)/isopropanol (75:25 V/V); analytical column: Thermo MabPac^{™} HIC-Butyl 5 µm 4.6 × 100 mm, PN. 088558; injection volume: 5 µL; flow rate: 1 mL/min; column temperature: 30°C; detector: PDA detector; detection wavelength: 280 nm. Elution gradient 1: mobile phase B from 20% to 80%, elution time 20 minutes; mobile phase B from 80% to 100%, elution time 2 minutes; mobile phase B held at 100% for 6 minutes; elution gradient 1: mobile phase B from 10% to 65%, elution time 20 minutes; mobile phase B from 65% to 100%, elution time 2 minutes; mobile phase B held at 100% for 6 minutes.

Hydrophobic interaction chromatography could be used to determine the drug-to-antibody ratio (DAR) in antibody-drug conjugates. Unconjugated antibody drugs had the weakest hydrophobicity and were eluted first; antibodies conjugated with 8 drugs had the strongest hydrophobicity and were eluted last. The peak area percentage represented the relative distribution of ADCs with a specific number of conjugated drugs, and the weighted average DAR was calculated as DAR = SUM (peak area of each component × corresponding DAR value) / total peak area based on the peak area percentage and the number of conjugated drugs.

### VI: Analysis of molecular size heterogeneity (SEC) of immune-stimulating antibody conjugates

Method A: chromatography column: Waters XBridge BEH200Å, 7.8 × 300 mm, 3.5 µm; mobile phase A: 100 mM PB + 200 mM arginine hydrochloride, pH 6.8; mobile phase B: isopropanol; injection volume: 10 µL; column temperature: 25°C; flow rate: 0.8 mL/min; isocratic elution. Method B: HPLC instrument: Waters Acquity Arc; mobile phase: 100 mM PB + 200 mM Arg·HCl + 5% IPA, pH 6.8; analytical column: TOSOH TSKgel G3000 SWxl, 7.8 × 300 mm, 5 µM, 450 Å, PN0008541; injection volume: 10 µL; flow rate: 0.5 mL/min; column temperature: 30°C; detector: PDA detector; detection wavelength: 280 nm; gradient: isocratic elution.

The immune-stimulating antibody conjugates were prepared using General Method A, as shown in Table 3 below:

**Table 3**

| No. | Linker-payload No. | Antibody | DAR (HIC) | SEC% |
|---|---|---|---|---|
| II-1 | I-2 | Antibody 1 | 7.91 | 98.59% |
| II-2 | I-3 | Antibody 1 | 7.93 | 96.62% |
| II-3 | I-2 | Antibody 2 | 7.94 | 98.51% |
| II-4 | I-3 | Antibody 2 | 7.96 | 96.42% |
| II-5 | I-3 | Antibody 1 | 3.10 | 99.05% |
| II-6 | I-3 | Antibody 3 | 6.62 | 100% |
| II-8 | I-5 | Antibody 1 | 4.21 | 79.35% |
| II-9 | I-6 | Antibody 1 | 7.81 | 96.89% |
| II-10 | I-7 | Antibody 1 | 3.58 | 99.38% |
| II-11 | I-8 | Antibody 1 | 4.22 | 99.54% |
| II-12 | I-8 | Antibody 3 | 3.80 | 96.38% |
| II-13 | I-9 | Antibody 1 | 4.20 | 99.07% |
| II-14 | I-9 | Antibody 3 | 3.64 | 98.53% |
| II-15 | I-10 | Antibody 1 | 3.93 | 99.61% |
| II-16 | I-11 | Antibody 1 | 3.72 | 99.68% |
| II-17 | I-12 | Antibody 1 | 3.74 | 99.57% |
| II-18 | I-13 | Antibody 1 | 3.97 | 99.28% |
| II-19 | I-14 | Antibody 1 | 3.17 | 98.00% |
| II-20 | I-15 | Antibody 1 | 3.90 | 98.44% |
| II-21 | I-16 | Antibody 1 | 3.78 | 99.63% |
| II-22 | I-21 | Antibody 1 | 3.70 | 98.72% |
| II-23 | I-22 | Antibody 1 | 3.81 | 98.93% |
| II-24 | I-23 | Antibody 1 | 3.62 | 98.55% |
| II-25 | I-24 | Antibody 1 | 3.66 | 98.50% |
| II-26 | I-24 | Antibody 3 | 3.58 | 95.00% |
| II-27 | I-25 | Antibody 1 | 4.00 | 99.00% |
| II-28 | I-26 | Antibody 1 | 3.77 | 98.85% |
| II-29 | I-27 | Antibody 1 | 3.81 | 98.40% |
| II-30 | I-28 | Antibody 1 | 3.73 | 100% |
| II-31 | I-30 | Antibody 1 | 4.26 | 98.86% |
| II-32 | I-31 | Antibody 1 | 4.18 | 98.85% |
| II-33 | I-33 | Antibody 1 | 4.41 | 98.96% |
| II-34 | I-34 | Antibody 1 | 4.06 | 99.40% |
| II-35 | I-34 | Antibody 3 | 3.79 | 96.00% |
| II-36 | I-35 | Antibody 1 | 3.72 | 99.52% |
| II-37 | I-36 | Antibody 1 | 3.70 | 80.43% |
| II-38 | I-38 | Antibody 1 | 4.50 | 98.89% |
| II-39 | I-29 | Antibody 1 | 4.03 | 98.62% |
| II-40 | I-29 | Antibody 1 | 7.71 | 98.32% |
| II-41 | I-29 | Antibody 3 | 6.57 | 95.56% |
| II-42 | I-3 | Antibody 1 | 3.71 | 98.49% |
| II-43 | I-3 | Antibody 1 | 7.31 | 100% |
| II-44 | I-3 | Antibody 4 | 7.96 | 94.64% |
| II-45 | I-38 | Antibody 1 | 7.62 | 96.51% |
| II-46 | I-38 | Antibody 5 | 2.00 | 97.63% |
| II-47 | I-38 | Antibody 7 | 4.07 | 98.52% |
| II-48 | I-38 | Antibody 8 | 4.06 | 96.88% |
| II-49 | I-39 | Antibody 1 | 3.58 | 100% |
| II-50 | I-40 | Antibody 1 | 3.82 | 100% |
| II-51 | I-54 | Antibody 1 | 7.83 | 98.88% |
| II-52 | I-52 | Antibody 1 | 4.12 | 98.15% |
| II-53 | I-55 | Antibody 1 | 7.95 | 96.85% |
| II-67 | I-66 | Antibody 1 | 3.88 | 97.86% |
| II-68 | I-76 | Antibody 1 | 3.17 | 97.63% |
| II-69 | I-77 | Antibody 1 | 3.55 | 97.63% |
| II-70 | I-78 | Antibody 1 | 3.56 | 98.34% |
| II-71 | I-67 | Antibody 1 | 3.46 | 97.84% |
| II-72 | I-68 | Antibody 1 | 3.77 | 98.32% |
| II-73 | I-69 | Antibody 1 | 4.08 | 98.74% |
| II-74 | I-79 | Antibody 1 | 3.67 | 98.14% |
| II-75 | I-80 | Antibody 1 | 3.94 | 94.27% |
| II-76 | I-81 | Antibody 1 | 4.03 | 95.37% |
| II-77 | I-82 | Antibody 1 | 3.80 | 88.49% |
| II-78 | I-70 | Antibody 1 | 3.66 | 99.01% |
| II-79 | I-71 | Antibody 1 | 3.83 | 99.30% |
| II-80 | I-83 | Antibody 1 | 3.83 | 98.20% |
| II-81 | I-72 | Antibody 1 | 3.77 | 99.29% |
| II-82 | I-84 | Antibody 1 | 4.02 | 98.60% |
| II-83 | I-38 | Antibody 11 | 3.67 | 97.50% |
| II-85 | I-38 | Antibody 13 | 4.34 | 98.00% |
| II-86 | I-38 | Antibody 7 | 2.30 | 99.23% |

The immune-stimulating antibody conjugates were prepared using General Method B, as shown in Table 4 below:

**Table 4**

| No. | Linker-payload No. | Antibody | DAR (HIC) | SEC% |
|---|---|---|---|---|
| II-54 | I-38 | Antibody 1 | 7.82 | 98.27% |
| II-55 | I-38 | Antibody 5 | 2.00 | 95.25% |
| II-56 | 1-38 | Antibody 9 | 2.32 | 99.48% |

The immune-stimulating antibody conjugates were prepared using General Method C, as shown in Table 5 below:

**Table 5**

| No. | Linker-payload No. | Antibody | DAR (HIC) | SEC% |
|---|---|---|---|---|
| II-7 | I-57 | Antibody 1 | 3.87 | 98.96% |
| II-57 | I-41 | Antibody 1 | 3.82 | 90.90% |
| II-58 | I-46 | Antibody 1 | 3.95 | 98.47% |
| II-59 | I-47 | Antibody 1 | 4.11 | 96.76% |
| II-60 | I-48 | Antibody 1 | 3.66 | 97.17% |
| II-61 | I-50 | Antibody 1 | 3.80 | 98.24% |
| II-62 | I-50 | Antibody 10 | 4.08 | 95.58% |
| II-63 | I-50 | Antibody 7 | 4.23 | 95.66% |
| II-64 | I-49 | Antibody 1 | 3.94 | 97.96% |
| II-65 | I-51 | Antibody 1 | 3.80 | 98.61% |
| II-66 | 1-37 | Antibody 1 | 3.83 | 91.66% |

The immune-stimulating antibody conjugates were prepared using General Method D, as shown in Table 6 below:

**Table 6**

| No. | Linker-payload No. | Antibody | DAR (HIC) | SEC% |
|---|---|---|---|---|
| II-84 | I-73 | Antibody 12 | 1.51 | 97.36% |

### Example 92: Synthesis of C-7

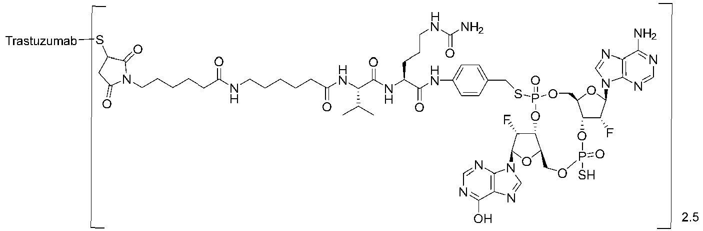

C-7 was prepared using antibody 1 and compound 17 (WO2019129880A1) according to preparation method 2 in General Method A, DAR: 2.5, SEC: 99.23%.

### Biological examples:

### Experimental Example 1: Activation level assay of type I interferon

THP-1 dual cells (purchased from Invivogen) were seeded into a 96-well plate at 100,000 cells/well, and phorbol 12-myristate 13-acetate (PMA) was added to a final concentration of 30 ng/mL to induce for 24 hours. After 24 hours, the supernatant was discarded, and the cells were rinsed twice with fresh culture medium. Subsequently, a 3-fold gradient dilution of the test compound prepared with PB buffer (50 mM HEPES, 100 mM KCl, 3 mM MgCl₂, 0.1 mM DTT, 85 mM sucrose, 1 mM ATP, 0.1 mM GTP, 0.2% bovine serum albumin, and 5 µg/mL digitonin) was added into the cells, in which the highest final concentration of the compound was 10 µM and the lowest final concentration was 0.0015 µM. The cells were incubated back in the incubator for 30 minutes, then the supernatant was discarded, the cells were rinsed twice with fresh culture medium again. Fresh culture medium was added, and the cells were placed back into the incubator for an additional 24 hours. After 24 hours, 10 µL of supernatant per well was taken into a new 96-well plate, 50 µL/well of QUANTI-Luc reagent (purchased from Invivogen) was added, and the Luciferase reading was immediately read using a TECAN M1000. The activation level of type I interferon was positively correlated with the detected luciferase intensity. A dose-response curve was plotted using GraphPad Prism software, and the EC₅₀ of the test compound was analyzed.

### Experimental Example 2: Cytokine detection in hPBMC system

Frozen human peripheral blood mononuclear cells (Allcells) were rapidly thawed in a 37°C water bath and added with 9 mL of RPMI 1640 medium containing 10% fetal bovine serum, 100 U/mL penicillin, 100 µg/mL streptomycin, and 1 mM sodium pyruvate (all from Gibco). The mixture was centrifuged at 400 × g for 5 minutes at room temperature, followed by discarding the supernatant. The cells were resuspended in the culture medium, and the cell density was adjusted to 4 × 10⁶/mL. The cell suspension was added to a 96-well flat-bottom plate (Corning) at 50 µL/well. 50 µL of culture medium was added to the culture system.

The test sample dilution at 3× working concentration was prepared with culture medium and was added to the cell suspension, 50 µL per well. The total system volume was 150 µL, with an initial concentration of the test sample of 0.67 µM and 3-fold serial dilutions. 50 µL of the culture medium was supplemented to the blank control, and the mixture was incubated in a 5% CO₂ incubator at 37°C for 20 hours. The mixture was centrifuged at 500 × g for 5 minutes at room temperature, and the supernatant was collected. The concentrations of TNF-α were detected and analyzed by HTRF (Cisbio) and Infinite M1000 PRO (TECAN).

The test results showed that II-1, II-7, II-10 to II-18, II-22, II-27, II-28, II-30, II-32, II-34, II-35, II-37, II-39, II-40, II-41, II-45, II-46, II-50, II-51, II-52, II-54, II-55, II-58 to II-61, and II-67 to II-82 had TNFα secretion values less than 500 pg/mL at a concentration of 500 nM.

### Experimental Example 3: Cytokine detection in hPBMC and BT474 co-culture system

Frozen human peripheral blood mononuclear cells (Allcells) were rapidly thawed in a 37°C water bath and added with 9 mL of RPMI 1640 medium containing 10% fetal bovine serum, 100 U/mL penicillin, 100 µg/mL streptomycin, and 1 mM sodium pyruvate (all from Gibco). The mixture was centrifuged at 400 × g for 5 minutes at room temperature, followed by discarding the supernatant. The cells were resuspended in the culture medium, and the cell density was adjusted to 4 × 10⁶/mL. The cell suspension was added to a 96-well flat-bottom plate (Corning) at 50 µL/well. Human breast cancer epithelial cells BT474 (Nanjing Cobioer Biosciences Co., Ltd.) in normal growth state were collected, resuspended in RPMI 1640 complete medium, and adjusted to a density of 4 × 10⁵/mL. 50 µL of the mixture was added to each well of a 96-well plate and mixed with hPBMC.

The test sample dilution at 3× working concentration was prepared with culture medium and was added to the cell suspension, 50 µL per well. The total system volume was 150 µL, with initial concentrations of 0.8 µM for compound 1-3 (5-fold serial dilutions) and 0.1 µM for other test samples (5-fold serial dilutions). 50 µL of the culture medium was supplemented to the blank control, and the mixture was incubated in a 5% CO₂ incubator at 37°C for 20 hours. After centrifugation at 500 rpm for 5 minutes at room temperature, the supernatant was collected. The concentrations of TNF-α were detected and analyzed by HTRF (Cisbio) and Infinite M1000 PRO (TECAN).

The test results showed that under the same concentration, II-5 had a significant inducing effect on TNF-α in the BT474 co-culture system, while in the individual hPBMC system, the inducing effect of II-5 on TNF-α was significantly weakened. The inducing effects of II-2 and II-5 on TNF-α in the BT474 co-culture system were significantly superior to those of compound 1-3, Trastuzumab, and the combination of Trastuzumab and 1-3.

The TNFα secretion values of II-1, II-3, II-4, II-5, II-7, II-8, II-19, II-20, II-23 to II-31, II-33, II-34, II-36, II-39 to II-43, II-49, II-53, II-57 to II-60, II-64, II-66, II-67, II-73, II-78, II-81, and II-82 at a concentration of 100 nM were between 1500 to 9000 pg/mL.

### Experimental Example 4: Cytokine detection in hPBMC and MDA-MB-468 co-culture system

Frozen human peripheral blood mononuclear cells (Cylin Bio) were rapidly thawed in a 37°C water bath and added with 9 mL of RPMI 1640 medium (all from Gibco) containing 10% fetal bovine serum, 1× penicillin-streptomycin-glutamine (Pen Strep Glutamine), 1 mM sodium pyruvate, 1× MEM non-essential amino acids (MEM NEAA), and 10 mM HEPES. The mixture was centrifuged at 400 × g for 5 minutes at room temperature, followed by discarding the supernatant. The cells were resuspended in the culture medium, and the cell density was adjusted to 4 × 10⁶/mL. The cell suspension was added to a 96-well flat-bottom plate (Corning) at 50 µL/well. Human breast cancer cells MDA-MB-468 (Nanjing Cobioer Biosciences Co., Ltd.) in normal growth state were collected, resuspended in RPMI 1640 complete medium, and adjusted to a density of 4 × 10⁵/mL. 50 µL of the mixture was added to each well of a 96-well plate and mixed with hPBMC.

The test sample dilution at 3× working concentration was prepared with culture medium and was added to the cell suspension, 50 µL per well. The total system volume was 150 µL, with an initial compound concentration of 500 nM and 5-fold serial dilutions. 50 µL of the culture medium was supplemented to the blank control, and the mixture was incubated in a 5% CO₂ incubator at 37°C for 20 hours. After centrifugation at 500 rpm for 5 minutes at room temperature, the supernatant was collected. The concentrations of TNF-α were detected and analyzed by HTRF (VKEY-BIO) and Infinite M1000 PRO (TECAN).

### Experimental Example 5: Cell proliferation assay

On day 1, SK-BR-3 human breast cancer cells (Nanjing Cobioer Biosciences Co. Ltd.) in the logarithmic growth phase were taken, digested with an appropriate amount of trypsin, formulated into a single cell suspension using RPMI 1640 (Gibco) medium containing 10% fetal bovine serum and 1% penicillin-streptomycin, counted to adjust the cell density, inoculated in a 96-well plate at 100 µL/well so that there were 2500 cells per well, and then the cell culture plate was incubated in a 37°C, 5% CO₂ incubator overnight. On day 2, ADCs were serially diluted 5-fold in the aforementioned RPMI 1640 medium and added to the cell culture plate at 50 µL/well, with the highest ADC concentration at 500 nM and the lowest at 0.00128 nM; the control group was the culture medium. The cell culture plate was briefly centrifuged and then incubated in a CO₂ incubator for 120 hours. On day 5, the cell culture plate was taken out, added with 50 µL of Cell Titer-Glo (Promega) detection reagent in each well, incubated in the dark for 10 minutes, and then luminescence was detected using a microplate reader (Tecan, Infinite M1000 Pro). Data were analyzed using Graphpad software, and curve fitting was performed with three-parameter equation and the IC₅₀ value of the inhibitor was calculated.

### Experimental Example 6: Plasma stability assay

The test samples were respectively added to human and mouse plasma, with a test sample concentration of 0.05 mg/mL, and 50 µL of test sample solution was taken on days 0, 2, 4, 7, and 9 into a new plate, and Coating Buffer was prepared with 1 µg/mL human Her2/ErbB2 protein (acro, Cat#HE2-H5225) and 5 µg/mL anti-small molecule antibody (4222-03), 30 µL was added to each well of a high-bind 384-well plate (High Bind Microplate, SpectraPlate, Cat#: 6007500), and was incubated overnight at 4°C. After washing three times with washing buffer (90 µL per well), 60 µL of blocking buffer (containing 5% BSA in PBS buffer) was added to each well, and was incubated at 37°Cfor 1 hour, then 30 µL of sample or standard was added and incubated at room temperature for 2 hours, washed three times with washing buffer (PBS buffer containing 0.05% Tween-20) (90 µL per well), 30 µL of detection antibody (Anti-Human IgG (Fab specific)-peroxidase: Sigma, Cat# A0293) was added, incubated at room temperature for 1 hour, washed three times with washing buffer (90 µL per well), 30 µL of TMB solution (A+B) (Solarbio, Cat#: PR1210-2 × 50 mL) was added and incubated at room temperature in the dark for 5 minutes. 30 µL of stop solution (2 M dilute sulfuric acid) was added, and the OD value was read at 450 nm wavelength. The results showed that the immune-stimulating antibody conjugates represented by Formula II had good plasma stability, especially II-4, II-38, II-42, II-43, II-46, II-49, II-57, II-58, II-61, and II-66, all of which had active half-lives greater than 4 days in human and rat plasma.

### Experimental Example 7: PK concentration detection

A 384-well plate was coated with human Her2/ErbB2 protein (acro, Cat#HE2-H5225) or 5 µg/mL of anti-small molecule antibody (4222-03) and stored overnight at 4°C in a refrigerator. The next day, the plate was washed three times with washing buffer (PBS buffer containing 0.05% Tween-20), then blocked with blocking buffer (PBS buffer containing 5% BSA) and incubated at 37°C for 1-2 hours. After another three washes with washing buffer, diluted standards and test samples were added to the wells and incubated at room temperature for 2 hours. The plate was washed with washing buffer three times again, followed by the addition of detection antibody (Anti-Human IgG (Fab specific)-peroxidase: Sigma, Cat# A0293) diluted in PBS buffer, and incubated at room temperature for 1 hour. Finally, the chromogenic solution was added, incubated at room temperature for 10 minutes, and the reaction was stopped with the stop solution (2 M dilute sulfuric acid). The OD value was measured at 450 nm wavelength.

### Experimental Example 8: In vivo efficacy assay of the mouse colon cancer CT26-hHER2 subcutaneous xenograft model

Cell culture: Mouse colon cancer CT26-hHER2 cells were maintained as monolayers in RPMI-1640 medium containing 10% fetal bovine serum at 37°C in a constant temperature incubator with 5% CO₂. Cells were subcultured twice weekly after treatment with trypsin-EDTA. Cells in the exponential growth phase were harvested and counted for inoculation.

Experimental animals: BALB/c mice, 6-8 weeks, 16-18 g, purchased from Beijing Vital River Laboratory Animal Technology Co., Ltd.

6 experimental groups were set for 1-3, II-1, II-2, II-3, and II-4, as shown in Table 7 below:

**Table 7**

| Group | Number of mice | Test compound | Dose | Dose (Based on 1-3 content) | Administration route | Administration schedule |
|---|---|---|---|---|---|---|
| 1 | 6 | Vehicle | -- | -- | i.v. | QW×2 |
| 2 | 6 | 1-3 | 0.3 mg/kg | 0.3 mg/kg | i.t. | QW×2 |
| 3 | 6 | II-1 | 1 mg/kg | 0.04 mg/kg | i.v. | QW×2 |
| 4 | 6 | II-2 | 0.25 mg/kg | 0.01 mg/kg | i.v. | QW×2 |
| 5 | 6 | II-3 | 0.25 mg/kg | 0.01 mg/kg | i.v. | QW×2 |
| 6 | 6 | II-4 | 0.25 mg/kg | 0.01 mg/kg | i.v. | QW×2 |

2 experimental groups were set for Trastuzumab, as shown in Table 8 below:

**Table 8**

| Group | Number of mice | Test compound | Dose | Administration route | Administration schedule |
|---|---|---|---|---|---|
| 7 | 8 | Vehicle | -- | i.t. | QW×1 |
| 8 | 8 | Trastuzumab | 25 mg/kg | i.p. | QW×1 |

6 experimental groups were set for Trastuzumab, II-22, II-25, II-30, and II-37, as shown in Table 9 below:

**Table 9**

| Group | Number of mice | Test compound | Dose | Administration route | Administration schedule |
|---|---|---|---|---|---|
| 9 | 5 | Vehicle | -- | i.p. | QW×2 |
| 10 | 3 | Trastuzumab | 1 mg/kg | i.p. | QW×2 |
| 11 | 3 | II-22 | 1 mg/kg | i.p. | QW×2 |
| 12 | 3 | II-25 | 1 mg/kg | i.p. | QW×2 |
| 13 | 3 | II-30 | 1 mg/kg | i.p. | QW×2 |
| 14 | 3 | II-37 | 1 mg/kg | i.p. | QW×2 |

12 experimental groups were set for Trastuzumab + I-3, II-4, II-6, II-38, II-43, II-42, II-44, II-49, II-53, II-57, and II-58, as shown in Table 10 below:

**Table 10**

| Group | Number of mice | Test compound | Dose | Administration route | Administration schedule |
|---|---|---|---|---|---|
| 15 | 5 | Vehicle | -- | i.p. | QW×2 |
| 16 | 5 | Trastuzumab + I-3 | 1 mg/kg + 0.06 mg/kg | i.p. | QW×2 |
| 17 | 5 | II-43 | 1 mg/kg | i.p. | QW×2 |
| 18 | 5 | II-6 | 1 mg/kg | i.p. | QW×2 |
| 19 | 5 | II-44 | 1 mg/kg | i.p. | QW×2 |
| 20 | 5 | II-42 | 1 mg/kg | i.p. | QW×2 |
| 21 | 5 | II-57 | 1 mg/kg | i.p. | QW×2 |
| 22 | 5 | II-58 | 1 mg/kg | i.p. | QW×2 |
| 23 | 5 | II-53 | 1 mg/kg | i.p. | QW×2 |
| 24 | 5 | II-38 | 1 mg/kg | i.p. | QW×2 |
| 25 | 5 | II-49 | 1 mg/kg | i.p. | QW×2 |
| 26 | 5 | II-4 | 1 mg/kg | i.p. | QW×2 |

6 experimental groups were set for II-45, II-51, II-54, II-61, and II-62, as shown in Table 11:

**Table 11**

| Group | Number of mice | Test compound | Dose | Administration route | Administration schedule |
|---|---|---|---|---|---|
| 27 | 5 | Vehicle | -- | i.p. | QW×2 |
| 28 | 5 | II-61 | 1 mg/kg | i.p. | QW×2 |
| 29 | 5 | II-62 | 1 mg/kg | i.p. | QW×2 |
| 30 | 5 | II-45 | 1 mg/kg | i.p. | QW×2 |
| 31 | 5 | II-54 | 1 mg/kg | i.p. | QW×2 |
| 32 | 5 | II-51 | 1 mg/kg | i.p. | QW×2 |

6 experimental groups were set for II-46, II-55, II-64, II-65, and I-65, as shown in Table 12:

**Table 12**

| Group | Number of mice | Test compound | Dose | Administration route | Administration schedule |
|---|---|---|---|---|---|
| 33 | 5 | Vehicle | -- | i.p. | QW×2 |
| 34 | 5 | II-46 | 1 mg/kg | i.p. | Q2W×2 |
| 35 | 5 | II-55 | 1 mg/kg | i.p. | Q2W×2 |
| 36 | 5 | II-64 | 1 mg/kg | i.p. | Q2W×2 |
| 37 | 5 | II-65 | 1 mg/kg | i.p. | Q2W×2 |
| 38 | 5 | I-65 | 0.15 mg/kg | i.p. | QW×2 |

| | | | | | |
|---|---|---|---|---|---|
| Note: i.t.: intratumoral injection, i.v.: intravenous injection, i.p.: intraperitoneal injection | | | | | |

Experimental method: CT26-hHER2 cell line (3.0 × 10⁶/mouse) was inoculated subcutaneously on the right back of experimental mice. The inoculation volume of each mouse was 0.1 mL. The growth of the tumor was observed regularly. When the tumor grew to about 100 mm³, the mice were randomly grouped according to the size of the tumor and their body weights, and the drug was administered in accordance with the administration schedule, and the body weights and tumor sizes of the mice were measured two times per week throughout the experimental process.

Tumor size calculation formula: tumor volume (mm³) = 0.5 × (tumor long diameter × tumor short diameter²).

The experimental results are shown in Tables 13-18:

**Table 13**

| Group | Tumor volume (Day 7, mm³) | Tumor volume (Day 24, mm³) | TGI (%) | T/C (%) | P value |
|---|---|---|---|---|---|
| 1 | 106.4±5.6 | 2070.1±402.4 | -- | -- | -- |
| 2 | 106.7±10.1 | 292.9±81.0 | 85.9 | 14.2 | 0.0015 |
| 3 | 105.8±8.4 | 0±0 | 100.0 | 0.0 | 0.0000 |
| 4 | 105.6±6.3 | 540.7±170.3 | 73.9 | 26.1 | 0.0057 |
| 5 | 105.5±5.7 | 1066.8±221.3 | 48.5 | 51.5 | 0.0538 |
| 6 | 106.0±9.4 | 520.8±230.5 | 74.8 | 25.2 | 0.0075 |

**Table 14**

| Group | Tumor volume (Day 8, mm³) | Tumor volume (Day 21, mm³) | TGI (%) | T/C (%) | P value |
|---|---|---|---|---|---|
| 7 | 105.0±5.8 | 1989.7±384.1 | -- | -- | -- |
| 8 | 106.4±6.6 | 1659.2±407.1 | 16.61 | 83.39 | 0.5644 |

**Table 15**

| Group | Tumor volume (Day 14, mm³) | Tumor volume (Day 27, mm³) | TGI (%) | T/C (%) | P value |
|---|---|---|---|---|---|
| 9 | 107.5±14.5 | 1847.4±364.3 | -- | -- | -- |
| 10 | 107.1±24.1 | 1882.8±1010.2 | -1.9 | 101.9 | 0.9693 |
| 11 | 107.1±33.7 | 1121.5±317.8 | 39.3 | 60.7 | 0.2262 |
| 12 | 107.0±8.1 | 351.4±135.0 | 81.0 | 19.0 | 0.0234 |
| 13 | 106.8±17.4 | 84.8±17.3 | 95.4 | 4.6 | 0.0110 |
| 14 | 107.4±23.6 | 176.5±82.2 | 90.4 | 9.6 | 0.0142 |

**Table 16**

| Group | Tumor volume (Day 8, mm³) | Tumor volume (Day 23, mm³) | TGI (%) | T/C (%) | P value |
|---|---|---|---|---|---|
| 15 | 96.5±10.4 | 2212.2±505.2 | N/A | N/A | N/A |
| 16 | 96.4±10.4 | 770.0±284.1 | 65.2 | 34.8 | 0.0376 |
| 17 | 96.2±9.0 | 355.0±219.4 | 84.0 | 16.0 | 0.0098 |
| 18 | 96.5±9.8 | 57.9±44.9 | 97.4 | 2.6 | 0.0028 |
| 19 | 96.5±9.6 | 200.4±172.1 | 90.9 | 9.1 | 0.0055 |
| 20 | 96.5±7.3 | 96.9±65.1 | 95.6 | 4.4 | 0.0032 |
| 21 | 96.3±8.6 | 248.9±97.3 | 88.7 | 11.3 | 0.0051 |
| 22 | 96.7±9.9 | 200.0±70.9 | 91.0 | 9.0 | 0.0043 |
| 23 | 96.8±11.6 | 476.0±235.1 | 78.5 | 21.5 | 0.0143 |
| 24 | 96.8±6.8 | 42.5±40.4 | 98.1 | 1.9 | 0.0027 |
| 25 | 96.5±11.5 | 529.4±193.5 | 76.1 | 23.9 | 0.0144 |
| 26 | 96.2±8.2 | 249.3±103.4 | 88.7 | 11.3 | 0.0052 |

**Table 17**

| Group | Tumor volume (Day 8, mm³) | Tumor volume (Day 25, mm³) | TGI (%) | T/C (%) | P value |
|---|---|---|---|---|---|
| 27 | 97.7±7.2 | 2425.7±416.2 | N/A | N/A | N/A |
| 28 | 98±7.3 | 86.6±57.2 | 96.4 | 3.6 | 0.0005 |
| 29 | 97.7±7.1 | 1343.5±205.3 | 44.6 | 55.4 | 0.0480 |
| 30 | 97.4±7.4 | 63.8±59.7 | 97.4 | 2.6 | 0.0005 |
| 31 | 97.4±7.1 | 8.5±8.5 | 99.6 | 0.4 | 0.0004 |
| 32 | 97.5±7.5 | 658.6±266.4 | 72.9 | 27.1 | 0.0072 |

**Table 18**

| Group | Tumor volume (Day 12, mm³) | Tumor volume (Day 24, mm³) | TGI (%) | T/C (%) | P value |
|---|---|---|---|---|---|
| 33 | 104.5±16.8 | 3292.6±667.2 | N/A | N/A | N/A |
| 34 | 104.0±16.1 | 650.0±142.1 | 80.3 | 19.7 | 0.0047 |
| 35 | 104.2±12.0 | 1100.1±363.9 | 66.6 | 33.4 | 0.0204 |
| 36 | 104.1±12.6 | 214.9±97.6 | 93.5 | 6.5 | 0.0018 |
| 37 | 103.9±11.8 | 1206.3±169.8 | 63.4 | 36.6 | 0.0163 |
| 38 | 104.1±12.7 | 692.7±238.8 | 79.0 | 21.0 | 0.0063 |

The results showed that, compared with compound 1-3 and Trastuzumab, the compounds of the present disclosure not only achieved systemic administration, but also were able to display better efficacy in the mouse colon cancer CT26-hHER2 subcutaneous xenograft model.

### Experimental Example 9: In vivo efficacy assay of the mouse colon cancer CT26 subcutaneous xenograft model

Cell culture: Mouse colon cancer CT26 cells were maintained as monolayers in RPMI-1640 medium containing 10% fetal bovine serum at 37°C in a constant temperature incubator with 5% CO₂. Cells were subcultured twice weekly after treatment with trypsin-EDTA. Cells in the exponential growth phase were harvested and counted for inoculation.

Experimental animals: BALB/c mice, 6-8 weeks, 16-18 g, purchased from Beijing Vital River Laboratory Animal Technology Co., Ltd.

12 experimental groups were set for II-42, II-58, I-3, I-38, I-43, I-44, I-53, I-58, I-59, I-61, and I-64, as shown in Table 19:

**Table 19**

| Group | Number of mice | Test compound | Dose | Administration route | Administration schedule |
|---|---|---|---|---|---|
| 1 | 4 | Vehicle | -- | i.p. | QW×2 |
| 2 | 4 | II-42 | 1 mg/kg | i.p. | QW×2 |
| 3 | 4 | II-58 | 1 mg/kg | i.p. | QW×2 |
| 4 | 4 | I-3 | 0.15 mg/kg | i.p. | QW×2 |
| 5 | 4 | I-43 | 0.15 mg/kg | i.p. | QW×2 |
| 6 | 4 | I-44 | 0.15 mg/kg | i.p. | QW×2 |
| 7 | 4 | I-58 | 0.15 mg/kg | i.p. | QW×2 |
| 8 | 4 | I-61 | 0.15 mg/kg | i.p. | QW×2 |
| 9 | 4 | I-64 | 0.15 mg/kg | i.p. | QW×2 |
| 10 | 4 | I-53 | 0.15 mg/kg | i.p. | QW×2 |
| 11 | 4 | I-38 | 0.15 mg/kg | i.p. | QW×2 |
| 12 | 4 | I-59 | *0.15* mg/kg | i.p. | QW×2 |

| | | | | | |
|---|---|---|---|---|---|
| Note: i.p.: intraperitoneal injection | | | | | |

Experimental method: CT26 cell line (3.0 × 10⁶/mouse) was inoculated subcutaneously on the right back of experimental mice. The inoculation volume of each mouse was 0.1 mL. The growth of the tumor was observed regularly. When the tumor grew to about 100 mm³, the mice were randomly grouped according to the size of the tumor and their body weights, and the drug was administered in accordance with the administration schedule, and the body weights and tumor sizes of the mice were measured two times per week throughout the experimental process.

Tumor size calculation formula: tumor volume (mm³) = 0.5 × (tumor long diameter × tumor short diameter²).

The experimental results are shown in Table 20:

**Table 20**

| Group | Tumor volume (Day 9, mm³) | Tumor volume (Day 24, mm³) | TGI (%) | T/C (%) | P value |
|---|---|---|---|---|---|
| 1 | 169.9±25.0 | 2377.7±225.7 | N/A | N/A | N/A |
| 2 | 170.6±24.8 | 937.9±202.2 | 60.6 | 39.4 | 0.003 |
| 3 | 170.6±31.8 | 827±172.6 | 65.2 | 34.8 | 0.002 |
| 4 | 169.9±19.2 | 0±0 | 100.0 | 0.0 | 0.000 |
| 5 | 170.1±16.8 | 1026.8±248.6 | 56.8 | 43.2 | 0.007 |
| 6 | 169.5±31.2 | 998.7±206.1 | 58.0 | 42.0 | 0.004 |
| 7 | 169.6±33.8 | 631±187.2 | 73.5 | 26.5 | 0.001 |
| 8 | 169.6±19.2 | 762.6±141.9 | 67.9 | 32.1 | 0.001 |
| 9 | 169.7±25.6 | 191.3±49.3 | 92.0 | 8.0 | 0.000 |
| 10 | 169.6±21.7 | 382.2±224.4 | 83.9 | 16.1 | 0.001 |
| 11 | 170.1±21.6 | 295.7±81.3 | 87.6 | 12.4 | 0.000 |
| 12 | 169.8±27.7 | 1239.5±274.4 | 47.9 | 52.1 | 0.019 |

### Experimental Example 10: In vivo efficacy assay of mouse melanoma B16F10-hHER2 subcutaneous xenograft model

Cell culture: Mouse melanoma B16F10-hHER2 cells were maintained as monolayers in DMEM medium containing 10% fetal bovine serum and 1 µg/mL puromycin in a constant temperature incubator at 37°C with 5% CO₂. Tumor cells were subcultured twice a week. Cells in the exponential growth phase were harvested and counted for inoculation.

Experimental animals: C57 mice, 6-9 weeks, 18-22 g, purchased from Beijing Vital River Laboratory Animal Technology Co., Ltd.

4 experimental groups were set for II-5, C-7, and Trastuzumab, as shown in Table 21 below:

**Table 21**

| Group | Number of mice | Test compound | Dose | Dose (small molecule content) | Administration route | Administration schedule |
|---|---|---|---|---|---|---|
| 1 | 6 | Vehicle | -- | -- | i.t. | 2 doses |
| 2 | 6 | Trastuzumab | 10 mg/kg | -- | i.p. | 2 doses |
| 3 | 6 | II-5 | 0.25 mg/kg | 0.01 mg/kg | i.p. | 2 doses |
| 4 | 6 | C-7 | 0.25 mg/kg | 0.01 mg/kg | i.p. | 2 doses |

| | | | | | | |
|---|---|---|---|---|---|---|
| Note: i.t.: intratumoral injection, i.p.: intraperitoneal injection | | | | | | |

Experimental method: B16F10-hHER2 cell line (1.0 × 10⁵/mouse) was inoculated subcutaneously on the right back of experimental mice. The inoculation volume of each mouse was 0.1 mL. The growth of the tumor was observed regularly. When the tumor grew to about 100 mm³, the mice were randomly grouped according to the size of the tumor and their body weights, and the drug was administered on days 14 and 21 in accordance with the administration schedule, and the body weights and tumor sizes of the mice were measured three times per week throughout the experimental process.

Tumor size calculation formula: tumor volume (mm³) = 0.5 × (tumor long diameter × tumor short diameter²).

The experimental results are shown in Table 22:

**Table 22**

| Group | Tumor volume (Day 14, mm³) | Tumor volume (Day 28, mm³) | TGI (%) | T/C (%) | P value |
|---|---|---|---|---|---|
| 1 | 92.1±15.9 | 1929.4±499.7 | N/A | N/A | N/A |
| 2 | 91.8±19.3 | 2256.7±593.2 | -33.6 | 133.6 | 0.6820 |
| 3 | 91.8±15.1 | 557.9±111.1 | 71.3 | 28.7 | 0.0231 |
| 4 | 91.9±19.6 | 2633.4±806.3 | -18.8 | 118.8 | 0.4529 |

The results showed that in the mouse melanoma B16F10-hHER2 subcutaneous xenograft model, the efficacy of II-5 was significantly superior to that of C-7 and Trastuzumab.

### Experimental Example 11: In vivo efficacy assay of human lung cancer cell NCI-H1373 subcutaneous xenograft model

Cell culture: Human lung cancer NCI-H1373 cells were maintained as monolayers in RPMI-1640 medium containing 10% fetal bovine serum at 37°C in a constant temperature incubator with 5% CO₂. Tumor cells were subcultured twice a week. Cells in the exponential growth phase were harvested and counted for inoculation.

Experimental animals: Balb/c nude mice, 6-8 weeks, 20-25 g, purchased from Jiangsu GemPharmatech Co., Ltd.

2 experimental groups were set for II-63, as shown in Table 23 below:

**Table 23**

| Group | Number of mice | Test compound | Dose | Administration route | Administration schedule |
|---|---|---|---|---|---|
| 1 | 3 | Vehicle | -- | i.v. | QW×2 |
| 2 | 3 | II-63 | 1 mg/kg | 1.v. | QW×2 |

| | | | | | |
|---|---|---|---|---|---|
| Note: i.v.: intravenous injection | | | | | |

Experimental method: NCI-H1373 cell line (5 × 10⁶/mouse) was inoculated subcutaneously on the right back of experimental mice. The inoculation volume of each mouse was 0.1 mL. The growth of the tumor was observed regularly. When the tumor grew to about 100 mm³, the mice were randomly grouped according to the size of the tumor and their body weights, and the drug was administered in accordance with the administration schedule, and the body weights and tumor sizes of the mice were measured two times per week throughout the experimental process.

Tumor size calculation formula: tumor volume (mm³) = 0.5 × (tumor long diameter × tumor short diameter²).

The experimental results are shown in Table 24:

**Table 24**

| Group | Tumor volume (Day 11, mm³) | Tumor volume (Day 20, mm³) | TGI (%) | T/C (%) | P value |
|---|---|---|---|---|---|
| 1 | 112.3±11.4 | 327.0±33.4 | N/A | N/A | N/A |
| 2 | 113.3±4.1 | 36.7±15.5 | 88.8 | 11.2 | 0.0036 |

### Experimental Example 12: In vivo efficacy assay of human pancreatic cancer cell HPAC subcutaneous xenograft model

Cell culture: Human pancreatic cancer HPAC cells were maintained as monolayers in RPMI-1640 medium containing 10% fetal bovine serum at 37°C in a constant temperature incubator with 5% CO₂. Tumor cells were subcultured twice a week. Cells in the exponential growth phase were harvested and counted for inoculation.

Experimental animals: Balb/c nude mice, 6-8 weeks, 20-25 g, purchased from Jiangsu GemPharmatech Co., Ltd.

2 experimental groups were set for II-63, as shown in Table 25 below:

**Table 25**

| Group | Number of mice | Test compound | Dose | Administration route | Administration schedule |
|---|---|---|---|---|---|
| 1 | 3 | Vehicle | -- | i.v. | QW×2 |
| 2 | 3 | II-63 | 1 mg/kg | 1.v. | QW×2 |

| | | | | | |
|---|---|---|---|---|---|
| Note: i.v.: intravenous injection | | | | | |

Experimental method: HPAC cell line (5 × 10⁶/mouse) was inoculated subcutaneously on the right back of experimental mice. The inoculation volume of each mouse was 0.1 mL. The growth of the tumor was observed regularly. When the tumor grew to about 100 mm³, the mice were randomly grouped according to the size of the tumor and their body weights, and the drug was administered in accordance with the administration schedule, and the body weights and tumor sizes of the mice were measured two times per week throughout the experimental process.

Tumor size calculation formula: tumor volume (mm³) = 0.5 × (tumor long diameter × tumor short diameter²).

The experimental results are shown in Table 26:

**Table 26**

| Group | Tumor volume (Day 7, mm³) | Tumor volume (Day 18, mm³) | TGI (%) | T/C | P value |
|---|---|---|---|---|---|
| 1 | 100.1±7.8 | 688.5±85.0 | N/A | N/A | N/A |
| 2 | 99.4±6.1 | 125.4±27.1 | 81.8 | 18.2 | 0.0032 |

### Experimental Example 13: In vivo efficacy assay of human breast cancer MDA-MB-468 subcutaneous xenograft model

Cell culture: Human breast cancer MDA-MB-468 cells were maintained as monolayers in a culture medium containing 10% fetal bovine serum and 90% DMEM in a constant temperature incubator at 37°C with 5% CO₂. Tumor cells were subcultured twice a week. Cells in the exponential growth phase were harvested and counted for inoculation.

Experimental animals: BALB/c-nude mice, 6-8 weeks, 18-22 g, purchased from Shanghai Lingchang Biotechnology Co., Ltd.

4 experimental groups were set for II-48 and II-63, as shown in Table 27 below:

**Table 27**

| Group | Number of mice | Test compound | Dose | Administration route | Administration schedule |
|---|---|---|---|---|---|
| 1 | 5 | Vehicle | -- | 1.v. | QW×3 |
| 2 | 5 | Antibody 8 | 10 mg/kg | i.v. | QW×3 |
| 3 | 5 | II-48 | 1 mg/kg | 1.v. | QW×3 |
| 4 | 5 | II-63 | 1 mg/kg | 1.v. | QW×3 |

| | | | | | |
|---|---|---|---|---|---|
| Note: i.v.: intravenous injection | | | | | |

Experimental method: MDA-MB-468 cell line (2.5 × 10⁶/mouse) was inoculated subcutaneously on the right back of experimental mice. The inoculation volume of each mouse was 0.2 mL. The growth of the tumor was observed regularly. When the tumor grew to about 250 mm³, the mice were randomly grouped according to the size of the tumor and their body weights, and the drug was administered in accordance with the administration schedule, and the body weights and tumor sizes of the mice were measured two times per week throughout the experimental process.

Tumor size calculation formula: tumor volume (mm³) = 0.5 × (tumor long diameter × tumor short diameter²).

The experimental results are shown in Table 28:

**Table 28**

| Group | Tumor volume (Day 0, mm³) | Tumor volume (Day 21, mm³) | TGI (%) |
|---|---|---|---|
| 1 | 249.4±24.9 | 479.5±61.8 | N/A |
| 2 | 249.3±28.5 | 397.5±70.9 | 17.4 |
| 3 | 249.1±20.8 | 10.6±11.5 | 97.8 |
| 4 | 249.6±22.1 | 50.9±50.5 | 88.7 |

### Experimental Example 14: In vivo efficacy assay of colon cancer B-hNECTIN4 MC38 subcutaneous xenograft model

Cell culture: Colon cancer B-hNECTIN4 MC38 cells (Biocytogen Pharmaceuticals (Beijing) Co., Ltd.) were maintained as monolayers in RPMI-1640 medium containing 10% fetal bovine serum at 37°C in a constant temperature incubator with 5% CO₂. Tumor cells were subcultured twice a week. Cells in the exponential growth phase were harvested and counted for inoculation.

Experimental animals: C57BL/6 mice, 6-8 weeks, 17-21 g, purchased from Beijing Vital River Laboratory Animal Technology Co., Ltd.

3 experimental groups were set for II-83, as shown in Table 29 below:

**Table 29**

| Group | Number of mice | Test compound | Dose | Administration route | Administration schedule |
|---|---|---|---|---|---|
| 1 | 5 | Vehicle | -- | i.v. | QW×2 |
| 2 | 5 | Antibody 11 | 1 mg/kg | i.v. | QW×2 |
| 3 | 5 | II-83 | 1 mg/kg | i.v. | QW×2 |

| | | | | | |
|---|---|---|---|---|---|
| Note: i.v.: intravenous injection | | | | | |

Experimental method: B-hNECTIN4 MC38 cell line (5 × 10⁵/mouse) was inoculated subcutaneously on the right back of experimental mice. The inoculation volume of each mouse was 0.1 mL. The growth of the tumor was observed regularly. When the tumor grew to about 100 mm³, the mice were randomly grouped according to the size of the tumor and their body weights, and the drug was administered in accordance with the administration schedule, and the body weights and tumor sizes of the mice were measured two times per week throughout the experimental process.

Tumor size calculation formula: tumor volume (mm³) = 0.5 × (tumor long diameter × tumor short diameter²).

The experimental results are shown in Table 30:

**Table 30**

| Group | Tumor volume (Day 0, mm³) | Tumor volume (Day 20, mm³) | TGI (%) | P value | Tumor clearance ratio |
|---|---|---|---|---|---|
| 1 | 103±3 | 1473±166 | N/A | N/A | N/A |
| 2 | 103±3 | 1971±225 | -36.4 | 0.4302 | 0/5 |
| 3 | 103±3 | 0±0 | 107.5 | 0.0015 | 5/5 |

### Experimental Example 15: In vivo efficacy assay of the mouse colon cancer CT26-hEGFR subcutaneous xenograft model

Cell culture: Mouse colon cancer CT26-hEGFR cells were maintained as monolayers in RPMI-1640 medium containing 10% fetal bovine serum at 37°C in a constant temperature incubator with 5% CO₂. Tumor cells were subcultured twice a week. Cells in the exponential growth phase were harvested and counted for inoculation.

Experimental animals: Balb/c mice, 6-8 weeks, 18-22 g, purchased from Jiangsu GemPharmatech Co., Ltd.

4 experimental groups were set for II-47, II-85, and II-86, as shown in Table 31 below:

**Table 31**

| Group | Number of mice | Test compound | Dose | Administration route | Administration schedule |
|---|---|---|---|---|---|
| 1 | 5 | Vehicle | -- | S.C. | QW×2 |
| 2 | 5 | II-47 | 1 mg/kg | S.C. | QW×2 |
| 3 | 5 | II-85 | 1 mg/kg | S.C. | QW×2 |
| 4 | 5 | II-86 | 1 mg/kg | S.C. | QW×2 |

| | | | | | |
|---|---|---|---|---|---|
| Note: s.c.: subcutaneous injection | | | | | |

Experimental method: CT26-hEGFR cell line (5 × 10⁶/mouse) was inoculated subcutaneously on the right back of experimental mice. The inoculation volume of each mouse was 0.1 mL. The growth of the tumor was observed regularly. When the tumor grew to about 100 mm³, the mice were randomly grouped according to the size of the tumor and their body weights, and the drug was administered in accordance with the administration schedule, and the body weights and tumor sizes of the mice were measured two times per week throughout the experimental process.

Tumor size calculation formula: tumor volume (mm³) = 0.5 × (tumor long diameter × tumor short diameter²).

The experimental results are shown in Table 32:

**Table 32**

| Group | Tumor volume (Day 0, mm³) | Tumor volume (Day 14, mm³) | TGI (%) | P value |
|---|---|---|---|---|
| 1 | 147.2±12.9 | 3847.3±813.8 | N/A | N/A |
| 2 | 147.3±12.5 | 969.1±236.6 | 75 | 0.0094 |
| 3 | 147.7±11.7 | 737.7±157.5 | 81 | 0.0055 |
| 4 | 147.1±14.0 | 743.9±296.4 | 81 | 0.0072 |

## Claims

1. An immune-stimulating antibody conjugate represented by Formula II or a pharmaceutically acceptable salt thereof, 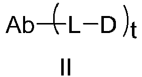
wherein Ab is an antibody;
t is 1 to 8;
L is a linker with the following combination: -(L₁)ₐ-(Z)_{b}-M-; L₁ is connected to D, and M is connected to Ab;
a is 0, 1, 2, 3, 4, 5, or 6; b is 0, 1, 2, 3, 4, 5, 6, 7, 8, 9, or 10;
D is a group formed by the loss of one hydrogen atom from a compound represented by 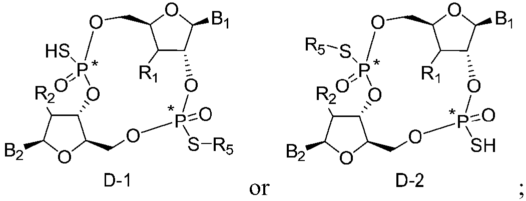 the ^{*}-marked P stereo configurations are each independently R, S, or R/S;
B₁ and B₂ are each independently 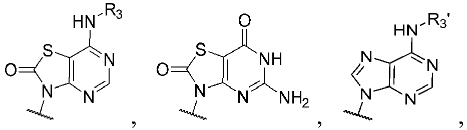 or 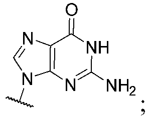 and at least one of B₁ or B₂ is 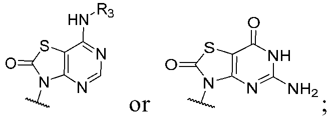
R₁ and R₂ are independently -OH, -N₃, F, -OCH₃, or -SCF₃;
R₃ and R_{3'} are independently H, -C(O)R₄, -C(O)OR₄, -OR₄, or -R₄;
R₄ is C₁₋₁₀ alkyl, C₆₋₁₀ aryl, 5- to 10-membered heteroaryl, C₃₋₁₀ cycloalkyl, 3- to 10-membered heterocycloalkyl, C₆₋₁₀ aryl-C₁₋₁₀ alkyl, 5- to 10-membered heteroaryl-C₁₋₁₀ alkyl, C₃₋₁₀ cycloalkyl-C₁₋₁₀ alkyl, or 3- to 10-membered heterocycloalkyl-C₁₋₁₀ alkyl; the R₄ is unsubstituted or optionally substituted at any position with 1 to 3 substituents selected from hydroxyl, thiol, dithiol, amino, C₁₋₆ alkylamino, and C₁₋₆ alkylamino-C₁₋₁₀ alkyl;
R₅ is H, -(L₂)_{d}-(Z)ₑ-(maleimido), or -(L₂)_{d}-(Z)ₑ-H;
d is 0, 1, 2, 3, 4, 5, or 6; e is 0, 1, 2, 3, 4, 5, 6, 7, 8, 9, or 10;
each L₁ and each L₂ is independently 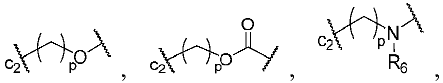 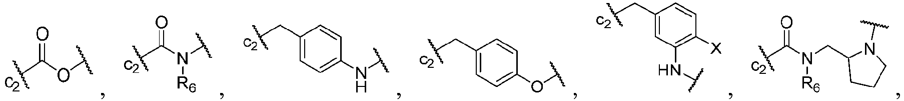 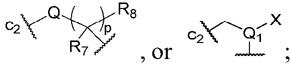 in L₁, the c₂ side is connected to D; in L₂, the c₂ side is connected to the thiol group in D;
X is 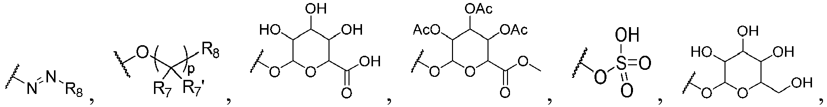 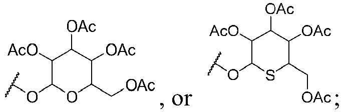
p is independently 1, 2, or 3;
Q is a linking bond, -C(O)O-, -C(O)N(R₆)-, or -C₁₋₃ alkylene-O-;
Q₁ is phenyl or pyridyl;
R₆ is H or C₁₋₆ alkyl; the C₁₋₆ alkyl is unsubstituted or optionally substituted at any position with one C₁₋₆ alkylamino or C₁₋₆ alkylsulfonyl;
R₇ and R_{7'} are each independently H or C₁₋₆ alkyl;
R₈ is phenyl or 5- to 10-membered heteroaryl; the R₈ is unsubstituted or optionally substituted at any position with 1-3 substituents selected from hydroxyl, amino, carboxyl, cyano, nitro, thiol, C₁₋₆ alkyl, C₃₋₈ cycloalkyl, halogen, halo-C₁₋₆ alkyl, C₁₋₆ alkylamino, C₁₋₆ alkoxy, halo-C₁₋₆ alkoxy, C₁₋₆ alkylthio, amido, hydroxyamino, aldehyde, acetyl, methoxycarbonyl, amino-C₁₋₆ alkyl, hydroxy-C₁₋₆ alkyl, and C₁₋₆ alkylsulfonyl;
each Z is independently -(A)ᵥ-, -PEG-, -C(O)-(CH₂)ₓ-, -NR₉-(CH₂)_{y}-, -O-(CH₂)_{y}-, -S-(CH₂)_{y}-, -(CH₂)ₓ-C₆₋₁₄ arylene-(CH₂)_{y}-, -(CH₂)ₓ-5- to 6-membered heteroarylene-(CH₂)_{y}-, - (CH₂)ₓ-C₃₋₆ cycloalkylene-(CH₂)_{y}-, -(CH₂)ₓ-3- to 6-membered heterocycloalkylene-(CH₂)_{y}-, - NR₉-(CH₂)ₓ-C(O)-, -O-(CH₂)ₓ-C(O)-, -S-(CH₂)ₓ-C(O)-, -(CH₂)ₓCH(-L₃-R₉)-C(O)-, - (CH₂)ₓCH(-L₃-R₉ₐ)-C(O)-, -(CH₂)ₓ-S-S-(CH₂)_{y}-, C₁₋₆ alkylene, or C₂₋₆ alkenylene;
L₃ is a linking bond, -NH-, or 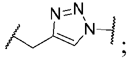
R₉ is independently hydrogen, C₁₋₆ alkyl, -PO(OH)₂, -PO(OCH₃)₂, -C(O)-(CH₂CH₂O)ₙ-CH3, -(CH₂CH₂O)ₙ-CH3, 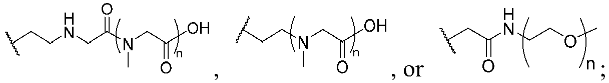
R₉ₐ is independently 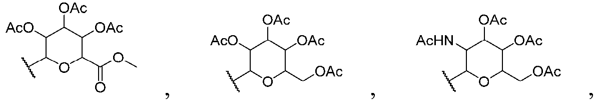 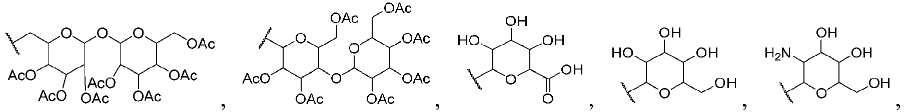 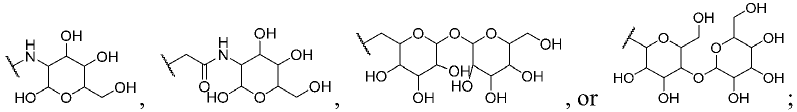
-PEG- is -(CH₂CH₂O)ₙ-(CH₂)ᵤ- or -(CH₂CH₂O)ₙ-(CH₂)ᵤ-C(O)-;
A is independently an amino acid residue;
x and y are independently 0, 1, 2, 3, 4, 5, 6, 7, 8, 9, or 10; v is independently 1, 2, 3, 4, or 5; n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50; u is independently 0, 1, 2, 3, 4, or 5;
M is a linker head connected to Ab.

2. The immune-stimulating antibody conjugate represented by Formula II or the pharmaceutically acceptable salt thereof according to claim 1, wherein the immune-stimulating antibody conjugate represented by Formula II satisfies one or more of the following conditions:
(1) Ab is an anti-HER2 antibody, an anti-EGFR antibody, or an anti-5T4 antibody;
(2) t is any value from 2 to 8;
(3) each L₁ and each L₂ is independently 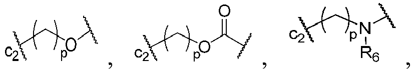 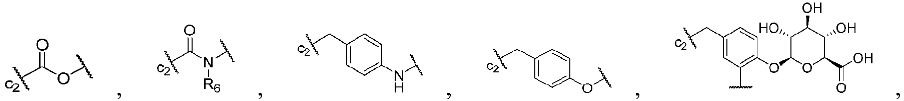 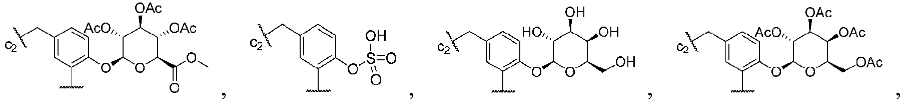 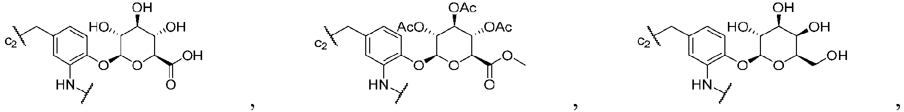 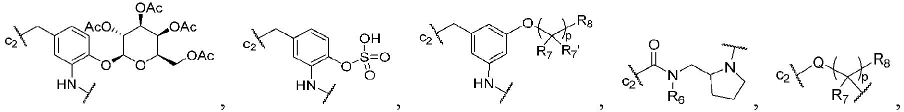 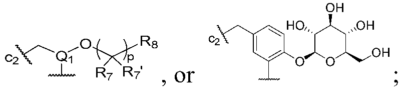 in L₁, the c₂ side is connected to D; in L₂, the c₂ side is connected to the thiol group in D;
(4) Z is independently -(A)ᵥ-, -PEG-, -C(O)-(CH₂)ₓ-, -NR₉-(CH₂)_{y}-, -O-(CH₂)_{y}-, -S-(CH₂)_{y}-, -(CH₂)ₓ-C₆₋₁₄ arylene-(CH₂)_{y}-, -(CH₂)ₓ-5- to 6-membered heteroarylene-(CH₂)_{y}-, - (CH₂)ₓ-C₃₋₆ cycloalkylene-(CH₂)_{y}-, -(CH₂)ₓ-3- to 6-membered heterocycloalkylene-(CH₂)_{y}-, - NR₉-(CH₂)ₓ-C(O)-, -O-(CH₂)ₓ-C(O)-, -S-(CH₂)ₓ-C(O)-, -(CH₂)ₓCH(NHR₉)-C(O)-, -(CH₂)ₓ-S-S-(CH₂)_{y}-, C₁₋₆ alkylene, or C₂₋₆ alkenylene; x and y are independently any integer of 0, 1, 2, 3, 4, 5, 6, 7, 8, 9 or 10; v is independently any integer of 1, 2, 3, 4 or 5; in the "5- to 6-membered heteroarylene" of -(CH₂)ₓ-5- to 6-membered heteroarylene-(CH₂)_{y}-, the heteroatom is selected from one or more kinds of N, O and S, and the number of heteroatoms is 1, 2 or 3; in the "3-to 6-membered heterocycloalkylene" of -(CH₂)ₓ-3- to 6-membered heterocycloalkylene-(CH₂)_{y}-, the heteroatom is selected from one or more kinds of N, O and S, and the number of heteroatoms is 1, 2 or 3;
(5) in M, the linker head is a group formed by click reaction, click-like reaction, thiol linkage, amino linkage, oxime linkage, or hydrazone linkage;
(6) D is a group formed by the loss of one hydrogen atom from a compound represented by 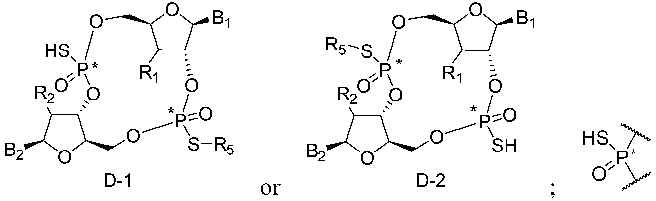 is 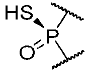 and/or 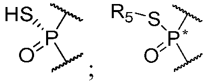 is

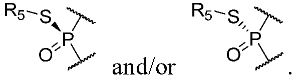

3. The immune-stimulating antibody conjugate represented by Formula II or the pharmaceutically acceptable salt thereof according to claim 2, wherein the immune-stimulating antibody conjugate represented by Formula II satisfies one or more of the following conditions:
(1) Ab is Trastuzumab, Pertuzumab, Cetuximab, huA1, Cetuximab, Nimotuzumab, or a variant thereof;
(2) t is any value from 6 to 8;
(3) -(L₁)ₐ- is absent, 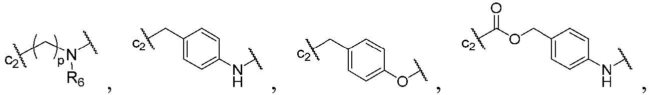 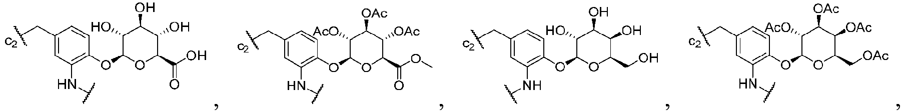 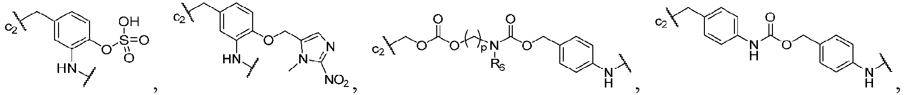 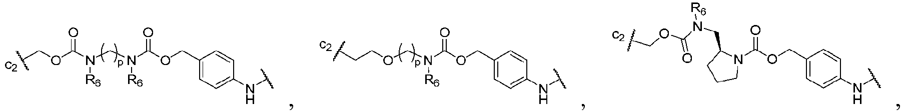 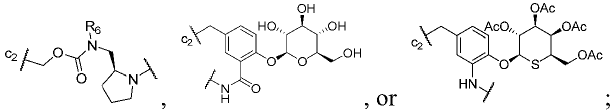 the c₂ side is connected to D;
(4) -(Z)_{b}- is any one of the following combinations: -(CH₂)ₓ-(c₁), -(CH₂CH₂O)ₙ-(CH₂)ᵤ-NHC(O)-(CH₂)ₓ-(c₁), -C(O)-(CH₂)ₓ-(c₁), -C(O)-(CH₂)ₓ-C₃₋₆ cycloalkylene-(CH₂)_{y}-(c₁), -C(O)-(CH₂)ₓ-phenylene-(CH₂)_{y}-(c₁), -C(O)-(CH₂CH₂O)ₙ-(CH₂)ᵤ-NHC(O)-(CH₂)ₓ-(c₁), -C(O)-(CH₂CH₂O)ₙ-(CH₂)ᵤ-NHC(O)-(CH₂)ₓ-phenylene-(CH₂)_{y}-(c₁), -(A)ᵥ-(c₁), -(A)ᵥ-C(O)-(CH₂)ₓ-(c₁), -(A)ᵥ-C(O)-(CH₂)ₓ-phenylene-(CH₂)_{y}-(c₁), -(A)ᵥ-C(O)-(CH₂CH₂O)ₙ-(CH₂)ᵤ-NHC(O)-(CH₂)ₓ-(c₁), -(A)ᵥ-C(O)-(CH₂CH₂O)ₙ-(CH₂)ᵤ-NHC(O)-(CH₂)ₓ-phenylene-(CH₂)_{y}-(c₁), -C(O)-(CH₂)ₓ-(A)ᵥ-C(O)-(CH₂)ₓ-(c₁), -(A)ᵥ-C(O)-(CH₂CH₂O)ₙ-(CH₂)ᵤ-(c₁), -C(O)-(CH₂)ₓ-NH-(c₁), - C(O)-CH(-L₃-R₉)-NHC(O)-(CH₂)ₓ-phenylene-(CH₂)_{y}-, or -C(O)-CH(-L₃-R₉)-NHC(O)-(CH₂)ₓ-, wherein the c₁ side is connected to M;
(5) in M, the linker head is 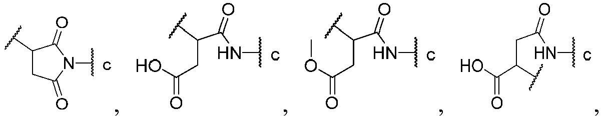 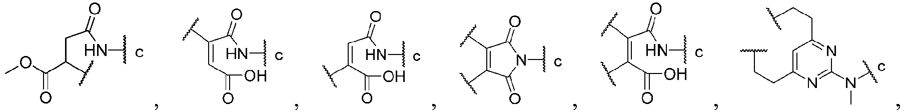 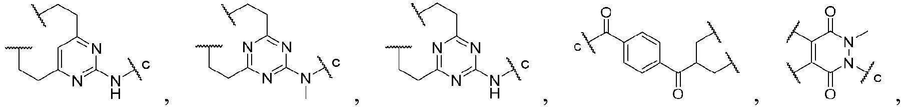 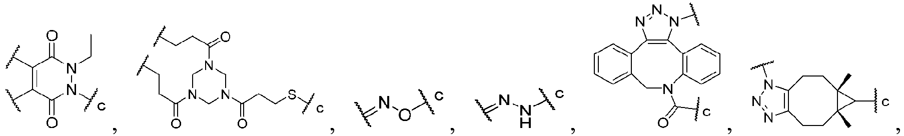 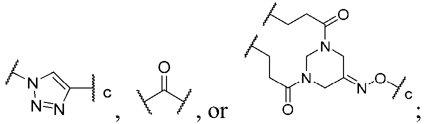 the linker head is connected to the rest of L through the c side;
(6) R₅ is H, -L₂-(Z)ₑ-(maleimido), or -L₂-(Z)ₑ-H.

4. The immune-stimulating antibody conjugate represented by Formula II or the pharmaceutically acceptable salt thereof according to claim 1, wherein the immune-stimulating antibody conjugate represented by Formula II satisfies one or more of the following conditions:
(1) -(L₁)ₐ- is 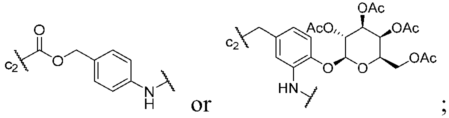 the c₂ side is connected to D;
(2) each A is independently 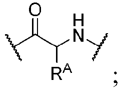 each R^{A} is independently an amino acid side chain or an amino acid side chain modified with 1 to 3 R₁₀; or R^{A} and the adjacent nitrogen atom form a five-membered heterocyclic group;
the R₁₀ is independently C₁₋₆ alkyl, C₁₋₆ alkylacyl, C₁₋₆ alkoxyacyl, -C(O)-(CH₂CH₂O)ₙ-CH₃, or -(CH₂CH₂O)ₙ-CH₃; the C₁₋₆ alkyl, C₁₋₆ alkylacyl, or C₁₋₆ alkoxyacyl is optionally substituted at any position with one R₈;
(3) R₆ is H or C₁₋₆ alkyl; the C₁₋₆ alkyl is unsubstituted or optionally substituted at any position with one dimethylamino or methylsulfonyl;
(4) B₁ and B₂ are each independently 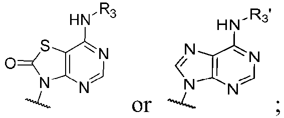 and at least one of B₁ or B₂ is

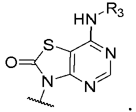

5. The immune-stimulating antibody conjugate represented by Formula II or the pharmaceutically acceptable salt thereof according to claim 1, wherein -(A)ᵥ- is 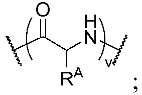 each R^{A} is independently hydrogen, methyl, ethyl, n-propyl, isopropyl, isobutyl, 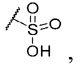 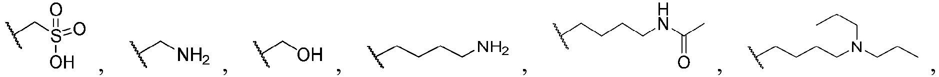 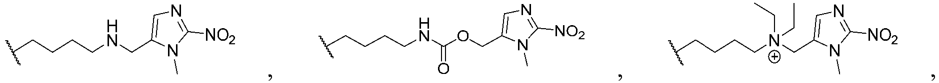 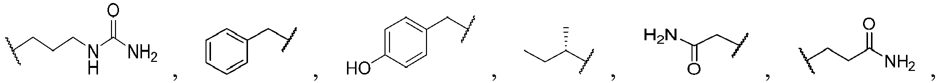 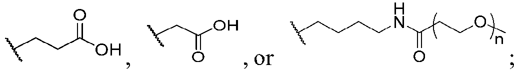 or R^{A} and the adjacent nitrogen atom form a five-membered heterocyclic group; v is 1, 2, 3, or 4; preferably, -(A)ᵥ- is

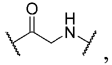

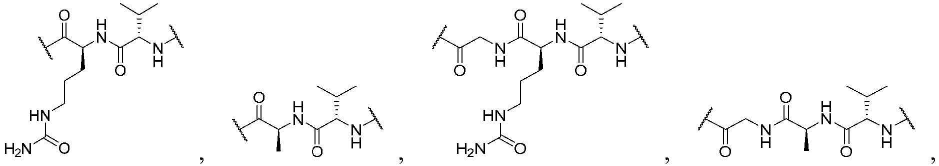

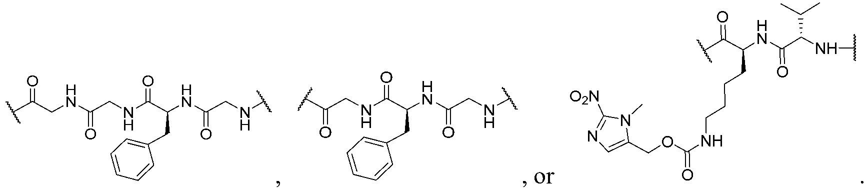

6. The immune-stimulating antibody conjugate represented by Formula II or the pharmaceutically acceptable salt thereof according to claim 1, wherein R₅ is -L₂-(Z)ₑ-H; -L₂-is 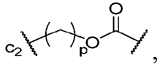 the c₂ side is connected to the thiol group in D; e is 1; -(Z)ₑ-H is C₁₋₆ alkyl;
or, R₅ is -L₂-(Z)ₑ-H; -L₂- is 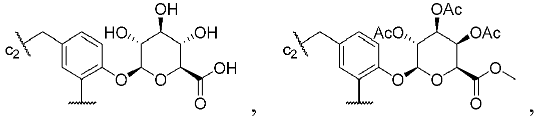 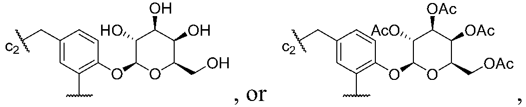 the c₂ side is connected to the thiol group in D; e is 3, -(Z)ₑ-H is -C(O)NH-(CH₂)_{y}-O-(CH₂)_{y}-H;
or, R₅ is 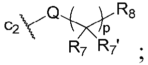 Q is a linking bond, -C(O)O-, or -C₁₋₃ alkylene-O-; R₇ and R_{7'} are each independently H or methyl;
or, R₅ is 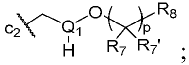 Q₁ is phenyl; R₇ and R_{7'} are each independently H or methyl;
or, R₅ is -L₂-(Z)ₑ-(maleimido); -L₂- is 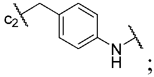 the c₂ side is connected to the thiol group in D; -(Z)ₑ- is -(A)ᵥ-C(O)-(CH₂)ₓ-(c₃); the c₃ side is connected to the maleimido;
and/or, R₈ is phenyl or 5- to 6-membered heteroaryl; the 5- to 6-membered heteroaryl is preferably pyridyl, pyrimidinyl, pyrazinyl, pyrrolyl, thienyl, furyl, pyrazolyl, thiazolyl, or oxazolyl; R₈ is unsubstituted or optionally substituted at any position with 1 to 3 substituents selected from hydroxyl, amino, cyano, carboxyl, nitro, thiol, C₁₋₆ alkyl, halogen, halo-C₁₋₆ alkyl, C₁₋₆ alkylamino, C₁₋₆ alkoxy, halo-C₁₋₆ alkoxy, C₁₋₆ alkylthio, amido, aldehyde, acetyl, methoxycarbonyl, amino-C₁₋₆ alkyl, hydroxy-C₁₋₆ alkyl, and methylsulfonyl.

7. The immune-stimulating antibody conjugate represented by Formula II or the pharmaceutically acceptable salt thereof according to claim 1, wherein D is a group formed by the loss of one hydrogen atom from the amino or thiol group in a compound represented by 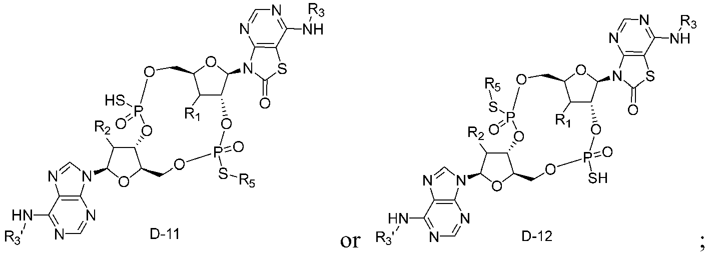
or, D is a group formed by the loss of one hydrogen atom from the amino group in a compound represented by 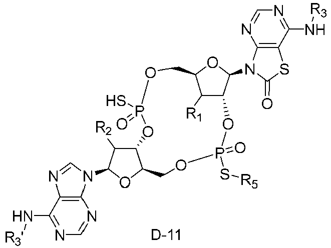 or 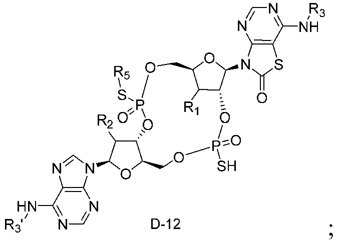 R₃ or R_{3'} are each independently 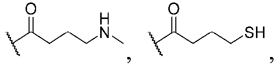 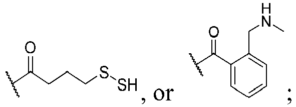
or, D is a group formed by the loss of one hydrogen atom from the thiol group in a compound represented by 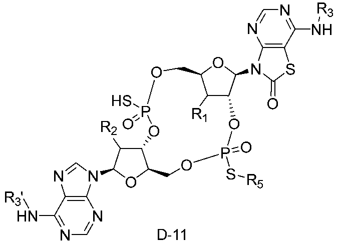 or 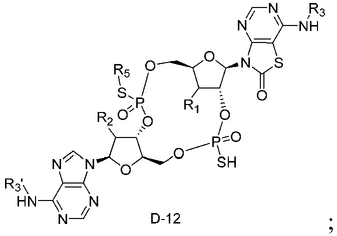
or, D is a group formed by the loss of one hydrogen atom from the thiol group in a compound represented by 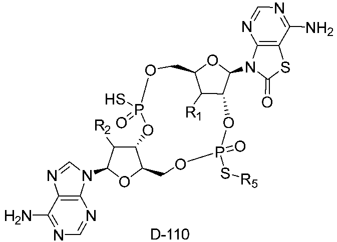 or

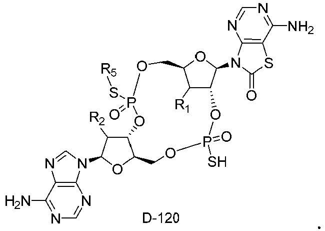

8. The immune-stimulating antibody conjugate represented by Formula II or the pharmaceutically acceptable salt thereof according to claim 1, wherein L is 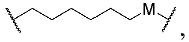 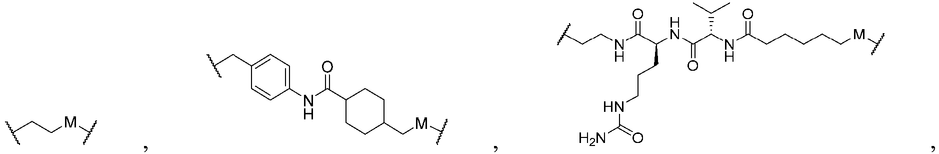 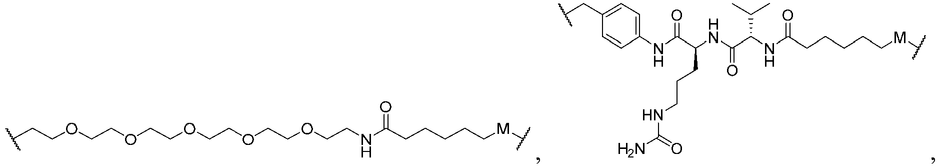 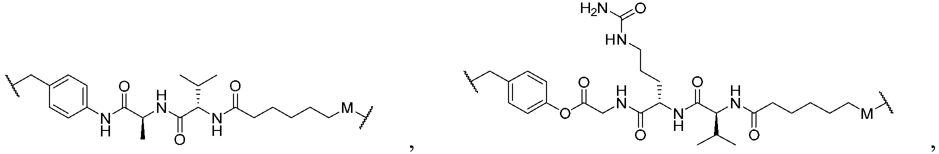 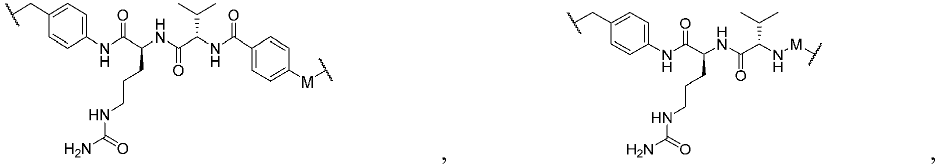 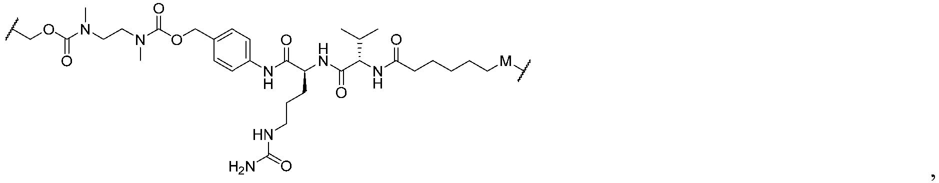 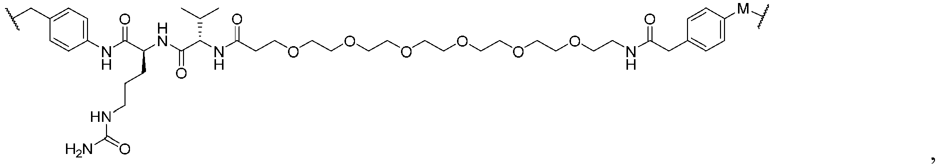 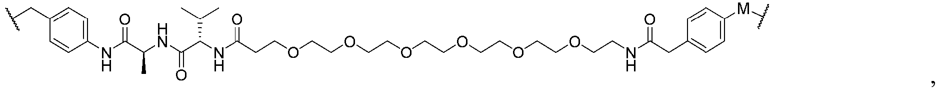 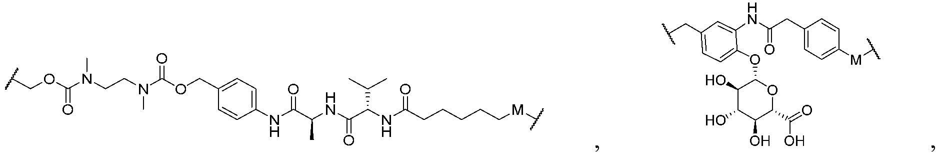 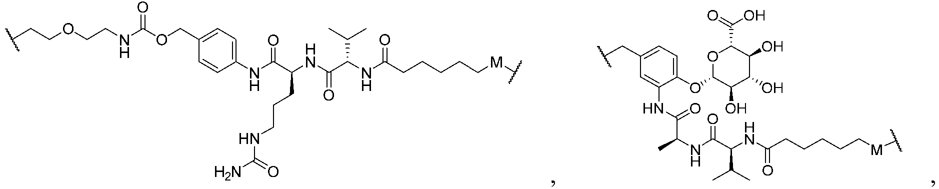 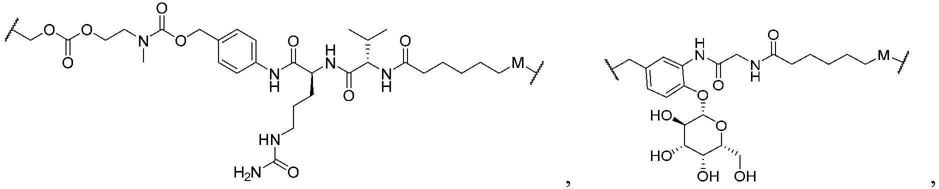 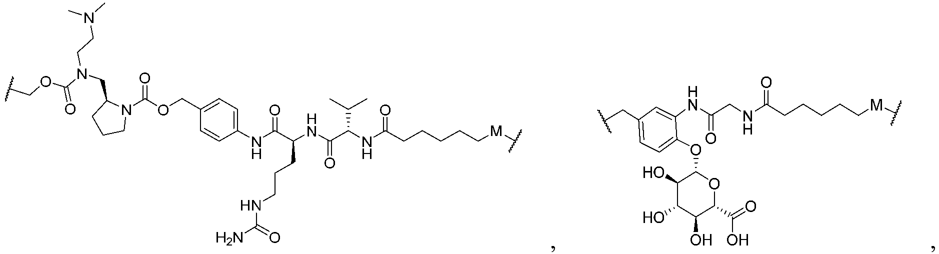 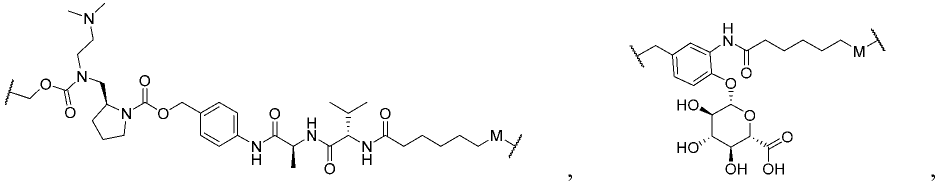 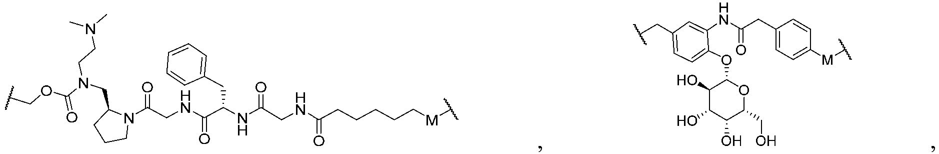 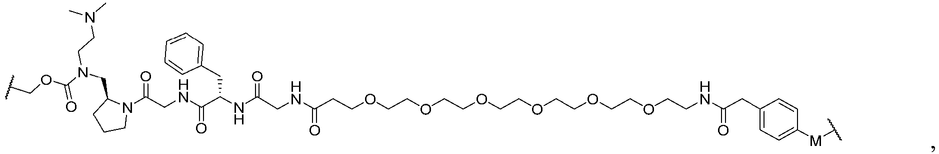 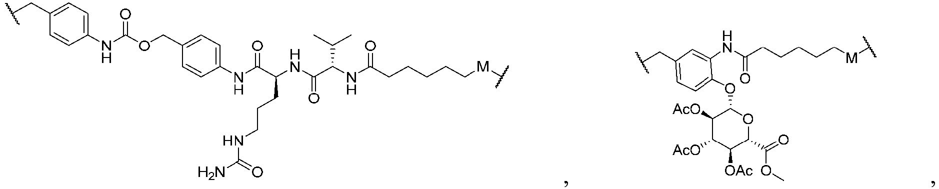 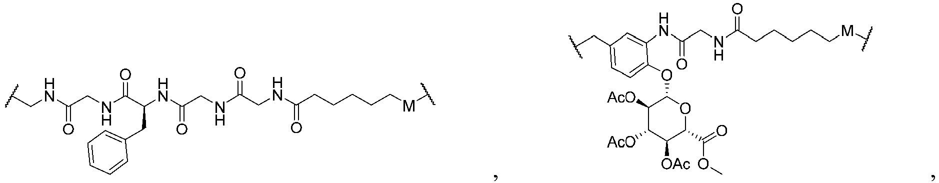 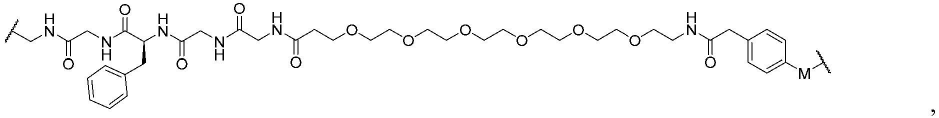 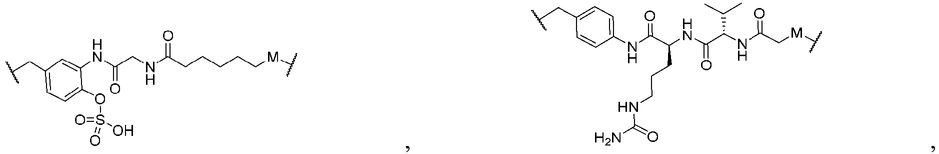 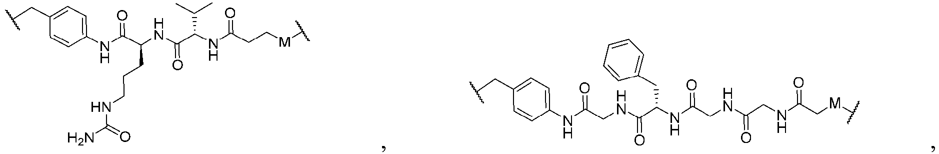 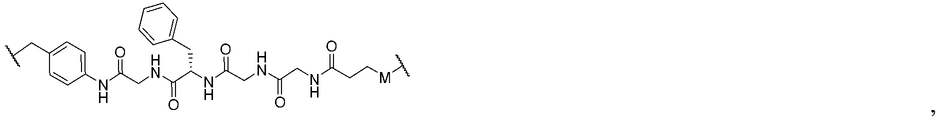 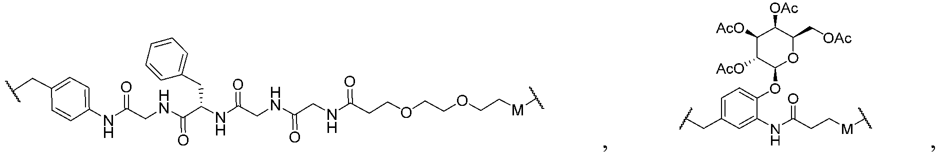 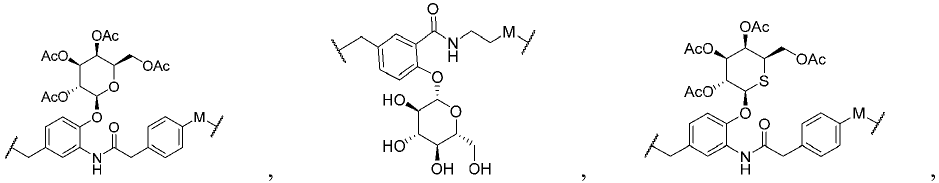 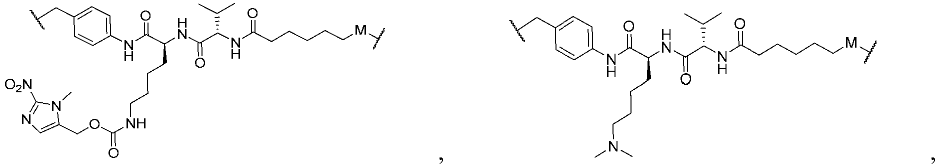 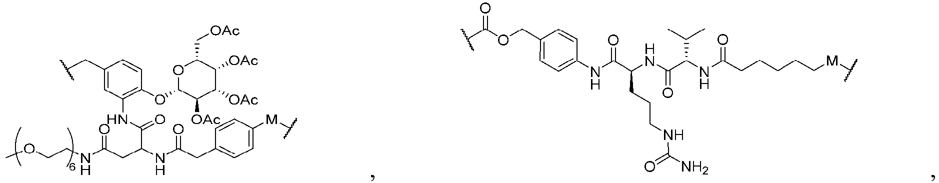 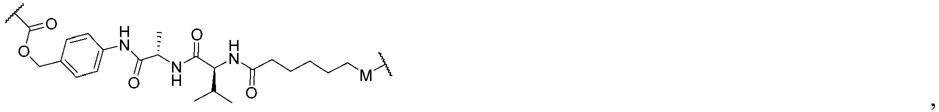 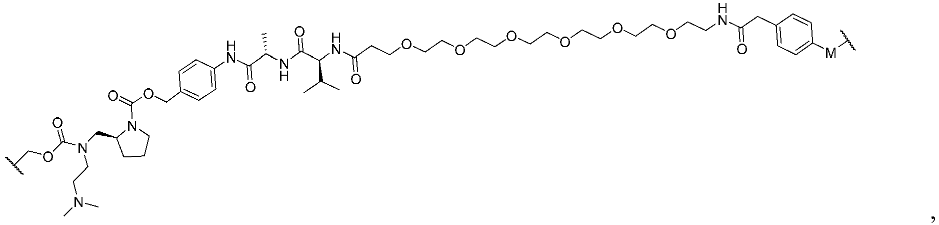 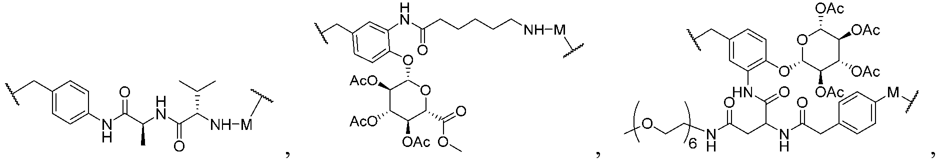 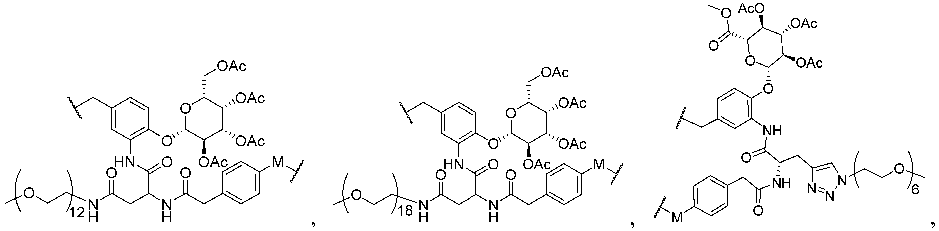 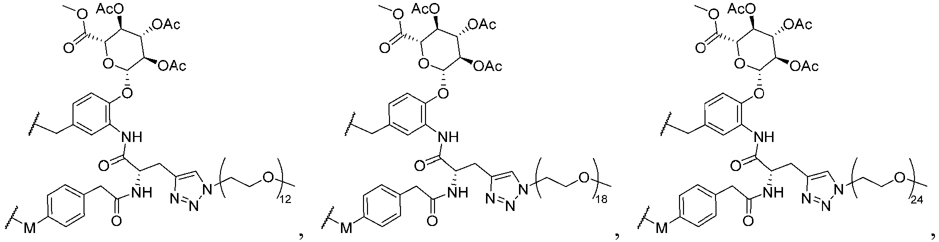 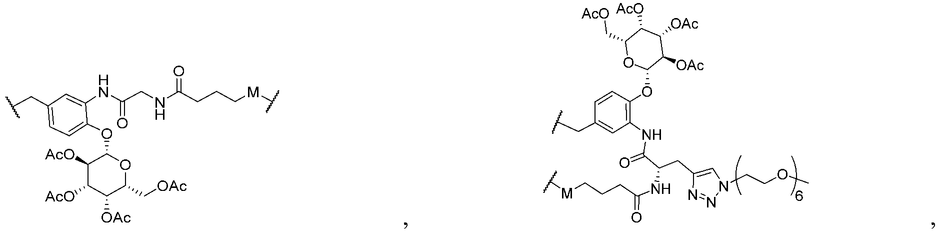 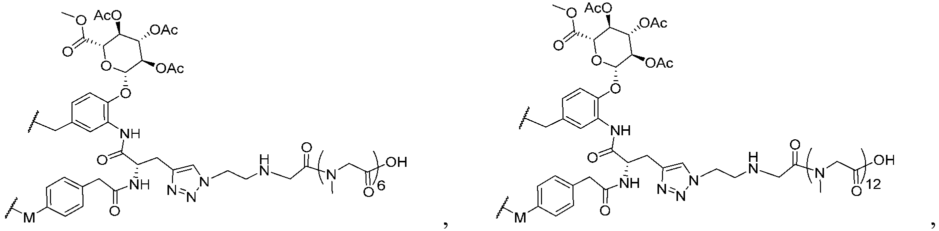 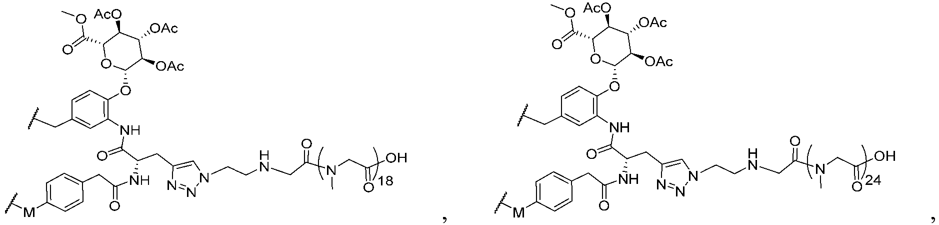 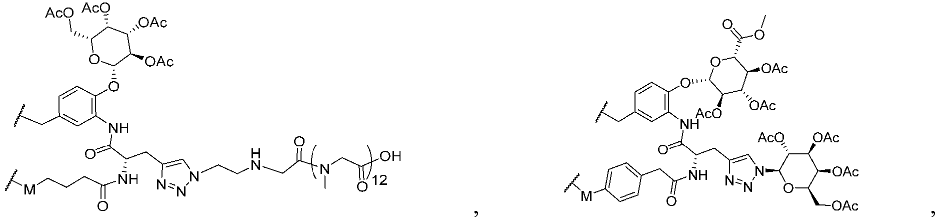 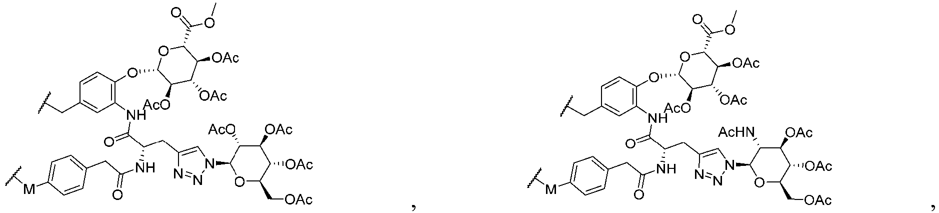 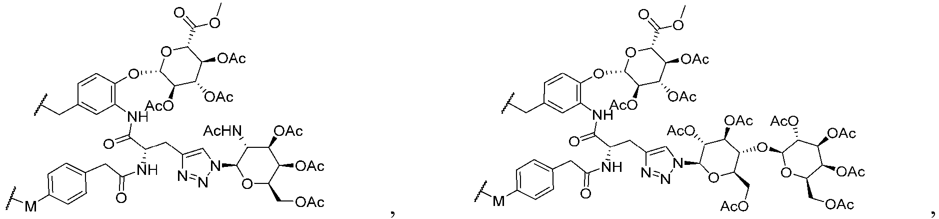 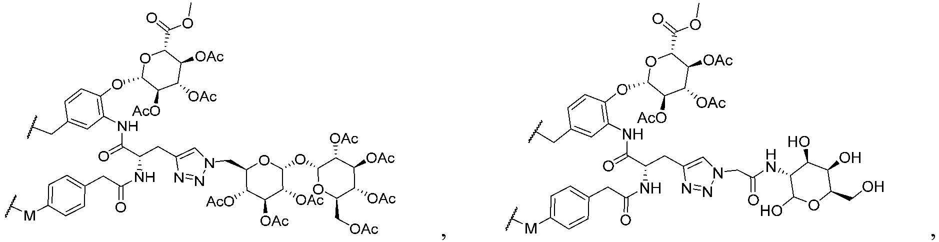 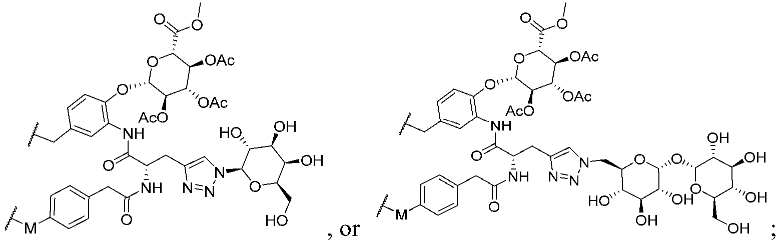 or, D is any one of the following structures: 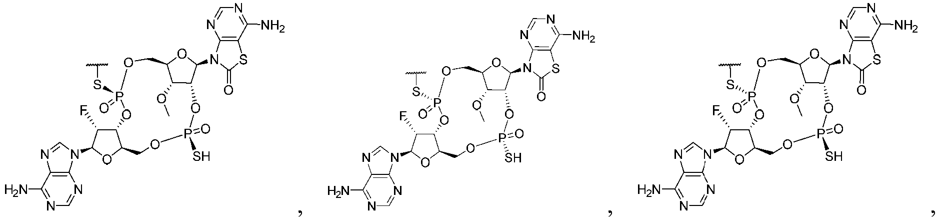 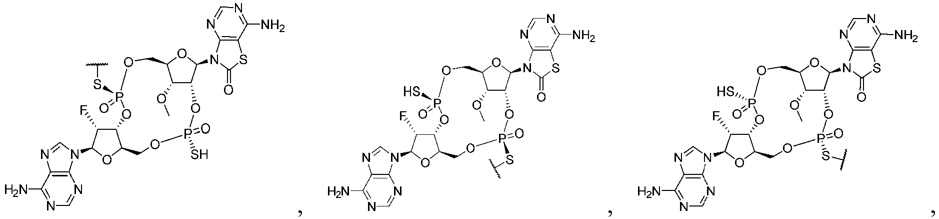 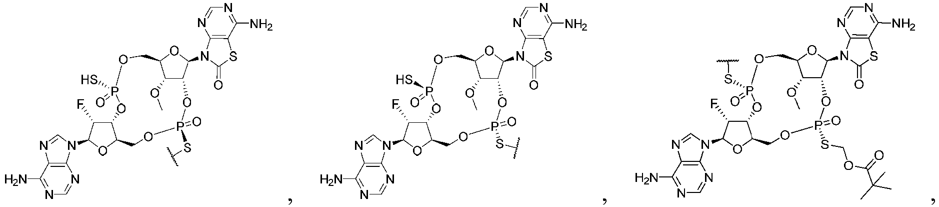 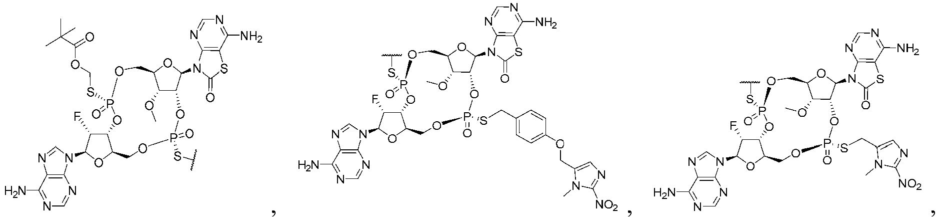 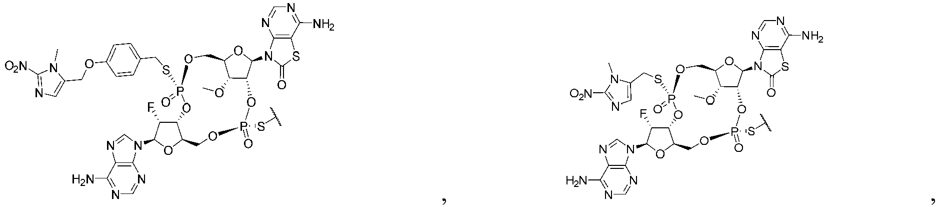 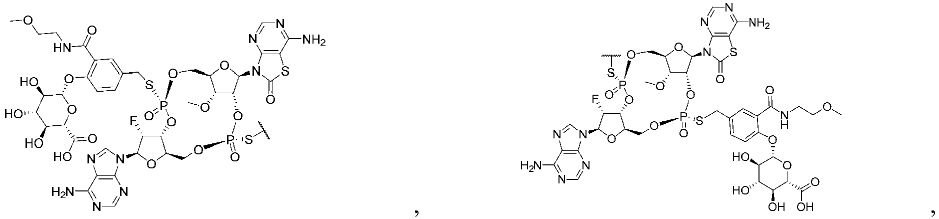 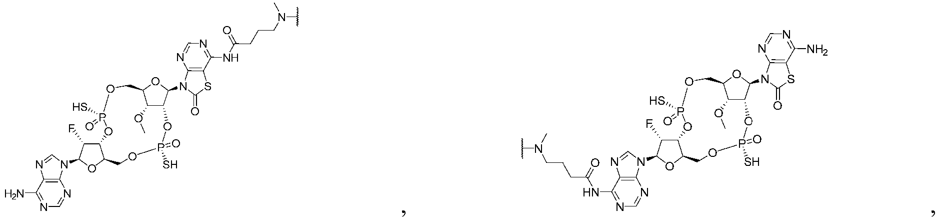 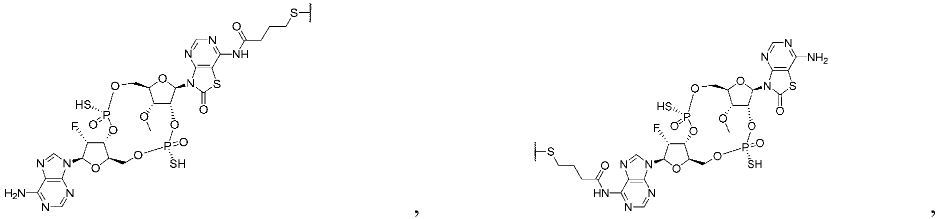 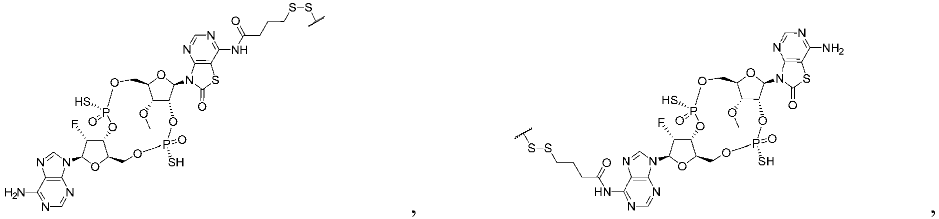 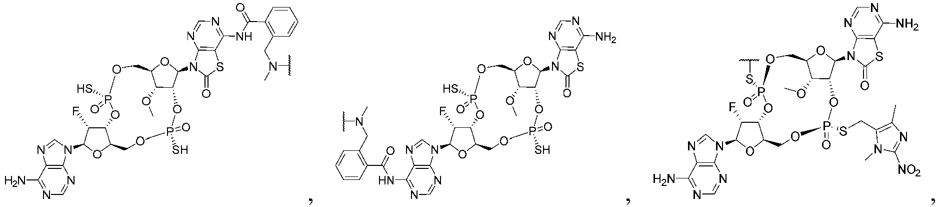 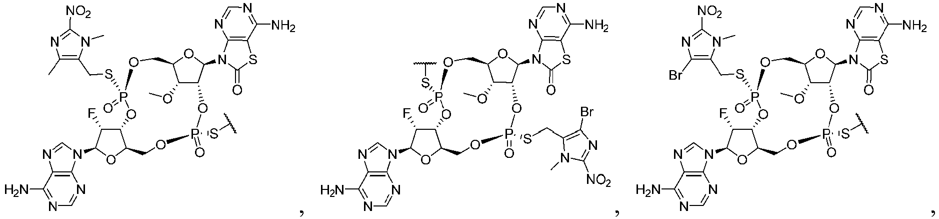 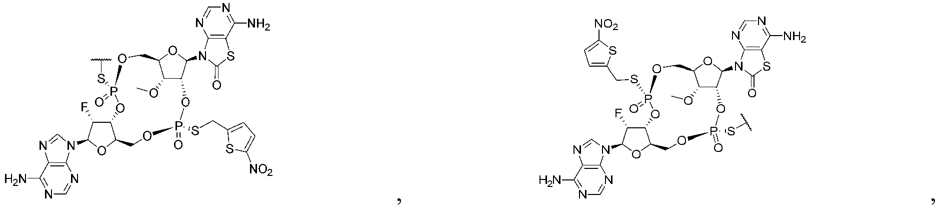 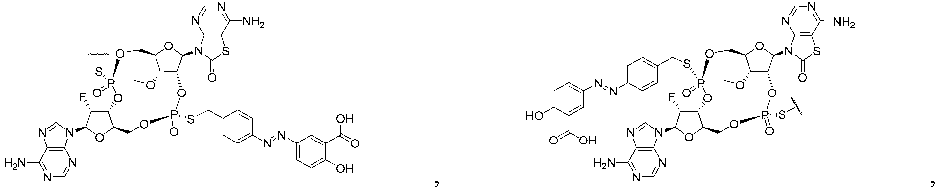 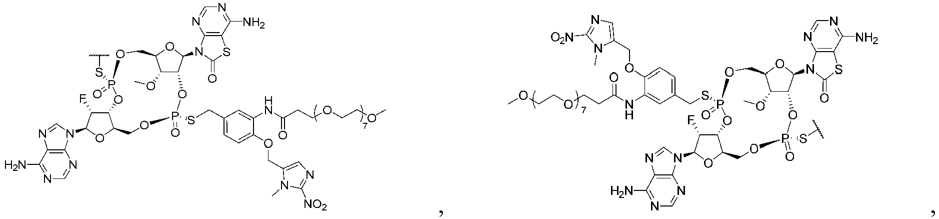 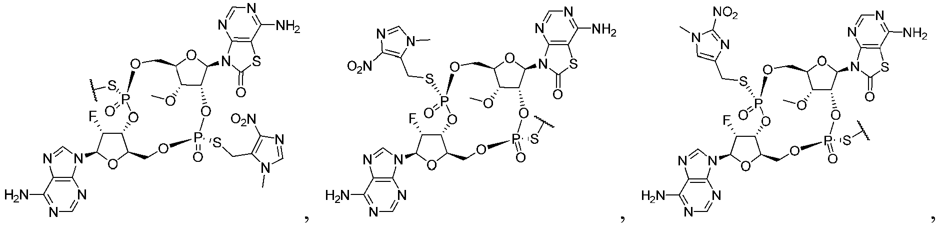 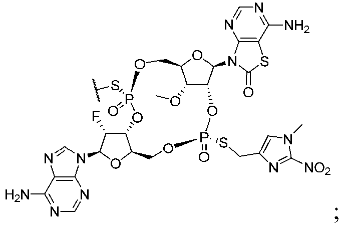 or a pharmaceutically acceptable salt thereof.

9. The immune-stimulating antibody conjugate represented by Formula II or the pharmaceutically acceptable salt thereof according to claim 1, wherein the immune-stimulating antibody conjugate represented by Formula II is any one of the immune-stimulating antibody conjugates listed in Table A.

10. A cyclic dinucleotide compound represented by Formula I or a pharmaceutically acceptable salt thereof,
D-LX (I)
wherein LX is a linker precursor with the following combination: -(L₁)ₐ-(Z)_{b}-M'; L₁ is connected to D;
M' is a linker head precursor;
L₁, Z, D, a, and b are as defined in any one of claims 1-9.

11. The cyclic dinucleotide compound represented by Formula I or the pharmaceutically acceptable salt thereof according to claim 10, wherein M' is a linker head precursor for reacting with the side-chain amino or thiol group of an antibody amino acid residue; preferably ethynyl, vinyl, hydroxyamino, 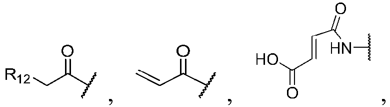 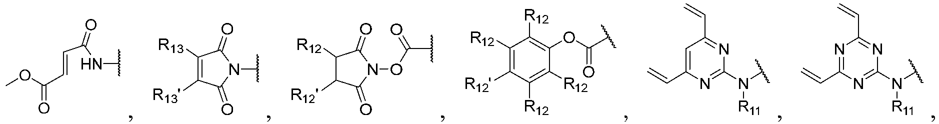 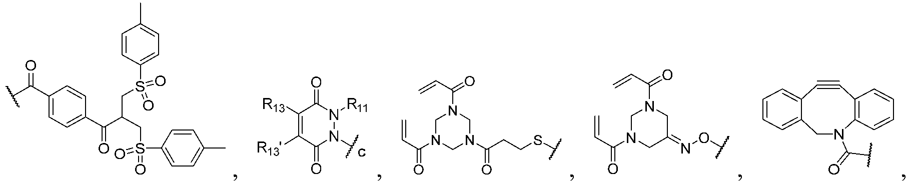 or 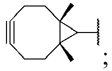
R₁₁ is hydrogen or C₁₋₄ alkyl;
each of R₁₂ and R_{12'} is independently halogen, nitro, or -SO₃⁻;
R₁₃ and R_{13'} are each independently hydrogen, halogen, phenylthio, or pyridylthio;
more preferably 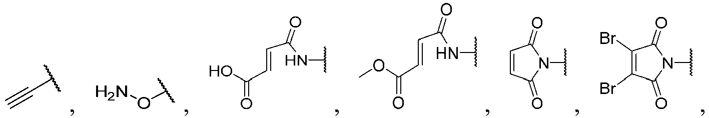 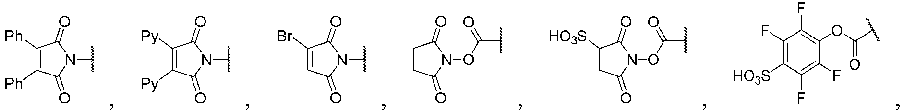 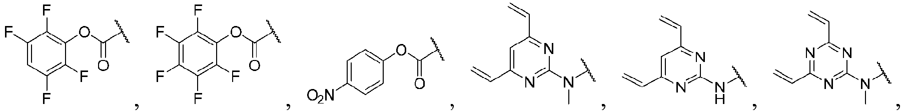 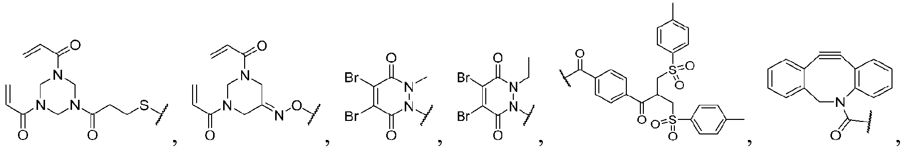 or

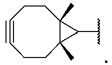

12. The cyclic dinucleotide compound represented by Formula I or the pharmaceutically acceptable salt thereof according to claim 10, wherein LX is

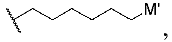

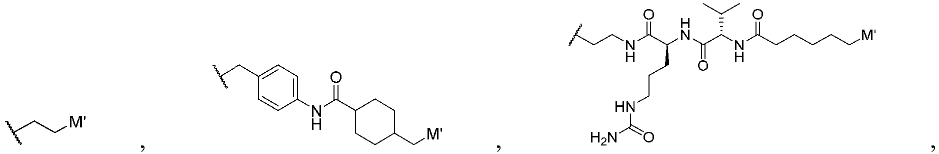

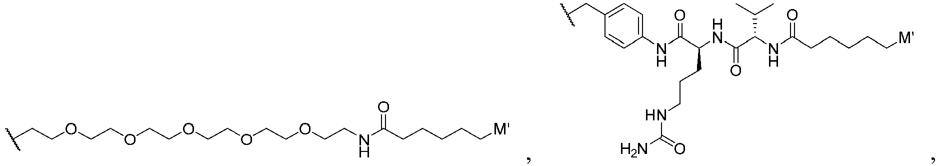

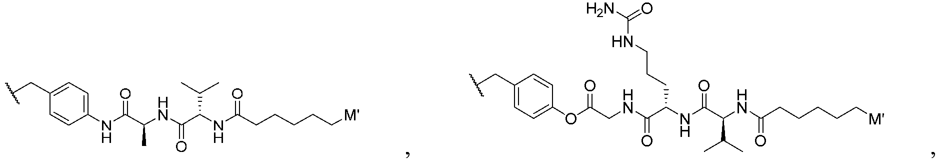

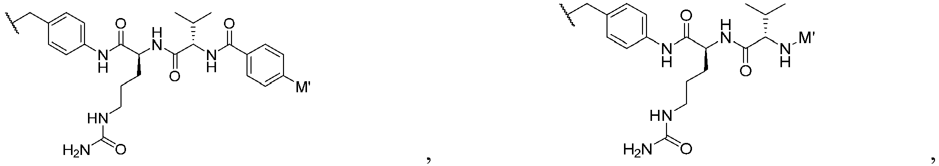

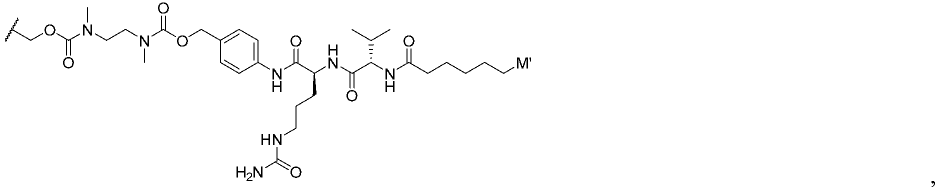

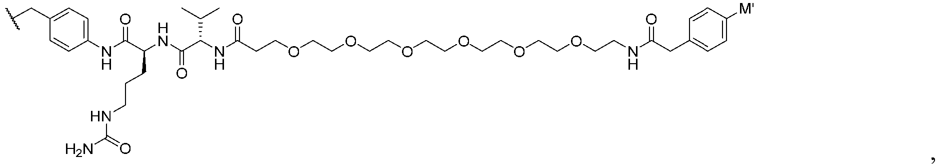

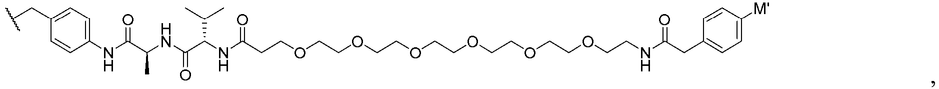

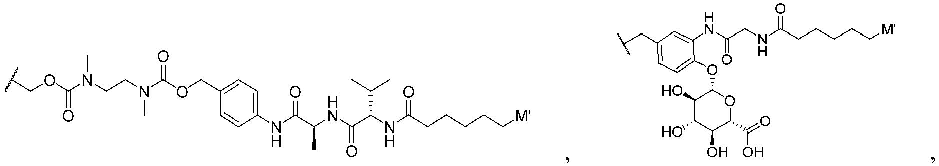

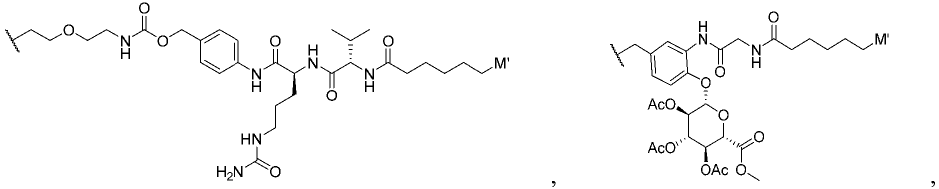

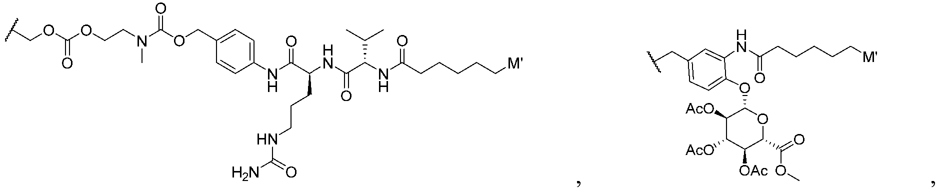

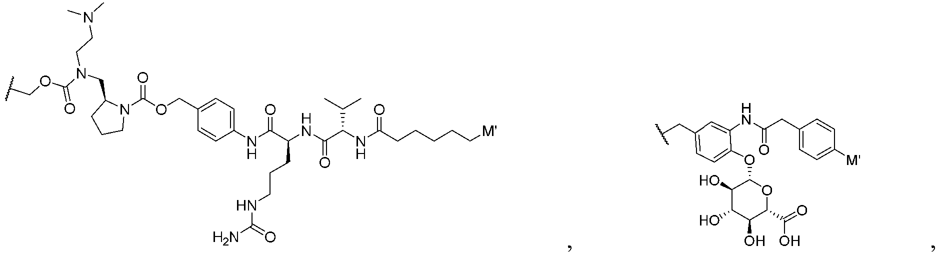

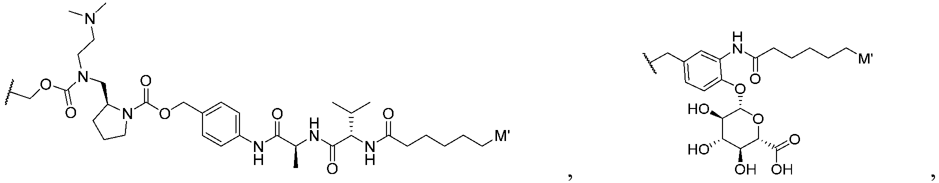

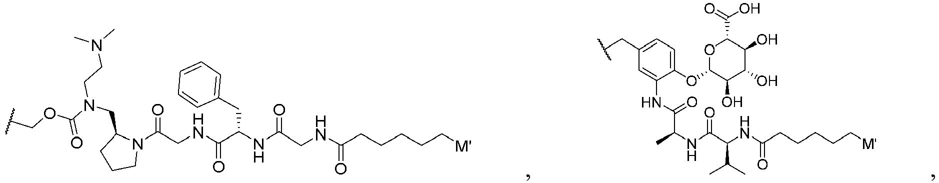

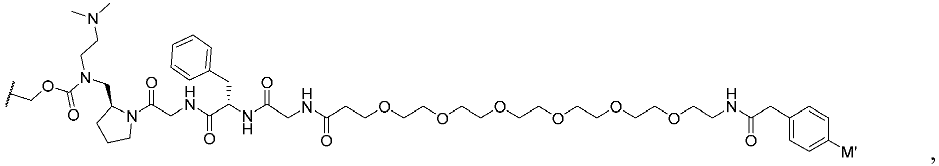

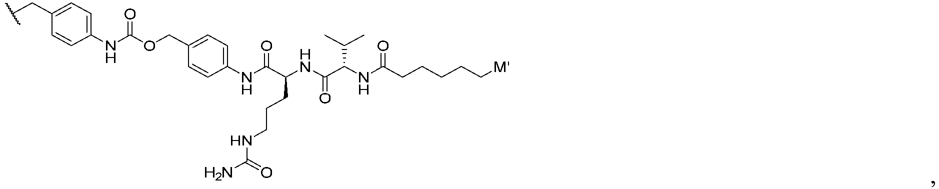

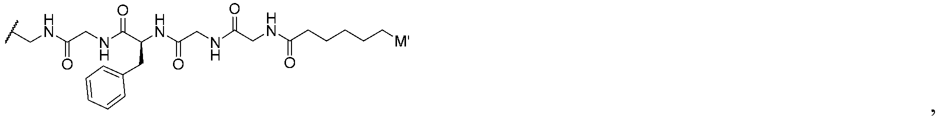

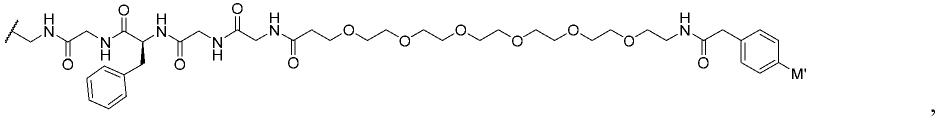

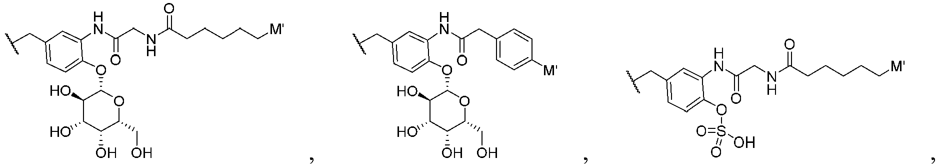

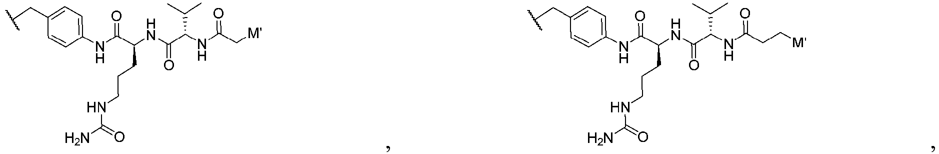

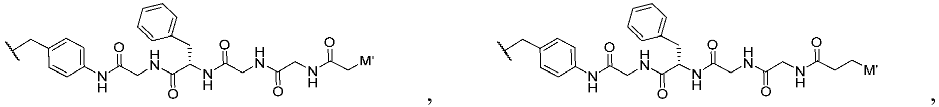

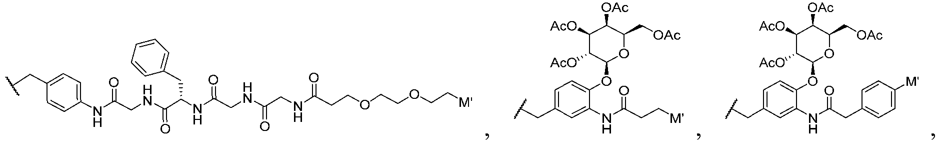

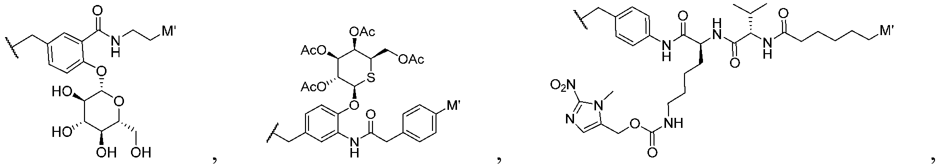

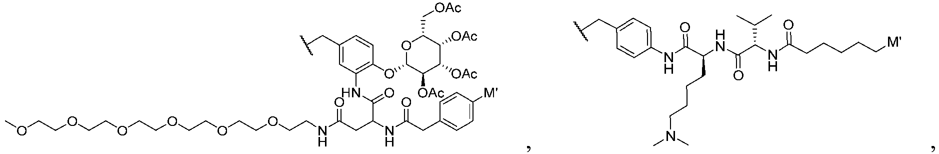

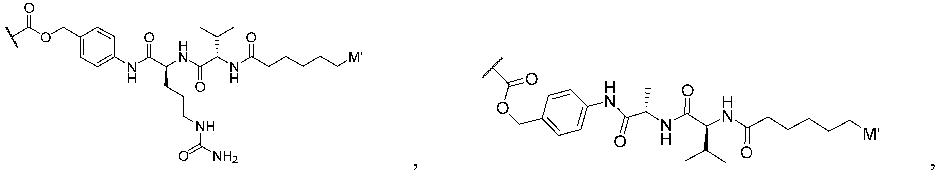

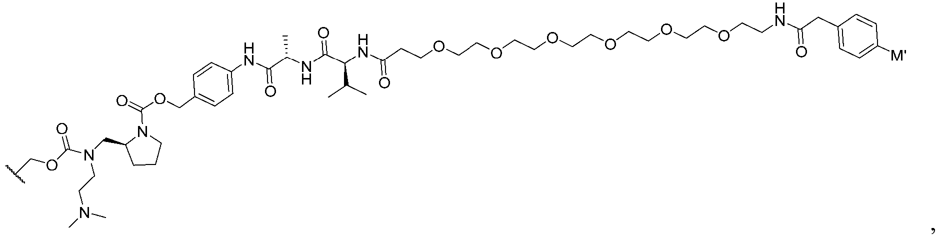

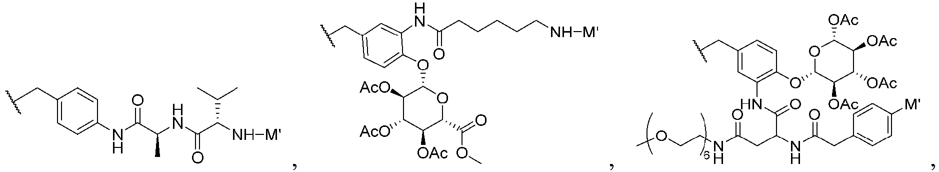

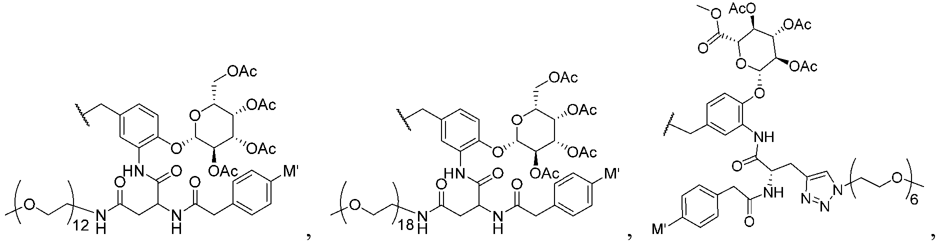

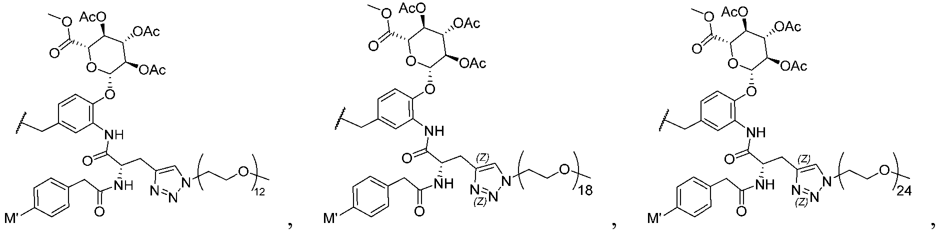

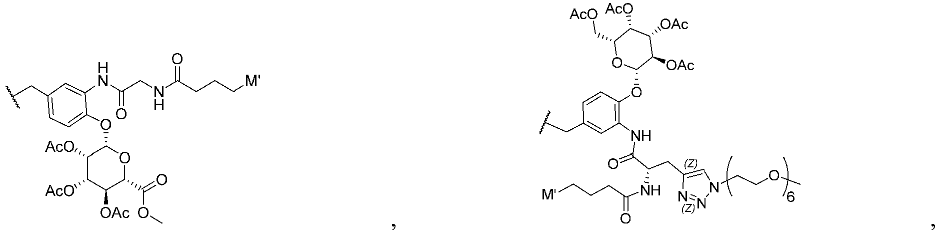

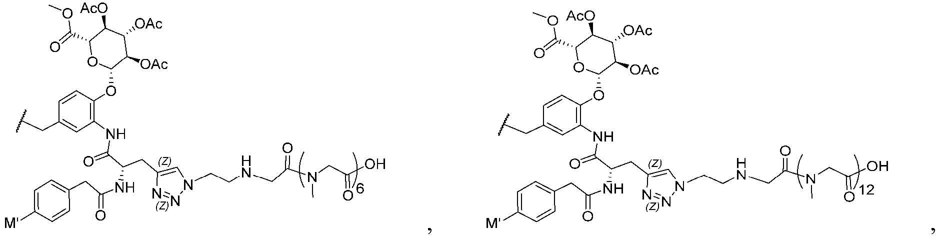

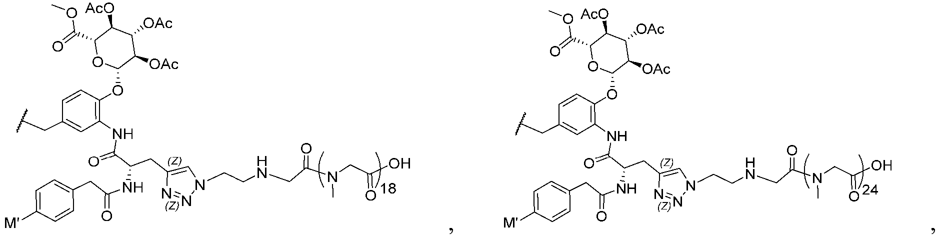

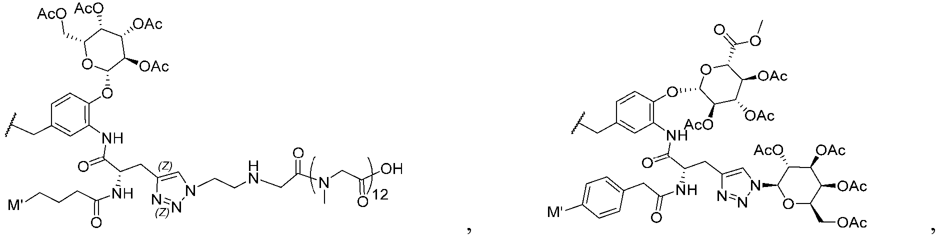

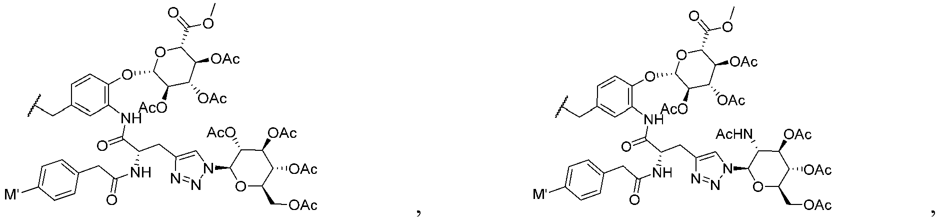

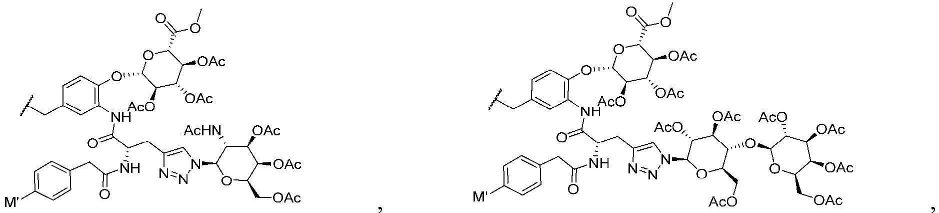

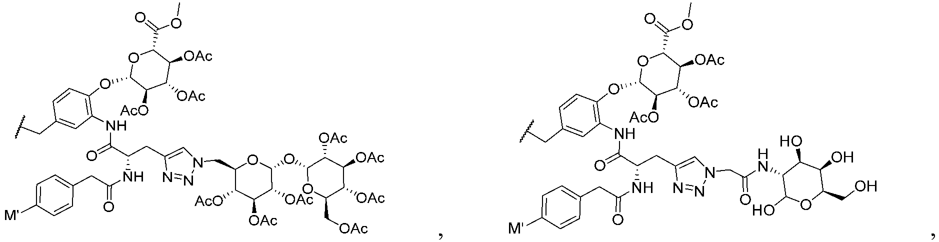

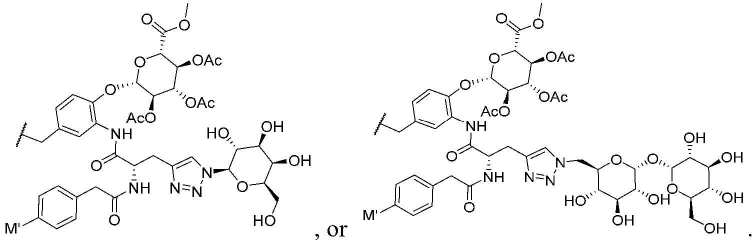

13. The cyclic dinucleotide compound represented by Formula I or the pharmaceutically acceptable salt thereof according to any one of claims 10-12, wherein the cyclic dinucleotide compound represented by Formula I is 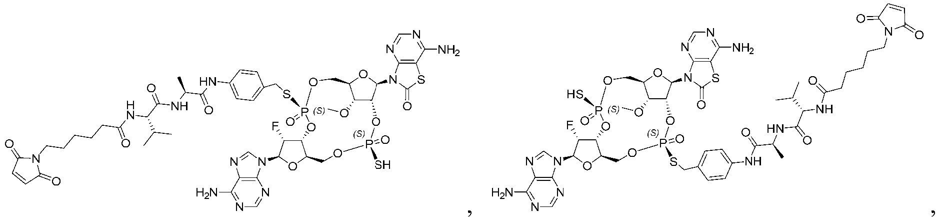 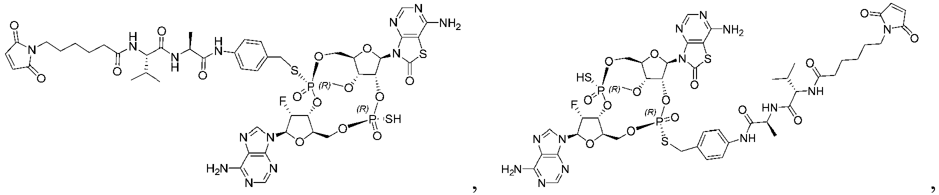 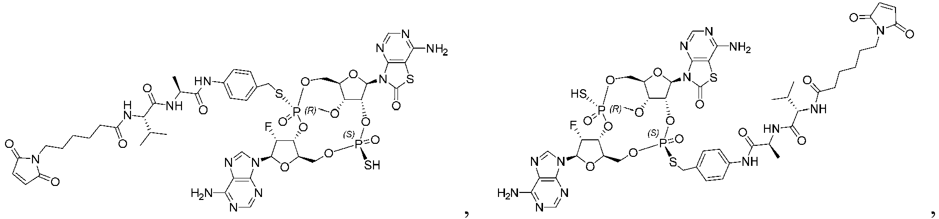 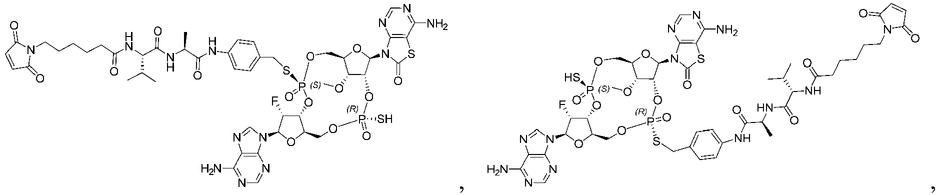 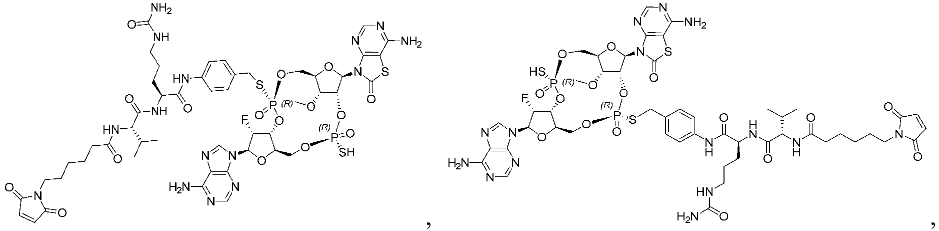 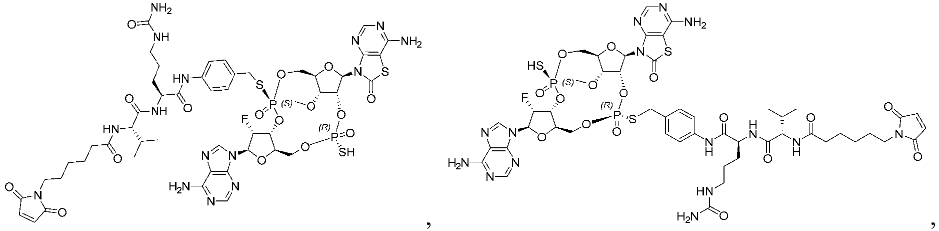 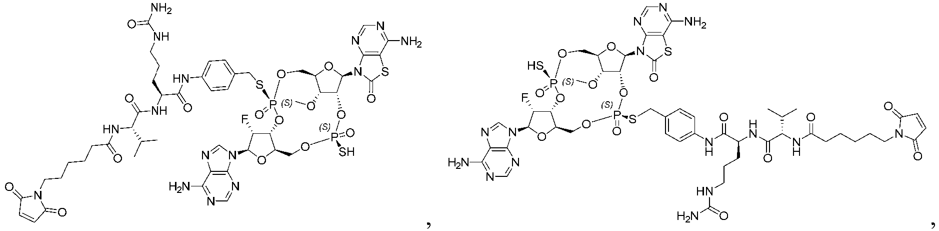 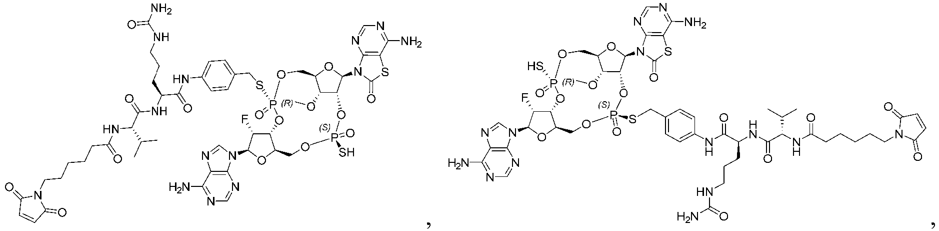 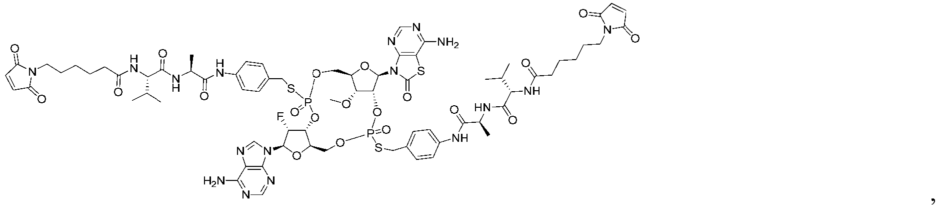 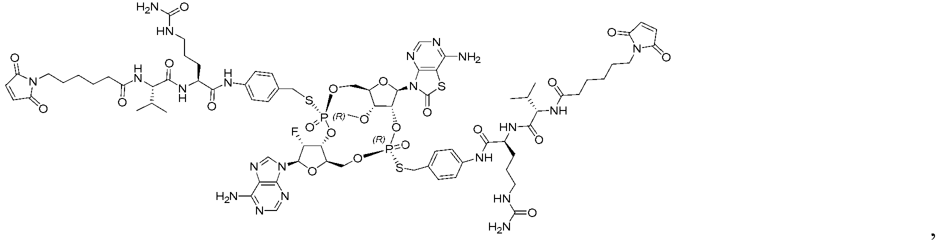 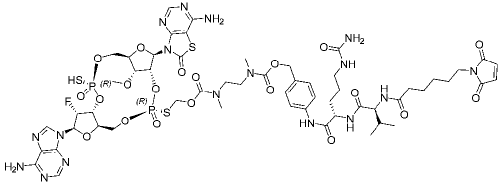 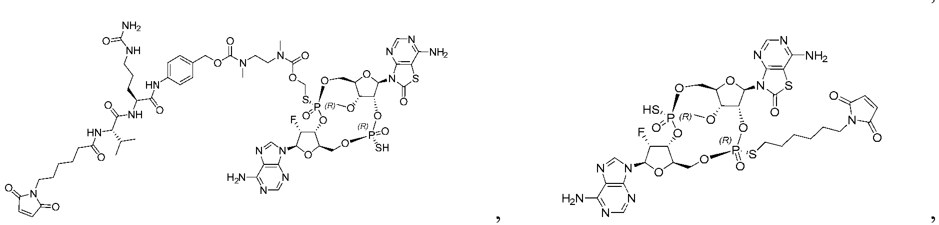 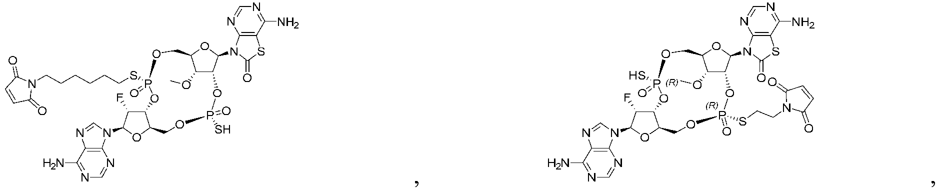 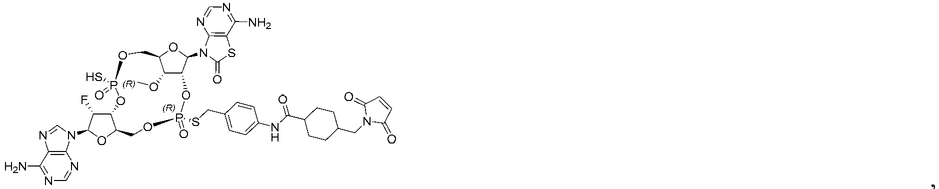 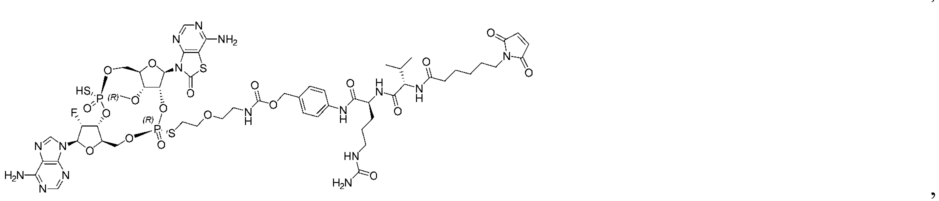 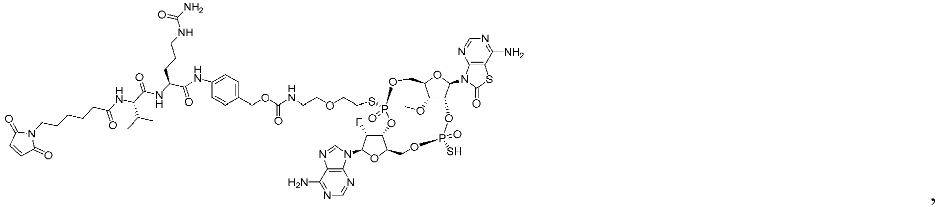 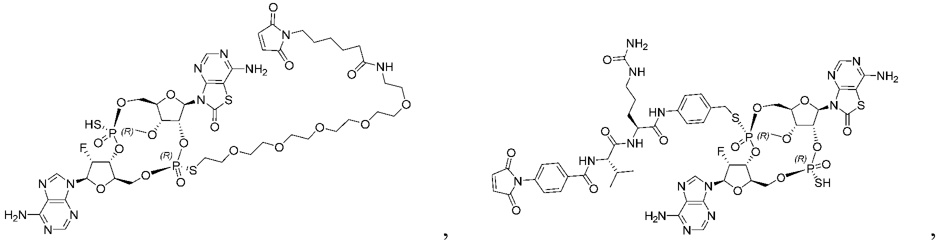 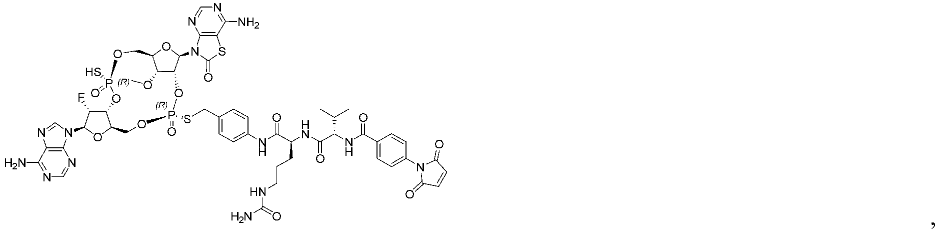 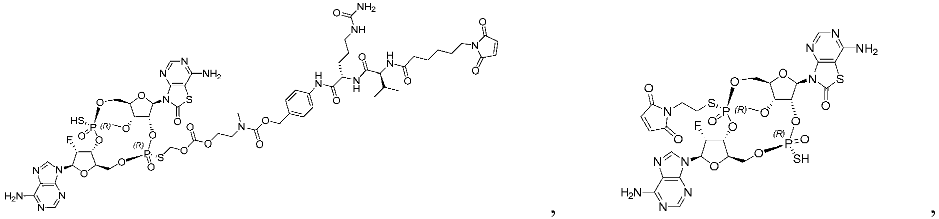 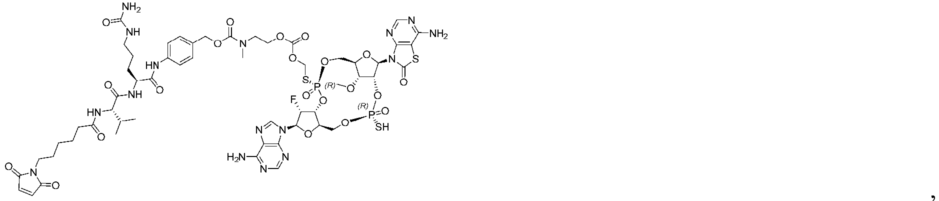 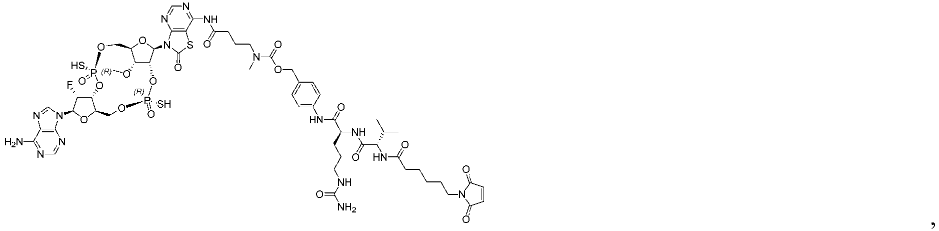 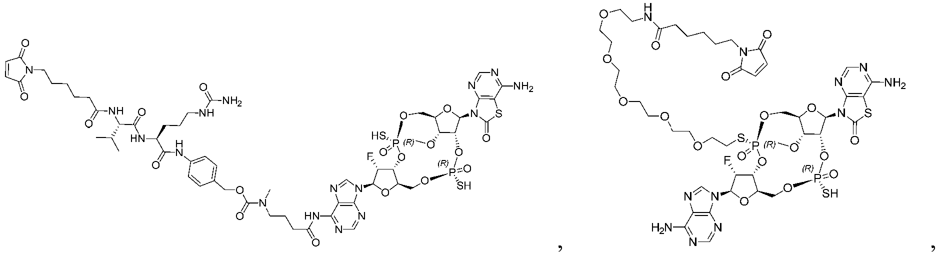 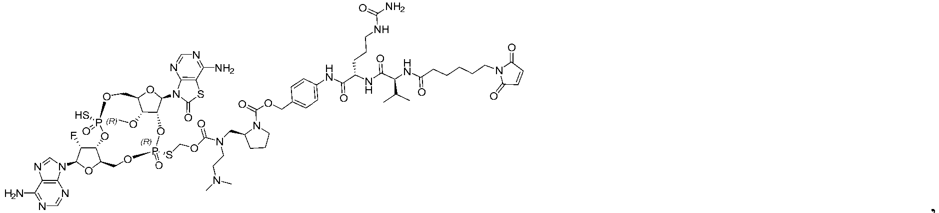 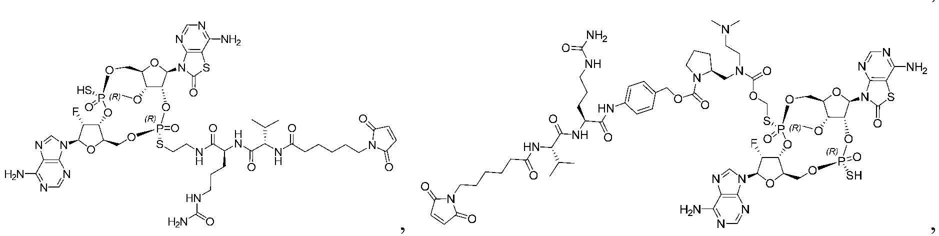 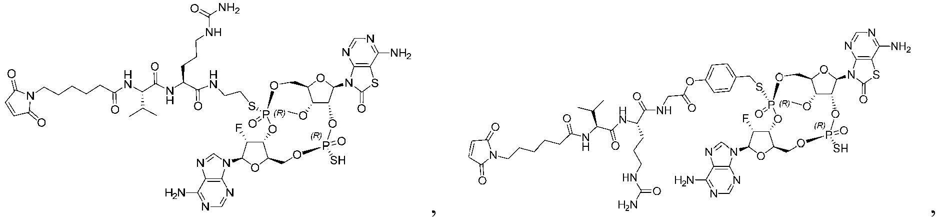 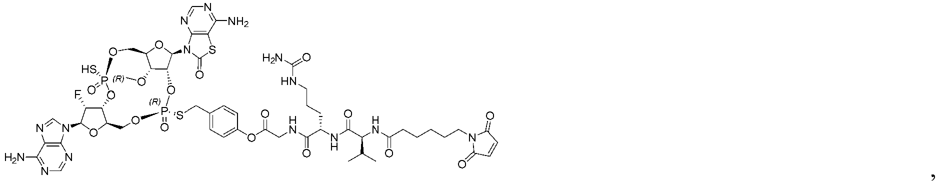 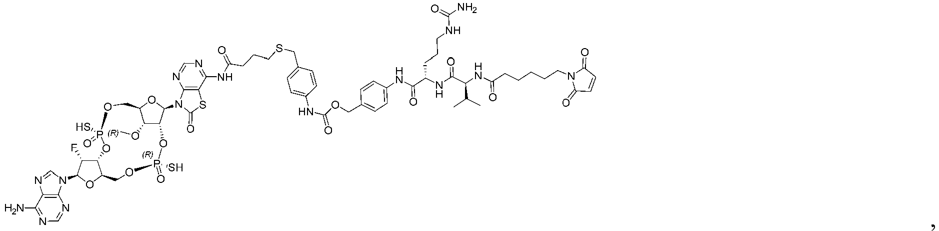 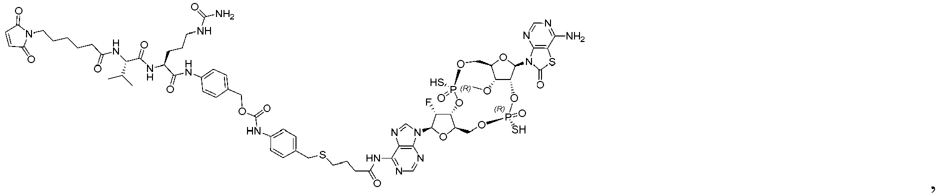 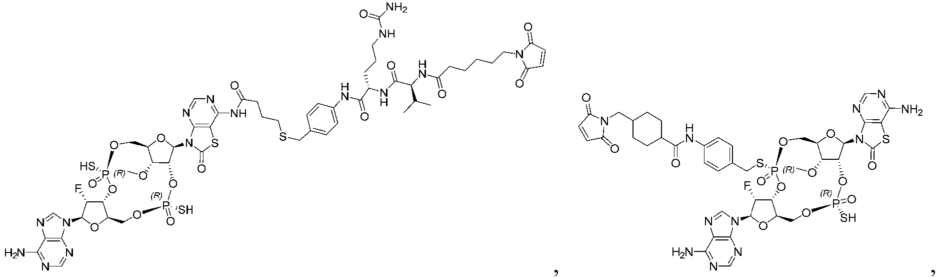 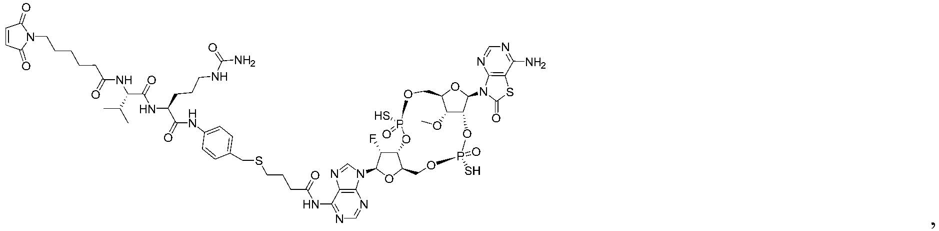 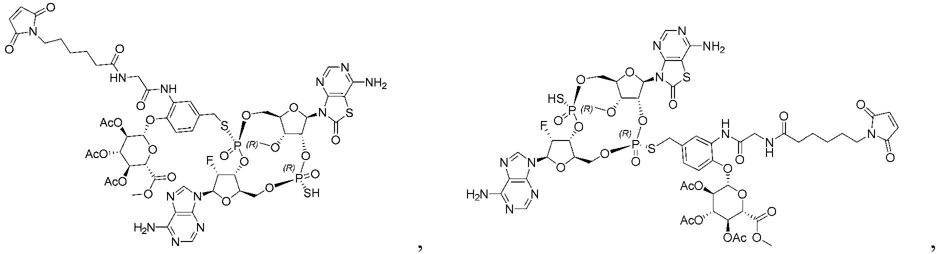 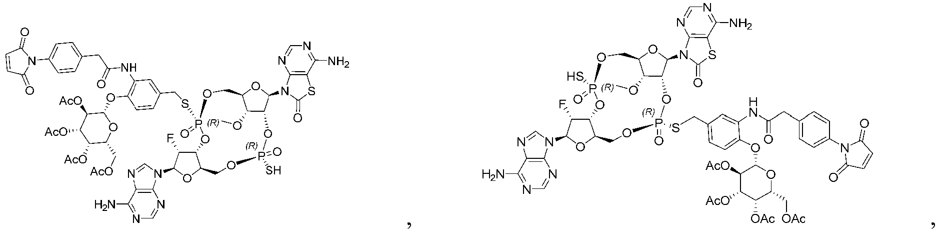 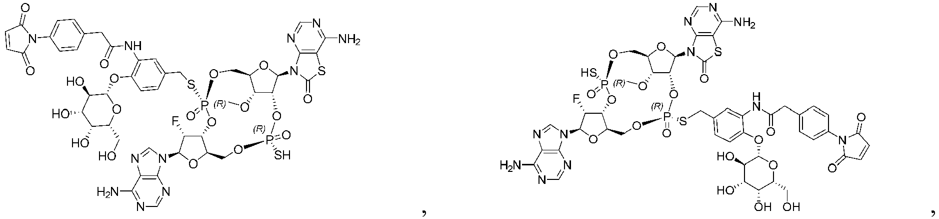 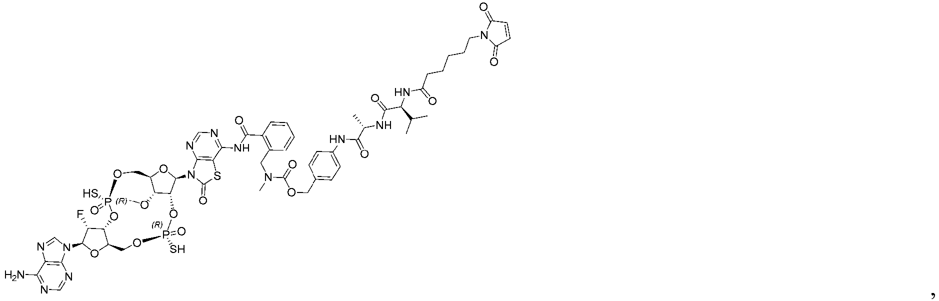 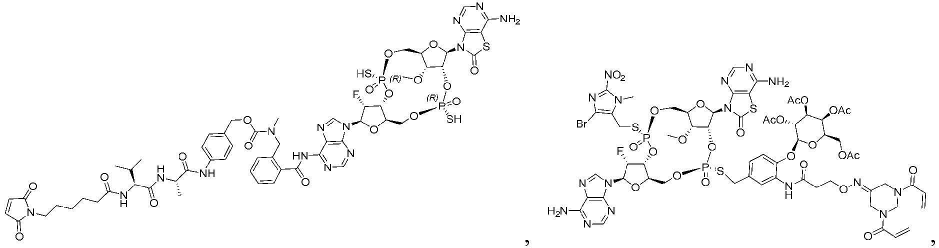 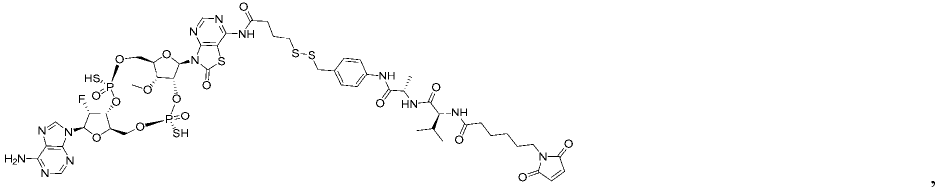 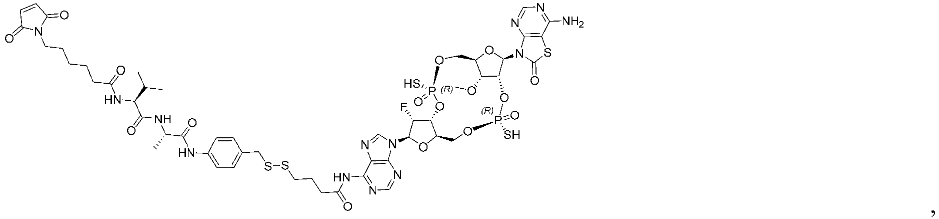 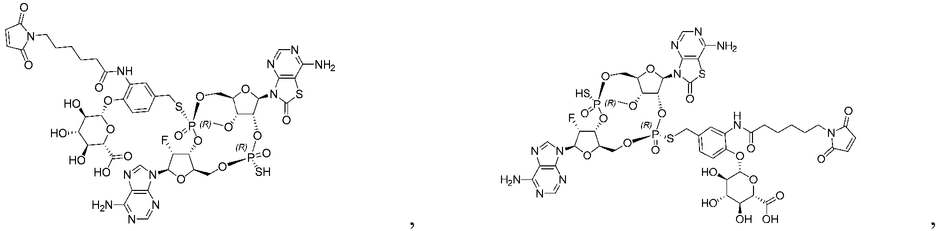 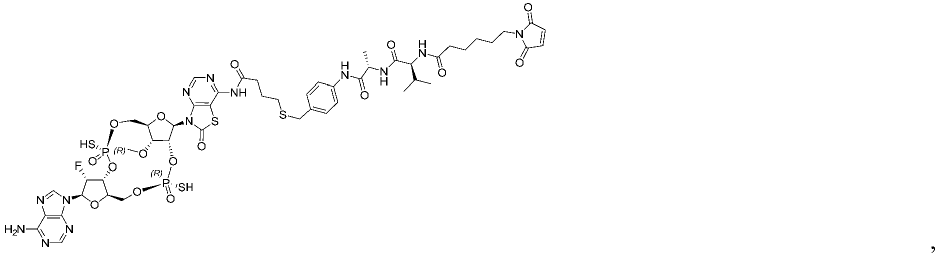 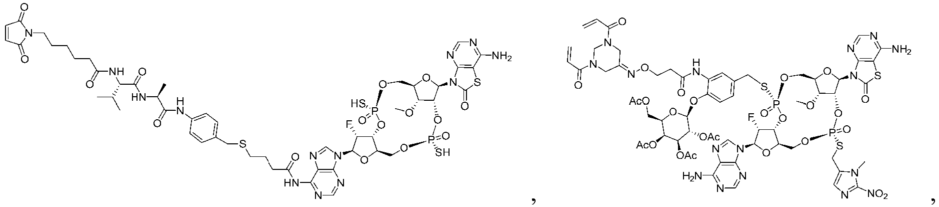 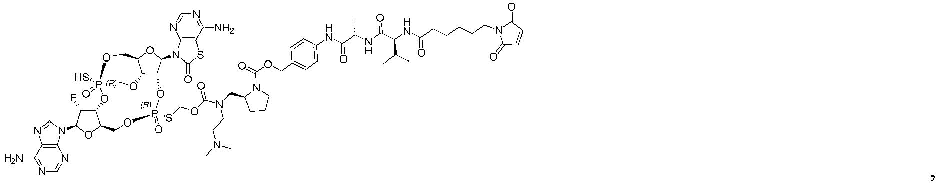 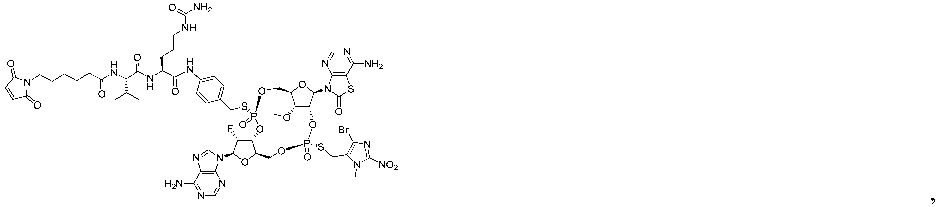 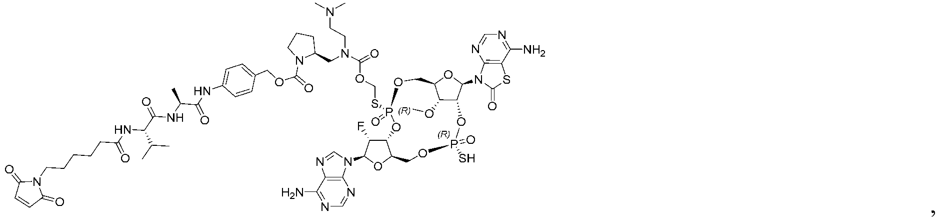 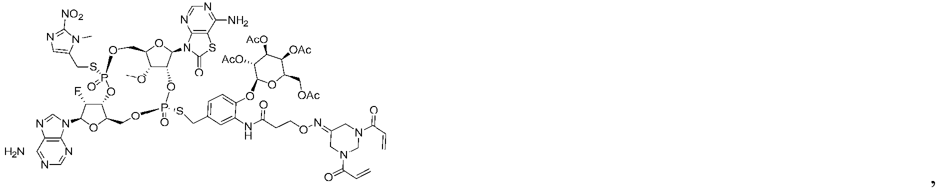 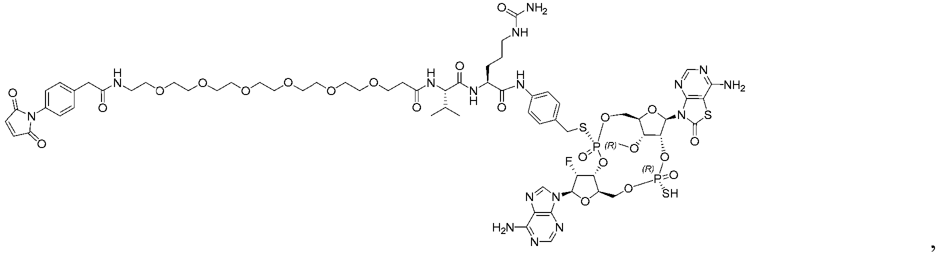 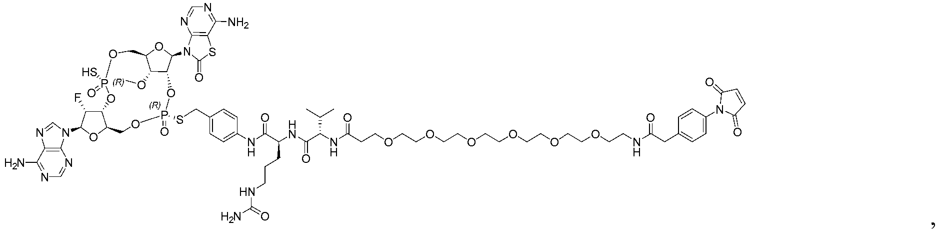 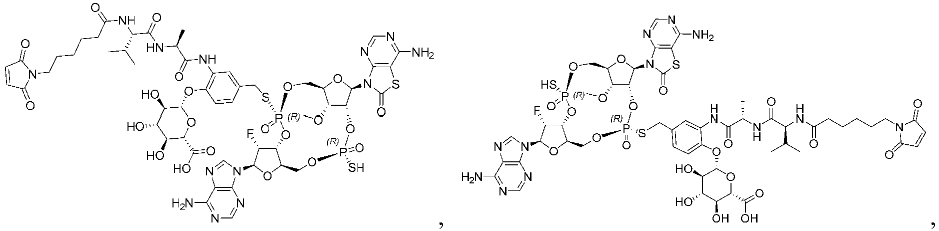 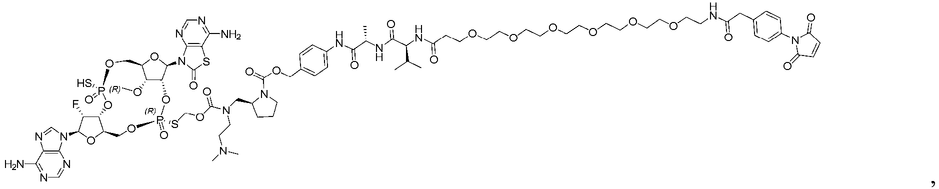 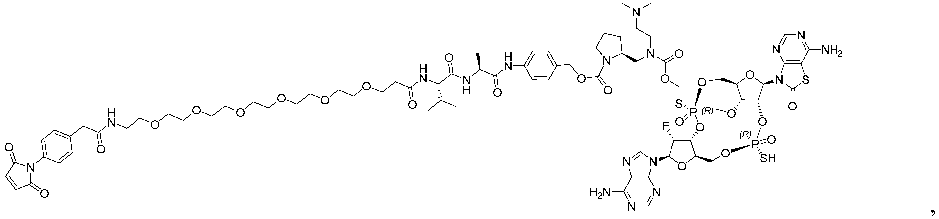 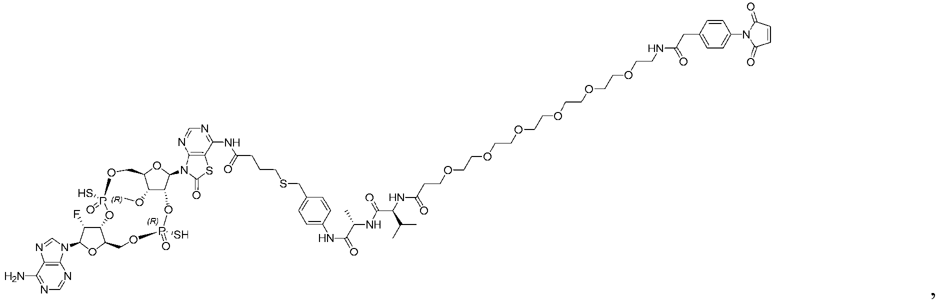 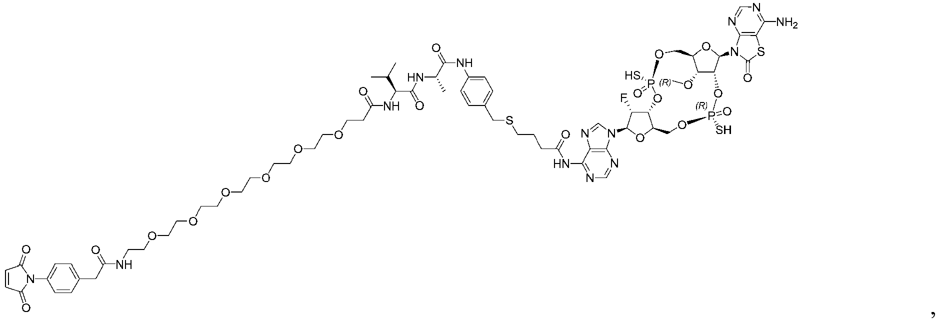 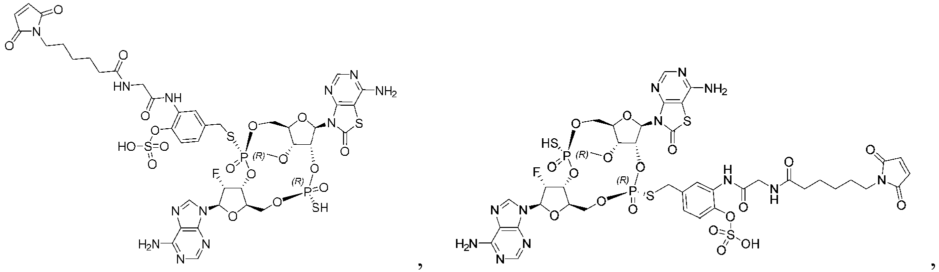 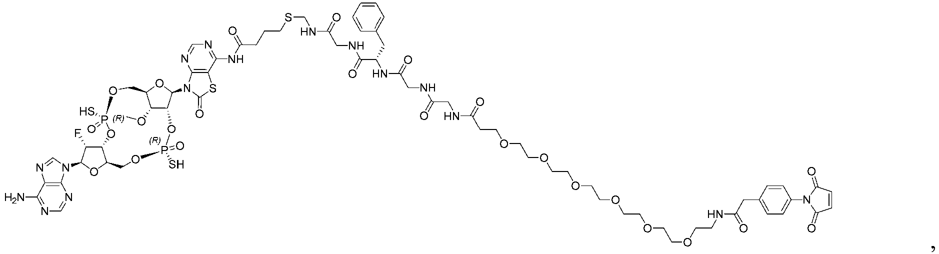 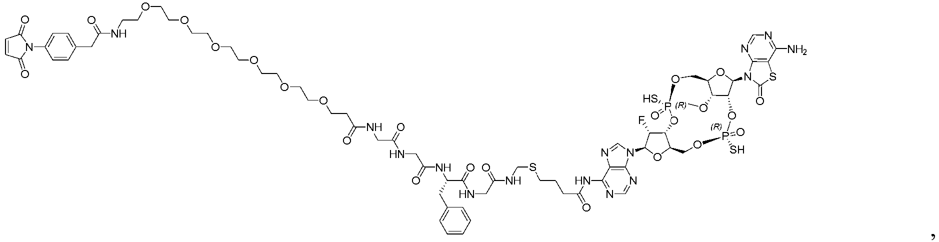 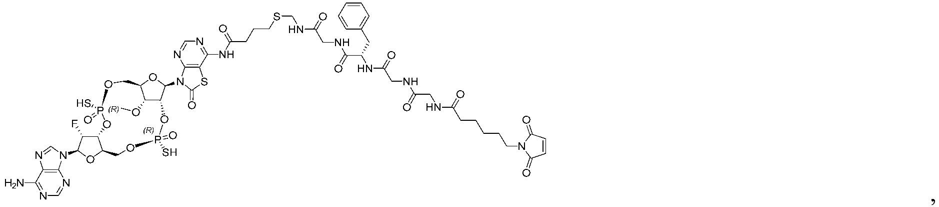 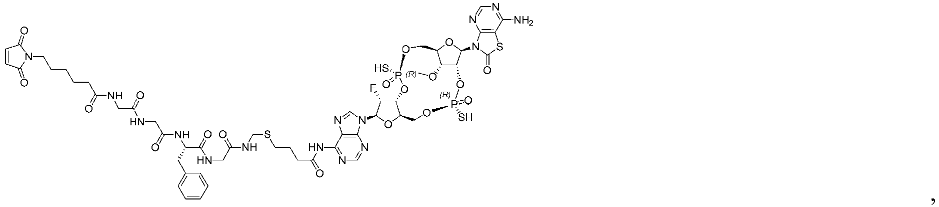 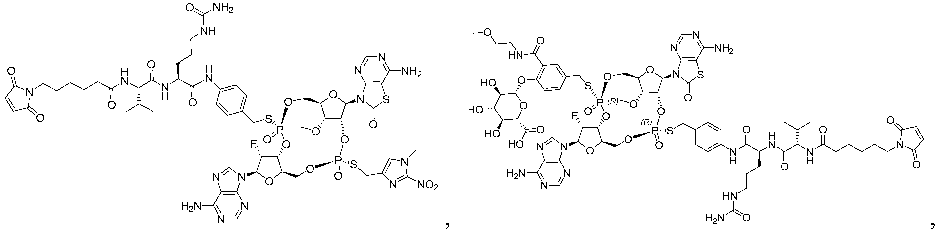 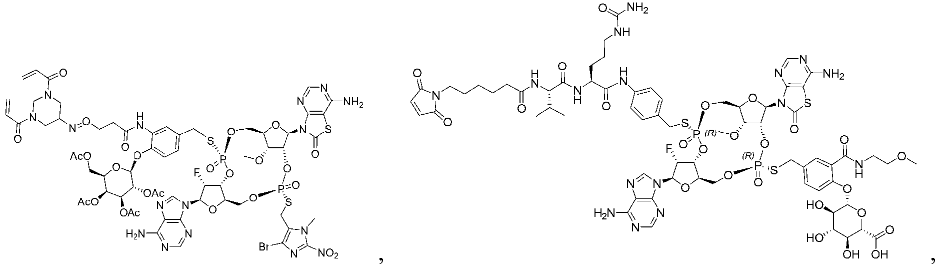 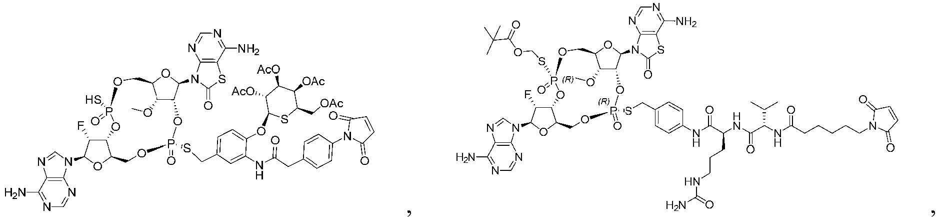 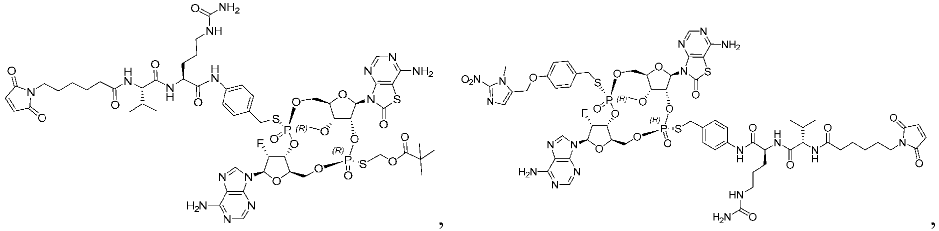 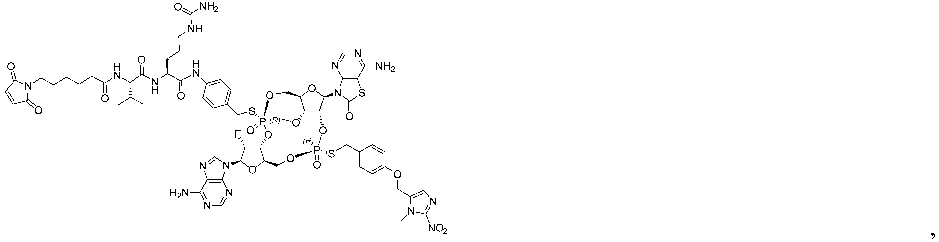 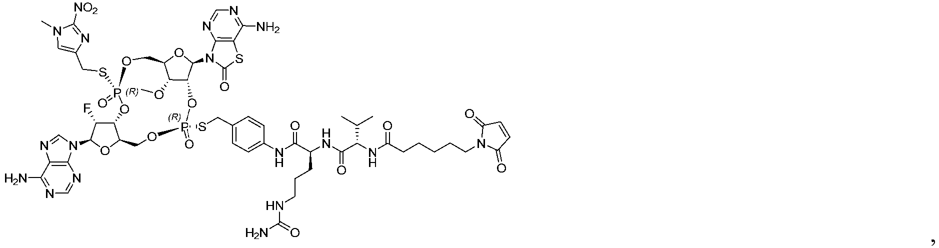 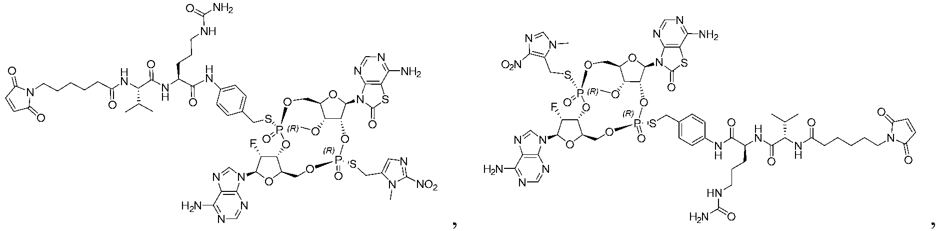 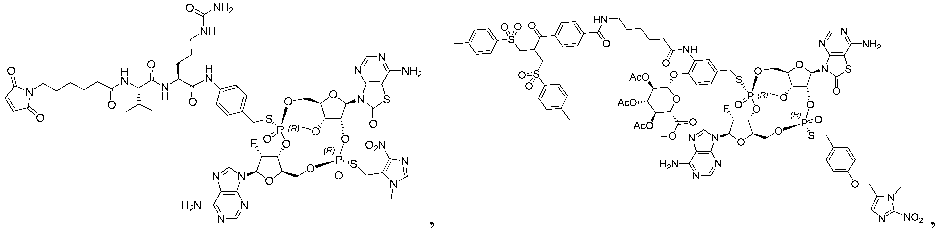 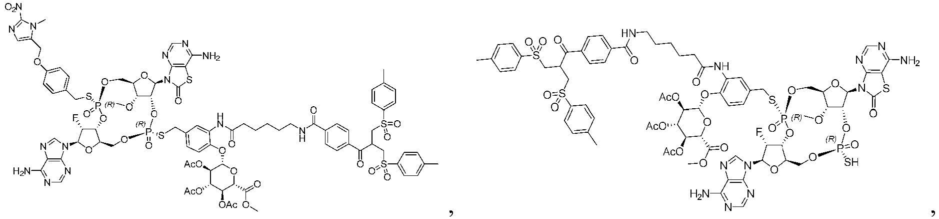 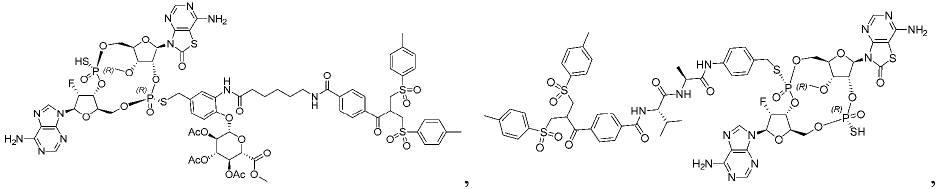 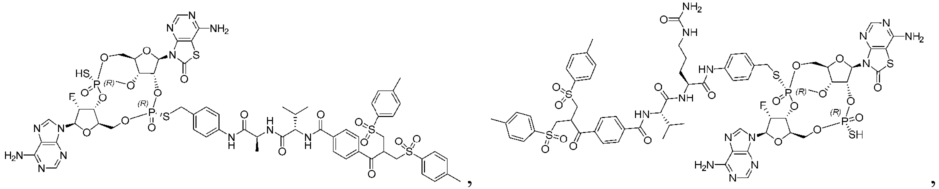 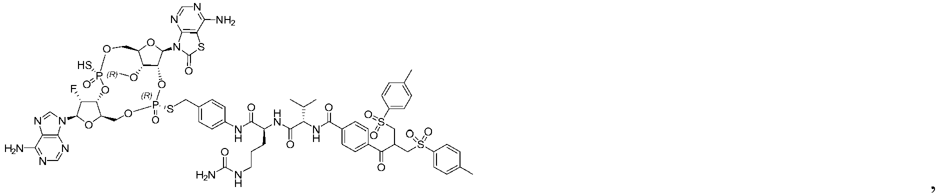 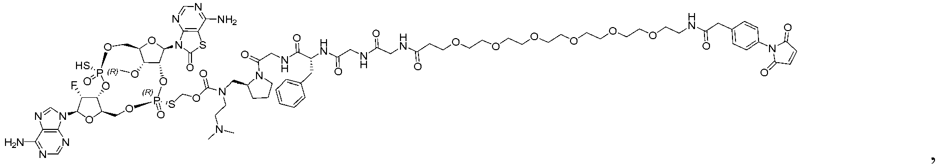 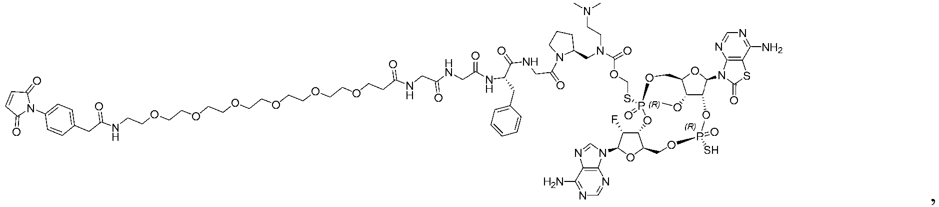 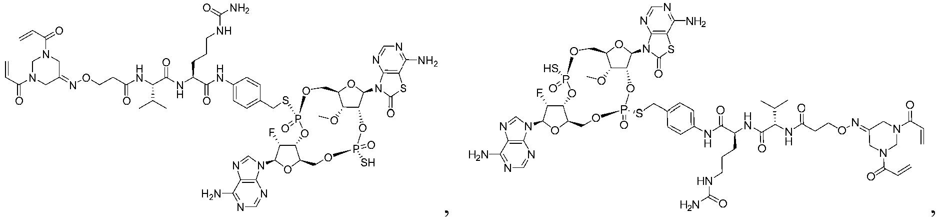 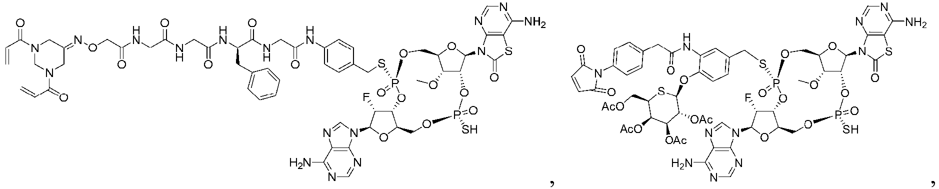 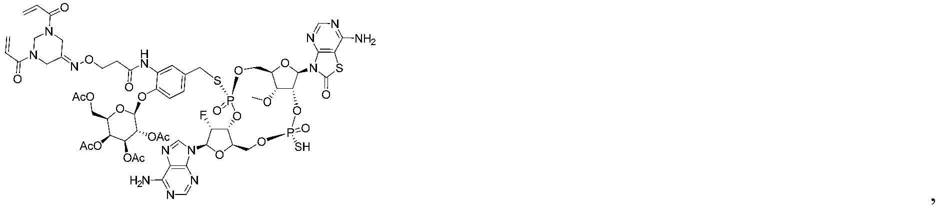 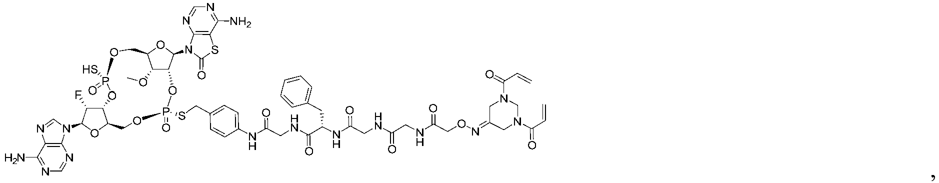 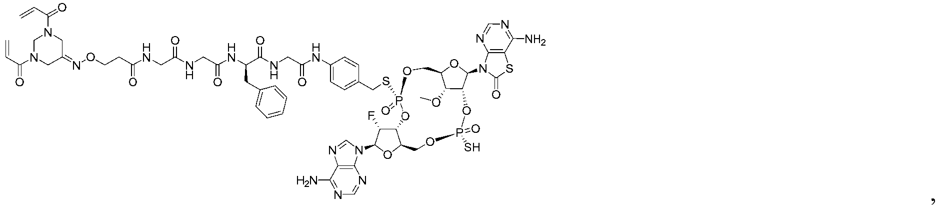 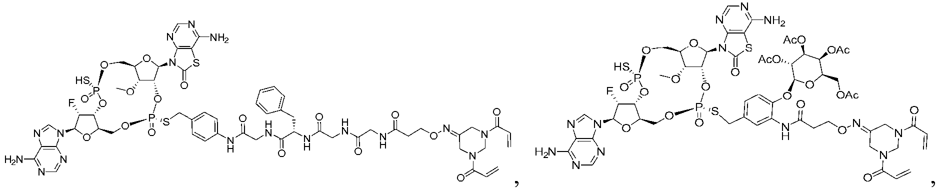 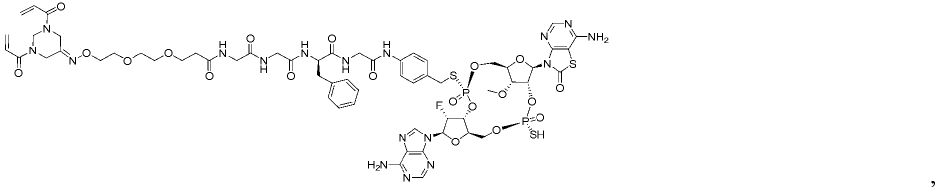 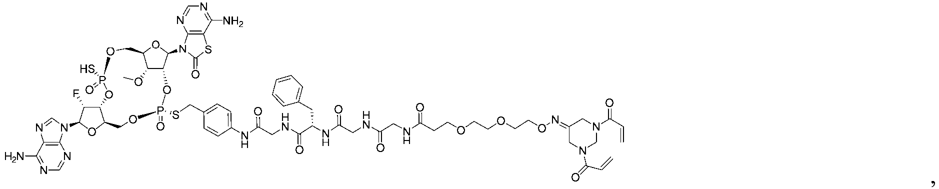 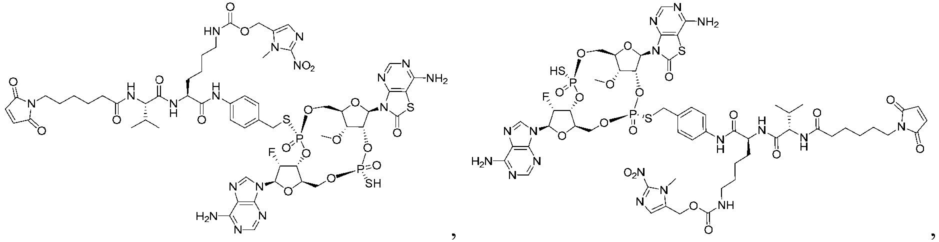 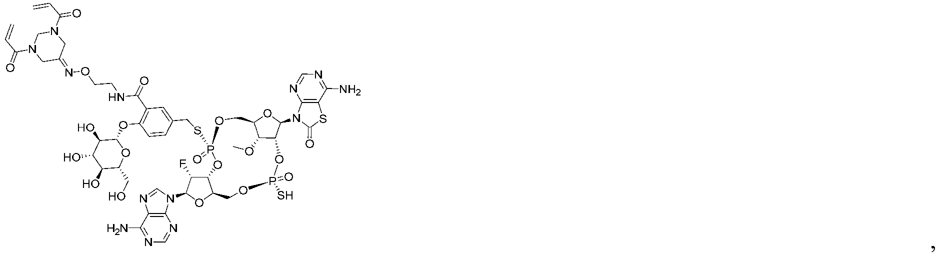 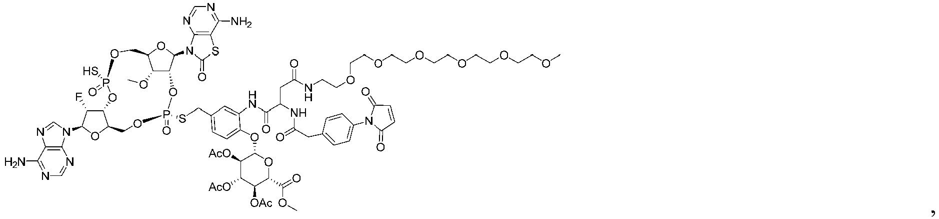 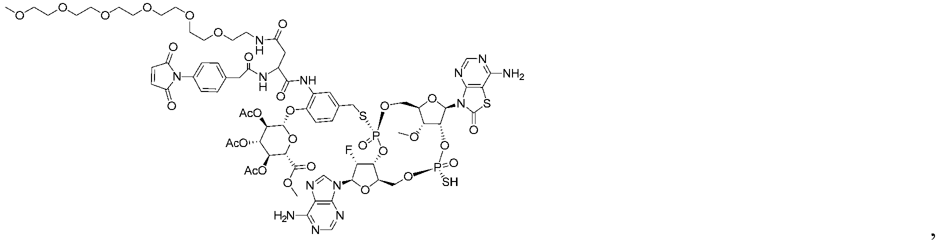 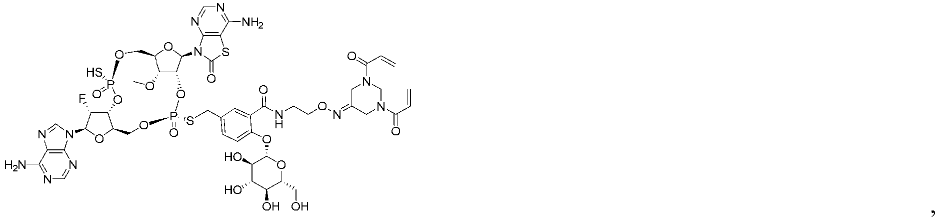 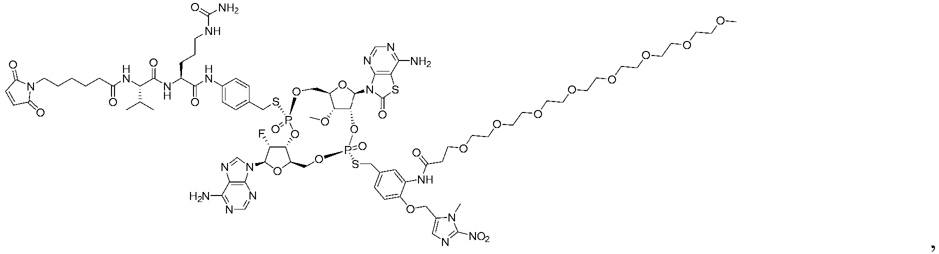 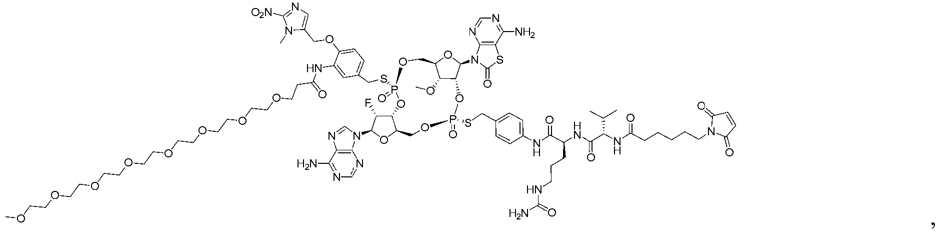 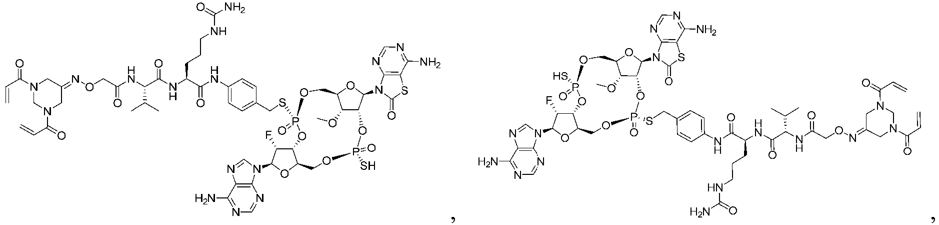 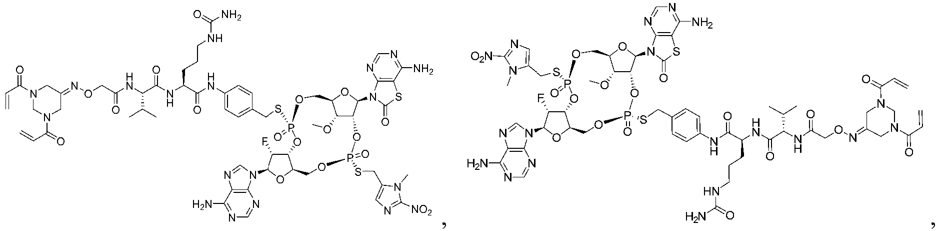 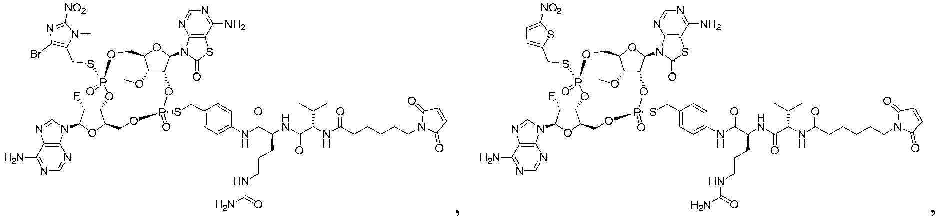 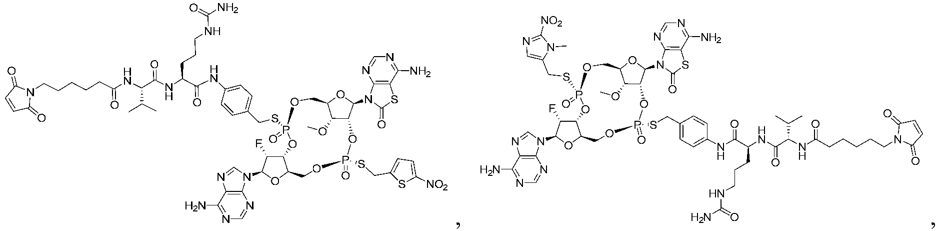 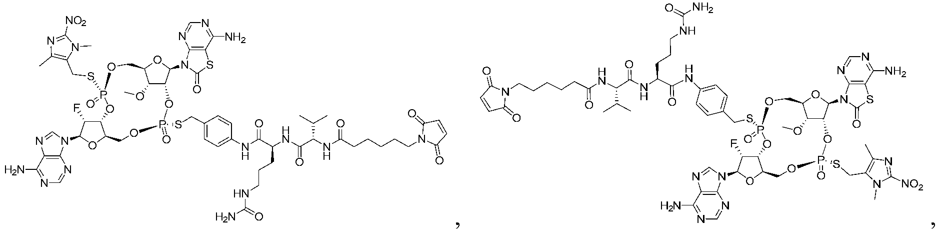 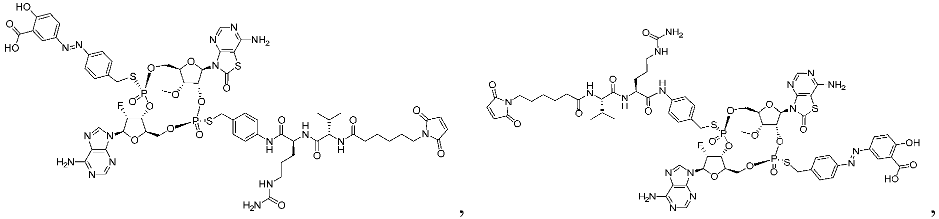 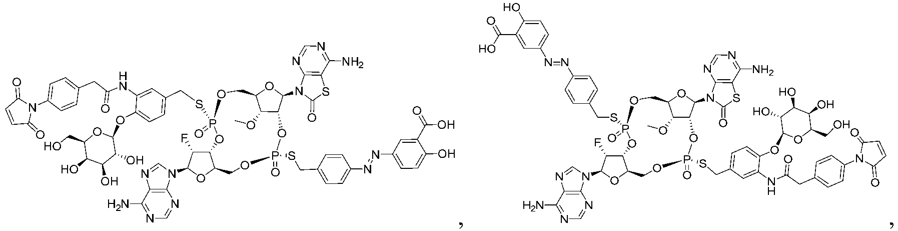 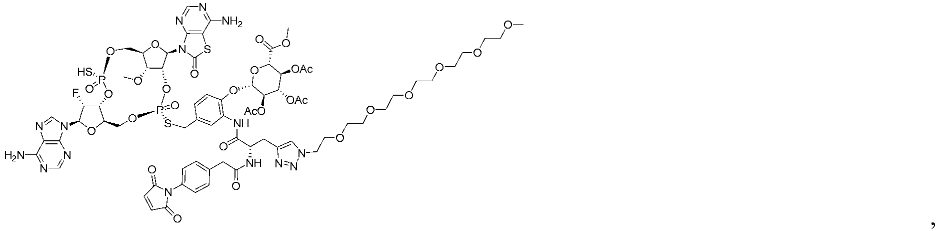 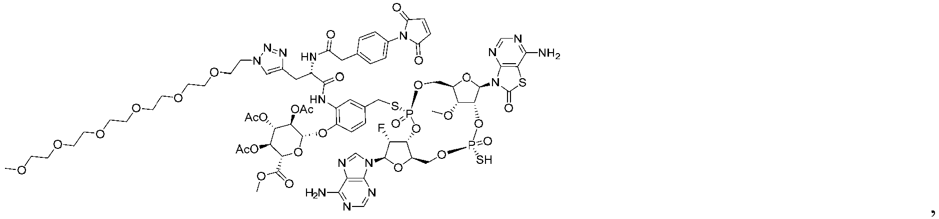 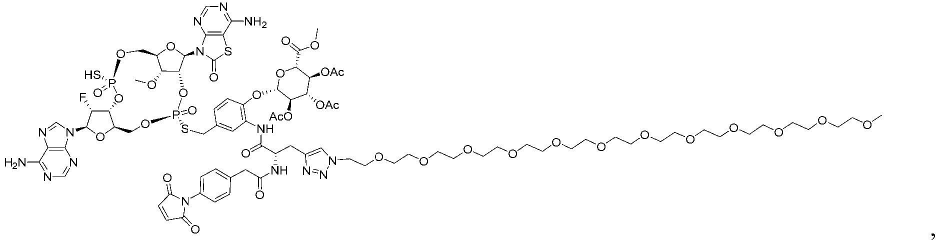 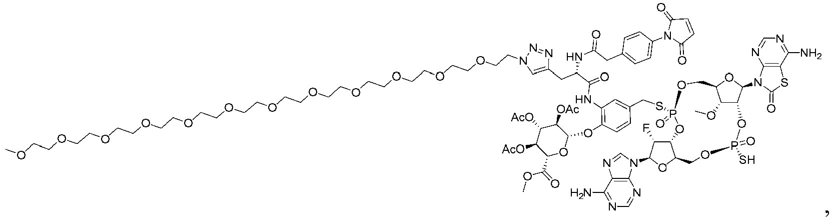 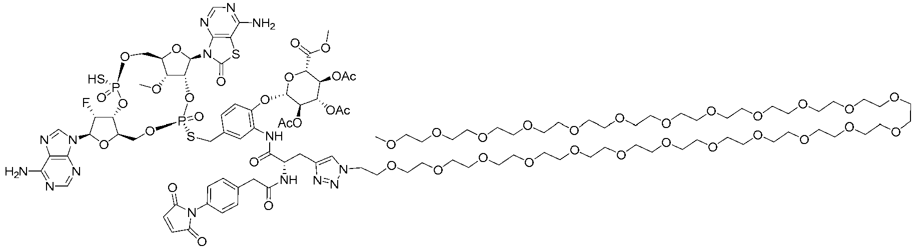 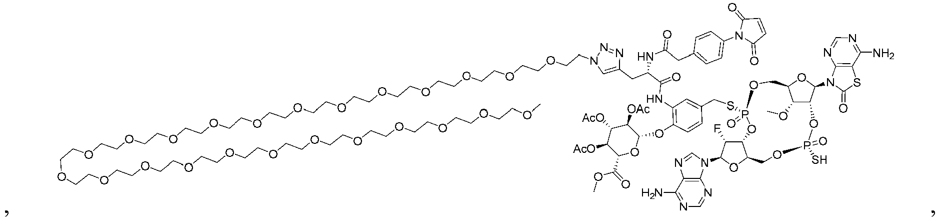 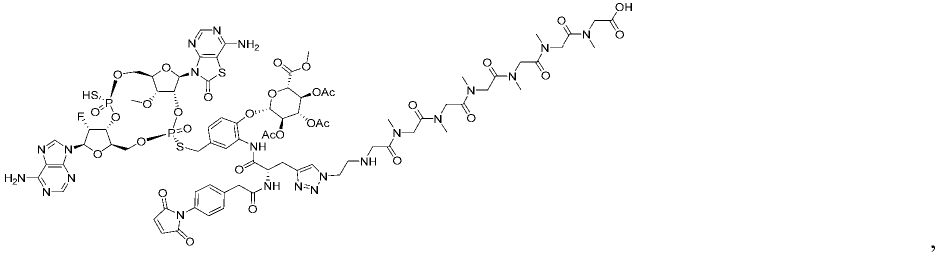 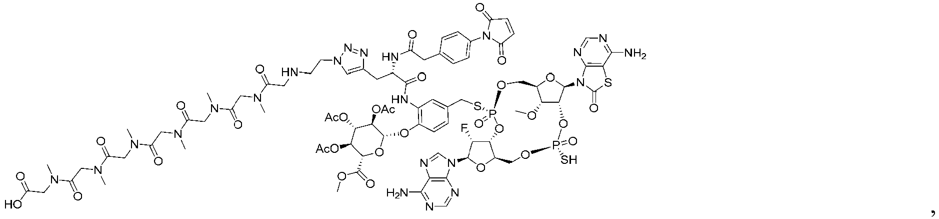 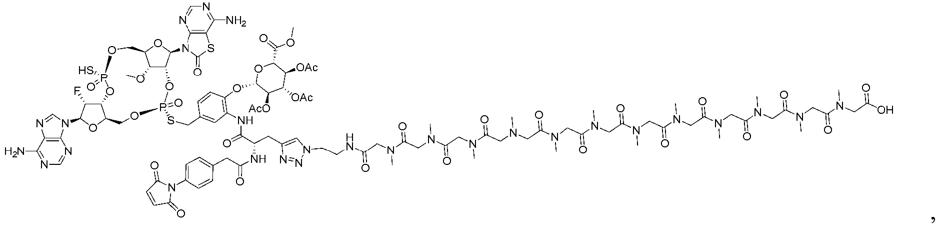 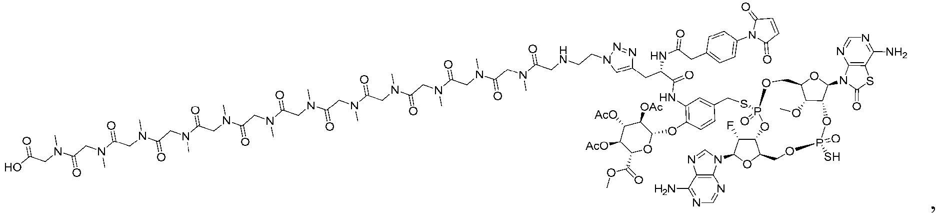 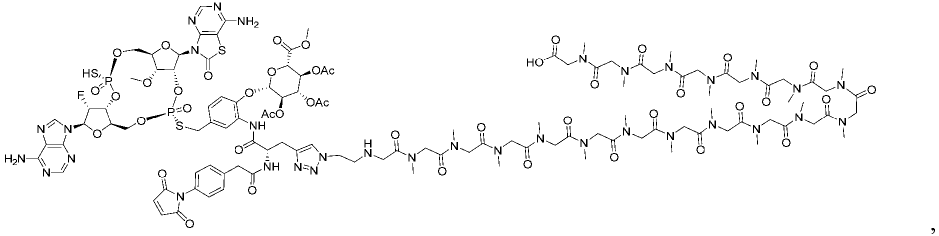 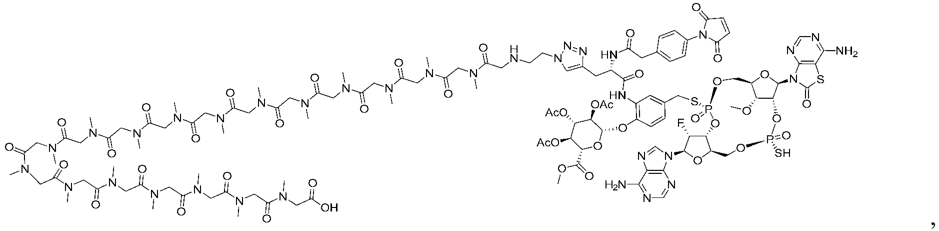 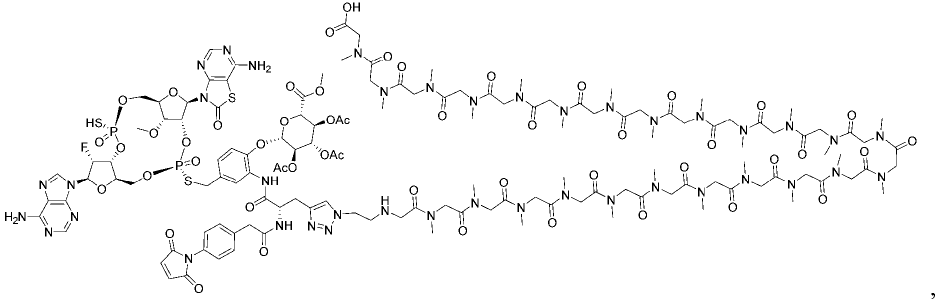 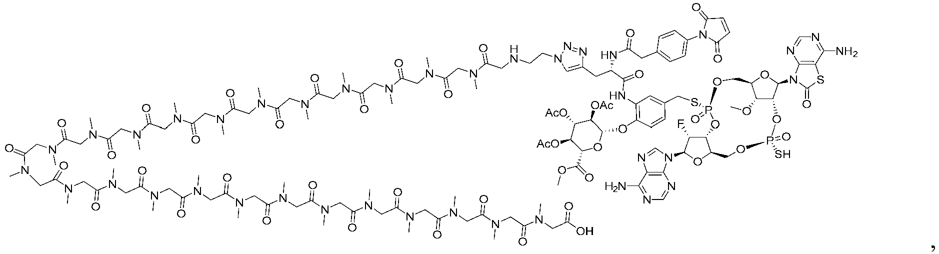 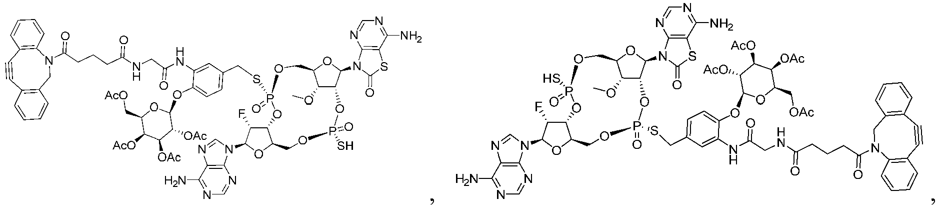 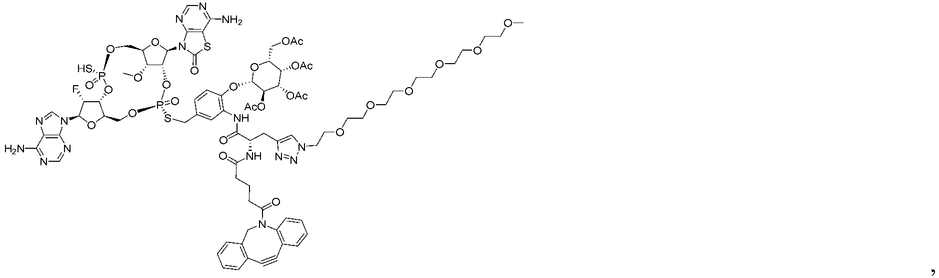 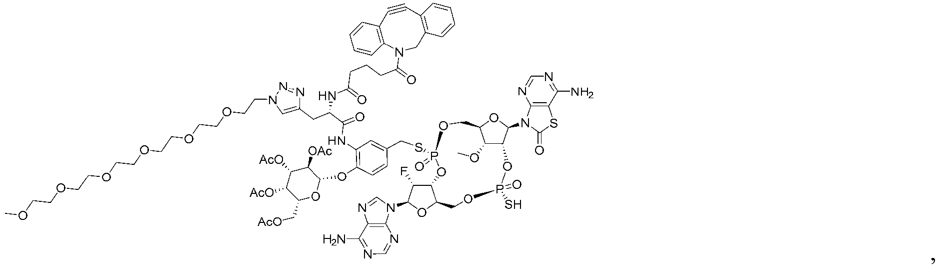 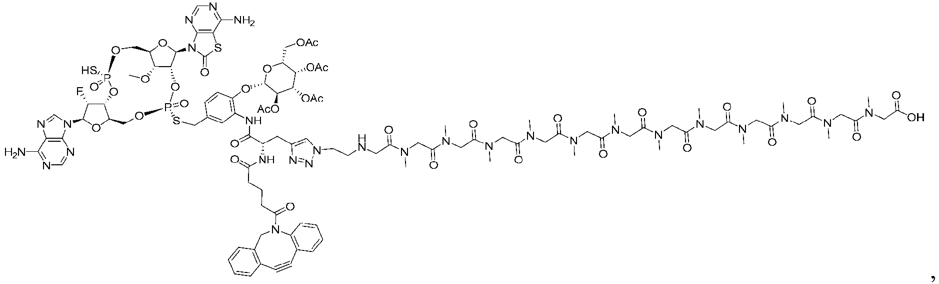 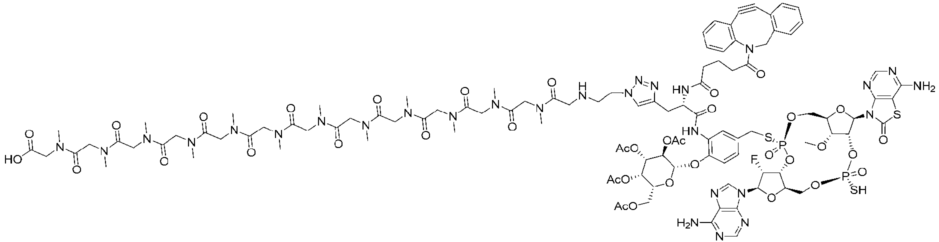 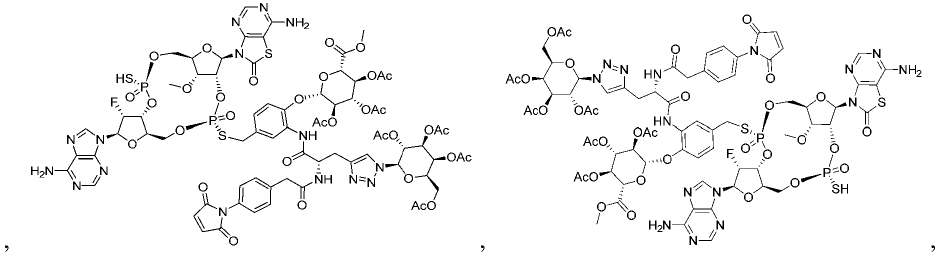 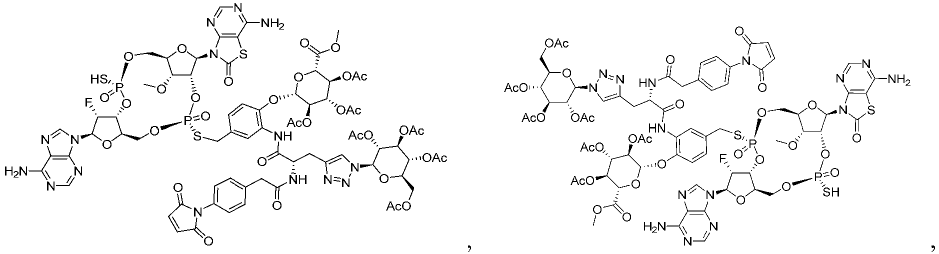 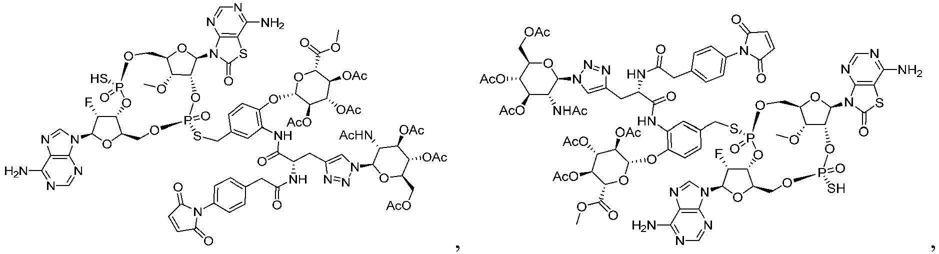 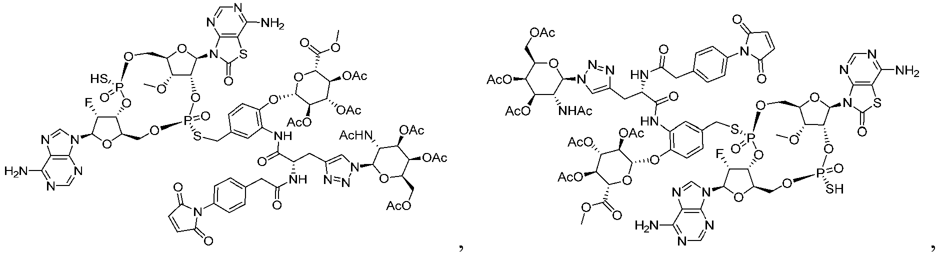 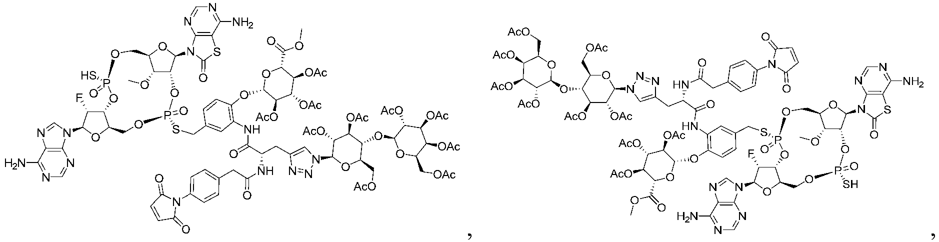 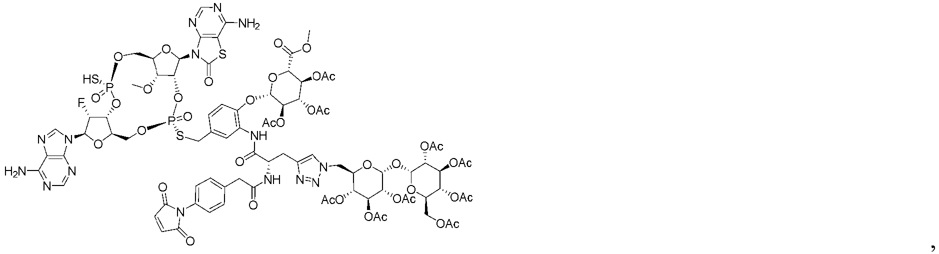 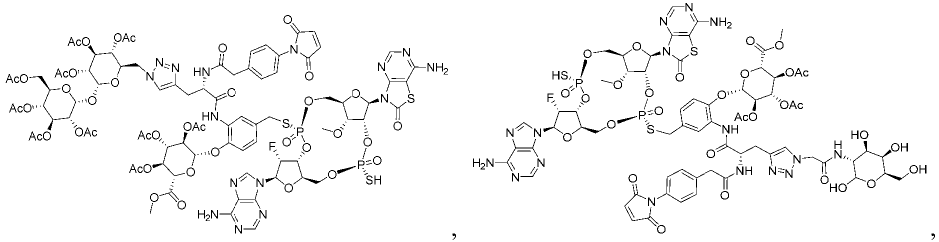 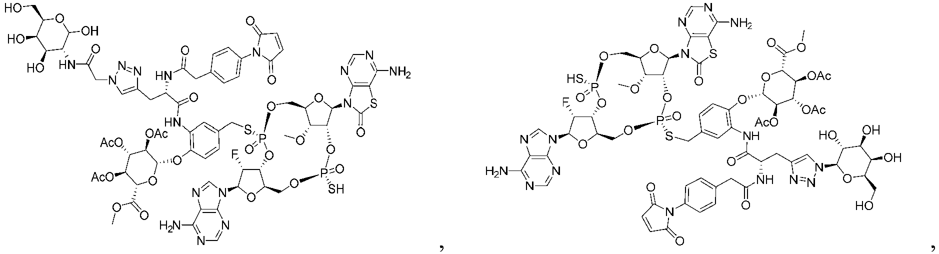 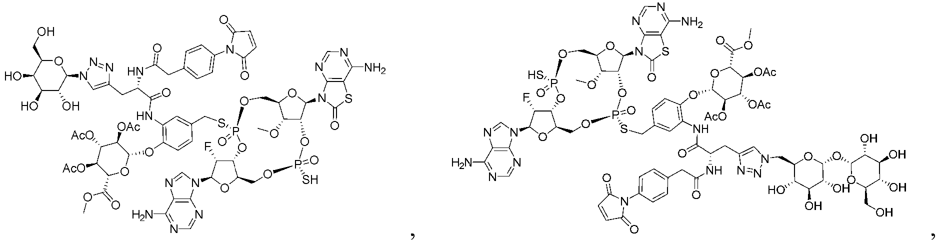 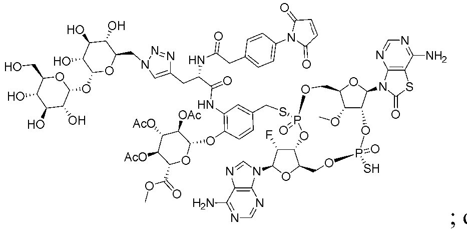 or a pharmaceutically acceptable salt thereof.

14. A compound represented by Formula D'-1 or D'-2, a stereoisomer thereof, or a pharmaceutically acceptable salt thereof; 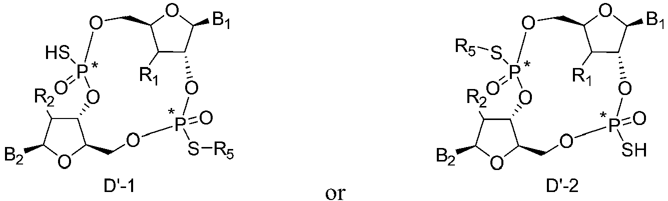
B₁ and B₂ are each independently 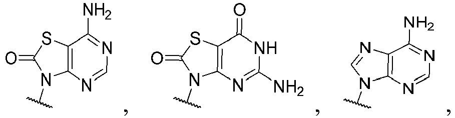 or 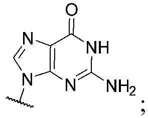 and at least one of B₁ or B₂ is 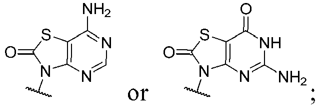 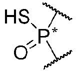
is 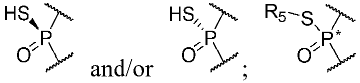 is 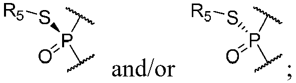
R₁ and R₂ are independently -OH, -N₃, F, -OCH₃, or -SCF₃;
R₅ is -(L₂)_{d}-(Z)ₑ-H;
d is 0, 1, 2, 3, 4, 5, or 6; e is 0, 1, 2, 3, 4, 5, 6, 7, 8, 9, or 10;
each L₂ is independently 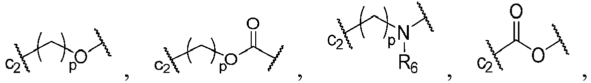 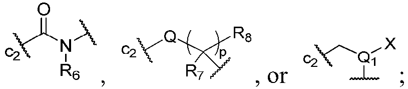 the c₂ side is connected to D;
X is 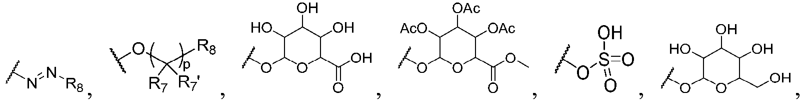 or 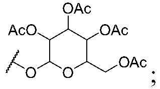
p is independently 1, 2, or 3;
Q is a linking bond, -C(O)O-, -C(O)N(R₆)-, or -C₁₋₃ alkylene-O-;
Q₁ is phenyl or pyridyl;
R₆ is H or C₁₋₆ alkyl; the C₁₋₆ alkyl is unsubstituted or optionally substituted at any position with one C₁₋₆ alkylamino or C₁₋₆ alkylsulfonyl;
R₇ and R_{7'} are each independently H or C₁₋₆ alkyl;
R₈ is phenyl or 5- to 10-membered heteroaryl; the R₈ is unsubstituted or optionally substituted at any position with 1-3 substituents selected from hydroxyl, amino, carboxyl, cyano, nitro, thiol, C₁₋₆ alkyl, C₃₋₈ cycloalkyl, halogen, halo-C₁₋₆ alkyl, C₁₋₆ alkylamino, C₁₋₆ alkoxy, halo-C₁₋₆ alkoxy, C₁₋₆ alkylthio, amido, hydroxyamino, aldehyde, acetyl, methoxycarbonyl, amino-C₁₋₆ alkyl, hydroxy-C₁₋₆ alkyl, and C₁₋₆ alkylsulfonyl;
each Z is independently -C(O)-(CH₂)ₓ-, -NH-(CH₂)_{y}-, -O-(CH₂)_{y}-, C₁₋₆ alkylene, or C₂₋₆ alkenylene;
each x and each y is independently 0, 1, 2, 3, 4, 5, 6, 7, 8, 9, or 10.

15. The compound represented by Formula D'-1 or D'-2, the stereoisomer thereof, or the pharmaceutically acceptable salt thereof according to claim 14, wherein Rs is -L₂-(Z)ₑ-H; -L₂- is 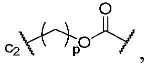 the c₂ side is connected to the thiol group in D; e is 1; -(Z)ₑ-H is C₁₋₆ alkyl;
or, R₅ is -L₂-(Z)ₑ-H; -L₂- is 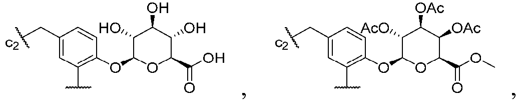 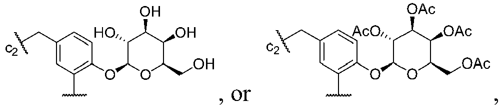 the c₂ side is connected to the thiol group in D; e is 3, -(Z)ₑ-H is -C(O)NH-(CH₂)_{y}-O-(CH₂)_{y}-H;
or, R₅ is 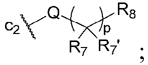 Q is a linking bond, -C(O)O-, or -C₁₋₃ alkylene-O-; R₇ and R_{7'} are each independently H or methyl;
or, R₅ is 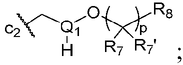 Q₁ is phenyl; R₇ and R_{7'} are each independently H or methyl;
and/or, R₈ is phenyl or 5- to 6-membered heteroaryl; the 5- to 6-membered heteroaryl is preferably pyridyl, pyrimidinyl, pyrazinyl, pyrrolyl, thienyl, furyl, pyrazolyl, thiazolyl, or oxazolyl; R₈ is unsubstituted or optionally substituted at any position with 1 to 3 substituents selected from hydroxyl, amino, cyano, nitro, thiol, C₁₋₆ alkyl, halogen, halo-C₁₋₆ alkyl, C₁₋₆ alkylamino, C₁₋₆ alkoxy, halo-C₁₋₆ alkoxy, C₁₋₆ alkylthio, amido, aldehyde, acetyl, methoxycarbonyl, amino-C₁₋₆ alkyl, hydroxy-C₁₋₆ alkyl, and methylsulfonyl.

16. The compound represented by Formula D'-1 or D'-2, the stereoisomer thereof, or the pharmaceutically acceptable salt thereof according to claim 15, wherein Rs is 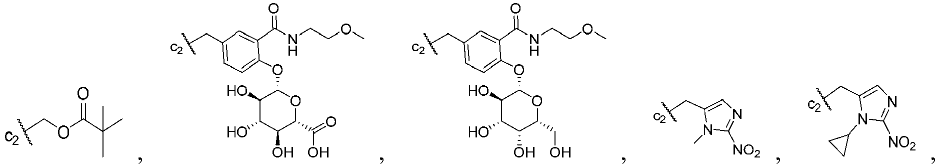 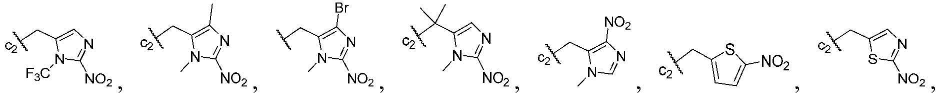 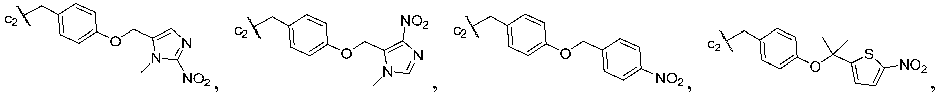 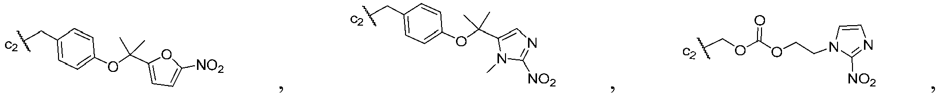 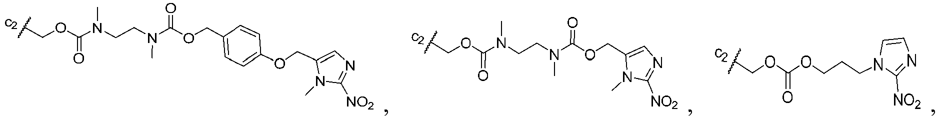 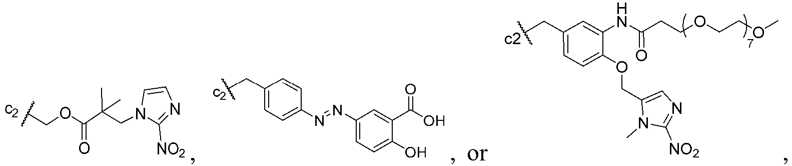 the c₂ side is connected to the thiol group in D.

17. The compound represented by Formula D'-1 or D'-2, the stereoisomer thereof, or the pharmaceutically acceptable salt thereof according to claim 14, wherein the compound represented by Formula D'-1 or D'-2 is any one of the following structures: 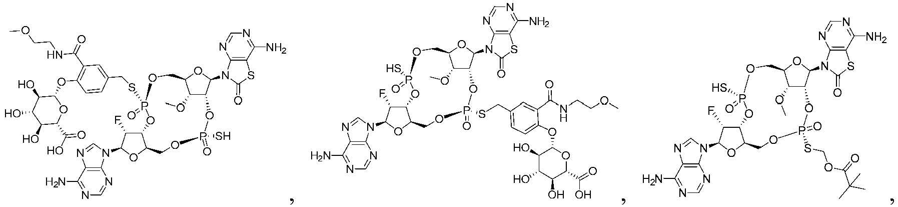 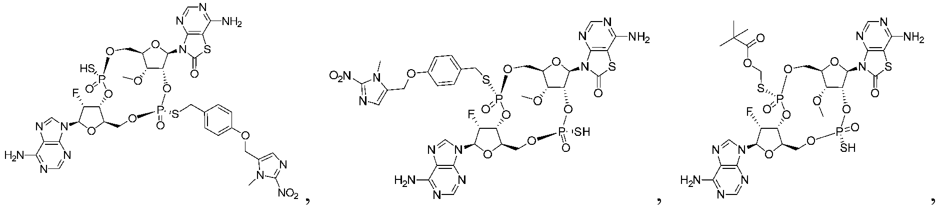 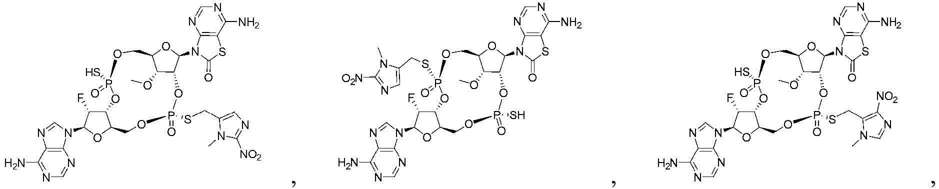 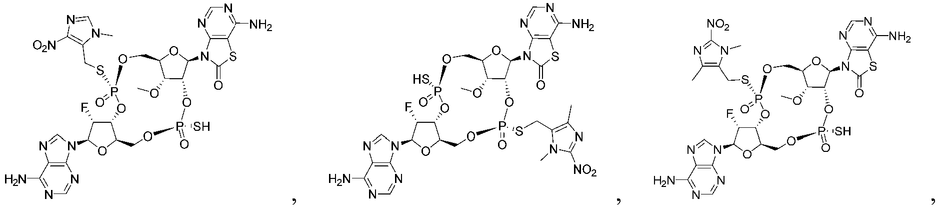 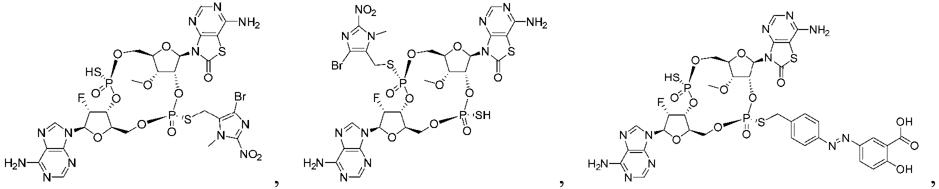 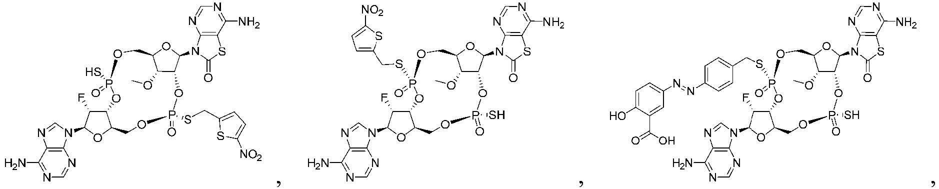 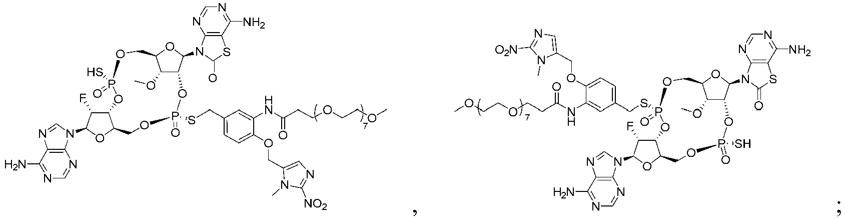 or a pharmaceutically acceptable salt thereof.

18. A pharmaceutical composition comprising substance K and a pharmaceutically acceptable excipient; wherein substance K is the immune-stimulating antibody conjugate represented by Formula II or the pharmaceutically acceptable salt thereof according to any one of claims 1-9, or the cyclic dinucleotide compound represented by Formula I or the pharmaceutically acceptable salt thereof according to any one of claims 10-13, or the compound represented by Formula D'-1 or D'-2, the stereoisomer thereof, or pharmaceutically acceptable salt thereof according to any one of claims 14-17.

19. Use of substance K or the pharmaceutical composition according to claim 17 in the manufacture of a medicament for treating and/or alleviating tumors, wherein substance K is the immune-stimulating antibody conjugate represented by Formula II or the pharmaceutically acceptable salt thereof according to any one of claims 1-9, or the cyclic dinucleotide compound represented by Formula I or the pharmaceutically acceptable salt thereof according to any one of claims 10-13, or the compound represented by Formula D'-1 or D'-2, the stereoisomer thereof, or pharmaceutically acceptable salt thereof according to any one of claims 14-17.

20. Use of substance K or the pharmaceutical composition according to claim 17 in the manufacture of a medicament for treating and/or alleviating tumors, wherein substance K is the immune-stimulating antibody conjugate represented by Formula II or the pharmaceutically acceptable salt thereof according to any one of claims 1-9, or the cyclic dinucleotide compound represented by Formula I or the pharmaceutically acceptable salt thereof according to any one of claims 10-13, or the compound represented by Formula D'-1 or D'-2, the stereoisomer thereof, or pharmaceutically acceptable salt thereof according to any one of claims 14-17, wherein substance K or the pharmaceutical composition is used in combination with one or more other types of therapeutic agents.
